# EUROPEAN PATENT SPECIFICATION

(11) **EP 1 996 590 B1**
(45) Date of publication and mention of the grant of the patent: **06.10.2010**
(21) Application number: 07712430.3
(22) Date of filing: 05.03.2007
(51) Int. Cl.: C07D 471/10, C07D 471/20, A61P 35/04

(54) **SPIROINDOLINONE DERIVATIVES**
SPIROINDOLINONDERIVATE
DÉRIVÉS DE SPIROINDOLINONE

(30) Priority: 13.03.2006 US 781958 P; 22.01.2007 US 881756 P
(43) Date of publication of application: 03.12.2008
(73) Proprietor: F. Hoffmann-La Roche AG, 4070 Basel (CH)
(72) Inventor: CHEN, Li, Shanghai 201203 (CN); DING, Qingjie, Bridgewater, New Jersey 08807 (US); LIU, Jin-Jun, Warren, New Jersey 07059 (US); YANG, Song, Shanghai 201210 (CN); ZHANG, Zhuming, Hillsborough, New Jersey 08844 (US)
(74) Representative: Halbig, Dirk
(86) International application number: PCT/EP2007/052038
(87) International publication number: WO 2007/104664

(56) References cited:
- WO-A1-01/05790

## Description

The present invention relates to spiroindolinone derivatives of the formula 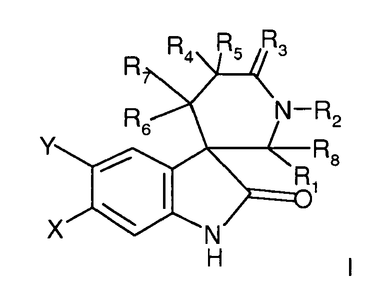 and pharmaceutically acceptable salts and esters thereof,
wherein R₁, R₂, R₃, R₄, R₅, R₆, R₇ and R₈, are as herein described.

The compounds have utility as antiproliferative agents, especially, as anticancer agents.

p53 is a tumor suppresser protein that plays a central role in protection against development of cancer. It guards cellular integrity and prevents the propagation of permanently damaged clones of cells by the induction of growth arrest or apoptosis. At the molecular level, p53 is a transcription factor that can activate a panel of genes implicated in the regulation of cell cycle and apoptosis. p53 is a potent cell cycle inhibitor which is tightly regulated by MDM2 at the cellular level. MDM2 and p53 form a feedback control loop. MDM2 can bind p53 and inhibit its ability to transactivate p53-regulated genes. In addition, MDM2 mediates the ubiquitin-dependent degradation of p53. p53 can activate the expression of the MDM2 gene, thus raising the cellular level of MDM2 protein. This feedback control loop insures that both MDM2 and p53 are kept at a low level in normal proliferating cells. MDM2 is also a cofactor for E2F, which plays a central role in cell cycle regulation.

The ratio of MDM2 to p53 (E2F) is dysregulated in many cancers. Frequently occurring molecular defects in the p16INK4/p19ARF locus, for instance, have been shown to affect MDM2 protein degradation. Inhibition of MDM2-p53 interaction in tumor cells with wild-type p53 should lead to accumulation of p53, cell cycle arrest and/or apoptosis. MDM2 antagonists, therefore, can offer a novel approach to cancer therapy as single agents or in combination with a broad spectrum of other antitumor therapies. The feasibility of this strategy has been shown by the use of different macromolecular tools for inhibition of MDM2-p53 interaction (e.g. antibodies, antisense oligonucleotides, peptides). MDM2 also binds E2F through a conserved binding region as p53 and activates E2F-dependent transcription of cyclin A, suggesting that MDM2 antagonists might have effects in p53 mutant cells.

A series of spiroindolinone as antagonists of MDM2 has previously been disclosed in J. Am Chem. Soc., 2005, 127, 10130.

The present invention provides spiroindolinone derivatives which are small molecule inhibitors of the MDM2-p53 interaction. In cell-free and cell-based assays, compounds of the present invention are shown to inhibit the interaction of MDM2 protein with a p53-like peptide. In cell-based assays, these compounds demonstrate mechanistic activity. Incubation of cancer cells with wild-type p53 leads to accumulation of p53 protein, induction of p53-regulated p21 gene, and cell cycle arrest in G1 and G2 phase, resulting in potent antiproliferative activity against wild-type p53 cells in vitro. In contrast, these activities were not observed in cancer cells with mutant p53 at comparable compound concentrations. Therefore, the activity of MDM2 antagonists is likely linked to its mechanism of action. These compounds can be potent and selective anticancer agents.

The present invention relates to 3,3'-spiroindolinones of the formula 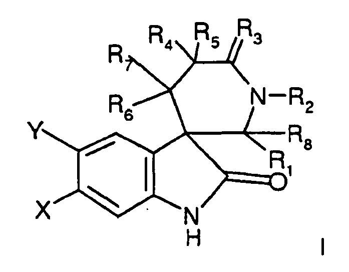 wherein
X is selected from the group consisting of hydrogen, halogen, cyano, nitro, ethynyl, cyclopropyl, methyl, ethyl, isopropyl, methoxy and vinyl;
Y is hydrogen or fluorine; R₄ and R₅ are hydrogen or lower alkyl having from 1 to 8 carbon atoms;
one of R₁ and R₈ is selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl, and substituted cycloalkenyl and the other is hydrogen;
one of R₆ and R₇ is selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl, and substituted cycloalkenyl and the other is hydrogen, cyano, or lower alkyl;
R₂ is selected from the group consisting of hydrogen, lower alkyl having from 1 to 8 carbon atoms and substituted lower alkyl having from 1 to 8 carbon atoms;
R₃ is selected from the group consisting of oxygen, sulfur and NNH(C=O)OR₉;
R₉ is lower alkyl having from 1 to 8 carbon atoms or substituted lower alkyl having from 1 to 8 carbon atoms;
and the pharmaceutically acceptable salts and esters thereof.

Preferred are compounds of formula I having a stereochemical structure as shown as formula II 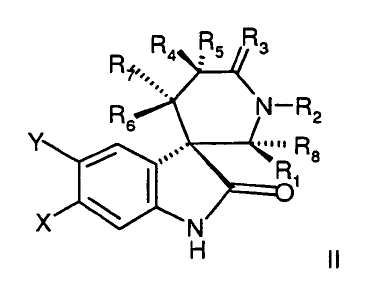 wherein
X is hydrogen, halogen, cyano, nitro, ethynyl, cyclopropyl, methyl, ethyl, isopropyl, methoxy, and vinyl;
Y is hydrogen or fluorine;
R₁ is hydrogen;
R₂ is hydrogen, lower alkyl having from 1 to 8 carbon atoms or substituted lower alkyl having from 1 to 8 carbon atoms;
R₄ and R₅ are hydrogen or lower alkyl having from 1 to 8 carbon atoms;
R₆ is hydrogen, cyano, or lower alkyl having from 1 to 8 carbon atoms;
R₃ is O, S or NNH(C=O)OR₉;
R₇/R₈ is independently selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl, and substituted cycloalkenyl;
R₉ is lower alkyl having from 1 to 8 carbon atoms or substituted lower alkyl having from 1 to 8 carbon atoms;

Further preferred are compounds of formula II wherein
X is Cl or Br;
Y is hydrogen;
R₁ is hydrogen;
R₄ and R₅ are both hydrogen;
R₆ is hydrogen;
R₃ is O;
R₇ is a substituted phenyl or substituted heteroaryl with the substituted phenyl or substituted heteroaryl selected from group consisting of: 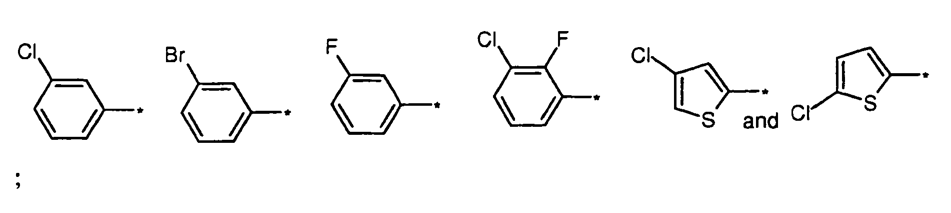 R₈ is independently selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, cycloalkyl, substituted cycloalkyl, cycloalkenyl and substituted cycloalkenyl; and
R₂ is hydrogen, lower alkyl having from 1 to 8 carbon atoms or substituted lower alkyl having from 1 to 8 carbon atoms;
with the proviso that when R₂ is lower alkyl having from 1 to 8 carbon atoms or substituted lower alkyl having from 1 to 8 carbon atoms, R₈ is selected from lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, cycloalkyl, substituted cycloalkyl, cycloalkenyl or substituted cycloalkenyl.

Also further preferred are compounds of formula II wherein
X is -Cl or -Br;
Y is hydrogen;
R₁ is hydrogen;
R₄ and R₅ are both hydrogen;
R₆ is hydrogen;
R₃ is O;
R₇ is a substituted phenyl or substituted heteroaryl with the substituted phenyl or substituted heteroaryl selected from group consisting of: 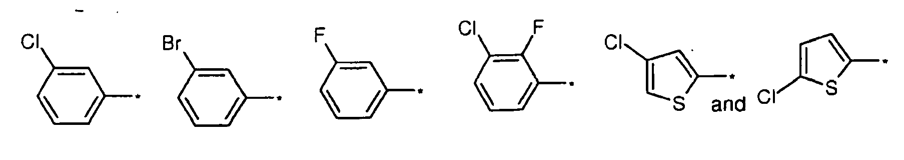 ; and
R₈ is selected from the group consisting of : 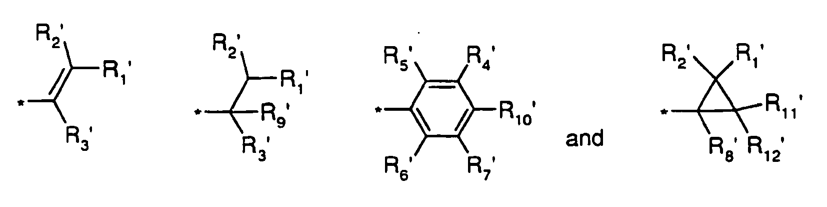 wherein
R₁' is hydrogen, methyl, ethyl, propyl, isopropyl, -CF₃, -F, -CHF₂, or -CH₂F;
R₂' is hydrogen, methyl, ethyl, propyl, isopropyl, -CF₃, -F, -CHF₂, or -CH₂F;
R₃' is hydrogen, -F, -CF₃,-CH2_{F} , methyl, ethyl, propyl ,isopropyl, cyclopropyl, tert-butyl or sec-butyl;
R₄' is hydrogen, -F, -Cl, -Br, -I, methyl, ethyl, cyclopropyl, cyano, methoxy, or ethynyl;
R₅' is hydrogen, -F or methyl;
R₆' is selected from the group consisting of hydrogen, halogen, hydroxy, cyano, lower alkyl having from 1 to 8 carbon atoms, substituted lower alkyl having from 1 to 8 carbon atoms, lower alkoxy having from 1 to 8 carbon atoms, substituted lower alkoxy having from 1 to 8 carbon atoms, lower alkynyl having from 2 to 6 carbon atoms, substituted lower alkynyl having from 2 to 6 carbon atoms;
R₇' is hydrogen, -F or methyl;
R₈' is hydrogen, -F, methyl, ethyl, propyl, isopropyl, tert-butyl or sec-butyl;
R₉' is hydrogen, hydroxyl, or -F;
R₁₀' is hydrogen or -F;
R₁₁' is hydrogen or methyl;
R₁₂' is hydrogen or methyl;
R₂ is hydrogen, lower alkyl having from 1 to 8 carbon atoms or substituted lower alkyl having from 1 to 8 carbon atoms;
with the proviso that when R₂ is lower alkyl having from 1 to 8 carbon atoms or substituted lower alkyl having from 1 to 8 carbon atoms, R₈ is selected from:

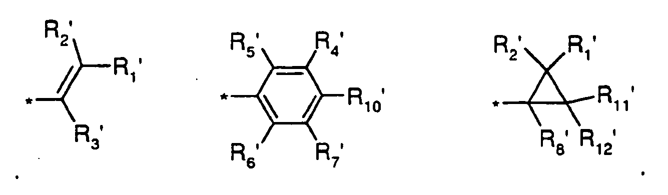

Also preferred are compounds of formula I having a stereochemical structure as shown in structure III 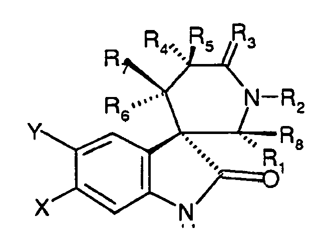 wherein
X is selected from the group consisting of hydrogen, halogen, cyano, nitro, ethynyl, cyclopropyl, methyl, ethyl, isopropyl, methoxy, and vinyl;
Y is hydrogen or fluorine;
R₁ is hydrogen;
R₂ is hydrogen, lower alkyl having from 1 to 8 carbon atoms or substituted lower alkyl having from 1 to 8 carbon atoms;
R₄ and R₅ are hydrogen or lower alkyl having from 1 to 8 carbon atoms;
R₆ is hydrogen, cyano or lower alkyl having from 1 to 8 carbon atoms;
R₃ is O, S or NNH(C=O)OR₉;
R₇/R₈ is independently selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl and substituted cycloalkenyl, and
R₉ is lower alkyl having from 1 to 8 carbon atoms or substituted lower alkyl having from 1 to 8 carbon atoms.

Further preferred are compounds of formula III wherein
X is -Cl or -Br;
Y is hydrogen;
R₁ is hydrogen;
R₂ is hydrogen, lower alkyl having from 1 to 8 carbon atoms or substituted lower alkyl having from 1 to 8 carbon atoms;
R₄ and R₅ are both hydrogen;
R₆ is hydrogen;
R₃ is O;
R₈ is a substituted phenyl with the substituted phenyl selected from group consisting of 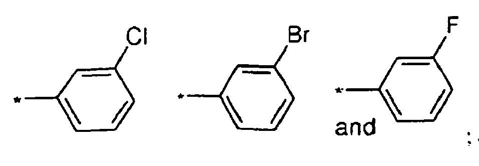 and
R₇ is selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl and substituted cycloalkenyl.

Also further preferred are the compound of formula I, II or III wherein
X is -Cl;
Y; R₁; R₂; R₄; R₅ and R₆ are hydrogen; R₃ is oxygen; and
R₇ and R₈ are phenyl groups, which are each independently unsubstituted or 1, 2 or 3 times substituted by
lower alkyl having from 1 to 8 carbon atoms, lower-alkenyl having from 2 to 8 carbon atoms, lower-alkynyl having from 2 to 6 carbon atoms, dioxo-lower-alkylene having from 1 to 8 carbon atoms (forming e.g. a benzodioxyl group), halogen, hydroxy, -CN, -CF₃, -NH₂, -N(H, lower-alkyl having from 1 to 8 carbon atoms), N(lower-alkyl having from 1 to 8 carbon atoms)₂, aminocarbonyl, carboxy, -NO₂, lower-alkoxy having from 1 to 8 carbon atoms, thio-lower-alkoxy having from 1 to 8 carbon atoms, lower-alkylsulfonyl having from 1 to 8 carbon atoms, aminosulfonyl, lower-alkyl (having from 1 to 8 carbon atoms)carbonyl, lower-alkyl (having from 1 to 8 carbon atoms)carbonyloxy, lower-alkoxy(having from 1 to 8 carbon atoms)carbonyl, lower-alkyl(having from 1 to 8 carbon atoms)carbonyl-NH, fluoro-lower-alkyl having from 1 to 8 carbon atoms, fluoro-lower-alkoxy having from 1 to 8 carbon atoms, lower-alkoxy(having from 1 to 8 carbon atoms)-carbonyl-lower-alkoxy(having from 1 to 8 carbon atoms), carboxy-lower-alkoxy(having from 1 to 8 carbon atoms), carbamoyl-lower-alkoxy(having from 1 to 8 carbon atoms), hydroxy-lower-alkoxy having from 1 to 8 carbon atoms, -NH₂-lower-alkoxy having from 1 to 8 carbon atoms, -N(H, lower-alkyl having from 1 to 8 carbon atoms)-lower-alkoxy having from 1 to 8 carbon atoms, -N(lower-alkyl having from 1 to 8 carbon atoms)₂-lower-alkoxy having from 1 to 8 carbon atoms, benzyloxy-lower-alkoxy having from 1 to 8 carbon atoms, mono- or di-(lower-alkyl having from 1 to 8 carbon atoms) substituted aminosulfonyl and lower alkyl having from 1 to 8 carbon atoms, which can optionally be substituted with halogen, hydroxy, -NH₂, -N(H, lower-alkyl having from 1 to 8 carbon atoms) or -N(lower-alkyl having from 1 to 8 carbon atoms)₂.

Especially preferred are the compounds of formula I, II or III wherein
X is -Cl;
Y; R₁; R₂; R₄; R₅ and R₆ are hydrogen;
R₃ is oxygen; and
R₇ and R₈ are phenyl groups, which are each independently unsubstituted or 1, 2 or 3 times substituted by a substituent independently selected from
halogen, C₂₋₄-alkynyl or -O-(C₁₋₄ alkyl), and
said C₂₋₄-alkynyl or -O-(C₁₋₄ alkyl) groups are unsubstituted or once substituted by hydroxyl or -N(C₁₋₄ alkyl)-S(O)₂-CH₃.

In particular, preferred compounds are those of the formula
(2'S, 3S, 4'S)-6-chloro-2'-(3-chlorophenyl)-4'-(2,2-dimethylpropyl) spiro[3H-indole-3,3'-piperidine]-2,6'(1H)-dione,
(2'SR, 3S, 4'R)-4'-(*tert*-butyl)-6-chloro-2'-(3-chlorophenyl) spiro[3H-indole-3,3'-piperidine]-2,6'(1H)-dione,
(2'S, 3S, 4'R)-6-chloro-2'-(3-chlorophenyl)-4'-isopropyl spiro[3H-indole-3,3'-piperidine]-2,6'(1H)-dione,
racemic (2'R, 3R, 4'S)-6-chloro-2',4'-bis(3-chlorophenyl) spiro[3H-indole-3,3'-piperidine]-2,6'(1H)-dione,
chiral (2'R, 3R, 4'S)-6-chloro-2',4'-bis(3-chlorophenyl) spiro[3H-indole-3,3'-piperidine]-2,6'(1H)-dione,
racemic (2'S, 3S, 4'R)-6-chloro-2'-(3-chlorophenyl)-4'-cyclopentyl spiro[3H-indole-3,3'-piperidine]-2,6'(1H)-dione,
racemic (2'S, 3S, 4'R)-6-chloro-2'-(3-chlorophenyl)-4'-cyclohexyl spiro[3H-indole-3,3'-piperidine]-2,6'(1H)-dione,
racemic (2'S, 3S, 4'R)-6-chloro-2'-(4-chlorophenyl)-4'-cyclohexyl spiro[3H-indole-3,3'-piperidine]-2,6'(1H)-dione,
racemic (2'R, 3R, 4'S)-6-chloro-2'-(4-chlorophenyl)-4'-(3-chlorophenyl) spiro[3H-indole-3,3'-piperidine]-2,6'(1H)-dione,
racemic (2'S, 3S, 4'R)-6-chloro-2'-(3-chlorophenyl)-4'-(4-chlorophenyl) spiro[3H-indole-3,3'-piperidine]-2,6'(1H)-dione,
racemic (2'R, 3R, 4'S)-6-chloro-4'-(3-chlorophenyl)-2'-(3-methylphenyl) spiro[3H-indole-3,3'-piperidine]-2,6'(1H)-dione,
racemic (2'R, 3R, 4'S)-6-chloro-4'-(3-chlorophenyl)-2'-(3-fluorophenyl) spiro[3H-indole-3,3'-piperidine]-2,6'(1H)-dione,
racemic (2'S, 3S, 4'R)-6-chloro-2'-(3-chlorophenyl)-4'-phenyl spiro[3H-indole-3,3'-piperidine]-2,6'(1H)-dione,
racemic (2'S, 3S, 4'R, 5'R)-6-chloro-2'-(3-chlorophenyl)-5'-methyl-4'-phenyl spiro[3H-indole-3,3'-piperidine]-2,6'(1H)-dione,
racemic (2'R, 3R, 4'S)-6-chloro-4'-(3-chlorophenyl)-2'-phenyl spiro[3H-indole-3,3'-piperidine]-2,6'(1H)-dione,
chiral (2'R, 3R, 4'S)-6-chloro-4'-(3-chlorophenyl)-2'-phenyl spiro[3H-indole-3,3'-piperidine]-2,6'(1H)-dione,
racemic (2'R, 3R, 4'S)-6-chloro-4'-(3-chlorophenyl)-2'-(3-methoxyphenyl) spiro[3H-indole-3,3'-piperidine]-2,6'(1H)-dione,
racemic (2'R, 3R, 4'S)-6-chloro-2'-(2-chlorophenyl)-4'-(3-chlorophenyl) spiro[3H-indole-3,3'-piperidine]-2,6'(1H)-dione,
chiral (2'R, 3R, 4'S)-6-chloro-2'-(2-chlorophenyl)-4'-(3-chlorophenyl) spiro[3H-indole-3,3'-piperidine]-2,6'(1H)-dione,
racemic (2'R, 3R, 4'S)-6-chloro-4'-(3-chlorophenyl)-2'-(2-methylphenyl) spiro[3H-indole-3,3'-piperidine]-2,6'(1H)-dione,
chiral (2'R, 3R, 4'S)-6-chloro-4'-(3-chlorophenyl)-2'-(2-methylphenyl) spiro[3H-indole-3,3'-piperidine]-2,6'(1H)-dione,
racemic (2'R, 3R, 4'S)-6-chloro-4'-(3-chlorophenyl)-2'-(2-ethylphenyl) spiro[3H-indole-3,3'-piperidine]-2,6'(1H)-dione,
chiral (2'R, 3R, 4'S)-6-chloro-4'-(3-chlorophenyl)-2'-(2-ethylphenyl) spiro[3H-indole-3,3'-piperidine]-2,6'(1H)-dione,
racemic (2'R, 3R, 4'S, 5'S)-6-chloro-4'-(3-chlorophenyl)-5'-methyl-2'-(2-methylphenyl) spiro[3H-indole-3,3'-piperidine]-2,6'(1H)-dione,
racemic (2'R, 3R, 4'S, 5'S)-6-chloro-2',4'-bis(3-chlorophenyl)-5'-methyl spiro[3H-indole-3,3'-piperidine]-2,6'(1H)-dione,
racemic (2'R, 3R, 4'S)-6-chloro-4'-(3-chlorophenyl)-2'-(2-isopropylphenyl) spiro[3H-indole-3,3'-piperidine]-2,6'(1H)-dione,
chiral (2'R, 3R, 4'S)-6-chloro-4'-(3-chlorophenyl)-2'-(2-isopropylphenyl) spiro[3H-indole-3,3'-piperidine]-2,6'(1H)-dione,
racemic (2'R, 3R, 4'S)-2'-(2-bromophenyl)-6-chloro-4'-(3-chlorophenyl) spiro[3H-indole-3,3'-piperidine]-2,6'(1H)-dione,
chiral (2'R, 3R, 4'S)-2'-(2-bromophenyl)-6-chloro-4'-(3-chlorophenyl) spiro[3H-indole-3,3'-piperidine]-2,6'(1H)-dione,
racemic (2'R, 3R, 4'S)-6-chloro-4'-(3-chlorophenyl)-2'-(3-cyanophenyl) spiro[3H-indole-3,3'-piperidine]-2,6'(1H)-dione,
racemic (2'R, 3R, 4'S)-6-chloro-4'-(3-chlorophenyl)-1'-methyl-2'-(2-methylphenyl) spiro[3H-indole-3,3'-piperidine]-2,6'(1H)-dione,
chiral (2'R, 3R, 4'S)-6-chloro-4'-(3-chlorophenyl)-1'-methyl-2'-(2-methylphenyl) spiro[3H-indole-3,3'-piperidine]-2,6'(1H)-dione,
racemic (2'R, 3R, 4'S)-6-chloro-4'-(3-chlorophenyl)-2'-(3-fluoro-2-methyl-phenyl) spiro[3H-indole-3,3'-piperidine]-2,6'(1H)-dione,
chiral (2'R, 3R, 4'S)-6-chloro-4'-(3-chlorophenyl)-2'-(3-fluoro-2-methyl-phenyl) spiro[3H-indole-3,3'-piperidine]-2,6'(1H)-dione,
racemic (2'R, 3R, 4'S)-6-chloro-4'-(3-chlorophenyl)-1'-ethyl-2'-(2-methylphenyl) spiro[3H-indole-3,3'-piperidine]-2,6'(1H)-dione,
chiral (2'R, 3R, 4'S)-6-chloro-4'-(3-chlorophenyl)-1'-ethyl-2'-(2-methylphenyl) spiro[3H-indole-3,3'-piperidine]-2,6'(1H)-dione,
racemic (2'R, 3R, 4'S)-6-chloro-4'-(3-chlorophenyl)-2'-(2,6-dimethylphenyl) spiro[3H-indole-3,3'-piperidine]-2,6'(1H)-dione,
racemic (2'R, 3R, 4'S)-6-chloro-4'-(3-chlorophenyl)-2'-(2,3-dimethylphenyl) spiro[3H-indole-3,3'-piperidine]-2,6'(1H)-dione,
racemic (2'R, 3R, 4'S)-6-chloro-4'-(3-chlorophenyl)-1'-[(methoxycarbonyl) methyl]-2'-(2-methylphenyl) spiro[3H-indole-3,3'-piperidine]-2,6'(1H)-dione,
racemic (2'S, 3R)-6-chloro-2'-(3-chlorophenyl)-4'-isopropyl-6'-thioxo spiro[3H-indole-3,3'-piperidine]-2(1H)-one,
racemic (2'R, 3R, 4'S)-6-chloro-4'-(3-chlorophenyl)-1'-[(hydroxycarbonyl)-methyl]-2'-(2-methylphenyl) spiro[3H-indole-3,3'-piperidine]-2,6'(1H)-dione,
racemic (2'R, 3R, 4'S)-6-chloro-4'-(3-chlorophenyl)-2'-[2-(trifluoromethyl)-phenyl] spiro[3H-indole-3,3'-piperidine]-2,6'(1H)-dione,
racemic (2'R, 3R, 4'S)-6-chloro-4'-(3-chlorophenyl)-6'-thioxo-2'-[2-(trifluoromethyl)-phenyl] spiro[3H-indole-3,3'-piperidine]-2(1H)-one,
racemic (2'R, 3R, 4'S)-6-chloro-4'-(3-chlorophenyl)-2'-(2-methylphenyl) -1'-[2-(4-morpholinyl)-carbonyl-methyl] spiro[3H-indole-3,3'-piperidine]-2,6'(1H)-dione,
chiral (2'R, 3R, 4'S)-6-chloro-4'-(3-chlorophenyl)-2'-(2-methylphenyl) -1'-[2-(4-morpholinyl)-carbonyl-methyl] spiro[3H-indole-3,3'-piperidine]-2,6'(1H)-dione,
racemic (2'R, 3R, 4'S)-6-chloro-4'-(3-chlorophenyl)-2'-[5-fluoro-2-(trifluoromethyl)-phenyl] spiro[3H-indole-3,3'-piperidine]-2,6'(1H)-dione,
racemic (2'R, 3R, 4'S)-6-chloro-4'-(3-chlorophenyl)-2'-(5-fluoro-2-methylphenyl) spiro[3H-indole-3,3'-piperidine]-2,6'(1H)-dione,
chiral (2'R, 3R, 4'S)-6-chloro-4'-(3-chlorophenyl)-2'-(5-fluoro-2-methylphenyl) spiro[3H-indole-3,3'-piperidine]-2,6'(1H)-dione,
racemic (2'R, 3R, 4'S)-6-chloro-4'-(3-chlorophenyl)-1'-[(cyclopropylamino)-carbonyl-methyl]-2'-(2-methylphenyl) spiro[3H-indole-3,3'-piperidine]-2,6'(1H)-dione,
racemic (2'R, 3R, 4'S)-6-chloro-[[2-[6-chloro-4'-(3-chlorophenyl)-2,3-dihydro-2'-(2-methylphenyl)-2,6-dioxospiro[3H-indole-3,3'-piperidin]-1-yl]-1-oxoethyl]-amino]-piperidine carboxylic acid *tert*-butyl ester,
racemic (2'R, 3R, 4'S)-6-chloro-4'-(3-chlorophenyl)-2'-(5-fluoro-2-methylphenyl)-6'-thioxo spiro[3H-indole-3,3'-piperidine]-2(1H)-one,
racemic (2'R, 3R, 4'S)-[6-chloro-4'-(3-chlorophenyl)-2,3-dihydro-2'-[2-(trifluoromethyl)phenyl)]-2-oxospiro[3H-indole-3,3'-piperidin]-6-ylene]-hydrazine carboxylic acid ethyl ester,
racemic (2'R, 3R, 4'S)-6-chloro-4'-(3-chlorophenyl)-2'-(2,4-difluorophenyl) spiro[3H-indole-3,3'-piperidine]-2,6'(1H)-dione,
racemic (2'R, 3R, 4'S)-6-chloro-4'-(3-chlorophenyl)-2'-(5-fluoro-2-methoxyphenyl) spiro[3H-indole-3,3'-piperidine]-2,6'(1H)-dione,
racemic (2'R, 3R, 4'S)-6-chloro-4'-(3-chlorophenyl)-2'-(1-naphthalenyl) spiro[3H-indole-3,3'-piperidine]-2,6'(1H)-dione,
racemic (2'R, 3R, 4'S)-6-chloro-4'-(3-chlorophenyl)-2'-(3-pyridinyl) spiro[3H-indole-3,3'-piperidine]-2,6'(1H)-dione,
racemic (2'R, 3R, 4'S)-6-chloro-4'-(3-chlorophenyl)-2'-(2, 3-difluoro-6-methoxyphenyl) spiro[3H-indole-3,3'-piperidine]-2,6'(1H)-dione,
racemic (2'R, 3R, 4'S)-6-chloro-4'-(3-chlorophenyl)-2'-(3, 4-difluorophenyl) spiro[3H-indole-3,3'-piperidine]-2,6'(1H)-dione,
racemic (2'R, 3R, 4'S)-6-chloro-4'-(3-chlorophenyl)-2'-(1-cyclohexenyl) spiro[3H-indole-3,3'-piperidine]-2,6'(1H)-dione,
racemic (2'R, 3R, 4'S)-6-chloro-4'-(3-chlorophenyl)-2'-(3, 4-difluorophenyl)- 6'-thioxo spiro[3H-indole-3,3'-piperidine]-2(1H)-one,
racemic (2'R, 3R, 4'S)-[6-chloro-4'-(3-chlorophenyl)-2'-(3, 4-difluorophenyl)-2,3-dihydro-2-oxospiro[3H-indole-3,3'-piperidin]-6-ylene] hydrazine carboxylic acid ethyl ester,
racemic (2'R, 3R, 4'S)-2'-(1,3-benzodioxol-4-yl)-6-chloro-4'-(3-chlorophenyl) spiro[3H-indole-3,3'-piperidine]-2,6'(1H)-dione,
racemic (2'R, 3R, 4'S)-[6-chloro-4'-(3-chlorophenyl)-2'-(5-fluoro-2-methylphenyl)-2,3-dihydro-2-oxospiro[3H-indole-3,3'-piperidin]-6-ylene] hydrazine carboxylic acid ethyl ester,
racemic (2'R, 3R, 4'S)-6-chloro-4'-(3-chlorophenyl)-2'-(2, 3-difluoro-6-methylphenyl) spiro[3H-indole-3,3'-piperidine]-2,6'(1H)-dione,
racemic (2'R, 3R, 4'S)-6-chloro-4'-(3-chlorophenyl)-5-fluoro-2'-(5-fluoro-2-methylphenyl spiro[3H-indole-3,3'-piperidine]-2,6'(1H)-dione,
racemic (2'R, 3R, 4'S)-6-chloro-4'-(3-chlorophenyl)-2'-(2, 3-difluoro-6-methylphenyl)-6'-thioxo spiro[3H-indole-3,3'-piperidine]-2(1H)-one,
racemic (2'R, 3R, 4'S) -6-chloro-4'-(3-chlorophenyl)-2'-(5-fluoro-2-methylphenyl)-1'-hydroxycarbonylmethyl spiro[3H-indole-3,3'-piperidine]-2,6'(1H)-dione,
racemic (2'R, 3R, 4'S)-6-chloro-4'-(3-chlorophenyl)-2'-(5-fluoro-2-methylphenyl)-1'-methyl spiro[3H-indole-3,3'-piperidine]- 2,6'(1H)-dione,
chiral (2'R, 3R, 4'S)-6-chloro-4'-(3-chlorophenyl)-2'-(5-fluoro-2-methylphenyl)-1'-methyl spiro[3H-indole-3,3'-piperidine]- 2,6'(1H)-dione,
racemic (2'R, 3R, 4'S)-6-chloro-4'-(3-chlorophenyl)-1'-fluorocarbonylmethyl-2'-(5-fluoro-2-methylphenyl) spiro[3H-indole-3,3'-piperidine]-2,6'(1H)-dione,
racemic (2'R, 3R, 4'S)-6-chloro-4'-(3-chlorophenyl)-2'-(5-fluoro-2-methylphenyl)-1'-[(1-methyl-piperidin-4-yl)aminocarbonyl)-methyl] spiro[3H-indole-3,3'-piperidine]-2,6'(1H)-dione,
chiral (2'R, 3R, 4'S)-6-chloro-4'-(3-chlorophenyl)-2'-(5-fluoro-2-methylphenyl)-1'-[(1-methyl-piperidin-4-yl)aminocarbonyl)-methyl] spiro[3H-indole-3,3'-piperidine]-2,6'(1H)-dione,
racemic (2'R, 3R, 4'S)-1'-[1-tert-butoxycarbonyl-piperidin-4-yl)aminocarbonyl-methyl] 6-chloro-4'-(3-chlorophenyl)-2'-(5-fluoro-2-methylphenyl) spiro[3H-indole-3,3'-piperidine]-2,6'(1H)-dione,
racemic (2'R, 3R, 4'S)-6-chloro-4'-(3-chlorophenyl)-2'-(5-fluoro-2-methylphenyl)-1'-[(piperidin-4-yl)aminocarbonyl-methyl] spiro[3H-indole-3,3'-piperidine]-2,6'(1H)-dione,
chiral (2'R, 3R, 4'S)-6-chloro-4'-(3-chlorophenyl)-2'-(5-fluoro-2-methylphenyl)-1'-[(piperidin-4-yl)aminocarbonyl-methyl] spiro[3H-indole-3,3'-piperidine]-2,6'(1H)-dione,
racemic (2'R, 3R, 4'S)-6-chloro-4'-(3-chlorophenyl)-2'-(5-fluoro-2-methylphenyl)-1'-(3-morpholin-4-yl-propyl) spiro[3H-indole-3,3'-piperidine]-2,6'(1H)-dione,
chiral (2'R, 3R, 4'S)-6-chloro-4'-(3-chlorophenyl)-2'-(5-fluoro-2-methylphenyl)-1'-(3-morpholin-4-yl-propyl) spiro[3H-indole-3,3'-piperidine]-2,6'(1H)-dione,
racemic (2'R, 3R, 4'S)-6-chloro-4'-(3-chlorophenyl)-2'-(1-isopropyl-4-methyl-pent-1-enyl) spiro[3H-indole-3,3'-piperidine]-2,6'(1H)-dione,
racemic (2'R, 3R, 4'S)-6-chloro-4'-(3-chlorophenyl)-2'-(2,3-difluoro-6-isopropoxyphenyl) spiro[3H-indole-3,3'-piperidine]-2,6'(1H)-dione,
chiral(2'R, 3R, 4'S)-6-chloro-4'-(3-chlorophenyl)-2'-(2,3-difluoro-6-isopropoxyphenyl) spiro[3H-indole-3,3'-piperidine]-2,6'(1H)-dione,
racemic (2'R, 3R, 4'S)-6-chloro-4'-(3-chlorophenyl)-2'-(5-fluoro-2-methylphenyl)-1'-(3-pyrrolidin-1-yl-propyl) spiro[3H-indole-3,3'-piperidine]-2,6'(1H)-dione,
racemic (2'R, 3R, 4'S)-6-chloro-4'-(3-chlorophenyl)-2'-(3-methoxycarbonylphenyl) spiro[3H-indole-3,3'-piperidine]-2,6'(1H)-dione,
racemic (2'R, 3R, 4'S)-1'-[3-(4-acetyl-piperazin-1-yl)-propyl]-6-chloro-4'-(3-chlorophenyl)-2'-(5-fluoro-2-methylphenyl) spiro[3H-indole-3,3'-piperidine]-2,6'(1H)-dione,
racemic (2'R, 3R, 4'S)-6-chloro-4'-(3-chlorophenyl)-2'-(1-ethyl-propenyl) spiro[3H-indole-3,3'-piperidine]-2,6'(1H)-dione,
chiral (2'R, 3R, 4'S)-6-chloro-4'-(3-chlorophenyl)-2'-(1-ethyl-propenyl) spiro[3H-indole-3,3'-piperidine]-2,6'(1H)-dione,
racemic (2'R, 3R, 4'S)-6-chloro-4'-(3-chlorophenyl)-1'-[3-(1,1-dioxo-thiomorpholin-4-yl)-propyl]-2'-(5-fluoro-2-methylphenyl)- spiro[3H-indole-3,3'-piperidine]-2,6'(1H)-dione,
chiral (2'R, 3R, 4'S)-6-chloro-4'-(3-chlorophenyl)-1'-[3-(1,1-dioxo-thiomorpholin-4-yl)-propyl]-2'-(5-fluoro-2-methylphenyl)-spiro[3H-indole-3,3'-piperidine]-2,6'(1H)-dione,
racemic (2'R, 3R, 4'S)-6-chloro-4'-(3-chlorophenyl)-2'-(2, 5-dimethyl-phenyl) spiro[3H-indole-3,3'-piperidine]-2,6'(1H)-dione,
chiral (2'R, 3R, 4'S)-6-chloro-4'-(3-chlorophenyl)-2'-(2, 5-dimethyl-phenyl) spiro[3H-indole-3,3'-piperidine]-2,6'(1H)-dione,
racemic (2'R, 3R, 4'S)-6-chloro-4'-(3-chlorophenyl)-2'-(2,5-dimethyl-2H-pyrazole-3-yl) spiro[3H-indole-3,3'-piperidine]-2,6'(1H)-dione,
racemic (2'R, 3R, 4'S)-6-chloro-4'-(3-chlorophenyl)-2'-(1-methyl-propenyl) spiro[3H-indole-3,3'-piperidine]-2,6'(1H)-dione,
racemic (2'R, 3R, 4'S)-6-chloro-4'-(3-chlorophenyl)-2'-(1-methyl-but-1-enyl) spiro[3H-indole-3,3'-piperidine]-2,6'(1H)-dione,
racemic (2'R, 3R, 4'S)-6-chloro-4'-(3-chlorophenyl)-2'-(1-ethyl-propyl) spiro[3H-indole-3,3'-piperidine]-2,6'(1H)-dione,
chiral (2'R, 3R, 4'S)-6-chloro-4'-(3-chlorophenyl)-2'-(1-ethyl-propyl) spiro[3H-indole-3,3'-piperidine]-2,6'(1H)-dione,
racemic (2'R, 3R, 4'S)-6-chloro-4'-(3-chlorophenyl)-2'-(2,3-difluoro-6-isopropoxyphenyl)-1'-(3-morpholin-4-yl-propyl) spiro[3H-indole-3,3'-piperidine]-2,6'(1H)-dione,
chiral (2'R, 3R, 4'S)-6-chloro-4'-(3-chlorophenyl)-2'-(2,3-difluoro-6-isopropoxyphenyl)-1'-(3-morpholin-4-yl-propyl) spiro[3H-indole-3,3'-piperidine]-2,6'(1H)-dione,
racemic (2'R, 3R, 4'S)-6-chloro-4'-(3-chlorophenyl)-2'-(1-ethylidene-pentyl) spiro[3H-indole-3,3'-piperidine]-2,6'(1H)-dione,
racemic (2'R, 3R, 4'S)-6-chloro-4'-(3-chlorophenyl)-2'-(2,3-difluoro-6-isopropoxyphenyl)-6'-thioxo spiro[3H-indole-3,3'-piperidine]-2(1H)-one,
racemic (2'R, 3R, 4'S)-[6-chloro-4'-(3-chlorophenyl)-2'-(2,3-difluoro-6-isopropoxyphenyl)-2,3-dihydro-2-oxospiro[3H-indole-3,3'-piperidin]-6-ylene] hydrazine carboxylic acid ethyl ester,
racemic (2'R, 3R, 4'S)-6-chloro-4'-(3-chlorophenyl)-2'-(cyclopent-1-enyl) spiro[3H-indole-3,3'-piperidine]-2,6'(1H)-dione,
racemic (2'R, 3R, 4'S)-6-chloro-4'-(3-chlorophenyl)-2'-cyclopentyl spiro[3H-indole-3,3'-piperidine]-2,6'(1H)-dione,
chiral (2'R, 3R, 4'S)-6-chloro-4'-(3-chlorophenyl)-2'-cyclopentyl spiro[3H-indole-3,3'-piperidine]-2,6'(1H)-dione,
racemic (2'R, 3R, 4'S)-6-chloro-4'-(3-chlorophenyl)-2'-(1-isopropyl-propenyl) spiro[3H-indole-3,3'-piperidine]-2,6'(1H)-dione,
chiral (2'R, 3R, 4'S)-6-chloro-4'-(3-chlorophenyl)-2'-(1-isopropyl-propenyl) spiro[3H-indole-3,3'-piperidine]-2,6'(1H)-dione,
racemic (2'R, 3R, 4'S)-6-chloro-4'-(3-chlorophenyl)-2'-(1-methylene-propyl) spiro[3H-indole-3,3'-piperidine]-2,6'(1H)-dione,
chiral (2'R, 3R, 4'S)-6-chloro-4'-(3-chlorophenyl)-2'-(1-methylene-propyl) spiro[3H-indole-3,3'-piperidine]-2,6'(1H)-dione,
racemic (2'R, 3R, 4'S)-6-chloro-4'-(3-chlorophenyl)-2'-(1-ethyl-propenyl)-1'-hydroxycarbonylmethyl-spiro[3H-indole-3,3'-piperidine]-2,6'(1H)-dione,
racemic (2'R, 3R, 4'S)-6-chloro-4'-(3-chlorophenyl)-2'-(1-ethyl-propenyl)-1'-[(2-hydroxy-1,1-dimethylethyl)aminocarbonyl-methyl] spiro[3H-indole-3,3'-piperidine]-2,6'(1H)-dione,
chiral (2'R, 3R, 4'S) -6-chloro-4'-(3-chlorophenyl)-2'-(1-ethyl-propenyl)-1'-[(2-hydroxy-1,1-dimethylethyl)aminocarbonyl-methyl] spiro[3H-indole-3,3'-piperidine]-2,6'(1H)-dione,
racemic (2'R, 3R, 4'S)-6-chloro-4'-(3-chlorophenyl)-2'-(1-ethyl-propenyl)-1'-(3-morpholin-4-yl-propyl) spiro[3H-indole-3,3'-piperidine]-2,6'(1H)-dione,
chiral (2'R, 3R, 4'S)-6-chloro-4'-(3-chlorophenyl)-2'-(1-ethyl-propenyl)-1'-(3-morpholin-4-yl-propyl) spiro[3H-indole-3,3'-piperidine]-2,6'(1H)-dione,
racemic (2'R, 3R, 4'S)-6-chloro-4'-(3-chlorophenyl)-2'-(2-methyl-1-methylene-propyl) spiro[3H-indole-3,3'-piperidine]-2,6'(1H)-dione,
racemic (2'R, 3R, 4'S)-6-chloro-4'-(3-chlorophenyl)-2'-(2,2-dimethyl-1-methylene-propyl) spiro[3H-indole-3,3'-piperidine]-2,6'(1H)-dione,
racemic (2'R, 3R, 4'S)-6-chloro-4'-(3-chlorophenyl)-2'-(1-ethyl-propenyl)-1'-[(2-morpholin-4-yl-ethyl)aminocarbonyl-methyl] spiro[3H-indole-3,3'-piperidine]-2,6'(1H)-dione,
racemic (2'R, 3R, 4'S)-6-chloro-4'-(3-chlorophenyl)-2'-isopropenyl spiro[3H-indole-3,3'-piperidine]-2,6'(1H)-dione,
chiral (2'R, 3R, 4'S)-6-chloro-4'-(3-chlorophenyl)-2'-isopropenyl spiro[3H-indole-3,3'-piperidine]-2,6'(1H)-dione,
racemic (2'R, 3R, 4'S)-6-chloro-4'-(3-chlorophenyl)-2'-(1-methylene-butyl) spiro[3H-indole-3,3'-piperidine]-2,6'(1H)-dione,
chiral (2'R, 3R, 4'S)-6-chloro-4'-(3-chlorophenyl)-2'-(1-methylene-butyl) spiro[3H-indole-3,3'-piperidine]-2,6'(1H)-dione,
racemic (2'R, 3R, 4'S)-6-chloro-4'-(3-chlorophenyl)-2'-(3-methyl-1-methylene-butyl) spiro[3H-indole-3,3'-piperidine]-2,6'(1H)-dione,
chiral (2'R, 3R, 4'S)-6-chloro-4'-(3-chlorophenyl)-2'-(3-methyl-1-methylene-butyl) spiro[3H-indole-3,3'-piperidine]-2,6'(1H)-dione,
racemic (2'R, 3R, 4'S)-6-chloro-4'-(3-chlorophenyl)-2'-(1-methylene-butyl)-6'-thioxo spiro[3H-indole-3,3'-piperidine]-2(1H)-one,
racemic (2'R, 3R, 4'S)-6-chloro-4'-(3-chlorophenyl)-2'-(4-ethoxy-1,2-difluorophenyl) spiro[3H-indole-3,3'-piperidine]-2,6'(1H)-dione,
chiral (2'R, 3R, 4'S)-6-chloro-4'-(3-chlorophenyl)-2'-(4-ethoxy-1,2-difluoro-phenyl) spiro[3H-indole-3,3'-piperidine]-2,6'(1H)-dione,
racemic (2'R, 3R, 4'S)-6-chloro-4'-(3-chloro-5-methoxycarbonyl-phenyl)-2'-(1-methylene-propyl) spiro[3H-indole-3,3'-piperidine]-2,6'(1H)-dione,
racemic (2'R, 3R, 4'S)-6-chloro-4'-(3-chloro-5-hydroxycarbonyl-phenyl)-2'-(1-methylene-propyl) spiro[3H-indole-3,3'-piperidine]-2,6'(1H)-dione,
racemic (2'R, 3R, 4'S)-6-chloro-4'-(3-chloro-5-fluorocarbonyl-phenyl)-2'-(1-methylene-propyl) spiro[3H-indole-3,3'-piperidine]-2,6'(1H)-dione,
racemic (2'R, 3R, 4'S)-6-chloro-4'- [3-chloro-5-(4-methanesulfonyl-piperazine-1-carbonyl)-phenyl]-2'-(1-methylene-propyl) spiro[3H-indole-3,3'-piperidine]-2,6'(1H)-dione,
racemic (2'R, 3R, 4'S)-6-chloro-4'-(3-chlorophenyl)-2'-(1-ethyl-propenyl)-1'-methyl spiro[3H-indole-3,3'-piperidine]- 2,6'(1H)-dione,
chiral (2'R, 3R, 4'S)-6-chloro-4'-(3-chlorophenyl)-2'-(1-ethyl-propenyl)-1'-methyl spiro[3H-indole-3,3'-piperidine]- 2,6'(1H)-dione,
racemic (2'R, 3R, 4'S)-6-chloro-4'-(3-chlorophenyl)-2'-isopropyl spiro[3H-indole-3,3'-piperidine]-2,6'(1H)-dione,
chiral (2'R, 3R, 4'S)-6-chloro-4'-(3-chlorophenyl)-2'-isopropyl spiro[3H-indole-3,3'-piperidine]-2,6'(1H)-dione,
racemic (2'R, 3R, 4'S)-6-chloro-4'-(3-chlorophenyl)-2'-(1-methylene-propyl)-1'-methyl spiro[3H-indole-3,3'-piperidine]-2,6'(1H)-dione,
chiral (2'R, 3R, 4'S)-6-chloro-4'-(3-chlorophenyl)-2'-(1-methylene-propyl)-1'-methyl spiro[3H-indole-3,3'-piperidine]- 2,6'(1H)-dione,
racemic (2'R, 3R, 4'S)-2'-sec-Butyl-6-chloro-4'-(3-chlorophenyl) spiro[3H-indole-3,3'-piperidine]-2,6'(1H)-dione,
racemic (2'R, 3R, 4'S)-6-chloro-4'-(3-chlorophenyl)-2'-(1-hydroxymethyl-vinyl)-spiro[3H-indole-3,3'-piperidine]-2,6'(1H)-dione,
racemic (2'R, 3R, 4'S)-6-chloro-4'-(3-chlorophenyl)-2'-(1-methoxymethyl-vinyl) spiro[3H-indole-3,3'-piperidine]-2,6'(1H)-dione,
racemic (2'R, 3R, 4'S)-6-chloro-4'-(3-chlorophenyl)-2'-[2,3-difluoro-6-(2-methoxy-ethoxy)-phenyl] spiro[3H-indole-3,3'-piperidine]-2,6'(1H)-dione,
chiral (2'R, 3R, 4'S)-6-chloro-4'-(3-chlorophenyl)-2'-[2,3-difluoro-6-(2-methoxy-ethoxy)-phenyl] spiro[3H-indole-3,3'-piperidine]-2,6'(1H)-dione,
racemic (2'R, 3R, 4'S)-6-chloro-4'-(3-chlorophenyl)-2'-(1,2-dimethyl-propenyl) spiro[3H-indole-3,3'-piperidine]-2,6'(1H)-dione,
racemic (2'R, 3R, 4'S)-6-chloro-4'-(3-chlorophenyl)-2'-propionyl spiro[3H-indole-3,3'-piperidine]-2,6'(1H)-dione,
racemic (2'R, 3R, 4'S)-6-chloro-4'-(3-chlorophenyl)-2'-(2,3-difluoro-6-propoxyphenyl) spiro[3H-indole-3,3'-piperidine]-2,6'(1H)-dione,
chiral (2'R, 3R, 4'S)-6-chloro-4'-(3-chlorophenyl)-2'-(2,3-difluoro-6-propoxyphenyl) spiro[3H-indole-3,3'-piperidine]-2,6'(1H)-dione,
racemic (2'R, 3R, 4'S)-6-chloro-4'-(3-chlorophenyl)-2'-(1-cyclopropyl-vinyl) spiro[3H-indole-3,3'-piperidine]-2,6'(1H)-dione,
racemic (2'R, 3R, 4'S)-6-chloro-4'-(3-chlorophenyl)-1'-hydroxycarbonylmethyl-2'-(1-methylene-propyl) spiro[3H-indole-3,3'-piperidine]-2,6'(1H)-dione,
racemic (2'R, 3R, 4'S)-6-chloro-4'-(3-chlorophenyl)-2'-(1-methylene-propyl)-1'-[(1-methanesulfonyl-piperidin-4-yl)aminocarbonyl-methyl] spiro[3H-indole-3,3'-piperidine]-2,6'(1H)-dione,
racemic (2'R, 3R, 4'S)-6-chloro-4'-(3-chlorophenyl)-2'-(1-methylene-propyl)-1'-(aminocarbonyl-methyl) spiro[3H-indole-3,3'-piperidine]-2,6'(1H)-dione,
chiral (2'R, 3R, 4'S)-6-chloro-4'-(3-chlorophenyl)-2'-(1-methylene-propyl)-1'-(aminocarbonyl-methyl) spiro[3H-indole-3,3'-piperidine]-2,6'(1H)-dione,
racemic (2'R, 3R, 4'S)-6-chloro-4'-(3-chlorophenyl)-2'-(1-ethyl-2-methyl-propenyl) spiro[3H-indole-3,3'-piperidine]-2,6'(1H)-dione,
racemic (2'R, 3R, 4'S)-6-chloro-4'-(3-chlorophenyl)-2'-[2,3-difluoro-6-(2-hydroxy-ethoxy)-phenyl] spiro[3H-indole-3,3'-piperidine]-2,6'(1H)-dione,
chiral (2'R, 3R, 4'S)-6-chloro-4'-(3-chlorophenyl)-2'-[2,3-difluoro-6-(2-hydroxy-ethoxy)-phenyl] spiro[3H-indole-3,3'-piperidine]-2,6'(1H)-dione,
racemic (2'R, 3R, 4'S)-6-chloro-4'-(3-chlorophenyl)-2'-(2,3-difluoro-6-hydroxyphenyl) spiro[3H-indole-3,3'-piperidine]-2,6'(1H)-dione,
racemic (2'R, 3R, 4'S)-6-chloro-4'-(3-chlorophenyl)-2'-(1-ethyl-1-hydroxy-propyl) spiro[3H-indote-3,3'-piperidine]-2,6'(1H)-dione,
racemic (2'R, 3R, 4'S)-6-chloro-4'-(3-chlorophenyl)-2'-(1-ethyl-3-methyl-oxiranyl) spiro[3H-indole-3,3'-piperidine]-2,6'(1H)-dione,
racemic (2'R, 3R, 4'S)-6-chloro-4'-(3-chlorophenyl)-2'-(1-fluoro-2-methyl-propenyl) spiro[3H-indole-3,3'-piperidine]-2,6'(1H)-dione,
racemic (2'R, 3R, 4'S)-6-chloro-4'-(3-chlorophenyl)-2'- isobutyryl spiro[3H-indole-3,3'-piperidine]-2,6'(1H)-dione,
racemic (2'R, 3R, 4'S)-6-chloro-4'-(3-chlorophenyl)-2'-isopropenyl -1'-hydroxycarbonylmethyl spiro[3H-indole-3,3'-piperidine]-2,6'(1H)-dione,
racemic (2'R, 3R, 4'S)-1'-(aminocarbonyl-methyl)-6-chloro-4'-(3-chlorophenyl)-2'-isopropenyl spiro[3H-indole-3,3'-piperidine]-2,6'(1H)-dione,
racemic (2'R, 3R, 4'S)-6-chloro-4'-(3-chlorophenyl)-1'-(cyclopropylaminocarbonyl-methyl)-2'-isopropenyl spiro[3H-indole-3,3'-piperidine]-2,6'(1H)-dione,
racemic (2'R, 3R, 4'S)-6-chloro-4'-(3-chlorophenyl)-2'-isopropenyl-1'-[(1-methyl-piperidin-4-yl)aminocarbonyl-methyl] spiro[3H-indole-3,3' piperidine]-2,6'(1H)-dione,
racemic (2'R, 3R, 4'S)-6-chloro-4'-(3-chlorophenyl)-2'-isopropenyl-1'-[(1-methylsulfonyl-piperidin-4-yl)aminocarbonyl-methyl] spiro[3H-indole-3,3' piperidine]-2,6'(1H)-dione,
racemic (2'R, 3R, 4'S)-6-chloro-4'-(3-chlorophenyl)-1'-[(2-hydroxyethyl)aminocarbonyl-methyl]-2'-isopropenyl-spiro[3H-indole-3,3'-piperidine]-2,6'(1H)-dione,
racemic (2'R, 3R, 4'S)-6-chloro-4'-(3-chlorophenyl)-1'-[(2-hydroxy-1,1-dimethylethyl)aminocarbonyl-methyl]-2'-isopropenyl-spiro[3H-indole-3,3'-piperidine]-2,6'(1H)-dione,
racemic (2'R, 3R, 4'R)-6-chloro-4'-(3-chloro-phenyl)-4'-cyano-2'-(1-methylene-propyl) spiro[3H-indole-3,3'-piperidine]-2,6'(1H)-dione,
racemic (2'R, 3R, 4'S)-1'-[3-(4-acetylamino-piperidin-1-yl)-propyl] -6-chloro-4'-(3-chlorophenyl)-2'-isopropenyl spiro[3H-indole-3,3' piperidine]-2,6'(1H)-dione,
racemic (2'R, 3R, 4'S)-4'-(3-chloro-phenyl)-6'-cyano-2'-(1-methylene-propyl) spiro[3H-indole-3,3'-piperidine]-2,6'(1H)-dione,
racemic (2'R, 3R, 4'S)-4'-(3-chloro-phenyl)-6-cyano-2'-(5-fluoro-2-methyl-phenyl) spiro[3H-indole-3,3'-piperidine]-2,6'(1H)-dione,
racemic (2'R, 3R, 4'S)-1'-[3-(4-acetyl-piperazin-1-yl)-propyl]-6-chloro-4'-(3-chlorophenyl)-2'-isopropenyl spiro[3H-indole-3,3'- piperidine]-2,6'(1H)-dione,
racemic (2'R, 3R, 4'S)-6-chloro-4'-(3-chlorophenyl)-2'-isopropenyl- 1'-(3-piperidin-1-yl-propyl) spiro[3H-indole-3,3' piperidine]-2,6'(1H)-dione,
racemic (2'R, 3R, 4'S)-2'-[2-(2-acetoxy-ethoxy)-5-methyl-phenyl]-6-chloro-4'-(3-chlorophenyl) spiro[3H-indole-3,3'-piperidine]-2,6'(1H)-dione,
racemic (2'R, 3R, 4'S)-6-chloro-4'-(3-chlorophenyl)-2'-[6-(2-hydroxy-ethoxy)-3-methyl-phenyl] spiro[3H-indole-3,3'-piperidine]-2,6'(1H)-dione,
racemic (2'R, 3R, 4'S)-6-chloro-4'-(3-chlorophenyl)-2'-[5-cyclopropyl-2-(2-hydroxy-ethoxy)-phenyl] spiro[3H-indole-3,3'-piperidine]-2,6'(1H)-dione,
racemic (2'R, 3R, 4'S)-6-chloro-2'-[5-chloro-2-(2-hydroxy-ethoxy)-phenyl]-4'-(3-chlorophenyl) spiro[3H-indole-3,3'-piperidine]-2,6'(1H)-dione,
racemic (2'R, 3R, 4'S)-6-chloro-2'-[3-chloro-2-fluoro-6-(2-hydroxy-ethoxy)-phenyl]-4'-(3-chlorophenyl) spiro[3H-indole-3,3'-piperidine]-2,6'(1H)-dione,
racemic (2'R, 3R, 4'S)-6-chloro-4'-(3,5-difluoro-phenyl)-2'-(1-methylene-propyl) spiro[3H-indole-3,3'-piperidine]-2,6'(1H)-dione,
racemic (2'R, 3R, 4'S)-6-chloro-4'-(3-cyano-phenyl)-2'-(1-methylene-propyl) spiro[3H-indole-3,3'-piperidine]-2,6'(1H)-dione,
racemic (2'R, 3R, 4'S)-4'-(3-bromo-phenyl)-6-chloro-2'-(1-methylene-propyl) spiro[3H-indole-3,3'-piperidine]-2,6'(1H)-dione,
racemic (2'R, 3R, 4'S)-6-chloro-4'-(3-methoxy-phenyl)-2'-(1-methylene-propyl) spiro[3H-indole-3,3'-piperidine]-2,6'(1H)-dione,
racemic (2'R, 3R, 4'S)-6-chloro-4'-(5-fluoro-2-methyl-phenyl)-2'-(1-methylene-propyl) spiro[3H-indole-3,3'-piperidine]-2,6'(1H)-dione,
racemic (2'R, 3R, 4'S)-6-chloro-4'-(3-fluoro-phenyl)-2'-(1-methylene-propyl) spiro[3H-indole-3,3'-piperidine]-2,6'(1H)-dione,
racemic (2'R, 3R, 4'S)-6-chloro-2'-(1-methylene-propyl)-4'-m-tolyl spiro[3H-indole-3,3'-piperidine]-2,6'(1H)-dione,
racemic (2'R, 3R, 4'S)-6-chloro-2'-(1-methylene-propyl)-4'-o-tolyl spiro[3H-indole-3,3'-piperidine]-2,6'(1H)-dione,
racemic (2'R, 3R, 4'S)-6-chloro-2'-(1-methylene-propyl)-4'-thiophen-3-yl spiro[3H-indole-3,3'-piperidine]-2,6'(1H)-dione,
racemic (2'R, 3R, 4'S)-6-chloro-4'-(3,5-dichloro-phenyl)-2'-(1-methylene-propyl) spiro[3H-indole-3,3'-piperidine]-2,6'(1H)-dione,
racemic (2'R, 3R, 4'S)-6-chloro-4'-(5-chloro-2-trifluoromethyl-phenyl)-2'-(1-methylene-propyl) spiro[3H-indole-3,3'-piperidine]-2,6'(1H)-dione,
racemic (2'R, 3R, 4'S)-4'-(3-Bromo-phenyl)-6-chloro-2'-isopropenyl spiro[3H-indole-3,3'-piperidine]-2,6'(1H)-dione,
racemic (2'S, 3S, 4'R)-6-chloro-2'-(3-chlorophenyl )- 4'-(5-fluoro-2-methylphenyl)- spiro[3H-indole-3,3'-piperidine]-2,6'(1H)-dione,
racemic (2'S, 3S, 4'R)-6-chloro-2'-(3-chlorophenyl)-4'-(5-fluoro-2-methylphenyl)-1'-hydroxycarbonylmethyl-spiro[3H-indole-3,3'-piperidine]-2,6'(1H)-dione,
racemic (2'S, 3S, 4'R)-6-chloro-2'-(3-chlorophenyl )- 4'-(5-fluoro-2-methylphenyl)-1'-(methylamino-carbonyl-methyl) spiro[3H-indole-3,3'-piperidine]-2,6'(1H)-dione,
racemic (2'S, 3S, 4'R)-6-chloro-2'-(3-chlorophenyl )- 1'-(dimethylamino-carbonylmethyl) -4'-(5-fluoro-2-methyl-phenyl)- spiro[3H-indole-3,3'-piperidine]-2,6'(1H)-dione,
racemic (2'S, 3S, 4'R) -1'-[(4-aminocarbonyl-piperidin-1-yl)carbonyl-methyl]-6-chloro-2'-(3-chlorophenyl )- 4'-(5-fluoro-2-methyl-phenyl) spiro[3H-indole-3,3'-piperidine]-2,6'(1H)-dione,
racemic (2'S, 3S, 4'R)-1'-[(3-aminocarbonyl-piperidin-1-yl)carbonyl-methyl] -6-chloro-2'-(3-chlorophenyl )- 4'-(5-fluoro-2-methyl-phenyl)- spiro[3H-indole-3,3'-piperidine]-2,6'(1H)-dione,
racemic (2'S, 3S, 4'R) -1'-(aminocarbonyl-methyl) -6-chloro-2'-(3-chlorophenyl)-4'-(5-fluoro-2-methyl-phenyl) spiro[3H-indole-3,3'-piperidine]-2,6'(1H)-dione,
racemic (2'S, 3S, 4'R)-6-chloro-2'-(3-chlorophenyl)-1'-(dimethylamino-propyl)-4'-(5-fluoro-2-methyl-phenyl)- spiro[3H-indole-3,3'-piperidine]-2,6'(1H)-dione,
racemic (2'S; 3S, 4'R)-6-chloro-4'-(3-chlorophenyl)-2'-(5-fluoro-2-methylphenyl)-1'-[(1-methanesulfonyl-piperidine-4-yl)carbonylamino-ethyl] spiro[3H-indole-3,3'-piperidine]-2,6'(1H)-dione,
racemic (2'R, 3R, 4'S)-6-chloro-4'-[2-(4-chloro-thiophenyl)]-2'-(5-fluoro-2-methylphenyl)-spiro[3H-indole-3,3'-piperidine]-2,6'(1H)=dione,
racemic (2'R, 3R, 4'S)-6-chloro-4'-[2-(5-chloro-thiophenyl)]-2'-(5-fluoro-2-methylphenyl)-spiro[3H-indole-3,3'-piperidine]- 2,6'(1H)-dione,
racemic (2'R, 3R, 4'S)-6-chloro-4'-(2,2-dimethylpropyl)-2'-(5-fluoro-2-methylphenyl)-spiro[3H-indole-3,3'-piperidine]- 2,6'(1H)-dione,
racemic (2'R, 3R, 4'S)-6-bromo-4'-(3-chlorophenyl)-2'-(2-methylphenyl) spiro[3H-indole-3,3'-piperidine]-2,6'(1H)-dione,
racemic (2'R, 3R, 4'S)-6-bromo-4'-(3-chlorophenyl)-2'-(5-fluoro-2-methylphenyl) spiro[3H-indole-3,3'-piperidine]-2,6'(1 )-dione,
chiral (2'R, 3R, 4'S)-6-chloro-4'-(3-chlorophenyl)-2'-(5-fluoro-2-methylphenyl) spiro[3H-indole-3,3'-piperidine]-2,6'(1H)-dione,
racemic (2'R, 3R, 4'S)-6-bromo-4'-(3-chlorophenyl)-2'-(2,5-dichlorophenyl) spiro[3H-indole-3,3'-piperidine]-2,6'(1H)-dione,
racemic (2'R, 3R, 4'S)-6-bromo-4'-(3-chlorophenyl)-2'-(5-chloro-2-methylphenyl) spiro[3H-indole-3,3'-piperidine]-2,6'(1H)-dione,
racemic (2'R, 3R, 4'S)-6-bromo-4'-(3-chlorophenyl )- 2'-(1-methylene-propyl)-spiro[3H-indole-3,3'-piperidine]- 2,6'(1H)-dione,
chiral (2'R, 3R, 4'S)-6-bromo-4'-(3-chlorophenyl )- 2'-(1-methylene-propyl)-spiro[3H-indole-3,3'-piperidine]- 2,6'(1H)-dione,
chiral (2R, 3R, 4'S)-6-bromo-4'-(3-chlorophenyl )-2'-(1-ethyl-cyclopropyl) spiro[3H-indole-3,3'-piperidine]-2,6'(1H)-dione,
racemic (2'R,3R,4'S)-6-cyclopropyl-4'-(3-chlorophenyl)-2'-(1-ethyl-cyclopropyl)-spiro[3H-indole-3,3'-piperidine]- 2,6'(1H)-dione,
racemic (2'R, 3R, 4'S) 2'-[2-(4-aminocarbonyl-piperidin-1-yl)methyl-5-fluorophenyl)-6-chloro-4'-(3-chlorophenyl)-spiro[3H-indole-3,3'-piperidine]- 2,6'(1H)-dione,
racemic (2'R, 3R, 4'S)-6-chloro-4'-(3-chlorophenyl)-2'-[5-fluoro-2-(4-methanesulfonyl-piperazin-1-yl)methyl-phenyl]-spiro[3H-indole-3,3'-piperidine]-2,6'(1H)-dione,
racemic (2'R, 3R, 4'S)-6-chloro-4'-(3-chlorophenyl)-2'-{5-fluoro-2-[(1-methanesulfonyl-piperidin-4-yl)carbonylamino-methyl]-phenyl}-spiro[3H-indole-3,3'-piperidine]- 2,6'(1H)-dione,
chiral (2'R, 3R, 4'S)-4'-(3-chlorophenyl)- 6-fluoro-2'-(5-fluoro-2-methylphenyl) spiro[3H-indole-3,3'-piperidine]-2,6'(1H)-dione,
racemic (2'R, 3R, 4'S)-4'-(3-chlorophenyl)-2'-(5-fluoro-2-methylphenyl)-6-methoxy spiro[3H-indole-3,3'-piperidine]-2,6'(1H)-dione,
racemic (2'R, 3R, 4'S)-4'-(3-chlorophenyl)-2'-(5-fluoro-2-methylphenyl) spiro[3H-indole-3,3'-piperidine]-2,6'(1H)-dione,
racemic (2'R, 3R, 4'S)-4'-(3-chlorophenyl)- 5-fluoro-2'-(5-fluoro-2-methylphenyl) spiro[3H-indole-3,3'-piperidine]-2,6'(1H)-dione,
chiral (2'R, 3R, 4'S)-6-chloro-4'-(3-chloro-phenyl)-2'-(1-methyl-cyclopropyl) spiro[3H-indole-3,3'-piperidine]-2,6'(1H)-dione,
racemic (2'R, 3R, 4'S)-6-bromo-4'-(3-chlorophenyl)-2'-isopropenyl spiro[3H-indole-3,3'-piperidine]-2,6'(1H)-dione,
chiral (2'R, 3R, 4'S)-6-bromo-4'-(3-chloro-phenyl)-2'-(1-methyl-cyclopropyl) spiro[3H-indole-3,3'-piperidine]-2,6'(1H)-dione,
racemic (2'R, 3R, 4'S)-4'-(3-chlorophenyl )-6-ethynyl-2'-isopropenyl spiro[3H-indole-3,3'-piperidine]-2,6'(1H)-dione;
chiral (2'R, 3'R, 4'S)-6-chloro-4'-(3-chlorophenyl)-2'-(5-fluoro-2-methylphenyl)-1'-[(1-methylsulphonyl-4-piperidinyl)aminocarbonyl-methyl] spiro[3H-indole-3,3'-piperidine]-2,6'(1H)-dione,
chiral (2'R,3'R,4'S)-6-chloro-4'-(3-chlorophenyl)-2'-(5-fluoro-2-methylphenyl)-1'-{[4-(1,1-dioxido-2-isothiazolidinyl)ethyl]piperazinyl-carbonyl-methyl} spiro[3H-indole-3,3'-piperidine]-2,6'(1H)-dione,
chiral (2'R,3'R,4'S)-6-chloro-4'-(3-chlorophenyl)-2'-(5-fluoro-2-methylphenyl)-1'-{[3-(methylsulphonyl)propyl]piperazinyl-carbonyl-methyl} spiro[3H-indole-3,3'-piperidine]-2,6'(1H)-dione,
racemic (2'R, 3R, 4'S)-2'-(2-bromo-5-fluorophenyl) 6-chloro-4'-(3-chlorophenyl) spiro[3H-indole-3,3'-piperidine]-2,6'(1H)-dione,
racemic (2'R, 3R, 4'S)-2'-6-chloro-4'-(3-chlorophenyl)- (2-ethynyl-5-fluorophenyl) spiro[3H-indole-3,3'-piperidine]-2,6'(1H)-dione,
racemic (2'R, 3R, 4'S)-2'-6-chloro-4'-(3-chlorophenyl)-15-fluoro-2-[3-(methanesulfony I-methyl-amino)-prop-1-ynyl-phenyl] spiro[3H-indole-3,3'-piperidine]-2,6'(1H)-dione,
racemic (2'R, 3R, 4'S)-2'-[5-bromo-2-(2-hydroxy-ethoxy)-phenyl]-6-chloro-4'-(3-chlorophenyl) spiro[3H-indole-3,3'-piperidine]-2,6'(1H)-dione,
racemic (2'R, 3R, 4'S)-6-chloro-4'-(3-chlorophenyl)-2'-[5-ethynyl-2-(2-hydroxy-ethoxy)-phenyl] spiro[3H-indole-3,3'-piperidine]-2,6'(1H)-dione,
chiral (2'R, 3R, 4'S)-6-chloro-4'-(3-chlorophenyl)-2'-[5-ethynyl-2-(2-hydroxy-ethoxy)-phenyl] spiro[3H-indole-3,3'-piperidine]-2,6'(1H)-dione,
racemic (2'R, 3R, 4'S)-2'-[3-bromo-2-fluoro-6-(2-hydroxy-ethoxy)-phenyl]-6-chloro-4'-(3-chlorophenyl) spiro[3H-indole-3,3'-piperidine]-2,6'(1H)-dione,
racemic (2'R, 3R, 4'S)-6-chloro-4'-(3-chlorophenyl)-2'-[2-fluoro-6-(2-hydroxy-ethoxy)-3-trimethylsilanylethynyl-phenyl] spiro[3H-indole-3,3'-piperidine]-2,6'(1H)-dione,
racemic (2'R, 3R, 4'S)-6-chloro-4'-(3-chlorophenyl)-2'-[3-ethynyl-2-fluoro-6-(2-hydroxy-ethoxy)-phenyl] spiro[3H-indole-3,3'-piperidine]-2,6'(1H)-dione and
racemic (2'R, 3R, 4'S)-6-chloro-4'-(3-chlorophenyl)-2'-[5-ethyl-2-(2-hydroxy-ethoxy)-phenyl] spiro[3H-indole-3,3'-piperidine]-2,6'(1H)-dione.

In the specification where indicated the various groups may be substituted by 1-5 or, preferably, 1-3 substituents independently selected from the group consisting of lower alkyl having from 1 to 8 carbon atoms, lower-alkenyl having from 2 to 8 carbon atoms, lower-alkynyl having from 2 to 6 carbon atoms, dioxo-lower-alkylene having from 1 to 8 carbon atoms (forming e.g. a benzodioxyl group), halogen, hydroxy, -CN, -CF₃, -NH₂, -N(H, lower-alkyl having from 1 to 8 carbon atoms), N(lower-alkyl having from 1 to 8 carbon atoms)₂, aminocarbonyl, carboxy, -NO₂, lower-alkoxy having from 1 to 8 carbon atoms, thio-lower-alkoxy having from 1 to 8 carbon atoms, lower-alkylsulfonyl having from 1 to 8 carbon atoms, aminosulfonyl, lower-alkyl (having from 1 to 8 carbon atoms)carbonyl, lower-alkyl(having from 1 to 8 carbon atoms)carbonyloxy, lower-alkoxy(having from 1 to 8 carbon atoms)carbonyl, lower-alkyl(having from 1 to 8 carbon atoms)carbonyl-NH, fluoro-lower-alkyl having from 1 to 8 carbon atoms, fluoro-lower-alkoxy having from 1 to 8 carbon atoms, lower-alkoxy(having from 1 to 8 carbon atoms)-carbonyl-lower-alkoxy(having from 1 to 8 carbon atoms), carboxy-lower-atkoxy(having from 1 to 8 carbon atoms), carbamoyl-lower-alkoxy(having from 1 to 8 carbon atoms), hydroxy-lower-alkoxy having from 1 to 8 carbon atoms, -NH₂-lower-alkoxy having from 1 to 8 carbon atoms, -N(H, lower-alkyl having from 1 to 8 carbon atoms)-lower-alkoxy having from 1 to 8 carbon atoms, -N(lower-alkyl having from 1 to 8 carbon atoms)₂-lower-alkoxy having from 1 to 8 carbon atoms, benzyloxy-lower-alkoxy having from 1 to 8 carbon atoms, mono- or di-(lower-alkyl having from 1 to 8 carbon atoms) substituted aminosulfonyl and lower alkyl having from 1 to 8 carbon atoms, which can optionally be substituted with halogen, hydroxy, -NH₂, -N(H, lower-alkyl having from 1 to 8 carbon atoms) or -N(lower-alkyl having from 1 to 8 carbon atoms)₂.

Preferred substituents for the aryl, heteroaryl and heterocycle rings are halogen, lower alkoxy having from 1 to 8 carbon atoms, lower alkyl having from 1 to 8 carbon atoms and amino.

If alkyl, alkenyl, alkynyl or similar groups are linked with both ends to the same moiety, cyclic structures may result, where two hydrogens of said moiety are being replaced by the two ends of the alkyl, alkenyl, alkynyl or similar group, thus creating cyclic structures, such as, tetralin, macrocycles or spiro compounds.

The term "alkyl" refers to straight- or branched-chain saturated hydrocarbon groups having from 1 to about 20 carbon atoms. In certain embodiments, alkyl substituents may be lower alkyl substituents. The term "lower alkyl" refers to alkyl groups having from 1 to 8 carbon atoms, and in certain embodiments from 1 to 4 carbon atoms. Preferably the term "lower alkyl" refers to C1-4 alkyl. Examples of alkyl groups include, but are not limited to, methyl, ethyl, n-propyl, i-propyl, n-butyl, s-butyl, t-butyl, n-pentyl, and s-pentyl.

As used herein, "cycloalkyl" is intended to refer to any stable monocyclic or polycyclic system which consists of carbon atoms only, any ring of which being saturated, and the term "cycloalkenyl" is intended to refer to any stable monocyclic or polycyclic system which consists of carbon atoms only, with at least one ring thereof being partially unsaturated. Examples of cycloalkyls include, but are not limited to, cyclopropyl, cyclobutyl, cyclopentyl, cyclohexyl, cycloheptyl, adamantyl, cyclooctyl, bicycloalkyls, including bicyclooctanes such as [2.2.2]bicyclooctane or [3.3.0]bicyclooctane, bicyclononanes such as [4.3.0]bicyclononane, and bicyclodecanes such as [4.4.0]bicyclodecane (decalin), or spiro compounds. Examples of cycloalkenyls include, but are not limited to, cyclopentenyl or cyclohexenyl.

The term "alkenyl" as used herein means an unsaturated straight-chain or branched aliphatic hydrocarbon group containing one double bond and having 2 to 8, preferably 2 to 6 carbon atoms. Examples of such "alkenyl group" are vinyl (ethenyl), allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl and 5-hexenyl.

The term "alkynyl" as used herein means an unsaturated straight-chain or branched aliphatic hydrocarbon group containing one triple bond and having 2 to 6, preferably 2 to 4 carbon atoms. Examples of such "alkynyl group" are ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl.

The term "halogen" as used in the definitions means fluorine, chlorine, iodine or bromine, preferably fluorine and chlorine.

"Aryl" means a monovalent, monocyclic or bicyclic, aromatic carbocyclic hydrocarbon radical, preferably a 6-10 member aromatic ring system. Preferred aryl groups include, but are not limited to, phenyl, naphthyl, tolyl, and xylyl.

"Heteroaryl" means an aromatic heterocyclic ring system containing up to two rings. Preferred heteroaryl groups include, but are not limited to, thienyl, furyl, indolyl, pyrrolyl, pyridinyl, pyrazinyl, oxazolyl, thiaxolyl, quinolinyl, pyrimidinyl, imidazole and tetrazolyl.

In the case of aryl or heteroaryl which are bicyclic it should be understood that one ring may be aryl while the other is heteroaryl and both being substituted or unsubstituted.

"Heterocycle" means a substituted or unsubstituted 5 to 8 membered, mono- or bicyclic, aromatic or non-aromatic hydrocarbon, wherein 1 to 3 carbon atoms are replaced by a hetero atom selected from nitrogen,oxygen or sulfur atom. Examples include pyrrolidin-2-yl; pyrrolidin-3-yl; piperidinyl; morpholin-4-yl and the like.

"Hetero atom" means an atom selected from N, O and S.

"Alkoxy, alkoxyl or lower alkoxy" refers to any of the above lower alkyl groups attached to an oxygen atom. Typical lower alkoxy groups include methoxy, ethoxy, isopropoxy or propoxy, butyloxy and the like. Further included within the meaning of alkoxy are multiple alkoxy side chains, e.g. ethoxy ethoxy, methoxy ethoxy, methoxy ethoxy ethoxy and the like and substituted alkoxy side chains,e.g., dimethylamino ethoxy, diethylamino ethoxy, dimethoxy-phosphoryl methoxy and the like. ,

"Pharmaceutically acceptable," such as pharmaceutically acceptable carrier, excipient, etc., means pharmacologically acceptable and substantially non-toxic to the subject to which the particular compound is administered.

"Pharmaceutically acceptable salt" refers to conventional acid-addition salts or base-addition salts that retain the biological effectiveness and properties of the compounds of the present invention and are formed from suitable non-toxic organic or inorganic acids or organic or inorganic bases. Sample acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, trifluoro acetic acid and the like. Sample base-addition salts include those derived from ammonium, potassium, sodium and, quaternary ammonium hydroxides, such as for example, tetramethyl ammonium hydroxide. Chemical modification of a pharmaceutical compound (i.e. drug) into a saft is a technique well known to pharmaceutical chemists to obtain improved physical and chemical stability, hygroscopicity, flowability and solubility of compounds. *See, e.g.,* Ansel et al., Pharmaceutical Dosage Forms and Drug Delivery Systems (6th Ed. 1995) at pp. 196 and 1456-1457.

The compounds of formulas I or II or III as well as their salts have at least one asymmetric carbon atom and therefore may be present as racemic mixtures or different stereoisomers. The various isomers can be isolated by known separation methods, e.g., chromatography. The invention includes all stereoisomers.

The compounds of the present invention are useful in the treatment or control of cell proliferative disorders, in particular oncological disorders. These compounds and formulations containing said compounds may be useful in the treatment or control of solid tumors, such as, for example, breast, colon, lung and prostate tumors.

A therapeutically effective amount of a compound in accordance with this invention means an amount of compound that is effective to prevent, alleviate or ameliorate symptoms of disease or prolong the survival of the subject being treated. Determination of a therapeutically effective amount is within the skill in the art.

The therapeutically effective amount or dosage of a compound according to this invention can vary within wide limits and may be determined in a manner known in the art. Such dosage will be adjusted to the individual requirements in each particular case including the specific compound(s) being administered, the route of administration, the condition being treated, as well as the patient being treated. In general, in the case of oral or parenteral administration to adult humans weighing approximately 70 Kg, a daily dosage of about 10 mg to about 10,000 mg, preferably from about 200 mg to about 1,000 mg, should be appropriate, although the upper limit may be exceeded when indicated. The daily dosage can be administered as a single dose or in divided doses, or for parenteral administration, it may be given as continuous infusion.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, as well as the particular mode of administration. The amount of active ingredient which can be combined with a carrier material to produce a single dosage form will generally be that amount of a formula I or II or III compound which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, sachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

"Effective amount" means an amount that is effective to prevent, alleviate or ameliorate symptoms of disease or prolong the survival of the subject being treated.

" IC₅₀" refers to the concentration of a particular compound required to inhibit 50% of a specific measured activity. IC₅₀ can be measured, *inter alia,* as is described subsequently.

"Pharmaceutically acceptable ester" refers to a conventionally esterified compound of formulas I or II or III having a carboxyl group or hydroxy group, which esters retain the biological effectiveness and properties of the compounds of formulas I and are cleaved *in vivo* (in the organism) to the corresponding active carboxylic acid or alcohol respectively.

Compounds of this invention in formula I or II or III can be synthesized according to the following general schemes. It will be readily apparent to those of ordinary skill in the art that compounds in formula I-III can be prepared by substitution of the reagents or agents in the general synthesis routes. The starting materials are either commercially available or can be synthesized by well-established literature methods known to those of ordinary skill in the art. The key step of the transformation is a convergent [4+2] cylcoaddition utilizing aza Diels-Alder reaction to generate a racemic mixture of spiroindolinone compounds in formula I in a stereoselective and efficient manner. By using purification of chiral chromatography, compounds in formula II or formula III can be obtained as an optically pure or enriched enantiomers.

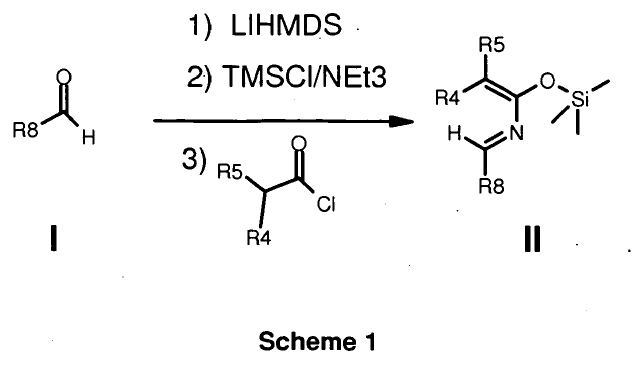

In general an appropriately selected aldehyde **I** can be reacted with lithium hexamethyldisilamide, chlorotrialkylsilane and a selectively substituted acyl chloride in a one-pot, multi-steps manner to generate 2-aza-1,3-butadiene **II (Scheme I)** and can be used as a crude product. Ghosez, L. and others have reported the preparation of 2-aza-1,3-butadienes and their use in aza Diels-Alder reaction to form heterocycle (Ref: Tetrahedron 1995, 11021; J. Am. Chem. Soc. 1999, 2617; and literatures cited therein). The appropriately selected aldehyde **I** are either commercially available or can be synthesized by well-established multiple literature methods.

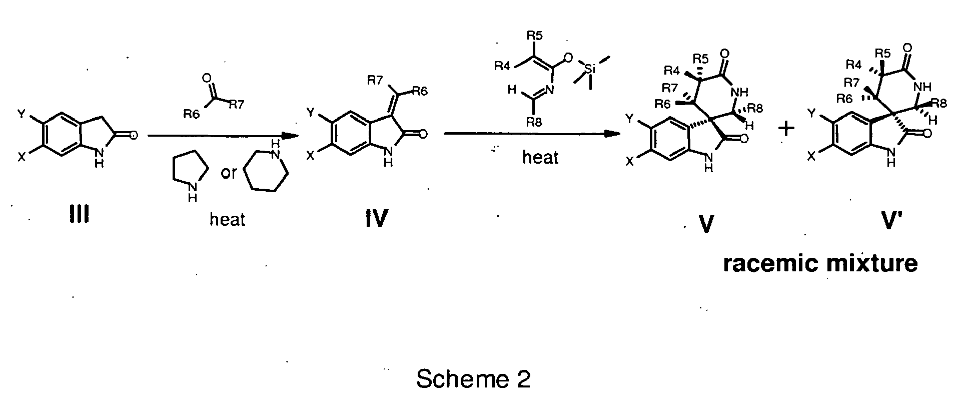

Oxindole **III** can be reacted with an appropriately substituted aldehyde or ketone in the presence of base under heated condition in either a protic like methanol, ethanol or an aprotic solvent like toluene, o-xylene to give intermediate **IV.** The commonly used base is either pyrrolidine or piperidine. Intermediate **IV** can then be reacted with 2-aza-1,3-butadiene **II** in toluene or o-xylene under heating from about 110 °C to 160 °C and anhydrous condition to afford a racemic mixture of spiroindolinone **V** and **V**' as the major products shown together with other minor stereoisomers. 6-substituted or 5,6-disubstituted oxindole **III** starting materials are either commercially available or prepared according to literature methods, for examples, Kraynack, E. A.; Dalgard, J. E.; Gaeta, F. C. A. Tetrahedron Letters, 1998, 39, 7679 - 7682, EP153818 for 5-fluro-6-chlorooxindole, etc

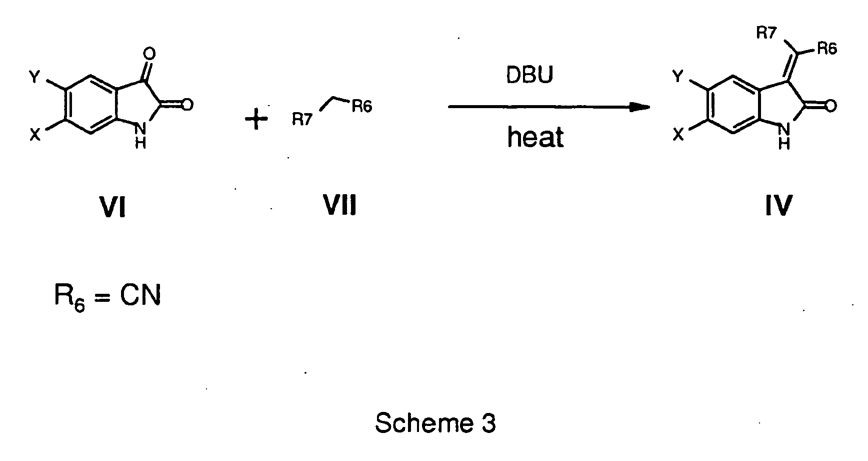

When R₆ is a strong electron withdrawing group in reagent **VII,** intermediate **IV** can be prepared alternatively from Isatin **VI** and reagent **VII.** For example, when R₆ is cyano and R₇ is a substituted aryl, heteroaryl which heteroaryl is an aromatic heterocyclic ring system containing up to two rings, Isatin **VI** can react with various R₇ substituted cyanide **VII** in the presence ofa base like DBU in methanol under heated condition to form **IV (Scheme 3).** 6-substituted or 5,6-disubstituted isatin **VI** starting materials are either commercially available or prepared according to literature methods

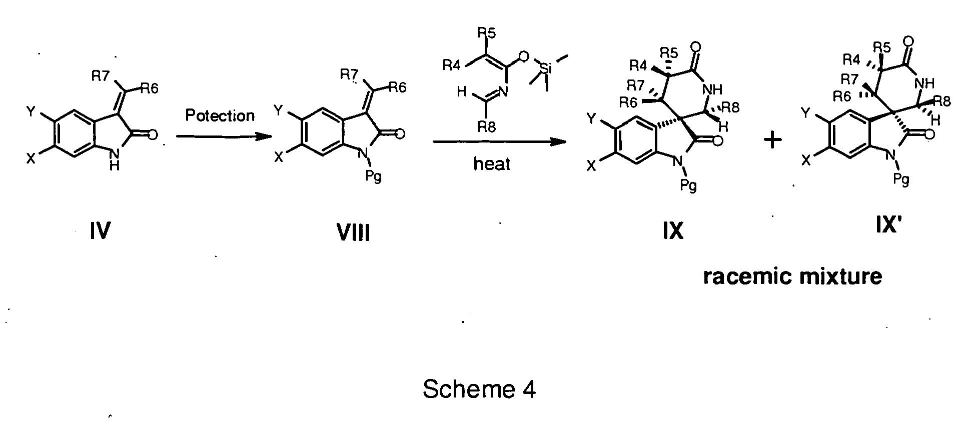

Intermediate **IV** can be protected to give intermediate **VIII.** The protective group can be attached by using ethyl chloroformate, di-tert-butyl dicarbonate, SEM-Cl, benzyl bromide, and a base like 4-(dimethylamine)pyridine (DMAP), triethylamine, NaH, or LiH according to well established literature procedures. Examples of protective group formation and their deprotection have been described and reviewed comprehensively by Greene, T.W. et al in "Protective Groups in Organic Synthesis, 2nd Edition. John Wiley & Sons Inc. In a similar manner intermediate **VIII** can be reacted with a selected 2-aza-butadiene **II** prepared in **Scheme 1** in toluene or o-xylene under heating from 110 °C to 160 °C and anhydrous condition to form intermediate **IX** and **IX'** as the major products shown as a racemic mixture of two enantiomers together with other minor stereoisomers **(Scheme 4).** Intermediate **IX** can be converted into **V** by a deprotection reaction **(Scheme 5).** A useful Pg can be ethyl carbamate, tert-butyl carbamate (BOC), or trimethylsilylethoxymethyl (SEM). Ethyl carbamate can be removed easily by treatment of **IX** with a base like NaOH in methanol or ethanol at room temperature. tert-butyl carbamate (BOC) can be readily removed by treatment of IX with trifluoroacetic acid at room temperature. Deprotection of trimethylsilylethoxymethyl (SEM) can be achieved by treatment with trifluoroacetic acid in dichloromethane at room temperature first, followed by heating with diisopropylethylamine in methanol.

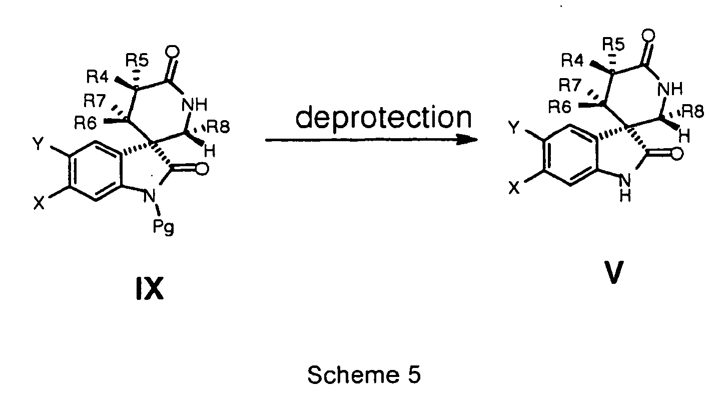

When R₈ is selected from a certain group such as lower alkyl having from 1 to 8 carbon atoms, substituted lower alkyl having from 1 to 8 carbon atoms, cycloalkyl, substituted cycloalkyl, alternative synthetic methods can be used to gain access to compounds **V** or intermediate **IX.** Typically, the compounds of **V** or intermediate **IX** with R₈ selected from a related lower alkenyl having from 2 to 8 carbon atoms, or substituted alkenyl having from 2 to 8 carbon atoms, or cycloalkenyl, or substituted cycloalkenyl would be prepared first according to the methods in **scheme 2** or **scheme 4,** followed by a catalytic hydrogenation reaction to give those **V** or **IX** with a R₈ as the corresponding lower alkyl having from 1 to 8 carbon atoms, or substituted lower alkyl having from 1 to 8 carbon atoms, or cycloalkyl, or substituted cycloalkyl. Treatment of the compounds of **V** or intermediate **IX** with R₈ selected from a related lower alkenyl having from 2 to 8 carbon atoms, or substituted alkenyl having from 2 to 8 carbon atoms with Simmons-Smith reagent (CH₂l₂-Et₂Zn) will lead to those **V** or **IX** with a R₈ as the corresponding substituted cyclopropyl group.

**V** can be selectively protected to give **IX** under controlled conditions. In this case, a useful protective group Pg here can be ethyl carbamate, or tert-butyl carbamate (BOC) **(Scheme 6).** The protective group can be attached by using ethyl chloroformate, or di-tert-butyl dicarbonate, and a base like 4-(dimethylamine)pyridine (DMAP) in dichloromethane at room or lowered temperature similar to the transformation from **IV** to **VIII** in **Scheme 4.**

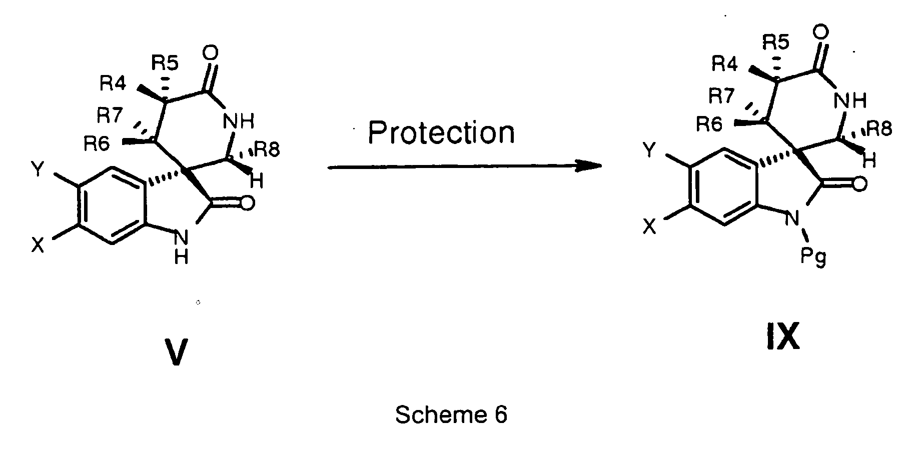

By using either an organic base diisopropylethyl amine or an inorganic base like Cs₂CO₃, LiH or NaH, and an appropriately selected alkylation reagent , N-alkylate intermediate **X** can be prepared from **IX.** A subsequent reaction to remove protective group (Pg) leads to various R₂ derivatized compound **XI (Scheme 7).**

When Pg is trimethylsiloxyethoxymethyl (SEM) group and R₂ is -(CH₂)ₙCl, R₂ can be functionalized with other substituting group. For example, intermediate **X** with -(CH₂)ₙCl can be reacted with HNR₁₀R₁₁ in neat or a solvent like isopropanol under heated condition, followed by treatment of trifluoroacetic acid and diisopropylamine to give compounds **XI** with R₂ as -(CH₂)ₙ NR₁₀R₁₁

When R₂ group is -(CH₂)ₘC(=O)OR', in which R' is hydrogen or a lower alkyl having from 1 to 8 carbon atoms group, compound **XI** can be converted to give compounds **XI** with R₂ as (CH₂)ₘC(=O)NR₁₀R₁₁ by using well-known methods for carboxamide formation.

R₁₀ or R₁₁ is independently selected from the group consisting of hydrogen, lower alkyl having from 1 to 8 carbon atoms, substituted lower alkyl having from 1 to 8 carbon atoms, lower alkoxy having from 1 to 8 carbon atoms, substituted lower alkoxy having from 1 to 8 carbon atoms, cycloalkyl, substituted cycloalkyl, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, or R₁₀ and R₁₁ may be linked to form a heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, heteroaryl which heteroaryl is an aromatic heterocyclic ring system containing up to two rings, or substituted heteroaryl which heteroaryl is an aromatic heterocyclic ring system containing up to two rings.
n=2,3,4
m=1,2,3

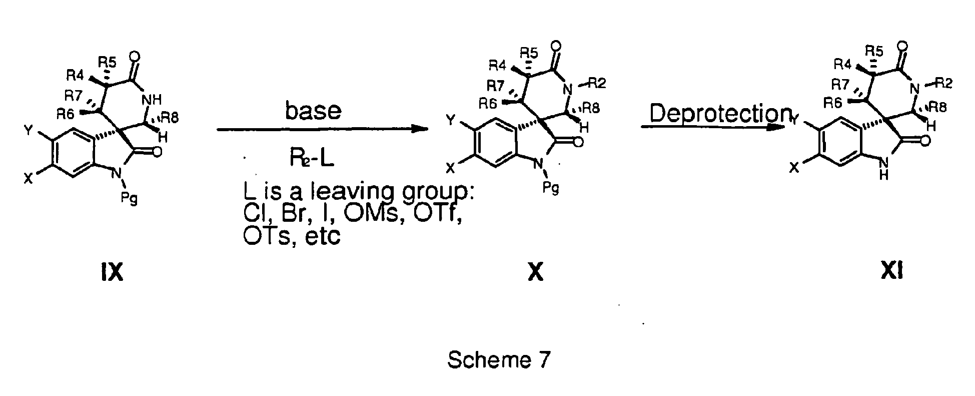

In one step compound **XI** can be selectively converted into thioamide analogue **XII** bv using Lawesson reagent or other similarly related reagents **(Scheme 8)**

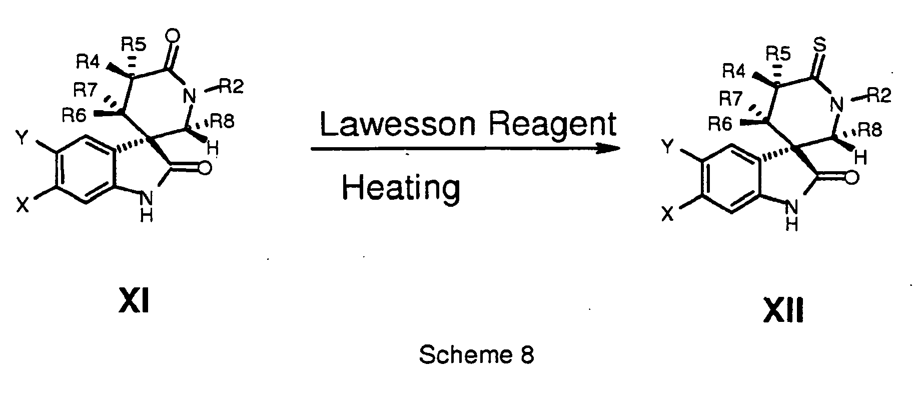

When R₂ is hydrogen, thioamide compound **XIII** can also be a useful intermediate to prepare various R₃ derivatized analogues. For example, compound **XIII** can be reacted with active nucleophilic, appropriately substituted hydrazide R₉-O(O=C)NHNH2, and a mercuric reagent like HgCl₂ or Hg(OAc)₂ to form analogues **XIV (Scheme 9).** R₉ is lower alkyl having from 1 to 8 carbon atoms or substituted lower alkyl having from 1 to 8 carbon atoms

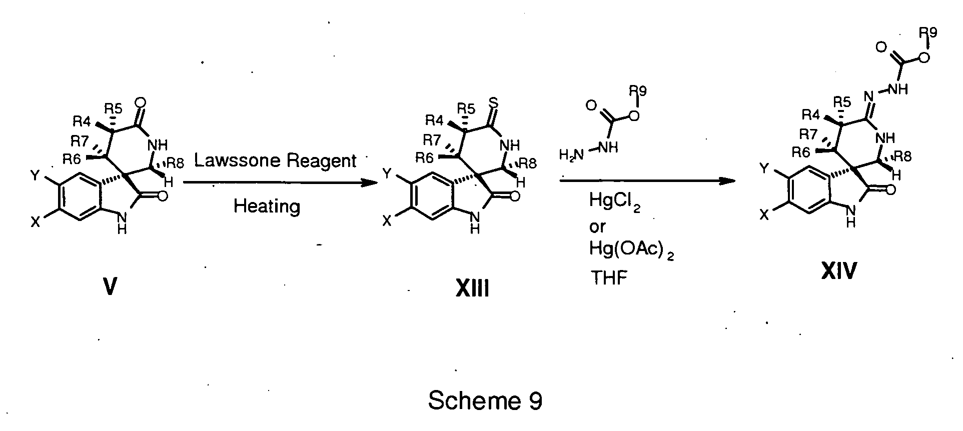

Compound **V** and **V'** can be readily resolved into two optically pure or enriched chiral enantiomers by separation using chiral Super Fluid Chromatography (SFC) or chiral HPLC or chiral column chromatography **(Scheme 10).** In a similar manner to the methods in the reaction schemes above, the chiral enantiomer of intermediate **X** and compounds **XI, XII, XIII, XIV** can be prepared by substitution of **V** with its enantiomer **V',** or substitution of **IX** with **IX'.** The compounds **V** and **V',** intermediates **IX** and **IX'** were generated initially as a racemic mixture and subsequently reacted without chiral separation to give the corresponding racemic mixture of **X, XI, XII, XIII,** or **XIV** together with their enantiomers . All these racemic mixture of **IX, X, XI, XII, XIII, XIV** and their enantiomers in the reaction schemes above can also be readily separated into optically pure or enriched chiral enatiomeric pairs in a similar manner as **scheme 10.**

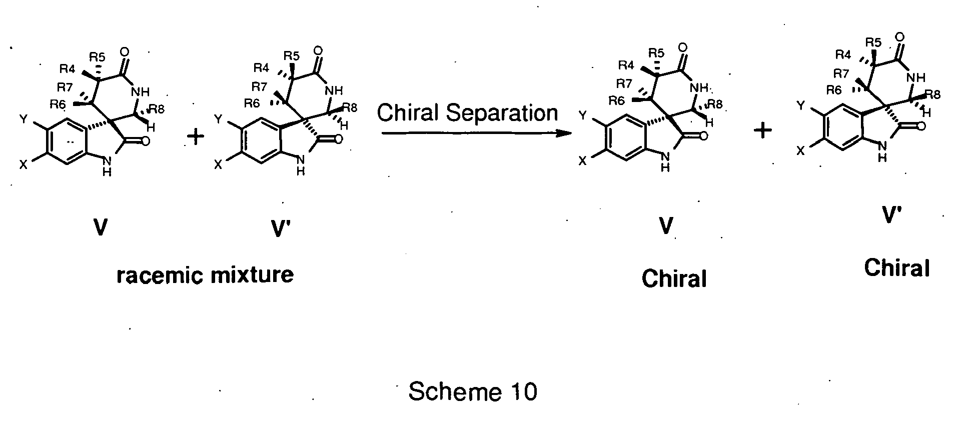

The following examples and references are provided to aid the understanding of the present invention, the true scope of which is set forth in the appended claims.

### Examples

### Example 1a

### Preparation of intermediate E/Z-6-chloro-3-(3,3-dimethyl-butylidene)-1,3-dihydro-indol-2-one

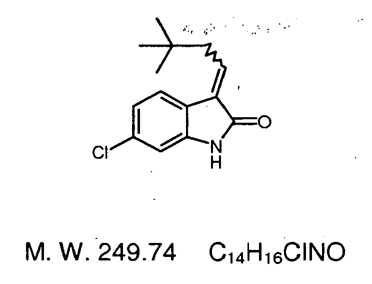

To the mixture of 6-chlorooxindole (0.26 g; 1.49 mmol) (Crescent) and 3,3-dimethyl-butyraldehyde (0.21 g, 2.09 mmol) (Aldrich) in methanol (20 mL) was added pyrrolidine (0.15 g, 2.09 mmol) (Aldrich) dropwise. The mixture was then heated at 100 °C for 1 h. The mixture was concentrated, and the residue was partitioned between ethyl acetate and water. The organic layer was separated, dried over Na₂SO₄, concentrated, and dried *in vacuo* to give the crude E/Z-6-chloro-3-(3,3-dimethyl-butylidene)-1,3-dihydro-indol-2-one as a white solid (Yield 0.37 g, 100%).

### Example 1b

### Preparation of intermediate 1-(3-chlorophenyl)-3-trimethylsilyoxy-2-aza-1,3-butadiene

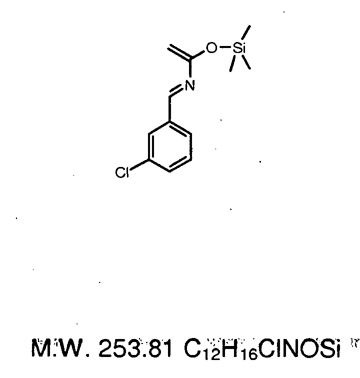

To 1,1,3,3,3-hexamethyldisilazane (2.18 mL, 10.5 mmol) (Aldrich) under nitrogen at room temperature was added *n*-butyllithium (2.5 M, 4.2 mL, 10.5 mmol) (Aldrich). The reaction mixture was stirred at room temperature for 10 minutes. Then dry tetrahydrofuran (30 mL) was added, followed by the addition of 3-chloro-benzaldehyde (1.19 mL, 10.5 mmol) (Aldrich). After the mixture was stirred at room temperature for 0.5 h, trimethylsilyl chloride (1.33 mL, 10.5 mmol) (Aldrich) was added dropwise. Then the temperature of the mixture was lowered to 0 °C on a cooling ice bath. To this mixture was added triethylamine (1.9 mL, 13.6 mmol) in one portion, followed by the dropwise addition of a solution of acetyl chloride (0.97 mL, 13.6 mmol) in diethyl ether (50 mL). The cooling bath was removed, and the mixture was stirred at room temperature for 1 h. The mixture was quickly filtered on celite under nitrogen, and filtrate was concentrated under reduced pressure to give crude 1-(3-chlorophenyl)-3-trimethylsilyoxy-2-aza-1,3-butadiene as a yellow gum and used for the next step without further purification.

Similar transformation has been reported by Ghosez, L., Bayard, Ph., Nshimyumukiza, P., Gouverneur, V., Sainte, F., Beaudegnies, R., Rivers, M., Frique-Hesbain, A.-M. and Wynants, C. in Tetrahedron 1995, 11021-11042.

### Example 1c

### Preparation of racemic (2'S, 3S, 4'S)-6-chloro-2'-(3-chlorophenyl)-4'-(2,2-dimethylpropyl) spiro[3H-indole-3,3'-piperidine]-2,6'(1H)-dione

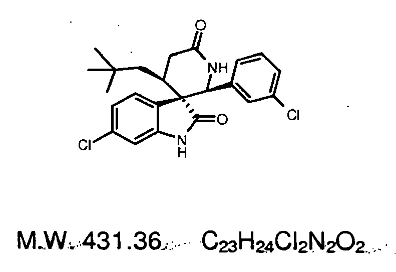

To a mixture of 1-(3-chlorophenyl)-3-trimethylsilyoxy-2-aza-1,3-butadiene (0.25 g, 1 mmol) prepared in example 1b, and toluene (4 mL) was added E/Z-6-chloro-3-(3,3-dimethyl-butylidene)-1,3-dihydro-indol-2-one (0.25 g, 1 mmol) prepared in example 1a. The reaction mixture was heated in a sealed tube under nitrogen at 110 °C for 18 h. The mixture was cooled to room temperature, and methanol (10 mL) was added. The mixture was concentrated and the residue was purified by chromatography (EtOAc/hexanes=2:1) to give racemic (2'S, 3S, 4'S)-6-chloro-2'-(3-chlorophenyl)-4'-(2,2-dimethylpropyl) spiro[3H-indole-3,3'-piperidine]-2,6'(1H)-dione as a brown solid (Yield 0.15 g, 35%).

HRMS(ES⁺) *m*/*z* Calcd for C₂₃H₂₄Cl₂N₂O₂+ H [(M+H)⁺]: 431.1288. Found: 431.1285

Similar transformation has been reported by Ghosez, L. and Jnoff, E. in J. Am. Chem. Soc 1999, 2617-2618.

### Example 2a

### Preparation of intermediate E-6-chloro-3-(2,2-dimethyl-propylidene)-1,3-dihydro-indol-2-one

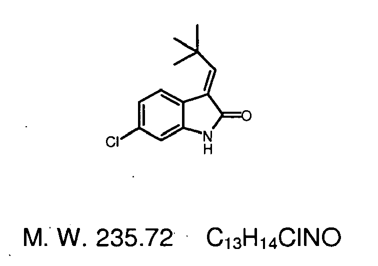

In a manner similar to the method described in example 1a 6-chlorooxindole (0.88 g, 5 mmol) was reacted with 2,2-dimethyl-propionaldehyde (0.43 g, 5 mmol) (Aldrich), pyrrolidine (0.36 g, 5 mmol) in methanol to give a mixture of E-and Z-6-Chloro-3-(2,2-dimethyl-propylidene)-1,3-dihydro-indol-2-one. Purification by chromatography (EtOAc:hexanes=1:2) led to E-6-chloro-3-(2,2-dimethyl-propylidene)-1,3-dihydro-indol-2-one as an off-white foam (Yield 0.82 g, 70%).

### Example 2b

### Preparation of racemic (2'SR, 3S, 4'R)-4'-(tert-butyl)-6-chloro-2'-(3-chlorophenyl) spiro[3H-indole-3,3'-piperidine]-2,6'(1H)-dione

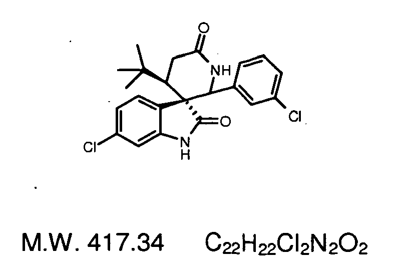

In a manner similar to the method described in example 1c, E-6-chloro-3-(2,2-dimethyl-propylidene)-1,3-dihydro-indol-2-one (0.23 g, 1 mmol) was reacted with 1-(3-chlorophenyl)-3-trimethylsilyoxy-2-aza-1,3-butadiene (0.63 g, 2.5 mmol) prepared in example 1 b, in toluene to give racemic (2'SR, 3S, 4'R)-4'-(*tert*-butyl)-6-chloro-2'-(3-chlorophenyl) spiro[3H-indole-3,3'-piperidine]-2,6'(1H)-dione as a mixture of two sets of diastereomers (Yield 0.21 g, 50%).

HRMS(ES⁺) *m*/*z* Calcd for C₂₂H₂₂Cl₂N₂O₂ + H [(M+H)⁺]: 417.1131. Found: 417.1129

### Example 3a

### Preparation of intermediate E/Z-6-chloro-3-isobutylidene-1,3-dihydro-indol-2-one

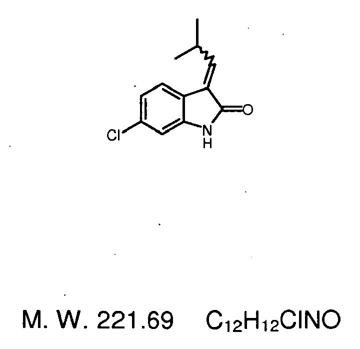

In a manner similar to the method described in example 1a, 6-chlorooxindole (0.85 g, 4.8 mmol) was reacted with 2-methyl-propionaldehyde (0.42 g, 5.8 mmol) (Aldrich), pyrrolidine (0.41 g, 5.8 mmol) in methanol (40 mL) to give a mixture of E/Z-6-chloro-3-isobutylidene -1,3-dihydro-indol-2-one as a brown foam (Yield 1.0 g, 100%).

### Example 3b

### Preparation of racemic (2'S, 3S, 4'R)-6-chloro-2'-(3-chlorophenyl)-4'-isopropyl spiro[3H-indole-3,3'-piperidine]-2,6'(1H)-dione

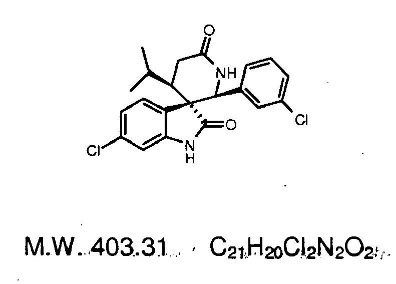

In a manner similar to the method described in example 1c, E/Z-6-chloro-3-isobutylidene-1,3-dihydro-indol-2-one (0.25 g, 1.1 mmol) was reacted with 1-(3-chlorophenyl)-3-trimethylsilyoxy-2-aza-1,3-butadiene (1.2 g, 4.7 mmol) prepared in example 1 b, in toluene to give racemic (2'S, 3S, 4'R)-6-chloro-2'-(3-chlorophenyl)-4'-isopropyl spiro[3H-indole-3,3'-piperidine]-2,6'(1H)-dione as a white solid (Yield 0.25 g, 56%).

HRMS(ES⁺) *m*/*z* Calcd for C₂₁H₂₀Cl₂N₂O₂+ H [(M+H)⁺]: 403.0975. Found: 403.0975.

### Example 4a

Preparation of intermediate E/Z-6-chloro-3-(3-chloro-benzylidene)-1,3-dihydro-indol-2-one

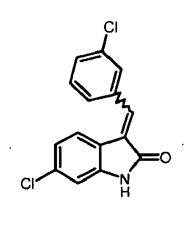

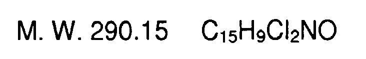

To the mixture of 6-chlorooxindole (16.2 g, 92 mmol) (Crescent) and 3-chloro-benzaldehyde (12.9 g, 92 mmol) (Aldrich) in methanol (109 mL) was added pyrrolidine (6.55 g, 92 mmol) (Aldrich) dropwise. The mixture was then heated at 70 °C for 3 h. After cooled to 4 °C, the mixture was filtered and resulting precipitate was collected, dried to give a mixture of E/Z-6-chloro-3-(3-chlorobenzylidene)-1,3-dihydro-indol-2-one as a bright yellow solid (Yield 25.2 g, 95 %).

### Example 4b

### Preparation of intermediate E/Z-6-chloro-3-(3-chloro-benzylidene)-2-oxo-2,3-dihydro-indole-1-carboxylic acid ethyl ester

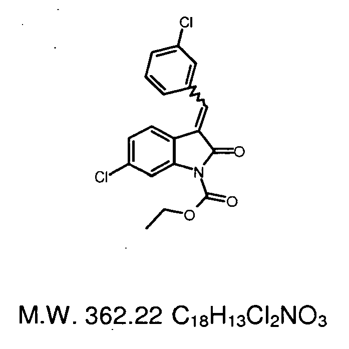

To a solution of E/Z-6-chloro-3-(3-chloro-benzylidene)-1,3-dihydro-indol-2-one prepared in example 4a (1.33 g, 4:6 mmol) in dichloromethane (50 mL) at 0 °C was added ethyl chloroformate (0.66 mL, 6.9 mmol) (Aldrich), followed by the addition of triethylamine (0.93 g, 9.2 mmol). The reaction mixture was stirred at 0 °C for 30 minutes. The mixture was then poured into aqueous HCl solution (1 N). The organic layer was separated and the aqueous layer was extracted with ethyl acetate. The organic layers were combined and dried over Na₂SO₄, and concentrated to give E/Z-6-chloro-3-(3-chloro-benzylidene)-2-oxo-2,3-dihydro-indole-1-carboxylic acid ethyl ester as a yellow solid and used for the next step without further purification (Yield 1.7 g, 100%).

### Example 4c

### Preparation of intermediate racemic (2'R, 3R, 4'S)-6-chloro-2',4'-bis(3-chlorophenyl)-2,3-dihydro-2,6'-dioxo spiro[indole-3,3'-piperidine]-1-carboxylic acid ethyl ester

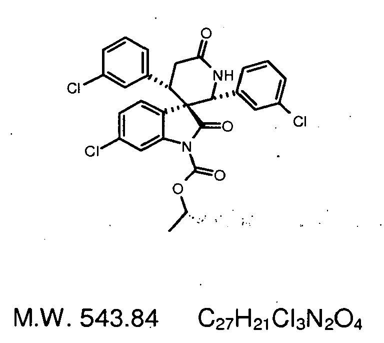

To a solution of 1-(3-chlorophenyl)-3-trimethylsilyoxy-2-aza-1,3-butadiene prepared in example 1b in toluene (20 mL) was added E/Z-6-chloro-3-(3-chlorobenzylidene)-2-oxo-2,3-dihydro-indole-1-carboxylic acid ethyl ester prepared in example 6b (0.3 g, 0.83 mmol). The reaction mixture was stirred under nitrogen in a sealed tube at 135 °C for 1 h. After the solution was cooled to room temperature, methanol (50 mL) was added, and then the mixture was concentrated. The residue was purified by chromatography (EtOAc:CH₂Cl₂ =1:3) to give racemic (2'R, 3R, 4'S)-6-chloro-2',4'-bis(3-chlorophenyl)-2,3-dihydro-2,6'-dioxo spiro[indole-3,3'-piperidine]-1-carboxylic acid ethyl ester as a yellow oil (Yield 0.45 g, 100%).

### Example 4d

### Preparation of racemic (2'R, 3R, 4'S)-6-chloro-2',4'-bis(3-chlorophenyl) spiro[3H-indole-3,3'-piperidine]-2,6'(1H)-dione

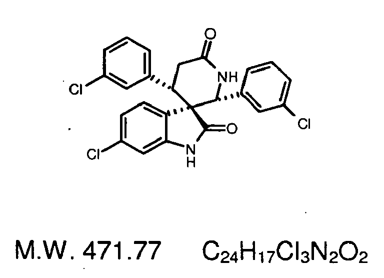

To a solution of racemic (2'R, 3R, 4'S)-6-chloro-2',4'-bis(3-chlorophenyl)-2,3-dihydro-2,6'-dioxo spiro[indole-3,3'-piperidine]-1-carboxylic acid ethyl ester prepared in example 4c (0.45g, 0.92 mmol) in methanol (30 mL) was added NaOH (66 mg, 1.65 mmol). The mixture was stirred at room temperature for 0.5 h. The solvent was removed and the residue was partitioned between ethyl acetate and aqueous HCl solution (1 N). The aqueous layer was extracted with ethyl acetate. The organic layers were combined and then concentrated. The residue was purified with chromatography (EtOAc:CH₂Cl₂= 1:3) to give racemic (2'R, 3R, 4'S)-6-chloro-2',4'-bis(3-chlorophenyl) spiro[3H-indole-3,3'-piperidine]-2,6'-dione as a white solid (Yield 0.2 g, 51 %).

HRMS(ES⁺) *m*/*z* Calcd for C₂₄H₁₇Cl₃N₂O₂+ H [(M+H)⁺]: 471.0429. Found: 471.0431.0.

### Example 4e

### Preparation of chiral (2'R, 3R, 4'S)-6-chloro-2',4'-bis(3-chlorophenyl) spiro[3H-indole-3,3'-piperidine]-2,6'(1H)-dione

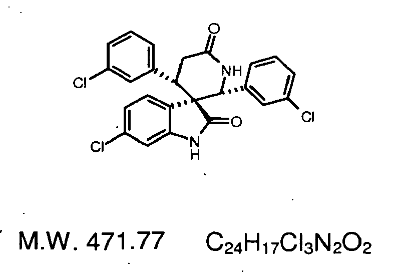

Separation of the two enantiomers from racemic (2'R, 3R, 4'S)-6-chloro-2',4'-bis(3-chlorophenyl) spiro[3H-indole-3,3'-piperidine]-2,6'(1H)-dione (84 mg) prepared in example 4d was conducted by chiral SFC to provide chiral (2'R, 3R, 4'S)-6-chloro-2',4'-bis(3-chlorophenyl) spiro[3H-indole-3,3'-piperidine]-2,6'(1H)-dione as a white solid (Yield 10 mg, 12%). HRMS(ES⁺) *mlz* Calcd for C₂₄H₁₇Cl₃N₂O₂ + H [(M+H)⁺]: 471.0429. Found: 471.0431 and chiral (2'S, 3S, 4'R)-6-chloro-2',4'-bis(3-chlorophenyl) spiro[3H-indole-3,3'-piperidine]-2,6'(1H)-dione as a white solid (Yield 17 mg, 20%). HRMS(ES⁺) *mlz* Calcd for C₂₄H₁₇Cl₃N₂O₂ + H [(M+H)⁺]: 471.0429. Found: 471.0428.

### Example 5a

### Preparation of intermediate E/Z-6-chloro-3-cyclopentylmethylene-1,3-dihydro-indol-2-one

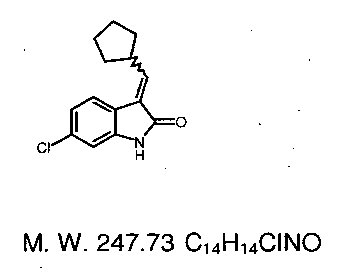

In a manner similar to the method described in example 1 a, 6-chlorooxindole (1.16 g, 6.59 mmol) was reacted with cyclopentanecarbaldehyde (0.77 g, 7.85 mmol) (Wiley) and piperidine (0.67 g, 7.85 mmol) in methanol to give a mixture of E-and Z-6-Chloro-3-cyclopentylmethylene-1,3-dihydro-indol-2-one as a brown oil (Yield 0.8 g, 49 %).

### Example 5b

### Preparation of intermediate E/Z-6-chloro-3-cyclopentylmethylene-2-oxo-2,3-dihydro-indole-1-carboxylic acid ethyl ester

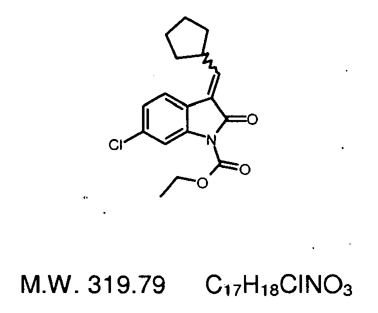

In a manner similar to the method described in example 4b, E/Z-6-chloro-3-cyclopentylmethylene-1,3-dihydro-indol-2-one (0.8 g, 3.2 mmol) was reacted with ethyl chloroformate (0.46 mL, 4.9 mmol) and triethylamine (0.9 mL, 6.4 mmol) in dichloromethane to give E/Z-6-chloro-3-cyclopentylmethylene-2-oxo-2,3-dihydro-indole-1-carboxylic acid ethyl ester as brown oil (Yield 0.6 g, 58%).

### Example 5c

### Preparation of intermediate racemic (2'S, 3S, 4'R)-6-chloro-2'-(3-chlorophenyl)-4'-cyclopentyl-2,3-dihydro-2,6'-dioxo spiro[indole-3,3'-piperidine]-1-carboxylic acid ethyl ester

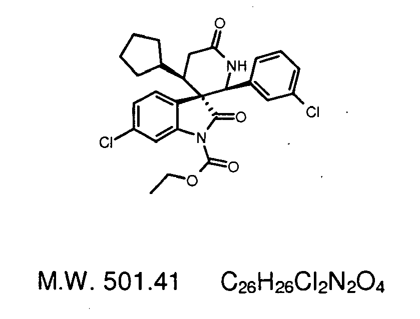

In a manner similar to the method described in example 4c, E/Z-6-chloro-3-cyclopentylmethylene-2-oxo-2,3-dihydro-indole-1-carboxylic acid ethyl ester (0.32 g, 1.00 mmol) was reacted with 1-(3-chlorophenyl)-3-trimethylsilyoxy-2-aza-1,3-butadiene prepared in example 1 b, in toluene to give racemic (2'S, 3S, 4'R)-6-chloro-2'-(3-chlorophenyl)-4'-cyclopentyl-2,3-dihydro-2,6'-dioxo spiro[indole-3,3'-piperidine]-1-carboxylic acid ethyl ester as an off-white foam (Yield 0.26 g, 52%).

### Example 5d

### Preparation of racemic (2'S, 3S, 4'R)-6-chloro-2'-(3-chlorophenyl)-4'-cyclopentyl spiro[3H-indole-3,3'-piperidine]-2,6'(1H)-dione

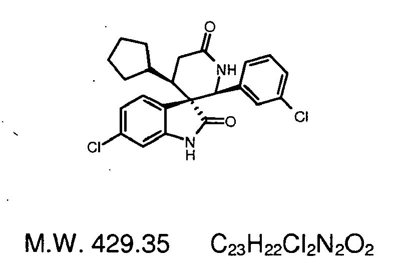

In a manner similar to the method described in example 4d, racemic (2'S, 3S, 4'R)-6-chloro-2'-(3-chlorophenyl)-4'-cyclopentyl-2,3-dihydro-2,6'-dioxo spiro[indole-3,3'-piperidine]-1-carboxylic acid ethyl ester (0.26 g, 0.52 mmol) was reacted with NaOH (37 mg, 0.93 mmol) in methanol to give racemic (2'S, 3S, 4'R)-6-chloro-2'-(3-chlorophenyl)-4'-cyclopentyl spiro[3H-indole-3,3'-piperidine]-2,6'(1H)-dione as a white solid (Yield 0.13 g, 59%).

HRMS(ES⁺) *m*/*z* Calcd for C₂₃H₂₂Cl₂N₂O₂+ H [(M+H)⁺]: 429.1131. Found: 429.1121.

### Example 6a

### Preparation of intermediate E/Z-6-chloro-3-cyclohexylmethylene-1,3-dihydro-indol-2-one

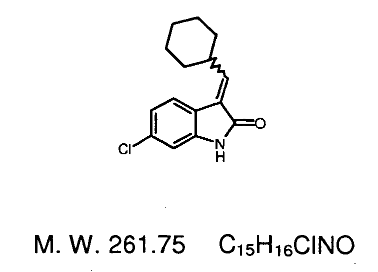

In a manner similar to the method described in example 1a, 6-chlorooxindole (2 g, 11.4 mmol) was reacted with cyclohexanecarbaldehyde (1.53 g, 13.6 mmol) (Aldrich) and piperidine (1.35 mL, 13.6 mmol) in methanol to give a mixture of E-and Z-6-chloro-3-cyclohexylmethylene-1,3-dihydro-indol-2-one as a brown solid (Yield 2.71 g, 91 %).

### Example 6b

### Preparation of intermediate E/Z -6-chloro-3-cyclohexylmethylene-2-oxo-2,3-dihydro-indole-1-carboxylic acid ethyl ester

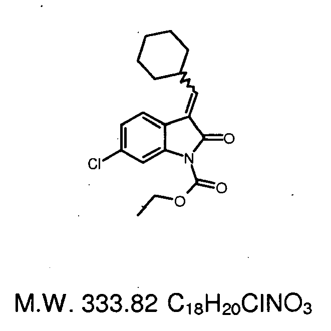

In a manner similar to the method described in example 4b, E/Z -6-chloro-3-cyclohexylmethylene-1,3-dihydro-indol-2-one (2.71 g, 10.4 mmol) was reacted with ethyl chloroformate (1.47 mL, 15.6 mmol) and triethylamine (2.89 mL, 20.7 mmol) in dichloromethane to give E/Z -6-chloro-3-cyclohexylmethylene-2-oxo-2,3-dihydro-indole-1-carboxylic acid ethyl ester as a brown solid (Yield 3.3 g, 95%).

### Example 6c

### Preparation of intermediate racemic (2'S, 3S, 4'R)-6-chloro-2'-(3-chlorophenyl)-4'-cyclohexyl-2,3-dihydro-2,6'-dioxo spiro[indole-3,3'-piperidine]-1-carboxylic acid ethyl ester

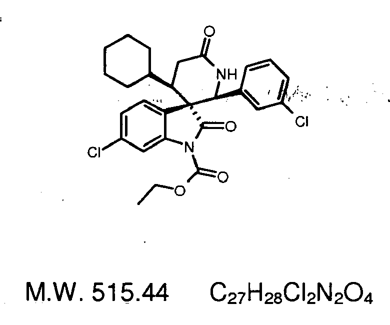

In a manner similar to the method described in example 4c, E/Z -6-chloro-3-cyclohexylmethylene-2-oxo-2,3-dihydro-indole-1-carboxylic acid ethyl ester (0.40 g, 1.20 mmol) was reacted with 1-(3-chlorophenyl)-3-trimethylsilyoxy-2-aza-1,3-butadiene prepared in example 1 b, in toluene to give racemic ((2'S, 3S, 4'R)-6-chloro-2'-(3-chlorophenyl)-4'-cyclohexyl-2,3-dihydro-2,6'-dioxo spiro[indole-3,3'-piperidine]-1-carboxylic acid ethyl ester as a yellow oil (Yield 0.2 g, 32%).

### Example 6d

### Preparation of racemic (2'S, 3S, 4'R)-6-chloro-2'-(3-chlorophenyl)-4'-cyclohexyl spiro[3H-indole-3,3'-piperidine]-2,6'(1H)-dione

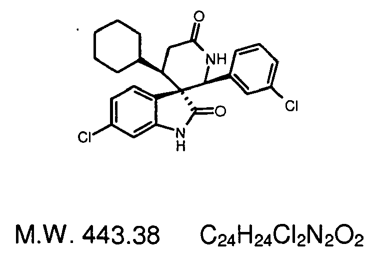

In a manner similar to the method described in example 4d, racemic (2'S, 3S, 4'R)-6-chloro-2'-(3-chlorophenyl)-4'-cyclohexyl-2,3-dihydro-2,6'-dioxo spiro[indole-3,3'-piperidine]-1-carboxylic acid ethyl ester (0.2 g, 0.39 mmol) was reacted with NaOH (28 mg, 0.70 mmol) in methanol to give racemic (2'S, 3S, 4'R)-6-chloro-2'-(3-chlorophenyl)-4'-cyclohexyl spiro[3H-indole-3,3'-piperidine]-2,6'(1H)-dione as a white solid (Yield 0.05 g, 29%).

HRMS(ES⁺) *m*/*z* Calcd for C₂₄H₂₄Cl₂N₂O₂ + H [(M+H)⁺]: 443.1288. Found: 443.1288.

### Example 7a

### Preparation of intermediate 1-(4-chlorophenyl)-3-trimethylsilyoxy-2-aza-1,3-butadiene

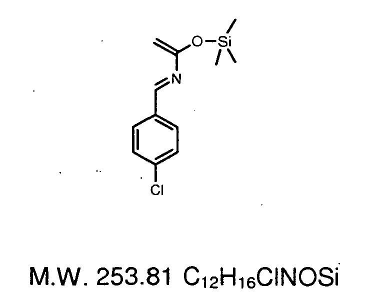

In a manner similar to the method described in example 1b, 4-chlorobenzaldehyde (1.48 g, 10.5 mmol) (Aldrich) was used as the starting material in place of 3-chloro-benzaldehyde to react with 1,1,1,3,3,3-hexamethyldisilazane (2.18 mL, 10.5 mmol), n-butyllithium (2.5 M, 4.2 mL, 10.5 mmol), trimethylsilyl chloride (1.33 mL, 10.5 mmol), triethylamine (1.9 mL, 13.6 mmol) and acetyl chloride (0.97 mL, 13.6 mmol) to give 1-(4-chlorophenyl)-3-trimethylsilyoxy-2-aza-1,3-butadiene as a yellow gum and used for the next step without further purification.

### Example 7b

### Preparation of intermediate racemic (2'S, 3S, 4'R)-6-chloro-2'-(4-chlorophenyl)-4'-cyclohexyl-2,3-dihydro-2,6'-dioxo spiro[indole-3,3'-piperidine]-1-carboxylic acid ethyl ester

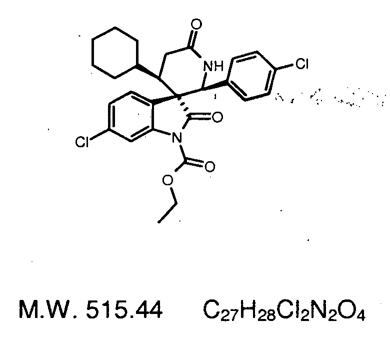

n a manner similar to the method described in example 4c, E/Z-6-chloro-3-cyclohexylmethylene-2-oxo-2,3-dihydro-indole-1-carboxylic acid ethyl ester (0.40 g, 1.20 mmol) prepared in example 6b was reacted with 1-(4-chlorophenyl)-3-trimethylsilyoxy-2-aza-1,3-butadiene prepared in example 7a, in toluene to give racemic (2'S, 3S, 4'R)-6-chloro-2'-(4-chlorophenyl)-4'-cyclohexyl-2,3-dihydro-2,6'-dioxo spiro[indole-3,3'-piperidine]-1-carboxylic acid ethyl ester as a white solid (Yield 0.45 g, 72%).

### Example 7c

### Preparation of racemic (2'S, 3S, 4'R)-6-chloro-2'-(4-chlorophenyl)-4'-cyclohexyl spiro[3H-indole-3,3'-piperidine]-2,6'(1H)-dione

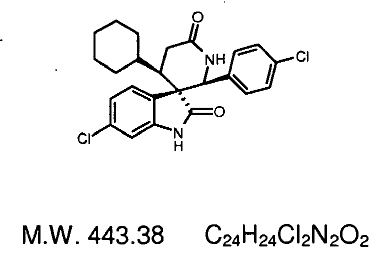

In a manner similar to the method described in example 4d, racemic (2'S, 3S, 4'R)-6-chloro-2'-(4-chlorophenyl)-4'-cyclohexyl-2,3-dihydro-2,6'-dioxo spiro[indole-3,3'-piperidine]-1-carboxylic acid ethyl ester (0.4 g, 0.78 mmol) was reacted with NaOH (56 mg, 1.4 mmol) in methanol to give racemic (2'S, 3S, 4'R)-6-chloro-2'-(4-chlorophenyl)-4'-cyclohexyl spiro[3H-indole-3,3'-piperidine]-2,6'(1H)-dione as a white solid (Yield 0.15 g, 44%).

HRMS(ES⁺) *m*/*z* Calcd for C₂₄H₂₄Cl₂N₂O₂ + H [(M+H)⁺]: 443.1288. Found: 443.1287.

### Example 8a

### Preparation of intermediate racemic (2'R, 3R, 4'S)-6-chloro-2'-(4-chlorophenyl)-4'-(3-chlorophenyl)-2,3-dihydro-2,6'-dioxo spiro[indole-3,3'-piperidine]-1-carboxylic acid ethyl ester

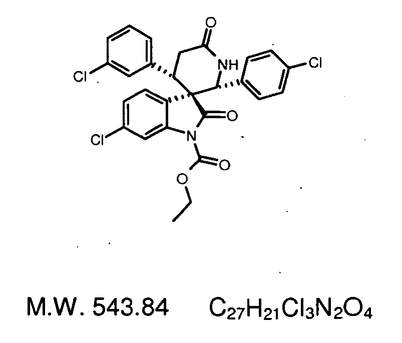

In a manner similar to the method described in example 4c, E/Z-6-chloro-3-(3-chloro-benzylidene)-2-oxo-2,3-dihydro-indole-1-carboxylic acid ethyl ester (0.3 g, 0.83 mmol) prepared in example 4b was reacted with 1-(4-chlorophenyl)-3-trimethylsilyoxy-2-aza-1,3-butadiene prepared in example 7a, in toluene to give racemic (2'R, 3R, 4'S)-6-chloro-2'-(4-chlorophenyl)-4'-(3-chlorophenyl)-2,3-dihydro-2,6'-dioxo spiro[indole-3,3'-piperidine]-1-carboxylic acid ethyl ester as a yellow solid (Yield 0.45 g, 72%).

### Example 8b

### Preparation of racemic (2'R, 3R, 4'S)-6-chloro-2'-(4-chlorophenyl)-4'-(3-chlorophenyl) spiro[3H-indole-3,3'-piperidine]-2,6'(1H)-dione

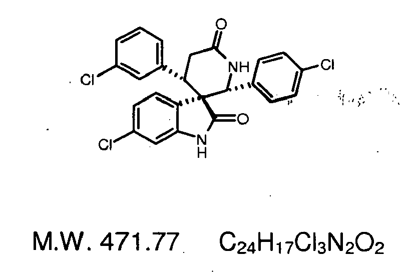

In a manner similar to the method described in example 4d, racemic (2'R, 3R, 4'S)-6-chloro-2'-(4-chlorophenyl)-4'-(3-chlorophenyl)-2,3-dihydro-2,6'-dioxo spiro[indole-3,3'-piperidine]-1-carboxylic acid ethyl ester (0.45g, 0.83 mmol) was reacted with NaOH (60 mg, 1.49 mmol) in methanol to give racemic (2'R, 3R, 4'S)-6-chloro-2'-(4-chlorophenyl)-4'-(3-chlorophenyl) spiro[3H-indole-3,3'-piperidine]-2,6'(1H)-dione as a white solid (Yield 0.2 g, 51%).

HRMS(ES⁺) *m*/*z* Calcd for C₂₄H₁₇Cl₃N₂O₂ + H [(M+H)⁺]: 471.0429. Found: 471.0427.

### Example 9a

### Preparation of intermediate E/Z-6-chloro-3-(4-chloro-benzylidene)-1,3-dihydro-indol-2-one

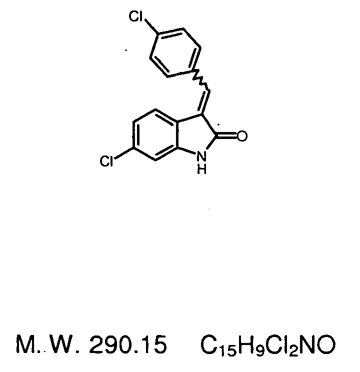

In a manner similar to the method described in example 1 a, 6-chlorooxindole (2 g, 11.4 mmol) was reacted with 4-chloro-benzaldehyde (1.91 g, 13.6 mmol)(1.53 g, 13.6 mmol) (Aldrich) and piperidine (1.34 mL, 13.6 mmol) in methanol to give a mixture of E-and Z-6-chloro-3-(4-chloro-benzylidene)-1,3-dihydro-indol-2-one as a yellow solid (Yield: 3.3 g, 100%).

### Example 9b

### Preparation of intermediate E/Z 6-chloro-3-(4-chloro-benzylidene)-2-oxo-2,3-dihydro-indole-1-carboxylic acid ethyl ester

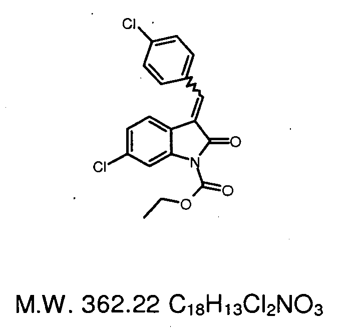

In a manner similar to the method described in example 4b, E/Z -6-chloro-3-(4-chloro-benzylidene)-1,3-dihydro-indol-2-one ( 3.3 g, 11.3 mmol) was reacted with ethyl chloroformate (1.62 mL, 17.0 mmol) and triethylamine (3.16 mL, 22.6 mmol) in dichloromethane to give E/Z-6-chloro-3-(4-chloro-benzylidene)-2-oxo-2,3-dihydro-indole-1-carboxylic acid ethyl ester as a yellow solid (Yield 3.0 g, 73%).

### Example 9c

### Preparation of intermediate racemic (2'S, 3S, 4'R)-6-chloro-2'-(3-chlorophenyl)-4'-(4-chlorophenyl)-2,3-dihydro-2,6'-dioxo spiro[indole-3,3'-piperidine]-1-carboxylic acid ethyl ester

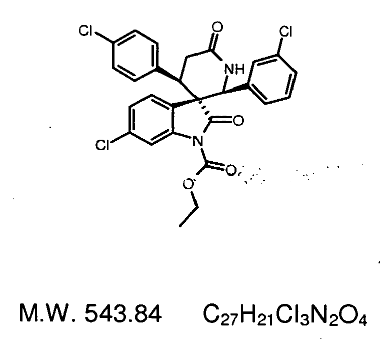

In a manner similar to the method described in example 4c, E/Z-6-chloro-3-(4-chloro-benzylidene)-2-oxo-2,3-dihydro-indole-1-carboxylic acid ethyl ester (0.35 g, 0.97 mmol) prepared in example 9b was reacted with 1-(3-chlorophenyl)-3-trimethylsilyoxy-2-aza-1,3-butadiene prepared in example 1 b, in toluene to give racemic (2'S, 3S, 4'R)-6-chloro-2'-(3-chlorophenyl)-4'-(4-chlorophenyl)-2,3-dihydro-2,6'-dioxo spiro[indole-3,3'-piperidine]-1-carboxylic acid ethyl ester as a yellow solid (Yield 0.5 g, 95%).

### Example 9d

### Preparation of racemic (2'S, 3S, 4'R)-6-chloro-2'-(3-chlorophenyl)-4'-(4-chlorophenyl) spiro[3H-indole-3,3'-piperidine]-2,6'(1H)-dione

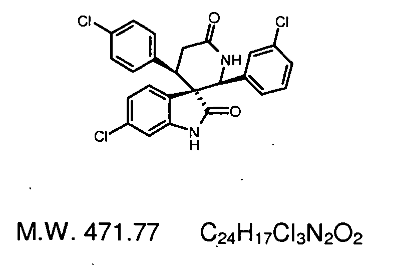

In a manner similar to the method described in example 4d, racemic (2'S, 3S, 4'R)-6-chloro-2'-(3-chlorophenyl)-4'-(4-chlorophenyl)-2,3-dihydro-2,6'-dioxo spiro[indole-3,3'-piperidine]-1-carboxylic acid ethyl ester (0.5 g, 0.92 mmol) was reacted with NaOH (67 mg, 1.67 mmol) in methanol to give racemic (2'S, 3S, 4'R)-6-chloro-2'-(3-chlorophenyl)-4'-(4-chlorophenyl) spiro[3H-indole-3,3'-piperidine]-2,6'(1H)-dione as a white solid (Yield 0.08 g, 18 %).

HRMS(ES⁺) *m*/*z* Calcd for C₂₄H₁₇Cl₃N₂O₂ + H [(M+H)⁺]: 471.0429. Found: 471.0427.

### Example 10a

### Preparation of intermediate 1-(3-methylphenyl)-3-trimethylsilyoxy-2-aza-1,3-butadiene

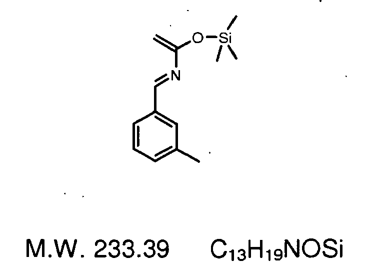

In a manner similar to the method described in example 1 b, 3-methylbenzaldehyde (1.30 g, 10.5 mmol) (Matrix) was used as the starting material in place of 3-chloro-benzaldehyde to react with 1,1,1,3,3,3-hexamethyldisilazane (2.18 mL, 10.5 mmol), n-butyllithium (2.5 M, 4.2 mL, 10.5 mmol), trimethylsilyl chloride (1.33 mL, 10.5 mmol), triethylamine (1.9 mL, 13.6 mmol) and acetyl chloride (0.97 mL, 13.6 mmol) to give 1-(3-methylphenyl)-3-trimethylsilyoxy-2-aza-1,3-butadiene as a yellow gum and used for the next step without further purification.

### Example 10b

### Preparation of intermediate racemic (2'R, 3R, 4'S)-6-chloro-4'-(3-chlorophenyl)-2,3-dihydro-2'-(3-methylphenyl)-2,6'-dioxo spiro[indole-3,3'-piperidine]-1-carboxylic acid ethyl ester

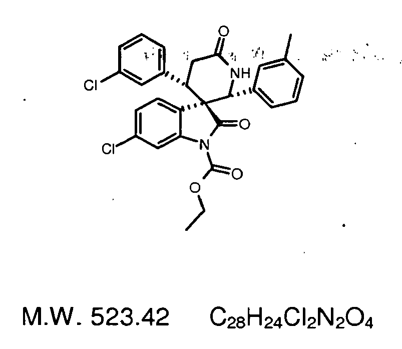

In a manner similar to the method described in example 4c, E/Z-6-chloro-3-(3-chloro-benzylidene)-2-oxo-2,3-dihydro-indole-1-carboxylic acid ethyl ester (0.4 g, 1.10 mmol) prepared in example 4b was reacted with 1-(3-methylphenyl)-3-trimethylsilyoxy-2-aza-1,3-butadiene prepared in example 10a, in toluene to give racemic (2'R, 3R, 4'S)-6-chloro-4'-(3-chlorophenyl)-2,3-dihydro-2'-(3-methylphenyl)-2,6'-dioxo spiro[indole-3,3'-piperidine]-1-carboxylic acid ethyl ester as a yellow oil (Yield 0.5 g, 86%).

### Example 10c

### Preparation of racemic (2'R, 3R, 4'S)-6-chloro-4'-(3-chlorophenyl)-2'-(3-methylphenyl) spiro[3H-indole-3,3'-piperidine]-2,6'(1H)-dione

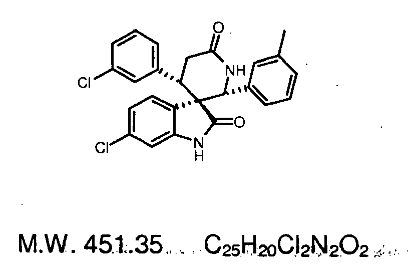

In a manner similar to the method described in example 4d, racemic (2'R, 3R, 4'S)-6-chloro-4'-(3-chlorophenyl)-2,3-dihydro-2'-(3-methylphenyl)-2,6'-dioxo spiro[indole-3,3'-piperidine]-1-carboxylic acid ethyl ester (0.5 g, 0.96 mmol) was reacted with NaOH (69 mg, 1.72 mmol) in methanol to give racemic (2'R, 3R, 4'S)-6-chloro-4'-(3-chlorophenyl)-2'-(3-methylphenyl) spiro[3H-indole-3,3'-piperidine]-2,6'(1H)-dione as a white solid (Yield 0.15 g, 35%).

HRMS(ES⁺) *m*/*z* Calcd for C₂₅H₂₀Cl₂N₂O₂ + H [(M+H)⁺]: 451.0975. Found: 451.0976.

### Example 11a

### Preparation of intermediate 1-(3-fluorophenyl)-3-trimethylsilyoxy-2-aza-1,3-butadiene

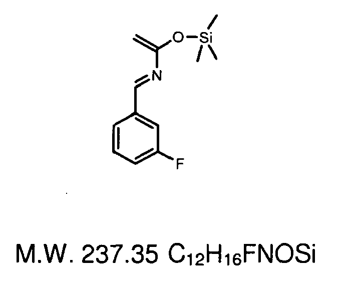

In a manner similar to the method described in example 1 b, 3-fluorobenzaldehyde (1.11 mL, 10.5 mmol) (Fluka) was used as the starting material in place of 3-chloro-benzaldehyde to react with 1,1,1,3,3,3-hexamethyldisilazane (2.18 mL, 10.5 mmol), *n*-butyllithium (2.5 M, 4.2 mL, 10.5 mmol), trimethylsilyl chloride (1.33 mL, 10.5 mmol), triethylamine (1.9 mL, 13.6 mmol) and acetyl chloride (0.97 mL, 13.6 mmol) to give 1-(3-fluorophenyl)-3-trimethylsilyoxy-2-aza-1,3-butadiene as a yellow gum and used for the next step without further purification.

### Example 11b

### Preparation of intermediate racemic (2'R, 3R, 4'S)-6-chloro-4'-(3-chlorophenyl)-2'-(3-fluorophenyl)-2,3-dihydro-2,6'-dioxo spiro[indole-3,3'-piperidine]-1-carboxylic acid ethyl ester

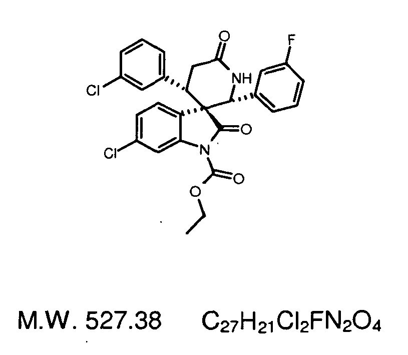

In a manner similar to the method described in example 4c, E/Z-6-chloro-3-(3-chloro-benzylidene)-2-oxo-2,3-dihydro-indole-1-carboxylic acid ethyl ester (0.25 g, 0.69 mmol) prepared in example 4b was reacted with 1-(3-fluorophenyl)-3-trimethylsilyoxy-2-aza-1,3-butadiene prepared in example 11a, in toluene to give racemic (2'R, 3R, 4'S)-6-chloro-4'-(3-chlorophenyl)-2'-(3-fluorophenyl)-2,3-dihydro-2,6'-dioxo spiro[indole-3,3'-piperidine]-1-carboxylic acid ethyl ester as a yellow oil (Yield 0.35 g, 97%).

### Example 11c

### Preparation of racemic (2'R, 3R, 4'S)-6-chloro-4'-(3-chlorophenyl)-2'-(3-fluorophenyl) spiro[3H-indole-3,3'-piperidine]-2,6'(1H)-dione

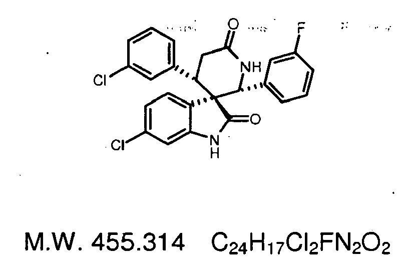

In a manner similar to the method described in example 4d, racemic (2'R, 3R, 4'S)-6-chloro-4'-(3-chlorophenyl)-2'-(3-fluorophenyl)-2,3-dihydro-2,6'-dioxo spiro[indole-3,3'-piperidine]-1-carboxylic acid ethyl ester (0.35g, 0.66 mmol) was reacted with NaOH (48 mg, 1.19 mmol) to give racemic (2'R, 3R, 4'S)-6-chloro-4'-(3-chlorophenyl)-2'-(3-fluorophenyl) spiro[3H-indole-3,3'-piperidine]-2,6'(1H)-dione as a white solid (Yield 0.15 g, 50%).

HRMS(ES⁺) *m*/*z* Calcd for C₂₄H₁₇Cl₂FN₂O₂ + H [(M+H)⁺]: 451.0975. Found: 451.0976.

### Example 12a

### Preparation of intermediate E/Z-3-benzylidene-6-chloro-1,3-dihydro-indol-2-one

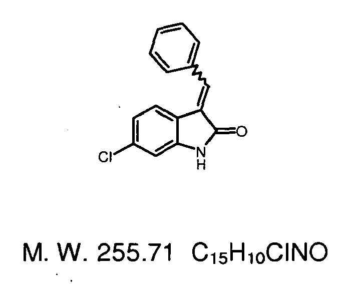

In a manner similar to the method described in example 1 a, 6-chlorooxindole (1.0 g, 5.7 mmol) was reacted with benzaldehyde (0.6 g, 5.7 mmol) (Aldrich) and pyrrolidine (0.4 g, 5.7 mmol) in methanol to give a mixture of E-and Z-3-benzylidene-6-chloro-1,3-dihydro-indol-2-one as a yellow solid (Yield 1.5 g, 100%).

### Example 12b

### Preparation of intermediate E/Z-3-benzylidene-6-chloro-2-oxo-2,3-dihydro-indole-1-carboxylic acid ethyl ester

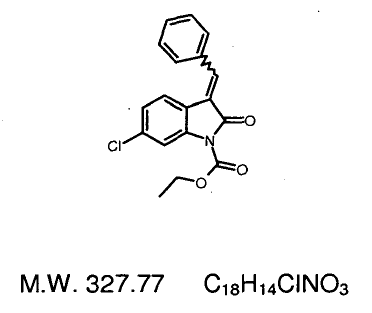

In a manner similar to the method described in example 4b, E/Z-3-benzylidene-6-chloro-1,3-dihydro-indol-2-one (1.5 g, 5.87 mmol) was reacted with ethyl chloroformate (0.83 mL, 8.8 mmol) and triethylamine (1.64 mL, 12 mmol) in dichloromethane to give E/Z-3-benzylidene-6-chloro-2-oxo-2,3-dihydro-indole-1-carboxylic acid ethyl ester as a yellow solid (Yield 2.0 g, 100%).

### Example 12c

### Preparation of racemic (2'S, 3S, 4'R)-6-chloro-2'-(3-chlorophenyl)-4'-phenyl spiro[3H-indole-3,3'-piperidine]-2,6'(1H)-dione

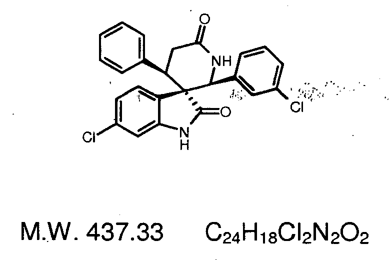

To a solution of 1-(3-chlorophenyl)-3-trimethylsilyoxy-2-aza-1,3-butadiene prepared in example 1c in toluene (30 mL) was added E/Z-3-benzylidene-6-chloro-2-oxo-2,3-dihydro-indole-1-carboxylic acid ethyl ester prepared in Example 12b (0.4 g, 1.22 mmol). The reaction mixture was stirred under nitrogen in a sealed tube at 140 °C for 1 h. After the solution was cooled to room temperature, methanol (40 mL) was added. The reaction mixture was filtered through a short pad of celite gel and washed with ethyl acetate. The filtrate was concentrated. The residue was dissolved in methanol (30 mL) and 1 N of NaOH solution (5 mL, 5 mmol) was added. The reaction mixture was stirred at room temperature for 0.5 h, then the mixture was concentrated. The residue was purified by chromatography (EtOAc:CH₂Cl₂ =1:4) to give racemic (2'S, 3S, 4'R)-6-chloro-2'-(3-chlorophenyl)-4'-phenyl spiro[3H-indole-3,3'-piperidine]-2,6'(1H)-dione as a yellow oil (Yield 0.5 g, 100%).

HRMS(ES⁺) *m*/*z* Calcd for C₂₄H₁₈Cl₂N₂O₂ + H [(M+H)⁺]: 437.0818. Found: 437.0817.

### Example 13a

### Preparation of intermediate 1-(3-chlorophenyl)-4-methyl-3-trimethylsilyoxy-2-aza-1,3-butadiene

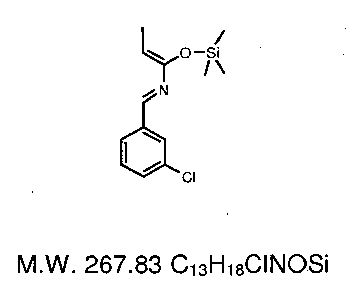

In a manner similar to the method described in example 1b, propionyl chloride (1.2 g, 13.mmol) (Aldrich) was used as the starting material in place of acetyl chloride to react with 1,1,3,3,3-hexamethyldisilazane (1.61 g, 10 mmol), n-butyllithium (2.5 M, 4 mL, 10 mmol), 3-chloro-benzaldehyde (1.4 g, 10 mmol) (Aldrich), trimethylsilyl chloride (1.1 g, 10 mmol) and triethylamine (1.36 g, 13 mmol) to give 1-(3-chlorophenyl)-4-methyl-3-trimethylsilyoxy-2-aza-1,3-butadiene as a yellow gum and used for the next step without further purification.

### Example 13b

### Preparation of racemic (2'S, 3S, 4'R, 5'R)-6-chloro-2'-(3-chlorophenyl)-5'-methyl-4'-phenyl spiro[3H-indole-3,3'-piperidine]-2,6'(1H)-dione

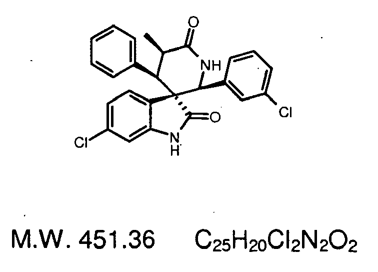

In a manner similar to the method described in example 12c, E/Z-3-benzylidene-6-chloro-2-oxo-2,3-dihydro-indole-1-carboxylic acid ethyl ester (0.32 g, 0.98 mmol) prepared in example 12b was reacted with 1-(3-chlorophenyl)-4-methyl-3-trimethylsilyoxy-2-aza-1,3-butadiene (1.5 g, 5.6 mmol) prepared in example 13a in toluene and then 2 N of NaOH solution (4 mL, 8 mmol) in methanol to give racemic (2'S, 3S, 4'R, 5'R)-6-chloro-2'-(3-chlorophenyl)-5'-methyl-4'-phenyl spiro[3H-indole-3,3'-piperidine]-2,6'(1H)-dione as a white foam (Yield 0.21 g, 48%).

HRMS(ES⁺) *m*/*z* Calcd for C₂₅H₂₀Cl₂N₂O₂ + H [(M+H)⁺]: 451.0975. Found: 451.0972.

### Example 14a

### Preparation of intermediate 1-phenyl-3-trimethylsilyoxy-2-aza-1,3-butadiene

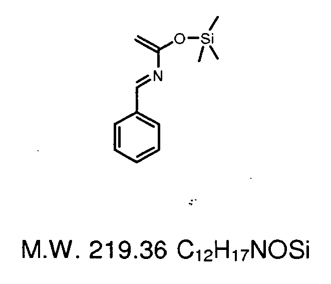

In a manner similar to the method described in example 1b, benzaldehyde (1.06 g, 10 mmol) (Aldrich) was used as the starting material in place of 3-chlorobenzaldehyde to react with 1,1,3,3,3-hexamethyldisilazane (1.6 mL, 10 mmol), n-butyllithium (2.5 M, 4 mL, 10 mmol), trimethylsilyl chloride (1.08 mL, 10 mmol), triethylamine (1.31 mL, 13 mmol) and acetyl chloride (1.02 g, 13 mmol) to give 1-phenyl-3-trimethylsilyoxy-2-aza-1,3-butadiene as a yellow gum and used for the next step without further purification.

### Example 14b

### Preparation of racemic (2'R, 3R, 4'S)-6-chloro-4'-(3-chlorophenyl)-2'-phenyl spiro[3H-indole-3,3'-piperidine]-2,6'(1H)-dione

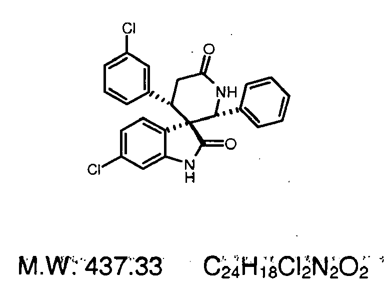

To a solution of 1-phenyl-3-trimethylsilyoxy-2-aza-1,3-butadiene (1.5 g, 6.8 mmol) prepared in example 14a in toluene (30 mL) was added E/Z-3-benzylidene-6-chloro-2-oxo-2,3-dihydro-indole-1-carboxylic acid ethyl ester (0.22 g, 0.61 mmol) prepared in example 4b. The reaction mixture was stirred under nitrogen in a sealed tube at 150 °C for 1.5 h. After the solution was cooled to room temperature, methanol (40 mL) was added. The reaction mixture was filtered through a short pad of celite gel and washed with ethyl acetate. The filtrate was concentrated. The residue was dissolved in ethanol (20 mL) and NaOH (0.2 g, 5 mmol) was added, followed by the addition of a couple drops of H₂O. After the reaction mixture was stirred at room temperature for 1 h, the mixture was concentrated. The residue was partitioned between ethyl acetate and 1 N of HCl solution. The organic layer was separated and concentrated. The residue was purified by chromatography (EtOAc:hexanes = 2:1) to give racemic (2'R, 3R, 4'S)-6-chloro-4'-(3-chlorophenyl)-2'-phenyl spiro[3H-indole-3,3'-piperidine]-2,6'(1H)-dione as a yellow solid (Yield 0.14 g, 52%).

HRMS(ES⁺) *m*/*z* Calcd for C₂₄H₁₈Cl₂N₂O₂ + H [(M+H)⁺]: 437.0818. Found: 437.0816.

### Example 14c

### Preparation of chiral (2'R, 3R, 4'S)-6-chloro-4'-(3-chlorophenyl)-2'-phenyl spiro[3H-indole-3,3'-piperidine]-2,6'(1H)-dione

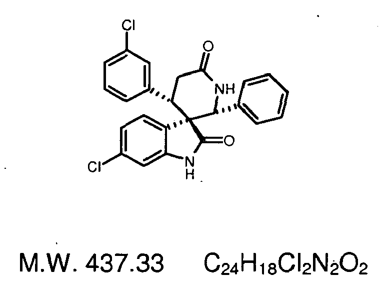

Separation of the two enantiomers from racemic (2'R, 3R, 4'S)-6-chloro-4'-(3-chlorophenyl)-2'-phenyl spiro[3H-indole-3,3'-piperidine]-2,6'(1H)-dione prepared in example 14b was conducted by chiral SFC to provide chiral (2'R, 3R, 4'S)-6-chloro-4'-(3-chlorophenyl)-2'-phenyl spiro[3H-indole-3,3'-piperidine]-2,6'(1H)-dione as a yellow solid (25 mg) and chiral (2'S, 3S, 4'R)-6-chloro-4'-(3-chlorophenyl)-2'-phenyl spiro[3H-indole-3,3'-piperidine]-2,6'(1H)-dione as a yellow solid (27 mg).

### Example 15a

### Preparation of intermediate 1-(3-methoxyphenyl)-3-trimethylsilyoxy-2-aza-1,3-butadiene

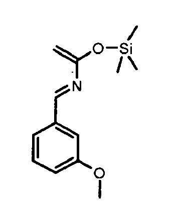

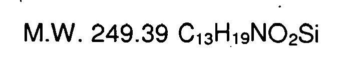

In a manner similar to the method described in example 1b, 3-methoxybenzaldehyde (1.3 g, 9.5 mmol) (Aldrich) was used as the starting material in place of 3-chloro-benzaldehyde to react with 1,1,1,3,3,3-hexamethyldisilazane (1.53 g, 9.5 mmol), n-butyllithium (2.5 M, 3.8 mL, 9.5 mmol), trimethylsilyl chloride (1.2 mL, 9.5 mmol), triethylamine (1.72 mL, 12.4 mmol) and acetyl chloride (0.88 mL, 12.4 mmol) to give 1-(3-methoxyphenyl)-3-trimethylsilyoxy-2-aza-1,3-butadiene as a yellow gum and used for the next step without further purification.

### Example 15b

### Preparation of racemic (2'R, 3R, 4'S)-6-chloro-4'-(3-chlorophenyl)-2'-(3-methoxyphenyl) spiro[3H-indole-3,3'-piperidine]-2,6'(1H)-dione

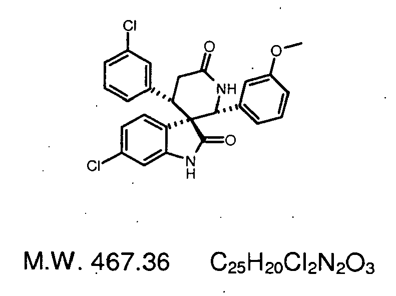

In a manner similar to the method described in example 14b, E/Z-6-chloro-3-(3-chloro-benzylidene)-2-oxo-2,3-dihydro-indole-1-carboxylic acid ethyl ester (0.23 g, 0.63 mmol) prepared in example 4b was reacted with 1-(3-methoxyphenyl)-3-trimethylsilyoxy-4-methyl-2-aza-1,3-butadiene (2 g, 8.0 mmol) prepared in example 15a in toluene and then 2 N of NaOH solution (4 mL, 8 mmol) in methanol to give racemic (2'R, 3R, 4'S)-6-chloro-4'-(3-chlorophenyl)-2'-(3-methoxyphenyl) spiro[3H-indole-3,3'-piperidine]-2,6'(1H)-dione

(Yield 0.2 g, 69%).

HRMS(ES⁺) *m*/*z* Calcd for C₂₅H₂₀Cl₂N₂O₃ + H [(M+H)⁺]: 467.0924. Found: 467.0925.

### Example 16a

### Preparation of intermediate 1-(2-chlorophenyl)-3-trimethylsilyoxy-2-aza-1,3-butadiene

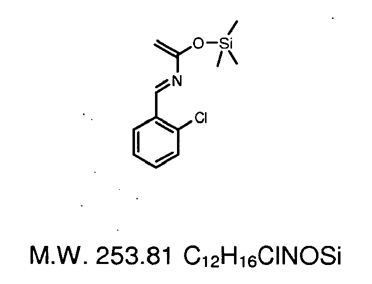

In a manner similar to the method described in example 1b, 2-chloro-benzaldehyde (1.3 g, 9.25 mmol) (Aldrich) was used as the starting material in place of 3-chloro-benzaldehyde to react with 1,1,1,3,3,3-hexamethyldisilazane (1.49 g, 9.25 mmol), n-butyllithium (2.5 M, 3.7 mL, 9.25 mmol), trimethylsilyl chloride (1.2 mL, 9.25 mmol), triethylamine (1.7 mL, 12.0 mmol) and acetyl chloride (0.85 mL, 12.0 mmol) to give 1-(2-chlorophenyl)-3-trimethylsilyoxy-2-aza-1,3-butadiene as a yellow gum and used for the next step without further purification.

### Example 16b

### Preparation of intermediate racemic (2'R, 3R, 4'S)-6-chloro-2'-(2-chlorophenyl)-4'-(3-chlorophenyl)-2,3-dihydro-2,6'-dioxo spiro[indole-3,3'-piperidine]-1-carboxylic acid ethyl ester

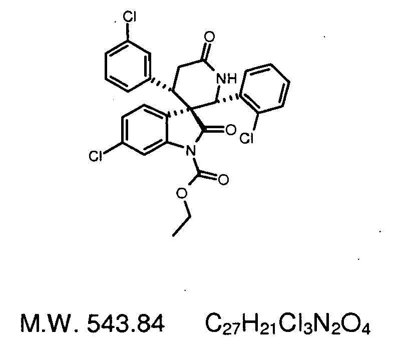

In a manner similar to the method described in example 4c, E/Z-6-chloro-3-(3-chloro-benzylidene)-2-oxo-2,3-dihydro-indole-1-carboxylic acid ethyl ester (0.31 g, 0.85 mmol) prepared in example 4b was reacted with 1-(2-chlorophenyl)-3-trimethylsilyoxy-2-aza-1,3-butadiene (2.1 g, 8.27 mmol) prepared in example 16a, in toluene to give racemic (2'R, 3R, 4'S)-6-chloro-2'-(2-chlorophenyl)-4'-(3-chlorophenyl)-2,3-dihydro-2,6'-dioxo spiro[indole-3,3'-piperidine]-1-carboxylic acid ethyl ester as a yellow gum (Yield 0.31 g, 67%).

### Example 16c

### Preparation of racemic (2'R, 3R, 4'S)-6-chloro-2'-(2-chlorophenyl)-4'-(3-chlorophenyl) spiro[3H-indole-3,3'-piperidine]-2,6'(1H)-dione

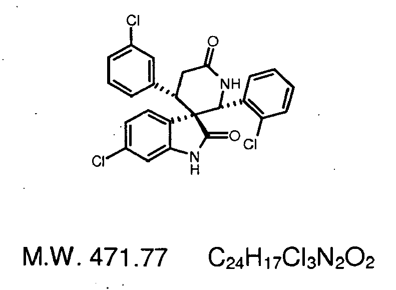

In a manner similar to the method described in example 4d, racemic (2'R, 3R, 4'S)-6-chloro-2'-(2-chlorophenyl)-4'-(3-chlorophenyl)-2,3-dihydro-2,6'-dioxo spiro[indole-3,3'-piperidine]-1-carboxylic acid ethyl ester (0.3 g, 0.55 mmol) was reacted with NaOH (2N, 5 mL, 10 mmol) in methanol to give racemic (2'R, 3R, 4'S)-6-chloro-2'-(2-chlorophenyl)₋4'-(3-chlorophenyl) spiro[3H-indole-3,3'-piperidine]-2,6'(1H)-dione as a white solid (Yield: 0.21 g, 81%).

HRMS(ES⁺) *m*/*z* Calcd for C₂₄H₁₇Cl₃N₂O₂ + H [(M+H)⁺]: 471.0429. Found: 471.0430.

### Example 16d

### Preparation of chiral (2'R, 3R, 4'S)-6-chloro-2'-(2-chlorophenyl)-4'-(3-chlorophenyl) spiro[3H-indole-3,3'-piperidine]-2,6'(1H)-dione

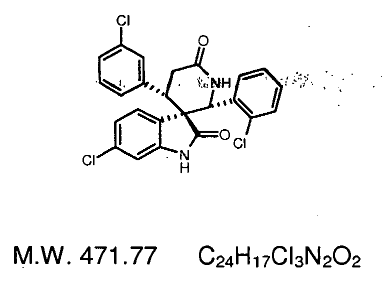

Separation of the two enantiomers from racemic (2'R, 3R, 4'S)-6-chloro-2'-(2-chlorophenyl)-4'-(3-chlorophenyl) spiro[3H-indole-3,3'-piperidine]-2,6'(1H)-dione prepared in example 16c was conducted by chiral SFC to provide chiral (2'R, 3R, 4'S)-6-chloro-2'-(2-chlorophenyl)-4'-(3-chlorophenyl) spiro[3H-indole-3,3'-piperidine]-2,6'(1H)-dione as a white solid (53 mg) and (2'S, 3S, 4'R)-6-chloro-2'-(2-chlorophenyl)-4'-(3-chlorophenyl) spiro[3H-indole-3,3'-piperidine]-2,6'(1H)-dione as a white solid (51 mg).

### Example 17a

### Preparation of intermediate 1-(2-methylphenyl)-3-trimethylsilyoxy-2-aza-1,3-butadiene

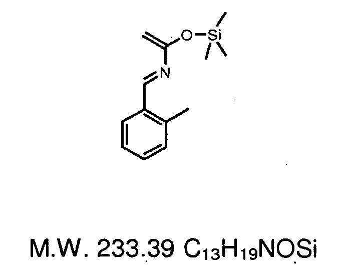

In a manner similar to the method described in example 1 b, 2-methylbenzaldehyde (1.2 g, 10 mmol) (Aldrich) was used as the starting material in place of 3-chloro-benzaldehyde to react with 1,1,1,3,3,3-hexamethyldisilazane (1.62 g, 10 mmol), n-butyllithium (2.5 M, 4 mL, 10 mmol), trimethylsilyl chloride (1.1 g, 10 mmol), triethylamine (1.3 g, 13 mmol) and acetyl chloride (1.0 g, 13 mmol) to give 1-(2-methylphenyl)-3-trimethylsilyoxy-2-aza-1,3-butadiene as a yellow gum and used for the next step without further purification.

### Example 17b

### Preparation of intermediate racemic (2'R, 3R, 4'S)-6-chloro-4'-(3-chlorophenyl)-2,3-dihydro-2'-(2-methylphenyl)-2,6'-dioxo spiro[indole-3,3'-piperidine]-1-carboxylic acid ethyl ester

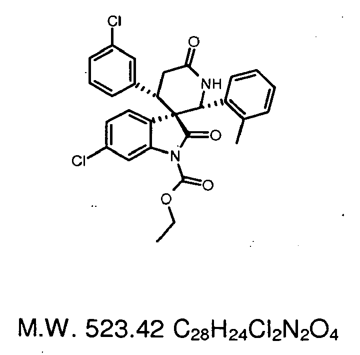

In a manner similar to the method described in example 4c, E/Z-6-chloro-3-(3-chloro-benzylidene)-2-oxo-2,3-dihydro-indole-1-carboxylic acid ethyl ester (0.32 g, 0.88 mmol) prepared in example 4b was reacted with 1-(2-methylphenyl)-3-trimethylsilyoxy-2-aza-1,3-butadiene (2.4 g, 1.71 mmol) prepared in example 17a, in toluene to give racemic (2'R, 3R, 4'S)-6-chloro-4'-(3-chlorophenyl)-2,3-dihydro-2'-(2-methylphenyl)-2,6'-dioxo spiro[indole-3,3'-piperidine]-1-carboxylic acid ethyl ester (Yield 0.4 g, 87%).

### Example 17c

### Preparation of racemic (2'R, 3R, 4'S)-6-chloro-4'-(3-chlorophenyl)-2'-(2-methylphenyl) spiro[3H-indole-3,3'-piperidine]-2,6'(1H)-dione

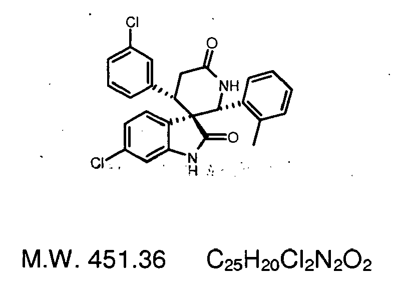

In a manner similar to the method described in example 4d, racemic (2'R, 3R, 4'S)-6-chloro-4'-(3-chlorophenyl)-2,3-dihydro-2'-(2-methylphenyl)-2,6'-dioxo spiro[indole-3,3'-piperidine]-1-carboxylic acid ethyl ester (0.4 g, 0.76 mmol) was reacted with 2 N of NaOH solution (10 mL, 20 mmol) in methanol to give racemic (2'R, 3R, 4'S)-6-chloro-4'-(3-chlorophenyl)-2'-(2-methylphenyl) spiro[3H-indole-3,3'-piperidine]-2,6'(1H)-dione (Yield 0.24 g, 70%).

HRMS(ES⁺) *m*/*z* Calcd for C₂₅H₂₀Cl₂N₂O₂ + H [(M+H)⁺]: 451.0975. Found: 451.0972.

### Example 17d

### Preparation of chiral (2'R, 3R, 4'S)-6-chloro-4'-(3-chlorophenyl)-2'-(2-methylphenyl) spiro[3H-indole-3,3'-piperidine]-2,6'(1H)-dione

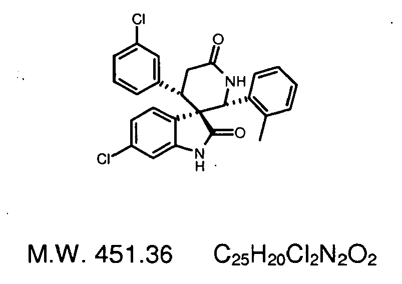

Separation of the two enantiomers from racemic (2'R, 3R, 4'S)-6-chloro-4'-(3-chlorophenyl)-2'-(2-methylphenyl) spiro[3H-indole-3,3'-piperidine]-2,6'(1H)-dione prepared in example 23c was conducted by chiral SFC to provide chiral (2'R, 3R, 4'S)-6-chloro-4'-(3-chlorophenyl)-2'-(2-methylphenyl) spiro[3H-indole-3,3'-piperidine]-2,6'(1H)-dione as a white solid (52 mg) and chiral (2'S, 3S, 4'R)-6-chloro-4'-(3-chlorophenyl)-2'-(2-methylphenyl) spiro[3H-indole-3,3'-piperidine]-2,6'(1H)-dione as a white solid (52 mg).

### Example 18a

### Preparation of intermediate 1-(2-ethylphenyl)-3-trimethylsilyoxy-2-aza-1,3-butadiene

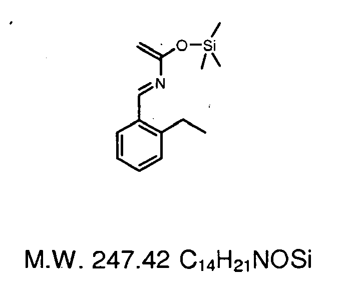

In a manner similar to the method described in example 1 b, 2-ethylbenzaldehyde (1.6 g, 11.8 mmol) (Aldrich) was used as the starting material in place of 3-chloro-benzaldehyde to react with 1,1,1,3,3,3-hexamethyldisilazane (1.96 g, 11.8 mmol), *n*-butyllithium (2.5 M, 4.7 mL, 11.8 mmol), trimethylsilyl chloride (1.50 mL, 11.8 mmol), triethylamine (2.13 mL, 15.3 mmol) and acetyl chloride (1.09 mL, 15.3 mmol) to give 1-(2-ethylphenyl)-3-trimethylsilyoxy-2-aza-1,3-butadiene as a yellow gum and used for the next step without further purification.

### Example 18b

### Preparation of racemic (2'R, 3R, 4'S)-6-chloro-4'-(3-chlorophenyl)-2'-(2-ethylphenyl) spiro[3H-indole-3,3'-piperidine]-2,6'(1H)-dione

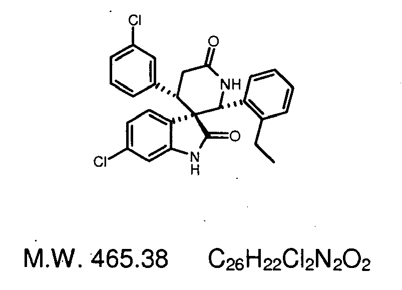

In a manner similar to the method described in example 14b, E/Z-6-chloro-3-(3-chloro-benzylidene)-2-oxo-2,3-dihydro-indole-1-carboxylic acid ethyl ester (0.4 g, 1.1 mmol) prepared in example 4b was reacted with 1-(2-ethylphenyl)-3-trimethylsilyoxy-4-methyl-2-aza-1,3-butadiene (3.2 g, 12.9 mmol) prepared in example 18a in toluene and then 2 N of NaOH solution (5 mL, 10 mmol) in methanol to give racemic (2'R, 3R, 4'S)-6-chloro-4'-(3-chlorophenyl)-2'-(2-ethylphenyl) spiro[3H-indole-3,3'-piperidine]-2,6'(1H)-dione (Yield 0.085 g, 17%).

HRMS(ES⁺) *m*/*z* Calcd for C₂₆H₂₂Cl₂N₂O₂ + H [(M+H)⁺]: 465.1131. Found: 465.1132.

### Example 18c

### Preparation of chiral (2'R, 3R, 4'S)-6-chloro-4'-(3-chlorophenyl)-2'-(2-ethylphenyl) spiro[3H-indole-3,3'-piperidine]-2,6'(1H)-dione

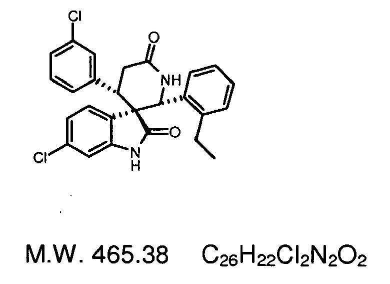

Separation of the two enantiomers from racemic (2'R, 3R, 4'S)-6-chloro-4'-(3-chlorophenyl)-2'-(2-ethylphenyl) spiro[3H-indole-3,3'-piperidine]-2,6'(1H)-dione prepared in example 17b was conducted by chiral SFC to provide chiral (2'R, 3R, 4'S)-6-chloro-4'-(3-chlorophenyl)-2'-(2-ethylphenyl) spiro[3H-indole-3,3'-piperidine]-2,6'(1H)-dione as an off-white solid (25 mg) and chiral (2'S, 3S, 4'R)-6-chloro-4'-(3-chlorophenyl)-2'-.(2-ethylphenyl) spiro[3H-indole-3,3'-piperidine]-2,6'(1H)-dione as an off-white solid (27 mg).

### Example 19a

### Preparation of intermediate 4-methyl-1-(2-methylphenyl)-3-trimethylsilyoxy-2-aza-1,3-butadiene

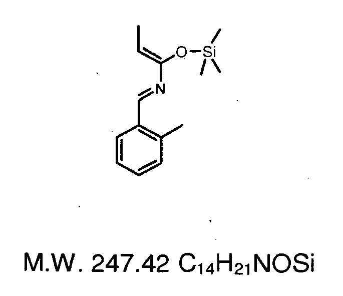

In a manner similar to the method described in example 13a, 2-methylbenzaldehyde (1.2 g, 10 mmol) (Aldrich) was used as the starting material in place of 3-chloro-benzaldehyde to react with 1,1,1,3,3,3-hexamethyldisilazane (1.61 g, 10 mmol), n-butyllithium (2.5 M, 4 mL, 10 mmol), trimethylsilyl chloride (1.1 g, 10 mmol), triethylamine (1.4 g, 13 mmol) and propionyl chloride (1.2 g, 13 mmol) to give 4-methyl-1-(2-methylphenyl)-3-trimethylsilyoxy-2-aza-1,3-butadiene as a yellow gum and used for the next step without further purification

### Example 19b

### Preparation of racemic (2'R, 3R, 4'S, 5'S)-6-chloro-4'-(3-chlorophenyl)-5'-methyl-2'-(2-methylphenyl) spiro[3H-indole-3,3'-piperidine]-2,6'(1H)-dione

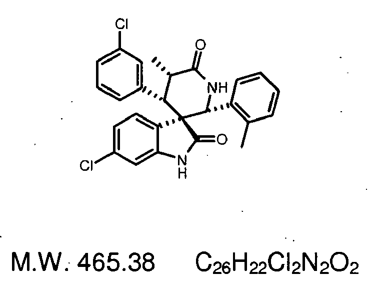

In a manner similar to the method described in example 14b, E/Z-6-chloro-3-(3-chloro-benzylidene)-2-oxo-2,3-dihydro-indole-1-carboxylic acid ethyl ester (0.36 g, 0.99 mmol) prepared in example 4b was reacted with 4-methyl-1-(2-methylphenyl)-3-trimethylsilyoxy-2-aza-1,3-butadiene) prepared in example 19a in toluene and then 2 N of NaOH solution (4 mL, 8 mmol) in methanol to give racemic (2'R, 3R, 4'S, 5'S)-6-chloro-4'-(3-chlorophenyl)-5'-methyl-2'-(2-methylphenyl) spiro[3H-indole-3,3'-piperidine]-2,6'(1H)-dione as a white solid (Yield 0.26 g, 56%).

HRMS(ES⁺) *m*/*z* Calcd for C₂₆H₂₂Cl₂N₂O₂ + H [(M+H)⁺]: 465.1131. Found: 465.1132.

### Example 20

### Preparation of racemic (2'R, 3R, 4'S, 5'S)-6-chloro-2',4'-bis(3-chlorophenyl)-5'-methyl spiro[3H-indole-3,3'-piperidine]-2,6'(1H)-dione

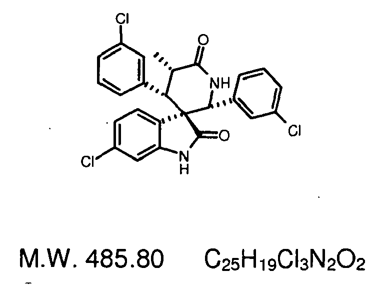

In a manner similar to the method described in example 14b, E/Z-6-chloro-3-(3-chloro-benzylidene)-2-oxo-2,3-dihydro-indole-1-carboxylic acid ethyl ester (0.36 g, 0.99 mmol) prepared in example 4b was reacted with 1-(3-chlorophenyl)-4-methyl-3-trimethylsilyoxy-2-aza-1,3-butadiene (2.2 g, 8.9 mmol) prepared in example 13a in toluene and then 2 N of NaOH solution (4 mL, 8 mmol) in methanol to give racemic (2'R, 3R, 4'S, 5'S)-6-chloro-2',4'-bis(3-chlorophenyl)-5'-methyl spiro[3H-indole-3,3'-piperidine]-2,6'(1H)-dione (Yield 0.26 g, 57%).

HRMS(ES⁺) *m*/*z* Calcd for C₂₅H₁₉Cl₃N₂O₂+ H [(M+H)⁺]: 485.0585. Found: 485.0583

### Example 21a

### Preparation of intermediate 1-(2-isopropylphenyl)-3-trimethylsilyoxy-2-aza-1,3-butadiene

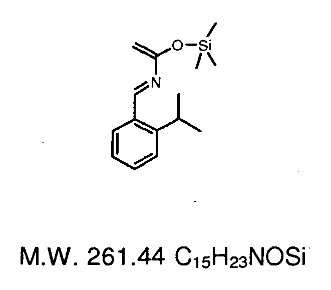

In a manner similar to the method described in example 1b, 2-isopropylbenzaldehyde (1.5 g, 10 mmol) (Aldrich) was used as the starting material in place of 3-chloro-benzaldehyde to react with 1,1,1,3,3,3-hexamethyldisilazane (1.61 g, 10 mmol), n-butyllithium (2.5 M, 4 mL, 10 mmol), trimethylsilyl chloride (1.1 g, 10 mmol), triethylamine (1.3 g, 13 mmol) and acetyl chloride (1.0 g, 13 mmol) to give 1-(2-isopropylphenyl)-3-trimethylsilyoxy-2-aza-1,3-butadiene as a yellow gum and used for the next step without further purification.

### Example 21b

### Preparation of racemic (2'R, 3R, 4'S)-6-chloro-4'-(3-chlorophenyl)-2'-(2-isopropylphenyl) spiro[3H-indole-3,3'-piperidine]-2,6'(1H)-dione

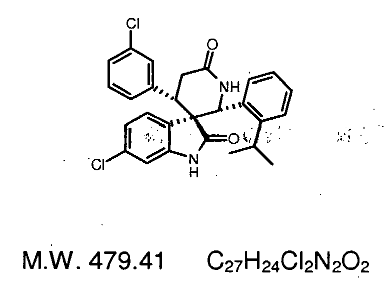

In a manner similar to the method described in example 14b, E/Z-6-chloro-3-(3-chloro-benzylidene)-2-oxo-2,3-dihydro-indole-1-carboxylic acid ethyl ester (0.38 g, 1.05 mmol) prepared in example 4b was reacted with 1-(2-isopropylphenyl)-3-trimethylsilyoxy-2-aza-1,3- butadiene (2.7 g, 10.3 mmol) prepared in example 21 a in toluene and then 2 N of NaOH solution (5 mL, 10 mmol) in methanol to give racemic (2'R, 3R, 4'S)-6-chloro-4'-(3-chlorophenyl)-2'-(2-isopropylphenyl) spiro[3H-indole-3,3'-piperidine]-2,6'(1H)-dione as a white solid (Yield 0.26 g, 52%).

HRMS(ES⁺) *m*/*z* Calcd for C₂₇H₂₄Cl₂N₂O₂ + H [(M+H)⁺]: 479.1288. Found: 479.1289.

### Example 21c

### Preparation of chiral (2'R, 3R, 4'S)-6-chloro-4'-(3-chlorophenyl)-2'-(2-isopropylphenyl) spiro[3H-indole-3,3'-piperidine]-2,6'(1H)-dione

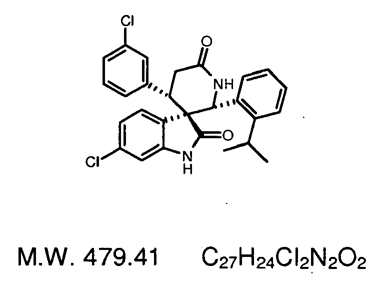

Separation of the two enantiomers from racemic (2'R, 3R, 4'S)-6-chloro-4'-(3-chlorophenyl)-2'-(2-isopropylphenyl) spiro[3H-indole-3,3'-piperidine]-2,6'(1H)-dione prepared in example 21b was conducted by chiral SFC to provide chiral (2'R, 3R, 4'S)-6-chloro-4'-(3-chlorophenyl)-2'-(2-isopropylphenyl) spiro[3H-indole-3,3'-piperidine]-2,6'(1H)-dione as a white solid (61 mg) and (2'S, 3S, 4'R)-6-chloro-4'-(3-chlorophenyl)-2'-(2-isopropylphenyl) spiro[3H-indole-3,3'-piperidine]-2,6'(1H)-dione as a white solid (66 mg).

### Example 22a

### Preparation of intermediate 1-(2-bromophenyl)-3-trimethylsilyoxy-2-aza-1,3-butadiene

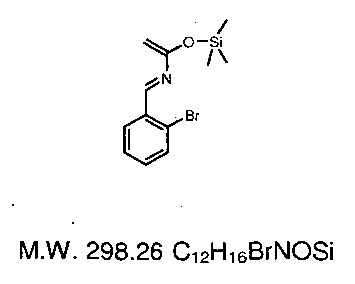

In a manner similar to the method described in example 1b, 2-bromobenzaldehyde (1.85 g, 10 mmol) (Aldrich) was used as the starting material in place of 3-chloro-benzaldehyde to react with 1,1,1,3,3,3-hexamethyldisilazane (1.62 g, 10 mmol), *n*-butyllithium (2.5 M, 4 mL, 10 mmol), trimethylsilyl chloride (1.1 g, 10 mmol), triethylamine (1.3 g, 13 mmol) and acetyl chloride (1.0 g, 13 mmol) to give 1-(2-bromophenyl)-3-trimethylsilyoxy-2-aza-1,3-butadiene as a yellow gum and used for the next step without further purification.

### Example 22b

### Preparation of intermediate racemic (2'R, 3R, 4'S)-2'-(2-bromophenyl)-6-chloro-4'-(3-chlorophenyl)-2,3-dihydro-2,6'-dioxo spiro[indole-3,3'-piperidine]-1-carboxylic acid ethyl ester

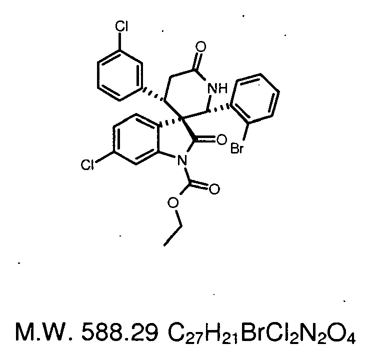

In a manner similar to the method described in example 4c, E/Z-6-chloro-3-(3-chloro-benzylidene)-2-oxo-2,3-dihydro-indole-1-carboxylic acid ethyl ester (0.4 g, 1.1 mmol) prepared in example 4b was reacted with 1-(2-bromophenyl)-3-trimethylsilyoxy-2-aza-1,3-butadiene (3.0 g, 10 mmol) prepared in example 22a, in toluene to give racemic (2'R, 3R, 4'S)-2'-(2-bromophenyl)-6-chloro-4'-(3-chlorophenyl)-2,3-dihydro-2,6'-dioxo spiro[indole-3,3'-piperidine]-1-carboxylic acid ethyl ester (0.45 g, 69%).

### Example.22c

### Preparation of racemic (2'R, 3R, 4'S)-2'-(2-bromophenyl)-6-chloro-4'-(3-chlorophenyl) spiro[3H-indole-3,3'-piperidine]-2,6'(1H)-dione

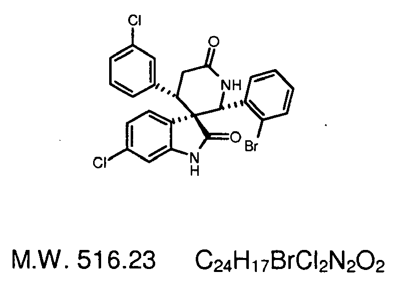

In a manner similar to the method described in example 4d, racemic (2'R, 3R, 4'S)-2'-(2-bromophenyl)-6-chloro-4'-(3-chlorophenyl)-2,3-dihydro-2,6'-dioxo spiro[indole-3,3'-piperidine]-1-carboxylic acid ethyl ester (0.45 g, 0.76 mmol) was reacted with NaOH in methanol (2N, 5 mL, 10 mmol) to give racemic (2'R, 3R, 4'S)-2'-(2-bromophenyl)-6-chloro-4'-(3-chlorophenyl) spiro[3H-indole-3,3'-piperidine]-2,6'(1H)-dione as a white solid (Yield 0.25 g, 64%).

HRMS(ES⁺) *m*/*z* Calcd for C₂₄H₁₇BrCl₂N₂O₂ + H [(M+H)⁺]: 514.9923. Found: 514.9926.

### Example 22d

### Preparation of chiral (2'R, 3R, 4'S)-2'-(2-bromophenyl)-6-chloro-4'-(3-chlorophenyl) spiro[3H-indole-3,3'-piperidine]-2,6'(1H)-dione

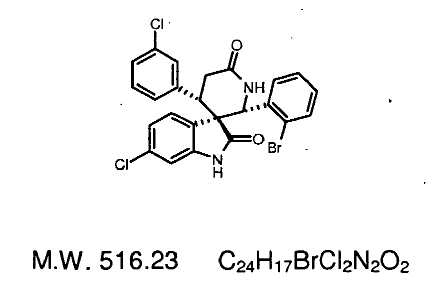

Separation of the two enantiomers from racemic (2'R, 3R, 4'S)-2'-(2-bromophenyl)-6-chloro-4'-(3-chlorophenyl) spiro[3H-indole-3,3'-piperidine]-2,6'(1H)-dione prepared in example 22c was conducted by chiral SFC to provide chiral (2'R, 3R, 4'S)-2'-(2-bromophenyl)-6-chloro-4'-(2-chlorophenyl) spiro[3H-indole-3,3'-piperidine]-2,6'(1H)-dione as a white solid (86 mg) and chiral (2'S, 3S, 4'R)-2'-(2-bromophenyl)-6-chloro-4'-(3-chlorophenyl) spiro[3H-indole-3,3'-piperidine]-2,6'(1H)-dione as a white solid (76 mg).

### Example 23a

### Preparation of intermediate 1-(3-cyanophenyl)-3-trimethylsilyoxy-2-aza-1, 3-butadiene

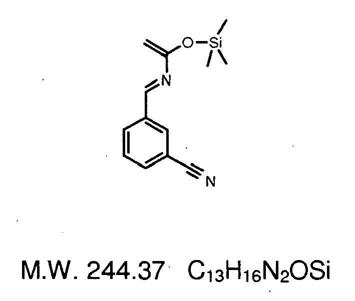

In a manner similar to the method described in example 1 b, 3-cyanobenzaldehyde (1.2 g, 10 mmol) (Aldrich) was used as the starting material in place of 3-chloro-benzaldehyde to react with 1,1,1,3,3,3-hexamethyldisilazane (1.62 g, 10 mmol), n-butyllithium (2.5 M, 4 mL, 10 mmol), trimethylsilyl chloride (1.1 g, 10 mmol), triethylamine (1.3 g, 13 mmol) and acetyl chloride (1.0 g, 13 mmol) to give 1-(3-cyanophenyl)-3-trimethylsilyoxy-2-aza-1,3-butadiene as a yellow gum and used for the next step without further purification.

### Example 23b

Preparation of racemic (2'R, 3R, 4'S)-6-chloro-4'-(3-chlorophenyl)-2'-(3-cyanophenyl) spiro[3H-indole-3,3'-piperidine]-2,6'(1H)-dione

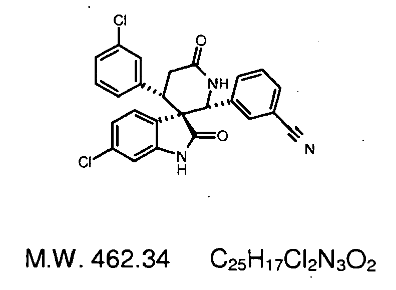

In a manner similar to the method described in example 14b, E/Z-6-chloro-3-(3-chloro-benzylidene)-2-oxo-2,3-dihydro-indole-1-carboxylic acid ethyl ester (0.42 g, 1.2 mmol) prepared in example 4b was reacted with 1-(3-cyanophenyl)-3-trimethylsilyoxy-2-aza-1,3-butadiene (2.0 g, 8.18 mmol) prepared in example 23a in toluene and then 2 N of NaOH solution (5 mL, 10 mmol) in methanol to give racemic (2'R, 3R, 4'S)-6-chloro-4'-(3-chlorophenyl)-2'-(3-cyanophenyl) spiro[3H-indole-3,3'-piperidine]-2,6'(1H)-dione as a white foam (Yield 0.11 g, 20%).

HRMS(ES⁺) *m*/*z* Calcd for C₂₅H₁₇Cl₂N₃O₂ + H [(M+H)⁺]: 462.0771 Found: 462.0771.

### Example 24a

### Preparation of intermediate E/Z-6-chloro-3-(3-chloro-benzylidene)-2-oxo-2,3-dihydro-indole-1-carboxylic acid tert-butyl ester

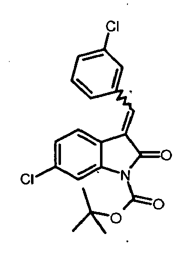

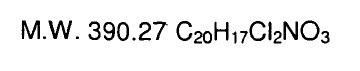

To a solution of E/Z-6-chloro-3-(3-chloro-benzylidene)-1,3-dihydro-indol-2-one prepared in example 4a (1 g, 3.4 mmol) in dichloromethane (50 mL) at room temperature was added Di-*tert*-butyl-dicarbonate (1.5 g, 6.9 mmol) (Aldrich), followed by the addition of 4-dimethylaminopyridine (1 g, 8.2 mmol). The reaction mixture was stirred at room temperature for 1 h. The mixture was then concentrated and the residue was purified by chromatography to give E/Z-6-chloro-3-(3-chloro-benzylidene)-2-oxo-2,3-dihydro-indole-1-carboxylic acid *tert-*butyl ester as an orange solid (Yield 1.3 g, 96%).

### Example 24b

### Preparation of intermediate racemic (2'R, 3R, 4'S)-6-chloro-4'-(3-chlorophenyl)-2,3-dihydro-2'-(2-methylphenyl)-2,6'-dioxo spiro[indole-3,3'-piperidine]-1-carboxylic acid tert-butyl ester

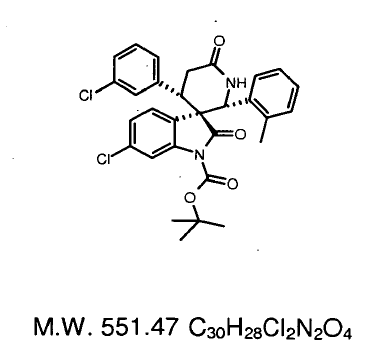

In a manner similar to the method described in example 4c, E/Z-6-chloro-3-(3-chloro-benzylidene)-2-oxo-2,3-dihydro-indole-1-carboxylic acid *tert*-butyl ester (1 g, 2.6 mmol) prepared in example 24a was reacted with 1-(2-methylphenyl)-3-trimethylsilyoxy-2-aza-1,3-butadiene (3.3 g, 14.1 mmol) prepared in example 17a, in toluene to give racemic (2'R, 3R, 4'S)-6-chloro-4'-(3-chlorophenyl)-2,3-dihydro-2'-(2-methylphenyl)-2,6'-dioxo spiro[indole-3,3'-piperidine]-1-carboxylic acid *tert-*butyl ester as a white foam (Yield: 0.92 g, 65%).

### Example 24c

### Preparation of intermediate racemic (2'R, 3R, 4'S)-6-chloro-4'-(3-chlorophenyl)-2,3-dihydro-1'-methyl-2'-(2-methylphenyl) -2,6'-dioxo spiro[indole-3,3'-piperidine]-1-carboxylic acid tert-butyl ester

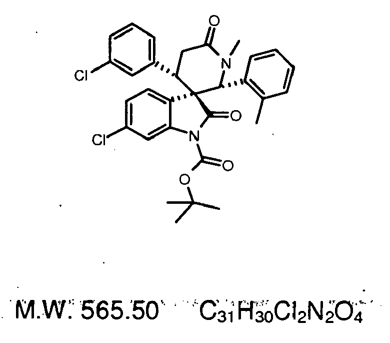

To a solution of racemic (2'R, 3R, 4'S)-6-chloro-4'-(3-chlorophenyl)-2,3-dihydro-2'-(2-methylphenyl)-2,6'-dioxo spiro[indole-3,3'-piperidine]-1-carboxylic acid *tert-*butyl ester (0.25 g, 0.45 mmol) in N,N-dimethyl-formamide (20 mL) at 0 °C was added LiH (90 mg, 11.2 mmol) (Aldrich), followed by the addition of iodomethane (0.39 g, 2.74 mmol). The reaction mixture was warmed up to room temperature and stirred at room temperature for 3 h. The mixture was diluted with ethyl acetate, and then washed with saturated NH₄Cl solution. The aqueous layer was extracted with ethyl acetate and the combined organic layer was dried over MgSO₄. The solvent was removed and the residue was purified by chromatography (EtOAc:hexanes=1:2) to give racemic (2'R, 3R, 4'S)-6-chloro-4'-(3-chlorophenyl)-2,3-dihydro-1'-methyl-2'-(2-methylphenyl) -2,6'-dioxo spiro[indole-3,3'-piperidine]-1-carboxylic acid *tert*-butyl ester (Yield 0.20 g, 77%).

### Example 24d

### Preparation of racemic (2'R, 3R, 4'S)-6-chloro-4'-(3-chlorophenyl)-1'-methyl-2'-(2-methylphenyl) spiro[3H-indole-3,3'-piperidine]-2,6'(1H)-dione

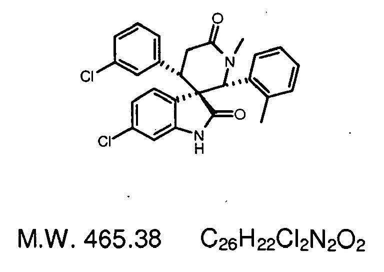

Trifluoroacetic acid (5 mL) was added to a solution of racemic (2'R, 3R, 4'S)-6-chloro-4'-(3-chlorophenyl)-2,3-dihydro-1'-methyl-2'-(2-methylphenyl)-2,6'-dioxo spiro[indole-3,3'-piperidine]-1-carboxylic acid *tert*-butyl ester (0.20 g, 0.35 mmol) prepared in example 24c in dichloromethane (10 mL). The mixture was stirred at room temperature for 1 h. The solvent was evaporated *in vacuo.* To this residue was added saturated NaHCO₃ solution, and extracted with ethyl acetate. The organic layers were combined, washed with water and brine, dried over MgSO₄. The solvent was removed and the residue was purified by chromatography (EtOAc:CH₂Cl₂=1:4) to give racemic (2'R, 3R, 4'S)-6-chloro-4'-(3-chlorophenyl)-1'-methyl-2'-(2-methylphenyl) spiro[3H-indole-3,3'-piperidine]-2,6'(1H)-dione as a white solid (Yield: 0.075 g, 46%).

HRMS(ES⁺) *m*/*z* Calcd for C₂₆H₂₂Cl₂N₂O₂ + H [(M+H)⁺]: 465.1131. Found: 465.1132.

### Example 24e

### Preparation of chiral (2'R, 3R, 4'S)-6-chloro-4'-(3-chlorophenyl)-1'-methyl-2'-(2-methylphenyl) spiro[3H-indole-3,3'-piperidine]-2,6'(1H)-dione

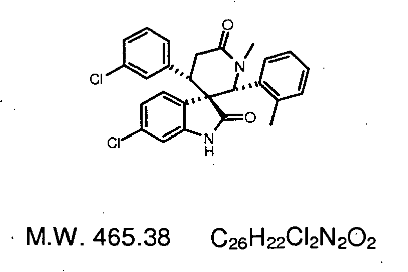

Separation of the two enantiomers from racemic (2'R, 3R, 4'S)-6-chloro-4'-(3-chlorophenyl)-1 '-methyl-2'-(2-methylphenyl) spiro[3H-indole-3,3'-piperidine]-2,6'(1H)-dione prepared in example 24d was conducted by chiral SFC to provide chiral (2'R, 3R, 4'S)-6-chloro-4'-(3-chlorophenyl)-1'-methyl-2'-(2-methylphenyl) spiro[3H-indole-3,3'-piperidine]-2,6'(1H)-dione as a white solid (24 mg) and chiral (2'S, 3S, 4'R)-6-chloro-4'-(3-chlorophenyl)-1'-methyl-2'-(2-methylphenyl) spiro[3H-indole-3,3'-piperidine]-2,6'(1H)-dione as a white solid (23 mg).

### Example 25a

### Preparation of intermediate 1-(3-fluoro-2-methyl-phenyl)-3-trimethylsilyoxy-2-aza-1,3-butadiene

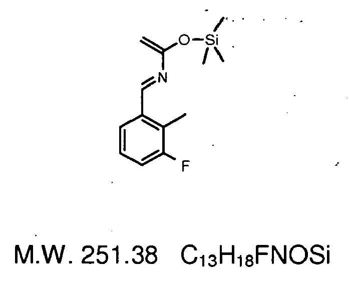

In a manner similar to the method described in example 1 b, 3-fluoro-2-methylbenzaldehyde (1.4 g, 10 mmol) (Aldrich) was used as the starting material in place of 3-chloro-benzaldehyde to react with 1,1,3,3,3-hexamethyldisilazane (1.62 g, 10 mmol), n-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(3-fluoro-2-methyl-phenyl)-3-trimethylsilyoxy-2-aza-1,3-butadiene as a yellow gum and used for the next step without further purification.

### Example 25b

### Preparation of intermediate racemic (2'R, 3R, 4'S)-6-chloro-4'-(3-chlorophenyl)-2'-(3-fluoro-2-methyl-phenyl)-2,3-dihydro-2,6'-dioxo spiro[indole-3,3'-piperidine]-1-carboxylic acid ethyl ester

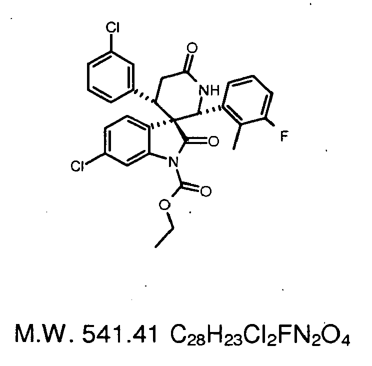

In a manner similar to the method described in example 4c, E/Z-6-chloro-3-(3-chloro-benzylidene)-2-oxo-2,3-dihydro-indole-1-carboxylic acid ethyl ester (0.39 g, 1.08 mmol) prepared in example 4b was reacted with 1-(3-fluoro-phenyl-2-methyl)-3-trimethylsilyoxy-2-aza-1,3-butadiene (2.6 g, 10.3 mmol) prepared in example 31 a, in toluene to give racemic (2'R, 3R, 4'S)-6-chloro-4'-(3-chlorophenyl)-2'-(3-fluoro-2-methyl-phenyl)-2,3-dihydro-2,6'-dioxo spiro[indole-3,3'-piperidine]-1-carboxylic acid ethyl ester (Yield 0.35 g, 60%).

### Example 25c

### Preparation of racemic (2'R, 3R, 4'S)-6-chloro-4'-(3-chlorophenyl)-2'-(3-fluoro-2-methyl-phenyl) spiro[3H-indole-3,3'-piperidine]-2,6'(1H)-dione

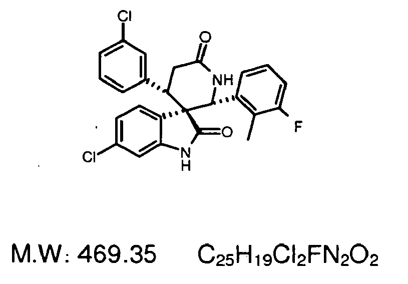

In a manner similar to the method described in example 4d, racemic (2'R, 3R, 4'S)-6-chloro-4'-(3-chlorophenyl)-2'-(3-fluoro-2-methyl-phenyl)-2,3-dihydro-2,6'-dioxo spiro[indole-3,3'-piperidine]-1-carboxylic acid ethyl ester (0.35 g, 0.65 mmol) was reacted with NaOH (2N, 5 mL, 10 mmol) in methanol to give racemic (2'R, 3R, 4'S)-6-chloro-4'-(3-chlorophenyl)-2'-(3-fluoro-2-methyl-phenyl) spiro[3H-indole-3,3'-piperidine]-2,6'(1H)-dione as a white solid (Yield 0.25 g, 83%).

HRMS(ES⁺) *m*/*z* Calcd for C₂₅H₁₉Cl₂FN₂O₂ + H [(M+H)⁺]: 469.0881. Found: 469.0885.

### Example 25d

### Preparation of chiral (2'R, 3R, 4'S)-6-chloro-4'-(3-chlorophenyl)-2'-(3-fluoro-2-methyl-phenyl) spiro[3H-indole-3,3'-piperidine]-2,6'(1H)-dione

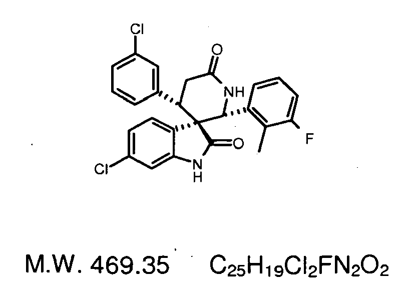

Separation of the two enantiomers from racemic (2'R, 3R, 4'S)-6-chloro-4'-(3-chlorophenyl)-2'-(3-fluoro-2-methyl-phenyl) spiro[3H-indole-3,3'-piperidine]-2,6'(1H)-dione prepared in example 25c was conducted by chiral SFC to provide chiral (2'R, 3R, 4'S)-6-chloro-4'-(3-chlorophenyl)-2'-(3-fluoro-2-methyl-phenyl) spiro[3H-indole-3,3'-piperidine]-2,6'(1H)-dione as a white solid (71 mg) and chiral (2'S, 3S, 4'R)-6-chloro-4'-(3-chlorophenyl)-2'-(3-fluoro-2-methyl-phenyl) spiro[3H-indole-3,3'-piperidine]-2,6'(1H)-dione as a white solid (74 mg).

### Example 26a

### Preparation of intermediate racemic (2'R, 3R, 4'S)-6-chloro-4'-(3-chlorophenyl)-1'-ethyl-2,3-dihydro-2'-(2-methylphenyl)-2,6'-dioxo spiro[indole-3,3'-piperidine]-1-carboxylic acid tert-butyl ester

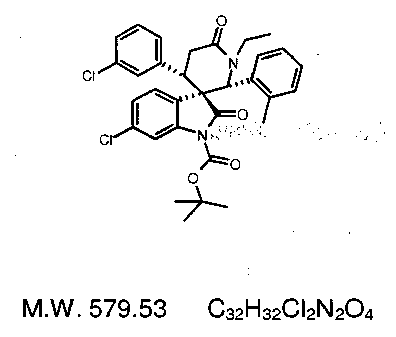

In a manner similar to the method described in example 24c, racemic (2'R, 3R, 4'S)-6-chloro-4'-(3-chlorophenyl)-2,3-dihydro-2'-(2-methylphenyl-2,6'-dioxo spiro[indole-3,3'-piperidine]-1-carboxylic acid *tert*-butyl ester (0.3 g, 0.54 mmol) prepared in example 24b was reacted with LiH (86 mg, 10.9 mmol) (Aldrich) and iodoethane (2 mL, 25 mmol) in N,N-dimethyl-formamide to give racemic (2'R, 3R, 4'S)-6-chloro-4'-(3-chlorophenyl)-1'-ethyl-2,3-dihydro-2'-(2-methylphenyl)-2,6'-dioxo spiro[indole-3,3'-piperidine]-1-carboxylic acid *tert*-butyl ester as a white foam (Yield: 0.2 g, 63%).

### Example 26b

### Preparation of racemic (2'R, 3R, 4'S)-6-chloro-4'-(3-chlorophenyl)-1'-ethyl-2'-(2-methylphenyl) spiro[3H-indole-3,3'-piperidine]-2,6'(1H)-dione

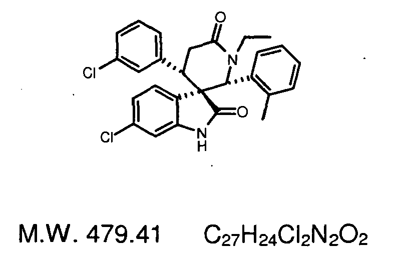

In a manner similar to the method described in example 24d, racemic (2'R, 3R, 4'S)-6-chloro-4'-(3-chlorophenyl)-l'-ethyl-2,3-dihydro-2'-(2-methylphenyl)-2,6'-dioxo spiro[indole-3,3'-piperidine]-1-carboxylic acid *tert*-butyl ester (0.2 g, 0.34 mmol) prepared in example 26a was reacted with trifluoroacetic acid in dichlormethane to give racemic (2'R, 3R, 4'S)-6-chloro-4'-(3-chlorophenyl)-1'-ethyl-2'-(2-methylphenyl) spiro[3H-indole-3,3'-piperidine]-2,6'(1H)-dione as an off-white foam (Yield: 0.11 g, 69%).

HRMS(ES⁺) *m*/*z* Calcd for C₂₇H₂₄Cl₂N₂O₂ + H [(M+H)⁺]: 479.1288. Found. 479.1284.

### Example 26c

### Preparation of chiral (2'R, 3R, 4'S)-6-chloro-4'-(3-chlorophenyl)-1'-ethyl-2'-(2-methylphenyl) spiro[3H-indole-3,3'-piperidine]-2,6'(1H)-dione

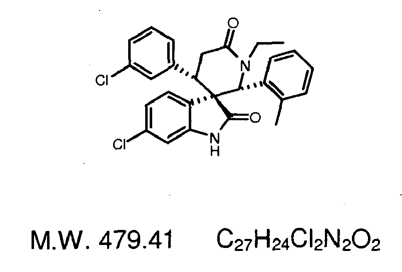

Separation of the two enantiomers from racemic (2'R, 3R, 4'S)-6-chloro-4'-(3-chlorophenyl)-1'-ethyl-2'-(2-methylphenyl) spiro[3H-indole-3,3'-piperidine]-2,6'(1H)-dione prepared in 26b was conducted by chiral SFC to provide chiral (2'R, 3R, 4'S)-6-chloro-4'-(3-chlorophenyl)-1'-ethyl-2'-(2-methylphenyl) spiro[3H-indole-3,3'-piperidine]-2,6'(1H)-dione as a white solid (29 mg) and chiral (2'S, 3S, 4'R)-6-chloro-4'-(3-chlorophenyl)-1'-ethyl-2'-(2-methylphenyl) spiro[3H-indole-3,3'-piperidine]-2,6'(1H)-dione as a white solid (27 mg).

### Example 27a

### Preparation of intermediate 1-(2,6-dimethylphenyl)-3-trimethylsilyoxy-2-aza-1,3-butadiene

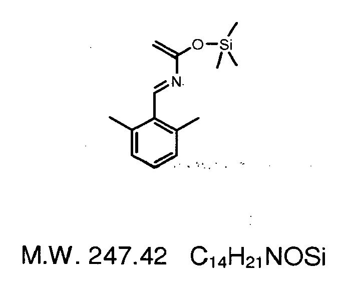

In a manner similar to the method described in example 1b, 2,6-dimethylbenzaldehyde (1.3 g, 10 mmol) (Aldrich) was used as the starting material in place of 3-chloro-benzaldehyde to react with 1,1,3,3,3-hexamethyldisilazane (1.61 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(2,6-dimethylphenyl)-3-trimethylsilyoxy-2-aza-1,3-butadiene as an orange oil and used for the next step without further purification.

### Example 27b

### Preparation of intermediate racemic (2'R, 3R, 4'S)-6-chloro-4'-(3-chlorophenyl)-2'-(2,6-dimethylphenyl)- 2,3-dihydro-2,6'-dioxo spiro[indole-3,3'-piperidine]-1-carboxylic acid ethyl ester

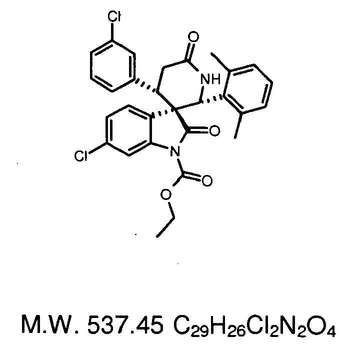

In a manner similar to the method described in example 4c, E/Z-6-chloro-3-(3-chloro-benzylidene)-2-oxo-2,3-dihydro-indole-1-carboxylic acid ethyl ester (0.4 g, 1.08 mmol) prepared in example 4b was reacted with 1-(2,6-dimethylphenyl)-3-trimethylsilyoxy-2-aza-1,3-butadiene (2.9 g, 11.7 mmol) prepared in example 27a, in toluene to give racemic (2'R, 3R, 4'S)-6-chloro-4'-(3-chlorophenyl)-2'-(2,6-dimethylphenyl)- 2,3-dihydro-2,6'-dioxo spiro[indole-3,3'-piperidine]-1-carboxylic acid ethyl ester (Yeld 0.41 g, 71%).

### Example 27c

### Preparation of racemic (2'R, 3R, 4'S)-6-chloro-4'-(3-chlorophenyl)-2'-(2,6-dimethylphenyl) spiro[3H-indole-3,3'-piperidine]-2,6'(1fH)-dione

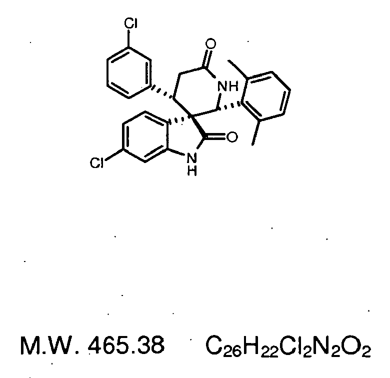

In a manner similar to the method described in example 4d, (2'R, 3R, 4'S)-6-chloro-4'-(3-chlorophenyl)-2'-(2,6-dimethylphenyl)- 2,3-dihydro-2,6'-dioxo spiro[indole-3,3'-piperidine]-1-carboxylic acid ethyl ester (0.41 g, 0.76 mmol) was reacted with NaOH (0.4 g, 10 mmol) in methanol to give racemic (2'R, 3R, 4'S)-6-chloro-4'-(3-chlorophenyl)-2'-(2,6-dimethylphenyl) spiro[3H-indole-3,3'-piperidine]-2,6'(1H)-dione (Yield 0.25 g, 71 %).

HRMS(ES⁺) *m*/*z* Calcd for C₂₆H₂₂Cl₂N₂O₂ + H [(M+H)⁺]: 465.1131. Found: 465.1131.

### Example 28a

### Preparation of intermediate 1-(2,3-dimethylphenyl)-3-trimethylsilyoxy-2-aza-1,3-butadiene

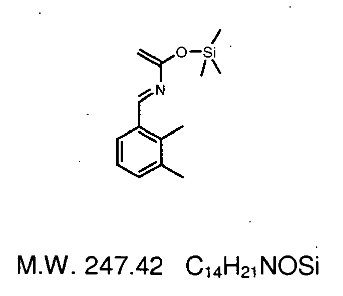

In a manner similar to the method described in example 1b, 2,3-dimethylbenzaldehyde (1.34 g, 10 mmol) (Aldrich) was used as the starting material in place of 3-chloro-benzaldehyde to react with 1,1,3,3,3-hexamethyldisilazane (1.61 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(2,3-dimethylphenyl)-3-trimethylsilyoxy-2-aza-1,3-butadiene as a yellow gum and used for the next step without further purification.

### Example 28b

### Preparation of racemic (2'R, 3R, 4'S)-6-chloro-4'-(3-chlorophenyl)-2'-(2,3-dimethylphenyl) spiro[3H-indole-3,3'-piperidine]-2,6'(1H)-dione

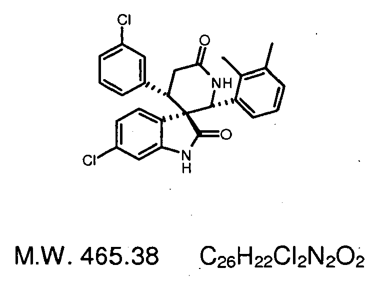

In a manner similar to the method described in example 14b, E/Z-6-chloro-3-(3-chloro-benzylidene)-2-oxo-2,3-dihydro-indole-1-carboxylic acid ethyl ester (0.3 g, 0.83 mmol) prepared in example 4b was reacted with 1-(2,3-dimethylphenyl)-3-trimethylsilyoxy-2-aza-1,3-butadiene (2.4 g, 9.72 mmol) prepared in example 28a in toluene and then NaOH (0.2 g, 5 mmol) in methanol to give racemic (2'R, 3R, 4'S)-6-chloro-4'-(3-chlorophenyl)-2'-(2,3-dimethylphenyl) spiro[3H-indole-3,3'-piperidine]-2,6'(1H)-dione (Yield 0.20 g, 53%).

HRMS(ES⁺) *m*/*z* Calcd for C₂₆H₂₂Cl₂N₂O₂ + H [(M+H)⁺]: 465.1131. Found: 465.1131.

### Example 29a

### Preparation of intermediate racemic (2'R, 3R, 4'S)-6-chloro-4'-(3-chlorophenyl)-2,3-dihydro-1'-[(methoxycarbonyl)-methyl]-2'-(2-methylphenyl)-2,6'-dioxo spiro[indole-3,3'-piperidine]-1-carboxylic acid tert-butyl ester

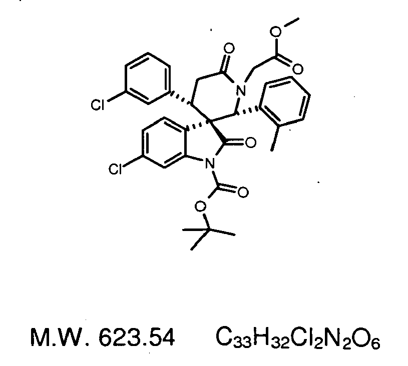

In a manner similar to the method described in example 24c, racemic (2'R, 3R, 4'S)-6-chloro-4'-(3-chlorophenyl)-2,3-dihydro-2'-(2-methylphenyl)-2,6'-dioxo spiro[indole-3,3'-piperidine]-1-carboxylic acid *tert*-butyl ester (1.02 g, 1.86 mmol) prepared in example 24b was reacted with LiH (86 mg, 10.9 mmol) (Aldrich) and methyl bromoacetate (0.57 g, 3.72 mmol) (Aldrich) to give racemic 2'R, 3R, 4'S)-6-chloro-4'-(3-chlorophenyl)-2,3-dihydro-1'-[(methoxycarbonyl)-methyl]-2'-(2-methylphenyl)-2,6'-dioxo spiro[indole-3,3'-piperidine]-1-carboxylic acid *tert*-butyl ester as a white solid (Yield: 0.37 g, 32%).

### Example 29b

### Preparation of racemic (2'R, 3R, 4'S)-6-chloro-4'-(3-chlorophenyl)-1'-[(methoxycarbonyl) methyl]-2'-(2-methylphenyl) spiro[3H-indole-3,3'-piperidine]-2,6'(1H)-dione

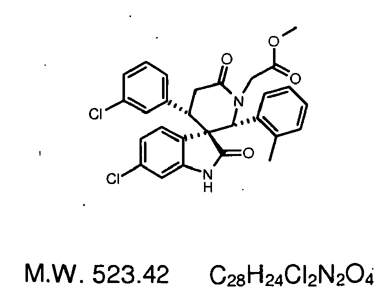

In a manner similar to the method described in example 24d, racemic 2'R, 3R, 4'S)-6-chloro-4'-(3-chlorophenyl)-2,3-dihydro-1'-[(methoxycarbonyl)-methyl]-2'-(2-methylphenyl)-2,6'-dioxo spiro[indole-3,3'-piperidine]-1-carboxylic acid *tert*-butyl ester (0.37 g, 0.59 mmol) prepared in example 29a was reacted with trifluoroacetic acid (20 mL) in dichlormethane to give racemic (2'R, 3R, 4'S)-6-chloro-4'-(3-chlorophenyl)-1'-[(methoxycarbonyl)-methyl]-2'-(2-methylphenyl) spiro[3H-indole-3,3'-piperidine]-2,6'(1H)-dione as a white solid (Yield: 0.21 g, 67%).

HRMS(ES⁺) *m*/*z* Calcd for C₂₈H₂₄Cl₂N₂O₄ + H [(M+H)⁺]: 523.1186. Found: 523.1183.

### Example 30

### Preparation of racemic (2'S, 3R)-6-chloro-2'-(3-chlorophenyl)-4'-isopropyl-6'-thioxo spiro[3H-indole-3,3'-piperidine]-2(1H)-one

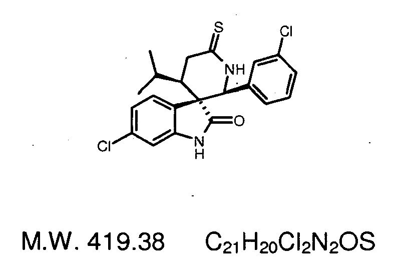

The mixture of racemic (2'S, 3R)-6-chloro-2'-(3-chlorophenyl)-4',4'-isopropyl spiro[3H-indole-3,3'-piperidine]-2,6'(1H)-dione (60 mg, 0.15 mmol) prepared in example 3b and 2,4-bis-(4-methoxyphenyl)-1,3-dithia-2,4-diphosphetane 2,4-disulfide (100 mg, 0.25 mmol) (Aldrich) in toluene (20 mL) was heated at 120 °C for 0.5 h. The mixture was cooled to room temperature and then concentrated. The residue was purified by chromatography (EtOAc:hexanes=1:1) to give racemic (2'S, 3R)-6-chloro-2'-(3-chlorophenyl)-4'-isopropyl-6'-thioxo spiro[3H-indole-3,3'-piperidine]-2(1H)-one as a white solid (Yield 60 mg, 92%).

HRMS(ES⁺) *m*/*z* Calcd for C₂₁H₂₀Cl₂N₂OS + H [(M+H)⁺]: 419.0746. Found: 419.0744.

### Example 31

### Preparation of racemic (2'R, 3R, 4'S)-6-chloro-4'-(3-chlorophenyl)-1'-[(hydroxycarbonyl)- methyl]-2'-(2-methylphenyl) spiro[3H-indole-3,3'-piperidine]-2,6'(1H)-dione

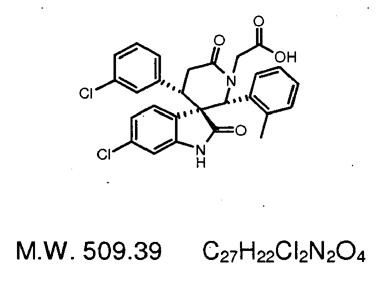

To the solution of racemic (2'R, 3R, 4'S)-6-chloro-4'-(3-chlorophenyl)-1'-[(methoxycarbonyl)-methyl]-2'-(2-methylphenyl) spiro[3H-indole-3,3'-piperidine]-2,6'(1H)-dione (0.21 g, 0.4 mmol) prepard in 29b in the mixture of methanol (10 mL) and tetrahydrofuran (20 mL) was added aqueous NaOH solution (1 N, 10 mL, 10 mmol). The reaction mixture was stirred at room temperature for overnight and then concentrated. The residue was neutralized to "pH" -7, then extracted with ethyl acetate. The organic layer was separated, washed with H₂O, brine, dried over MgSO₄ and concentrated to give racemic (2'R, 3R, 4'S)-6-chloro-4'-(3-chlorophenyl)-1'-[(hydroxycarbonyl)-methyl]-2'-(2-methylphenyl) spiro[3H-indole-3,3'-piperidine]-2,6'(1H)-dione as a white foam.

HRMS(ES⁺) *m*/*z* Calcd for C₂₇H₂₂Cl₂N₂O₄ + H [(M+H)⁺]: 509.1030. Found: 509.1028.

### Example 32a

### Preparation of intermediate 1-[2-(trifluoromethyl)-phenyl]-3-trimethylsilyoxy-2-aza-1,3-butadiene

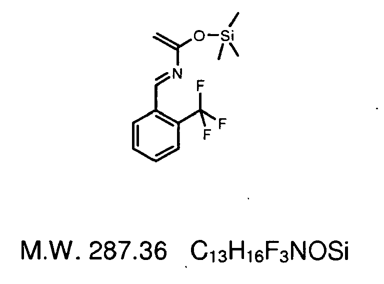

In a manner similar to the method described in example 1b, 2-(trifluoromethyl)-benzaldehyde (1.75 g, 10 mmol) (Aldrich) was used as the starting material in place of 3-chloro-benzaldehyde to react with 1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-[2-(trifluoromethyl)-phenyl]-3-trimethylsilyoxy-2-aza-1,3-butadiene as a yellow gum and used for the next step without further purification.

### Example 32b

### Preparation of racemic (2'R, 3R, 4'S)-6-chloro-4'-(3-chlorophenyl)-2'-[2-(trifluoromethyl)-phenyl] spiro[3H-indole-3,3'-piperidine]-2,6'(1H)-dione

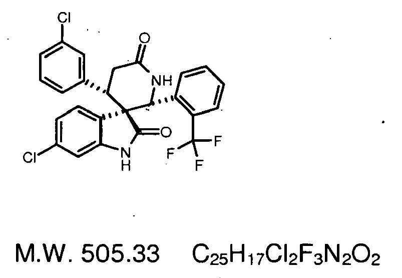

In a manner similar to the method described in example 14b, E/Z-6-chloro-3-(3-chloro-benzylidene)-2-oxo-2,3-dihydro-indole-1-carboxylic acid ethyl ester (0.5 g, 1.38 mmol) prepared in example 4b was reacted with 1-[2-(trifluoromethyl)-phenyl]-3-trimethylsilyoxy-2-aza-1,3-butadiene (3.2 g, 11.1 mmol) prepared in example 32a in toluene and then NaOH (0.2 g, 5 mmol) in methanol to give racemic (2'R, 3R, 4'S)-6-chloro-4'-(3-chlorophenyl)-2'-[2-(trifluoromethyl)-phenyl] spiro[3H-indole-3,3'-piperidine]-2,6'(1H)-dione as an off-white solid (Yield 0.45 g, 64%).

HRMS(ES⁺) *m*/*z* Calcd for C₂₅H₁₇Cl₂F₃N₂O₂ + H [(M+H)⁺]: 505.0692. Found: 505.0688.

### Example 33

### Preparation of racemic (2'R, 3R, 4'S)-6-chloro-4'-(3-chlorophenyl)-6'-thioxo-2'-[2-(trifluoromethyl)-phenyl] spiro[3H-indole-3,3'-piperidine]-2(1H)-one

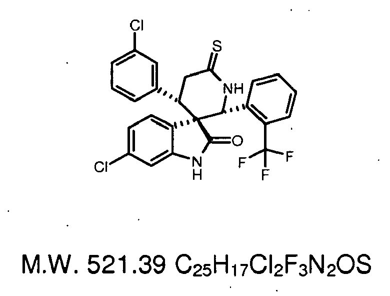

In a manner similar to the method described in example 30, racemic (2'R, 3R, 4'S)-6-chloro-4'-(3-chlorophenyl)-2'-[2-(trifluoromethyl)-phenyl] spiro[3H-indole-3,3'-piperidine]-2,6'(1H)-dione (0.4 g, 0.79 mmol) prepared in example 32b was reacted with 2,4-bis-(4-methoxyphenyl)-1,3-dithia-2,4-diphosphetane 2,4-disulfide (0.4 g, 0.99 mmol) in toluene to give racemic (2'R, 3R, 4'S)-6-chloro-4'-(3-chlorophenyl)-6'-thioxo-2'-[2-(trifluoromethyl)-phenyl] spiro[3H-indole-3,3'-piperidine]-2(1H)-one (Yield 0.15 g, 36%).

HRMS(ES⁺) *m*/*z* Calcd for C₂₅H₁₇Cl₂F₃N₂OS+H [(M+H)⁺]: 521.0464. Found: 521.0458.

### Example 34a

### Preparation of intermediate racemic (2'R, 3R, 4'S)-6-chloro-4'-(3-chlorophenyl)-1'-[(fluorocarbonyl)- methyl]-2'-(2-methylphenyl) spiro[3H-indole-3,3'-piperidine]-2,6'(1H)-dione

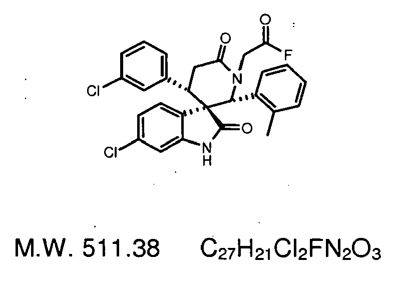

To the solution of (2'R, 3R, 4'S)-6-chloro-4'-(3-chlorophenyl)-1'-[(hydroxycarbonyl)-methyl]-2'-(2-methylphenyl) spiro[3H-indole-3,3'-piperidine]-2,6'(1H)-dione (0.12 g, 0.24 mmol) prepard in example 31 in dichloromethane (40 mL) at 0 °C was added cyanuric fluoride (48 mg, 0.35 mmol) (Alfa) and pyridine (37 mg, 0.48 mmol). After the mixture was stirred at 0 °C for 2 h, the mixture was partitioned between H₂O and dichloromethane. The organic layer was separated and the aqueous layer was extracted with dichloromethane. The organic layers were combined, dried over MgSO₄, concentrated to give racemic (2'R, 3R, 4'S)-6-chloro-4'-(3-chlorophenyl)-1'-[(fluorocarbonyl)-methyl]-2'-(2-methylphenyl) spiro[3H-indole-3,3'-piperidine]-2,6'(1H)-dione as a yellow gum and used for the next step without further purification (Yield: 0.12 g, 98%).

### Example 34b

### Preparation of racemic (2'R, 3R, 4'S)-6-chloro-4'-(3-chlorophenyl)-2'-(2-methylphenyl) -1'-[2-(4-morpholinyl)-carbonyl-methyl] spiro[3H-indole-3,3'-piperidine]-2,6'(1H)-dione

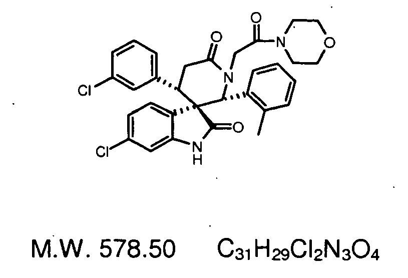

To a solution of racemic (2'R, 3R, 4'S)-6-chloro-4'-(3-chlorophenyl)-1'-[(fluorocarbonyl)- methyl]-2'-(2-methylphenyl) spiro[3H-indole-3,3'-piperidine]-2,6'(1H)-dione (0.12 g, 0.23 mmol) prepard in 34a in tetrahydrofuran (10 mL) in a sealed tube was added morpholine (41 mg, 0.47 mmol), N-methylmorpholine (47 mg, 0.47 mmol) and 4-dimethylaminopyridine (3 mg, 0.025 mmol). After the mixture was heated under microwave irridiation at 100 °C for 10 min, the mixture was diluted with ethyl acetate, washed with 1 N of HCl aqueous solution and H₂O. The organic layer was separated, dried over Na₂SO₄ and concentrated. The residue was purified by chromatography (MeOH:EtOAc = 1:19) to give racemic (2'R, 3R, 4'S)-6-chloro-4'-(3-chlorophenyl)-2'-(2-methylphenyl)-1'-[2-(4-morpholinyl)-carbonyl-methyl] spiro[3H-indole-3,3'-piperidine]-2,6'(1H)-dione as a white solid (Yield: 0.11 g, 85%).

HRMS(ES⁺) *m*/*z* Calcd for C₃₁H₂₉Cl₂N₃O₄+ H [(M+H)⁺]: 578.1608. Found: 578.1600.

### Example 34c

### Preparation of chiral (2'R, 3R, 4'S)-6-chloro-4'-(3-chlorophenyl)-2'-(2-methylphenyl) -1'-[2-(4-morpholinyl)-carbonyl-methyl] spiro[3H-indole-3,3'-piperidine]-2,6'(1H)-dione

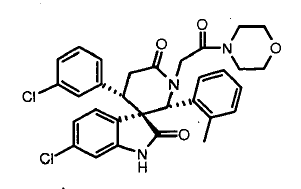

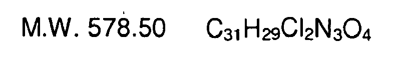

Separation of the two enantiomers from racemic (2'R, 3R, 4'S)-6-chloro-4'-(3-chlorophenyl)-2'-(2-methylphenyl)-1'-[2-(4-morpholinyl)-2-oxoethyl] spiro[3H-indole-3,3'-piperidine]-2,6'(1H)-dione (51 mg) prepared in example 34b was conducted by chiral SFC to provide chiral (2'R, 3R, 4'S)-6-chloro-4'-(3-chlorophenyl)-2'-(2-methylphenyl)-1'-[2-(4-morpholinyl)-2-oxoethyl] spiro[3H-indole-3,3'-piperidine]-2,6'(1H)-dione as a white solid (Yield: 15 mg, 29%) and chiral (2'S, 3S, 4'R)-6-chloro-4'-(3-chlorophenyl)-2'-(2-methylphenyl)-1'-[2-(4-morpholinyl)-carbonyl-methyl] spiro[3H-indole-3,3'-piperidine]-2,6'(1H)-dione as a white solid (Yield: 14 mg, 27%).

### Example 35a

### Preparation of intermediate 1-[5-fluoro-2-(trifluoromethyl)-phenyl]-3-trimethylsilyoxy-2-aza-1,3-butadiene

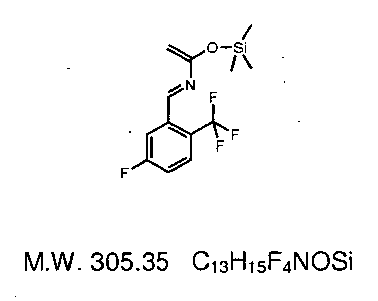

In a manner similar to the method described in example 1b, 5-fluoro-2-(trifluoromethyl)- benzaldehyde (1.9 g, 10 mmol) (Matrix) was used as the starting material in place of 3-chloro-benzaldehyde to react with 1,1,3,3,3-hexamethyldisilazane (1.61 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-[5-fluoro-2-(trifluoromethyl)-phenyl]-3-trimethylsilyoxy-2-aza-1,3-butadiene as a yellow gum and used for the next step without further purification.

### Example 35b

### Preparation of racemic (2'R, 3R, 4'S)-6-chloro-4'-(3-chlorophenyl)-2'-[5-fluoro-2-(trifluoromethyl)-phenyl] spiro[3H-indole-3,3'-piperidine]-2,6'(1H)-dione

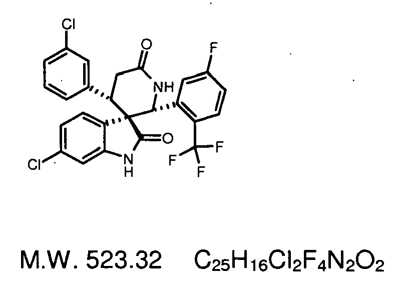

In a manner similar to the method described in example 14b, E/Z-6-chloro-3-(3-chloro-benzylidene)-2-oxo-2,3-dihydro-indole-1-carboxylic acid ethyl ester (0.41 g, 1.13 mmol) prepared in example 4b was reacted 1-[5-fluoro-2-(trifluoromethyl)-phenyl]-3-trimethylsilyoxy-2-aza-1,3-butadiene (2.9 g, 9.5 mmol) prepared in example 35a in toluene and then NaOH (0.2 g, 5 mmol) in methanol to give racemic (2'R, 3R, 4'S)-6-chloro-4'-(3-chlorophenyl)-2'-[5-fluoro-2-(trifluoromethyl)-phenyl] spiro[3H-indole-3,3'-piperidine]-2,6'(1H)-dione as an off-white solid (Yield 0.31 g, 52%).

HRMS(ES⁺) *m*/*z* Calcd for C₂₅H₁₆Cl₂F₄N₂O₂ + H [(M+H)⁺]: 523.0598. Found: 523.0593.

### Example 36a

### Preparation of intermediate 1-(5-fluoro-2-methylphenyl)-3-trimethylsilyoxy-2-aza-1,3-butadiene

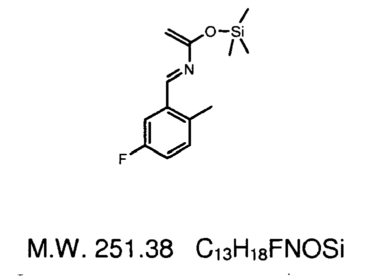

In a manner similar to the method described in example 1b, 5-fluoro-2-methylbenzaldehyde (1.38 g, 10 mmol) (Platte) was used as the starting material in place of 3-chloro-benzaldehyde to react with 1,1,1,3,3,3-hexamethyldisilazane (1.61 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(5-fluoro-2-methylphenyl)-3-trimethylsilyoxy-2-aza-1,3-butadiene as a yellow gum and used for the next step without further purification.

### Example 36b

### Preparation of racemic (2'R, 3R, 4'S)-6-chloro-4'-(3-chlorophenyl)-2'-(5-fluoro-2-methylphenyl) spiro[3H-indole-3,3'-piperidine]-2,6'(1H)-dione

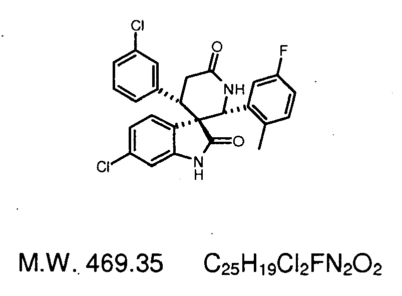

In a manner similar to the method described in example 14b, E/Z-6-chloro-3-(3-chloro-benzylidene)-2-oxo-2,3-dihydro-indole-1-carboxylic acid ethyl ester (0.25 g, 0.69 mmol) prepared in example 4b was reacted with 1-(5-fluoro-2-methylphenyl)-3-trimethylsilyoxy-2-aza-1,3-butadiene (2.5 g, 9.9 mmol) prepared in example 36a in toluene and then NaOH (1 N, 5 mL, 5 mmol) in methanol to give racemic (2'R, 3R, 4'S)-6-chloro-4'-(3-chlorophenyl)-2'-(5-fluoro-2-methylphenyl) spiro[3H-indole-3,3'-piperidine]-2,6'(1H)-dione (Yield 0.13 g, 41 %).

HRMS(ES⁺) *m*/*z* Calcd for C₂₅H₁₉Cl₂FN₂O₂ + H [(M+H)⁺]: 469.0881. Found: 469.0881.

### Example 36c

### Preparation of chiral (2'R, 3R, 4'S)-6-chloro-4'-(3-chlorophenyl)-2'-(5-fluoro-2-methylphenyl) spiro[3H-indole-3,3'-piperidine]-2,6'(1H)-dione

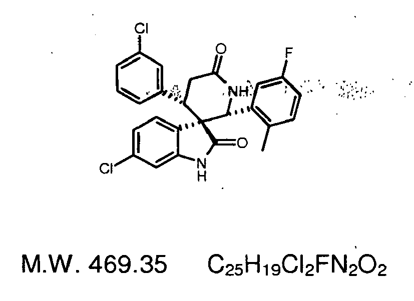

Separation of the two enantiomers (35 mg) from racemic (2'R, 3R, 4'S)-6-chloro-4'-(3-chlorophenyl)-2'-(5-fluoro-2-methylphenyl) spiro[3H-indole-3,3'-piperidine]-2,6'(1H)-dione prepared in example 36b was conducted by chiral SFC to provide chiral (2'R, 3R, 4'S)-6-chloro-4'-(3-chlorophenyl)-2'-(5-fluoro-2-methylphenyl) spiro[3H-indole-3,3'-piperidine]-2,6'(1H)-dione as a white solid (Yield: 10.7 mg, 30 %) and chiral (2'S, 3S, 4'R)-6-chloro-4'-(3-chlorophenyl)-2'-(5-fluoro-2-methylphenyl) spiro[3H-indole-3,3'-piperidine]-2,6'(1H)-dione as a white solid (Yield: 11 mg, 31%). ¹H NMR (300 MHz, DMSO-d6): δ = 2.33 (s, 3 H), 2.72 (dd, J = 6.6, 18.2 Hz, 1 H), 3.03 (dd, J = 12.3, 18.2 Hz, 1 H), 4.02 (dd, J = 6.6, 12.3 Hz, 1 H), 5.44 (d, J= 1.5 Hz, 1 H), 6.36 (dd, J = 3, 11.4 Hz, 1 H), 6.47 (d, J = 2 Hz, 1 H), 6.81-6.89 (m, 2 H), 7.11 (dd, J = 8.1, 15 Hz, 1 H), 7.15 (br t, J = 7 Hz, 1 H), 7.20 (dd, J = 2, 8.1 Hz, 1 H), 7.62 (d, J = 8.1 Hz, 1 H), 8.09 (s, 1 H), 10.23 (s, 1 H) ppm.

### Example 37

### Preparation of racemic (2'R, 3R, 4'S)-6-chloro-4'-(3-chlorophenyl)-1'-[(cyclopropylamino)- carbonyl-methyl]-2'-(2-methylphenyl) spiro[3H-indole-3,3'-piperidine]-2,6'(1H)-dione

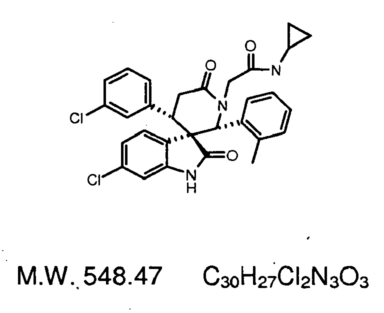

In a manner similar to the method described in example 34b, racemic (2'R, 3R, 4'S)-6-chloro-4'-(3-chlorophenyl)-1'-[(fluorocarbonyl)-methyl]-2'-(2-methylphenyl) spiro[3H-indole-3,3'-piperidine]-2,6'(1H)-dione (0.12 g, 0.23 mmol) prepard in example 34a was reacted with cyclopropylamine (0.1 g, 1.8 mmol) (Aldrich), N-methylmorpholine (48 mg, 0.47 mmol) and 4-dimethylaminopyridine (2 mg, 0.017 mmol) in tetrahydrofuran to give racemic (2'R, 3R, 4'S)-6-chloro-4'-(3-chlorophenyl)-1'-[(cyclopropylamino)-carbonyl-methyl]-2'-(2-methylphenyl) spiro[3H-indole-3,3'-piperidine]-2,6'(1H)-dione as an off-white solid (Yield 0.039 g, 30%).

HRMS(ES⁺) *m*/*z* Calcd for C₃₀H₂₇Cl₂N₃O₃ [(M+H)⁺]: 548.1502. Found: 548.1501.

### Example 38

### Preparation of racemic (2'R, 3R, 4'S)-6-chloro-[[2-[6-chloro-4'-(3-chlorophenyl)-2,3-dihydro-2'-(2-methylphenyl)-2,6-dioxospiro[3H-indole-3,3'-piperidin]-1-yl]-1-oxoethyl]-amino]-piperidine carboxylic acid tert-butyl ester

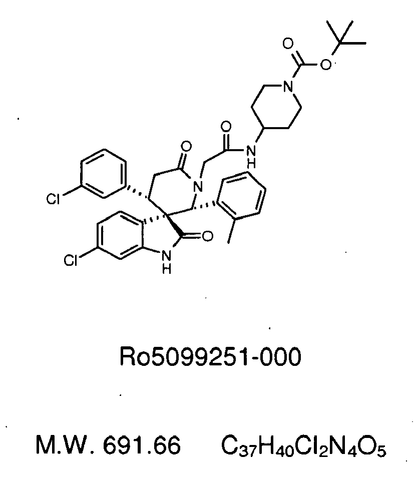

In a manner similar to the method described in example 34b, racemic (2'R, 3R, 4'S)-6-chloro-4'-(3-chlorophenyl)-1'-[(fluorocarbonyl)-methyl]-2'-(2-methylphenyl) spiro[3H-indole-3,3'-piperidine]-2,6'(1H)-dione (0.12 g, 0.23 mmol) prepard in example 34a was reacted with 4-amino-piperidine-1-carboxylic acid *tert*-butyl ester (0.1 g, 0.5 mmol) (Aldrich), N-methylmorpholine (48 mg, 0.47 mmol) and 4-dimethylaminopyridine (2 mg, 0.017 mmol) in tetrahydrofuran to give racemic (2'R, 3R, 4'S)-6-chloro-[[2-[6-chloro-4'-(3-chlorophenyl)-2,3-dihydro-2'-(2-methylphenyl)-2,6-dioxospiro[3H-indole-3,3'-piperidin]-1-yl]-1-oxoethyl]-amino]-piperidine carboxylic acid *tert*-butyl ester as an off-white solid (Yield 0.036 g, 22%).

HRMS(ES⁺) *m*/*z* Calcd for C₃₇H₄₀Cl₂N₄O₅ [(M+H)⁺]: 691.2449. Found: 691.2441.

### Example 39

### Preparation of racemic (2'R, 3R, 4'S)-6-chloro-4'-(3-chlorophenyl)-2'-(5-fluoro-2-methylphenyl)-6'-thioxo spiro[3H-indole-3,3'-piperidine]-2(1H)-one

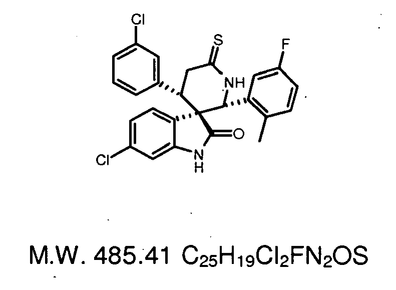

In a manner similar to the method described in example 30, racemic (2'R, 3R, 4'S)-6-chloro-4'-(3-chlorophenyl)-2'-(5-fluoro-2-methylphenyl) spiro[3H-indole-3,3'-piperidine]-2,6'(1H)-dione (0.1 g, 0.21 mmol) prepared in example 36b was reacted with 2,4-bis-(4-methoxyphenyl)-1,3-dithia-2,4-diphosphetane 2,4-disulfide (0.2 g, 0.49 mmol) in toluene to give racemic (2'R, 3R, 4'S)-6-chloro-4'-(3-chlorophenyl)-2'-(5-fluoro-2-methylphenyl)-6'-thioxo spiro[3H-indole-3,3'-piperidine]-2(1H)-one as a white solid (Yield 0.095 g, 92%).

HRMS(ES⁺) *m*/*z* Calcd for C₂₅H₁₉Cl₂FN₂OS +H [(M+H)⁺]: 485.0652. Found: 485.0648.

### Example 40

### Preparation of racemic (2'R, 3R, 4'S)-[6-chloro-4'-(3-chlorophenyl)-2,3-dihydro-2'-[2-(trifluoromethyl)phenyl)]-2-oxospiro[3H-indole-3,3'-piperidin]-6-ylene]-hydrazine carboxylic acid ethyl ester

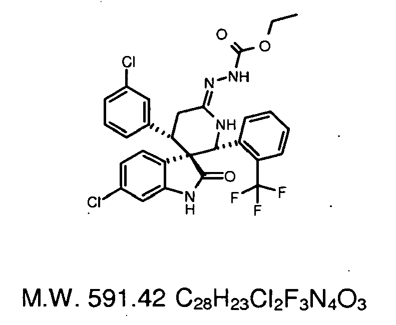

To a solution of racemic (2'R, 3R, 4'S)-6-chloro-4'-(3-chlorophenyl)-6'-thioxo-2'-[2-(trifluoromethyl)-phenyl] spiro[3H-indole-3,3'-piperidine]-2(1H)-one (0.035 g, 0.067 mmol) prepard in example 33 in tetrahydrofuran (30 mL) was added ethyl carbazate (0.019 g, 0.134 mmol) (Aldrich) and mercuric acetate (0.042 g, 0.134 mmol). After the reaction mixture was stirred at room temperature for 2 h, the reaction mixture was filtered through a short pad of celite. The filtrate was concentrated and the residue was purified by chromatography (EtOAc) to give racemic (2'R, 3R, 4'S)-[6-chloro-4'-(3-chlorophenyl)-2,3-dihydro-2'-[2-(trifluoromethyl)-phenyl)]-2-oxospiro[3H-indole-3,3'-piperidin]-6-ylene]-hydrazinecarboxylic acid ethyl ester as a white solid (Yield: 0.036 g, 91%).

HRMS(ES⁺) *m*/*z* Calcd for C₂₈H₂₃Cl₂F₃N₄O₃ + H [(M+H)⁺]: 591.1172. Found: 591.1168

### Example 41a

### Preparation of intermediate 1-(2, 4-difluorophenyl)-3-trimethylsilyoxy-2-aza-1,3-butadiene

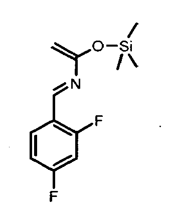

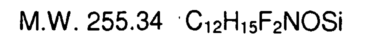

In a manner similar to the method described in example 1b, 2,4-difluorobenzaldehyde (1.4 g, 10 mmol) (Aldrich) was used as the starting material in place of 3-chloroben-zaldehyde to react with 1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(2,4-difluorophenyl)-3-trimethylsilyoxy-2-aza-1,3-butadiene as a yellow gum and used for the next step without further purification.

### Example 41b

### Preparation of racemic (2'R, 3R, 4'S)-6-chloro-4'-(3-chlorophenyl)-2'-(2,4-difluorophenyl) spiro[3H-indole-3,3'-piperidine]-2,6'(1H)-dione

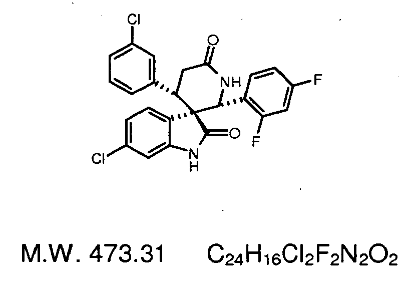

To a solution of 1-(2,4-difluorophenyl)-3-trimethylsilyoxy-2-aza-1,3-butadiene prepared in example 41a (2.4 g, 9.40 mmol) in toluene (30 mL) was added E/Z-6-chloro-3-(3-chlorobenzylidene)-2-oxo-2,3-dihydro-indole-1-carboxylic acid *tert-*butyl ester prepared in Example 24a (0.3 g, 0.77 mmol). The reaction mixture was stirred under nitrogen in a sealed tube at 140 °C for 0.5 h. After the solution was cooled to room temperature, methanol (10 mL) was added. The reaction mixture was filtered through a short pad of celite gel and washed with ethyl acetate. The filtrate was concentrated. The residue was dissolved in dichloromethane (20 mL) and trifluoroactic acid (15 mL) was added. After the reaction mixture was stirred at room temperature for 1 h, the mixture was concentrated. The residue was partitioned between saturated NaHCO₃ solution and ethyl acetate. The aqueous layer was extracted with ethyl acetate. The combined organic layer was dried over Na₂SO₄ and concentrated. The residue was purified by chromatography (EtOAc:hexanes =2:1) to give racemic (2'R, 3R, 4'S)-6-chloro-4'-(3-chlorophenyl)-2'-(2,4-difluorophenyl) spiro[3H-indole-3,3'-piperidine]-2,6'(1H)-dione as an off-white solid (Yield 0.23 g, 63.9%).

HRMS(ES⁺) *m*/*z* Calcd for C₂₄H₁₆Cl₂F₂N₂O₂ + H [(M+H)⁺]: 473.0630. Found: 473.0630.

### Example 42a

### Preparation of intermediate 1-(5-fluoro-2-methoxyphenyl)-3-trimethylsilyoxy-2-aza-1,3-butadiene

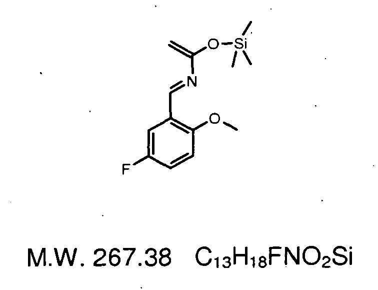

In a manner similar to the method described in example 1b, 5-fluoro-2-methoxybenzaldehyde (1.5 g, 10 mmol) (Aldrich) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(5-fluoro-2-methoxyphenyl)-3-trimethylsilyoxy-2-aza-1,3-butadiene as a yellow gum and used for the next step without further purification.

### Example 42b

### Preparation of racemic (2'R, 3R, 4'S)-6-chloro-4'-(3-chlorophenyl)-2'-(5-fluoro-2-methoxyphenyl) spiro[3H-indole-3,3'-piperidine]-2,6'(1H)-dione

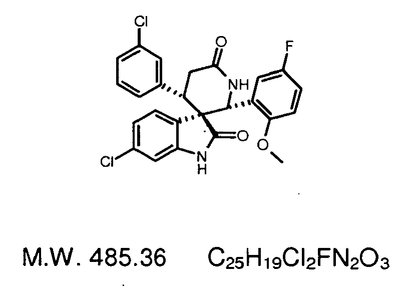

In a manner similar to the method described in example 41b, E/Z-6-chloro-3'-(3-chloro-benzylidene)-2-oxo-2,3-dihydro-indole-1-carboxylic acid *tert*-butyl ester prepared in example 24a (0.3 g, 0.77 mmol) was reacted with 1-(5-fluoro-2-methoxyphenyl)-3-trimethylsilyoxy-2-aza-1,3-butadiene (2.4 g, 9.0 mmol) prepared in example 42a in toluene and then trifluoroacetic acid (15 mL) in dichloromethane to give racemic (2'R, 3R, 4'S)-6-chloro-4'-(3-chlorophenyl)-2'-(5-fluoro-2-methoxyphenyl) spiro[3H-indole-3,3'-piperidine]-2,6'(1H)-dione (Yield 0.18 g, 49%).

HRMS(ES⁺) *m*/*z* Calcd for C₂₅H₁₉Cl₂FN₂O₃ + H [(M+H)⁺]: 485.0830. Found: 485.0827.

### Example 43a

### Preparation of intermediate 1-(1-naphthalenyl)-3-trimethylsilyoxy-2-aza-1,3-butadiene

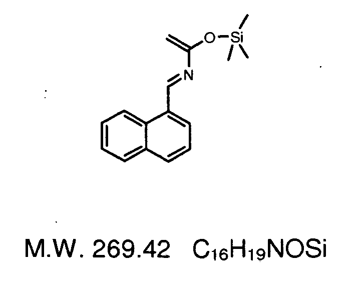

In a manner similar to the method described in example 1b, naphthalene-1-carbaldehyde (1.6 g, 10 mmol) (Lancaster) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(1-naphthalenyl)-3-trimethylsilyoxy-2-aza-1,3-butadiene as a yellow gum and used for the next step without further purification.

### Example 43b

### Preparation of racemic (2'R, 3R, 4'S)-6-chloro-4'-(3-chlorophenyl)-2'-(1-naphthalenyl) spiro[3H-indole-3,3'-piperidine]-2,6'(1H)-dione

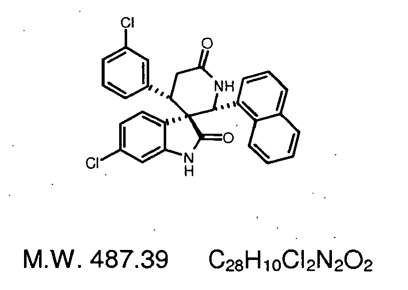

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.3 g, 0.77 mmol) was reacted with 1-(1-naphthalenyl)-3-trimethylsilyoxy-2-aza-1,3-butadiene (2.7 g, 10.0 mmol) prepared in example 43a in toluene and then trifluroacetic acid (15 mL) in dichloromethane to give racemic (2'R, 3R, 4'S)-6-chloro-4'-(3-chlorophenyl)-2'-(1-naphthalenyl) spiro[3H-indole-3,3'-piperidine]-2,6'(1H)-dione (Yield 0.21 g, 57%).

HRMS(ES⁺) *m*/*z* Calcd for C₂₈H₁₀Cl₂N₂O₂ + H [(M+H)⁺]: 487.0975. Found: 487.0975.

### Example 44a

### Preparation of intermediate 1-(3-pyridinyl)-3-trimethylsilyoxy-2-aza-1,3-butadiene

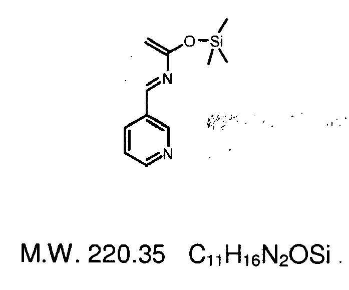

In a manner similar to the method described in example 1b, pyridine-3-carbaldehyde (1.1 g, 10 mmol) (Aldrich) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(3-pyridinyl)-3-trimethylsilyoxy-2-aza-1,3-butadiene as a yellow gum and used for the next step without further purification.

### Example 44b

### Preparation of racemic (2'R, 3R, 4'S)-6-chloro-4'-(3-chlorophenyl)-2'-(3-pyridinyl) spiro[3H-indole-3,3'-piperidine]-2,6'(1H)-dione

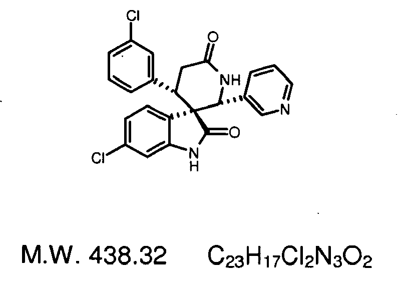

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.3 g, 0.77 mmol) was reacted with 1-(3-pyridinyl)-3-trimethylsilyoxy-2-aza-1,3-butadiene (2.1 g, 9.5 mmol) prepared in example 44a in toluene and then trifluoroacetic acid (10 mL) in dichloromethane to give racemic (2'R, 3R, 4'S)-6-chloro-4'-(3-chlorophenyl)-2'-(3-pyridinyl) spiro[3H-indole-3,3'-piperidine]-2,6'(1H)-dione (0.21 g, 62%).

HRMS(ES⁺) *m*/*z* Calcd for C₂₃H₁₇Cl₂N₃O₂ + H [(M+H)⁺]: 438.0771. Found: 438.0771.

### Example 45a

### Preparation of intermediate 1-(2,3-difluoro-6-methoxyphenyl)-3-trimethylsilyoxy-2-aza-1,3-butadiene

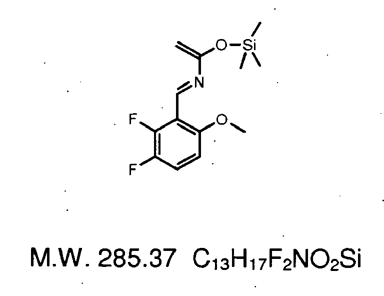

In a manner similar to the method described in example 1b, 2,3-difluoro-6-methoxy-benzaldehyde (1.8 g, 10 mmol) (Apollo) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(2,3-difluoro-6-methoxyphenyl)-3-trimethylsilyoxy-2-aza-1,3-butadiene as an orange gum and used for the next step without further purification.

### Example 45b

### Preparation of racemic (2'R, 3R, 4'S)-6-chloro-4'-(3-chlorophenyl)-2'-(2, 3-difluoro-6-methoxyphenyl) spiro[3H-indole-3,3'-piperidine]-2,6'(1H)-dione

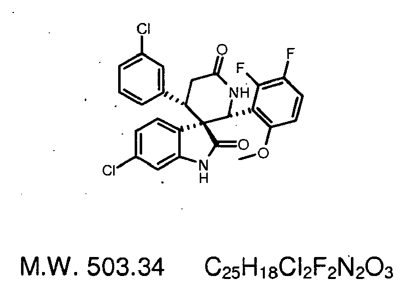

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.3 g, 0.77 mmol) was reacted with 1-(2, 3-difluoro-6-methoxyphenyl)-3-trimethylsilyoxy-2-aza-1,3-butadiene (2.5 g, 8.8 mmol) prepared in example 45a in toluene and then trifluoroacetic acid (15 mL) in dichloromethane to give racemic (2'R, 3R, 4'S)-6-chloro-4'-(3-chlorophenyl)-2'-(2,3-difluoro-6-methoxyphenyl) spiro[3H-indole-3,3'-piperidine]-2,6'(1H)-dione (0.22 g, 56%).

HRMS(ES⁺) *m*/*z* Calcd for C₂₅H₁₈Cl₂F₂N₂O₃ + H [(M+H)⁺]: 503.0736. Found: 503.0735.

### Example 46a

### Preparation of intermediate 1-(3, 4-difluorophenyl)-3-trimethylsilyoxy-2-aza-1,3-butadiene

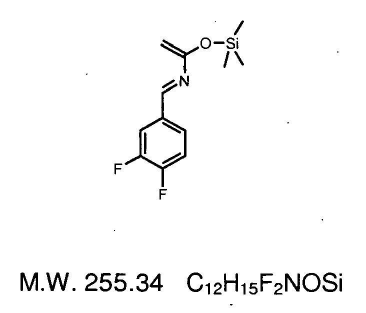

In a manner similar to the method described in example 1b, 3,4-difluorobenzaldehyde (1.4 g, 10 mmol) (Aldrich) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(3,4-difluorophenyl)-3-trimethylsilyoxy-2-aza-1,3-butadiene as an orange gum and used for the next step without further purification.

### Example 46b

### Preparation of racemic (2'R, 3R, 4'S)-6-chloro-4'-(3-chlorophenyl)-2'-(3, 4-difluorophenyl) spiro[3H-indole-3,3'-piperidine]-2,6'(1H)-dione

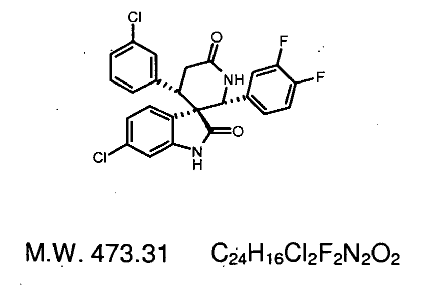

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.3 g, 0.77 mmol) was reacted with 1-(3,4-difluorophenyl)-3-trimethylsilyoxy-2-aza-1,3-butadiene (2.1 g, 8.2 mmol) prepared in example 46a in toluene and then trifluoroacetic acid (10 mL) in dichloromethane to give racemic (2'R, 3R, 4'S)-6-chloro-4'-(3-chlorophenyl)-2'-(3, 4-difluorophenyl) spiro[3H-indole-3,3'-piperidine]-2,6'(1H)-dione (0.4 g, 100% ).

HRMS(ES⁺) *m*/*z* Calcd for C₂₄H₁₆Cl₂F₂N₂O₂ + H [(M+H)⁺]: 473.0630. Found: 473.0631.

### Example 47a

### Preparation of intermediate 1'-(1-cyclohexenyl)-3-trimethylsilyoxy-2-aza-1,3-butadiene

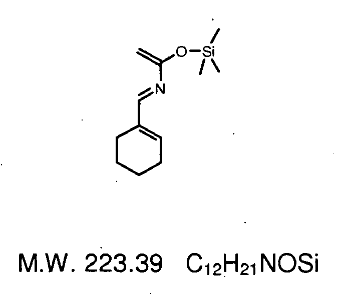

In a manner similar to the method described in example 1b, 1-cyclohexene-1-carboxaldehyde (1.1 g, 10 mmol) (Aldrich) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(1-cyclohexenyl)-3-trimethylsilyoxy-2-aza-1,3-butadiene as an off-white gum and used for the next step without further purification.

### Example 47b

### Preparation of racemic (2'R, 3R, 4'S)-6-chloro-4'-(3-chlorophenyl)-2'-(1-cyclohexenyl) spiro[3H-indole-3,3'-piperidine]-2,6'(1H)-dione

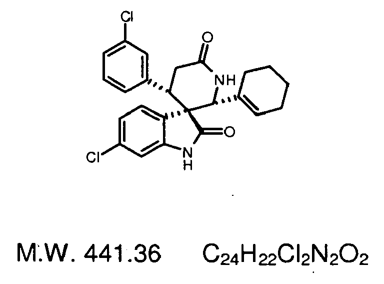

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.26 g, 0.67 mmol) was reacted with 1-(1-cyclohexenyl)-3-trimethylsilyoxy-2-aza-1,3-butadiene (2.1 g, 9.42 mmol) prepared in example 47a in toluene and then trifluoroacetic acid (10 mL) in dichloromethane to give racemic (2'R, 3R, 4'S)-6-chloro-4'-(3-chlorophenyl)-2'-(1-cyclohexenyl) spiro[3H-indole-3,3'-piperidine]-2,6'(1H)-dione as an off-white solid (0.14 g, 48% ).

HRMS(ES⁺) *m*/*z* Calcd for C₂₄H₂₂Cl₂N₂O₂ + H [(M+H)⁺]: 441.1131. Found: 441.1131.

### Example 48

### Preparation of racemic (2'R, 3R, 4'S)-6-chloro-4'-(3-chlorophenyl)-2'-(3, 4-difluorophenyl)- 6'-thioxo spiro[3H-indole-3,3'-piperidine]-2(1H)-one

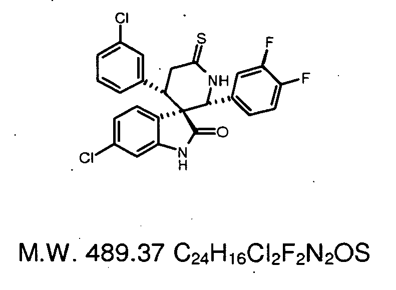

In a manner similar to the method described in example 30, racemic (2'R, 3R, 4'S)-6-chloro-4'-(3-chlorophenyl)-2'-(3, 4-difluorophenyl) spiro[3H-indole-3,3'-piperidine]-2,6'(1H)-dione (0.3 g, 0.63 mmol) prepared in example 46b was reacted with 2,4-bis-(4-methoxyphenyl)-1,3-dithia-2,4-diphosphetane 2,4-disulfide (0.32 g, 0.77 mmol) in toluene to give racemic (2'R, 3R, 4'S)-6-chloro-4'-(3-chlorophenyl)-2'-(3, 4-difluorophenyl)-6'-thioxo spiro[3H-indole-3,3'-piperidine]-2(1H)-one (Yield 0.29 g, 94%).

HRMS(ES⁺) *m*/*z* Calcd for C₂₄H₁₆Cl₂F₂N₂OS +H [(M+H)⁺]: 489.0401. Found: 489.0402.

### Example 49

### Preparation of racemic (2'R, 3R, 4'S)-[6-chloro-4'-(3-chlorophenyl)-2'-(3, 4-difluorophenyl)-2,3-dihydro-2-oxospiro[3H-indole-3,3'-piperidin]-6-ylene] hydrazine carboxylic acid ethyl ester

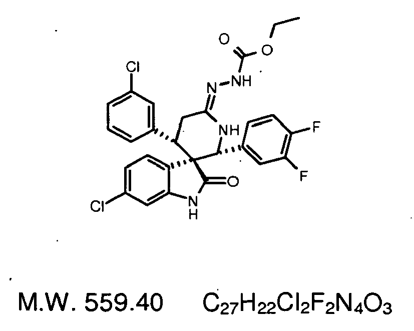

In a manner similar to the method described in example 40, racemic (2'R, 3R, 4'S)-6-chloro-4'-(3-chlorophenyl)-2'-(3, 4-difluorophenyl)- 6'-thioxo spiro[3H-indole-3,3'-piperidine]-2(1H)-one (0.24 g, 0.49 mmol) prepard in example 48 was reacted with ethyl carbazate (0.1 g, 0.99 mmol), mercuric acetate (0.24 g, 0.76 mmol) and triethylamine (0.1 g, 0.99 mmol) in tetrahydrofuran (20 mL) to give racemic (2'R, 3R, 4'S)-[6-chloro-4'-(3-chlorophenyl)-2'-(3, 4-difluorophenyl)-2,3-dihydro-2-oxospiro[3H-indole-3,3'-piperidin]-6-ylene] hydrazine carboxylic acid ethyl ester as a white solid (Yield 0.21 g, 77.8 %).

HRMS(ES⁺) *m*/*z* Calcd for C₂₇H₂₂Cl₂F₂N₄O₃ + H [(M+H)⁺]: 559.1110. Found: 559.1109.

### Example 50a

### Preparation of intermediate 1-(1,3-benzodioxol-4-yl)-3-trimethylsilyoxy-2-aza-1,3-butadiene

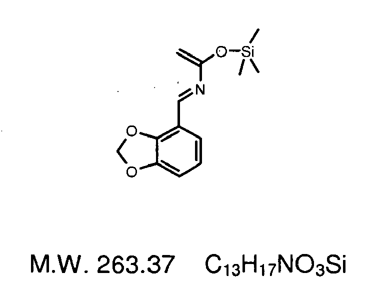

In a manner similar to the method described in example 1b, 2,3-(methylenedioxy)- benzaldehyde (1.5 g, 10 mmol) (Aldrich) was used as the starting material in place of 3-chloro-benzaldehyde to react with 1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(1,3-benzodioxol-4-yl)-3-trimethylsilyoxy-2-aza-1,3-butadiene as a yellow oil and used for the next step without further purification.

### Example 50b

### Preparation of racemic (2'R, 3R, 4'S)-2'-(1,3-benzodioxol-4-yl)-6-chloro-4'-(3-chlorophenyl) spiro[3H-indole-3,3'-piperidine]-2,6'(1H)-dione

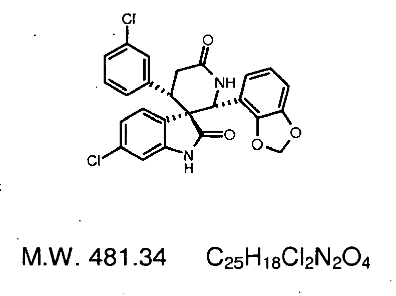

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.25 g, 0.67 mmol) was reacted with 1-(1,3-benzodioxol-4-yl)-3-trimethylsilyoxy-2-aza-1,3-butadiene (2.1 g, 7.98 mmol) prepared in example 50a in toluene and then trifluoroacetic acid (10 mL) in dichloromethane to give racemic (2'R, 3R, 4'S)-2'-(1,3-benzodioxol-4-yl)-6-chloro-4'-(3-chlorophenyl) spiro[3H-indole-3,3'-piperidine]-2,6'(1H)-dione as an off-white solid (0.20 g, 62%).

HRMS(ES⁺) *m*/*z* Calcd for C₂₅H₁₈Cl₂N₂O₄ + H [(M+H)⁺]: 481.0717. Found: 481.0717.

### Example 51

### Preparation of racemic (2'R, 3R, 4'S)-[6-chloro-4'-(3-chlorophenyl)-2'-(5-fluoro-2-methylphenyl)-2,3-dihydro-2-oxospiro[3H-indole-3,3'-piperidin]-6-ylene] hydrazine carboxylic acid ethyl ester

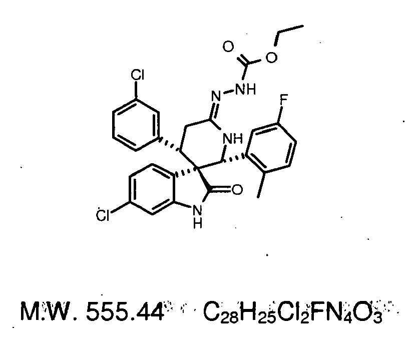

In a manner similar to the method described in example 40, (2'R, 3R, 4'S)-6-chloro-4'-(3-chlorophenyl)-2'-(5-fluoro-2-methylphenyl)-6'-thioxo spiro[3H-indole-3,3'-piperidine]-2(1H)-one (0.17 g, 0.35 mmol) prepared in example 39 was reacted with ethyl carbazate (0.15 g, 1.49 mmol), mercuric acetate (0.26 g, 0.82 mmol) and triethylamine (0.17 g, 1.69 mmol) in tetrahydrofuran (20 mL) to give racemic (2'R, 3R, 4'S)-[6-chloro-4'-(3-chlorophenyl)-2'-(5-fluoro-2-methylphenyl)-2,3-dihydro-2-oxospiro[3H-indole-3,3'-piperidin]-6-ylene] hydrazine carboxylic acid ethyl ester as a white solid (Yield 0.14 g, 73.7%).

### Example 52a

### Preparation of intermediate 2,3-difluoro-6-methyl-benzaldehyde

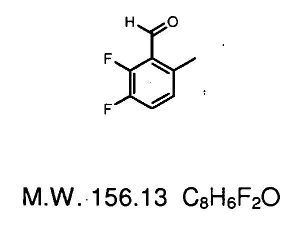

To a solution of 1,2-difluoro-4-methyl-benzene (5.0 g, 39 mmol) in tetrahydrofuran (200 mL) at -78 °C was added lithium diisopropyl amine (24 mL, 1.8 M in THF, 43 mmol) d during a period of 15 mins. The mixture was stirred at -78 °C for another 20 min. Then N,N-dimethyl-formamide (3.6 mL, 47 mmol) was added in one portion. The mixture was stirred at -78 °C for 10 min, then quenched with acetic acid (9.4 g, 1.56 mmol) and followed by the addition of water (37.6 mL). The mixture was partitioned between ethyl acetate and water. The organic layer was separated, concentrated. The residue was purified by chromatography (EtOAc:hexanes =1:1) to give 2,3-difluoro-6-methyl-benzaldehyde as colorless oil (Yield: 3.5 g, 57.5%).

### Example 52b

### Preparation of intermediate 1-(2, 3-difluoro-6-methylphenyl)-3-trimethylsilyoxy-2-aza-1,3-butadiene

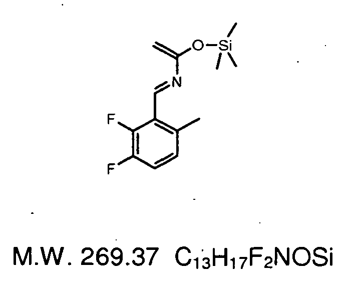

In a manner similar to the method described in example 1b, 2,3-difluoro-6-methylbenzaldehyde (1.56 g, 10 mmol) prepared in example 52a was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(2, 3-difluoro-6-methylphenyl)-3-trimethylsilyoxy-2-aza-1,3-butadiene as an orange gum and used for the next step without further purification.

### Example 52c

### Preparation of racemic (2'R, 3R, 4'S)-6-chloro-4'-(3-chlorophenyl)-2'-(2, 3-difluoro-6-methylphenyl) spiro[3H-indole-3,3'-piperidine]-2,6'(1H)-dione

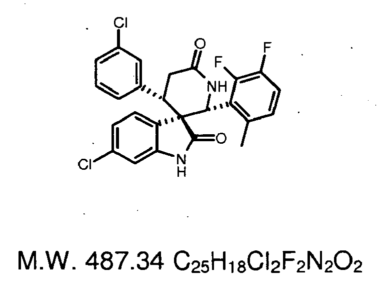

In a manner similar to the method described in example 41b, E/Z-6-chloro-3-(3-chlorobenzylidene)-2-oxo-2,3-dihydro-indole-1-carboxylic acid *tert*-butyl ester prepared in example 24a (0.4 g, 1.03 mmol) was reacted with 1-(2, 3-difluoro-6-methylphenyl)-3-trimethylsilyoxy-2-aza-1,3-butadiene (2.7 g, 10.0 mmol) prepared in example 52b in toluene and then trifluoroacetic acid (10 mL) in dichloromethane to give racemic (2'R, 3R, 4'S)-6-chloro-4'-(3-chlorophenyl)-2'-(2, 3-difluoro-6-methylphenyl) spiro[3H-indole-3,3'-piperidine]-2,6'(1H)-dione (Yield: 0.41 g, 83.7% ).

HRMS(ES⁺) *m*/*z* Calcd for C₂₅H₁₈Cl₂F₂N₂O₂ + H [(M+H)⁺]: 487.0786 Found: 487.0780.

### Example 53a

### Preparation of intermediate E/Z-6-chloro-3-(3-chloro-benzylidene)-5-fluoro-1,3-dihydro-indol-2-one

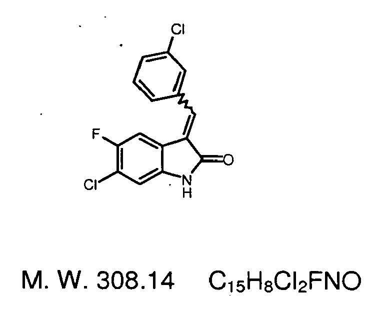

In a manner similar to the method described in example 1a, 6-chloro-5-fluoro-1,3-dihydro-indol-2-one (0.25 g, 1.35 mmol, prepared in procedure described in EP153818) was reacted with 3-chloro-benzaldehyde (0.34 g, 2.44 mmol) and pyrolidine (0.19 g, 2.68 mmol) in methanol to give a mixture of E-and Z-6-chloro-3-(3-chloro-benzylidene)-5-fluoro-1,3-dihydro-indol-2-one as a yellow solid.

### Example 53b

### Preparation of intermediate E/Z-6-chloro-3-(3-chloro-benzylidene)-5-fluoro-2-oxo-2,3-dihydro-indole-1-carboxylic acid tert-butyl ester

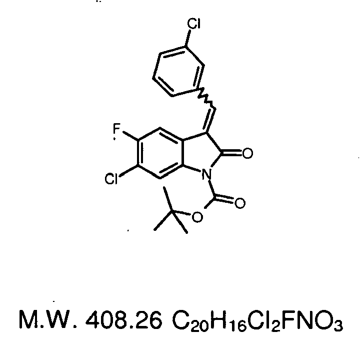

In a manner similar to the method described in example 24a, E/Z-6-chloro-3-(3-chloro-benzylidene)-5-fluoro-1,3-dihydro-indol-2-one (0.45 g, 1.46 mmol) was reacted with di-*tert*-butyl-dicarbonate (0.4 g, 1.83 mmol) (Aldrich), triethyl amine (0.5 g, 4.95 mmol) and 4-dimethylaminopyridine (5 mg) in dichloromethane (30 mL) to give E/Z-6-chloro-3-(3-chloro-benzylidene)-5-fluoro-2-oxo-2,3-dihydro-indole-1-carboxylic acid *tert*-butyl ester as a yellow solid (Yield: 0.6 g, 100% ).

### Example 53c

### Preparation of racemic (2'R, 3R, 4'S)-6-chloro-4'-(3-chlorophenyl)-5-fluoro-2'-(5-fluoro-2-methylphenyl) spiro[3H-indole-3,3'-piperidine]-2,6'(1H)-dione

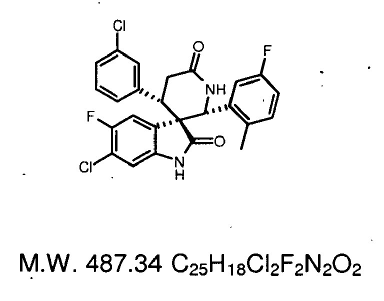

In a manner similar to the method described in example 41b, E/Z-6-chloro-3-(3-chlorobenzylidene)-5-fluoro-2-oxo-2,3-dihydro-indole-1-carboxylic acid *tert*-butyl ester prepared i example 53b (0.4 g, 0.98 mmol) was reacted with 1-(5-fluoro-2-methylphenyl)-3-trimethylsilyoxy-2-aza-1,3-butadiene (2.1 g, 8.37 mmol) prepared in example 36a in toluene and then trifluoroacetic acid (10 mL) in dichloromethane to give racemic (2'R, 3R, 4'S)-6-chloro-4'-(3-chlorophenyl)-5-fluoro-2'-(5-fluoro-2-methylphenyl) spiro[3H-indole-3,3' piperidine]-2,6'(1H)-dione (Yield: 0.35 g, 72.9% ).

HRMS(ES⁺) *m*/*z* Calcd for C₂₅H₁₈Cl₂F₂N₂O₂ + H [(M+H)⁺]: 487.0786. Found: 487.0779.

### Example 54

### Preparation of racemic (2'R, 3R, 4'S)-6-chloro-4'-(3-chlorophenyl)-2'-(2, 3-difluoro-6-methylphenyl)-6'-thioxo spiro[3H-indole-3,3'-piperidine]-2(1H)-one

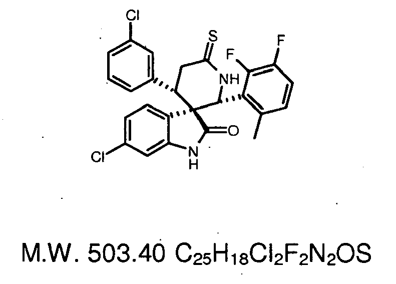

In a manner similar to the method described in example 30, racemic (2'R, 3R, 4'S)-6-chloro-4'-(3-chlorophenyl)-2'-(2, 3-difluoro-6-methylphenyl) spiro[3H-indole-3,3'-piperidine]-2,6'(1H)-dione (0.18 g, 0.37 mmol) prepared in example 52c was reacted with 2,4-bis-(4-methoxyphenyl)-1,3-dithia-2,4-diphosphetane 2,4-disulfide (0.32 g, 0.96 mmol) in toluene to give racemic (2'R, 3R, 4'S)-6-chloro-4'-(3-chlorophenyl)-2'-(2, 3-difluoro-6-methylphenyl)-6'-thioxo spiro[3H-indole-3,3'-piperidine]-2(1H)-one (Yield 0.13 g, 69.8%).

HRMS(ES⁺) *m*/*z* Calcd for C₂₅H₁₈Cl₂F₂N₂OS +H [(M+H)⁺]: 503.0558. Found: 503.0554.

### Example 55a

### Preparation of intermediate E/Z-6-chloro-3-(3-chloro-benzylidene)-1-(2-trimethylsilanyl-ethoxymethyl)-1,3-dihydro-indole-2-one

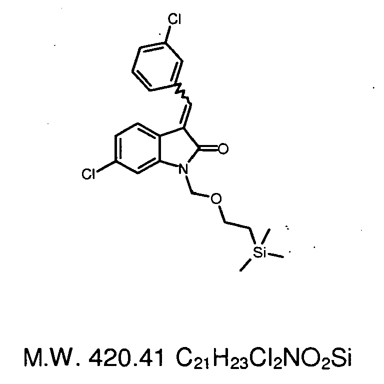

To a solution of E/Z-6-chloro-3-(3-chloro-benzylidene)-1,3-dihydro-indol-2-one prepared in example 4a (2.3 g, 7.9 mmol) in N,N-dimethyl-formamide (20 mL) at 0 °C was added NaH (60% in mineral oil) (0.32 g, 7.9 mmol) (Aldrich), followed by the dropwise addition of 2-(trimethylsilyl)ethoxymethyl chloride (1.32 g, 7.9 mmol) in tetrahydrofuran (20 mL). The reaction mixture was stirred at 0 °C for 0.5 h, then poured into ice-water. The crude was extracted with ethyl acetate twice. The combined organic layer was dried over Na₂SO₄. The solvent was removed and the residue was purified by chromatography (EtOAc:hexanes=1:5) to give E/Z-6-chloro-3-(3-chloro-benzylidene)-1-(2-trimethylsilanyl-ethoxymethyl)-1,3-dihydro-indole-2-one as yellow oil (Yield 3.0 g, 90%).

### Example 55b

### Preparation of intermediate racemic (2'R, 3R, 4'S)-6-chloro-4'-(3-chlorophenyl)-2'-(5-fluoro-2-methylphenyl)-2,3-dihydro-2,6'-dioxo spiro[indole-3,3'-piperidine]-1-methoxyethyl trimethylsilane

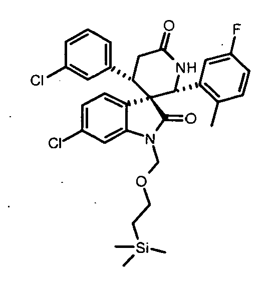

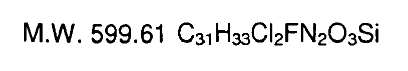

To a solution of 1-(5-fluoro-2-methylphenyl)-3-trimethylsilyoxy-2-aza-1,3-butadiene prepared in example 36a in toluene (50 mL) was added E/Z-6-chloro-3-(3-chloro-benzylidene)-1-(2-trimethylsilanyl-ethoxymethyl)-1,3-dihydro-indole-2-one prepared in example 55a (3.0 g, 7.14 mmol). The reaction mixture was stirred under nitrogen in a sealed tube at 148 °C for 40 min. After the solution was cooled to room temperature, methanol (50 mL) was added, and then the mixture was concentrated. The residue was purified by chromatography (EtOAc:Hexane =2:1) to give racemic (2'R, 3R, 4'S)-6-chloro-4'-(3-chlorophenyl)-2'-(5-fluoro-2-methylphenyl)-2,3-dihydro-2,6'-dioxo spiro[indole-3,3'-piperidine]-1-methoxyethyl trimethylsilane as an off-white solid (Yield 2.1 g, 49%).

### Example 55c

### Preparation of intermediate racemic (2'R, 3R, 4'S)-6-chloro-4'-(3-chlorophenyl)-2'-(5-fluoro-2-methylphenyl)-2,3-dihydro-1'-[(tert-butoxycarbonyl) methyl]-2,6'-dioxo spiro[indole-3,3'-piperidine]-1-methoxyethyl trimethylsilane

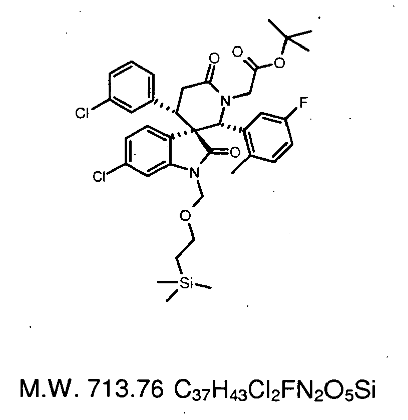

To a solution of racemic (2'R, 3R, 4'S)-6-chloro-4'-(3-chlorophenyl)- 2'-(5-fluoro-2-methylphenyl)-2,3-dihydro-2,6'-dioxo spiro[indole-3,3'-piperidine]-1-methoxyethyl trimethylsilane (1.0 g, 1.67 mmol) prepared in example 55b in N,N-dimethyl-formamide (20 mL) at room temperature was added bromo-acetic acid tert-butyl ester (0.8 g, 4.1 mmol) and cesium carbonate (3.0 g, 9.20 mmol). The reaction mixture was stirred under nitrogen for 4 h, then poured into saturated aqueous NH₄Cl solution. The mixture was extracted with ethyl acetate. The organic layers were combined, dried over Na₂SO₄ and concentrated. The residue was purified by chromatography (EtOAc:Hexanes=1:4) to give racemic (2'R, 3R, 4'S)-6-chloro-4'-(3-chlorophenyl)-2'-(5-fluoro-2-methylphenyl)-2,3-dihydro-1'-[(*tert*-butoxycarbonyl) methyl]-2,6'-dioxo spiro[indole-3,3'-piperidine]-1-methoxyethyl trimethylsilane as a white foam (Yield 0.58 g, 48.7%).

### Example 55d

### Preparation of racemic (2'R, 3R, 4'S) -6-chloro-4'-(3-chlorophenyl)-2'-(5-fluoro-2-methylphenyl)-1'-hydroxycarbonylmethyl spiro[3H-indole-3,3'-piperidine]-2,6'(1H)-dione

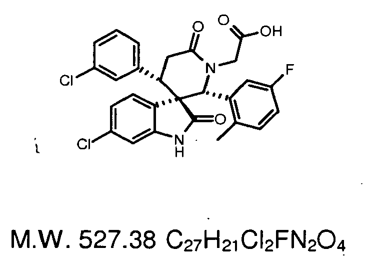

To a solution of racemic (2'R, 3R, 4'S)-6-chloro-4'-(3-chlorophenyl)-2'-(5-fluoro-2-methylphenyl)-2,3-dihydro-1'-[(*tert*-butoxycarbonyl)methyl]-2,6'-dioxo spiro[indole-3,3'-piperidine]-1-methoxyethyl trimethylsilane (0.54 g, 0.76 mmol) prepared in example 55c in dichloromethane (10 mL) was added trifluoroacetic acid (20 mL). The reaction mixture was stirred at room temperature for 18 h. The reaction mixture was concentrated. The residue was redissolved in methanol (10 mL). To the resulting solution was added N,N'-diisopropylethylamine (1 mL, 5.53 mmol) and the crude was refluxed for 1 h. The reaction mixture was concentrated and the residue was partitioned between ethyl acetate and HCl aqueous solution (1 N). The organic layer was separated, dried over MgSO₄ and concentrated. The residue was triturated with ethyl acetate and hexane to give racemic (2'R, 3R, 4'S) -6-chloro-4'-(3-chlorophenyl)-2'-(5-fluoro-2-methylphenyl)-1'-hydroxycarbonylmethyl spiro[3H-indole-3,3'-piperidine]-2,6'(1H)-dione as a white solid (Yield 0.3 g, 75%).

HRMS(ES⁺) *m*/*z* Calcd for C₂₇H₂₁Cl₂FN₂O₄ +H [(M+H)⁺]: 527.0935 Found: 527.0926.

### Example 56a

### Preparation of intermediate racemic (2'R, 3R, 4'S)-6-chloro-4'-(3-chlorophenyl)-2'-(5-fluoro-2-methylphenyl)-2,3-dihydro-1'-methyl-2,6'-dioxo spiro[indole-3,3'-piperidine]-1-methoxyethyl trimethylsilane

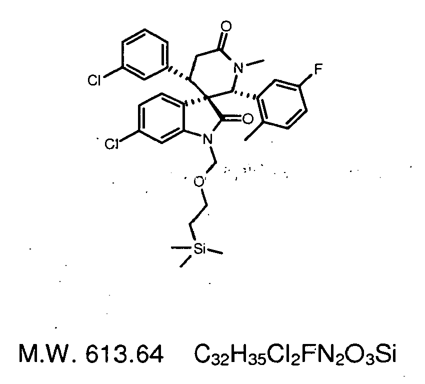

In a manner similar to the method described in example 24c, racemic (2'R, 3R, 4'S)-6-chloro-4'-(3-chlorophenyl)- 2'-(5-fluoro-2-methylphenyl)-2,3-dihydro-2,6'-dioxo spiro[indole-3,3'-piperidine]-1-methoxyethyl trimethylsilane (0.3 g, 0.5 mmol) prepared in example 55b was reacted with LiH (0.17 g, 21.4 mmol) (Aldrich) and iodomethane (4 g, 28.2 mmol) in N,N-dimethyl-formamide (40 mL) to give racemic (2'R, 3R, 4'S)-6-chloro-4'-(3-chlorophenyl)-2'-(5-fluoro-2-methylphenyl)-2,3-dihydro-1'-methyl-2,6'-dioxo spiro[indole-3,3'-piperidine]-1-methoxyethyl trimethylsilane as a white solid (Yield: 0.16 g, 51.6%).

### Example 56b

### Preparation of intermediate racemic (2'R, 3R, 4'S)-6-chloro-4'-(3-chlorophenyl)-2'-(5-fluoro-2-methylphenyl)-2,3-dihydro-1'-methyl-2,6'-dioxo spiro[indole-3,3'-piperidine]-1-ethanol

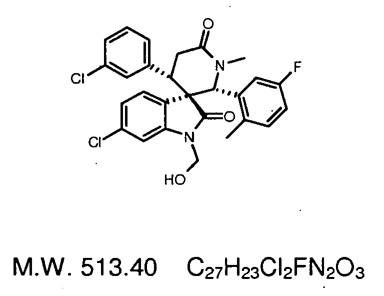

To a solution of racemic (2'R, 3R, 4'S)-6-chloro-4'-(3-chlorophenyl)-2'-(5-fluoro-2-methylphenyl)-2,3-dihydro-1'-methyl-2,6'-dioxo spiro[indole-3,3'-piperidine]-1-methoxyethyl trimethylsilane (0.16 g, 0.26 mmol) prepared in example 56a in dichloromethane (10 mL) was added trifluoroacetic acid (20 mL). The reaction mixture was stirred at room temperature for 3 h. The reaction mixture was concentrated. The residue was neutralized with saturated NaHCO₃ aqueous solution, then extracted with ethyl acetate. The organic layer was separated, dried over MgSO₄ and concentrated. The residue was purified by chromatography (EtOAc:CH₂Cl₂=1:1) to give racemic (2'R, 3R, 4'S)-6-chloro-4'-(3-chlorophenyl)-2'-(5-fluoro-2-methylphenyl)-2,3-dihydro-1'-methyl-2,6'-dioxo spiro[indole-3,3'-piperidine]-1-ethanol (Yield 90 mg, 67.7%).

### Example 56c

### Preparation of racemic (2'R, 3R, 4'S)-6-chloro-4'-(3-chlorophenyl)-2'-(5-fluoro-2-methylphenyl)-1'-methyl spiro[3H-indole-3,3'-piperidine]- 2,6'(1H)-dione

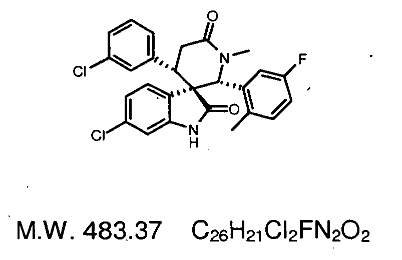

To a solution of racemic (2'R, 3R, 4'S)-6-chloro-4'-(3-chlorophenyl)-2'-(5-fluoro-2-methylphenyl)-2,3-dihydro-1'-methyl-2,6'-dioxo spiro[indole-3,3'-piperidine]-1-ethanol (0.09 g, 0.175 mmol) prepared in example 56b in methanol (10 mL) was added N,N'-diisopropylethylamine (2 mL, 11.1 mmol) and the crude was refluxed for 1 h. The reaction mixture was concentrated and the residue was partitioned between ethyl acetate and water. The organic layer was separated, and concentrated. The residue was purified by chromatography (EtOAc:CH₂Cl₂=1:2) to give racemic (2'R, 3R, 4'S)-6-chloro-4'-(3-chlorophenyl)-2'-(5-fluoro-2-methylphenyl)-1'-methyl spiro[3H-indole-3,3'-piperidine]-2,6'(1H)-dione as a white solid (Yield 0.06 g, 70.6%).

HRMS(ES⁺) *m*/*z* Calcd for C₂₆H₂₁Cl₂FN₂O₂ +H [(M+H)⁺]: 483.1037. Found: 483.1042.

### Example 56d

### Preparation of chiral (2'R, 3R, 4'S)-6-chloro-4'-(3-chlorophenyl)-2'-(5-fluoro-2-methylphenyl)-1'-methyl spiro[3H-indole-3,3'-piperidine]- 2,6'(1H)-dione

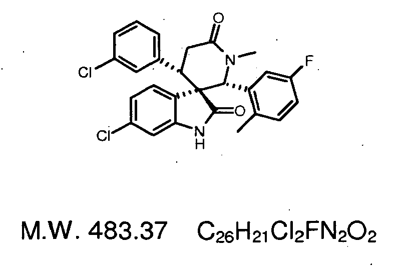

Separation of the two enantiomers from racemic (2'R, 3R, 4'S)-6-chloro-4'-(3-chlorophenyl)-2'-(5-fluoro-2-methylphenyl)-1'-methyl spiro[3H-indole-3,3'-piperidine]- 2,6'(1H)-dione (50 mg) prepared in example 56c was conducted by chiral SFC to provide chiral (2'R, 3R, 4'S)-6-chloro-4'-(3-chlorophenyl)-2'-(5-fluoro-2-methylphenyl)-1'-methyl spiro[3H-indole-3,3'-piperidine]- 2,6'(1H)-dione as a white solid (Yield: 20 mg, 40%) and chiral (2'S, 3S, 4'R)-6-chloro-4'-(3-chlorophenyl)-2'-(5-fluoro-2-methylphenyl)-1'-methyl spiro[3H-indole-3,3'-piperidine]-2,6'(1H)-dione as a white solid (Yield: 20 mg, 40%).

### Example 57a

### reparation of racemic (2'R, 3R, 4'S)-6-chloro-4'-(3-chlorophenyl)-1'-fluorocarbonylmethyl-2'-(5-fluoro-2-methylphenyl) spiro[3H-indole-3,3'-piperidine]-2,6'(1H)-dione

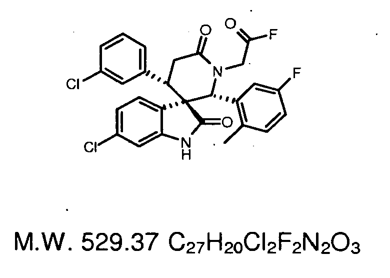

In a manner similar to the method described in example 34a, racemic (2'R, 3R,4'S)-6-chloro-4'-(3-chlorophenyl)-2'-(5-fluoro-2-methylphenyl)-1'-(hydroxycarbonyl-methyl) spiro[3H-indole-3,3'-piperidine]-2,6'(1H)-dione (0.1 g, 0.19 mmol) prepared in example 55d was reacted with cyanuric fluoride (51 mg, 0.38 mmol) (Alfa) and pyridine (45 mg, 0.57 mmol) in dichloromethane to give racemic (2'R, 3R, 4'S)-6-chloro-4'-(3-chlorophenyl)-1'-fluorocarbonylmethyl-2'-(5-fluoro-2-methylphenyl) spiro[3H-indole-3,3'-piperidine]-2,6'(1H)-dione (yield: 0.1 g, 100%).

### Example 57b

### Preparation of racemic (2'R, 3R, 4'S)-6-chloro-4'-(3-chlorophenyl)-2'-(5-fluoro-2-methylphenyl)-1'-[(1-methyl-piperidin-4-yl)aminocarbonyl)-methyl] spiro[3H-indole-3,3'-piperidine]-2,6'(1H)-dione

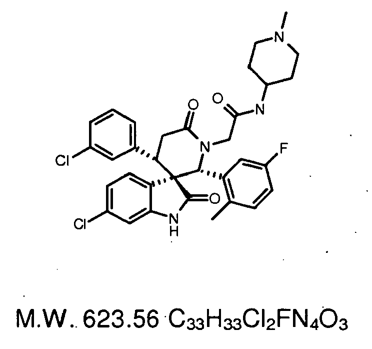

In a manner similar to the method described in example 34b, racemic (2'R, 3R, 4'S)-6-chloro-4'-(3-chlorophenyl)-1'-[(fluorocarbonyl)-methyl]-2'-(5-fluoro-2-methylphenyl) spiro[3H-indole-3,3'-piperidine]-2,6'(1H)-dione (0.1 g, 0.18 mmol) prepard in example 57a was reacted with 1-methyl-piperidin-4-ylamine (0.1 g, 0.88 mmol), N-methylmorpholine (0.1 g, 0.99 mmol) and 4-dimethylaminopyridine (1 mg, 0.008 mmol) in tetrahydrofuran to give racemic (2'R, 3R, 4'S)-6-chloro-4'-(3-chlorophenyl)-2'-(5-fluoro-2-methylphenyl)-1'-[(1-methyl-piperidin-4-yl)aminocarbonyl)-methyl] spiro[3H-indole-3,3'-piperidine]-2,6'(1H)-dione as an off-white solid (Yield 0.041 g, 36.7%).

HRMS(ES⁺) *m*/*z* Calcd for C₃₃H₃₃Cl₂FN₄O₃ +H [(M+H)⁺]: 623.1987. Found: 623.1989.

### Example 57c

### Preparation of chiral (2'R, 3R, 4'S)-6-chloro-4'-(3-chlorophenyl)-2'-(5-fluoro-2-methylphenyl)-1'-[(1-methyl-piperidin-4-yl)aminocarbonyl)-methyl] spiro[3H-indole-3,3'-piperidine]-2,6'(1H)-dione

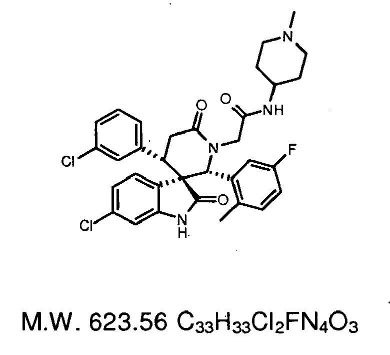

Separation of the two enantiomers from (2'R, 3R, 4'S)-6-chloro-4'-(3-chlorophenyl)-2'-(5-fluoro-2-methylphenyl)-1'-[(1-methyl-piperidin-4-yl)aminocarbonyl)-methyl] spiro[3H-indole-3,3'-piperidine]-2,6'(1H)-dione (30 mg) prepared in example 57b was conducted by chiral SFC to provide chiral (2'R, 3R, 4'S)-6-chloro-4'-(3-chlorophenyl)-2'-(5-fluoro-2-methylphenyl)-1'-[(1-methyl-piperidin-4-yl)aminocarbonyl)-methyl] spiro[3H-indole-3,3'-piperidine]-2,6'(1H)-dione as a white solid (Yield: 10 mg, 17%) and chiral (2'S, 3S, 4'R)-6-chloro-4'-(3-chlorophenyl)-2'-(5-fluoro-2-methylphenyl)-1'-[(1-methyl-piperidin-4-yl)aminocarbonyl)-methyl] spiro[3H-indole-3,3'-piperidine]-2,6'(1H)-dione as a white solid (Yield: 10 mg, 17%).

### Example 58

### Preparation of racemic (2'R, 3R, 4'S)-1'-[1-tert-butoxycarbonyl-piperidin-4-yl)aminocarbonyl-methyl] 6-chloro-4'-(3-chlorophenyl)-2'-(5-fluoro-2-methylphenyl) spiro[3H-indole-3,3'-piperidine]-2,6'(1H)-dione

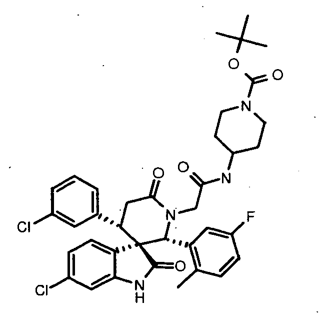

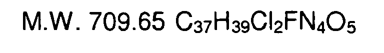

In a manner similar to the method described in example 34b, racemic (2'R, 3R, 4'S)-6-chloro-4'-(3-chlorophenyl)-1'-fluorocarbonylmethyl-2'-(5-fluoro-2-methylphenyl) spiro[3H-indole-3,3'-piperidine]-2,6'(1H)-dione (0.1 g, 0.18 mmol) prepard in example 57a was reacted with 4-amino-piperidine-1-carboxylic acid tert-butyl ester (0.1 g, 0.50 mmol), N-methylmorpholine (0.1 g, 0.99 mmol) and 4-dimethylaminopyridine (2 mg, 0.017 mmol) in tetrahydrofuran to give racemic (2'R, 3R, 4'S)-1'-[1-tert-butoxycarbonyl-piperidin-4-yl)aminocarbonyl-methyl] 6-chloro-4'-(3-chlorophenyl)-2'-(5-fluoro-2-methylphenyl) spiro[3H-indole-3,3'-piperidine]-2,6'(1H)-dione as a white solid (Yield 0.075 g, 58.6%).

HRMS(ES⁺) *m*/*z* Calcd for C₃₇H₃₉Cl₂FN₄O₅ +H [(M+H)⁺]: 709.2355. Found: 709.2354.

### Example 59a

### Preparation of racemic (2'R, 3R, 4'S)-6-chloro-4'-(3-chlorophenyl)-2'-(5-fluoro-2-methylphenyl)-1'-[(piperidin-4-yl)aminocarbonyl-methyl] spiro[3H-indole-3,3'-piperidine]-2,6'(1H)-dione

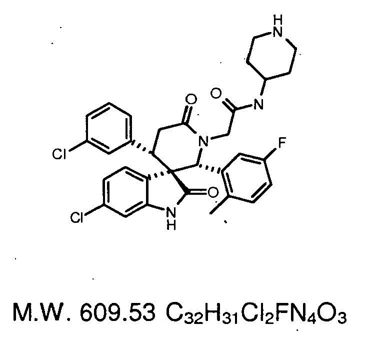

To a solution of racemic (2'R, 3R, 4'S)-1'-[1-tert-butoxycarbonyl-piperidin-4-yl)aminocarbonyl-methyl] 6-chloro-4'-(3-chlorophenyl)-2'-(5-fluoro-2-methylphenyl) spiro[3H-indole-3,3'-piperidine]-2,6'(1H)-dione (0.07 g, 0.099 mmol) prepared in 58 in dichloromethane (20 mL) was added trifluoroacetic acid (5 mL). The reaction mixture was stirred at room temperature for 1 h, then concentrated. The residue was neutralized with saturated NaHCO₃ solution, extracted with ethyl acetate. The organic layer was separated, dried over MgSO₄, concentrated to give racemic (2'R, 3R, 4'S)-6-chloro-4'-(3-chlorophenyl)-2'-(5-fluoro-2-methylphenyl)-1'-[(piperidin-4-yl)aminocarbonyl-methyl] spiro[3H-indole-3,3'-piperidine]-2,6'(1H)-dione as a white solid (Yield 0.06 g, 99%).

HRMS(ES⁺) *m*/*z* Calcd for C₃₂H₃₁Cl₂FN₄O₃ +H [(M+H)⁺]: 609.1830 Found: 609.1820.

### Example 59b

### Preparation of chiral (2'R, 3R, 4'S)-6-chloro-4'-(3-chlorophenyl)-2'-(5-fluoro-2-methylphenyl)-1'-[(piperidin-4-yl)aminocarbonyl-methyl] spiro[3H-indole-3,3'-piperidine]-2,6'(1H)-dione

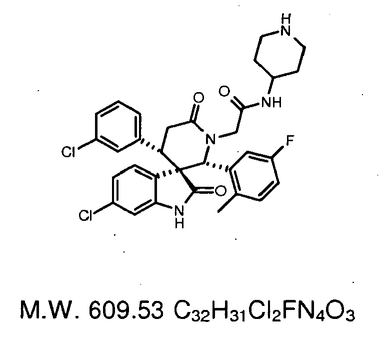

Separation of the two enantiomers from racemic (2'R, 3R, 4'S)-6-chloro-4'-(3-chlorophenyl)-2'-(5-fluoro-2-methylphenyl)-1'-[(piperidin-4-yl)aminocarbonyl-methyl] spiro[3H-indole-3,3'-piperidine]-2,6'(1H)-dione (40 mg) prepared in example 59a was conducted by chiral SFC to provide chiral (2'R, 3R, 4'S)-6-chloro-4'-(3-chlorophenyl)-2'-(5-fluoro-2-methylphenyl)-1'-[(piperidin-4-yl)aminocarbonyl-methyl] spiro[3H-indole-3,3'-piperidine]-2,6'(1H)-dione as a white solid (Yield: 12 mg, 30%) and chiral (2'S, 3S, 4'R) -6-chloro-4'-(3-chlorophenyl)-2'-(5-fluoro-2-methylphenyl)-1'-[(piperidin-4-yl)aminocarbonyl-methyl] spiro[3H-indole-3,3'-piperidine]-2,6'(1H)-dione as a white solid (Yield: 12 mg, 30%).

### Example 60a

### Preparation of intermediate racemic (2'R, 3R, 4'S)-6-chloro-4'-(3-chlorophenyl)-1'-(3-chloro-propyl)-2'-(5-fluoro-2-methylphenyl)-2,3-dihydro-2,6'-dioxo spiro[indole-3,3'-piperidine]-1-methoxyethyl trimethylsilane

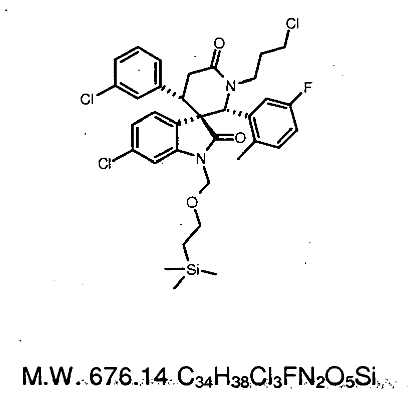

In a manner similar to the method described in example 24c, racemic (2'R, 3R, 4'S)-6-chloro-4'-(3-chlorophenyl)- 2'-(5-fluoro-2-methylphenyl)-2,3-dihydro-2,6'-dioxo spiro[indole-3,3'-piperidine]-1-methoxyethyl trimethylsilane (0.8 g, 1.33 mmol) prepared in example 55b was reacted with LiH (0.5 g, 62.5 mmol) and 1-chloro-3-iodo-propane (3.9 g, 19.1 mmol) in N,N-dimethyl-formamide (20 mL) to give racemic (2'R, 3R, 4'S)-6-chloro-4'-(3-chlorophenyl)-1'-(3-chloro-propyl)-2'-(5-fluoro-2-methylphenyl)-2,3-dihydro-2,6'-dioxo spiro[indole-3,3'-piperidine]-1-methoxyethyl trimethylsilane as a white form (Yield: 0.43 g, 47.8%).

### Example 60b

### Preparation of racemic (2'R, 3R, 4'S)-6-chloro-4'-(3-chlorophenyl)-2'-(5-fluoro-2-methylphenyl)-1'-(3-morpholin-4-yl-propyl) spiro[3H-indole-3,3'-piperidine]-2,6'(1H)-dione

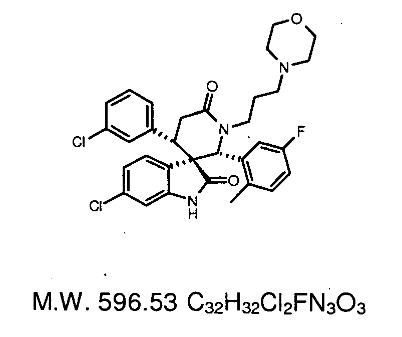

The mixture of racemic (2'R, 3R, 4'S)-6-chloro-4'-(3-chlorophenyl)-2'-(5-fluoro-2-methylphenyl)-2,3-dihydro-1'-(3-chloro-propyl)-2,6'-dioxo spiro[indole-3,3'-piperidine]-1-methoxyethyl trimethylsilane (0.24 g, 0.36 mmol) prepared in example 60a and morpholine (10 mL) was heated at 120 °C for 1 h. The mixture was concentrated to dryness. The resulting residue was added dichloromethane (20 mL) and then trifluoroacetic acid (20 mL). The reaction mixture was stirred at room temperature for 4 h. The reaction mixture was concentrated and the residue was partitioned between ethyl acetate and saturated NaHCO₃ solution. The organic layer was separated and concentrated. The residue was redissolved in methanol (10 mL). To the resulting solution was added N,N'-diisopropylethylamine (2 mL, 11.0 mmol) and the crude was heated at 100 °C for 1 h. The reaction mixture was concentrated and the residue was purified by chromatography (MeOH:EtOAc=1:9) to give racemic (2'R, 3R, 4'S)-6-chloro-4'-(3-chlorophenyl)-2'-(5-fluoro-2-methylphenyl)-1'-(3-morpholin-4-yl-propyl) spiro[3H-indole-3,3'-piperidine]-2,6'(1H)-dione as a white solid (Yield 0.08 g, 37.9%).

HRMS(ES⁺) *m*/*z* Calcd for C₃₂H₃₂Cl₂FN₃O₃ +H [(M+H)⁺]: 596.1878 Found: 596.1877.

### Example 60c

### Preparation of chiral (2'R, 3R, 4'S)-6-chloro-4'-(3-chlorophenyl)-2'-(5-fluoro-2-methylphenyl)-1'-(3-morpholin-4-yl-propyl) spiro[3H-indole-3,3'-piperidine]-2,6'(1H)-dione

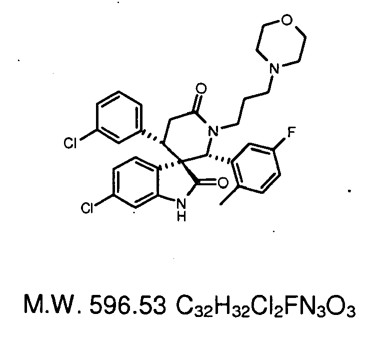

Separation of the two enantiomers from racemic (2'R, 3R, 4'S)-6-chloro-4'-(3-chlorophenyl)-2'-(5-fluoro-2-methylphenyl)-1'-(3-morpholin-4-yl-propyl) spiro[3H-indole-3,3'-piperidine]-2,6'(1H)-dione (100 mg) prepared in example 60b was conducted by chiral SFC to provide chiral (2'R, 3R, 4'S)-6-chloro-4'-(3-chlorophenyl)-2'-(5-fluoro-2-methylphenyl)-1'-(3-morpholin-4-yl-propyl) spiro[3H-indole-3,3'-piperidine]-2,6'(1H)-dione as a white solid (Yield: 34 mg, 34%) and chiral (2'S, 3S, 4'R)-6-chloro-4'-(3-chlorophenyl)-2'-(5-fluoro-2-methylphenyl)-1'-(3-morpholin-4-yl-propyl) spiro[3H-indole-3,3'-piperidine]-2,6'(1H)-dione as a white solid (Yield: 35 mg, 35%).

### Example 61a

### Preparation of intermediate 1-(1-isopropyl-4-methyl-1-pentenyl)-3-trimethylsilyoxy-2-aza-1,3-butadiene

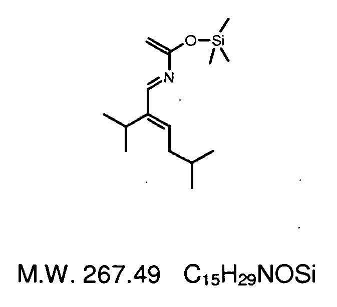

In a manner similar to the method described in example 1b, 2-isopropyl-5-methyl-2-hexenal (1.54 g, 10 mmol) (Aldrich) was used as the starting material in place of 3-chloro-benzaidehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(1-isopropyl-4-methyl-1-pentenyl)-3-trimethylsilyoxy-2-aza-1,3-butadiene and used for the next step without further purification.

### Example 61b

### Preparation of racemic (2'R, 3R, 4'S)-6-chloro-4'-(3-chlorophenyl)-2'-(1-isopropyl-4-methyl-pent-1-enyl) spiro[3H-indole-3,3'-piperidine]-2,6'(1H)-dione

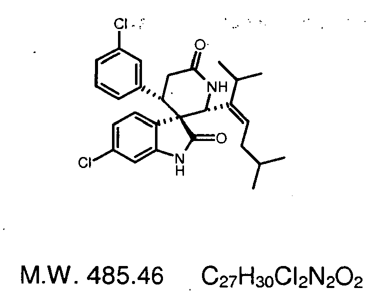

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.3 g, 0.77 mmol) was reacted with 1-(1-isopropyl-4-methyl-1-pentenyl)-3-trimethylsilyoxy-2-aza-1,3-butadiene (2.1 g, 8.2 mmol) prepared in example 61a in toluene and then trifluoroacetic acid (10 mL) in dichloromethane to give racemic (2'R, 3R, 4'S)-6-chloro-4'-(3-chlorophenyl)-2'-(1-isopropyl-4-methyl-pent-1-enyl) spiro[3H-indole-3,3'-piperidine]-2,6'(1H)-dione as an off-white solid (0.18 g, 48.7%).

HRMS(ES⁺) *m*/*z* Calcd for C₂₇H₃₀Cl₂N₂O₂ + H [(M+H)⁺]: 485.1757. Found: 485.1755.

### Example 62a

### Preparation of intermediate 1,2-difluoro-4-isopropoxy-benzene

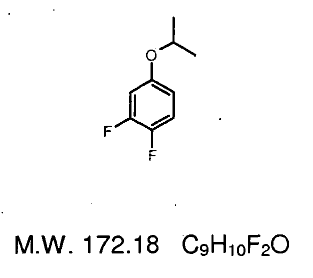

To a solution of 3,4-difluoro-phenol (5 g, 38.4 mmol) in acetone (50 mL) was added potassium carbonate (54 g, 38.4 mmo) and 2-iodo-propane. The reaction mixture was heated at refluxing for 24 h. The crude was cooled down and filtered through a short pad of celite. The filtrate was concentrated and the residue was purified by chromatography (EtOAc:Hexanes=1:9) to give 1,2-difluoro-4-isopropoxy-benzene as colorless oil (Yield 6.12 g, 92.3%).

### Example 62b

### Preparation of intermediate 2,3-difluoro-6-isopropoxy-benzalddehyde

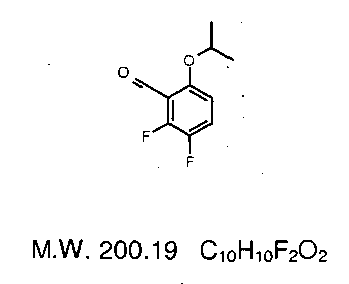

In a manner similar to the method described in example 52a, 1,2-difluoro-4-isopropoxy-benzene (5.77 g, 33.5 mmol) prepared in example 62a was reacted with lithium diisopropyl amine (20.5 mL, 1.8 M in THF, 36.9 mmol), N,N-dimethylformamide (3.11 mL, 40.2 mmol) and quenched with acetic acid (8.0 g, 134 mmol) in tetrahydrofuran to give 2,3-difluoro-6-isopropoxy-benzaldehyde as a white crystal (Yield: 6.02 g, 89.9%).

### Example 62c

### Preparation of intermediate 1-(2,3-difluoro-6-isopropoxy-phenyl)-3-trimethylsilyoxy-2-aza-1,3-butadiene

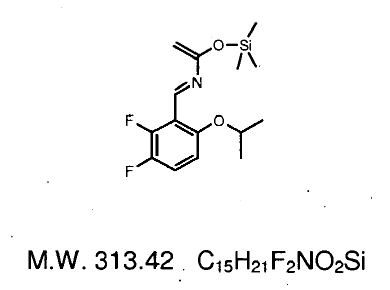

In a manner similar to the method described in example 1b, 2,3-difluoro-6-isopropoxy-benzaldehyde (2.0 g, 10 mmol) prepared in example 62b was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(2,3-difluoro-6-isopropoxy-phenyl)-3-trimethylsilyoxy-2-aza-1,3-butadiene as yellow oil and used for the next step without further purification.

### Example 62d

### Preparation of racemic (2'R, 3R, 4'S)-6-chloro-4'-(3-chlorophenyl)-2'-(2,3-difluoro-6-isopropoxy-phenyl) spiro[3H-indole-3,3'-piperidine]-2,6'(1H)-dione

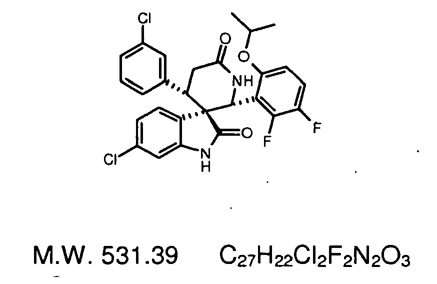

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.4 g, 1.02 mmol) was reacted with 1-(2,3-difluoro-6-isopropoxy-phenyl)-3-trimethylsilyoxy-2-aza-1,3-butadiene (2.5 g, 7.98 mmol) prepared in example 62c in toluene and then trifluoroacetic acid (10 mL) in dichloromethane to give racemic (2'R, 3R, 4'S)-6-chloro-4'-(3-chlorophenyl)-2'-(2,3-difluoro-6-isopropoxy-phenyl) spiro[3H-indole-3,3'-piperidine]-2,6'(1H)-dione as a white solid (0.41 g, 75.9% ).

HRMS(ES⁺) *m*/*z* Calcd for C₂₇H₂₂Cl₂F₂N₂O₃ + H [(M+H)⁺]: 531.1049. Found: 531.1049.

### Example 62e

### Preparation of chiral (2'R, 3R, 4'S)-6-chloro-4'-(3-chlorophenyl)-2'-(2,3-difluoro-6-isopropoxy-phenyl) spiro[3H-indole-3,3'-piperidine]-2,6'(1H)-dione

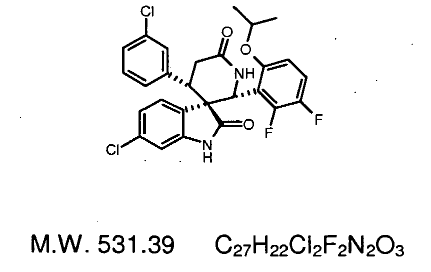

Separation of the two enantiomers from (2'R, 3R, 4'S)-6-chloro-4'-(3-chlorophenyl)-2'-(2,3-difluoro-6-isopropoxy-phenyl) spiro[3H-indole-3,3'-piperidine]-2,6'(1H)-dione prepared in example 62d was conducted by chiral SFC to provide chiral (2'R, 3R, 4'S)-6-chloro-4'-(3-chlorophenyl)-2'-(2,3-difluoro-6-isopropoxy-phenyl) spiro[3H-indole-3,3'-piperidine]-2,6'(1H)-dione as a white solid and chiral (2'S, 3S, 4'R)-6-chloro-4'-(3-chlorophenyl)-2'-(2,3-difluoro-6-isopropoxy-phenyl) spiro[3H-indole-3,3'-piperidine]-2,6'(1H)-dione as a white solid

### Example 63

### Preparation of racemic (2'R, 3R, 4'S)-6-chloro-4'-(3-chlorophenyl)-2'-(5-fluoro-2-methylphenyl)-1'-(3-pyrrolidin-1-yl-propyl) spiro[3H-indole-3,3'-piperidine]-2,6'(1H)-dione

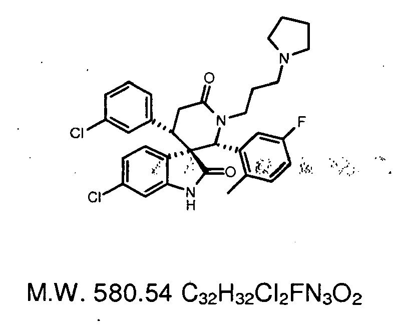

In a manner similar to the method described in example 60b, racemic (2'R, 3R, 4'S)-6-chloro-4'-(3-chlorophenyl)-1'-(3-chloro-propyl)-2'-(5-fluoro-2-methylphenyl)-2,3-dihydro-2,6'-dioxo spiro[indole-3,3'-piperidine]-1-methoxyethyl trimethylsilane prepared in example 60a (91 mg, 0.134 mmol) was reacted with pyrrolodine (2 mL), trifluoroacetic acid (10 mL) and then N,N'-diisopropylethylamine (2 mL) to give racemic (2'R, 3R, 4'S)-6-chloro-4'-(3-chlorophenyl)-2'-(5-fluoro-2-methylphenyl)-1'-(3-pyrrolidin-1-yl-propyl) spiro[3H-indole-3,3'-piperidine]-2,6'(1H)-dione as a white solid (21 mg, 26.9%).

HRMS(ES⁺) *m*/*z* Calcd for C₃₂H₃₂Cl₂FN₃O₂ + H [(M+H)⁺]: 580.1929. Found: 580.1926.

### Example 64a

### Preparation of intermediate 1-(3-methoxycarboxyl-phenyl)-3-trimethylsilyoxy-2-aza-1,3-butadiene

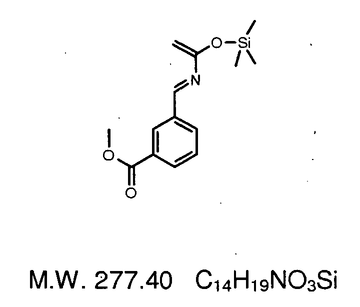

In a manner similar to the method described in example 1b, 3-formyl-benzoic acid methyl ester (1.5 g, 10 mmol) (Acros) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(3-methoxycarboxyl-phenyl)-3-trimethylsilyoxy-2-aza-1,3-butadiene as yellow gum and used for the next step without further purification.

### Example 64b

### Preparation of racemic (2'R, 3R, 4'S)-6-chloro-4'-(3-chlorophenyl)-2'-(3-methoxycarbonyl-phenyl) spiro[3H-indole-3,3'-piperidine]-2,6'(1H)-dione

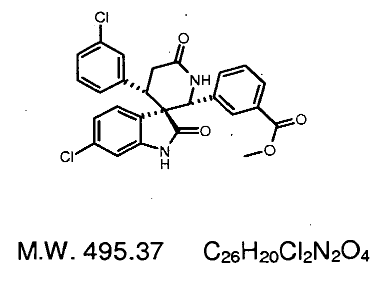

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.4 g, 1.02 mmol) was reacted with 1-(3-methoxycarbonyl-phenyl)-3-trimethylsilyoxy-2-aza-1,3-butadiene (2.4 g, 8.65 mmol) prepared in example 64a in toluene and then trifluoroacetic acid (10 mL) in dichloromethane to give racemic (2'R, 3R, 4'S)-6-chloro-4'-(3-chlorophenyl)-2'-(3-methoxycarbonyl-phenyl) spiro[3H-indole-3,3'-piperidine]-2,6'(1H)-dione as a white solid (0.28 g, 56%).

HRMS(ES⁺) *m*/*z* Calcd for C₂₆H₂₀Cl₂N₂O₄ + H [(M+H)⁺]: 495.0873 Found: 495.0872.

### Example 65

### Preparation of racemic (2'R, 3R, 4'S)-1'-[3-(4-acetyl-piperazin-1-yl)-propyl]-6-chloro-4'-(3-chlorophenyl)-2°-(5-fluoro-2-methylphenyl)spiro[3H-indole-3,3'-piperidine]-2,6'(1H)-dione

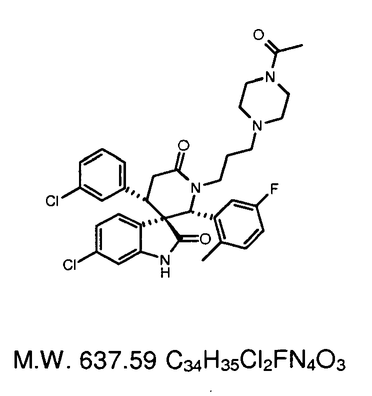

In a manner similar to the method described in example 60b, racemic (2'R, 3R, 4'S)-6-chloro-4'-(3-chlorophenyl)-1 '-(3-chloro-propyl)-2'-(5-fluoro-2-methylphenyl)-2,3-dihydro-2,6'-dioxo spiro[indole-3,3'-piperidine]-1-methoxyethyl trimethylsilane prepared in example 60a (88 mg, 0.134 mmol) was reacted with pyrrolodine (0.4 g, 3.1 mmol), trifluoroacetic acid (10 mL) and then N,N'-diisopropylethylamine (2 mL) to give racemic (2'R, 3R, 4'S)-1'-[3-(4-acetyl-piperazin-1-yl)-propyl]-6-chloro-4'-(3-chlorophenyl)-2'-(5-fluoro-2-methylphenyl) spiro[3H-indole-3,3'-piperidine]-2,6'(1H)-dione as a white solid (33 mg, 38.8% ).

HRMS(ES⁺) *m*/*z* Calcd for C₃₄H₃₅Cl₂FN₄O₃ + H [(M+H)⁺]: 637.2143 Found: 637.2139.

### Example 66a

### Preparation of intermediate 1-(1-ethyl-propenyl)-3-trimethylsilyoxy-2-aza-1,3-butadiene

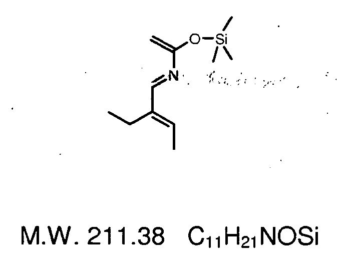

In a manner similar to the method described in example 1b, 2-ethyl-but-2-enal (1.54 g, 10 mmol) (TCI-US) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(1-ethyl-propenyl)-3-trimethylsilyoxy-2-aza-1,3-butadiene and used for the next step without further purification.

### Example 66b

### Preparation of racemic (2'R, 3R, 4'S)-6-chloro-4'-(3-chlorophenyl)-2'-(1-ethyl-propenyl) spiro[3H-indole-3,3'-piperidine]-2,6'(1H)-dione

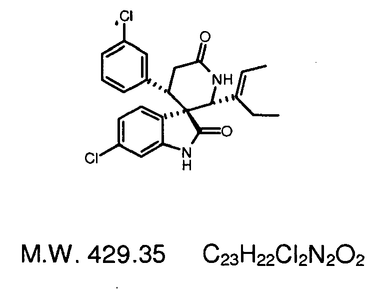

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.4 g, 1.02 mmol) was reacted with 1-(1-ethyl-propenyl)-3-trimethylsilyoxy-2-aza-1,3-butadiene (2.1 g, 9.93 mmol) prepared in example 66a in toluene and then trifluoroacetic acid (20 mL) in dichloromethane to give racemic (2'R, 3R, 4'S)-6-chloro-4'-(3-chlorophenyl)-2'-(1-ethyl-propenyl) spiro[3H-indole-3,3'-piperidine]-2,6'(1H)-dione as a white solid (0.24 g, 54.5% ).

HRMS(ES⁺) *m*/*z* Calcd for C₂₃H₂₂Cl₂N₂O₂ + H [(M+H)⁺]: 429.1131. Found: 429.1129.

### Example 66c

### Preparation of chiral (2'R, 3R, 4'S)-6-chloro-4'-(3-chlorophenyl)-2'-(1-ethyl-propenyl) spiro[3H-indole-3,3'-piperidine]-2,6'(1H)-dione

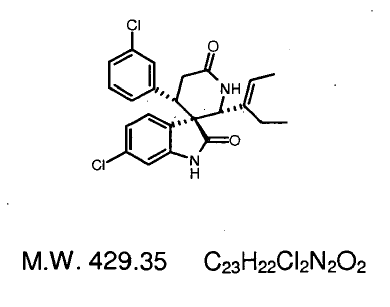

Separation of the two enantiomers (2'R, 3R, 4'S)-6-chloro-4'-(3-chlorophenyl)-2'-(1-ethyl-propenyl) spiro[3H-indole-3,3'-piperidine]-2,6'(1H)-dione prepared in example 66b was conducted by chiral SFC to provide chiral (2'R, 3R, 4'S)-6-chloro-4'-(3-chlorophenyl)-2'-(1-ethyl-propenyl) spiro[3H-indole-3,3'-piperidine]-2,6'(1H)-dione as a white solid and chiral (2'S, 3S, 4'R)-6-chloro-4'-(3-chlorophenyl)-2'-(1-ethyl-propenyl) spiro[3H-indole-3,3'-piperidine]-2,6'(1H)-dione as a white solid.

### Example 67a

### Preparation of racemic (2'R, 3R, 4'S)-6-chloro-4'-(3-chlorophenyl)-1'-[3-(1,1-dioxo-thiomorpholin-4-yl)-propyl]-2'-(5-fluoro-2-methylphenyl)- spiro[3H-indole-3,3'-piperidine]-2,6'(1H)-dione

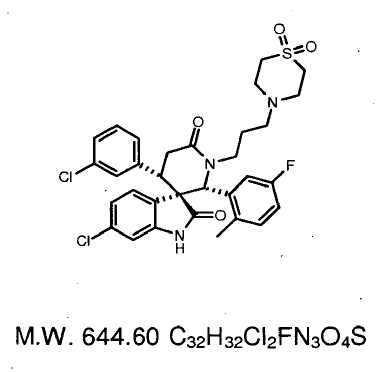

In a manner similar to the method described in example 60b, racemic (2'R, 3R, 4'S)-6-chloro-4'-(3-chlorophenyl)-1'-(3-chloro-propyl)-2'-(5-fluoro-2-methylphenyl)-2,3-dihydro-2,6'-dioxo spiro[indole-3,3'-piperidine]-1-methoxyethyl trimethylsilane prepared in example 60a (0.21 mg, 0.31 mmol) was reacted with thiomorpholin 1,1-dioxide (0.47 g, 3.48 mmol), trifluoroacetic acid (10 mL) and then N,N'-diisopropylethylamine (2 mL) to give racemic (2'R, 3R, 4'S)-6-chloro-4'-(3-chlorophenyl)-1'-[3-(1,1 -dioxo-thiomorpholin-4-yl)-propyl]-2'-(5-fluoro-2-methylphenyl)- spiro[3H-indole-3,3'-piperidine]-2,6'(1H)-dione as a white solid (72 mg, 36.2%).

HRMS(ES⁺) *m*/*z* Calcd for C₃₂H₃₂Cl₂FN₃O₄S + H [(M+H)⁺]: 644.1548. Found: 644.1542.

### Example 67b

### Preparation of chiral (2'R, 3R, 4'S)-6-chloro-4'-(3-chlorophenyl)-1'-[3-(1,1-dioxo-thiomorpholin-4-yl)-propyl]-2'-(5-fluoro-2-methylphenyl)- spiro[3H-indole-3,3'-piperidine]-2,6'(1H)-dione

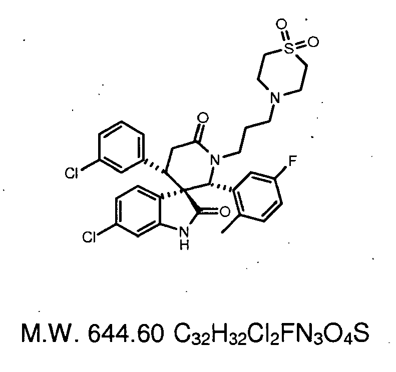

Separation of the two enantiomers from racemic (2'R, 3R, 4'S)-6-chloro-4'-(3-chlorophenyl)-1'-[3-(1,1-dioxo-thiomorpholin-4-yl)-propyl]-2'-(5-fluoro-2-methylphenyl)- spiro[3H-indole-3,3'-piperidine]-2,6'(1H)-dione prepared in example 67a was conducted by chiral SFC to provide chiral (2'R, 3R, 4'S)-6-chloro-4'-(3-chlorophenyl)-1'-[3-(1,1-dioxo-thiomorpholin-4-yl)-propyl]-2'-(5-fluoro-2-methylphenyl)- spiro[3H-indole-3,3'-piperidine]-2,6'(1H)-dione as a white solid and chiral (2'S, 3S, 4'R)-6-chloro-4'-(3-chlorophenyl)-1'-[3-(1,1-dioxo-thiomorpholin-4-yl)-propyl]-2'-(5-fluoro-2-methylphenyl)- spiro[3H-indole-3,3'-piperidine]-2,6'(1H)-dione as a white solid.

### Example 68a

### Preparation of intermediate 1-(2, 5-dimethyl-phenyl)-3-trimethylsilyoxy-2-aza-1,3-butadiene

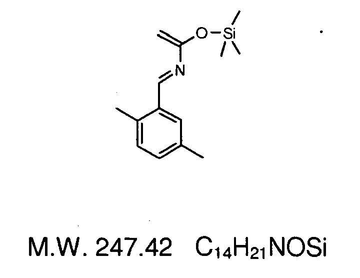

In a manner similar to the method described in example 1b, 2,5-dimethyl-benzaldehyde (1.34 g, 10 mmol) (Aldrich) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(2, 5-dimethyl-phenyl)-3-trimethylsilyoxy-2-aza-1,3-butadiene as yellow gum and used for the next step without further purification.

### Example 68b

### Preparation of racemic (2'R, 3R, 4'S)-6-chloro-4'-(3-chlorophenyl)-2'-(2, 5-dimethyl-phenyl) spiro[3H-indole-3,3'-piperidine]-2,6'(1H)-dione

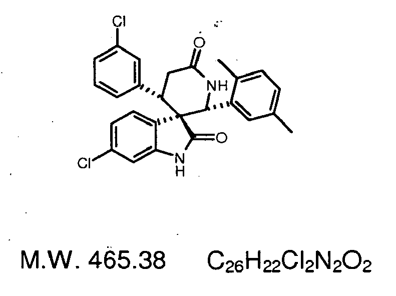

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.4 g, 1.02 mmol) was reacted with 1-(2, 5-dimethyl-phenyl)-3-trimethylsilyoxy-2-aza-1,3-butadiene (2.3 g, 9.31 mmol) prepared in example 68a in toluene and then trifluoroacetic acid (20 mL) in dichloromethane to give racemic (2'R, 3R, 4'S)-6-chloro-4'-(3-chlorophenyl)-2'-(2, 5-dimethyl-phenyl) spiro[3H-indole-3,3'-piperidine]-2,6'(1H)-dione as a white solid.

HRMS(ES⁺) *m*/*z* Calcd for C₂₆H₂₂Cl₂N₂O₂+ H [(M+H)⁺]: 465.1131 Found: 465.1128.

### Example 68c

### Preparation of chiral (2'R, 3R, 4'S)-6-chloro-4'-(3-chlorophenyl)-2'-(2, 5-dimethyl-phenyl) spiro[3H-indole-3,3'-piperidine]-2,6'(1H)-dione

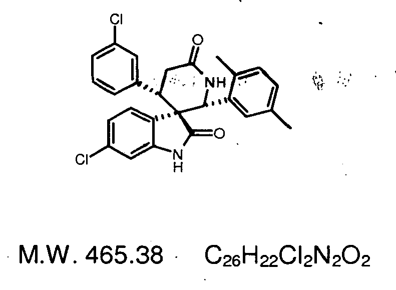

Separation of the two enantiomers from racemic (2'R, 3R, 4'S)-6-chloro-4'-(3-chlorophenyl)-2'-(2, 5-dimethyl-phenyl) spiro[3H-indole-3,3'-piperidine]-2,6'(1H)-dione prepared in example 68b was conducted by chiral SFC to provide chiral (2'R, 3R, 4'S)-6-chloro-4'-(3-chlorophenyl)-2'-(2, 5-dimethyl-phenyl) spiro[3H-indole-3,3'-piperidine]-2,6'(1H)-dione as a white solid and chiral ((2'S, 3S, 4'R)-6-chloro-4'-(3-chlorophenyl)-2'-(2, 5-dimethyl-phenyl) spiro[3H-indole-3,3'-piperidine]-2,6'(1H)-dione as a white solid.

### Example 69a

### Preparation of intermediate 1-(2,5-dimethyl-2H-pyrazole-3-yl)-3-trimethylsilyoxy-2-aza-1,3-butadiene

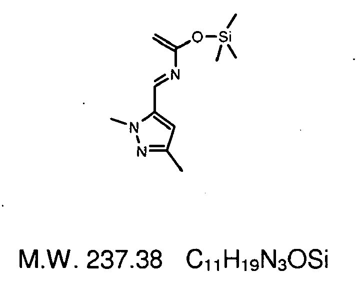

In a manner similar to the method described in example 1b, 2,5-dimethyl-2H-pyrazole-3-carbaldehyde (1.24 g, 10 mmol) (ASDI-INTER) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(2,5-dimethyl-2H-pyrazole-3-yl)-3-trimethylsilyoxy-2-aza-1,3-butadiene as yellow gum and used for the next step without further purification.

### Example 69b

### Preparation of racemic (2'R, 3R, 4'S)-6-chloro-4'-(3-chlorophenyl)-2'-(2,5-dimethyl-2H-pyrazole-3-yl) spiro[3H-indole-3,3'-piperidine]-2,6'(1H)-dione

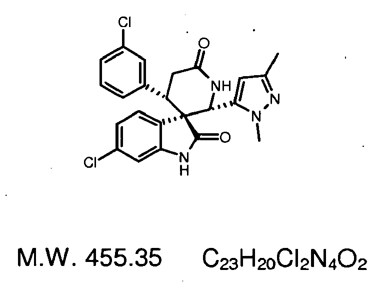

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.4 g, 1.02 mmol) was reacted with 1-(2,5-dimethyl-2H-pyrazole-3-yl)-3-trimethylsilyoxy-2-aza-1,3-butadiene (2.5 g, 10.5 mmol) prepared in example 69a in toluene and then trifluoroacetic acid (15 mL) in dichloromethane to give racemic (2'R, 3R, 4'S)-6-chloro-4'-(3-chlorophenyl)-2'-(2,5-dimethyl-2H-pyrazole-3-yl) spiro[3H-indole-3,3'-piperidine]-2,6'(1H)-dione as a white solid (Yield 0.14 g, 30.4%).

HRMS(ES⁺) *m*/*z* Calcd for C₂₃H₂₀Cl₂N₄O₂ + H [(M+H)⁺]: 455.1036 Found: 455.1035.

### Example 70a

### Preparation of intermediate 1-(1-methyl-propenyl)-3-trimethylsilyoxy-2-aza-1,3-butadiene

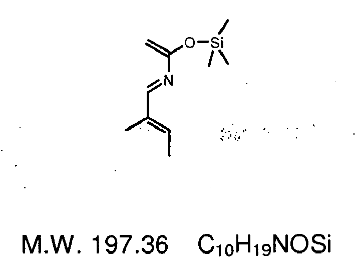

In a manner similar to the method described in example 1b, 2-methyl-but-2-enal (0.84 g, 10 mmol) (EASTMAN) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(1-methyl-propenyl)-3-trimethylsilyoxy-2-aza-1,3-butadiene and used for the next step without further purification.

### Example 70b

### Preparation of racemic (2'R, 3R, 4'S)-6-chloro-4'-(3-chlorophenyl)-2'-(1-methyl-propenyl) spiro[3H-indole-3,3'-piperidine]-2,6'(1H)-dione

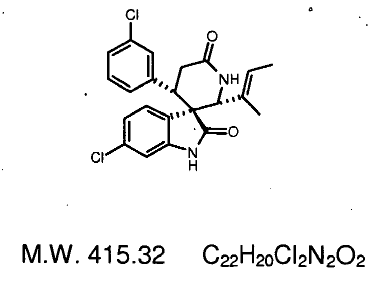

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.35 g, 0.89 mmol) was reacted with 1-(1-methyl-propenyl)-3-trimethylsilyoxy-2-aza-1,3-butadiene (2.1 g, 9.93 mmol) prepared in example 70a in toluene and then trifluoroacetic acid (20 mL) in dichloromethane to give racemic (2'R, 3R, 4'S)-6-chloro-4'-(3-chlorophenyl)-2'-(1-methyl-propenyl) spiro[3H-indole-3,3'-piperidine]-2,6'(1H)-dione as a white solid (0.19 g, 51.4% ).

HRMS(ES⁺) *m*/*z* Calcd for C₂₂H₂₀Cl₂N₂O₂ + H [(M+H)⁺]: 415.0975. Found: 415.0975.

### Example 71a

### Preparation of intermediate 1-(1-methyl-but-1-enyl)-3-trimethylsilyoxy-2-aza-1,3-butadiene

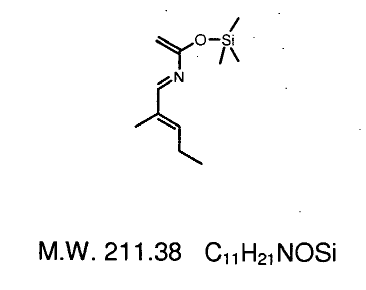

In a manner similar to the method described in example 1b, 2-methyl-pent-2-enal (2.0 g, 20 mmol) (Aldrich) was used as the starting material in place of 3-chloro-benzaldehyde to react with 1,1,1,3,3,3-hexamethyldisilazane (3.2 g, 20 mmol), *n-*butyllithium (2.5 M, 8 mL, 20 mmol), trimethylsilyl chloride (2.2 g, 20 mmol), triethylamine (2.7 g, 26 mmol) and acetyl chloride (2.0 g, 26 mmol) to give 1-(1-methyl-but-1-enyl)-3-trimethylsilyoxy-2-aza-1,3-butadiene and used for the next step without further purification.

### Example 71b

### Preparation of racemic (2'R, 3R, 4'S)-6-chloro-4'-(3-chlorophenyl)-2'-(1-methyl-but-1-enyl) spiro[3H-indole-3,3'-piperidine]-2,6'(1H)-dione

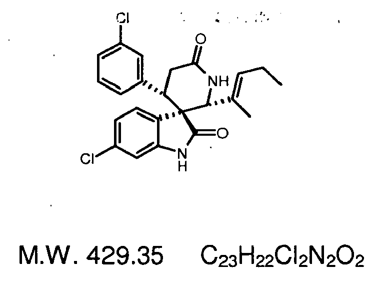

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.4 g, 1.02 mmol) was reacted with 1-(1-methyl-but-1-enyl)-3-trimethylsilyoxy-2-aza-1,3-butadiene(3.2 g, 15.2 mmol) prepared in example 71a in toluene and then trifluoroacetic acid (10 mL) in dichloromethane to give racemic (2'R, 3R, 4'S)-6-chloro-4'-(3-chlorophenyl)-2'-(1-methyl-but-1-enyl) spiro[3H-indole-3,3'-piperidine]-2,6'(1H)-dione as a white solid (0.24 g, 54.5%).

HRMS(ES⁺) *m*/*z* Calcd for C₂₃H₂₂Cl₂N₂O₂ + H [(M+H)⁺]: 429.1131. Found: 429.1127.

### Example 72a

### Preparation of racemic (2'R, 3R, 4'S)-6-chloro-4'-(3-chlorophenyl)-2'-(1-ethylpropyl) spiro[3H-indole-3,3'-piperidine]-2,6'(1H)-dione

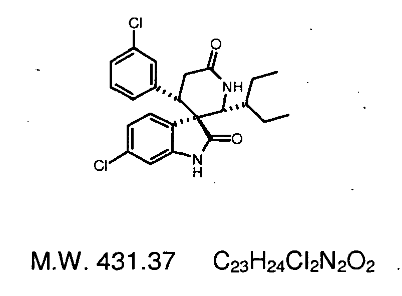

To a solution of racemic (2'R, 3R, 4'S)-6-chloro-4'-(3-chlorophenyl)-2'-(1-ethyl-propenyl) spiro[3H-indole-3,3'-piperidine]-2,6'(1H)-dione (0.3 g, 0.70 mmol) prepared in example 66b in ethyl acetate (30 mL) was added platinum oxide (0.35 g, 1.54 mmol). The resulting suspension was vigorously shaken under hydrogen (50 psi) for 6 h. The mixture was filtered through a short pad of celite. The filtrate was concentrated. The residue was purified by chromatography (EtOAc:CH₂Cl₂=1:1) to give racemic (2'R, 3R, 4'S)-6-chloro-4'-(3-chlorophenyl)-2'-(1-ethyl-propyl) spiro[3H-indole-3,3'-piperidine]-2,6'(1H)-dione as a white solid (Yield 0.11g, 37.7%).

HRMS(ES⁺) *m*/*z* Calcd for C₂₃H₂₄Cl₂N₂O₂ +H [(M+H)⁺]: 431.1288 Found: 431.1285.

### Example 72b

### Preparation of chiral (2'R, 3R, 4'S)-6-chloro-4'-(3-chlorophenyl)-2'-(1-ethylpropyl) spiro[3H-indole-3,3'-piperidine]-2,6'(1H)-dione

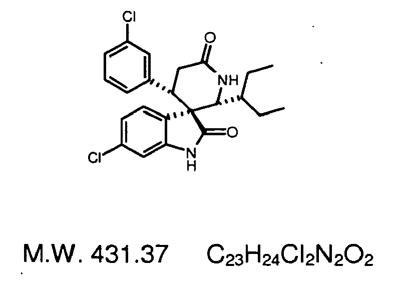

Separation of the two enantiomers from racemic (2'R, 3R, 4'S)-6-chloro-4'-(3-chlorophenyl)-2'-(1-ethyl-propyl) spiro[3H-indole-3,3'-piperidine]-2,6'(1H)-dione (60 mg) was conducted by chiral SFC to provide chiral (2'R, 3R, 4'S)-6-chloro-4'-(3-chlorophenyl)-2'-(1-ethyl-propyl) spiro[3H-indole-3,3'-piperidine]-2,6'(1H)-dione as a white solid (Yield: 24 mg, 40%) and chiral (2'S, 3S, 4'R)- 6-chloro-4'-(3-chlorophenyl)-2'-(1-ethyl-propyl) spiro[3H-indole-3,3'-piperidine]-2,6'(1H)-dione (Yield: 24 mg, 40%).

### Example 73a

### Preparation of intermediate racemic (2'R, 3R, 4'S)-6-chloro-4'-(3-chlorophenyl)-2'-(2,3-difluoro-6-isopropoxy-phenyl)-2,3-dihydro-2,6'-dioxo spiro[indole-3,3'-piperidine]-1-methoxyethyl trimethylsilane

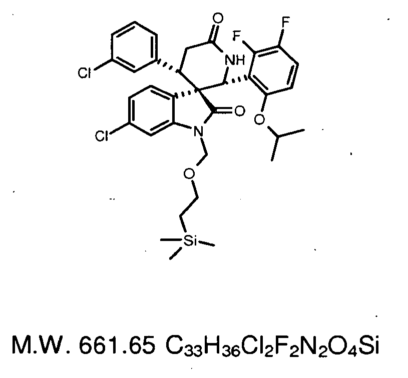

In a manner similar to the method described in example 55b, E/Z-6-chloro-3-(3-chloro-benzylidene)-1-(2-trimethylsilanyl-ethoxymethyl)-1,3-dihydro-indole-2-one prepared in example 55a (3.4 g, 8.10 mmol) was reacted with 1-(2,3-difluoro-6-isopropoxy-phenyl)-3-trimethylsilyoxy-2-aza-1,3-butadiene (16 g, 51.1 mmol) prepared in example 62c in toluene to give racemic (2'R, 3R, 4'S)-6-chloro-4'-(3-chlorophenyl)-2'-(2,3-difluoro-6-isopropoxy-phenyl)-2,3-dihydro-2,6'-dioxo spiro[indole-3,3'-piperidine]-1-methoxyethyl trimethylsilane as an off-white solid.

### Example 73b

### Preparation of intermediate racemic (2'R, 3R, 4'S)-6-chloro-4'-(3-chloro-phenyl)-1'-(3-chloro-propyl)-2'-(2,3-difluoro-6-isopropoxy-phenyl)-2,3-dihydro-2,6'-dioxo spiro[indole-3,3'-piperidine]-1-methoxyethyl trimethylsilane

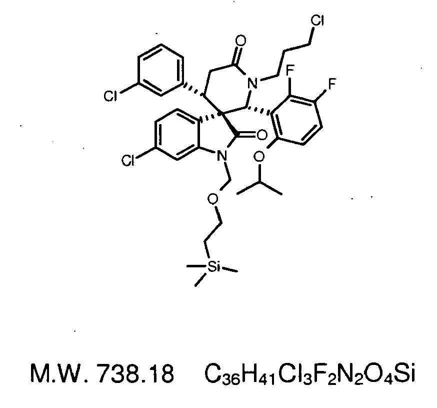

In a manner similar to the method described in example 24c, racemic (2'R, 3R, 4'S)-6-chloro-4'-(3-chlorophenyl)-2'-(2,3-difluoro-6-isopropoxy-phenyl)-2,3-dihydro-2,6'-dioxo spiro[indole-3,3'-piperidine]-1-methoxyethyl trimethylsilane (1.8 g, 2.72 mmol) prepared in example 73a was reacted with LiH (1.0 g, 125 mmol) and 1-chloro-3-iodo-propane (5.0 g, 24.5 mmol) in N,N-dimethylformamide (40 mL) to give racemic (2'R, 3R, 4'S)-6-chloro-4'-(3-chloro-phenyl)-1'-(3-chloro-propyl)-2'-(2,3-difluoro-6-isopropoxy-phenyl)-2,3-dihydro-2,6'-dioxo spiro[indole-3,3'-piperidine]-1-methoxyethyl trimethylsilane as a white form (Yield: 0.67 g, 33.5%).

### Example 73c

### Preparation of racemic (2'R, 3R, 4'S)-6-chloro-4'-(3-chlorophenyl)-2'-(2,3-difluoro-6-isopropoxy-phenyl)-1'-(3-morpholin-4-yl-propyl) spiro[3H-indole-3,3'-piperidine]-2,6'(1H)-dione

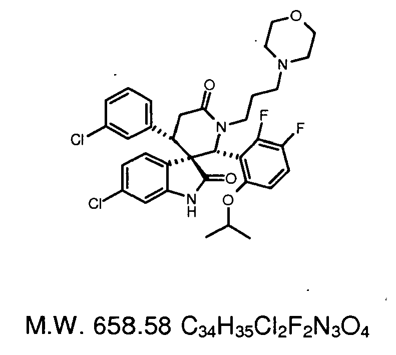

In a manner similar to the method described in example 60b, racemic (2'R, 3R, 4'S)-6-chloro-4'-(3-chloro-phenyl)-1'-(3-chloro-propyl)-2'-(2,3-difluoro-6-isopropoxy-phenyl)-2,3-dihydro-2,6'-dioxo spiro[indole-3,3'-piperidine]-1-methoxyethyl trimethylsilane prepared in example 73b (0.3 g, 0.41 mmol) was reacted with morpholine (10 mL), trifluoroacetic acid (10 mL) and then N,N'-diisopropylethylamine (1 mL) to give racemic (2'R, 3R, 4'S)-6-chloro-4'-(3-chlorophenyl)-2'-(2,3-difluoro-6-isopropoxy-phenyl)-1'-(3-morpholin-4-yl-propyl) spiro[3H-indole-3,3'-piperidine]-2,6'(1H)-dione as a white solid (0.15 g, 55.6% ).

HRMS(ES⁺) *m*/*z* CalCd for C₃₄H₃₅Cl₂F₂N₃O₄ + H [(M+H)⁺]: 658.2046. Found: 658.2038.

### Example 73d

### Preparation of chiral (2'R, 3R, 4'S)-6-chloro-4'-(3-chlorophenyl)-2'-(2,3-difluoro-6-isopropoxy-phenyl)-1'-(3-morpholin-4-yl-propyl) spiro[3H-indole-3,3'-piperidine]-2,6'(1H)-dione

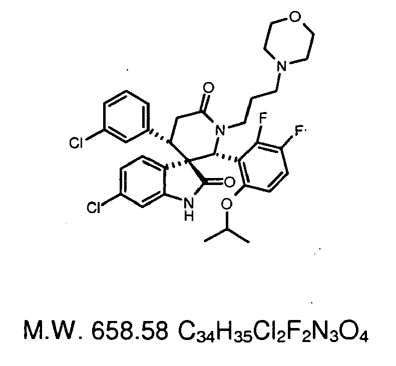

Separation of the two enantiomers from racemic (2'R, 3R, 4'S)-6-chloro-4'-(3-chlorophenyl)-2'-(2,3-difluoro-6-isopropoxy-phenyl)-1'-(3-morpholin-4-yl-propyl) spiro[3H-indole-3,3'-piperidine]-2,6'(1H)-dione (120 mg) prepared in example 73c was conducted by chiral SFC to provide chiral (2'R, 3R, 4'S)-6-chloro-4'-(3-chlorophenyl)-2'-(2,3-difluoro-6-isopropoxy-phenyl)-1'-(3-morpholin-4-yl-propyl) spiro[3H-indole-3,3'-piperidine]-2,6'(1H)-dione as a white solid (Yield: 55 mg, 46%) and chiral (2'S,3S,4'R)-6-chloro-4'-(3-chlorophenyl)-2-(2,3-difluoro-6-isopropoxy-phenyl)-1'-(3-morpholin-4-yl-propyl) spiro[3H-indole-3,3'-piperidine]-2,6'(1H)-dione as a white solid (Yield: 54 mg, 45%).

### Example 74a

### Preparation of intermediate 1-(1-ethylidene-pentyl)-3-trimethylsilyoxy-2-aza-1,3-butadiene

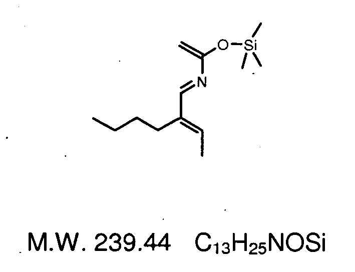

In a manner similar to the method described in example 1b, 2-ethyllidene-hexanal (1.1 g, 8.68 mmol) (Aldrich) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (2.7 g, 13 mmol) and acetyl chloride (1.0 g, 26 mmol) to give 1-(1-ethylidene-pentyl)-3-trimethylsilyoxy-2-aza-1,3-butadiene and used for the next step without further purification.

### Example 74b

### Preparation of racemic (2'R, 3R, 4'S)-6-chloro-4'-(3-chlorophenyl)-2'-(1-ethylidene-pentyl) spiro[3H-indole-3,3'-piperidine]-2,6'(1H)-dione

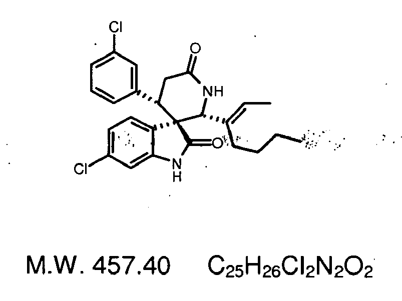

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.4 g, 1.02 mmol) was reacted with 1-(1-ethylidene-pentyl)-3-trimethylsilyoxy-2-aza-1,3-butadiene (2.1 g, 8.77 mmol) prepared in example 74a in toluene and then trifluoroacetic acid (15 mL) in dichloromethane to give racemic (2'R, 3R, 4'S)-6-chloro-4'-(3-chlorophenyl)-2'-(1-ethylidene-pentyl) spiro[3H-indole-3,3'-piperidine]-2,6'(1H)-dione as an off-white solid (0.13 g, 27.7% ).

HRMS(ES⁺) *m*/*z* Calcd for C₂₅H₂₆Cl₂N₂O₂+ H [(M+H)⁺]: 457.1444 Found: 457.1443.

### Example 75

### Preparation of racemic (2'R, 3R, 4'S)-6-chloro-4'-(3-chlorophenyl)-2'-(2,3-difluoro-6-isopropoxy-phenyl)-6'-thioxo spiro[3H-indole-3,3'-piperidine]-2(1H)-one

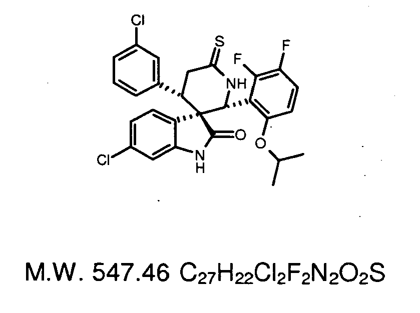

In a manner similar to the method described in example 30, racemic (2'R, 3R, 4'S)-6-chloro-4'-(3-chlorophenyl)-2'=(2,3-difluoro-6-isopropoxy-phenyl) spiro[3H-indole-3,3'-piperidine]-2,6'(1H)-dione (0.45 g, 0.85 mmol) prepared in example 62d was reacted with 2,4-bis-(4-methoxyphenyl)-1,3-dithia-2,4-diphosphetane 2,4-disulfide (0.6 g, 1.8 mmol) in toluene to give racemic (2'R, 3R, 4'S)-6-chloro-4'-(3-chlorophenyl)-2'-(2,3-difluoro-6-isopropoxy-phenyl)-6'-thioxo spiro[3H-indole-3,3'-piperidine]-2(1H)-one (Yield 0.36 g, 78.3%).

HRMS(ES⁺) *m*/*z* Calcd for C₂₇H₂₂Cl₂F₂N₂O₂S +H [(M+H)⁺]: 547.0820. Found: 547.0821.

### Example 76

### Preparation of racemic (2'R, 3R, 4'S)-[6-chloro-4'-(3-chlorophenyl)-2'-(2,3-difluoro-6-isopropoxy-phenyl)-2,3-dihydro-2-oxospiro[3H-indole-3,3'-piperidin]-6-ylene] hydrazine carboxylic acid ethyl ester

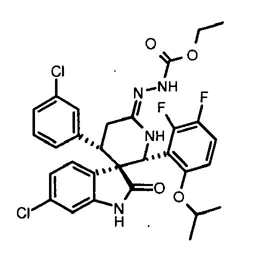

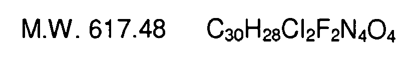

In a manner similar to the method described in example 40, racemic (2'R, 3R, 4'S)-6-chloro-4'-(3-chlorophenyl)-2'-(2,3-difluoro-6-isopropoxy-phenyl)-6'-thioxo spiro[3H-indole-3,3'-piperidine]-2(1H)-one (0.30 g, 0.55 mmol) prepard in example 75 was reacted with ethyl carbazate (0.3 g, 2.97 mmol), mercuric acetate (0.30 g, 0.95 mmol) and triethylamine (0.1 g, 0.99 mmol) in tetrahydrofuran (40 mL) to give racemic (2'R, 3R, 4'S)-[6-chloro-4'-(3-chlorophenyl)-2'-(2,3-difluoro-6-isopropoxy-phenyl)-2,3-dihydro-2-oxospiro[3H-indole-3,3'-piperidin]-6-ylene] hydrazine carboxylic acid ethyl ester as a white solid (Yield 0.25 g, 73.5 %).

HRMS(ES⁺) *m*/*z* Calcd for C₃₀H₂₈Cl₂F₂N₄O₄ + H [(M+H)⁺]: 617.1529. Found: 617.1523.

### Example 77a

### Preparation of intermediate cyclopent-1-enecarbaldehyde

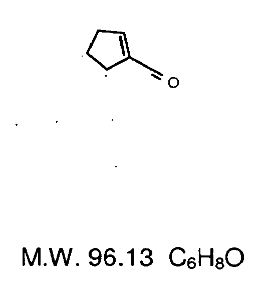

To an acidic solution of sodium periodate (28.3 g, 0.13 mol) (Aldrich) in water (250 mL) was added the solution of 1,2-cycohexanediol (12 g, 0.10 mol) (Acros) in ethyl ether (150 mL). The mixture was stirred vigorously for 0.5 h at room temperature. After addition of KOH aqueous solution (20%, 38.4 mL), the reaction mixture was stirred for an additional 1 h. The mixture was extracted with ethyl ether. The organic layers were combined and dried. The solvent was removed to give cyclopent-1-enecarbaldehyde as yellow oil (Yield: 7.2 g, 75% )

### Example 77b

### Preparation of intermediate 1-(cyclopent-1-enyl)-3-trimethylsilyoxy-2-aza-1,3-butadiene

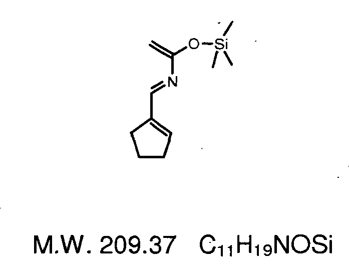

In a manner similar to the method described in example 1b, 2 cyclopent-1-enecarbaldehyde (1.4 g, 14.6 mmol) prepared in example 77a was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (2.7 g, 13 mmol) and acetyl chloride (1.0 g, 26 mmol) to give 1-(cyclopent-1-enyl)-3-trimethylsilyoxy-2-aza-1,3-butadiene and used for the next step without further purification.

### Example 77c

### Preparation of racemic (2'R, 3R, 4'S)-6-chloro-4'-(3-chlorophenyl)-2'-(cyclopent-1-enyl) spiro[3H-indole-3,3'-piperidine]-2,6'(1H)-dione

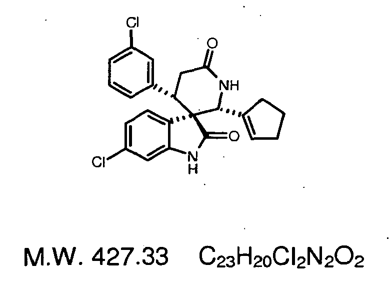

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.4 g, 1.02 mmol) was reacted with 1-(cyclopent-1-enyl)-3-trimethylsilyoxy-2-aza-1,3-butadiene (2.0 g, 9.55 mmol) prepared in example 77b in toluene and then trifluoroacetic acid (10 mL) in dichloromethane to give racemic (2'R, 3R, 4'S)-6-chloro-4'-(3-chlorophenyl)-2'-(cyclopent-1-enyl) spiro[3H-indole-3,3'-piperidine]-2,6'(1H)-dione as a white solid (0.12 g, 27.3%).

HRMS(ES⁺) *m*/*z* Calcd for C₂₃H₂₀Cl₂N₂O₂ + H [(M+H)⁺]: 427.0975 Found: 427.0972.

### Example 78a

### Preparation of racemic (2'R, 3R, 4'S)-6-chloro-4'-(3-chlorophenyl)-2'-cyclopentyl spiro[3H-indole-3,3'-piperidine]-2,6'(1H)-dione

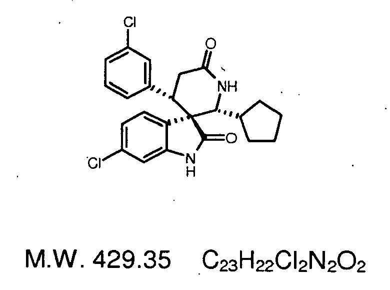

In a manner similar to the method described in example 72, racemic (2'R, 3R, 4'S)-6-chloro-4'-(3-chlorophenyl)-2'-(cyclopent-1-enyl) spiro[3H-indole-3,3'-piperidine]-2,6'(1H)-dione (0.1 g, 0.23 mmol) prepared in example 77c was treated with platinum oxide in ethyl acetate under hydrogen (50 psi) to give racemic (2'R, 3R, 4'S)-6-chloro-4'-(3-chlorophenyl)-2'-cyclopentyl spiro[3H-indole-3,3'-piperidine]-2,6'(1H)-dione as a white solid (0.031 g, 31 % ).

HRMS(ES⁺) *m*/*z* Calcd for C₂₃H₂₂Cl₂N₂O₂ + H [(M+H)⁺]: 429.1131 Found: 429.1131.

### Example 78b

### Preparation of chiral (2'R, 3R, 4'S)-6-chloro-4'-(3-chlorophenyl)-2'-cyclopentyl spiro[3H-indole-3,3'-piperidine]-2,6'(1H)-dione

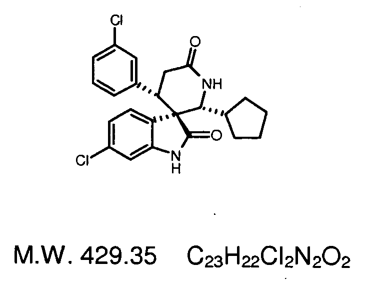

Separation of the two enantiomers from (2'R, 3R, 4'S)-6-chloro-4'-(3-chlorophenyl)-2'-cyclopentyl spiro[3H-indole-3,3'-piperidine]-2,6'(1H)-dione (30 mg) prepared in example 78a was conducted by chiral SFC to provide chiral (2'R, 3R, 4'S)-6-chloro-4'-(3-chlorophenyl)-2'-cyclopentyl spiro[3H-indole-3,3'-piperidine]-2,6'(1H)-dione as a white solid (6 mg, 20%) and chiral (2'S, 3S, 4'R)-6-chloro-4'-(3-chlorophenyl)-2'-cyclopentyl spiro[3H-indole-3,3'-piperidine]-2,6'(1H)-dione as a white solid (6 mg, 20%).

### Example 79a

### Preparation of intermediate E-2-Isopropyl-but-2-en-1-ol

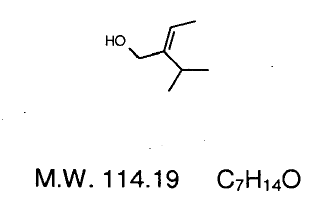

To a solution of 2-butyn-1-ol (14 g, 0.2 mol) (Aldrich) and Cul (32 g, 0.2 mol) (Aldrich) in ether at 0 °C was added isopropylmagnesium chloride (2 M, 300 mL, 0.6 mol) solution in tetrahydrofuran dropwise. The reaction mixture was stirred at room temperature for 24 h. The reaction mixture was quenched with aqueous saturated NH₄Cl solution, extracted with ether twice. The organic layers were combined, washed with water, brine, dried over MgSO₄, filtered and concentrated. The residue was purified with chromatography (EtOAc:Hexane = 1:8) to give E-2-isopropyl-but-2-en-1-ol as pale yellow oil (Yield 5.3 g, 23%).

The same transformation has been reported by Duboudin, J. G.; Jousseaume, B. in J. Organometallic Chem. (1979), 168(1), 1-11 and J. Organometallic Chem. (1975), 91 (1), C1-C3.

### Example 79b

### Preparation of intermediate E-2-Isopropyl-but-2-enal

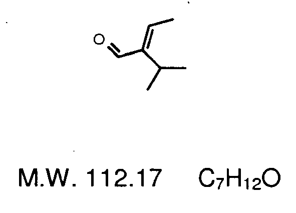

To a solution of oxalyl chloride (6.49 g, 51 mmol) (Aldrich) in dichloromethane (50 mL) at -78 °C was added a solution of dimethyl sulfoxide (7.25 mL, 102 mmol) in dichloromethane (40 mL) dropwise. After 5 mins, the solution of E-2-isopropyl-but-2-en-1-ol (5.3 g, 46 mmol) prepared in example 79a in dichloromethane (10 mL) was added dropwise. The reaction mixture was stirred at -78 °C for 15 min. Triethylamine (22 mL, 0.19 mol) was added and the reaction mixture was slowly warmed to room temperature and stirred at room temperature for 45 mins. Water was added, and organic layer was separated. The aqueous layer was extracted with ether. The organic layers were combined, washed with 10% of HCl, saturated NaHCO₃, brine, dried over MgSO₄, and concentrated to give crude E-2-Isopropyl-but-2-enal as a yellow oil (Yield 5.3 g, 98%).

### Example 79c

### Preparation of intermediate 1-(1-isopropyl-propenyl)-3-trimethylsilyoxy-2-aza-1,3-butadiene

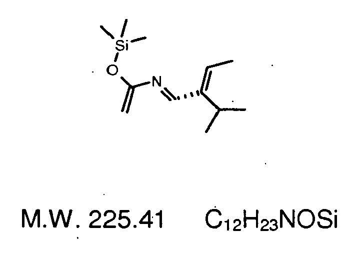

In a manner similar to the method described in example 1b, E-2-isopropyl-but-2-enal prepared in example 79 b (2.2 g, 20 mmol) was used as the starting material in place of 3-chloro-benzaldehyde to react with 1,1,1.3,3,3-hexamethyldisilazane (3.2 g, 20 mmol), *n*-butyllithium (2.5 M, 4 mL, 20 mmol), trimethylsilyl chloride (2.2 g, 20 mmol), triethylamine (2.72 g, 26 mmol) and acetyl chloride (2 g, 26 mmol) to give 1-(1-isopropyl-propenyl)-3-trimethylsilyoxy-2-aza-1,3-butadiene and used for the next step without further purification.

### Example 79d

### Preparation of racemic (2'R, 3R, 4'S)-6-chloro-4'-(3-chlorophenyl)-2'-(1-isopropyl-propenyl) spiro[3H-indole-3,3'-piperidine]-2,6'(1H)-dione

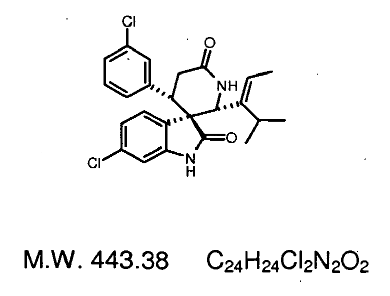

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.5 g, 1.3 mmol) was reacted with 1-(1-ethyl-propenyl)-3-trimethylsilyoxy-2-aza-1,3-butadiene (3.7 g, 16.4 mmol) prepared in example 79c in toluene and then trifluoroacetic acid (20 mL) in dichloromethane to give racemic (2'R, 3R, 4'S)-6-chloro-4'-(3-chlorophenyl)-2'-(1-isopropyl-propenyl) spiro[3H-indole-3,3'-piperidine]-2,6'(1H)-dione as a white solid (0.28 g, 50%).

HRMS(ES⁺) *m*/*z* Calcd for C₂₄H₂₄Cl₂N₂O₂ + H [(M+H)⁺]: 443.1288. Found: 443.1284

### Example 79e

### Preparation of chiral (2'R, 3R, 4'S)-6-chloro-4'-(3-chlorophenyl)-2'-(1-isopropyl-propenyl) spiro[3H-indole-3,3'-piperidine]-2,6'(1H)-dione

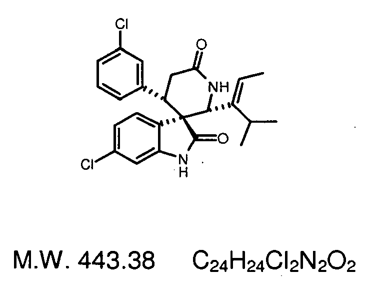

Separation of the two enantiomers (2'R, 3R, 4'S)-6-chloro-4'-(3-chlorophenyl)-2'-(1-ethyl-propenyl) spiro[3H-indole-3,3'-piperidine]-2,6'(1H)-dione (40 mg) prepared in example 79d was conducted by chiral SFC to provide chiral (2'R, 3R, 4'S)-6-chloro-4'-(3-chlorophenyl)-2'-(1-isopropyl-propenyl) spiro[3H-indole-3,3'-piperidine]-2,6'(1H)-dione as a white solid (12 mg, 30%) and chiral (2'S, 3S, 4'R)-6-chloro-4'-(3-chlorophenyl)-2'-(1-isopropyl-propenyl) spiro[3H-indole-3,3'-piperidine]-2,6'(1H)-dione as a white solid (12 mg, 30%).

### Example 80a

### Preparation of intermediate 1-(1-methylene-propyl)-3-trimethylsilyoxy-2-aza-1,3-butadiene

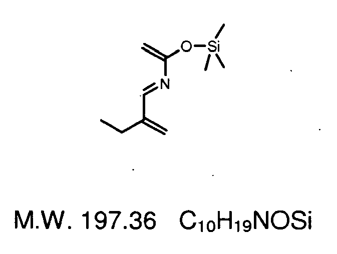

In a manner similar to the method described in example 1b, ethylacrolein (2.1 g, 22 mmol) (TCl-US) was used as the starting material in place of 3-chloro-benzaldehyde to react with 1,1,1,3,3,3-hexamethyldisilazane (3.2 g, 20 mmol), *n-*butyllithium (2.5 M, 8 mL, 20 mmol), trimethylsilyl chloride (2.2 g, 20 mmol), triethylamine (2.9 g, 27 mmol) and acetyl chloride (2 g, 27 mmol) to give 1-(1-methylene-propyl)-3-trimethylsilyoxy-2-aza-1,3-butadiene and used for the next step without further purification.

### Example 80b

### Preparation of racemic (2'R, 3R, 4'S)-6-chloro-4'-(3-chlorophenyl)-2'-(1-methylene-propyl) spiro[3H-indole-3,3'-piperidine]-2,6'(1H)-dione

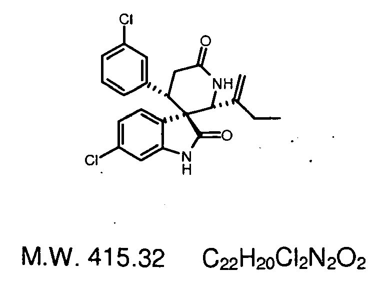

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.42 g, 1.1 mmol) was reacted with 1-(1-methylene-propyl)-3-trimethylsilyoxy-2-aza-1,3-butadiene (3.2 g, 16.2 mmol) prepared in example 80a in toluene and then trifluoroacetic acid (10 mL) in dichloromethane to give racemic (2'R, 3R, 4'S)-6-chloro-4'-(3-chlorophenyl)-2'-(1-methylene-propyl) spiro[3H-indole-3,3'-piperidine]-2,6'(1H)-dione as a white solid (0.31 g, 67%).

HRMS(ES⁺) *m*/*z* Calcd for C₂₂H₂₀Cl₂N₂O₂ + H [(M+H)⁺]: 415.0975. Found: 415.0975

### Example 80c

### Preparation of chiral (2'R, 3R, 4'S)-6-chloro-4'-(3-chlorophenyl)-2'-(1-methylene-propyl) spiro[3H-indole-3,3'-piperidine]-2,6'(1H)-dione

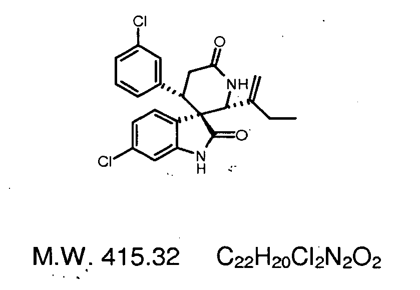

Separation of the two enantiomers of racemic (2'R, 3R, 4'S)-6-chloro-4'-(3-chlorophenyl)-2'-(1-methylene-propyl) spiro[3H-indole-3,3'-piperidine]-2,6'(1H)-dione (150 mg) prepared in example 80b was conducted by chiral SFC to provide chiral (2'R, 3R, 4'S)-6-chloro-4'-(3-chlorophenyl)-2'-(1-methylene-propyl) spiro[3H-indole-3,3'-piperidine]-2,6'(1H)-dione as a white solid (63 mg, 42% ) and chiral (2'S, 3S, 4'R)-6-chloro-4'-(3-chlorophenyl)-2'-(1-methylene-propyl) spiro[3H-indole-3,3'-piperidine]-2,6'(1H)-dione as a white solid (60 mg, 40%).

¹H NMR (300 MHz, DMSO-d6): δ = 0.68 (t, J = 7.5 Hz, 3 H), 1.35-1.51 (m, 1 H), 1.72-1.85 (m, 1 H), 2.56 (dd, J = 6.0, 18.3 Hz, 1 H), 2.90 (dd, J = 12.3, 18.3 Hz, 1 H), 3.77 (dd, J = 6.0, 12.3 Hz, 1 H), 4.53 (s, 1 H), 4.70 (s, 1 H), 4.78 (s, 1 H), 6.61 (d, J = 1.8 Hz, 1 H), 6.62 (d, J = 6 Hz, 1 H), 6.74 (s, 1 H), 7.04-7.13 (m, 2 H), 7.18(br d, J = 8.6 Hz, 1 H), 7.35 (d, J = 8.6 Hz, 1 H), 7.93 (s, 1 H), 10.45 (s, 1 H) ppm.

### Example 81a

### Preparation of intermediate racemic (2'R, 3R, 4'S)-6-chloro-4'-(3-chlorophenyl)-2'-(1-ethyl-propenyl)-2,3-dihydro-2,6'-dioxo-spiro[indole-3,3'-piperidine]-1-methoxyethyl trimethylsilane

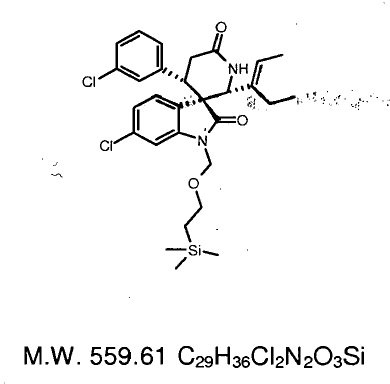

In a manner similar to the method described in example 55b, E/Z-6-chloro-3-(3-chloro-benzylidene)-1-(2-trimethylsilanyl-ethoxymethyl)-1,3-dihydro-indole-2-one (5.4 g, 12.8 mmol) prepared in example 55a was reacted with 1-(1-ethyl-propenyl)-3-trimethylsilyoxy-2-aza-1,3-butadiene (20 g, 95 mmol) prepared in example 66a in toluene (200 mL) to give racemic (2'R, 3R, 4'S)-6-chloro-4'-(3-chloro-phenyl)-2'-(1-ethyl-propenyl)-2,3-dihydro-2,6'-dioxo spiro[indole-3,3'-piperidine]-1-methoxyethyl trimethylsilane as a white gum (Yield 6.1 g, 85%).

### Example 81b

### Preparation of intermediate racemic (2'R, 3R, 4'S)-1'-[(tert-butoxycarbonyl) methyl]-6-chloro-4'-(3-chlorophenyl)-2'-(1-ethyl-propenyl)-2,3-dihydro-2,6'-dioxo spiro[indole-3,3'-piperidine]-1-methoxyethyl trimethylsilane

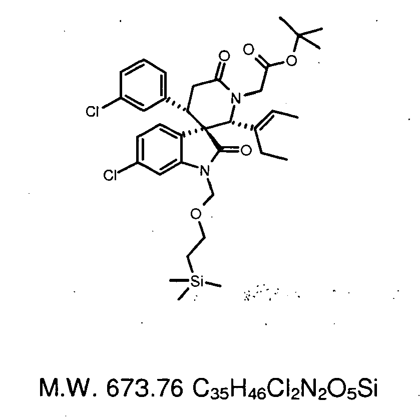

In a manner similar to the method described in example 55c, racemic (2'R, 3R, 4'S)-6-chloro-4'-(3-chloro-phenyl)-2'-(1-ethyl-propenyl)-2,3-dihydro-2,6'-dioxo spiro[indole-3,3'-piperidine]-1-methoxyethyl trimethylsilane (1.6 g, 2.8 mmol) prepared in example 81a was reacted with bromo-acetic acid tert-butyl ester and cesium carbonate in N,N-dimethylformamide to give racemic (2'R, 3R, 4'S)-1'-[(*tert*-butoxycarbonyl) methyl]-6-chloro-4'-(3-chlorophenyl)-2'-(1-ethyl-propenyl)-2,3-dihydro-2,6'-dioxo spiro[indole-3,3'-piperidine]-1-methoxyethyl trimethylsilane as a white foam (Yield 0.7 g, 37%).

### Example 81c

### Preparation of racemic (2'R, 3R, 4'S)-6-chloro-4'-(3-chlorophenyl)-2'-(1-ethyl-propenyl)-1'-hydroxycarbonylmethyl-spiro[3H-indole-3,3'-piperidine]-2,6'(1H)-dione

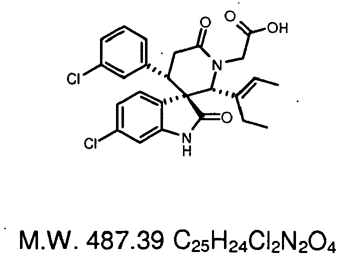

In a manner similar to the method described in example 55d, racemic (2'R, 3R, 4'S)-1'-[(*tert*-butoxycarbonyl) methyl]-6-chloro-4'-(3-chlorophenyl)-2'-(1-ethyl-propenyl)-2,3-dihydro-2,6'-dioxo spiro[indole-3,3'-piperidine]-1-methoxyethyl trimethylsilane (0.6 g, 0.89 mmol) prepared in example 81b was reacted to form racemic (2'R, 3R, 4'S)-6-chloro-4'-(3-chlorophenyl)-2'-(1-ethyl-propenyl)-1'-hydroxycarbonylmethyl-spiro[3H-indole-3,3'-piperidine]-2,6'(1H)-dione as a white solid (0135 g, 80%).

HRMS(ES⁺) *m*/*z* Calcd for C₂₅H₂₄Cl₂N₂O₄ +H [(M+H)⁺]: 487.1186, Found: 487.1186 .

### Example 82a

### Preparation of intermediate racemic (2'R, 3R, 4'S)-6-chloro-4'-(3-chlorophenyl)-2'-(1-ethyl-propenyl)-1'-fluorocarbonylmethyl spiro[3H-indole-3,3'-piperidine]-2,6'(1H)-dione

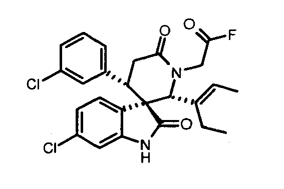

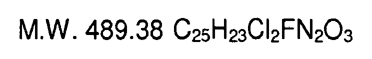

In a manner similar to the method described in example 34a, racemic (2'R, 3R,4'S)-6-chloro-4'-(3-chlorophenyl)-2'-(1-ethyl-propenyl)-1-hydroxycarbonyl-methyl spiro[3H-indole-3,3'-piperidine]-2,6'(1H)-dione (0.16 g, 0.33 mmol) prepared in example 81c was reacted with cyanuric fluoride (0.044 mL, 1.64 mmol) (Alfa) and pyridine (0.13 g, 1.64 mmol) in dichloromethane to give racemic (2'R, 3R, 4'S)-6-chloro-4'-(3-chlorophenyl)-2'-(1-ethyl-propenyl)-1'-fluorocarbonylmethyl-spiro[3H-indole-3,3'-piperidine]-2,6'(1H)-dione (yield: 0.12 g, 75%).

### Example 82b

### Preparation of racemic (2'R, 3R, 4'S)-6-chloro-4'-(3-chlorophenyl)-2-(1-ethyl-propenyl)-1'-[(2-hydroxy-1,1-dimethylethyl)aminocarbonyl-methyl] spiro[3H-indole-3,3'-piperidine]-2,6'(1H)-dione

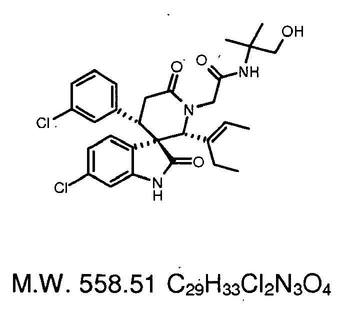

In a manner similar to the method described in example 34b, racemic (2'R, 3R, 4'S)-6-chloro-4'-(3-chlorophenyl)- 2'-(1-ethyl-propenyl)-1'-fluorocarbonylmethyl-spiro[3H-indole-3,3'-piperidine]-2,6'(1H)-dione (0.12 g, 0.24 mmol) prepard in example 82a was reacted with 2-amino-2-methyl-propan-1-ol (73 mg, 0.82 mmol), N-methylmorpholine (0.2 g, 1.98 mmol) and 4-dimethylaminopyridine (2 mg, 0.016 mmol) in tetrahydrofuran to give racemic racemic (2'R, 3R, 4'S)-6-chloro-4'-(3-chlorophenyl)-2'-(1-ethyl-propenyl)-1'-[(2-hydroxy-1,1-dimethylethyl)aminocarbonyl-methyl] spiro[3H-indole-3,3'-piperidine]-2,6'(1H)-dione as an off-white solid (Yield 92 mg, 67%).

HRMS(ES⁺) *m*/*z* Calcd for C₂₉H₃₃Cl₂N₃O₄ +H [(M+H)⁺]: 558.1921. Found: 558.1921

### Example 82c

### Preparation of chiral (2'R, 3R, 4'S) -6-chloro-4'-(3-chlorophenyl)-2'-(1-ethyl-propenyl)-1'-[(2-hydroxy-1,1-dimethylethyl)aminocarbonyl-methyl] spiro[3H-indole-3,3'-piperidine]-2,6'(1H)-dione

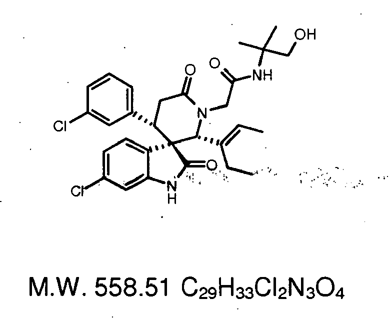

Separation of the two enantiomers from racemic (2'R, 3R, 4'S) -6-chloro-4'-(3-chlorophenyl)-2'-(1-ethyl-propenyl)-1'-[(2-hydroxy-1-,1-dimethylethyl) aminocarbonyl-methyl] spiro[3H-indole-3,3'-piperidine]-2,6'(1H)-dione (50 mg) prepared in example 82b was conducted by chiral SFC to provide chiral (2'R, 3R, 4'S-6-chloro-4'-(3-chlorophenyl)-2'-(1-ethyl-propenyl)-1'-[(2-hydroxy-1,1-dimethylethyl)aminocarbonyl-methyl] spiro[3H-indole-3,3'-piperidine]-2,6'(1H)-dione as a white solid (Yield: 21 mg, 42%) and chiral (2'S, 3S, 4'R) -6-chloro-4'-(3-chlorophenyl)-2'-(1-ethyl-propenyl)-1'-[(2-hydroxy-1,1-dimethylethyl)aminocarbonyl-methyl] spiro[3H-indole-3,3'-piperidine]-2,6'(1H)-dione as a white solid (Yield: 19 mg, 38%).

### Example 83a

### Preparation of intermediate racemic (2'R, 3R, 4'S)-6-chloro-4'-(3-chloro-phenyl)-1'-(3-chloro-propyl)-2'-(1-ethyl-propenyl)-2,3-dihydro-2,6'-dioxo spiro[indole-3,3'-piperidine]-1-methoxyethyl trimethylsilane

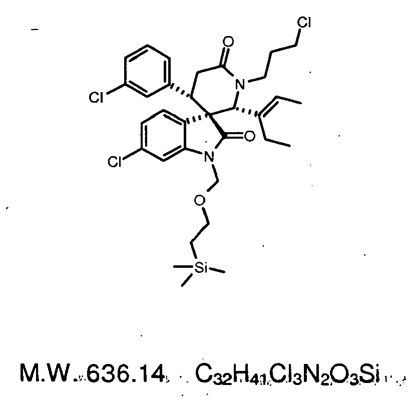

In a manner similar to the method described in example 24c, racemic (2'R, 3R, 4'S)-6-chloro-4'-(3-chloro-phenyl)-2'-(1-ethyl-propenyl)-2,3-dihydro-2,6'-dioxo spiro[indole-3,3'-piperidine]-1-methoxyethyl trimethylsilane (2.4 g, 4.3 mmol) prepared in example 81a was reacted with LiH (1 g, 125 mmol) and 1-chloro-3-iodo-propane (8 g, 39 mmol) in N,N-dimethyl-formamide (40 mL) to give racemic (2'R, 3R, 4'S)-6-chloro-4'-(3-chloro-phenyl)-1'-(3-chloro-propyl)-2'-(1-ethyl-propenyl)-2,3-dihydro-2,6'-dioxo spiro[indole-3,3'-piperidine]-1-methoxyethyl trimethylsilane as a white form (Yield: 0.61 g, 22%).

### Example 83b

### Preparation of racemic (2'R, 3R, 4'S)-6-chloro-4'-(3-chlorophenyl)-2'-(1-ethyl-propenyl)-1'-(3-morpholin-4-yl-propyl) spiro[3H-indole-3,3'-piperidine]-2,6'(1H)-dione

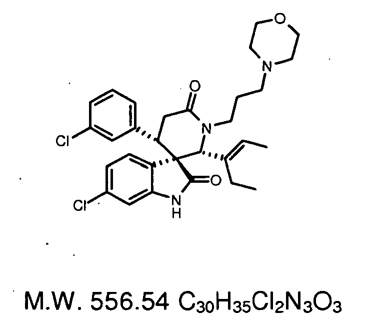

In a manner similar to the method described in example 60b, racemic (2'R, 3R, 4'S)-6-chloro-4'-(3-chloro-phenyl)-1'-(3-chloro-propyl)-2'-(1-ethyl-propenyl)-2,3-dihydro-2,6'-dioxo spiro[indole-3,3'-piperidine]-1-methoxyethyl trimethylsilane prepared in example 83a (0.3 g, 0.47 mmol) was reacted with morpholine (12 mL), trifluroacetic acid (5 mL) and then N,N-diisopropylethylamine (2 mL) to give racemic (2'R, 3R, 4'S)-6-chloro-4'-(3-chlorophenyl)-2'-(1-ethyl-propenyl)-1'-(3-morpholin-4-yl-propyl)spiro[3H-indole-3,3'-piperidine]-2,6'(1H)-dione as a white solid (0.15 g, 57% ).

HRMS(ES⁺) *m*/*z* Calcd for C₃₀H₃₅Cl₂N₃O₃ + H [(M+H)⁺]: 556.2128. Found: 556.2123.

### Example 83c

### Preparation of chiral (2'R, 3R, 4'S)-6-chioro-4'-(3-chlorophenyl)-2'-(1-ethyl-propenyl)-1'-(3-morpholin-4-yl-propyl) spiro[3H-indole-3,3'-piperidine]-2,6'(1H)-dione

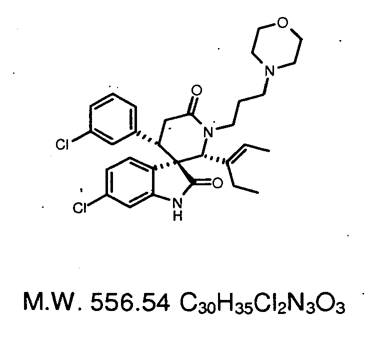

Separation of the two enantiomers from racemic (2'R, 3R, 4'S)-6-chloro-4'-(3-chlorophenyl)-2'-(1-ethyl-propenyl)-1'-(3-morpholin-4-yl-propyl) spiro[3H-indole-3,3'-piperidine]-2,6'(1H)-dione (150 mg) prepared in example 83b was conducted by chiral SFC to provide chiral (2'R, 3R, 4'S)-6-chloro-4'-(3-chlorophenyl)-2'-(1-ethyl-propenyl)-1'-(3-morpholin-4-yl-propyl) spiro[3H-indole-3,3'-piperidine]-2,6'(1H)-dione as a white solid (Yield: 61 mg, 41%) and chiral (2'S, 3S, 4'R)-6-chloro-4'-(3-chlorophenyl)-2'-(1-ethyl-propenyl)-1'-(3-morpholin-4-yl-propyl) spiro[3H-indole-3,3'-piperidine]-2,6'(1H)-dione as a white solid (Yield: 63 mg, 42%).

### Example 84a

### Preparation of intermediate 3-Methyl-2-methylene-butan-1-ol

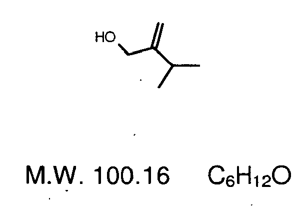

In a manner similar to the method described in example 79a, isopropyl magnesium chloride (2M in ether, 300 mL, 0.6 mol) was reacted with propargyl alcohol (11.2 g, 0.2 mol, Aldrich) and Cul (32g, 0.2mol) to give 3-methyl-2-methylene-butan-1-ol as a colorless oil (Yield, 2.4 g, 12%)

### Example 84b

### Preparation of intermediate 3-Methyl-2-methylene-butyraldehyde

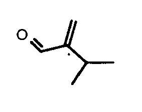

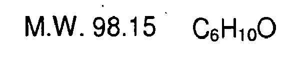

In a manner similar to the method described in example 79b, 3-methyl-2-methylene-butan-1-ol (2.4 g, 24 mmol) prepared in example 84a was oxidized to give 3-methyl-2-methylene-butyraldehyde as a yellow oil (Yield: 1.6 g, 68%)

### Example 84c

### Preparation of intermediate 1-(2-methyl-1-methylene-propyl)-3-trimethylsilyoxy-2-aza-1,3-butadiene

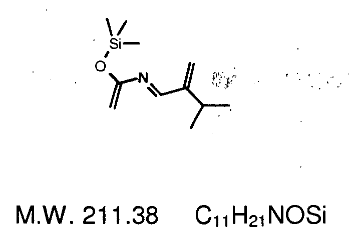

In a manner similar to the method described in example 1b, 3-methyl-2-methylene-butyraldehyde prepared in example 84b (1.3 g, 13 mmol) was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.36 g, 13 mmol) and acetyl chloride (1.02 g, 13 mmol) to give 1-(2-methyl-1-methylene-propyl)-3-trimethylsilyoxy-2-aza-1,3-butadiene and used for the next step without further purification.

### Example 84d

### Preparation of racemic (2'R, 3R, 4'S)-6-chloro-4'-(3-chlorophenyl)-2'-(2-methyl-1-methylene-propyl) spiro[3H-indole-3,3'-piperidine]-2,6'(1H)-dione

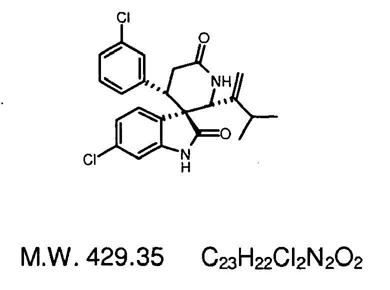

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.41 g, 1.1 mmol) was reacted with 1-(2-methyl-1-methylene-propyl)-3-trimethylsilyoxy-2-aza-1,3-butadiene (2.1 g, 10 mmol) prepared in example 84c in toluene and then trifluoroacetic acid (10 mL) in dichloromethane to give racemic (2'R, 3R, 4'S)-6-chloro-4'-(3-chlorophenyl)-2'-(2 -methyl-1-methylene-propyl) spiro[3H-indole-3,3'-piperidine]-2,6'(1H)-dione as a off white solid (0.13 g, 28%).

HRMS(ES⁺) *m*/*z* Calcd for C₂₃H₂₂Cl₂N₂O₂ + H [(M+H)⁺]: 429.1131. Found: 429.1133

### Example 85a

### Preparation of intermediate 3,3-Dimethyl-2-methylene-butan-1-ol

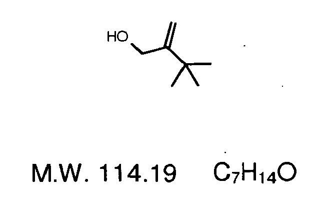

In a manner similar to the method described in example 79a, tert-butyl magnesium chloride (2 M in ether, 300 mL, 0.6 mol) was reacted with propargyl alcohol (11.2 g, 0.2 mol, Aldrich) and Cul (32g, 0.2mol) to give 3,3-dimethyl-2-methylene-butan-1-ol as a plae yellow oil (Yield, 12.3 g, 54%)

### Example 85b

### Preparation of intermediate 3,3-Dimethyl-2-methylene-butyraldehyde

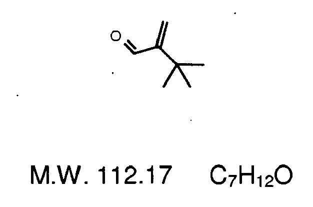

In a manner similar to the method described in example 79b, 3,3-dimethyl-2-methylene-butan-1-ol (12.3 g, 0.11 mol) prepared in example 85a was oxidized to give 3,3-dimethyl-2-methylene-butyraldehyde as a yellow oil (Yield: 8.3 g, 67%)

### Example 85c

### Preparation of intermediate 1-(2, 2-dimethyl-1-methylene-propyl)-3-trimethylsilyoxy-2-aza-1,3-butadiene

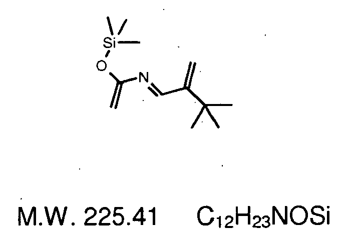

In a manner similar to the method described in example 1b, 3,3-dimethyl-2-methylene-butyraldehyde prepared in example 85 b (2.2 g, 20 mmol) was used as the starting material in place of 3-chloro-benzaldehyde to react with 1,1,1,3,3,3-hexamethyldisilazane (3.2 g, 20 mmol), *n*-butyllithium (2.5 M, 8 mL, 20 mmol), trimethylsilyl chloride (2.2 g, 20 mmol), triethylamine (2.7 g, 27 mmol) and acetyl chloride (2 g, 27 mmol) to give 1-(2, 2-dimethyl-1-methylene-propyl)-3-trimethylsilyoxy-2-aza-1,3-butadiene and used for the next step without further purification.

### Example 85d

### Preparation of racemic (2'R, 3R, 4'S)-6-chloro-4'-(3-chlorophenyl)-2'-(2,2-dimethyl-1-methylene-propyl) spiro[3H-indole-3,3'-piperidine]-2,6'(1H)-dione

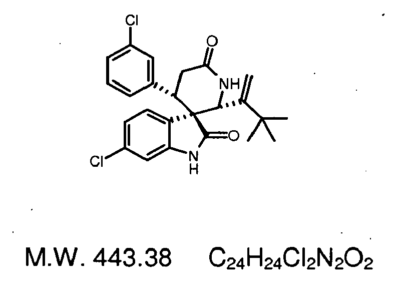

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.41 g, 1.1 mmol) was reacted with 1-(2, 2-dimethyl-1-methylene-propyl)-3-trimethylsilyoxy-2-aza-1,3-butadiene (3.4 g, 15 mmol) prepared in example 85c in toluene and then trifluoroacetic acid (10 mL) in dichloromethane to give racemic (2'R, 3R, 4'S)-6-chloro-4'-(3-chlorophenyl)-2'-(2,2-dimethyl-1-methylene-propyl) spiro[3H-indole-3,3'-piperidine]-2,6'(1H)-dione as a off white solid (0.27 g, 55%).

HRMS(ES⁺) *m*/*z* Calcd for C₂₄H₂₄Cl₂N₂O₂ + H [(M+H)⁺]: 443.1288. Found: 443.1288

### Example 86

### Preparation of racemic (2'R, 3R, 4'S)-6-chloro-4'-(3-chlorophenyl)-2'-(1-ethyl-propenyl)-1'-[(2-morpholin-4-yl-ethyl)aminocarbonyl-methyl] spiro[3H-indole-3,3'-piperidine]-2,6'(1H)-dione

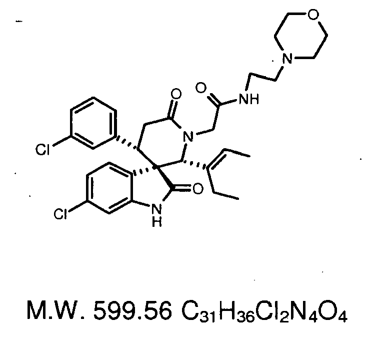

In a manner similar to the method described in example 34b, racemic (2'R, 3R, 4'S)-6-chloro-4'-(3-chlorophenyl)-1'-fluorocarbonylmethyl-2'-(1-ethyl-propenyl) spiro[3H-indole-3,3'-piperidine]-2,6'(1H)-dione (0.12 g, 0.24 mmol) prepard in example 83a was reacted with 4-(2-aminoethyl)morpoline (85 mg, 0.66 mmol) (Aldrich), N-methylmorpholine (0.1 g, 0.98 mmol) and 4-dimethylaminopyridine (2 mg, 0.015 mmol) in tetrahydrofuran to give racemic (2'R, 3R, 4'S)-6-chloro-4'-(3-chlorophenyl)-2'-(1-ethyl-propenyl)-1'-[(2-morpholin-4-yl-ethyl)aminocarbonyl-methyl] spiro[3H-indole-3,3'-piperidine]-2,6'(1H)-dione as an off-white solid (Yield 31 mg, 22%).

HRMS(ES⁺) *m*/*z* Calcd for C₃₁H₃₆Cl₂N₄O₄ + H [(M+H)⁺]: 599.2187. Found: 599.2185

### Example 87a

### Preparation of intermediate 1- isopropenyl-3-trimethylsilyoxy-2-aza-1,3-butadiene

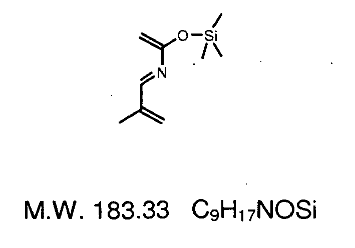

In a manner similar to the method described in example 1b, 2-methacrolein (2 g, 20 mmol) (Acros) was used as the starting material in place of 3-chloro-benzaldehyde to react with 1,1,1,3,3,3-hexamethyldisilazane (3.2 g, 20 mmol), *n-*butyllithium (2.5 M, 8 mL, 20 mmol), trimethylsilyl chloride (2.2 g, 20 mmol), triethylamine (2.7 g, 27 mmol) and acetyl chloride (2.0 g, 27 mmol) to give 1-isopropenyl-3-trimethylsilyoxy-2-aza-1,3-butadiene and used for the next step without further purification.

### Example 87b

### Preparation of racemic (2'R, 3R, 4'S)-6-chloro-4'-(3-chlorophenyl)-2'-isopropenyl spiro[3H-indole-3,3'-piperidine]-2,6'(1H)-dione

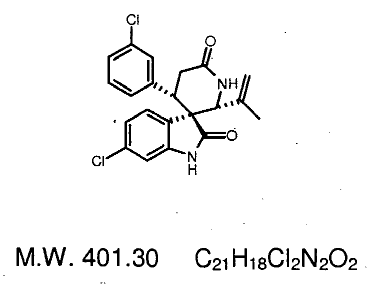

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.41 g, 1.1 mmol) was reacted with 1-isopropenyl-3-trimethylsilyoxy-2-aza-1,3-butadiene (3.4 g, 18 mmol) prepared in example 87a in toluene and then trifluoroacetic acid (20 mL) in dichloromethane to give racemic (2'R, 3R, 4'S)-6-chloro-4'-(3-chlorophenyl)-2'-(1-methylene-propyl) spiro[3H-indole-3,3'-piperidine]-2,6'(1H)-dione as a white solid (0.27 g, 61%).

HRMS(ES⁺) *m*/*z* Calcd for C₂₁H₁₈Cl₂N₂O₂+ H [(M+H)⁺]: 401.0818. Found: 401.0818

### Example 87c

### Preparation of chiral (2'R, 3R, 4'S)-6-chloro-4'-(3-chlorophenyl)-2'-isopropenyl spiro[3H-indole-3,3'-piperidine]-2,6'(1H)-dione

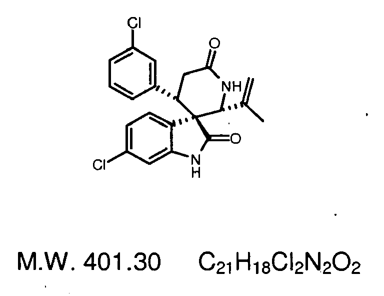

Separation of the two enantiomers from racemic (2'R, 3R, 4'S)-6-chloro-4'-(3-chlorophenyl)-2'-isopropenyl spiro[3H-indole-3,3'-piperidine]-2,6'(1H)-dione (80 mg) prepared in example 87b was conducted by chiral SFC to provide chiral (2'R, 3R, 4'S)-6-chloro-4'-(3-chlorophenyl)-2'-isopropenyl spiro[3H-indole-3,3'-piperidine]-2,6'(1H)-dione as a white solid (28 mg, 35%) and chiral (2'S, 3S, 4'R)-6-chloro-4'-(3-chlorophenyl)-2'-isopropenyl spiro[3H-indole-3,3'-piperidine]-2,6'(1H)-dione as a white solid (26 mg, 33%).

¹H NMR (300 MHz, DMSO-*d6*): δ = 1.24 (s, 3 H), 2.55 (dd, J = 6.0, 18.0 Hz, 1 H), 2.89 (dd, J = 13.2, 18.0 Hz, 1 H), 3.75 (dd, J = 6.0, 13.2 Hz, 1 H), 4.49 (s, 1 H), 4.71 (s, 1 H), 4.77 (s, 1 H), 6.60-6.63 (m, 2 H), 6.74 (t, J = 1.8 Hz, 1 H), 7.04-7.12 (m, 2 H), 7.17 (br d, J = 8.1 Hz, 1 H); 7.34 (d, J = 8.1 Hz, 1 H), 8.00 (s, 1 H), 10.44 (s, 1H) ppm.

### Example 88a

### Preparation of intermediate 2-Methylene-pentan-1-ol

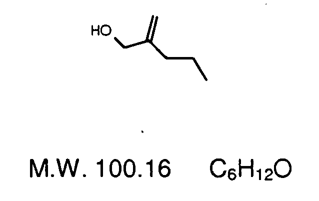

In a manner similar to the method described in example 79a, propylmagnesium chloride (2 M in ether, 375 mL, 0.75 mol) was reacted with propargyl alcohol (14 g, 0.25 mol)and Cul (40g, 0.25mol) to give 2-methylene-pentan-1-ol as a colorless oil (Yield, 16.9 g, 67%)

### Example 88b

### Preparation of intermediate 2-Methylene-pentanal

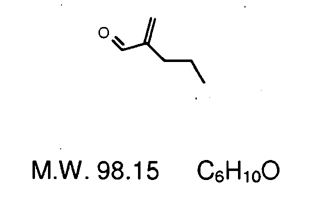

In a manner similar to the method described in example 79b, 2-methylene-pentan-1-ol (16.5 g, 0.165 mol) prepared in example 88a was oxidized to give 2-methylene-pentanal as a yellow oil (Yield: 6.7 g, 41%)

### Example 88c

### Preparation of intermediate 1-(1-methylene-butyl)-3-trimethylsilyoxy-2-aza-1,3-butadiene

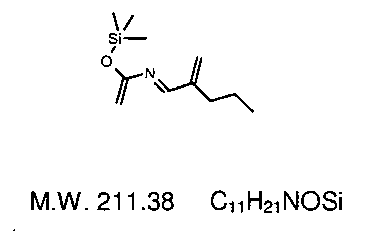

In a manner similar to the method described in example 1b, 2-methylene-pentanal prepared in example 88b (2.1 g, 21 mmol) was used as the starting material in place of 3-chloro-benzaldehyde to react with 1,1,1,3,3,3-hexamethyldisilazane (3.2 g, 20 mmol), *n-*butyllithium (2.5 M, 8 mL, 20 mmol), trimethylsilyl chloride (2.2 g, 20 mmol), triethylamine (2.7 g, 27 mmol) and acetyl chloride (2 g, 27 mmol) to give 1-(1-methylene-butyl)-3-trimethylsilyoxy-2-aza-1,3-butadiene and used for the next step without further purification.

### Example 88d

### Preparation of racemic (2'R, 3R, 4'S)-6-chloro-4'-(3-chlorophenyl)-2'-(1-methylene-butyl) spiro[3H-indole-3,3'-piperidine]-2,6'(1H)-dione

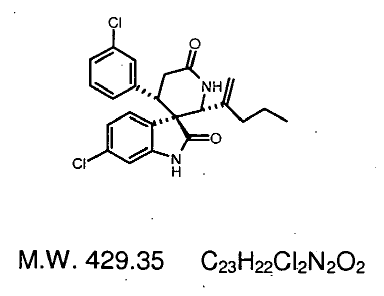

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.45 g, xx mmol) was reacted with 1-(1-methylene-butyl)-3-trimethylsilyoxy-2-aza-1,3-butadiene (0.45 g, 1.1mmol) prepared in example 88c in toluene and then trifluoroacetic acid (10 mL) in dichloromethane to give racemic (2'R, 3R, 4'S)-6-chloro-4'-(3-chlorophenyl)-2'-(1-methylene-butyl) spiro[3H-indole-3,3'-piperidine]-2,6'(1H)-dione as a off white solid (0.34 g, 72% ).

HRMS(ES⁺) *m*/*z* Calcd for C₂₃H₂₂Cl₂N₂O₂ + H [(M+H)⁺]: 429.1131. Found: 429.1131

### Example 88e

### Preparation of chiral (2'R, 3R, 4'S)-6-chloro-4'-(3-chlorophenyl)-2'-(1-methylene-butyl) spiro[3H-indole-3,3'-piperidine]-2,6'(1H)-dione

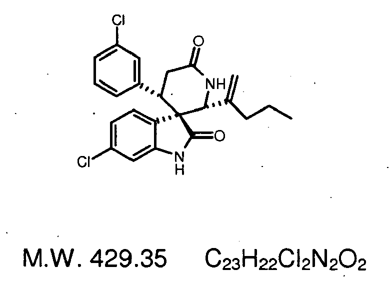

Separation of the two enantiomers from racemic (2'R, 3R, 4'S)-6-chloro-4'-(3-chlorophenyl)-2'-(1-methylene-butyl) spiro[3H-indole-3,3'-piperidine]-2,6'(1H)-dione (90 mg) prepared in example 88d was conducted by chiral SFC to provide chiral (2'R, 3R, 4'S)-6-chloro-4'-(3-chlorophenyl)-2'-(1-methylene-butyl) spiro[3H-indole-3,3'-piperidine]-2,6'(1H)-dione as a white solid (32 mg, 36%) and chiral (2'S, 3S, 4'R)-6-chloro-4'-(3-chlorophenyl)-2'-(1-methylene-butyl) spiro[3H-indole-3,3'-piperidine]-2,6'(1H)-dione as a white solid (30 mg, 33%).

### Example 89a

### Preparation of intermediate 4-Methyl-2-methylene-pentan-1-ol

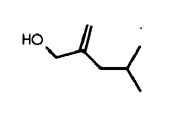

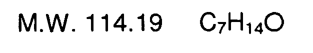

In a manner similar to the method described in example 79a, isobutyl magnesium chloride (2 M in ether, 375 mL, 0.75 mol) was reacted with propargyl alcohol (14 g, 0.25 mol) and Cul (40g, 0.25mol) to give 4-methyl-2-methylene-pentan-1-ol as a colorless oil (Yield, 27 g, 95%)

### Example 89b

### Preparation of intermediate 4-Methyl-2-methylene-pentanal

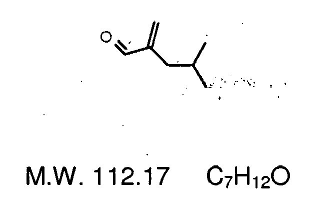

In a manner similar to the method described in example 79b, 4-methyl-2-methylene-pentan-1-ol prepared in example 89a was oxidized to give 4-methyl-2-methylene-pentanal as a yellow oil (Yield: 21 g, 77%)

### Example 89c

### Preparation of intermediate 1-(3-Methyl-1-methylene-butyl)-3-trimethylsilyoxy-2-aza-1,3-butadiene

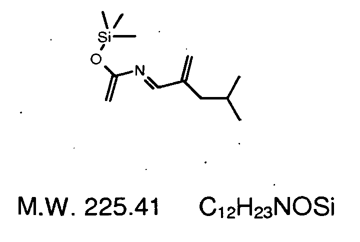

In a manner similar to the method described in example 1b, 4-methyl-2-methylene-pentanal prepared in example 89b (11 g, 100 mmol) was used as the starting material in place of 3-chloro-benzaldehyde to react with 1,1,1,3,3,3-hexamethyldisilazane (16 g, 100 mmol), *n*-butyllithium (2.5 M, 40 mL, 100 mmol), trimethylsilyl chloride (11 g, 100 mmol), triethylamine (13.6 g, 14 mmol) and acetyl chloride (10.2 g, 14 mmol) to give 1-(3-methyl-1-methylene-butyl)-3-trimethylsilyoxy-2-aza-1,3-butadiene and used for the next step without further purification.

### Example 89d

### Preparation of racemic (2'R, 3R, 4'S)-6-chloro-4'-(3-chlorophenyl)-2'-(3-methyl-1-methylene-butyl) spiro[3H-indole-3,3'-piperidine]-2,6'(1H)-dione

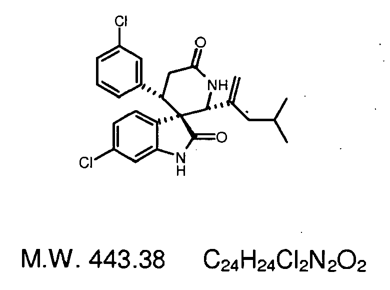

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (2 g, 5 mmol) was reacted with 1-(3-methyl-1-methylene-butyl)-3-trimethylsilyoxy-2-aza-1,3-butadiene (21 g, 93 mmol) prepared in example 89c in toluene and then trifluoroacetic acid (20 mL) in dichloromethane to give racemic (2'R, 3R, 4'S)-6-chloro-4'-(3-chlorophenyl)-2'-(3-methyl-1-methylene-butyl) spiro[3H-indole-3,3'-piperidine]-2,6'(1H)-dione as a white solid (1.3 g, 59% ).

HRMS(ES⁺) *m*/*z* Calcd for C₂₄H₂₄Cl₂N₂O₂ + H [(M+H)⁺]: 443.1288. Found: 443.1285

### Example 89e

### Preparation of chiral (2'R, 3R, 4'S)-6-chloro-4'-(3-chlorophenyl)-2'-(3-methyl-1-methylene-butyl) spiro[3H-indole-3,3'-piperidine]-2,6'(1H)-dione

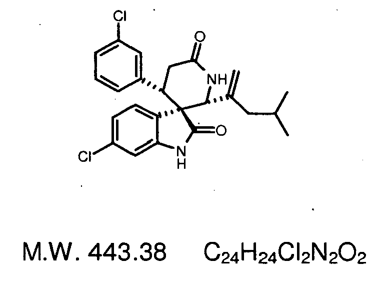

Separation of the two enantiomers from racemic (2'R, 3R, 4'S)-6-chloro-4'-(3-chlorophenyl)-2'-(3-methyl-1-methylene-butyl) spiro[3H-indole-3,3'-piperidine]-2,6'(1H)-dione (300 mg) prepared in example 89d was conducted by chiral SFC to provide chiral (2'R, 3R, 4'S)-6-chloro-4'-(3-chlorophenyl)-2'-(3-methyl-1-methylene-butyl) spiro[3H-indole-3,3'-piperidine]-2,6'(1H)-dione as a white solid (130 mg, 43%) and chiral (2'S, 3S, 4'R)-6-chloro-4'-(3-chlorophenyl)-2'-(1-methylene-butyl) spiro[3H-indole-3,3'-piperidine]-2,6'(1H)-dione as a white solid (130 mg, 43%).

### Example 90

### Preparation of racemic (2'R, 3R, 4'S)-6-chloro-4'-(3-chlorophenyl)-2'-(1-methylene-butyl)-6'-thioxo spiro[3H-indole-3,3'-piperidine]-2(1H)-one

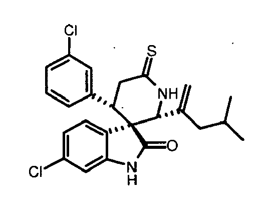

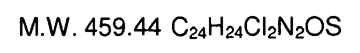

In a manner similar to the method described in example 30, racemic (2'R, 3R, 4'S)-6-chloro-4'-(3-chlorophenyl)-2'-(1-methylene-butyl) spiro[3H-indole-3,3'-piperidine]-2,6'(1H)-dione (1.4 g, 3.1 mmol) prepared in example 89d was reacted with 2,4-bis-(4-methoxyphenyl)-1,3-dithia-2,4-diphosphetane 2,4-disulfide (1.7 g, 4.25 mmol) in toluene to give racemic (2'R, 3R, 4'S)-6-chloro-4'-(3-chlorophenyl)-2'-(1-methylene-butyl)-6'-thioxo spiro[3H-indole-3,3'-piperidine]-2(1H)-one as a off white solid (Yield 1.2 g, 84%).

HRMS(ES⁺) *m*/*z* Calcd for C₂₄H₂₄Cl₂N₂OS +H [(M+H)⁺]: 459.1059. Found: 459.1055.

### Example 91a

### Preparation of intermediate 4-Ethoxy-1,2-difluoro-benzene

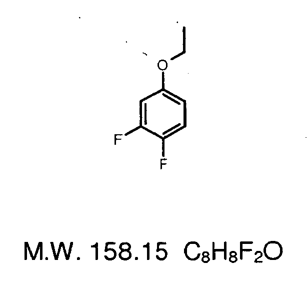

To a solution of 3,4-difluoro-phenol (10 g, 77 mmol) in N,N-dimethylformamide (50 mL) was added potassium carbonate (20 g, 145 mmo) and iodoethane (50 g, 320 mmol, Aldrich). The reaction mixture was heated at refluxing for 48 h. The crude was cooled to room temperature and filtered through a short pad of celite. The filtrate was concentrated and the residue was purified by chromatography (EtOAc:Hexanes=1:8) to give 4-ethoxy-1,2-difluoro-benzene as colorless oil (Yield 11 g, 90%).

### Example 91b

### Preparation of intermediate 6-Ethoxy-2,3-difluoro-benzaldehyde

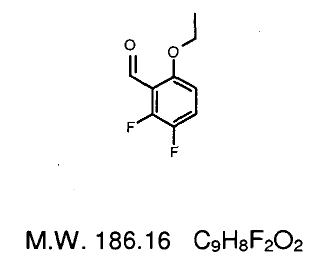

In a manner similar to the method described in example 52a, 4-ethoxy-1,2-difluoro-benzene (10 g, 63 mmol) prepared in example 91a was reacted with lithium diisopropylamine (39 mL, 1.8 M in THF, 70 mmol), N,N-dimethylformamide (5.88 mL, 76 mmol) and quenched with acetic acid (15.2 g, 253 mmol) in tetrahydrofuran to give 6-ethoxy-2,3-difluoro-benzaldehyde as a off white solid (Yield: 8.9 g, 76%).

### Example 91c

### Preparation of intermediate 1-(4-ethoxy-1,2-difluoro-phenyl)-3-trimethylsilyoxy-2-aza-1,3-butadiene

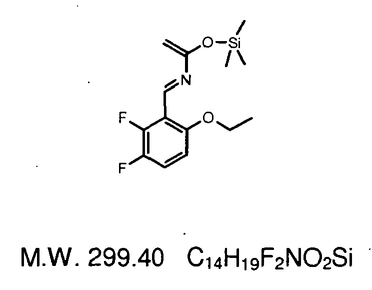

In a manner similar to the method described in example 1b, 6-ethoxy-2,3-difluoro-benzaldehyde (1.9 g, 11 mmol) prepared in example 91b was used as the starting material in place of 3-chloro-benzaldehyde to react with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-(4-ethoxy-1,2-difluoro-phenyl)-3-trimethylsilyoxy-2-aza-1,3-butadiene as yellow oil and used for the next step without further purification.

### Example 91d

### Preparation of racemic (2'R, 3R, 4'S)-6-chloro-4'-(3-chlorophenyl)-2'-(4-ethoxy-1,2-difluoro-phenyl) spiro[3H-indole-3,3'-piperidine]-2,6'(1H)-dione

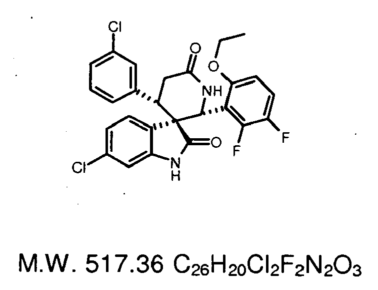

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.6 g, 1.5 mmol) was reacted with 1-(4-ethoxy-1,2-difluoro-phenyl)-3-trimethylsilyoxy-2-aza-1,3-butadiene (2.3 g, 7.6 mmol) prepared in example 91 c in toluene and then trifluoroacetic acid (10 mL) in dichloromethane to give racemic (2'R, 3R, 4'S)-6-chloro-4'-(3-chlorophenyl)-2'-(4-ethoxy-1,2-difluoro-phenyl) spiro[3H-indole-3,3'-piperidine]-2,6'(1H)-dione as a white solid (0.39 g, 50%).

HRMS(ES⁺) *m*/*z* Calcd for C₂₆H₂₀Cl₂F₂N₂O₃ + H [(M+H)⁺]: 517.0892. Found: 517.0889.

### Example 91e

### Preparation of chiral (2'R, 3R, 4'S)-6-chloro-4'-(3-chlorophenyl)-2'-(4-ethoxy-1,2-difluoro-phenyl) spiro[3H-indole-3,3'-piperidine]-2,6'(1H)-dione

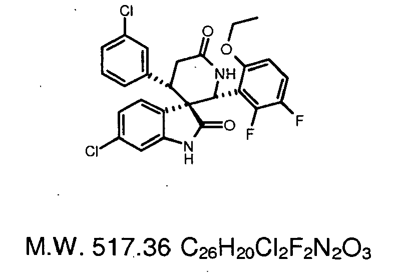

Separation of the two enantiomers from (2'R, 3R, 4'S)-6-chloro-4'-(3-chlorophenyl)-2-(4-ethoxy-1,2-difluoro-phenyl) spiro[3H-indole-3,3'-piperidine]-2,6'(1H)-dione (98 mg) prepared in example 91 d was conducted by chiral SFC to provide chiral (2'R, 3R, 4'S)-6-chloro-4'-(3-chlorophenyl)-2'-(4-ethoxy-1,2-difluoro-phenyl) spiro[3H-indole-3,3'-piperidine]-2,6'(1H)-dione as a white solid (36 mg, 36%) and chiral (2'S, 3S, 4'R)-6-chloro-4'-(3-chlorophenyl)-2'-(4-ethoxy-1,2-difluoro-phenyl) spiro[3H-indole-3,3'-piperidine]-2,6'(1H)-dione as a white solid (33 mg, 33%).

### Example 92a

### Preparation of intermediate E/Z-3-Chloro-5-(6-chloro-2-oxo-1,2-dihydro-indol-3-ylidenemethyl)-benzoic acid methyl ester

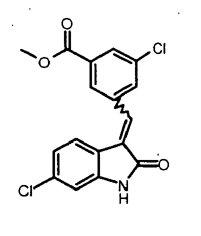

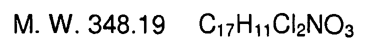

In a manner similar to the method described in example 1a, 6-chlorooxindole (3.2 g, 18 mmol) was reacted with 3-chloro-5-formyl-benzoic acid methyl ester (3.92 g, 18 mmol) and pyrrolidine (1.3 g, 18 mmol) in methanol to give a mixture of E-and Z-3-Chloro-5-(6-chloro-2-oxo-1,2-dihydro-indol-3-ylidenemethyl)-benzoic acid methyl ester as a yellow solid (Yield: 6.1 g, 97%).

3-chloro-5-formyl-benzoic acid methyl ester was prepared using dimethyl 5-chloroisophthalate (Matrix.) as the starting material according to the procedure described by Mitchelotti et al. in US5254584

### Example 92b

### Preparation of intermediate E/Z-6-Chloro-3-(3-chloro-5-methoxycarbonylbenzylidene)-2-oxo-2,3-dihydro-indole-1-carboxylic acid tert-butyl ester

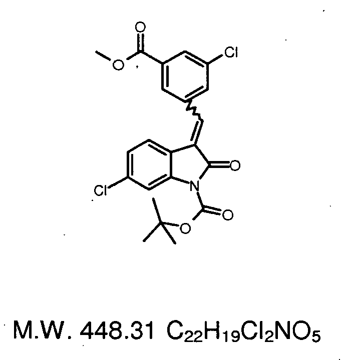

In a manner similar to the method described in example 24a, E/Z-3-Chloro-5-(6-chloro-2-oxo-1,2-dihydro-indol-3-ylidenemethyl)-benzoic acid methyl ester (3 g, 8.6 mmoll) was reacted with di-*tert*-butyl-dicarbonate (1.9 g, 8.6 mmol) (Aldrich), triethylamine (0.87 g, 8.6 mmol) and 4-dimethylaminopyridine (5 mg) in dichloromethane (100 mL) to give E/Z-6-Chloro-3-(3-chloro-5-methoxycarbonylbenzylidene)-2-oxo-2,3-dihydro-indole-1-carboxylic acid tert-butyl ester as a yellow solid (Yield: 3.7 g, 96%).

### Example 92c

### Preparation of racemic (2'R, 3R, 4'S)-6-chloro-4'-(3-chloro-5-methoxycarbonyl-phenyl)-2'-(1-methylene-propyl) spiro[3H-indole-3,3'-piperidine]-2,6'(1H)-dione

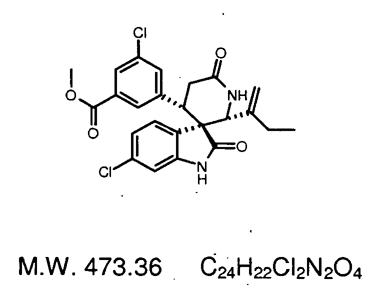

In a manner similar to the method described in example 41b, E/Z-6-Chloro-3-(3-chloro-5-methoxycarbonyl-benzylidene)-2-oxo-2,3-dihydro-indole-1-carboxylic acid tert-butyl ester prepared in example 92b (2.4 g, 5.3 mmol) was reacted with 1-(1-methylene-propyl)-3-trimethylsilyoxy-2-aza-1,3-butadiene (13 g, 66 mmol) prepared in example 80a in toluene and then trifluoroacetic acid (20 mL) in dichloromethane to give racemic (2'R, 3R, 4'S)-6-chloro-4'-(3-chloro-5-methoxycarbonyl-phenyl)-2'-(1-methylene-propyl) spiro[3H-indole-3,3'-piperidine]-2,6'(1H)-dione as a white solid (1.6 g, 64% ).

HRMS(ES⁺) *m*/*z* Calcd for C₂₄H₂₂Cl₂N₂O₄+ H [(M+H)⁺]: 473.1030. Found: 473.1031.

### Example 93

### Preparation of racemic (2'R, 3R, 4'S)-6-chloro-4'-(3-chloro-5-hydroxycarbonyl-phenyl)-2'-(1-methylene-propyl) spiro[3H-indole-3,3'-piperidine]-2,6'(1H)-dione

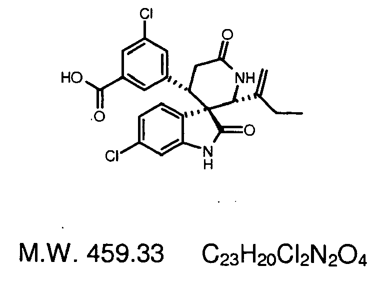

To a solution of racemic (2'R, 3R, 4'S)-6-chloro-4'-(3-chloro--5-methoxycarbonyl-phenyl)-2'-(1-methylene-propyl) spiro[3H-indole-3,3'-piperidine]-2,6'(1H)-dione (1.6 g, 33 mmol) prepared in example 92c in the mixture of tetrahydrofuran (60 mL) and methanol (30 mL) was added the solution of sodium hydroxide (1 N, 30 mL). After the reaction mixture was stirred at room temperature for 2 h, the crude was concentrated. The.residue was neutralized to "pH"∼4 with the dilute hydrochloride solution, then extracted with ethyl acetate. The organic layer was separated, dried over MgSO₄, concentrated to give racemic (2'R, 3R, 4'S)-6-chloro-4'-(3-chloro-5-hydroxycarbonyl-phenyl)-2'-(1-methylene-propyl) spiro[3H-indole-3,3'-piperidine]-2,6'(1H)-dione as a white solid (Yield 1.5 g, 99%).

HRMS(ES⁺) *mlz* Calcd for C₂₃H₂₀Cl₂N₂O₄+ H [(M+H)⁺]: 459.0873. Found: 459.0873.

### Example 94a

### Preparation of racemic (2'R, 3R, 4'S)-6-chloro-4'-(3-chloro-5-fluorocarbonyl-phenyl)-2'-(1-methylene-propyl) spiro[3H-indole-3,3'-piperidine]-2,6'(1H)-dione

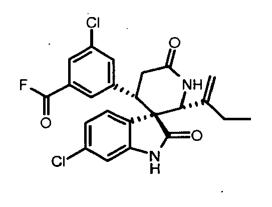

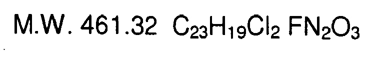

To the solution of racemic (2'R, 3R, 4'S)-6-chloro-4'-(3-chloro-5-hydroxycarbonyl-phenyl)-2'-(1-methylene-propyl) spiro[3H-indole-3,3'-piperidine]-2,6'(1H)-dione (0.2 g, 0.43 mmol) prepared in example 93 in dichloromethane (20 mL) at 0 °C was added cyanuric fluoride (48 mg, 0.35 mmol) (Alfa) and pyridine (37 mg, 0.48 mmol). After the mixture was stirred at 0 °C for 2 h, the mixture was partitioned between H₂O and dichloromethane. The organic layer was separated and the aqueous layer was extracted with dichloromethane. The organic layers were combined, dried over MgSO₄, concentrated to give racemic (2'R, 3R, 4'S)-6-chloro-4'-(3-chloro-5-fluorocarbonyl-phenyl)-2'-(1-methylene-propyl) spiro[3H-indole-3,3'-piperidine]-2,6'(1H)-dione as a yellow gum and used for the next step without further purification (Yield: 0.2 g, 100%).

### Example 94b

### Preparation of racemic (2'R, 3R, 4'S)-6-chloro-4'- [3-chloro-5-(4-methanesulfonyl-piperazine-1-carbonyl)-phenyl]-2'-(1-methylene-propyl) spiro[3H-indole-3,3'-piperidine]-2,6'(1H)-dione

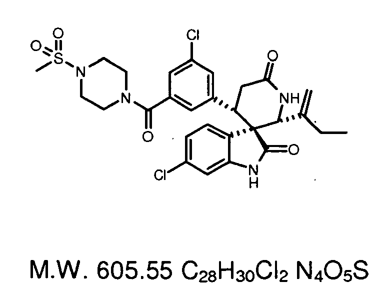

In a manner similar to the method described in example 34b, racemic (2'R, 3R, 4'S)-6-chloro-4'-(3-chloro-5-fluorocarbonyl-phenyl)-2'-(1-methylene-propyl) spiro[3H-indole-3,3'-piperidine]-2,6'(1H)-dione (0.16 g, 36 mmol) prepared in example 94a was reacted with N-methylsulfonylpiperazine (59 mg, 36 mmol), N-methylmorpholine (0.1 g, 0.99 mmol) and 4-dimethylaminopyridine (1 mg, 0.008 mmol) in tetrahydrofuran to give racemic (2'R, 3R, 4'S)-6-chloro-4'- [3-chloro-5-(4-methanesulfonyl-piperazine-1-carbonyl)-phenyl]-2'-(1-methylene-propyl) spiro[3H-indole-3,3'-piperidine]-2,6'(1H)-dione as an off-white solid (Yield 0.2 g, 92%).

HRMS(ES⁺) *m*/*z* Calcd for C₂₈H₃₀Cl₂ N₄O₅S + H [(M+H)⁺]: 619.1543. Found: 619.1544.

### Example 95a

### Preparation of racemic (2'R, 3R, 4'S)-6-chloro-4'-(3-chlorophenyl)-2'-(1-ethyl-propenyl)-1'-methyl spiro[3H-indole-3,3'-piperidine]- 2,6'(1H)-dione

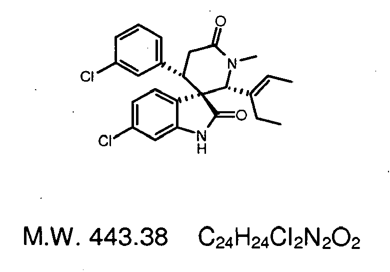

In a manner similar to the methods described in example 56a, 56b, 56c, racemic (2'R, 3R, 4'S)-6-chloro-4'-(3-chloro-phenyl)-2'-(1-ethyl-propenyl)-2,3-dihydro-2,6'-dioxo spiro[indole-3,3'-piperidine]-1-methoxyethyl trimethylsilane (1 g, 1.8 mmol) prepared in example 81a was reacted to give racemic (2'R, 3R, 4'S)-6-chloro-4'-(3-chlorophenyl)-2'-(1-ethyl-propenyl)-1'-methyl spiro[3H-indole-3,3'-piperidine]-2,6'(1H)-dione as a white solid (Yield, 0.50 g, 65%)

HRMS(ES⁺) *m*/*z* Calcd for C₂₄H₂₄Cl₂N₂O₂ +H [(M+H)⁺]: 443.1288. Found: 443.1288.

### Example 95b

### Preparation of chiral (2'R, 3R, 4'S)-6-chloro-4'-(3-chlorophenyl)-2'-(1-ethyl-propenyl)-1'-methyl spiro[3H-indole-3,3'-piperidine]- 2,6'(1H)-dione

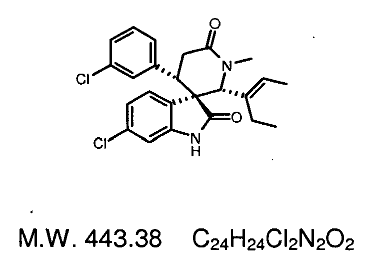

Separation of the two enantiomers from racemic (2'R, 3R, 4'S)-6-chloro-4'-(3-chlorophenyl)-2'-(1-ethyl-propenyl)-1'-methyl spiro[3H-indole-3,3'-piperidine]-2,6'(1H)-dione (85 mg) was conducted by chiral SFC to provide chiral (2'R, 3R, 4'S)-6-chloro-4'-(3-chlorophenyl)-2'-(1-ethyl-propenyl)-1'-methyl spiro[3H-indole-3,3'-piperidine]- 2,6'(1H)-dione as a white solid (Yield: 33 mg, 39%) and chiral (2'S, 3S, 4'R)-6-chloro-4'-(3-chlorophenyl)-2'-(1-ethyl-propenyl)-1'-methyl spiro[3H-indole-3,3'-piperidine]- 2,6'(1H)-dione (Yield: 26 mg, 31 %).

### Example 96a

### Preparation of racemic (2'R, 3R, 4'S)-6-chloro-4'-(3-chlorophenyl)-2'-isopropyl spiro[3H-indole-3,3'-piperidine]-2,6'(1H)-dione

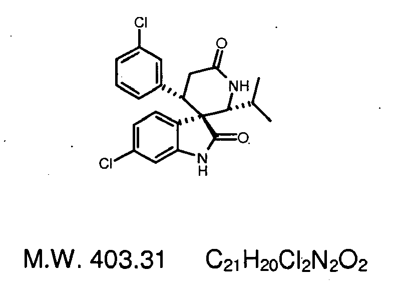

In a manner similar to the method described in example 72a, racemic (2'R, 3R, 4'S)-6-chloro-4'-(3-chlorophenyl)-2'-(1-methylene-propyl) spiro[3H-indole-3,3'-piperidine]-2,6'(1H)-dione (0.1 g, 0.25 mmol) prepared in example 87b was hydrogenated to give racemic (2'R, 3R, 4'S)-6-chloro-4'-(3-chlorophenyl)-2'-isopropyl spiro[3H-indole-3,3'-piperidine]-2,6'(1H)-dione as a white solid (Yield 61 mg, 60%).

HRMS(ES⁺) *m*/*z* Calcd for C₂₁H₂₀Cl₂N₂O₂+H [(M+H)⁺]: 403.0975 Found: 403.0976.

### Example 96b

### Preparation of chiral (2'R, 3R, 4'S)-6-chloro-4'-(3-chlorophenyl)-2'-isopropyl spiro[3H-indole-3,3'-piperidine]-2,6'(1H)-dione

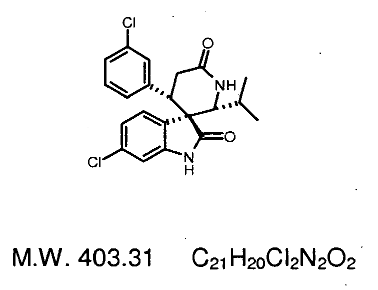

Separation of the two enantiomers from racemic (2'R, 3R, 4'S)-6-chloro-4'-(3-chlorophenyl)-2'-isopropyl spiro[3H-indole-3,3'-piperidine]-2,6'(1H)-dione (50 mg) was conducted by chiral SFC to provide chiral (2'R, 3R, 4'S)-6-chloro-4'-(3-chlorophenyl)-2'-isopropyl spiro[3H-indole-3,3'-piperidine]-2,6'(1H)-dione as a white solid (Yield: 15 mg, 30%) and chiral (2'S, 3S, 4'R)-6-chloro-4'-(3-chlorophenyl)-2'-isopropyl spiro[3H-indole-3,3'-piperidine]-2,6'(1H)-dione (Yield: 14 mg, 28%).

### Example 97a

### Preparation of intermediate racemic (2'R, 3R, 4'S)-6-chloro-4'-(3-chlorophenyl)-2'-(1-methylene-propyl)-2,3-dihydro-2,6'-dioxo-spiro[indole-3,3'-piperidine]-1-methoxyethyl trimethylsilane

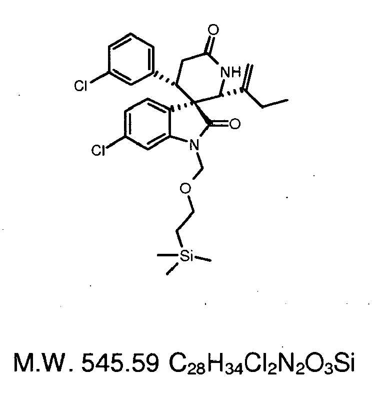

In a manner similar to the method described in example 55b, E/Z-6-chloro-3-(3-chloro-benzylidene)-1-(2-trimethylsilanyl-ethoxymethyl)-1,3-dihydro-indole-2-one (3 g, 7.1 mmol) prepared in example 55a was reacted with 1-(1-methylene-propyl)-3-trimethylsilyoxy-2-aza-1,3-butadiene (19 g, 96 mmol) prepared in example 80a in toluene (200 mL) to give racemic (2'R, 3R, 4'S)-6-chloro-4'-(3-chloro-phenyl)-2'-(1-methylene-propyl)-2,3-dihydro-2,6'-dioxo spiro[indole-3,3'-piperidine]-1-methoxyethyl trimethylsilane as a white foam (Yield 3.5 g, 90%).

### Example 97b

### Preparation of racemic (2'R; 3R, 4'S)-6-chloro-4'-(3-chlorophenyl)-2'-(1-methylene-propyl)-1'-methyl spiro[3H-indole-3,3'-piperidine]- 2,6'(1H)-dione

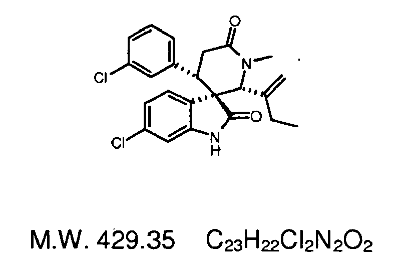

In a manner similar to the methods described in example 56a, 56b, 56c, racemic (2'R, 3R, 4'S)-6-chloro-4'-(3-chloro-phenyl)-2'-(1-methylene-propyl)-2,3-dihydro-2,6'-dioxo spiro[indole-3,3'-piperidine]-1-methoxyethyl trimethylsilane (0.6 g, 1.1 mmol) prepared in example 97a was reacted to form racemic (2'R, 3R, 4'S)-6-chloro-4'-(3-chlorophenyl)-2'-(1-methylene-propyl)-1'-methyl spiro[3H-indole-3,3'-piperidine]-2,6'(1H)-dione as a white solid (Yield, 0.16 g, 35%)

HRMS(ES⁺) *m*/*z* Calcd for C₂₃H₂₂Cl₂N₂O₂ +H [(M+H)⁺]: 429.1131. Found: 429.1131.

### Example 97c

### Preparation of chiral (2'R, 3R, 4'S)-6-chloro-4'-(3-chlorophenyl)-2'-(1-methylene-propyl)-1'-methyl spiro[3H-indole-3,3'-piperidine]- 2,6'(1H)-dione

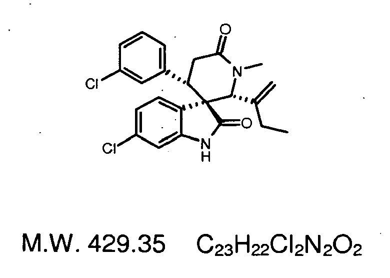

Separation of the two enantiomers from racemic (2'R, 3R, 4'S)-6-chloro-4'-(3-chlorophenyl)-2'-(1-methylene-propyl)-1'-methyl spiro[3H-indole-3,3'-piperidine]-2,6'(1H)-dione (160 mg) was conducted by chiral SFC to provide chiral (2'R, 3R, 4'S)-6-chloro-4'-(3-chlorophenyl)-2'-(1-methylene-propyl)-1'-methyl spiro[3H-indole-3,3'-piperidine]- 2,6'(1H)-dione as a white solid (Yield: 80 mg, 50%) and chiral (2'S, 3S, 4'R)-6-chloro-4'-(3-chlorophenyl)-2'-(1-methylene-propyl)-1'-methyl spiro[3H-indole-3,3'-piperidine]- 2,6'(1H)-dione (Yield: 80 mg, 50%).

### Example 98

### Preparation of racemic (2'R, 3R, 4'S)-2'-sec-Butyl-6-chloro-4'-(3-chlorophenyl) spiro[3H-indole-3,3'-piperidine]-2,6'(1H)-dione

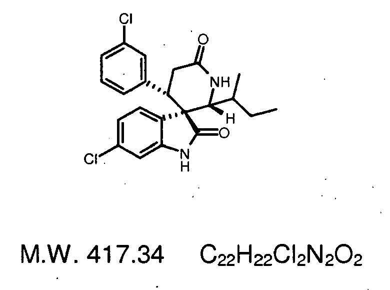

In a manner similar to the method described in example 72a, (2'R, 3R, 4'S)-6-chloro-4'-(3-chlorophenyl)-2'-(1-methylene-propyl) spiro[3H-indole-3,3'-piperidine]-2,6'(1H)-dione (0.38 g, 0.91 mmol) prepared in example 80b was hydrogenated to give racemic (2'R, 3R, 4'S)-2'-sec-butyl-6-chloro-4'-(3-chlorophenyl) spiro[3H-indole-3,3'-piperidine]-2,6'(1H)-dione as a white solid (Yield 0.25 g, 66%).

HRMS(ES⁺) *m*/*z* Calcd for C₂₂H₂₂Cl₂N₂O₂+H [(M+H)⁺]: 417.1131 Found: 417.1134

### Example 99a

### Preparation of intermediate 2-(tert-butyl-dimethyl-silanyloxymethyl)-prop-2-en-1-ol

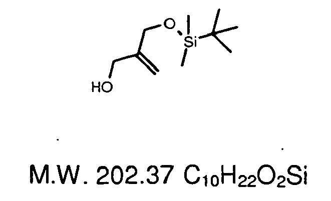

To a solution of 2-methylene-propane-1,3-diol (5.3 g, 60.15 mmol) (Aldrich) in tetrahydrofuran (100 mL) was added sodium hydride (3.61 g, 90.3 mmo). After the reaction mixture was stirred at room temperature for 20 min, tert-butyldimethylchlorosilane (10.89 g, 72.2 mmol) was added. The reaction mixture was stirred at room temperature for 3 h, then poured into water and extracted with ethyl acetate. The organic layers were combined, washed with water, brine, dried over dried over MgSO₄, filtered and concentrated to give 2-(tert-butyldimethyl-silanyloxymethyl)-prop-2-en-1-ol as colorless oil (Yield 12.0 g, 99 %).

### Example 99b

### Preparation of intermediate 2-(tert-butyl-dimethyl-silanyloxymethyl)-propenal

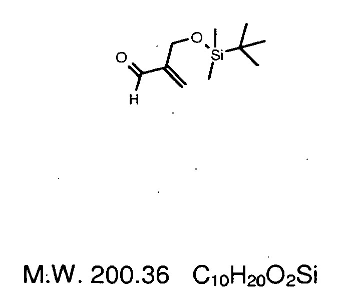

2-(Tert-butyl-dimethyl-silanyloxymethyl)-prop-2-en-1-ol (6 g, 29.7 mmol) prepared in example 99a was dissolved in dichloromethane (296 mL) containing both the molecular seieves (4 A) (Aldrich) and N-methyl morpholine oxide (5.2 g, 44.47 mmol). After stirring the mixture for 1 h, (Tetra-n-butyl ammonium per-ruthenate) (0.47 g, 1.48 mmol) (Aldrich) was added and the reaction mixture was stirred at room temperature for 1 h. Then another batch of (Tetra-n-butyl ammonium per-ruthenate) (0.23 g, 0.74 mmol) was added and the mixture was stirred at room temperature for another 1 h. The reaction mixture was diluted with dichloromethane. The organic layer was washed with Na₂S₂O₃ solution, brine and saturated copper (II) sulfate solution, then dried over dried over MgSO₄, filtered and concentrated. The residue was purified with chromatography to give 2-(tert-butyl-dimethyl-silanyloxymethyl)-propenal to as colorless oil (Yield 1.3 g, 22 %).

### Example 99c

### Preparation of intermediate 1-[1-(tert-butyl-dimethyl-silanyloxymethyl)-vinyl]-3-trimethylsilyoxy-2-aza-1,3-butadiene

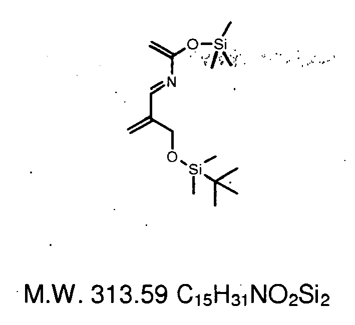

In a manner similar to the method described in example 1b, 2-(tert-butyldimethyl-silanyloxymethyl)-propenal (1.05 g, 5.25 mmol) prepared in example 99b was used as the starting material in place of 3-chloro-benzaldehyde to react with 1,1,1,3,3,3-hexamethyldisilazane (1.09 mL, 5.25 mmol), *n*-butyllithium (2.5 M, 2.1 mL, 5.25 mmol), trimethylsilyl chloride (0.67 mL, 5.25 mmol), triethylamine (0.95 mL, 6.8 mmol) and acetyl chloride (0.48 mL, 6.8 mmol) to give 1-[1-(tert-butyl-dimethyl-silanyloxymethyl)-vinyl]-3-trimethylsilyoxy-2-aza-1,3-butadiene as a yellow gum and used for the next step without further purification.

### Example 99d

### Preparation of racemic (2'R, 3R, 4'S)-6-chloro-4'-(3-chlorophenyl)-2'-(1-hydroxymethyl-vinyl)-spiro[3H-indole-3,3'-piperidine]-2,6'(1H)-dione

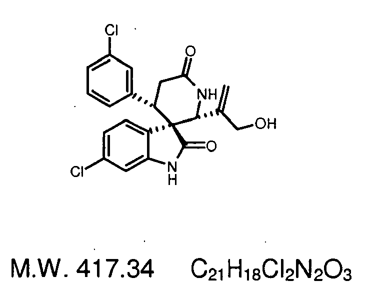

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.2 g, 0.51 mmol) was reacted with 1-[1-(tert-butyldimethyl-silanyloxymethyl)-vinyl]-3-trimethylsilyoxy-2-aza-1,3-butadiene prepared in example 99c in toluene and then trifluoroacetic acid (20 mL) in dichloromethane to give racemic (2'R, 3R, 4'S)-6-chloro-4'-(3-chlorophenyl)-2'-(1-hydroxymethyl-vinyl)-spiro[3H-indole-3,3'-piperidine]-2,6'(1H)-dione as a yellow solid.

HRMS(ES⁺) *m*/*z* Calcd for C₂₁H₁₈Cl₂N₂O₃+ H [(M+H)⁺]: 417.0767 Found: 417.0767.

### Example 100a

### Preparation of intermediate 2-methoxymethyl-propenal

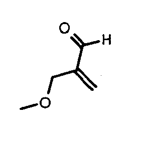

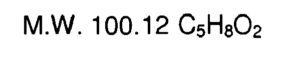

A stirred mixture of acrolein (22.4 g, 0.4 mol) (Aldrich), methanol (62 g, 1.82 mol), triethanolamine (1.5 g, 0.01 mol) and 85% phosphoric acid (1.14 g, 0.0098 mol) in water was heated at refluxing temperature for overnight, then cooled to room temperature and filtered. The filtrate is diluted with water and then treated with a 37% formaldehyde solution (32.4 g, 0.4 mol) (Aldrich), concentrated sulfuric acid (2.32 g, 0.02 mol) and dibutylamine (5.4 g, 0.04 mol) (Aldrich). The crude mixture was heated at refluxing temperature for 4 h, cooled to room temperature and extracted with dichloromethane. The organic layers were combined, washed with brine, dried over MgSO₄, filtered and concentrated. The residue was purified with chromatography (hexanes) to give 2-methoxymethyl-propenal as colorless oil (Yield 2.8 g, 7.0 %).

### Example 100b

### Preparation of intermediate 1-(1-methoxymethyl-vinyl)-3-trimethylsilyoxy-2-aza-1,3-butadiene

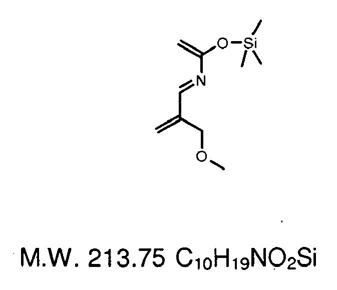

In a manner similar to the method described in example 1b, 2-methoxymethyl-propenal (1.35 mL, 10.5 mmol) prepared in example 100a was used as the starting material in place of 3-chloro-benzaldehyde to react with 1,1,1,3,3,3-hexamethyldisilazane (2.18 mL, 10.5 mmol), *n*-butyllithium (2.5 M, 4.2 mL, 10.5 mmol), trimethylsilyl chloride (1.33 mL, 10.5 mmol), triethylamine (1.9 mL, 13.6 mmol) and acetyl chloride (0.97 mL, 13.6 mmol) to give 1-(1-methoxymethyl-vinyl)-3-trimethylsilyoxy-2-aza-1,3-butadiene as a yellow gum and used for the next step without further purification.

### Example 100c

### Preparation of racemic (2'R, 3R, 4'S)-6-chloro-4'-(3-chlorophenyl)-2'-(1-methoxymethyl-vinyl) spiro[3H-indole-3,3'-piperidine]-2,6'(1H)-dione

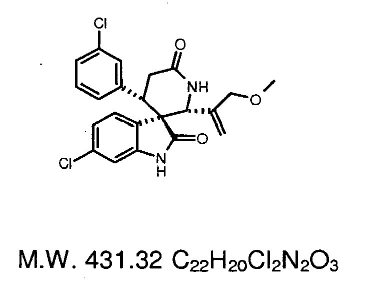

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.4 g, 1.02 mmol) was reacted with 1-(1-methoxymethyl-vinyl)-3-trimethylsilyoxy-2-aza-1,3-butadiene prepared in example 158b in toluene and then trifluoroacetic acid (20 mL) in dichloromethane to give racemic (2'R, 3R, 4'S)-6-chloro-4'-(3-chlorophenyl)-2'-(1-methoxymethyl-vinyl) spiro[3H-indole-3,3'-piperidine]-2,6'(1H)-dione as a white solid (Yield: 0.12 g, 27.2%).

HRMS(ES⁺) *m*/*z* Calcd for C₂₂H₂₀Cl₂N₂O₃ + H [(M+H)⁺]: 431.0924 Found: 431.0928.

### Example 101a

### Preparation of intermediate 1,2-difluoro-4-(2-methoxy-ethoxy)-benzene

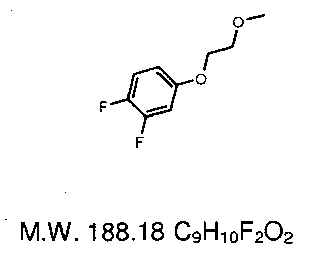

To a solution of 3, 4-difluoro-phenol (10 g, 76.9 mmol) (Aldrich) in N,N-dimethylformamide (100 mL) was added sodium hydride (4.6 g, 115 mmo) and 1-bromo-2-methoxy-ethane (12.8 g, 92.2 mmol) (Aldrich). The reaction mixture was stirred at room temperature for 2 h, then heated at 80 °C for 16 h. The reaction mixture was cooled to room temperature and poured into water, extracted with ethyl acetate. The organic layers were combined, washed with water, brine, dried over dried over MgSO₄, filtered and concentrated. The residue was purified with chromatography (EtOAc:Hexane = 1:10) to give 1,2-difluoro-4-(2-methoxy-ethoxy)-benzene as colorless oil (Yield 6.3 g, 44 %).

### Example 101b

### Preparation of intermediate 2,3-difluoro-6-(2-methoxy-ethoxy)-benzaldehyde

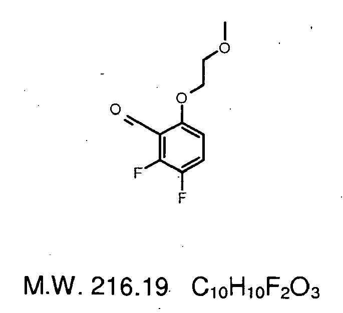

In a manner similar to the method described in example 52a, 1,2-difluoro-4-(2-methoxy-ethoxy)-benzene (6.3 g, 33.4 mmol) prepared in example 101a was reacted with lithium diisopropyl amine (20.1 mL, 2.0 M in THF, 40.1 mmol), N,N-dimethyl-formamide (3.11 mL, 40.1 mmol) and quenched with acetic acid (8.1 g, 134 mmol) and water (41.2 mL) in tetrahydrofuran to give 2,3-difluoro-6-(2-methoxy-ethoxy)-benzaldehyde as yellow oil (Yield: 5.8 g, 80.6%).

### Example 101c

### Preparation of intermediate 1-[2,3-difluoro-6-(2-methoxy-ethoxy)-phenyl]-3-trimethylsilyoxy-2-aza-1,3-butadiene

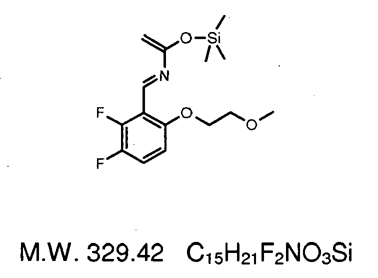

In a manner similar to the method described in example 1b, 2,3-difluoro-6-(2-methoxy-ethoxy)-benzaldehyde (2.23 g, 10.5 mmol) prepared in example 101b was reacted with 1,1,1,3,3,3-hexamethyldisilazane (1.7 g, 10.5 mmol), *n-*butyllithium (2.5 M, 4.2 mL, 10.5 mmol), trimethylsilyl chloride (1.33 g, 10.5 mmol), triethylamine (1.9 mL, 13 mmol) and acetyl chloride (0.97, 13 mmol) to give 1-[2,3-difluoro-6-(2-methoxy-ethoxy)-phenyl]-3-trimethylsilyoxy-2-aza-1,3-butadiene as yellow gum and used for the next step without further purification.

### Example 101d

### Preparation of racemic (2'R, 3R, 4'S)-6-chloro-4'-(3-chlorophenyl)-2'-[2,3-difluoro-6-(2-methoxy-ethoxy)-phenyl] spiro[3H-indole-3,3'-piperidine]-2,6'(1H)-dione

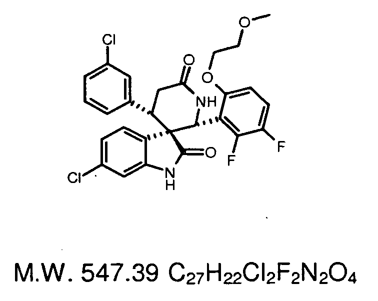

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.4 g, 1.02 mmol) was reacted with 1-[2,3-difluoro-6-(2-methoxy-ethoxy)-phenyl]-3-trimethylsilyoxy-2-aza-1,3-butadiene prepared in example 101 c in toluene and then trifluoroacetic acid (8 mL) in dichloromethane to give racemic (2'R, 3R, 4'S)-6-chloro-4'-(3-chlorophenyl)-2'-[2,3-difluoro-6-(2-methoxy-ethoxy)-phenyl] spiro[3H-indole-3,3'-piperidine]-2,6'(1H)-dione as a white solid (0.12 g, 21.4%).

HRMS(ES⁺) *m*/*z* Calcd for C₂₇H₂₂Cl₂F₂N₂O₄ + H [(M+H)⁺]: 547.0998 Found: 547.0997.

### Example 101e

### Preparation of chiral (2'R, 3R, 4'S)-6-chloro-4'-(3-chlorophenyl)-2'-[2,3-difluoro-6-(2-methoxy-ethoxy)-phenyl] spiro[3H-indole-3,3'-piperidine]-2,6'(1H)-dione

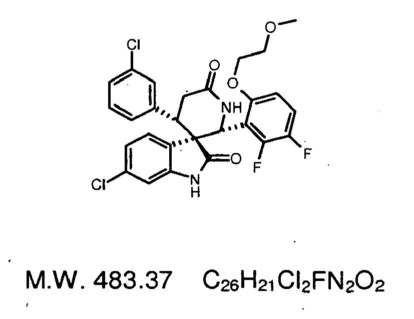

Separation of the two enantiomers from racemic (2'R, 3R, 4'S)-6-chloro-4'-(3-chlorophenyl)-2'-[2,3-difluoro-6-(2-methoxy-ethoxy)-phenyl] spiro[3H-indole-3,3'-piperidine]-2,6'(1H)-dione (80 mg) prepared in example 101d was conducted by chiral SFC to provide chiral (2'R, 3R, 4'S)-6-chloro-4'-(3-chlorophenyl)-2'-[2,3-difluoro-6-(2-methoxy-ethoxy)-phenyl] spiro[3H-indole-3,3'-piperidine]-2,6'(1H)-dione as a white solid (Yield: 35 mg, 43.7%) and chiral (2'S, 3S, 4'R)-6-chloro-4'-(3-chlorophenyl)-2'-[2,3-difluoro-6-(2-methoxy-ethoxy)-phenyl] spiro[3H-indole-3,3'-piperidine]-2,6'(1H)-dione as a white solid (Yield: 33 mg, 41.2%).

### Example 102a

### Preparation of intermediate 2,3-dimethyl-but-2-enoic acid ethyl ester

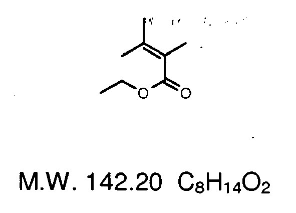

Triethyl phosphonopropionate (23.8 g, 0.1 mol) (Aldrich) was added dropwise to a stirred solution of sodium hydride (2.64 g, 0.11 mol) in dimethoxyethane (100 mL) at 0 °C. After stirring at room temperature for 20 mins, acetone (5.8 g, 0.1 mol) was added and the reaction mixture was refluxed for overnight. The two phase mixture was cooled, diluted with water and extracted with ether. The combined organic layers were dried over MgSO₄, filtered and concentrated. The residue was purified with chromatography (Hexane) to give 2,3-dimethyl-but-2-enoic acid ethyl ester as colorless oil (Yield 9.6 g, 67.7 %).

### Example 102b

### Preparation of intermediate 2, 3-dimethyl-but-2-en-1-ol

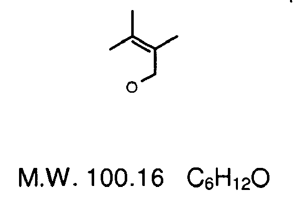

The solution of 2,3-dimethyl-but-2-enoic acid ethyl ester (9.6 g, 67.7 mmol) in ether (100 mL) was added to a stirred solution of LAH (1 M in ether, 67 mL, 67.7 mmol) dropwisely. After stirring at room temperature for 30 mins, the reaction mixture was quenched with saturate NH₄Cl solution, extracted with ether. The organic layers were combined, washed with water, brine, dried over MgSO₄ and filtered. The solvent was removed to give 2,3-dimethyl-but-2-en-1-ol as colorless oil (Yield 4.4 g, 66 %).

### Example 102c

### Preparation of intermediate 2,3-dimethyl-but-2-enal

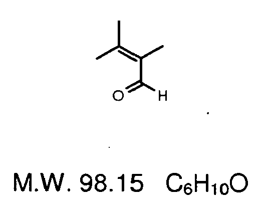

To a solution of oxalyl chloride (6.13 g, 48.3 mmol) (Aldrich) in dichloromethane (50 mL) at -78 °C was added the solution of dimethyl sulfoxide (6.85 mL, 96.6 mmol) in dichloromethane dropwise. After 5 mins, the solution of 2, 3-dimethyl-but-2-en-1-ol (4.4 g, 43.9 mmol) prepared in example 160b in dichloromethane (10 mL) was added dropwise. The reaction mixture was stirred at -78 °C for 15 mins. Triethyl amine (22 mL, 0.19 mol) was added and the reaction mixture was slowly warmed up to room temperature and stirred at room temperature for 45 mins. The water was added. The organic layers were separated, combined, washed with 10% of HCl, saturated NaHCO₃, brine, dried over MgSO₄, filtered and concentrated to give 2,3-dimethyl-but-2-enal as yellow liquid (Yield 4.0 g, 93 %).

### Example 102d

### Preparation of intermediate 1-(1,2-dimethyl-propenyl)-3-trimethylsilyoxy-2-aza-1,3-butadiene

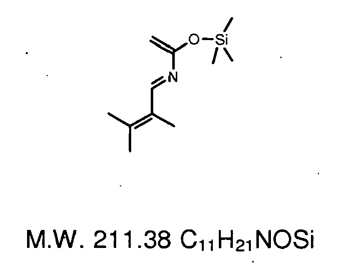

In a manner similar to the method described in example 1b, 2,3-dimethyl-but-2-enal (1.03 g, 10.5 mmol) prepared in example 102c was used as the starting material in place of 3-chloro-benzaldehyde to react with 1,1,1,3,3,3-hexamethyldisilazane (2.18 mL, 10.5 mmol), *n*-butyllithium (2.5 M, 4.2 mL, 10.5 mmol), trimethylsilyl chloride (1.33 mL, 10.5 mmol), triethylamine (1.9 mL, 13.6 mmol) and acetyl chloride (0.97 mL, 13.6 mmol) to give 1-(1,2-dimethyl-propenyl)-3-trimethylsilyoxy-2-aza-1,3-butadiene as a yellow gum and used for the next step without further purification.

### Example 102e

### Preparation of racemic (2'R, 3R, 4'S)-6-chloro-4'-(3-chlorophenyl)-2'-(1,2-dimethyl-propenyl) spiro[3H-indole-3,3'-piperidine]-2,6'(1H)-dione

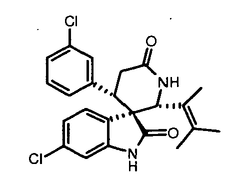

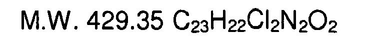

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.4 g, 1.02 mmol) was reacted with 1-(1,2-dimethyl-propenyl)-3-trimethylsilyoxy-2-aza-1,3-butadiene prepared in example 102d in toluene.and then trifluroacetic acid (20 mL) in dichloromethane to give racemic (2'R, 3R, 4'S)-6-chloro-4'-(3-chlorophenyl)-2"-(1,2-dimethyl-propenyl) spiro[3H-indole-3,3'-piperidine]-2,6'(1H)-dione as a yellow solid.

HRMS(ES⁺) *m*/*z* Calcd for C₂₃H₂₂Cl₂N₂O₂ + H [(M+H)⁺]: 429.1131 Found: 429.1132.

### Example 103

### Preparation of racemic (2'R, 3R, 4'S)-6-chloro-4'-(3-chlorophenyl)-2'-propionyl spiro[3H-indole-3,3'-piperidine]-2,6'(1H)-dione

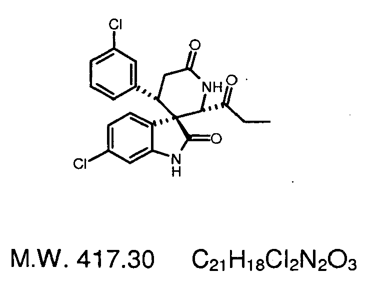

To a solution of racemic (2'R, 3R, 4'S)-6-chloro-4'-(3-chlorophenyl)-2'-(1-methylene-propyl) spiro[3H-indole-3,3'-piperidine]-2,6'(1H)-dione (300 mg, 0.72 mmol) prepared in example 80b in methanol and dichloromethane (1:1, 50 mL) at -78 °C was passed a stream of ozone until the color of the solution turned blue. The reaction mixture was degassed with nitrogen, then methyl disulfide (5 mL) was added. The reaction was slowly warmed to room temperature and stirred overnight. The mixture was concentrated, and the residue was purified by chromatography (EtOAc: CH2Cl2=1:1) to give the racemic (2'R, 3R, 4'S)-6-chloro-4'-(3-chlorophenyl)-2'-propionyl spiro[3H-indole-3,3'-piperidine]-2,6'(1H)-dione as a white solid (Yield 160 mg, 50%)

HRMS(ES⁺) *m*/*z* Calcd for C₂₁H₁₈Cl₂N₂O₃+H [(M+H)⁺]: 417.0767. Found: 417.0770.

### Example 104a

### Preparation of intermediate 1,2-difluoro-4-propoxy-benzene

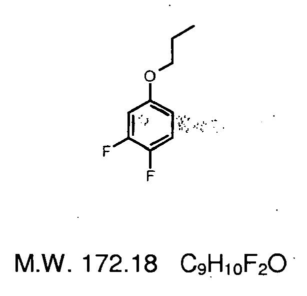

To a solution of 3,4-difluoro-phenol (20 g, 154 mmol) in N,N-dimethylformamide (70 mL) was added potassium carbonate (30 g, 217 mmo) and iodopropane (50 g, 293 mmol, Aldrich). The reaction mixture was heated at refluxing for 48 h. The crude was cooled to room temperature and filtered through a short pad of celite. The filtrate was concentrated and the residue was purified by chromatography (EtOAc:Hexanes=1:8) to give 1,2-difluoro-4-propoxy-benzene as a colorless oil (Yield 17 g, 100%).

### Example 104b

### Preparation of intermediate 2,3-difluoro-6-propoxy-benzaldehyde

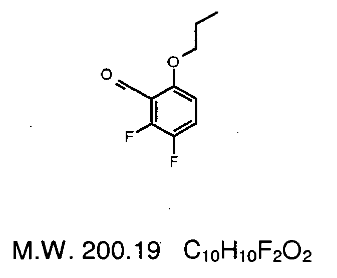

In a manner similar to the method described in example 52a, 1,2-difluoro-4-propoxy-benzene (17 g, 98.8 mmol) prepared in example 104a was reacted with lithium diisopropyl amine (59.2 mL, 2.0 M in THF, 0.118 mmol), N,N-dimethylformamide (9.17 mL, 0.118 mol) and quenched with acetic acid (23.7 g, 0.395 mol) and water (41.2 mL) in tetrahydrofuran to give 2,3-difluoro-6-propoxy-benzaldehyde as a yellow solid (Yield: 8.2 g, 42%).

### Example 104c

### Preparation of intermediate 1-(2,3-difluoro-6-propoxy-phenyl)-3-trimethylsilyoxy-2-aza-1,3-butadiene

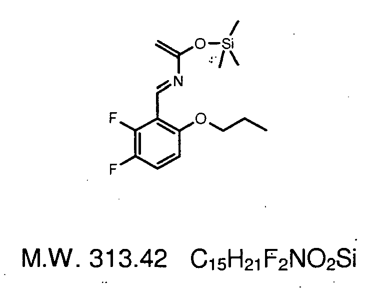

In a manner similar to the method described in example 1b, 2,3-difluoro-6-propoxy-benzaldehyde (2.1 g, 10.5 mmol) prepared in example 104b was used as the starting material in place of 3-chloro-benzaldehyde to react with 1,1,1,3,3,3-hexamethyldisilazane (1.7 g, 10.5 mmol), *n*-butyllithium (2.5 M, 4.2 mL, 10.5 mmol), trimethylsilyl chloride (1.33 g, 10.5 mmol), triethylamine (1.9 mL, 13 mmol) and acetyl chloride (0.97, 13 mmol) to give 1-(2,3-difluoro-6-propoxyphenyl)-3-trimethylsilyoxy-2-aza-1,3-butadiene as yellow gum and used for the next step without further purification.

### Example 104d

### Preparation of racemic (2'R, 3R, 4'S)-6-chloro-4'-(3-chlorophenyl)-2'-(2,3-difluoro-6-propoxy-phenyl) spiro[3H-indole-3,3'-piperidine]-2,6'(1H)-dione

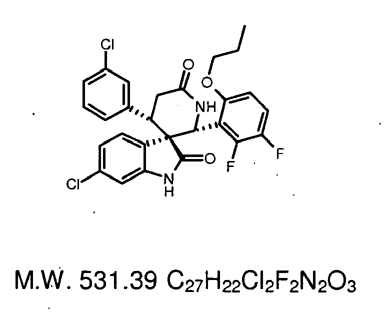

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.4 g, 1.02 mmol) was reacted with 1-(2,3-difluoro-6-propoxy-phenyl)-3-trimethylsilyoxy-2-aza-1,3-butadiene prepared in example 104c in toluene and then trifluoroacetic acid (8 mL) in dichloromethane to give racemic (2'R, 3R, 4'S)-6-chloro-4'-(3-chlorophenyl)-2'-(2,3-difluoro-6-propoxy-phenyl) spiro[3H-indole-3,3'-piperidine]-2,6'(1H)-dione as yellow oil.

HRMS(ES⁺) *m*/*z* Calcd for C₂₇H₂₂Cl₂F₂N₂O₃ + H [(M+H)⁺]: 531.1049 Found: 531.1049.

### Example 104e

### Preparation of chiral (2'R, 3R, 4'S)-6-chloro-4'-(3-chlorophenyl)-2'-(2,3-difluoro-6-propoxy-phenyl) spiro[3H-indole-3,3'-piperidine]-2,6'(1H)-dione

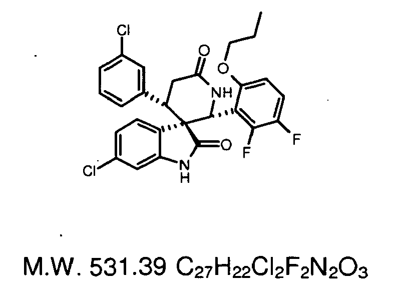

Separation of the two enantiomers from racemic (2'R, 3R, 4'S)-6-chloro-4'-(3-chlorophenyl)-2'-(2,3-difluoro-6-propoxy-phenyl) spiro[3H-indole-3,3'-piperidine]-2,6'(1H)-dione (0.67 g) prepared in example 104d was conducted by chiral SFC to provide chiral (2'R, 3R, 4'S)-6-chloro-4'-(3-chlorophenyl)-2'-(2,3-difluoro-6-propoxy-phenyl) spiro[3H-indole-3,3'-piperidine]-2,6'(1H)-dione as a white solid (Yield: 135 mg, 20.1 %) and chiral (2'S, 3S, 4'R)-6-chloro-4'-(3-chlorophenyl)-2'-(2,3-difluoro-6-propoxy-phenyl) spiro[3H-indole-3,3'-piperidine]-2,6'(1H)-dione as a white solid (Yield: 150 mg, 22.3%).

### Example 105a

### Preparation of intermediate 2-cyclopropyl-prop-2-en-1-ol

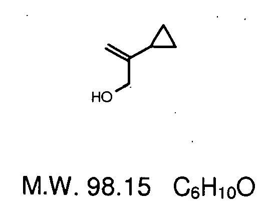

To a solution of propargyl alcohol (4.2 g, 74.9 mmol) (Aldrich) and Cul (14.28 g, 74.9 mmol) (Aldrich) in ether at 0 °C was added cyclopropylmagnesium chloride (0.5 M, 0.45 L, 0.225 mol) solution in tetrahydrofuran dropwise. The the reaction mixture was stirred at room temperature for 48 h. The reaction mixture was quenched with saturate NH₄Cl solution, extracted with ether. The organic layers were combined, washed with water, brine, dried over MgSO₄, filtered and concentrated. The residue was purified with chromatography (EtOAc:Hexane = 1:8) to give 2-cyclopropyl-prop-2-en-1-ol as yellow oil (Yield 5.9 g, 80.9 %).

### Example 105b

### Preparation of intermediate 2-cyclopropyl-propenal

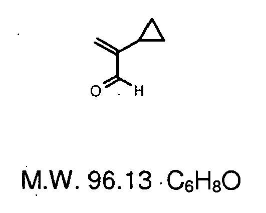

In a manner similar to the method described in example 102c, 2-cyclopropyl-prop-2-en-1-ol (5.95 g, 60.7 mmol) prepared in example 105a was reacted with oxalyl chloride (8.48 g, 60.7 mmol), dimethyl sulfoxide (9.47 mL, 133.5 mmol) and triethylamine (30.4 mL, 218 mmol) in dichloromethane to give 2-cyclopropyl-propenal as yellow oil (Yield: 4.8 g, 34.5 %).

### Example 105c

### Preparation of intermediate 1-(1-cyclopropyl-vinyl)-3-trimethylsilyoxy-2-aza-1,3-butadiene

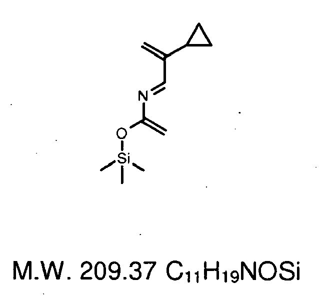

In a manner similar to the method described in example 1b, 2-cyclopropyl-propenal (1.0 g, 10.5 mmol) prepared in example 105b was used as the starting material in place of 3-chloro-benzaldehyde to react with 1,1,1,3,3,3-hexamethyldisilazane (2.18 mL, 10.5 mmol), *n*-butyllithium (2.5 M, 4.2 mL, 10.5 mmol), trimethylsilyl chloride (1.33 mL, 10.5 mmol), triethylamine (1.9 mL, 13.6 mmol) and acetyl chloride (0.97 mL, 13.6 mmol) to give 1-(1-cyclopropyl-vinyl)-3-trimethylsilyoxy-2-aza-1,3-butadiene as a yellow gum and used for the next step without further purification.

### Example 105d

### Preparation of racemic (2'R, 3R, 4'S)-6-chloro-4'-(3-chlorophenyl)-2'-(1-cyclopropyl-vinyl) spiro[3H-indole-3,3'-piperidine]-2,6'(1H)-dione

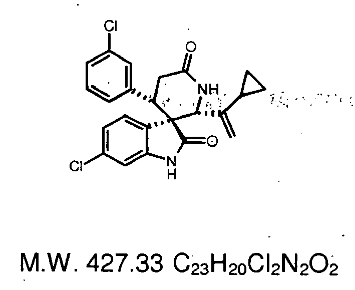

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.35 g, 0.90 mmol) was reacted with 1-(1-cyclopropyl-vinyl)-3-trimethylsilyoxy-2-aza-1,3-butadiene prepared in example 105c in toluene and then trifluoroacetic acid (20 mL) in dichloromethane to give racemic (2'R, 3R, 4'S)-6-chloro-4'-(3-chlorophenyl)-2'-(1-cyclopropyl-vinyl) spiro[3H-indole-3,3'-piperidine]-2,6'(1H)-dione as a yellow solid.

HRMS(ES⁺) *m*/*z* Calcd for C₂₃H₂₀Cl₂N₂O₂ + H [(M+H)⁺]: 427.0975 Found: 427.0973.

### Example 106

### Preparation of racemic (2'R, 3R, 4'S)-6-chloro-4'-(3-chlorophenyl)-1'-hydroxycarbonylmethyl-2'-(1-methylene-propyl) spiro[3H-indole-3,3'-piperidine]-2,6'(1H)-dione

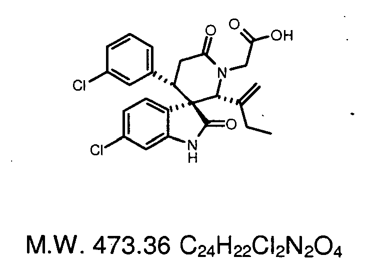

In a manner similar to the methods described in example 81b, 81 c, racemic (2'R, 3R, 4'S)-6-chloro-4'-(3-chloro-phenyl)-2'-(1-methylene-propyl)-2,3-dihydro-2,6'-dioxo spiro[indole-3,3'-piperidine]-1-methoxyethyl trimethylsilane (7 g, 12.8 mmol) prepared in example 97a was reacted to form racemic (2'R, 3R, 4'S)-6-chloro-4'-(3-chlorophenyl)-1'-hydroxycarbonylmethyl-2'-(1-methylene-propyl) spiro[3H-indole-3,3'-piperidine]-2,6'(1H)-dione as a white solid (3.6 g, 59%).

HRMS(ES⁺) *mlz* Calcd for C₂₄H₂₂Cl₂N₂O₄ +H [(M+H)⁺]: 473.1030 Found: 473.1032.

### Example 107a

### Preparation of intermediate racemic (2'R, 3R, 4'S)-6-chloro-4'-(3-chlorophenyl)-1'-fluorocarbonylmethyl-2'-(1-methylene-propyl) spiro[3H-indole-3,3'-piperidine]-2,6'(1H)-dione

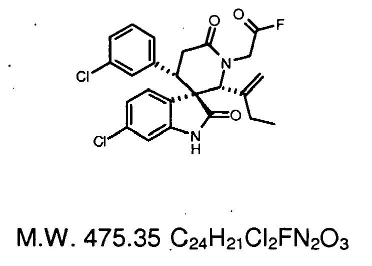

In a manner similar to the method described in example 82a, racemic (2'R, 3R, 4'S)-6-chloro-4'-(3-chlorophenyl)-1'-hydroxycarbonylmethyl-2'-(1-methylene-propyl) spiro[3H-indole-3,3'-piperidine]-2,6'(1H)-dione (1.2 g, 2.5 mmol) prepared in example 166 was reacted to form racemic (2'R, 3R, 4'S)-6-chloro-4'-(3-chlorophenyl)-1'-fluorocarbonylmethyl-2'-(1-methylene-propyl) spiro[3H-indole-3,3'-piperidine]-2,6'(1H)-dione (yield: 1.1 g, 92%).

### Example 107b

### Preparation of racemic (2'R, 3R, 4'S)-6-chloro-4'-(3-chlorophenyl)-2'-(1-methylene-propyl)-1'-[(1-methanesulfonyl-piperidin-4-yl)aminocarbonyl-methyl] spiro[3H-indole-3,3'-piperidine]-2,6'(1H)-dione

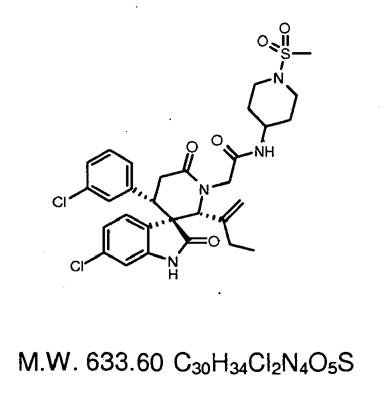

In a manner similar to the method described in example 34b, racemic (2'R, 3R, 4'S)-6-chloro-4'-(3-chlorophenyl)-1'-fluorocarbonylmethyl-2'-(1-methylene-propyl) spiro[3H-indole-3,3'-piperidine]-2,6'(1H)-dione (0.1 g, 0.21 mmol) prepared in example 107a was reacted with 1-methanesulfonyl-piperidin-4-ylamine trifluoroacetic acid salt (0.2 g, 0.34 mmol), N-methylmorpholine (0.2 g, 2 mmol) and 4-dimethylaminopyridine (1 mg) in tetrahydrofuran to give racemic (2'R, 3R, 4'S)-6-chloro-4'-(3-chlorophenyl)-2'-(1-methylene-propyl)-1'-[(1-methanesulfonyl-piperidin-4-yl)aminocarbonyl-methyl] spiro[3H-indole-3,3'-piperidine]-2,6'(1H)-dione as an off-white solid (Yield 58 mg, 44%).

HRMS(ES⁺) *m*/*z* Calcd for C₃₀H₃₄Cl₂N₄O₅S +H [(M+H)⁺]: 633.1700. Found: 633.1701.

### Example 108a

### Preparation of racemic (2'R, 3R, 4'S)-6-chloro-4'-(3-chlorophenyl)-2'-(1-methylene-propyl)-1'-(aminocarbonyl-methyl) spiro[3H-indole-3,3'-piperidine]-2,6'(1H)-dione

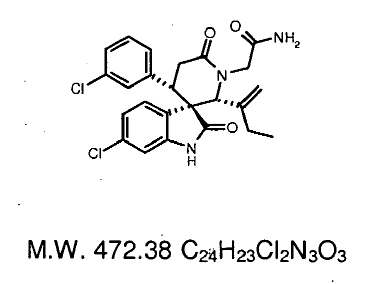

Racemic (2'R, 3R, 4'S)-6-chloro-4'-(3-chlorophenyl)-1'-fluorocarbonylmethyl-2'-(1-methylene-propyl) spiro[3H-indole-3,3'-piperidine]-2,6'(1H)-dione (0.1 g, 0.21 mmol) prepared in example 107a was stirred in a methanolic ammonia solution (7N, 10 mL) at room temperature for 18 h. The reaction mixture was concentrated, and the residue was purified by chromatography (EtOAc: MeOH=10:1) to give racemic (2'R, 3R, 4'S)-6-chloro-4'-(3-chlorophenyl)-2'-(1-methylene-propyl)-1'-[aminocarbonyl-methyl] spiro[3H-indole-3,3'-piperidine]-2,6'(1H)-dione as a yellow solid (Yield 41 mg, 40%).

HRMS(ES⁺) *m*/*z* Calcd for C₂₄H₂₃Cl₂N₃O₃ +Na [(M+Na)⁺]: 494.1008 Found: 494.1008

### Example 108b

### Preparation of chiral (2'R, 3R, 4'S)-6-chloro-4'-(3-chlorophenyl)-2'-(1-methylene-propyl)-1'-(aminocarbonyl-methyl) spiro[3H-indole-3,3'-piperidine]-2,6'(1H)-dione

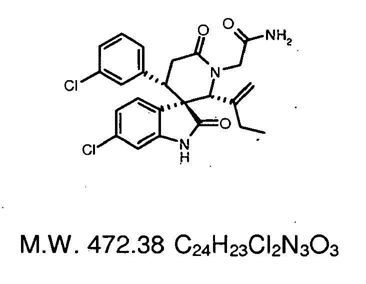

Separation of the two enantiomers from racemic (2'R, 3R, 4'S)-6-chloro-4'-(3-chlorophenyl)-2'-(1-methylene-propyl)-1'-[aminocarbonyl-methyl] spiro[3H-indole-3,3'-piperidine]-2,6'(1H)-dione (32 mg) was conducted by chiral SFC to provide chiral (2'R, 3R, 4'S)-6-chloro-4'-(3-chlorophenyl)-2'-(1-methylene-propyl)-1'-(aminocarbonyl-methyl) spiro[3H-indole-3,3'-piperidine]-2,6'(1H)-dione as a white solid (Yield: 9 mg, 28%) and chiral (2'S, 3S, 4'R)-6-chloro-4'-(3-chlorophenyl)-2'-(1-methylene-propyl)-1'-(aminocarbonyl-methyl) spiro[3H-indole,3,3'-piperidine]-2,6'(1H)-dione (Yield: 9 mg, 28%).

### Example 109a

### Preparation of intermediate 2-ethyl-3-methyl-but-2-enoic acid ethyl ester

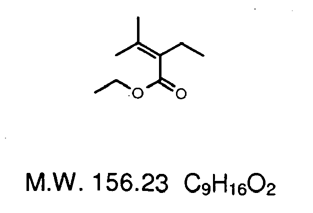

In a manner similar to the method described in example 102a, triethyl 2-phosphonobutyrat (25.2 g, 0.1 mol) was reacted with NaH (2.64 g, 0.11 mol), and then acetone (5.8 g, 0.1 mol) in dimethoxyethane to give 2-ethyl-3-methyl-but-2-enoic acid ethyl ester as colorless oil (Yield: 11.4 g, 73% ).

### Example 109b

### Preparation of intermediate 2-ethyl-3-methyl-but-2-en-1-ol

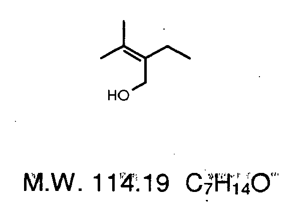

In a manner similar to the method described in example 102b, 2-ethyl-3-methyl-but-2-enoic acid ethyl ester (11.4 g, 73 mmol) prepared in example 109a was reacted with LAH (1 M in ether, 73 mL, 73 mmol) in diethyl ether to give 2-ethyl-3-methyl-but-2-en-1-ol as colorless oil (Yield: 7.62 g, 91.5% ).

### Example 109c

### Preparation of intermediate 2-ethyl-3-methyl-but-2-enal

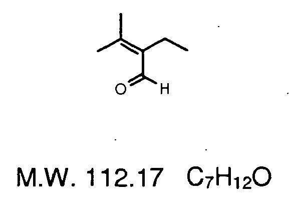

In a manner similar to the method described in example 102c, 2-ethyl-3-methyl-but-2-en-1-ol (7.62 g, 66.8 mmol) prepared in example 109b was reacted with oxalyl chloride (9.3 g, 73.5 mmol), dimethyl sulfoxide (10.44 mL, 146.9 mmol) and triethylamine (33.4 mL, 240 mmol) in dichloromethane to give 2-ethyl-3-methyl-but-2-enal as yellow oil (Yield: 7.5 g, 99 %).

### Example 109d

### Preparation of intermediate 1-(1-ethyl-2-methyl-propenyl)-3-trimethylsilyoxy-2-aza-1,3-butadiene

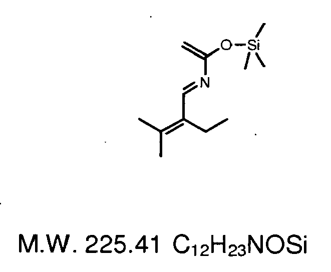

In a manner similar to the method described in example 1b, 2-ethyl-3-methyl-but-2-enal (1.18 g, 10.5 mmol) prepared in example 109c was used as the starting material in place of 3-chloro-benzaldehyde to react with 1,1,1,3,3,3-hexamethyldisilazane (2.18 mL, 10.5 mmol), *n*-butyllithium (2.5 M, 4.2 mL, 10.5 mmol), trimethylsilyl chloride (1.33 mL, 10.5 mmol), triethylamine (1.9 mL, 13.6 mmol) and acetyl chloride (0.97 mL, 13.6 mmol) to give 1-(1-ethyl-2-methyl-propenyl)-3-trimethylsilyoxy-2-aza-1,3-butadiene as a yellow gum and used for the next step without further purification.

### Example 109e

### Preparation of racemic (2'R, 3R, 4'S)-6-chloro-4'-(3-chlorophenyl)-2'-(1-ethyl-2-methyl-propenyl) spiro[3H-indole-3,3'-piperidine]-2,6'(1H)-dione

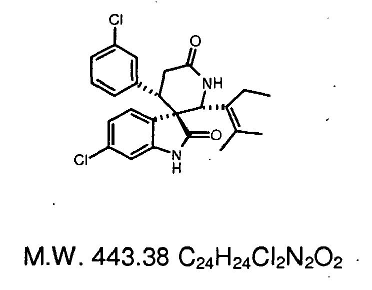

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.35 g, 0.89 mmol) was reacted with 1-(1-ethyl-2-methyl-propenyl)-3-trimethylsilyoxy-2-aza-1,3-butadiene prepared in example 109d in toluene and then trifluoroacetic acid (20 mL) in dichloromethane to give racemic (2'R, 3R, 4'S)-6-chloro-4'-(3-chlorophenyl)-2'-(1-ethyl-2-methyl-propenyl) spiro[3H-indole-3,3'-piperidine]-2,6'(1H)-dione as a yellow solid.

HRMS(ES⁺) *m*/*z* Calcd for C₂₄H₂₄Cl₂N₂O₂ + H [(M+H)⁺]: 443.1288 Found: 443.1287.

### Example 110a

### Preparation of racemic (2'R, 3R, 4'S)-6-chloro-4'-(3-chlorophenyl)-2'-[2,3-difluoro-6-(2-hydroxy-ethoxy)-phenyl] spiro[3H-indole-3,3'-piperidine]-2,6'(1H)-dione

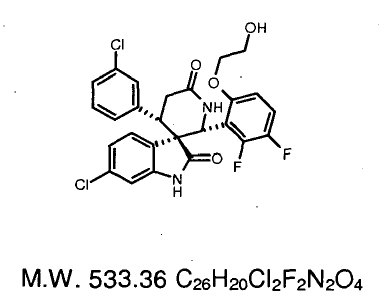

To a suspension of racemic (2'R, 3R, 4'S)-6-chloro-4'-(3-chlorophenyl)-2'-[2,3-difluoro-6-(2-methoxy-ethoxy)-phenyl] spiro[3H-indole-3,3'-piperidine]-2,6'(1H)-dione (0.12 g, 0.22 mmol) prepared in example 101d in dichloromethane (5 mL) at -78 °C was added boron tribromide (1 M, 2.19 ml, 2.19 mmol) dropwise. The reaction was gradually warmed up to room temperature and stirred at room temperature for 2 h. Then the crude was diluted with dichloromethane. The organic layer was washed with water, brine, dried over MgSO₄, filtered and concentrated. The residue was purified with chromatography (EtOAc) to give racemic (2'R, 3R, 4'S)-6-chloro-4'-(3-chlorophenyl)-2'-[2,3-difluoro-6-(2-hydroxy-ethoxy)-phenyl] spiro[3H-indole-3,3'-piperidine]-2,6'(1H)-dione as white solid (Yield: 46 mg, 39.3 %).

HRMS(ES⁺) *m*/*z* Calcd for C₂₆H₂₀Cl₂F₂N₂O₄ + H [(M+H)⁺]: 533.0841. Found: 533.0842.

### Example 110b

### Preparation of chiral (2'R, 3R, 4'S)-6-chloro-4'-(3-chlorophenyl)-2'-[2,3-difluoro-6-(2-hydroxy-ethoxy)-phenyl] spiro[3H-indole-3,3'-piperidine]-2,6'(1H)-dione

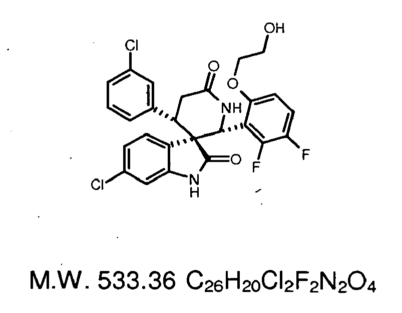

Separation of the two enantiomers from racemic (2'R, 3R, 4'S)-6-chloro-4'-(3-chlorophenyl)-2'-[2,3-difluoro-6-(2-hydroxy-ethoxy)-phenyl] spiro[3H-indole-3,3'-piperidine]-2,6'(1H)-dione (70 mg) was conducted by chiral SFC to provide chiral (2'R, 3R, 4'S)-6-chloro-4'-(3-chlorophenyl)-2'-[2,3-difluoro-6-(2-hydroxy-ethoxy)-phenyl] spiro[3H-indole-3,3'-piperidine]-2,6'(1H)-dione as a white solid (Yield: 30 mg, 42.8%) and chiral (2'S, 3S, 4'R)-6-chloro-4'-(3-chlorophenyl)-2'-[2,3-difluoro-6-(2-hydroxy-ethoxy)-phenyl] spiro[3H-indole-3,3'-piperidine]-2,6'(1H)-dione as a white solid (Yield: 20 mg, 28.5%).

### Example 111

### Preparation of racemic (2'R, 3R, 4'S)-6-chloro-4'-(3-chlorophenyl)-2'-(2,3-difluoro-6-hydroxy-phenyl) spiro[3H-indole-3,3'-piperidine]-2,6'(1H)-dione

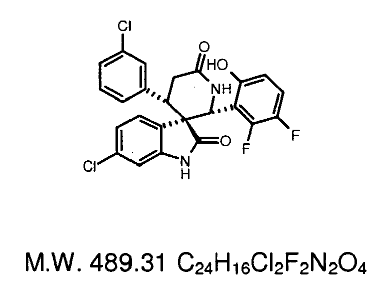

In preparation of racemic (2'R, 3R, 4'S)-6-chloro-4'-(3-chlorophenyl)-2'-[2,3-difluoro-6-(2-hydroxy-ethoxy)-phenyl] spiro[3H-indole-3,3'-piperidine]-2,6'(1H)-dione in example 110a, racemic (2'R, 3R, 4'S)-6-chloro-4'-(3-chlorophenyl)-2'-(2,3-difluoro-6-hydroxy-phenyl) spiro[3H-indole-3,3'-piperidine]-2,6'(1H)-dione was obtained as a side product.

HRMS(ES⁺) *m*/*z* Calcd for C₂₄H₁₆Cl₂F₂N₂O₄ + H [(M+H)⁺]: 489.0579. Found: 489.0580.

### Example 112

### Preparation of racemic (2'R, 3R, 4'S)-6-chloro-4'-(3-chlorophenyl)-2'-(1-ethyl-1-hydroxy-propyl) spiro[3H-indole-3,3'-piperidine]-2,6'(1H)-dione

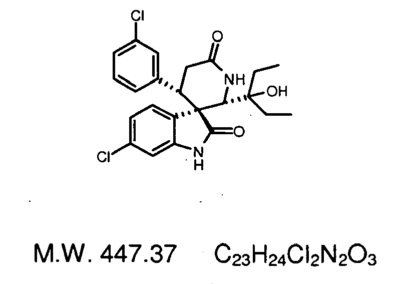

To a solution of racemic (2'R, 3R, 4'S)-6-chloro-4'-(3-chlorophenyl)-2'-propionyl spiro[3H-indole-3,3'-piperidine]-2,6'(1H)-dione (100 mg, 0.24 mmol) prepared in example 103 in tetrahydrofuran (3 mL) at -78 °C was added a tetrahydrofuran solution of ethylmagnesium chloride (2M, 6 mL, 12 mmol). The reaction mixture was stirred at -78 °C for 0.5 h, then slowly warmed to room temperature and stirred for 2 h. The mixture was poured into saturated aqueous NH₄Cl solution, and the mixture was extracted with ethyl acetate. The organic layer was separated, dried over MgSO₄, concentrated. The residue was purified by chromatography (EtOAc:MeOH=10:1) to give racemic (2'R, 3R, 4'S)-6-chloro-4-(3-chlorophenyl)-2'-(1-ethyl-1-hydroxy-propyl) spiro[3H-indole-3,3'-piperidine]-2,6'(1H)-dione as a white solid (Yield 100 mg, 93%)

HRMS(ES⁺) *m*/*z* Calcd for C₂₃H₂₄Cl₂N₂O₃+H [(M+H)⁺]: 447.1237. Found: 447.1237 .

### Example 113a

### Preparation of intermediate racemic (2'R, 3R, 4'S)-6-chloro-4'-(3-chlorophenyl)-2'-(1-ethyl-3-methyl-oxiranyl)-2,3-dihydro-2,6'-dioxo-spiro[indole-3,3'-piperidine]-1-methoxyethyl trimethylsilane

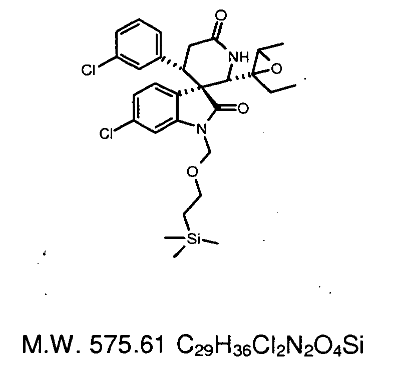

To a solution of racemic (2'R, 3R, 4'S)-6-chloro-4'-(3-chloro-phenyl)-2'-(1-ethyl-propenyl)-2,3-dihydro-2,6'-dioxo spiro[indole-3,3'-piperidine]-1-methoxyethyl trimethylsilane (1.2 g, 2.1 mmol) prepared in example 81a in dichloromethane (50 mL) was added m-chloroperoxybenzoic acid (77%, 8.9 g, ) and NaHCO₃ (2 g). The reaction mixture was stirred at room temperature for 18 h, the poured into an aqueous solution of Na₂SO₃, then extracted with ethyl acetate. The organic layer was separated, dried over Na₂SO₄, concenatrated. The residue was purified by chromatography (EtOAc:hexanes=1:1) to give racemic (2'R, 3R, 4'S)-6-chloro-4'-(3-chlorophenyl)- 2'-(1-ethyl-3-methyl-oxiranyl)-2,3-dihydro-2,6'-dioxo spiro[indole-3,3'-piperidine]-1-methoxyethyl trimethylsilane as a white foam (Yield, 1.0 g, 82%).

### Example 113b

### Preparation of racemic (2'R, 3R, 4'S)-6-chloro-4'-(3-chlorophenyl)-2'-(1-ethyl-3-methyl-oxiranyl) spiro[3H-indole-3,3'-piperidine]-2,6'(1H)-dione

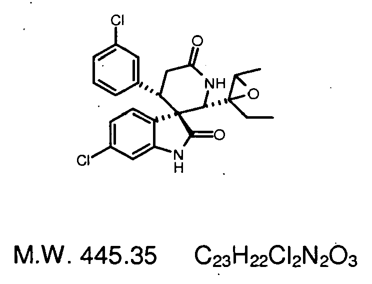

In a manner similar to the methods described in example 56b and 56c, racemic (2'R, 3R, 4'S)-6-chloro-4'-(3-chlorophenyl)- 2'-(1-ethyl-3-methyl-oxiranyl)-2,3-dihydro-2,6'-dioxo spiro[indole-3,3'-piperidine]-1-methoxyethyl trimethylsilane (0.19 g, 0.33 mmol) prepared in example 113a was reacted to give racemic (2'R, 3R, 4'S)-6-chloro-4'-(3-chlorophenyl)-2'-(1-ethyl-3-methyl-oxiranyl) spiro[3H-indole-3,3'-piperidine]-2,6'(1H)-dione as a white solid (Yield, 0.42 g, 29%)

HRMS(ES⁺) *m*/*z* Calcd for C₂₃H₂₂Cl₂N₂O₃+H [(M+H)⁺]: 445.1080. Found: 445.1081.

### Example 114a

### Preparation of intermediate 2-fluoro-3-methyl-but-2-enoic acid ethyl ester

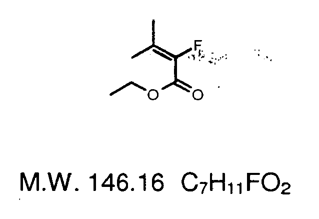

Triethyl 2-fluoro-2-phosphonoacetate (20 g, 82.6 mmol) (Aldrich) was added dropwise to a stirred solution of sodium hydride (3.6 g, 90.8 mmol) in dimethoxyethane (100 mL) at 0 °C. After stirring at room temperature for 20 min, acetone (4.78 g, 82.6 mmol) was added and the reaction mixture was refluxed for overnight. The two phase mixture was cooled, diluted with water and extracted with ether. The combined organic layers were dried over MgSO₄, filtered and concentrated. The residue was purified with chromatography (Hexanes) to give 2-fluoro-3-methyl-but-2-enoic acid ethyl ester as colorless oil (Yield: 9.0 g, 61.6 %).

### Example 114b

### Preparation of intermediate 2-fluoro-3-methyl-but-2-en-1-ol

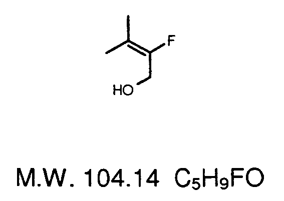

The solution of 2-fluoro-3-methyl-but-2-enoic acid ethyl ester (9.0 g, 61.6 mmol) in ether (100 mL) was added to a stirred solution of LAH (1 M in ether, 61.6 mL, 67.7 mmol) dropwise. After stirring at room temperature for 30 mins, the reaction mixture was quenched with saturate NH₄Cl solution, extracted with ether. The organic layers were combined, washed with water, brine, dried over MgSO₄ and filtered. The solvent was removed to give 2-fluoro-3-methyl-but-2-en-1-ol as colorless oil (Yield: 5.21 g, 81.3%).

### Example 114c

### Preparation of intermediate 2-fluoro-3-methyl-but-2-enal

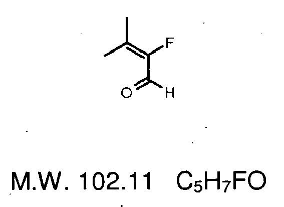

To a solution of oxalyl chloride (6.98 g, 50 mmol) (Aldrich) in dichloromethane (50 mL) at -78 °C was added the solution of dimethyl sulfoxide (7.80 mL, 110 mmol) in dichloromethane dropwise. After 5 mins, the solution of 2-fluoro-3-methyl-but-2-en-1-ol (5.21 g, 50 mmol) prepared in example 114b in dichloromethane (10 mL) was added dropwise. The reaction mixture was stirred at -78 °C for 15 min. Triethylamine (25 mL, 0.18 mol) was added and the reaction mixture was slowly warmed up to room temperature and stirred at room temperature for 45 min. The water was added. The organic layers were separated, combined, washed with 10% of HCl, saturated NaHCO₃, brine, dried over MgSO₄, filtered and concentrated to give 2-fluoro-3-methyl-but-2-enal as yellow liquid (Yield: 4.02g, 82.4 %).

### Example 114d

### Preparation of intermediate 1-(1-fluoro-2-methyl-propenyl)-3-trimethylsilyoxy-2-aza-1,3-butadiene

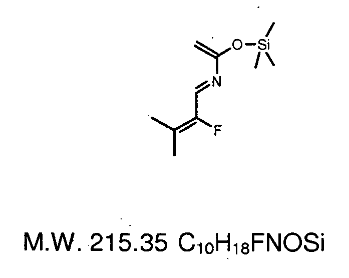

In a manner similar to the method described in example 1b, 2-fluoro-3-methyl-but-2-enal (2.14 g, 21 mmol) prepared in example 114c was used as the starting material in place of 3-chloro-benzaldehyde to react with 1,1,1,3,3,3-hexamethyldisilazane (4.36 mL, 21 mmol), *n*-butyllithium (2.5 M, 8.4 mL, 21 mmol), trimethylsilyl chloride (2.66 mL, 21 mmol), triethylamine (3.8 mL, 27.2 mmol) and acetyl chloride (1.94 mL, 27.2 mmol) to give 1-(1-fluoro-2-methyl-propenyl)-3-trimethylsilyoxy-2-aza-1,3-butadiene as a yellow gum and used for the next step without further purification.

### Example 114e

### Preparation of racemic (2'R, 3R, 4'S)-6-chloro-4'-(3-chlorophenyl)-2'-(1-fluoro-2-methyl-propenyl) spiro[3H-indole-3,3'-piperidine]-2,6'(1H)-dione

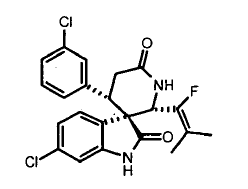

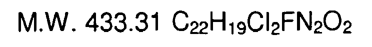

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (1.0 g, 2.56 mmol) was reacted with 1-(1-fluoro-2-methyl-propenyl)-3-trimethylsilyoxy-2-aza-1,3-butadiene prepared in example 114e in toluene and then trifluoroacetic acid (20 mL) in dichloromethane to give racemic (2'R, 3R, 4'S)-6-chloro-4'-(3-chlorophenyl)-2'-(1-fluoro-2-methyl-propenyl) spiro[3H-indole-3,3'-piperidine]-2,6'(1H)-dione as a yellow solid.

HRMS(ES⁺) *m*/*z* Calcd for C₂₂H₁₉Cl₂FN₂O₂+ H [(M+H)⁺]: 433.0881 Found: 433.0879.

### Example 115

### Preparation of racemic (2'R, 3R, 4'S)-6-chloro-4'-(3-chlorophenyl)-2'- isobutyryl spiro[3H-indole-3,3'-piperidine]-2,6'(1H)-dione

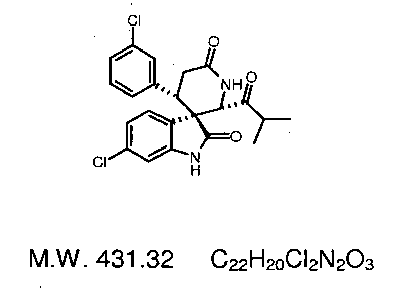

In a manner similar to the methods described in example 103, racemic (2'R, 3R, 4'S)-6-chloro-4'-(3-chlorophenyl)-2'-(2-methyl-1-methylene-propyl) spiro[3H-indole-3,3'-piperidine]-2,6'(1H)-dione (0.2 g, 0.47 mmol) prepared in example 139d was reacted to form racemic (2'R, 3R, 4'S)-6-chloro-4'-(3-chlorophenyl)-2'-isobutyryl spiro[3H-indole-3,3'-piperidine]-2,6'(1H)-dione as a white solid (Yield, 0.13 g, 66%).

HRMS(ES⁺) *m*/*z* Calcd for C₂₂H₂₀Cl₂N₂O₃+H [(M+H)⁺]: 431.0924. Found: 431.0925.

### Example 116a

### Preparation of intermediate racemic (2'R, 3R, 4'S)-6-chloro-4'-(3-chlorophenyl)-2'-isopropenyl-2,3-dihydro-2,6'-dioxo spiro[indole-3,3'-piperidine]-1-methoxyethyl trimethylsilane

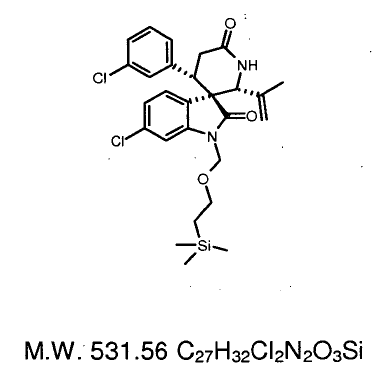

In a manner similar to the method described in example 55b, E/Z-6-chloro-3-(3-chlorobenzylidene)-1-(2-trimethylsilanyl-ethoxymethyl)-1,3-dihydro-indole-2-one (8 g, 20 mmol) prepared in example 55a was reacted with 1-isopropenyl-3-trimethylsilyoxy-2-aza-1,3-butadiene (21 g, 99 mmol) prepared in example 87a in toluene (200 mL) to give racemic (2'R, 3R, 4'S)-6-chloro-4'-(3-chlorophenyl)-2'-isopropenyl-2,3-dihydro-2,6'-dioxo spiro[indole-3,3'-piperidine]-1-methoxyethyl trimethylsilane as a white gum (3.5 g, 33%)

### Example 116b

### Preparation of intermediate racemic (2'R, 3R, 4'S)-6-chloro-4'-(3-chlorophenyl)-2'-isopropenyl-2,3-dihydro-1'-[(tert-butoxycarbonyl) methyl]-2,6'-dioxo spiro[indole-3,3'-piperidine]-1-methoxyethyl trimethylsilane

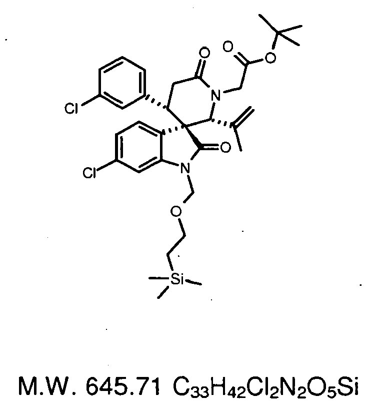

In a manner similar to the method described in example 55c, racemic (2'R, 3R, 4'S)-6-chloro-4'-(3-chlorophenyl)-2'-isopropenyl-2,3-dihydro-2,6'-dioxo spiro[indole-3,3'-piperidine]-1-methoxyethyl trimethylsilane (3 g, 5.64 mmol) prepared in example 116a was reacted with bromo-acetic acid tert-butyl ester and cesium carbonate in N,N-dimethyl-formamide to give racemic (2'R, 3R, 4'S)-6-chloro-4'-(3-chlorophenyl)-2'-isopropenyl-2,3-dihydro-1'-[(*tert*-butoxycarbonyl) methyl]-2,6'-dioxo spiro[indole-3,3'-piperidine]-1-methoxyethyl trimethylsilane as a white foam (Yield: 2.98 g, 79%).

### Example 116c

### Preparation of racemic (2'R, 3R, 4'S)-6-chloro-4'-(3-chlorophenyl)-2'-isopropenyl -1'-hydroxycarbonylmethyl spiro[3H-indole-3,3'-piperidine]-2,6'(1H)-dione

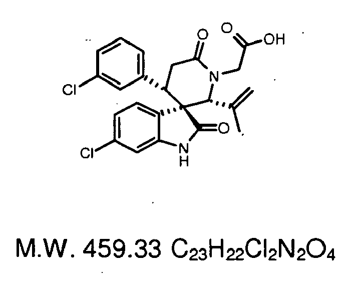

In a manner similar to the method described in example 55d, racemic (2'R, 3R, 4'S)-6-chloro-4'-(3-chlorophenyl)-2'-isopropenyl-2,3-dihydro-1'-[(*tert-*butoxycarbonyl) methyl]-2,6'-dioxo spiro[indole-3,3'-piperidine]-1-methoxyethyl trimethylsilane (2.88 g, 4.46 mmol) preparared in example 116b was reacted with trifluoroacetic acid, then ethyl-diisopropyl-amine to give racemic (2'R, 3R, 4'S)-6-chloro-4'-(3-chlorophenyl)-2'-isopropenyl-1'-hydroxycarbonylmethyl spiro[3H-indole-3,3'-piperidine]-2,6'(1H)-dione as yellow oil (Yield 1.7 g, 85.8 %).

### Example 116d

### Preparation of intermediate racemic (2'R, 3R, 4'S)-6-chloro-4'-(3-chlorophenyl)-1'-fluorocarbonylmethyl-2'-isopropenyl spiro[3H-indole-3,3'-piperidine]-2,6'(1H)-dione

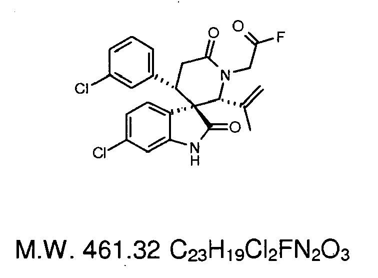

In a manner similar to the method described in example 34a, racemic (2'R, 3R, 4'S)-6-chloro-4'-(3-chlorophenyl)-2'-isopropenyl-1-[hydroxycarbonyl-methyl] spiro[3H-indole-3,3'-piperidine]-2,6'(1H)-dione (0.42 g, 0.91 mmol) prepared in example 116c was reacted with cyanuric fluoride (0.31 mL, 2.01 mmol) (Alfa) and pyridine (0.14 g, 1.82 mmol) in dichloromethane to give racemic (2'R, 3R, 4'S)-6-chloro-4'-(3-chlorophenyl)-1'-fluorocarbonylmethyl-2'-isopropenyl spiro[3H-indole-3,3'-piperidine]-2,6'(1H)-dione (Yield: 0.38 g, 90%).

### Example 116e

### Preparation of racemic (2'R, 3R, 4'S)-1'-(aminocarbonyl-methyl)-6-chloro-4'-(3-chlorophenyl)-2'-isopropenyl spiro[3H-indole-3,3'-piperidine]-2,6'(1H)-dione

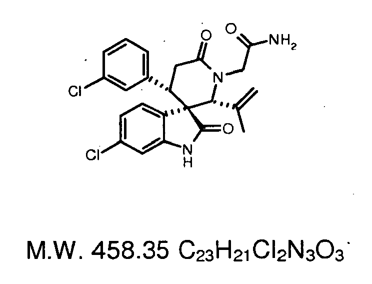

To a solution of ammonia (2 M, 10 mL) in methanol was added racemic (2'R, 3R, 4'S)-6-chloro-4'-(3-chlorophenyl)-1'-fluorocarbonylmethyl-2'-isopropenyl spiro[3H-indole-3,3'-piperidine]-2,6'(1H)-dione (0.18 g, 0.39 mmol) prepared in example 116d. The reaction mixture was stirred at room temperature for overnight. The solvent was removed and the residue was partitioned between ethyl acetate and water. The organic layer was separated, combined and concentrated. The residue was purified with chromatography (MeOH:EtOAc = 5:95) to give racemic (2'R, 3R, 4'S)-1'-(aminocarbonyl-methyl)-6-chloro-4'-(3-chlorophenyl)-2'-isopropenylspiro[3H-indole-3,3'-piperidine]-2,6'(1H)-dione as a white solid (Yield: 70 mg, 38.9%).

HRMS(ES⁺) *m*/*z* Calcd for C₂₃H₂₁Cl₂N₃O₃+ H [(M+H)⁺]: 458.1033. Found: 458.1033.

### Example 117

### Preparation of racemic (2'R, 3R, 4'S)-6-chloro-4'-(3-chlorophenyl)-1'-(cyclopropylaminocarbonyl-methyl)-2'-isopropenyl spiro[3H-indole-3,3'-piperidine]-2,6'(1H)-dione

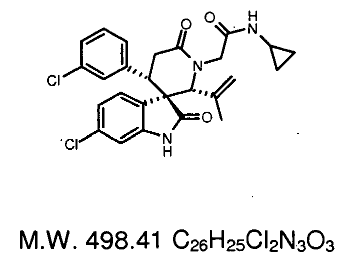

In a manner similar to the method described in example 34b, racemic (2'R, 3R, 4'S)-6-chloro-4'-(3-chlorophenyl)-1'-fluorocarbonylmethyl-2'-isopropenyl spiro[3H-indole-3,3'-piperidine]-2,6'(1H)-dione (0.2 g, 0.433 mmol) prepard in example 116d was reacted with 2-amino-2-methyl-propan-1-ol (49 mg, 0.86 mmol), N-methylmorpholine (90 mg, 0.86 mmol) and 4-dimethylaminopyridine (5 mg) in tetrahydrofuran to give racemic (2'R, 3R, 4'S)-6-chloro-4'-(3-chlorophenyl)-1'-(cyclopropylaminocarbonyl-methyl)-2'-isopropenyl spiro[3H-indole-3,3'-piperidine]-2,6'(1H)-dione as an off-white solid (Yield: 80 mg, 38%).

HRMS(ES⁺) *m*/*z* Calcd for C₂₆H₂₅Cl₂N₃O₃ +H [(M+H)⁺]: 498.1346 Found: 498.1345.

### Example 118

Preparation of racemic (2'R, 3R, 4'S)-6-chloro-4'-(3-chlorophenyl)-2'-isopropenyl-1'-[(1-methyl-piperidin-4-yl)aminocarbonyl-methyl] spiro[3H-indole-3,3' piperidine]-2,6'(1H)-dione

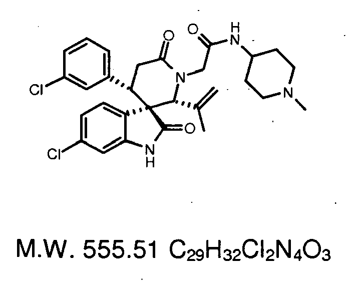

In a manner similar to the method described in example 34b, racemic (2'R, 3R, 4'S)-6-chloro-4'-(3-chlorophenyl)-1'-fluorocarbonylmethyl-2'-isopropenyl spiro[3H-indole-3,3'-piperidine]-2,6'(1H)-dione (0.2 g, 0.433 mmol) prepared in example 116d was reacted with 1-methyl-piperidin-4-ylamine (0.134 mg, 0.95 mmol), N-methylmorpholine (99 mg, 0.95 mmol) and 4-dimethylaminopyridine (5 mg) in tetrahydrofuran to give racemic (2'R, 3R, 4'S)-6-chloro-4'-(3-chlorophenyl)-2'-isopropenyl-1'-[(1-methyl-piperidin-4-yl)aminocarbonyl-methyl] spiro[3H-indole-3,3' piperidine]-2,6'(1H)-dione as an off-white solid (Yield: 75 mg, 31.2%).

HRMS(ES⁺) *m*/*z* Calcd for C₂₉H₃₂Cl₂N₄O₃ +H [(M+H)⁺]: 555.1924 Found: 555.1925.

### Example 119

### Preparation of racemic (2'R, 3R, 4'S)-6-chloro-4'-(3-chlorophenyl)-2'-isopropenyl-1'-[(1-methylsulfonyl-piperidin-4-yl)aminocarbonyl-methyl] spiro[3H-indole-3,3' piperidine]-2,6'(1H)-dione

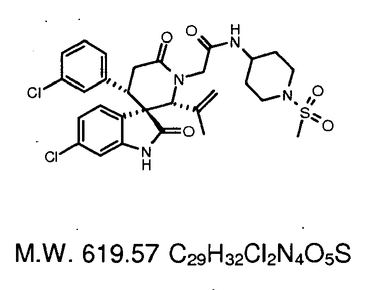

In a manner similar to the method described in example 34b, racemic (2'R, 3R, 4'S)-6-chloro-4'-(3-chlorophenyl)-1'-fluorocarbonylmethyl-2'-isopropenyl spiro[3H-indole-3,3'-piperidine]-2,6'(1H)-dione (0.2 g, 0.433 mmol) prepared in example 116d was reacted with 1-methylsulfonyl-piperidin-4-yl-amine (0.15 g, 0.86 mmol), N-methylmorpholine (90 mg, 0.86 mmol) and 4-dimethylaminopyridine (5 mg) in tetrahydrofuran to give racemic (2'R, 3R, 4'S)-6-chloro-4'-(3-chlorophenyl)-2'-isopropenyl-1'-[(1-methylsulfonyl-piperidin-4-yl)aminocarbonyl-methyl] spiro[3H-indole-3,3' piperidine]-2,6'(1H)-dione as an off-white solid (Yield: 92 mg, 38.3%).

HRMS(ES⁺) *m*/*z* Calcd for C₂₉H₃₂Cl₂N₄O₅S +H [(M+H)⁺]: 619.1543 Found: 619.1541.

### Example 120

### Preparation of racemic (2'R, 3R, 4'S)-6-chloro-4'-(3-chlorophenyl)-1'-[(2-hydroxyethyl)aminocarbonyl-methyl]-2'-isopropenyl-spiro[3H-indole-3,3'-piperidine]-2,6'(1H)-dione

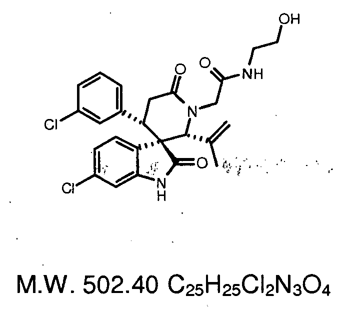

In a manner similar to the method described in example 34b, racemic (2'R, 3R, 4'S)-6-chloro-4'-(3-chlorophenyl)-1'-fluorocarbonylmethyl-2'-isopropenyl spiro[3H-indole-3,3'-piperidine]-2,6'(1H)-dione (0.2 g, 0.433 mmol) prepard in example 116d was reacted with 2-amino-ethanol (61 mg, 0.86 mmol), N-methylmorpholine (90 mg, 0.86 mmol) and 4-dimethylaminopyridine (5 mg) in tetrahydrofuran to give racemic (2'R, 3R, 4'S)-6-chloro-4'-(3-chlorophenyl)-1'-[(2-hydroxy-ethyl)aminocarbonyl-methyl]-2'-isopropenyl-spiro[3H-indole-3,3'-piperidine]-2,6'(1H)-dione as an off-white solid (Yield: 80 mg, 36.8%).

HRMS(ES⁺) *m*/*z* Calcd for C₂₅H₂₅Cl₂N₃O₄ +H [(M+H)⁺]: 502.1295 Found: 502.1296.

### Example 121

### Preparation of racemic (2'R, 3R, 4'S)-6-chloro-4'-(3-chlorophenyl)-1'-[(2-hydroxy-1,1-dimethyl-ethyl)aminocarbonyl-methyl]-2'-isopropenyl-spiro[3H-indole-3,3'-piperidine]-2,6'(1H)-dione .

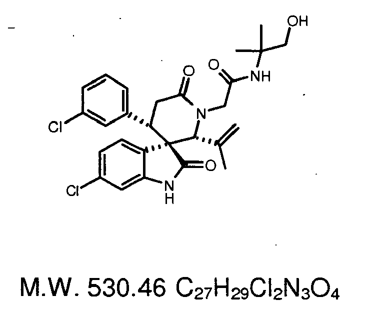

In a manner similar to the method described in example 34b, racemic (2'R, 3R, 4'S)-6-chloro-4'-(3-chlorophenyl)-1'-fluorocarbonylmethyl-2'-isopropenyl spiro[3H-indole-3,3'-piperidine]-2,6'(1H)-dione (0.18 g, 0.39 mmol) prepared in example 116d was reacted with 2-amino-2-methyl-propan-1-ol (69 mg, 0.78 mmol), N-methylmorpholine (81 mg, 0.78 mmol) and 4-dimethylaminopyridine (5 mg) in tetrahydrofuran to give racemic (2'R, 3R, 4'S)-6-chloro-4'-(3-chlorophenyl)-1'-[(2-hydroxy-1,1-dimethyl-ethyl)aminocarbonyl-methyl]-2'-isopropenyl-spiro[3H-indole-3,3'-piperidine]-2,6'(1H)-dione as an off-white solid (Yield: 74 mg, 35.9%).

HRMS(ES⁺) *m*/*z* Calcd for C₂₇H₂₉Cl₂N₃O₄ +H [(M+H)⁺]: 502.1295 Found: 502.1296.

### Example 122a

### Preparation of intermediate Z-(6-Chloro-2-oxo-1,2-dihydro-indol-3-ylidene)-(3-chloro-phenyl)-acetonitrile

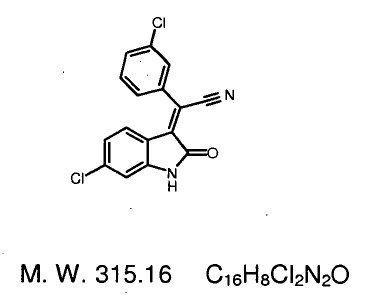

To the mixture of 3-chlorobenzyl cyanide (1.8 g, 12 mmol) (Aldrich) and 6-chloroisotin (1.81 g, 10 mmol) (TCI-US) in ethanol (40 mL) was added DBU (1.9 g, 12.5 mmol) (Aldrich). The reaction mixture was heated at 100 °C for 1 h. TCL analysis indicated the formation of mixture of E- and Z- isomers of the desired product and almost complete consumption of starting materials. The mixture was cooled to room temperature, concentrated to a small volume, then diluted with ethyl ether, washed with dilute HCl aqueous solution. The organic layer was separated, dried over Na₂SO₄, concentrated, and purified by chromatography (EtOAc:hexanes=1:2) to give the desire Z-(6-Chloro-2-oxo-1,2-dihydro-indol-3-ylidene)-(3-chloro-phenyl)-acetonitrile as an orange solid (0.9 g, 29%). The other isomer E-(6-Chloro-2-oxo-1,2-dihydro-indol-3-ylidene)-(3-chloro-phenyl)-acetonitrile was also obtained as an orange solid (0.9 g, 29%).

### Example 122b

### Preparation of intermediate Z-6-Chloro-3-[(3-chloro-phenyl)-cyano-methylene]-2-oxo-2,3-dihydro-indole-1-carboxylic acid tert-butyl ester

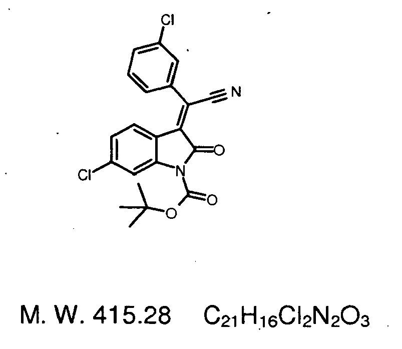

In a manner similar to the method described in example 24a, Z-(6-Chloro-2-oxo-1,2-dihydro-indol-3-ylidene)-(3-chloro-phenyl)-acetonitrile (0.4 g, 1.3 mmoll) was reacted with di-*tert*-butyl-dicarbonate (1.9 g, 8.6 mmol) (Aldrich), triethylamine (0.87 g, 8.6 mmol) and 4-dimethylaminopyridine (5 mg) in dichloromethane (100 mL) to give Z-6-Chloro-3-[(3-chloro-phenyl)-cyano-methylene]-2-oxo-2,3-dihydro-indole-1-carboxylic acid tert-butyl ester as a yellow solid (Yield: 0.3 g, 56%).

### Example 122c

### Preparation of racemic (2'R, 3R, 4'R)-6-chloro-4'-(3-chloro-phenyl)-4'-cyano-2'-(1-methylene-propyl) spiro[3H-indole-3,3'-piperidine]-2,6'(1H)-dione

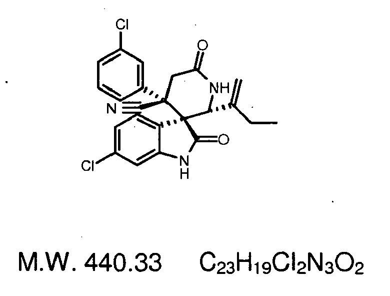

In a manner similar to the method described in example 41b, Z-6-Chloro-3-[(3-chloro-phenyl)-cyano-methylene]-2-oxo-2,3-dihydro-indole-1-carboxylic acid tert-butyl ester prepared in example 122b (0.3 g, 0.72 mmol) was reacted with 1-(1-methylene-propyl)-3-trimethylsilyoxy-2-aza-1,3-butadiene (2 g, 10 mmol) prepared in example 80a in toluene and then trifluoroacetic acid (10 mL) in dichloromethane to give racemic (2'R, 3R, 4'R)-6-chloro-4'-(3-chloro-phenyl)-4'-cyano-2'-(1-methylene-propyl) spiro[3H-indole-3,3'-piperidine]-2,6'(1H)-dione as a off white solid (0.1 g, 31%).

HRMS(ES⁺) *m*/*z* Calcd for C₂₃H₁₉Cl₂N₃O₂+ H [(M+H)⁺]: 440.0927. Found: 440.0927.

### Example 123a

### Preparation of intermediate racemic (2'R, 3R, 4'S)-6-chloro-4'-(3-chloro-phenyl)-1'-(3-chloro-propyl)-2'-isopropenyl-2,3-dihydro-2,6'-dioxo spiro[indole-3,3'-piperidine]-1-methoxyethyl trimethylsilane

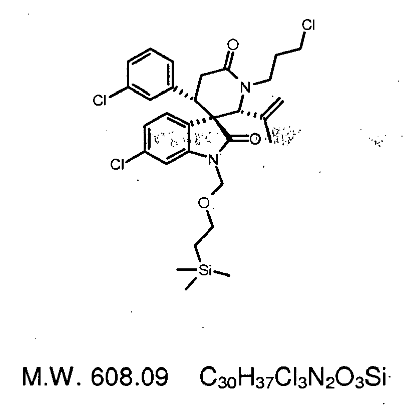

In a manner similar to the method described in example 24c, racemic (2'R, 3R, 4'S)-6-chloro-4'-(3-chloro-phenyl)-2'-isopropenyl-2,3-dihydro-2,6'-dioxo spiro[indole-3,3'-piperidine]-1-methoxyethyl trimethylsilane (1.57 g, 2.95 mmol) prepared in example 116a was reacted with LiH (2.3 g, 29.5 mmol) and 1-chloro-3-iodo-propane (6.04 g, 29.5 mmol) in N,N-dimethyl-formamide (100 mL) to give racemic (2'R, 3R, 4'S)-6-chloro-4'-(3-chloro-phenyl)-1'-(3-chloro-propyl)-2'-isopropenyl-2,3-dihydro-2,6'-dioxo spiro[indole-3,3'-piperidine]-1-methoxyethyl trimethylsilane as a white form (Yield: 0.6 g, 33.5%).

### Example 123b

### Preparation of racemic (2'R, 3R, 4'S)-1'-[3-(4-acetylamino-piperidin-1-yl)-propyl] - 6-chloro-4'-(3-chlorophenyl)-2'-isopropenyl spiro[3H-indole-3,3' piperidine]-2,6'(1H)-dione

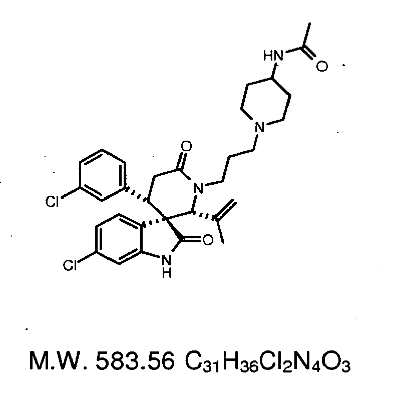

In a manner similar to the method described in example 60b, (2'R, 3R, 4'S)-6-chloro-4'-(3-chloro-phenyl)-1'-(3-chloro-propyl)-2'-isopropenyl-2,3-dihydro-2,6'-dioxo spiro[indole-3,3'-piperidine]-1-methoxyethyl trimethylsilane prepared in example 123a (0.12 g, 0.20 mmol) was reacted with N-piperidin-4-yl-acetamide (0.56 g, 4 mmol), trifluoroacetic acid (2 mL) and then N,N'-diisopropylethylamine (2 mL) to give racemic (2'R, 3R, 4'S)-1'-[3-(4-acetylamino-piperidin-1-yl)-propyl]-6-chloro-4'-(3-chlorophenyl)-2'-isopropenyl spiro[3H-indole-3,3' piperidine]-2,6'(1H)-dione as a brown solid (Yield: 47 mg, 40.8% ).

HRMS(ES⁺) *m*/*z* Calcd for C₃₁H₃₆Cl₂N₄O₃+ H [(M+H)⁺]: 583.2237 Found: 583.2239.

### Example 124a

### Preparation of intermediate E/Z-6-chloro-3-(3-chloro-benzylidene)-1,3-dihydro-indol-2-one

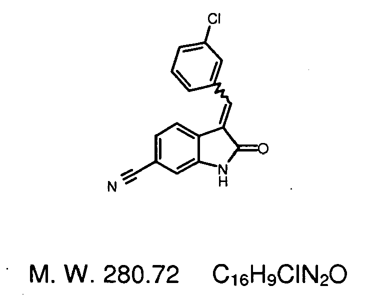

In a manner similar to the method described in example 1a, 6-cyanooxindole (1 g, 6.44 mmol) (Combi-blocks) was reacted with 3-chloro-benzaldehyde (0.73 mL, 6.44 mmol) (Aldrich) and piperidine (0.635 mL, 6.44 mmol) in methanol to give a mixture of E-and Z-6-cyano-3-(3-chloro-benzylidene)-1,3-dihydro-indol-2-one as a brown solid (Yield: 1.34 g, 74.4%).

### Example 124b

### Preparation of intermediate E/Z-6-cyano-3-(3-chloro-benzylidene)-2-oxo-2,3-dihydro-indole-1-carboxylic acid tert-butyl ester

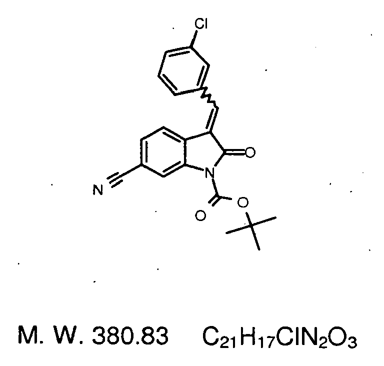

In a manner similar to the method described in example 24a, E/Z-6-chloro-3-(3-chloro-benzylidene)-1,3-dihydro-indol-2-one (1.34 g, 4.77 mmoll) was reacted with di-*tert*-butyl-dicarbonate (1.91 g, 8.73 mmol) (Aldrich), triethylamine (3.29 mL, 23.6 mmol) and 4-dimethylaminopyridine (25 mg) in dichloromethane (100 mL) to give E/Z-6-cyano-3-(3-chloro-benzylidene)-2-oxo-2,3-dihydro-indole-1-carboxylic acid *tert*-butyl ester as a yellow solid (Yield: 1.7 g, 93.4%).

### Example 124c

### Preparation of racemic (2'R, 3R, 4'S)-4'-(3-chloro-phenyl)-6'-cyano-2'-(1-methylene-propyl) spiro[3H-indole-3,3'-piperidine]-2,6'(1H)-dione

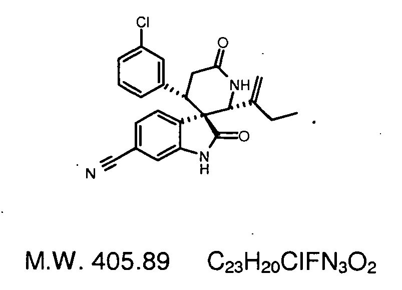

In a manner similar to the method described in example 41b, E/Z-6-cyano-3-(3-chlorobenzylidene)-2-oxo-2,3-dihydro-indole-1-carboxylic acid *tert*-butyl ester prepared in example 124b (0.4 g, 1.05 mmol) was reacted with 1-(1-methylene-propyl)-3-trimethylsilyoxy-2-aza-1,3-butadiene prepared in example 80a in toluene and then trifluoroacetic acid (20 mL) in dichloromethane to give racemic (2'R, 3R, 4'S)-4'-(3-chlorophenyl)-6'-cyano-2'-(1-methylene-propyl) spiro[3H-indole-3,3'-piperidine]-2,6'(1H)-dione as a yellow solid (Yield: 90 mg, 21.1 % ).

HRMS(ES⁺) *m*/*z* Calcd for C₂₃H₂₀ClFN₃O₂+ H [(M+H)⁺]: 406.1317 Found: 406.1316.

### Example 125

### Preparation of racemic (2'R, 3R, 4'S)-4'-(3-chloro-phenyl)-6-cyano-2'-(5-fluoro-2-methyl-phenyl) spiro[3H-indole-3,3'-piperidine]-2,6'(1H)-dione

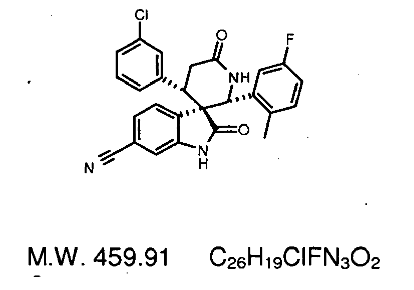

In a manner similar to the method described in example 41b, E/Z-6-cyano-3-(3-chloro-benzylidene)-2-oxo-2,3-dihydro-indole-1-carboxylic acid *tert*-butyl ester prepared in example 124b (0.4 g, 1.05 mmol) was reacted with 1-(5-fluoro-2-methyl-phenyl)-3-trimethylsilyoxy-2-aza-1,3-butadiene prepared in example 36a in toluene and then trifluoroacetic acid (20 mL) in dichloromethane to give racemic (2'R, 3R, 4'S)-4'-(3-chloro-phenyl)-6'-cyano-2'-(5-fluoro-2-methyl-phenyl) spiro[3H-indole-3,3'-piperidine]-2,6'(1H)-dione as a yellow solid (Yield: 0.12 g, 16.9%).

HRMS(ES⁺) *m*/*z* Calcd for C₂ₛH₁₉ClFN₃O₂+ H [(M+H)⁺]: 460.1223. Found: 460.1223.

### Example 126

### Preparation of racemic (2'R, 3R, 4'S)-1'-[3-(4-acetyl-piperazin-1-yl)-propyl]-6-chloro-4'-(3-chlorophenyl)-2'-isopropenyl spiro[3H-indole-3,3'- piperidine]-2,6'(H)-dione

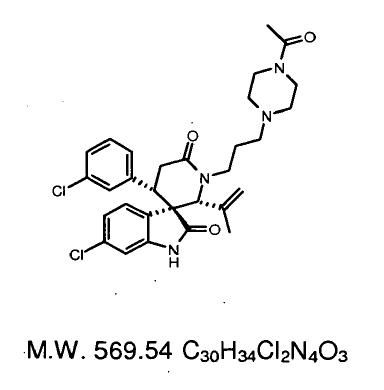

In a manner similar to the method described in example 60b, (2'R, 3R, 4'S)-6-chloro-4'-(3-chloro-phenyl)-1'-(3-chloro-propyl)-2'-isopropenyl-2,3-dihydro-2,6'-dioxo spiro[indole-3,3'-piperidine]-1-methoxyethyl trimethylsilane prepared in example 123a (0.2 g, 0.20 mmol) was reacted with 1-piperazin-1-yl-ethanone (0.51 g, 4 mmol ), trifluoroacetic acid (2 mL) and then N,N'-diisopropylethylamine (2 mL) to give racemic (2'R, 3R, 4'S)-1'-[3-(4-acetyl-piperazin-1-yl)-propyl]-6-chloro-4'-(3-chlorophenyl)-2'-isopropenyl spiro[3H-indole-3,3' piperidine]-2,6'(1H)-dione as a yellow solid (Yield: 95 mg, 50.8% ).

HRMS(ES⁺) *m*/*z* Calcd for C₃₀H₃₄Cl₂N₄O₃ + H [(M+H)⁺]: 569.2081 Found: 569.2079.

### Example 127

### Preparation of racemic (2'R, 3R, 4'S)-6-chloro-4'-(3-chlorophenyl)-2'-isopropenyl-1'-(3-piperidin-1-yl-propyl) spiro[3H-indole-3,3' piperidine]-2,6'(1H)-dione

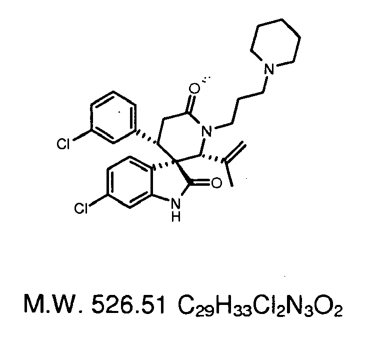

In a manner similar to the method described in example 60b, (2'R, 3R, 4'S)-6-chloro-4'-(3-chloro-phenyl)-1'-(3-chloro-propyl)-2'-isopropenyl-2,3-dihydro-2,6'-dioxo spiro[indole-3,3'-piperidine]-1-methoxyethyl trimethylsilane prepared in example 123a (0.12 g, 0.20 mmol) was reacted with piperidine (2 mL), trifluoroacetic acid (1 mL) and then N,N'-diisopropylethylamine (1.5 mL) to give racemic (2'R, 3R, 4'S)-6-chloro-4'-(3-chlorophenyl)-2'-isopropenyl-1'-(3-piperidin-1-yl-propyl) spiro[3H-indole-3,3' piperidine]-2,6'(1H)-dione as a yellow solid (Yield: 40 mg, 23.1%).

HRMS(ES⁺) *m*/*z* Calcd for C₂₉H₃₃Cl₂N₃O₂+ H [(M+H)⁺]: 526.2023 Found: 526.2020.

### Example 128a

### Preparation of intermediate Acetic acid 2-(2-formyl-4-methyl-phenoxy)-ethyl ester

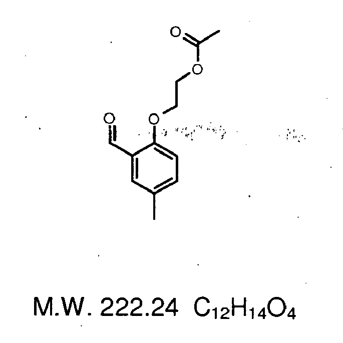

To a solution of 2-hydroxy-5-methyl-benzaldehyde (2.4 g, 18 mmol) (Aldrich) in N,N-dimethylformamide (30 mL) was added cesium carbonate (6 g, 18 mmo), potassium iodide (3 g, 18 mmol) and 2-bromoethyl acetate (7 g, 42 mmol), Aldrich). The reaction mixture was heated at 100 °C for 3 h. The mixture was cooled to room temperature and diluted with ethyl ether, washed with water, brine, separated, and concentrated. The residue was purified by chromatography (EtOAc:Hexanes=1:6) to give acetic acid 2-(2-formyl-4-methyl-phenoxy)-ethyl ester as a yellow oil (Yield 4 g, 100%).

### Example 128b

### Preparation of intermediate 1-[2-(2-Acetoxy-ethoxy)-5-methyl-phenyl]-3-trimethylsilyoxy-2-aza-1,3-butadiene

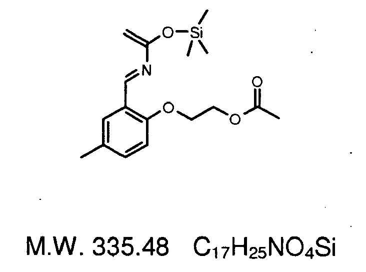

In a manner similar to the method described in example 1b, acetic acid 2-(2-formyl-4-methyl-phenoxy)-ethyl ester (2.5 g, 11 mmol) prepared in example 128a was reacted with 1,1,1,3,3,3-hexamethyldisilazane (1.6 g, 10 mmol), *n-*butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-[2-(2-acetoxy-ethoxy)-5-methyl-phenyl]-3-trimethylsilyoxy-2-aza-1,3-butadiene as yellow oil and used for the next step without further purification.

### Example 128c

### Preparation of racemic (2'R, 3R, 4'S)-2'-[2-(2-acetoxy-ethoxy)-5-methyl-phenyl]-6-chloro-4'-(3-chlorophenyl) spiro[3H-indole-3,3'-piperidine]-2,6'(1H)-dione

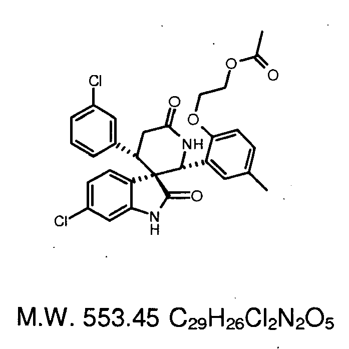

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.5 g, 1.28 mmol) was reacted with 1-[2-(2-acetoxy-ethoxy)-5-methyl-phenyl]-3-trimethylsilyoxy-2-aza-1,3-butadiene (2 g, 6 mmol) prepared in example 128b in toluene and then trifluoroacetic acid (10 mL) in dichloromethane to give racemic (2'R, 3R, 4'S)-2'-[2-(2-acetoxy-ethoxy)-5-methyl-phenyl]-6-chloro-4'-(3-chlorophenyl) spiro[3H-indole-3,3'-piperidine]-2,6'(1H)-dione as a white solid (0.2 g, 28% ).

HRMS(ES⁺) *m*/*z* Calcd for C₂₉H₂₆Cl₂N₂O₅+ H [(M+H)⁺]: 553.1292. Found: 553.1291.

### Example 129

### Preparation of racemic (2'R, 3R, 4'S)-6-chloro-4'-(3-chlorophenyl)-2'-[6-(2-hydroxy-ethoxy)-3-methyl-phenyl] spiro[3H-indole-3,3'-piperidine]-2,6'(1H)-dione

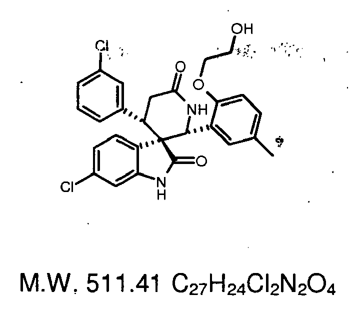

To a solution of racemic (2'R, 3R, 4'S)-2'-[2-(2-acetoxy-ethoxy)-5-methyl-phenyl]-6-chloro-4'-(3-chlorophenyl) spiro[3H-indole-3,3'-piperidine]-2,6'(1H)-dione (0.16 g, 0.29 mmol) prepared in example 128c in tetrahydrofuran (30 mL) and methanol (10 mL) was added sodium hydroxide aqueous solution (1N, 10 mL). the reaction mixture was stirred at room temperature for 3 h, then neutralized to "pH" 7 by aqueous HCl. The mixture was then extracted with ethyl acetate. The organic layer was separated, concentrated, and purified by chromatography (EtOAc:MeOH=19:1) to give racemic (2'R, 3R, 4'S)-6-chloro-4'-(3-chlorophenyl)-2'-[2-(2-hydroxy-ethoxy)-5-methyl-phenyl] spiro[3H-indole-3,3'-piperidine]-2,6'(1H)-dione as a white solid (70 mg, 47%)

HRMS(ES⁺) *m*/*z* Calcd for C₂₇H₂₄Cl₂N₂O₄+ H [(M+H)⁺]: 511.1186. Found: 511.1185.

### Example 130a

### Preparation of intermediate 5-bromo-2-[2-(tert-butyl-dimethyl-silanyloxy)-ethoxy]-benzaldehyde

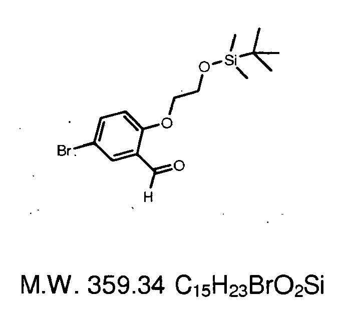

To a solution of 5-bromo-2-hydroxy-benzaldehyde (14 g, 69.6 mmol) (Aldrich) in N, N-dimethylformamide (300 mL) was added K₂CO₃ (29 g, 208.9 mmo) and (2-bromo-ethoxy)-tert-butyl-dimethyl-silane (20 g, 83.5 mmol) (Aldrich). The reaction mixture was stirred at 60 °C for 16 h. The reaction mixture was cooled to room temperature and poured into water, extracted with ethyl acetate. The organic layers were combined, washed with water, brine, dried over MgSO₄, filtered and concentrated to give 5-bromo-2-[2-(tert-butyl-dimethyl-silanyloxy)-ethoxy]-benzaldehyde as brown oil (Yield: 25 g, 100 %).

### Example 130b

### Preparation of intermediate 2-[2-(tert-butyl-dimethyl-silanyloxy)-ethoxy]-5-cyclopropyl-benzaldehyde

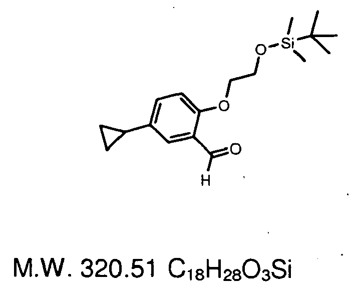

To a solution of 5-bromo-2-[2-(tert-butyl-dimethyl-silanyloxy)-ethoxy]-benzaldehyde (5 g, 13.9 mmol) prepared in example 130a in toluene (75 mL) and water (7.5 mL) was added cyclopropylboronic acid (1.7 g, 19.8 mmol) (Aldrich) and potassium phosphate (14.6 g, 68.9 mmol). After the reaction mixture was degassed for 5 min, dichloro-bis-(tricyclohexyl-phosphine) (1.03 g, 1.39 mmol) (Strem) was added and the reaction mixture was heated at 100 °C under nitrogen for 4 h. The reaction mixture was cooled to room temperature and diluted with water, extracted with ethyl acetate. The organic layers were combined, washed with water, brine, dried over MgSO₄, filtered and concentrated. The residue was purified with chromatography to give 2-[2-(tert-butyl-dimethyl-silanyloxy)-ethoxy]-5-cyclopropyl-benzaldehyde as yellow oil (Yield: 3.2 g, 76.2 %).

### Example 130c

### Preparation of intermediate 1-{2-[2-(tert-butyl-dimethyl-silanyloxy)-ethoxy]-5-cyclopropyl-phenyl}-3-trimethylsilyoxy-2-aza-1,3-butadiene

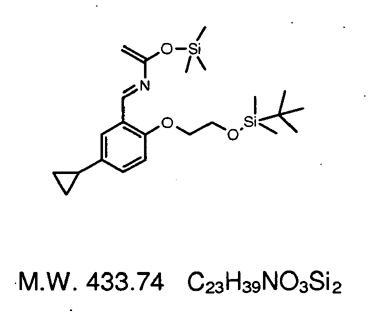

In a manner similar to the method described in example 1b, 2-[2-(tert-butyldimethyl-silanyloxy)-ethoxy]-5-cyclopropyl-benzaldehyde (3.36 g, 10.5 mmol) prepared in example 130b was used as the starting material in place of 3-chloro-benzaldehyde to react with 1,1,1,3,3,3-hexamethyldisilazane (2.18 mL, 10.5 mmol), *n*-butyllithium (2.5 M, 4.2 mL, 10.5 mmol), trimethylsilyl chloride (1.33 mL, 10.5 mmol), triethylamine (1.9 mL, 13.6 mmol) and acetyl chloride (0.97 mL, 13.6 mmol) to give 1-{2-[2-(tert-butyl-dimethyl-silanyloxy)-ethoxy]-5-cyclopropyl-phenyl}-3-trimethylsilyoxy-2-aza-1,3-butadiene as a yellow gum and used for the next step without further purification.

### Example 130d

### Preparation of racemic (2'R, 3R, 4'S)-6-chloro-4'-(3-chlorophenyl)-2'-[5-cyclopropyl-2-(2-hydroxy-ethoxy)-phenyl]spiro[3H-indole-3,3'-piperidine]-2,6'(1H)-dione

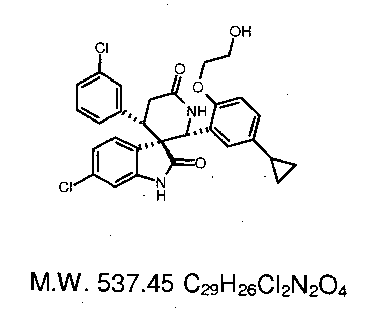

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.5 g, 1.28 mmol) was reacted with 1-{2-[2-(tert-butyldimethyl-silanyloxy)-ethoxy]-5-cyclopropyl-phenyl}-3-trimethylsilyoxy-2-aza-1,3-butadiene prepared in example 130c in toluene and then trifluoroacetic acid (20 mL) in dichloromethane to give racemic (2'R, 3R, 4'S)-6-chloro-4'-(3-chlorophenyl)-2'-[5-cyclopropyl-2-(2-hydroxy-ethoxy)-phenyl] spiro[3H-indole-3,3'-piperidine]-2,6'(1H)-dione as a white solid (0.14 g, 20.3% ).

HRMS(ES⁺) *m*/*z* Calcd for C₂₉H₂₆Cl₂N₂O₄ + H [(M+H)⁺]: 537.1343 Found: 537.1343.

### Example 131a

### Preparation of intermediate 2-[2-(tert-Butyl-dimethyl-silanyloxy)-ethoxy]-5-chloro-benzaldehyde

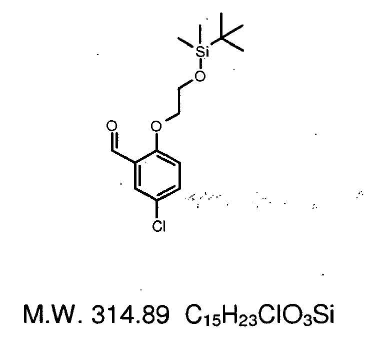

To a solution of 5-chlorosalicylaldehyde (5 g, 32 mmol) (Aldrich) in N,N-dimethylformamide (40 mL) was added potassium carbonate (20 g, 145 mmo), and (2-bromo-ethoxy)-tert-butyl-dimethyl-silane (10 g, 42 mmol, Aldrich). The reaction mixture was heated at 60 °C for 18 h. The crude was cooled to room temperature, diluted with ethyl acetate, washed with water, brine. The organic layer was separated, concentrated, and the residue was purified by chromatography (EtOAc:Hexanes=1:8, then 1:4) to give 2-[2-(tert-butyl-dimethyl-silanyloxy)-ethoxy]-5-chloro-benzaldehyde as a white solid (Yield 10 g, 99%).

### Example 131b

### Preparation of intermediate 1-[2-(tert-butyl-dimethyl-silanyloxy)-ethoxy]-5-chlorophenyl]-3-trimethylsilyoxy-2-aza-1,3-butadiene

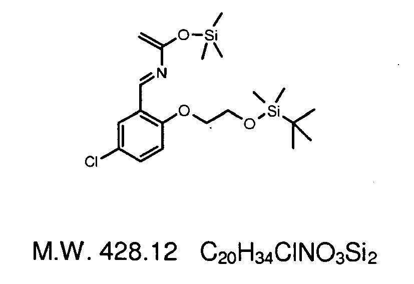

In a manner similar to the method described in example 1b, 2-[2-(tert-butyldimethyl-silanyloxy)-ethoxy]-5-chloro-benzaldehyde (5.2 g, 17 mmol) prepared in example 131a was reacted with 1,1,1 ,3,3,3-hexamethyldisilazane (2.4g, 15 mmol), *n*-butyllithium (2.5 M, 6 mL, 15 mmol), trimethylsilyl chloride (1.6 g, 15 mmol), triethylamine (1.6 g, 20 mmol) and acetyl chloride (1.5 g, 20 mmol) to give 1-[2-(tert-butyl-dimethyl-silanyloxy)-ethoxy]-5-chloro-phenyl]-3-trimethylsilyoxy-2-aza-1,3-butadiene as yellow oil and used for the next step without further purification.

### Example 131c

### Preparation of racemic (2'R, 3R, 4'S)-6-chloro-2'-[5-chloro-2-(2-hydroxy-ethoxy)-phenyl]-4'-(3-chlorophenyl) spiro[3H-indole-3,3'-piperidine]-2,6'(1H)-dione

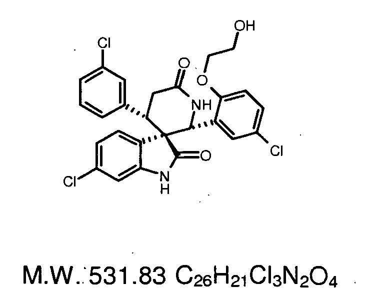

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.6 g, 1.5 mmol) was reacted with 1-[2-(tert-butyldimethyl-silanyloxy)-ethoxy]-5-chloro-phenyl]-3-trimethylsilyoxy-2-aza-1,3-butadiene (5.2 g, 12 mmol) prepared in example 131b in toluene and then trifluoroacetic acid (10 mL) in dichloromethane to give racemic (2'R, 3R, 4'S)-6-chloro-2'-[5-chloro-2-(2-hydroxy-ethoxy)-phenyl]-4'-(3-chlorophenyl) spiro[3H-indole-3,3'-piperidine]-2,6'(1H)-dione as a white solid (0.25 g, 31 % ).

HRMS(ES⁺) *m*/*z* Calcd for C₂₆H₂₁Cl₃N₂O₄+ H [(M+H)⁺]: 531.0640. Found: 531.0640.

### Example 132a

### Preparation of intermediate tert-Butyl-[2-(4-chloro-3-fluoro-phenoxy)-ethoxy]-dimethyl-silane

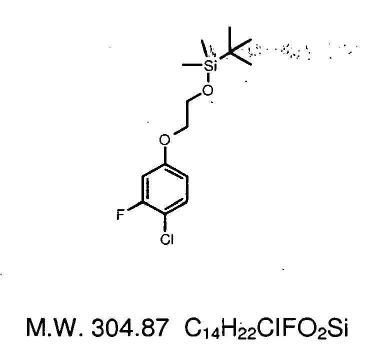

To a solution of 4-chloro-3-fluoro-phenol (10 g, 68 mmol) in N, N-dimethyformamide (50 mL) was added potassium carbonate (19 g, 136 mmol), potassium iodide (11 g, 68 mmol), and (2-bromo-ethoxy)-tert-butyl-dimethylsilane (18 g, 75 mmol, Aldrich). The reaction mixture was heated at 100 °C for 24 h. The crude was cooled, diluted with ethyl ether, washed with water, NaHCO₃ solution, and brine. The organic layer was separated, dried over Na₂SO₄, and concentrated. The filtrate was concentrated and the residue was purified by chromatography (EtOAc:Hexanes=1:20) to give tert-butyl-[2-(4-chloro-3-fluoro-phenoxy)-ethoxy]-dimethyl-silane as colorless oil (Yield 14 g, 69%).

### Example 132b

### Preparation of intermediate 6-[2-(tert-Butyl-dimethyl-silanyloxy)-ethoxy]-3-chloro-2-fluoro-benzaldehyde

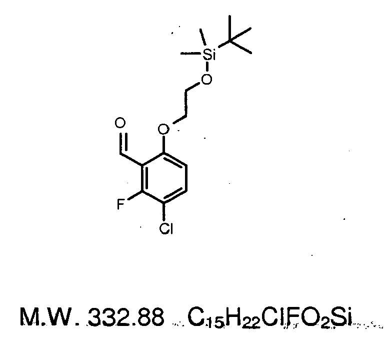

In a manner similar to the method described in example 52a, tert-butyl-[2-(4-chloro-3-fluoro-phenoxy)-ethoxy]-dimethyl-silane (14.3 g, 47 mmol) prepared in example 132a was reacted with lithium diisopropylamine (34 mL, 1.8 M in THF, 61 mmol), N,N-dimethyl-formamide (4.7 mL, 61 mmol) and quenched with acetic acid (14 g, 234 mmol) in tetrahydrofuran to give 6-[2-(tert-butyl-dimethyl-silanyloxy)-ethoxy]-3-chloro-2-fluoro-benzaldehyde as a white solid (Yield: 6.4 g, 41%).

### Example 132c

### Preparation of intermediate 1-[2-(tert-butyl-dimethyl-silanyloxy)-ethoxy-3-chloro-2-fluoro-phenyl]-3-trimethylsilyoxy-2-aza-1,3-butadiene

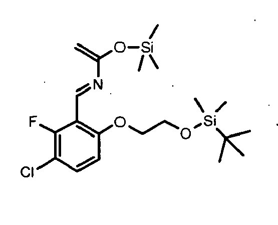

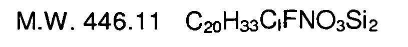

In a manner similar to the method described in example 1b, 6-[2-(tert-butyldimethyl-silanyloxy)-ethoxy]-3-chloro-2-fluoro-benzaldehyde (3.3 g, 10 mmol) prepared in example 132b was reacted with 1,1,1,3,3,3-hexamethyldisilazane (1.6 g, 10 mmol), *n*-butyllithium (2.5 M, 4 mL, 10 mmol), trimethylsilyl chloride (1.1 g, 10 mmol), triethylamine (1.4 g, 13 mmol) and acetyl chloride (1.0 g, 13 mmol) to give 1-[2-(tert-butyl-dimethyl-silanyloxy)-ethoxy-3-chloro-2-fluorophenyl]-3-trimethylsilyoxy-2-aza-1,3-butadiene as an orange oil and used for the next step without further purification.

### Example 132d

### Preparation of racemic (2'R, 3R, 4S)-6-chloro-2'-[3[-chloro-2-fluoro-6-(2-hydroxy-ethoxy)-phenyl]-4'-(3-chloropheriyl) spiro[3H-indole-3,3'-piperidine]-2,6'(1H)-dione

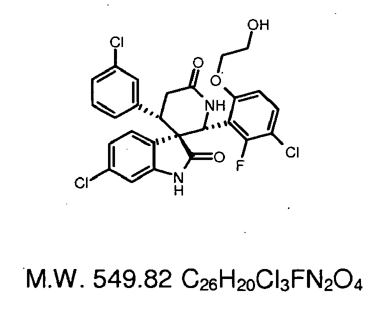

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.5 g, 1.28 mmol) was reacted with 1-[2-(tert-butyldimethyl-silanyloxy)-ethoxy-phenyl]-3-trimethylsilyoxy-2-aza-1,3-butadiene (3 g, 6.7 mmol) prepared in example 132c in toluene and then trifluoroacetic acid (10 mL) in dichloromethane to give racemic (2'R, 3R, 4'S)-6-chloro-2'-[3-chloro-2-fluoro-6-(2-hydroxy-ethoxy)-phenyl]-4'-(3-chlorophenyl) spiro[3H-indole-3,3'-piperidine]-2,6'(1H)-dione as a white solid (0.28 g, 40%).

HRMS(ES⁺) *m*/*z* Calcd for C₂₆H₂₀Cl₃FN₂O₄ + H [(M+H)⁺]: 549.0546 Found: 549.0545.

### Example 133a

### Preparation of intermediate E/Z-6-Chloro-3-(3,5-difluoro-benzylidene)-1-(2-trimethylsilanyl-ethoxymethyl)-1,3-dihydro-indol-2-one

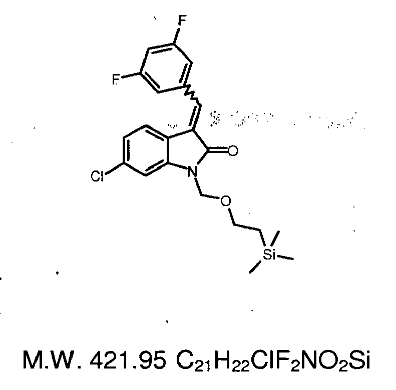

In a manner similar to the methods described in example 4a, 55a, 3,5-difluoro-benzaldehyde (0.89 g, 6.27 mmol) in place of 3-chlorobenzaldehyde was reacted in two steps to give E/Z-6-Chloro-3-(3,5-difluoro-benzylidene)-1-(2-trimethylsilanyl-ethoxymethyl)-1,3-dihydro-indol-2-one as a yellow oil (1.25 g, 50% for two steps).

### Example 133b

### Preparation of racemic (2'R, 3R, 4'S)-6-chloro-4'-(3,5-difluoro-phenyl)-2'-(1-methylene-propyl) spiro[3H-indole-3,3'-piperidine]-2,6'(1H)-dione

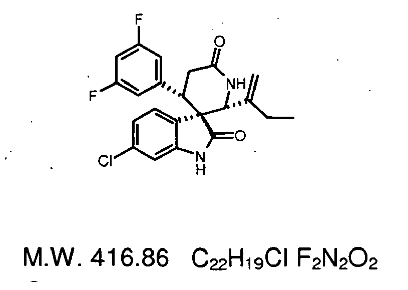

In a manner similar to the method described in example 97a, 55d, E/Z-6-Chloro-3-(3,5-difluoro-benzylidene)-1-(2-tri methylsilanyl-ethoxymethyl)-1,3-di hydro-i ndol-2-one (0.21 g, 0.5 mmol) prepared in example 133a was reacted with 1-(1-methylene-propyl)-3-trimethylsilyoxy-2-aza-1,3-butadiene (1 g, 5.25 mmol) prepared in example 80a in toluene, then treated with trifluoroacetic acid (5 ml) in dichloromethane, followed by treatment with diisopropylethylamine (1 mL) in methanol to give racemic (2'R, 3R, 4'S)-6-chloro-4'-(3-chlorophenyl)-2'-(1-methylene-propyl) spiro[3H-indole-3,3'-piperidine]-2,6'(1H)-dione as a white solid (35 mg, 18% for two steps).

HRMS(ES+) *m*/*z* Calcd for C₂₂H₁₉ClN₂O₂F₂+H[(M+H)]: 417.1176; Found: 417.1177

### Example 134a

### Preparation of intermediate E/Z-3-(6-Chloro-2-oxo-1,2-dihydro-indol-3-ylidenemethyl)-benzonitrile

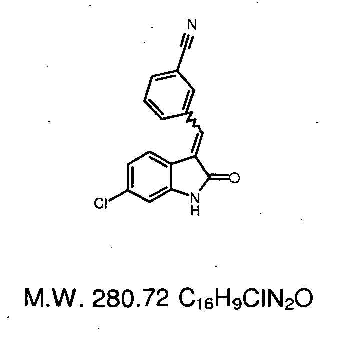

In a manner similar to the method described in example 1a, 3-formyl-benzonitrile was used in place of 3-chlorobenzaldehyde to form E/Z-3-(6-Chloro-2-oxo-1,2-dihydro-indol-3-ylidenemethyl)-benzonitrile as a yellow solid.

### Example 134b

### Preparation of racemic (2'R, 3R, 4'S)-6-chloro-4'-(3-cyano-phenyl)-2'-(1-methylene-propyl) spiro[3H-indole-3,3'-piperidine]-2,6'(1H)-dione

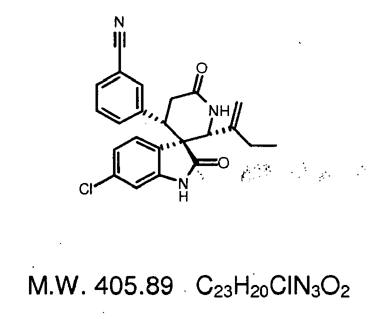

E/Z-3-(6-Chloro-2-oxo-1,2-dihydro-indol-3-ylidenemethyl)-benzonitrile (280 mg, 1 mmol) prepared in example 134a was heated with 1-(1-methylene-propyl)-3-trimethylsilyoxy-2-aza-1,3-butadiene freshly prepared in example 80a (2.06 g, 10.5 mmol) in toluene (20 mL) in a sealed tube at 135 °C for 1 h, then cooled to room temperature. Methanol (80 mL) was added, and the mixture was filtered through a short pad of celite. The filtrate was concentrated, the residue was purified by chromatography (EtOAc) to give racemic (2'R, 3R, 4'S)-6-chloro-4'-(3-cyano-phenyl)-2'-(1-methylene-propyl) spiro[3H-indole-3,3'-piperidine]-2,6'(1H)-dione as a solid (180 mg, 35%).

HRMS(ES+)*m*/*z* Calcd for C₂₃H₂₀ClN₃O₂+H [(M+H)]:406.1317; Found: 406.1315

### Example 135

### Preparation of racemic (2'R, 3R, 4'S)-4'-(3-Bromo-phenyl)-6-chloro-2'-(1-methylene-propyl) spiro[3H-indole-3,3'-piperidine]-2,6'(1H)-dione

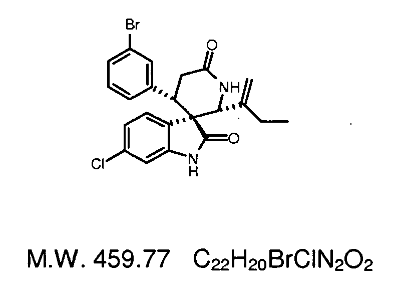

In a manner simitar to the methods described in example 134a, 134b, 3-bromobenzaldehyde was used in place of 3-formyl-benzonitrile in two steps to give racemic (2'R, 3R, 4'S)-4'-(3-bromo-phenyl)-6-chloro-2'-(1-methylene-propyl) spiro[3H-indole-3,3'-piperidine]-2,6'(1H)-dione as a solid (142 mg).

HRMS(ES+) *m*/*z* Calcd for C₂₂H₂₀BrClN₂O₂+H [(M+H)]:459.0470; Found: 459.0469

### Example 136

### Preparation of racemic (2'R, 3R, 4'S)-6-chloro-4'-(3-methoxy-phenyl)-2'-(1-methylene-propyl) spiro[3H-indole-3,3'-piperidine]-2,6'(1H)-dione

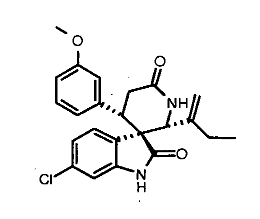

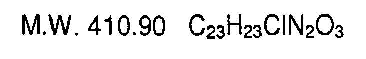

In a manner similar to the methods described in example 134a, 134b, 3-methoxy-benzaldehyde was used in place of 3-formyl-benzonitrile to give racemic (2'R, 3R, 4'S)-6-chloro-4'-(3-methoxy-phenyl)-2'-(1-methylene-propyl) spiro[3H-indole-3,3'-piperidine]-2,6'(1H)-dione as a solid (20 mg).

HRMS(ES+) m/z Calcd for C₂₃H₂₃ClN₂O₃+H[(M+H)]:433.1289; Found; 433.1288

### Example 137

### Preparation of racemic (2'R, 3R, 4'S)-6-chloro-4'-(5-fluoro-2-methyl-phenyl)-2'-(1-methylene-prppyl) spiro[3H-indole-3,3'-piperidine]-2,6'-(1H)-dione

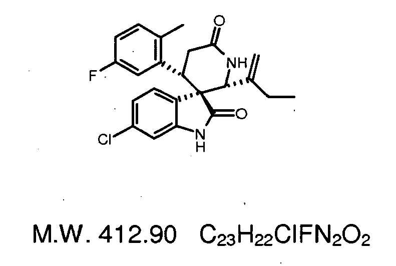

In a manner similar to the methods described in example 134a, 134b, 5-fluoro-2-methyl-benzaldehyde was used in place of 3-formyl-benzonitrile to give racemic (2'R, 3R, 4'S)-6-chloro-4'-(5-fluoro-2-methyl-phenyl)-2'-(1-methylene-propyl) spiro[3H-indole-3,3'-piperidine]-2,6'(1H)-dione as a white solid (Yield 100 mg, 15%).

HRMS(ES+) *m*/*z* Calcd for C₂₃H₂₂ClFN₂O₂+Na[(M+Na)]: 435.1246; Found:435.1243

### Example 138

### Preparation of racemic (2'R, 3R, 4'S)-6-chloro-4'-(3-fluoro-phenyl)-2'-(1-methylene-propyl)spiro[3H-indole-3,3'-piperidine]-2,6'(1H)-dione

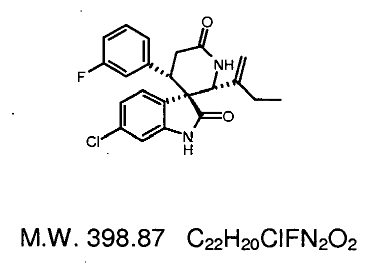

In a manner similar to the methods described in example 134a, 134b, 3-fluoro-benzaldehyde was used in place of 3-formyl-benzonitrile to give racemic (2'R, 3R, 4'S)-6-chloro-4'-(3-fluoro-phenyl)-2'-(1-methylene-propyl) spiro[3H-indole-3,3'-piperidine]-2,6'(1H)-dione as a white solid (Yield 100 mg, 17%).

HRMS(ES+) *m*/*z* Calcd for C₂₂H₂₀ClFN₂O₂+H [(M+H)]:399.1270; Found:399.1267

### Example 139

### Preparation of racemic (2'R, 3R, 4'S)-6-chloro-2'-(1-methylene-propyl)-4'-m-tolyl spiro[3H-indole-3,3'-piperidine]-2,6'(1H)-dione

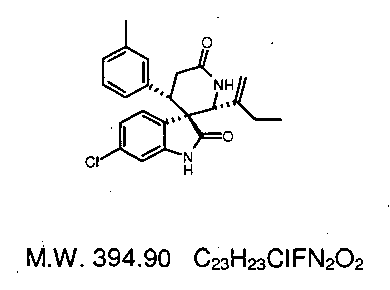

In a manner similar to the methods described in example 134a, 134b, 3-methylbenzaldehyde was used in place of 3-formyl-benzonitrile to give racemic (2'R, 3R, 4'S)-6-chloro-2'-(1-methylene-propyl)-4'-m-tolyl spiro[3H-indole-3,3'-piperidine]-2,6'(1H)-dione as a white solid (Yield 130 mg, 33%).

HRMS(ES+) *m*/*z* Calcd for C₂₃H₂₃ClFN₂O₂+H [(M+H)]:395.1521; Found: 395.1521

### Example 140

### Preparation of racemic (2'R, 3R, 4'S)-6-chloro-2'-(1-methylene-propyl)-4'-o-tolyl spiro[3H-indole-3,3'-piperidine]-2,6'(1H)-dione

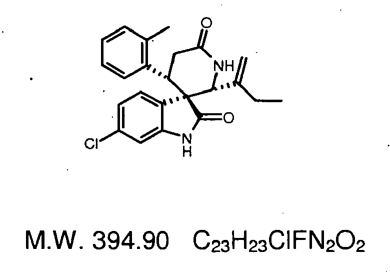

In a manner similar to the methods described in example 134a, 134b, 2-methylbenzaldehyde was used in place of 3-formyl-benzonitrile to give racemic (2'R, 3R, 4'S)-6-chloro-2'-(1-methylene-propyl)-4'-o-tolyl spiro[3H-indole-3,3'-piperidine]-2,6'(1H)-dione as a white solid (Yield 55 mg, 14%).

HRMS(ES+) *m*/*z* Calcd for C₂₃H₂₃ClFN₂O₂+H[(M+H)]:395.1521; Found:395.1521

### Example 141

### Preparation of racemic (2'R, 3R, 4'S)-6-chloro-2'-(1-methylene-propyl)-4'-thiophen-3-yl spiro[3H-indole-3,3'-piperidine]-2,6'(1H)-dione

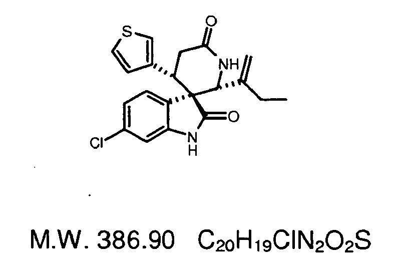

In a manner similar to the methods described in example 134a, 134b, thiophene-3-carbaldehyde was used in place of 3-formyl-benzonitrile to give racemic (2'R, 3R, 4'S)-6-chloro-2'-(1-methylene-propyl)-4'-thiophen-3-yl spiro[3H-indole-3,3'-piperidine]-2,6'(1H)-dione as a white solid (Yield 74 mg, 19%).

HRMS(ES+) *m*/*z* Calcd for C₂₀H₁₉ClN₂O₂S +H[(M+H)]:387.0929; Found:387.0929

### Example 142

### Preparation of racemic (2'R, 3R, 4'S)-6-chloro-4'-(3,5-dichloro-phenyl)-2'-(1-methylene-propyl) spiro[3H-indole-3,3'-piperidine]-2,6'(1H)-dione

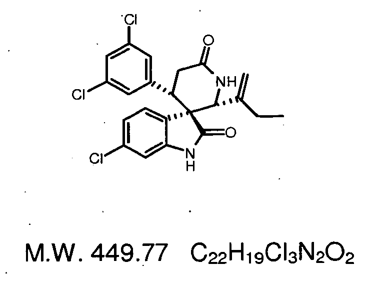

In a manner similar to the methods described in example 134a, 134b, 3,5-dichloro-benzaldehyde was used in place of 3-formyl-benzonitrile to give racemic (2'R, 3R, 4'S)-6-chloro-4'-(3,5-dichloro-phenyl)-2'-(1-methylene-propyl) spiro[3H-indole-3,3'-piperidine]-2,6'(1H)-dione as a white solid (Yield 50 mg, 11%).

HRMS(ES+)*m*/*z* Calcd for C₂₂H₁₉Cl₃N₂O₂+H [(M+H)]: 449.0585; Found:449.0585

### Example 143

### Preparation of racemic (2'R, 3R, 4'S)-6-chloro-4'-(5-chloro-2-trifluoromethylphenyl)-2'-(1-methylene-propyl) spiro[3H-indole-3,3'-piperidine]-2,6'(1H)-dione

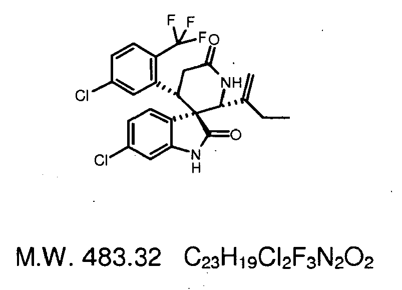

in a manner similar to the methods described in example 134a, 134b, 5-chloro-2-trifluoromethyl-benzaldehyde was used in place of 3-formyl-benzonitrile to give racemic (2'R, 3R, 4'S)-6-chloro-4'-(5-chloro-2-trifluoromethyl-phenyl)-2'-(1-methylene-propyl) spiro[3H-indole-3,3'-piperidine]-2,6'(1H)-dione as a white solid (Yield 50 mg, 10%).

HRMS(ES+) *m*/*z* Calcd for C₂₃H₁₉Cl₂F₃N₂O₂+H[(M+H)]:483.0849; Found:483.0848

### Example 144

### Preparation of racemic (2'R, 3R, 4'S)-4'-(3-Bromo-phenyl)-6-chloro-2'-isopropenyl spiro[3H-indole-3,3'-piperidine]-2,6'(1H)-dione

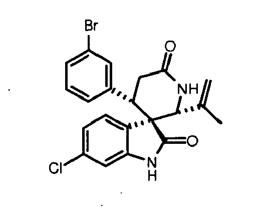

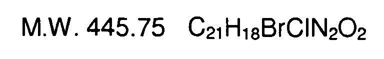

In a manner similar to the method described in example 197, 1- isopropenyl-3-trimethylsilyoxy-2-aza-1,3-butadiene freshly prepared in example 142a was used in place of 1-(1-methylene-propyl)-3-trimethylsilyoxy-2-aza-1,3-butadiene prepared in example 135a to give racemic (2'R, 3R, 4'S)-4'-(3-bromo-phenyl)-6-chloro-2'-isopropenyl spiro[3H-indole-3,3'-piperidine]-2,6'(1H)-dione as a white solid (Yield 21 mg).

HRMS(ES+) *m*/*z* Calcd for C₂₁H₁₈BrClN₂O₂+H [(M+H)]:445.0313; Found:445.0313

### Example 145a

### Preparation of intermediate E/ Z-6-chloro-3-(5-fluoro-2-methyl-benzylidene)-1,3-dihydro-indol-2-one

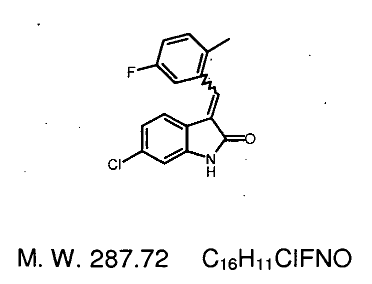

To the mixture of 6-chlorooxindole (3.6 g, 21.6 mmol) and 5-Fluoro-2-methylbenzaldehyde (3.0 g, 21.6 mmol) in methanol (25 mL) was added pyrrolidine (1.53 g, 21.6 mmol) dropwise. The mixture was then heated at 70 °C for 3 h. After cooled to 4 °C, the mixture was filtered and resulting precipitate was collected, dried to give E/Z-6-chloro-3-(3-chloro-benzylidene)-1,3-dihydro-indol-2-one as a bright yellow solid (Yield 5.3 g, 85 %).

### Example 145b

### Preparation of intermediate E/ Z-6-chloro-3-(5-fluoro-2-methyl-benzylidene)-2-oxo-2,3-dihydro-indole-1-carboxylic acid ethyl ester

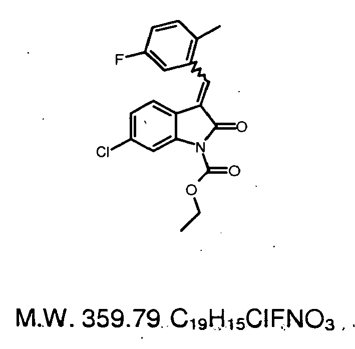

To a solution of E/Z-6-chloro-3-(5-fluoro-2-methyl-benzylidene)-1,3-dihydro-indol-2-one prepared in example 145a (0.20 g, 0.71 mmol) in dichloromethane (3 mL) at 0 °C was added ethyl chloroformate (0.10 mL, 1.1 mmol) , followed by the addition of triethylamine (0.14 g, 1.4 mmol). The reaction mixture was stirred at 0 °C for 30 minutes. The mixture was then poured into aqueous HCl solution (1 N). The organic layer was separated and the aqueous layer was extracted with ethyl acetate. The organic layers were combined and dried over Na₂SO₄, and concentrated to give E/Z-6-chloro-3-(5-fluoro-2-methyl-benzylidene)-2-oxo-2,3-dihydro-indole-1-carboxylic acid ethyl ester as a yellow solid. The crude product was purified by chromatography to obtain 75 mg yellow solid (Yield 1.7 g, 30%).

### Example 145c

### Preparation of intermediate racemic (2'S, 3S, 4'R)-6-chloro-2'-(3-chlorophenyl )-4'-(5-fluoro-2-methyl-phenyl)-2,3-dihydro-2,6'-dioxo spiro[indole-3,3'-piperidine]-1-carboxylic acid ethyl ester

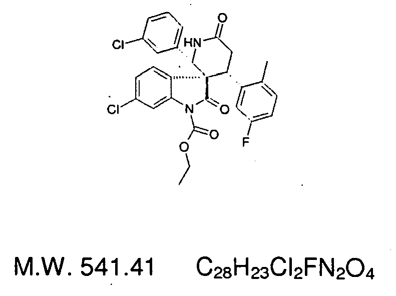

To a toluene (15ml) solution of 1-(3-chlorophenyl)-3-trimethylsilyoxy-2-aza-1,3-butadiene prepared in example 1b in toluene (30ml) was added E/Z-6-chloro-3-(5-fluoro-2-methyl-benzylidene)-2-oxo-2,3-dihydro-indole-1-carboxylic acid ethyl ester (0.16 g, 0.45 mmol) prepared in example 145b. The reaction tube was sealed and heated by microwave irradiation at 135 °C for 35 min. After the solution was cooled to room temperature, methanol (25 mL) was added, and then the mixture was concentrated. The residue was purified by chromatography (EtOAc:CH₂Cl₂.=1:3) to give racemic (2'S, 3S, 4'R)-6-chloro-2'-(3-chlorophenyl)-4'-(5-fluoro-2-methyl-phenyl)-2,3-dihydro-2,6'-dioxo spiro[indole-3,3'-piperidine]-1-carboxylic acid ethyl ester as a yellow oil (Yield 200mg, 82%).

### Example 145d

### Preparation of racemic (2'S, 3S, 4'R)-6-chloro-2'-(3-chlorophenyl)- 4'-(5-fluoro-2-methyl-phenyl)- spiro[3H-indole-3,3'-piperidine]-2,6'(1H)-dione

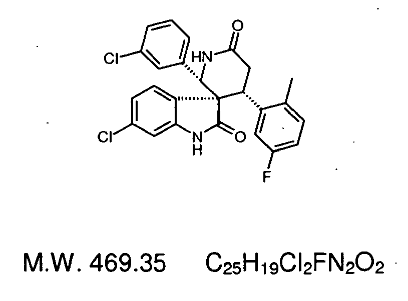

To a solution of racemic (2'S, 3S, 4'R)-6-chloro-2'-(3-chlorophenyl )- 4'-(5-fluoro-2-methyl-phenyl)-2,3-dihydro-2,6'-dioxo-spiro[indole-3,3'-piperidine]-1-carboxylic acid ethyl ester prepared in example 145c (0.20g, 0.31 mmol) in methanol (12 mL) was added NaOH (22 mg, 0.56mmol). The mixture was stirred at room temperature for 0.5 h. The solvent was removed and the residue was partitioned between ethyl acetate and aqueous HCl solution (1 N). The aqueous layer was extracted with ethyl acetate. The organic layers were combined and then concentrated. The residue was purified with Prep-HPLC to give racemic (2'S, 3S, 4'R)-6-chloro-2'-(3-chlorophenyl)-4'-(5-fluoro-2-methyl-phenyl)- spiro[3H-indole-3,3'-piperidine]-2,6'(1H)-dione (9 mg).

MS *m*/*z* (M+H)⁺: 469

### Example 146a

### Preparation of intermediate E/Z-6-chloro-3-(5-fluoro-2-methyl-benzylidene)-1-(2-trimethylsilanyl-ethoxymethyl)-1,3-dihydro-indole-2-one

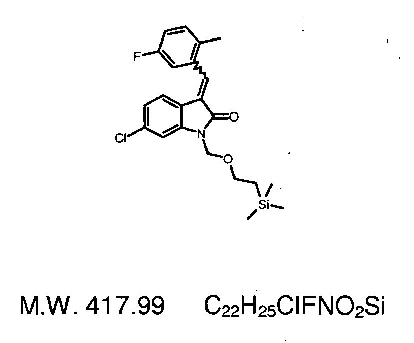

To a solution E/Z-6-chloro-3-(3-chloro-benzylidene)-1,3-dihydro-indol-2-one prepared in example 145a (5.0 g, 17.4 mmol) in N,N-dimethyl-formamide (40 mL) at 0 °C was added NaH (60% in mineral oil) (0.70 g, 17.4 mmol), followed by the dropwise addition of 2-(trimethylsilyl)ethoxymethyl chloride (2.9 g, 17.4 mmol) in tetrahydrofuran (40 mL). The reaction mixture was stirred at 0 °C for 0.5 h, then poured into ice-water. The crude was extracted with ethyl acetate twice. The combined organic layer was dried over Na₂SO₄. The solvent was removed and the residue was purified by chromatography (hexanes) to give E/Z-6-chloro-3-(5-fluoro-2-methyl-benzylidene)-1-(2-trimethylsilanyl-ethoxymethyl)-1,3-dihydro-indole-2-one as yellow oil (Yield 5.7 g, 78%).

### Example 146b

### Preparation of intermediate racemic (2'S, 3S, 4'R)-6-chloro-2'-(3-chlorophenyl )-4'-(5-fluoro-2-methyl-phenyl)-2,3-dihydro-2,6'-dioxo spiro[indole-3,3'-piperidine]-1- methoxyethyl trimethylsilane

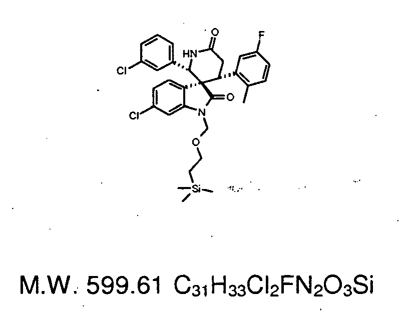

To a toluene(12ml) solution of 1-(3-chlorophenyl)-3-trimethylsilyoxy-2-aza-1,3-butadiene prepared in example 1b in toluene (50 mL) was added E/Z-6-chloro-3-(5-fluoro-2-methyl-benzylidene)-1-(2-tri methylsilanyl-ethoxymethyl)-1,3-di hydro-indole-2-one (0.30 g, 0.72 mmol) prepared in example 146a.The reaction tube was then placed into the cavity of a focused monomode microwave reactor and the contents of the flask were irradiated for 35 min at 135 °C. After the solution was cooled to room temperature, methanol (25 mL) was added. The reaction solutions were concentrated. The residue was purified by chromatography (EtOAc:CH₂Cl₂ =1:3) to give racemic (2'S, 3S, 4'R)-6-chloro-2'-(3-chlorophenyl )-4'-(5-fluoro-2-methyl-phenyl)-2,3-dihydro-2,6'-dioxo spiro [indole-3,3'-piperidine]-1- methoxyethyl trimethylsilane as a yellow solid (0.45 g)

### Example 146c

### Preparation of intermediate racemic (2'S, 3S, 4'R)-6-chloro-2'-(3-chlorophenyl )-4'-(5-fluoro-2-methyl-phenyl)-2,3-dihydro-1'-[(tert-butoxycarbonyl) methyl]-2,6'-dioxo spiro[indole-3,3'-piperidine]-1- methoxyethyl trimethylsilane

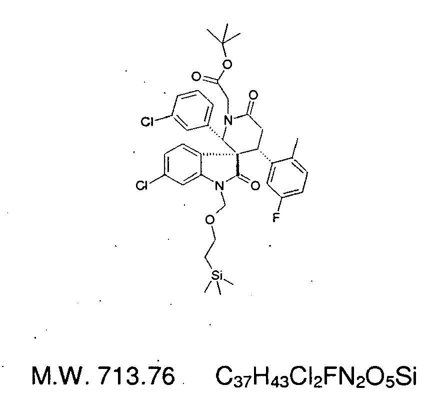

To a solution of racemic (2'S, 3S, 4'R)-6-chloro-2'-(3-chlorophenyl )- 4'-(5-fluoro-2-methyl-phenyl)-2,3-dihydro-2,6'-dioxo spiro[indole-3,3'-piperidine]-1-methoxyethyl trimethylsilane (2.5 g, 4.18 mmol) prepared in example 146b in N,N-dimethyl-formamide (20 mL) at room temperature was added bromo-acetic acid tert-butyl ester (2.0 g, 10.4 mmol) and cesium carbonate (7.5 g, 23.0 mmol). The reaction mixture was stirred under nitrogen for 4 h, then was poured into saturated aqueous NH₄Cl solution. The mixture was extracted with ethyl acetate. The organic layers were combined, dried over Na₂SO₄ and concentrated. The residue was purified by chromatography (EtOAc:Hexanes=1:4) to give racemic (2'S, 3S, 4'R)-6-chloro-2'-(3-chlorophenyl)- 4'-(5-fluoro-2-methyl-phenyl)-2,3-dihydro-1'-[(*tert*-butoxycarbonyl)methyl]-2,6'-dioxo spiro [indole -3,3'-piperidine]-1- methoxyethyl trimethylsilane as a yellow solid (Yield 0.85 g).

### Example 146d

### Preparation of racemic (2'S, 3S, 4'R)-6-chloro-2'-(3-chlorophenyl)- 4'-(5-fluoro-2-methyl-phenyl)-1'-hydroxycarbonylmethyl-spiro[3H-indole-3,3'-piperidine]-2,6'(1H)-dione

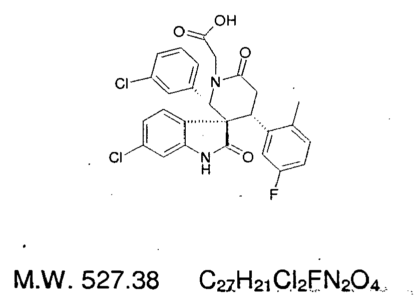

To a solution of racemic (2'S, 3S, 4'R)-6-chloro-2'-(3-chlorophenyl )-4'-(5-fluoro-2-methyl-phenyl)-2,3-dihydro-1'-[(*tert*-butoxycarbonyl) methyl]-2,6'-dioxo spiro[indole-3,3'-piperidine]-1- methoxyethyl trimethylsilane (0.85 g, 1.19 mmol) prepared in example 146d in dichloromethane (10 mL) was added trifluoroacetic acid (20 mL). The reaction mixture was stirred at room temperature for 18 h. The reaction mixture was concentrated. The residue was redissolved in methanol (10 mL). To the resulting solution was added N,N'-diisopropylethylamine (1.57 mL, 8.70 mmol) and the crude was refluxed for 1 h. The reaction mixture was concentrated and the residue was partitioned between ethyl acetate and HCl aqueous solution (1 N). The organic layer was separated, dried over MgSO₄ and concentrated: The residue was triturated with ethyl acetate and hexane to give racemic (2'S, 3S, 4'R)-6-chloro-2'-(3-chlorophenyl)-4'-(5-fluoro-2-methyl-phenyl)-1'-hydroxycarbonylmethyl- spiro[3H-indole-3,3'-piperidine]-2,6'(1H)-dione as a yellow solid (Yield 440 mg, 70%).

MS *m*/*z* (M+H)⁺: 527

### Example 147

### Preparation of racemic (2'S, 3S, 4'R)-6-chloro-2'-(3-chlorophenyl )- 4'-(5-fluoro-2-methyl-phenyl)-1'-(methylamino-carbonyl-methyl) spiro[3H-indole-3,3'-piperidine]-2,6'(1H)-dione

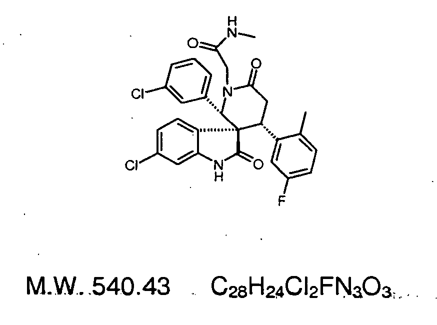

The mixture of racemic (2'S, 3S, 4'R)-6-chloro-2'-(3-chlorophenyl )-4'-(5-fluoro-2-methyl-phenyl)- 1'-hydroxycarbonylmethyl-spiro[3H-indole-3,3'-piperidine]-2,6'(1H)-dione (70 mg, 0.13 mmol) prepared in example 146d, Methylamine hydrochloride (11 mg, 0.16 mmol), EDC.HCl (31 mg, 0.16 mmol), HOSt (22 mg, 0.16 mmol) and DIPEA (69 mg, 0.533 mmol) in DMF (2 mL) was stirred at room temperature for overnight. The crude was then purified with Prep-HPLC to give racemic (2'S, 3S, 4'R)-6-chloro-2'-(3-chlorophenyl )- 4'-(5-fluoro-2-methylphenyl)-1'-(methylamino-carbonyl-methyl) spiro[3H-indole-3,3'-piperidine]-2,6'(1H)-dione (29 mg as a white solid.

MS *m*/*z* [M+H]⁺ : 540

### Example 148

### Preparation of racemic (2'S, 3S, 4'R)-6-chloro-2'-(3-chlorophenyl)- 1'-(dimethylamino-carbonyl-methyl) -4'-(5-fluoro-2-methyl-phenyl)- spiro[3H-indole-3,3'-piperidine]-2,6'(1H)-dione

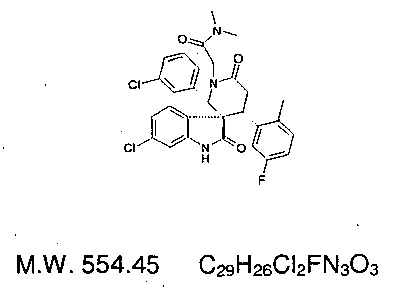

In a similar manner to the method described in example 147, racemic (2'S, 3S, 4'R)-6-chloro-2'-(3-chlorophenyl)-1'-(dimethylamino-carbonyl-methyl)-4'-(5-fluoro-2-methyl-phenyl)-spiro[3H-indole-3,3'-piperidine]-2,6'(1H)-dione was prepared.

MS *m*/*z* (M+H)⁺: 554

### Example 149

### Preparation of racemic (2'S, 3S, 4'R) -1'-[(4-aminocarbonyl-piperidin-1-yl)carbonyl-methyl]-6-chloro-2'-(3-chlorophenyl)- 4'-(5-fluoro-2-methyl-phenyl) spiro[3H-indole-3,3'-piperidine]-2,6'(1H)-dione

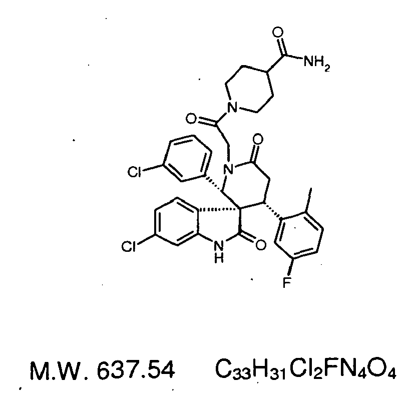

In a similar manner described in example 147, racemic (2'S, 3S, 4'R) -1'-[(4-aminocarbonyl-piperidin-1-yl)carbonyl-methyl]-6-chloro-2'-(3-chlorophenyl )- 4'-(5-fluoro-2-methyl-phenyl) spiro[3H-indole-3,3'-piperidine]-2,6'(1H)-dione was prepared.

MS *m*/*z* (M+H)⁺: 637

### Example 150

### Preparation of racemic (2'S, 3S, 4'R)-1'-[(3-aminocarbonyl-piperidin-1-yl)carbonyl-methyl] -6-chloro-2'-(3-chlorophenyl )- 4'-(5-fluoro-2-methyl-phenyl)-spiro[3H-indole-3,3'-piperidine]-2,6'(1H)-dione

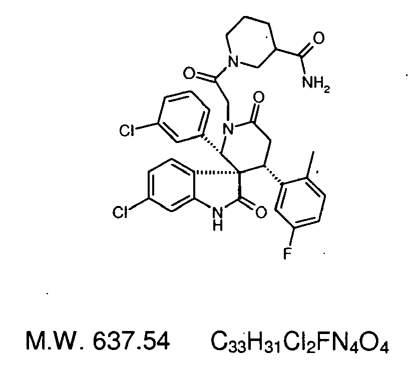

In a similar manner described in example 147, racemic (2'S, 3S, 4'R)-1'-[(3-aminocarbonyl-piperidin-1-yl)carbonyl-methyl] -6-chloro-2'-(3-chlorophenyl)-4'-(5-fluoro-2-methyl-phenyl)-spiro[3H-indole-3,3'-piperidine]-2,6'(1H)-dione was prepared.

MS *m*/*z* (M+H)⁺: 637

### Example 151

### Preparation of racemic (2'S, 3S, 4'R) -1'-(aminocarbonyl-methyl) -6-chloro-2'-(3-chlorophenyl)- 4'-(5-fluoro-2-methyl-phenyl) spiro[3H-indole-3,3'-piperidine]-2,6'(1H)-dione

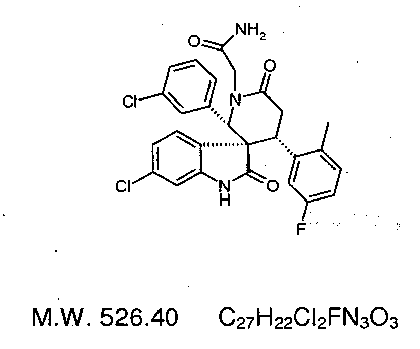

In a manner similar to the method described in example 146d and 146e racemic (2'S, 3S, 4'R)-6-chloro-2'-(3-chlorophenyl)-4'-(5-fluoro-2-methyl-phenyl)-2,3-dihydro-2,6'-dioxo spiro[indole-3,3'-piperidine]-1-methoxyethyl trimethylsilane (50 mg g, 0.08 mmol) was reacted with 2-bromo-acetamide (35 mg, 0.25 mmol) and cesium carbonate (163 mg, 0.50 mmol) in DMF(2 ml).The resulting residue was dissolved in a mixture solution of trifluoroacetic acid (5 mL) and dichloromethane(5 mL). The reaction mixture was stirred at room temperature for 18 h. The reaction mixture was concentrated. The residue was redissolved in methanol (10 mL). To the resulting solution was added N,N'-diisopropylethylamine and the crude was refluxed for 1 h. The reaction mixture was concentrated and the residue was partitioned between ethyl acetate and HCl aqueous solution (1 N). The organic layer was seperarted, dried over MgSO₄ and concentrated. The residue was triturated purified by Prep-HPLC to give racemic (2'S, 3S, 4'R) -1'-(aminocarbonyl-methyl)-6-chloro-2'-(3-chlorophenyl)-4'-(5-fluoro-2-methyl-phenyl) spiro[3H-indole-3,3'-piperidine]-2,6'(1H)-dione as a white solid (8 mg).

MS *m*/*z* (M+H)⁺: 526

### Example 152a

### Preparation of intermediate racemic (2'S, 3S, 4'R)-6-chloro-1'-(3-chloro-propyl)-2'-(3-chlorophenyl )- 4'-(5-fluoro-2-methyl-phenyl)-2,3-dihydro-2,6'-dioxo spiro[indole-3,3'-piperidine]-1- methoxyethyl trimethylsilane

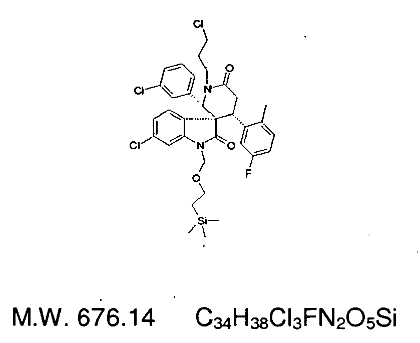

Racemic (2'S,3S,4'R)-6-chloro-2'-(3-chlorophenyl)-4-(5-fluoro-2-methyl-phenyl)-2,3-dihydro-2,6'-dioxo spiro[indole-3,3'-piperidine]-1-methoxyethyl trimethylsilane (0.40 g, 0.67 mmol) prepared in example 146a was reacted with LiH (0.13 g, 16.70 mmol) and 1-chloro-3-bromo-propane (0.63 g, 4.01 mmol) in N,N-dimethyl-formamide (3 mL), a catalytic amount of KI. After the solution was stirred overnight, the solution was poured into water. The water layer was extracted with ethyl acetate and the combined organic layers were dried, concentrated to obtain the crude product. The crude product was purified by chromatography to give racemic (2'S, 3S, 4'R)-6-chloro-1'-(3-chloro-propyl)-2'-(3-chlorophenyl)-4'-(5-fluoro-2-methyl-phenyl)-2,3-dihydro-2,6'-dioxo spiro[indole-3,3'-piperidine]-1-methoxyethyl trimethylsilane as a yellow solid (Yield: 0.11 g, 24%).

### Example 152b

### Preparation of racemic (2'S, 3S, 4'R)-6-chloro-2'-(3-chlorophenyl)-1'-(dimethylamino-propyl)-4'-(5-fluoro-2-methyl-phenyl)- spiro[3H-indole-3,3'-piperidine]-2,6'(1H)-dione

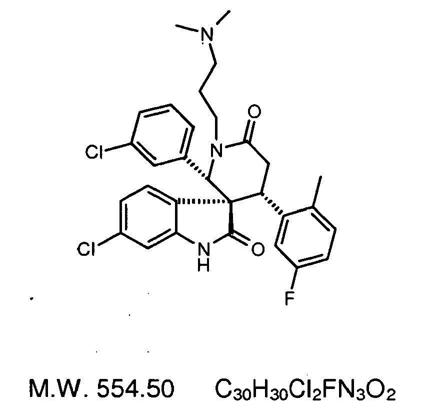

To a solution of racemic (2'S, 3S, 4'R)-6-chloro-1'-(3-chloro-propyl)-2'-(3-chlorophenyl )- 4'-(5-fluoro-2-methyl-phenyl)-2,3-dihydro-2,6'-dioxo spiro[indole-3,3'-piperidine]-1- methoxyethyl trimethylsilane (0.11 g, 0.16 mmol) prepared in example 152a in N,N-dimethyl-formamide (3 mL) at room temperature was added dimethylamine hydrochloride (0.53 g, 6.53 mmol) and cesium carbonate (2.66 g, 8.16 mmol), and a catalytic amount of KI . The reaction mixture was stirred overnight, then the solution was poured into saturated aqueous NH₄Cl solution. The mixture was extracted with ethyl acetate. The organic layers were combined, dried over Na₂SO₄ and concentrated to obtain 110 mg yellow solid. To the yellow solid was added dichloromethane (10 mL) and trifluoracetic acid (10 mL). The reaction mixture was stirred at room temperature for 4 h. The reaction mixture was concentrated and the residue was partitioned between ethyl acetate and saturated NaHCO₃ solution. The organic layer was separated and concentrated. The residue was redissolved in methanol (10 mL). To the resulting solution was added N,N'-diisopropylethylamine (2 mL) and the crude was heated at 100 °C for 1 h. The reaction mixture was concentrated and the residue was purified by Prep-HPLC to give of racemic (2'S, 3S, 4'R)-6-chloro-2'-(3-chlorophenyl)-1'-(dimethylamino-propyl)-4'-(5-fluoro-2-methyl-phenyl)-spiro[3H-indole-3,3'-piperidine]-2,6'(1H)-dione as a white solid (10 mg).

MS *m*/*z* (M+H)⁺: 554

### Example 153a

### Preparation of intermediate racemic (2'S, 3S, 4'R)-1'-[(tert-butoxycarbonylamino) ethyl]-6-chloro-2'-(3-chlorophenyl )- 4'-(5-fluoro-2-methyl-phenyl)-2,3-dihydro-2,6'-dioxo spiro[indole-3,3'-piperidine]-1- methoxyethyl trimethylsilane

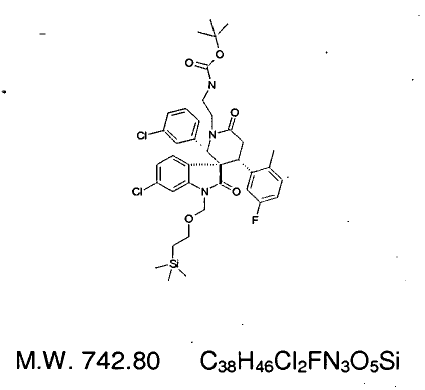

In a manner similar to the method described in example 152a, racemic (2'S, 3S, 4'R)-6-chloro-2'-(3-chlorophenyl)-4'-(5-fluoro-2-methyl-phenyl)-2,3-dihydro-2,6'-dioxo spiro[indole-3,3'-piperidine]-1- methoxyethyl trimethylsilane (0.45 g, 0.75 mmol) was reacted with LiH (0.15 g, 18.8 mmol) and (2-bromo-ethyl)-carbamic acid tert-butyl ester (1.0 g, 4.52 mmol) in N,N-dimethyl-formamide (3 mL), a catalytic amount of KI to give racemic (2'S, 3S, 4'R)-1'-[(*tert-*butoxycarbonylamino) ethyl]-6-chlorb-2'-(3-chlorophenyl )- 4'-(5-fluoro-2-methylphenyl)-2,3-dihydro-2,6'-dioxo spiro[indole-3,3'-piperidine]-1- methoxyethyl trimethylsilane as a yellow solid (80 mg).

### Example 153b

### Preparation of racemic (2'S, 3S, 4'R)-6-chloro-4'-(3-chlorophenyl)-2'-(5-fluoro-2-methylphenyl)-1'-[(1-methanesulfonyl-piperidine-4-yl)carbonylamino-ethyl] spiro[3H-indole-3,3'-piperidine]-2,6'(1H)-dione

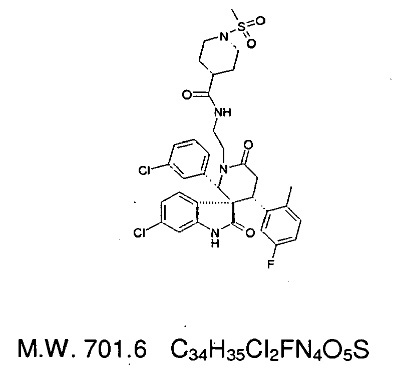

Racemic (2'S, 3S, 4'R)-6-chloro-2'-(3-chlorophenyl )- 4'-(5-fluoro-2-methylphenyl)-2,3-dihydro-1'-[(*tert*-butoxycarbamoy)ethyl]-2,6'-dioxo spiro[indole-3,3'-piperidine]-1- methoxyethyl trimethylsilane prepared in example 153a (80 mg) was added dichloromethane (5 mL) and then trifluoroacetic acid (5 mL). The reaction mixture was stirred at room temperature for 4 h. The reaction mixture was concentrated and the residue was partitioned between ethyl acetate and saturated NaHCO₃ solution. The organic layer was separated and concentrated. The residue was redissolved in methanol (5mL). To the resulting solution was added N,N'-diisopropylethylamine (2 mL) and the crude was heated at 100 °C for 1 h. The reaction mixture was concentrated. The mixture of the residue , 1-methanesulfonyl-piperidine-4-carboxylic acid (60 mg, 0.29 mmol), EDC.HCl (56 mg, 0.29 mmol), HOBt (39 mg, 0.29 mmol) and DIPEA (101 mg, 0.78 mmol) in DMF (2 mL) was stirred at room temperature for overnight. The crude was then purified with Prep-HPLC to give racemic (2'S, 3S, 4'R)-6-chloro-4'-(3-chlorophenyl)-2'-(5-fluoro-2-methylphenyl)-1'-[(1-methanesulfonyl-piperidine-4-yl)carbonylamino-ethyl] spiro[3H-indole-3,3'-piperidine]-2,6'(1H)-dione as a yellow solid (14 mg).

MS *m*/*z* (M+H)⁺: 701

### Example 154a

### Preparation of intermediate E/ Z-6-chloro-3-(4-chloro-thiophen-2-ylmethylene)-1,3-dihydro-indol-2-one

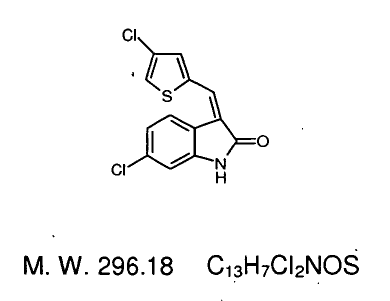

In a manner similar to the method described in example 1a, 6-chlorooxindole (1.2 g, 7.2 mmol) was reacted with 4-chloro-thiophene-2-carbaldehyde (1.05 g, 7.2 mmol) and pyrrolidine (0.7 g, 9.9 mmol) to give E/Z-6-chloro-3-(4-chloro-thiophen-2-ylmethylene)-1,3-dihydro-indol-2-one as a yellow solid (Yield 1.3 g, 62%)

### Example 154b

### Preparation of racemic (2'R, 3R, 4'S)-6-chloro-4'-[2-(4-chloro-thiophenyl)]-2'-(5-fluoro-2-methylphenyl)-spiro[3H-indole-3,3'-piperidine]-2,6'(1H)-dione

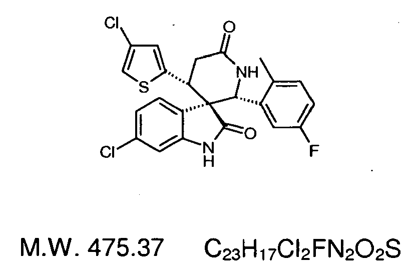

In a manner similar to the method described in example 1 c, E/ Z-6-chloro-3-(4-chloro-thiophen-2-ylmethylene)-1,3-dihydro-indol-2-one (0.2 g, 0.68 mmol) prepared in example 154a was reacted with 1-(5-fluoro-2-methyl-phenyl)-3-trimethylsilyoxy-2-aza-1,3-butadiene prepared in example 36a to give racemic (2'R, 3R, 4'S)-6-chloro-4'-[2-(4-chloro-thiophenyl)]-2'-(5-fluoro-2-methylphenyl)-spiro[3H-indole-3,3'-piperidine]- 2,6'(1H)-dione as an white solid (7 mg).

MS *m*/*z* (M+H)⁺: 475

### Example 155a

### Preparation of intermediate E/ Z-6-chloro-3-(5-chloro-thiophen-2-ylmethylene)-1,3-dihydro-indol-2-one

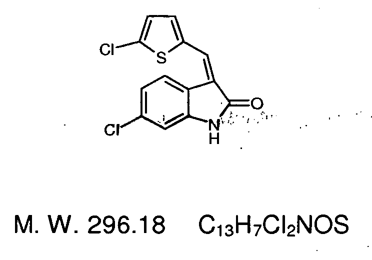

In a manner similar to the method described in example 1a, 6-chlorooxindole (1.2 g, 7.2 mmol) was reacted with 5-chloro-thiophene-2-carbaldehyde (1.05 g, 7.2 mmol) and pyrrolidine (0.7 g, 9.9 mmol) to give E/Z-6-chloro-3-(5-chloro-thiophen-2-ylmethylene)-1,3-dihydro-indol-2-one as a yellow solid (Yield 1.5 g, 71%)

### Example 155b

### Preparation of racemic (2'R, 3R, 4'S)-6-chloro-4'-[2-(5-chloro-thiophenyl)]-2'-(5-fluoro-2-methylphenyl)-spiro[3H-indole-3,3'-piperidine]-2,6'(1H)-dione

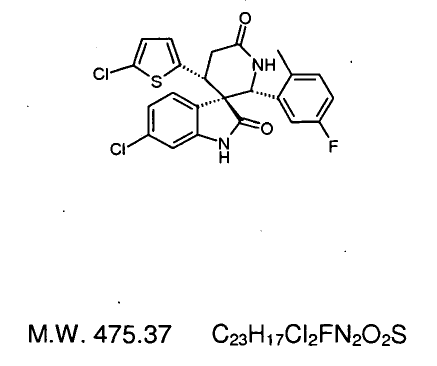

In a manner similar to the method described in example 1c, E/ Z-6-chloro-3-(5-chloro-thiophen-2-ylmethylene)-1,3-dihydro-indol-2-one (0.2 g, 0.68 mmol) prepard in example 155a was reacted with 1-(5-fluoro-2-methyl-phenyl)-3-trimethylsilyoxy-2-aza-1,3-butadiene prepared in example 6b to give racemic (2'R, 3R, 4'S)-6-chloro-4'-[2-(5-chloro-thiophenyl)]-2'-(5-fluoro-2-methylphenyl)-spiro[3H-indole-3,3'-piperidine]- 2,6'(1H)-dione as an white solid (6 mg).

MS *m*/*z* (M+H)⁺: 475

### Example 156

### Preparation of racemic (2'R, 3R, 4'S)-6-chloro-4'-(2,2-dimethylpropyl)-2'-(5-fluoro-2-methylphenyl)-spiro[3H-indole-3,3'-piperidine]- 2,6'(1H)-dione

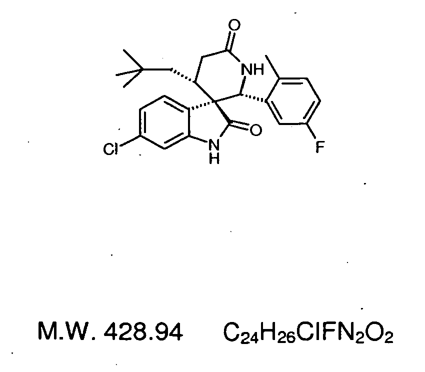

In a manner similar to the method described in example 1c, E/Z- 6-Chloro-3-(3,3-dimethyl-butylidene)-1,3-dihydro-indol-2-one (0.2 g, 0.80 mmol) prepared in example 1a was reacted with 1-(5-fluoro-2-methyl-phenyl)-3-trimethylsilyoxy-2-aza-1,3-butadiene prepared in example 36a to give racemic (2'R, 3R, 4'S)-6-chloro-4'-(2,2-dimethylpropyl)-2'-(5-fluoro-2-methylphenyl)-spiro[3H-indole-3,3'-piperidine]- 2,6'(1H)-dione as an white solid (7 mg).

MS *m*/*z* (M+H)⁺: 429

### Example 157a

### Preparation of intermediate E/Z-6-bromo-3-(3-chloro-benzylidene)-1,3-dihydro-indol-2-one

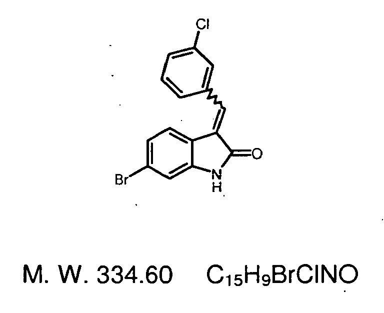

To the mixture of 6-bromooxindole (16.2 g, 92 mmol) (Combi-block) and 3-chloro-benzaldehyde (12.9 g, 92 mmol) in methanol (109 mL) was added pyrrolidine (6.55 g, 92 mmol) dropwise. The mixture was then heated at 65 °C for 3 h. After cooled to 4 °C, the mixture was filtered and resulting precipitate was collected, dried to give a mixture of E/Z-6-chloro-3-(3-chloro-benzylidene)-1,3-dihydro-indol-2-one as a bright yellow solid (Yield 16.2 g, 63 %).

### Example 157b

### Preparation of racemic (2'R, 3R, 4'S)-6-bromo-4'-(3-chlorophenyl)-2'-(2-methylphenyl) spiro[3H-indole-3,3'-piperidine]-2,6'(1H)-dione

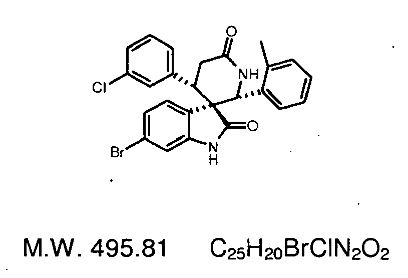

To a solution of 1-(2-methylphenyl)-3-trimethylsilyoxy-2-aza-1,3-butadiene prepared in example 17a in toluene (20 mL) was added E/Z-6-bromo-3-(3-chlorobenzylidene)-2-oxo-2,3-dihydro-indole prepared in example 157a (0.3 g, 0.83 mmol). The reaction mixture was stirred and heated under microwave irradiation in a sealed tube at 135 °C for 0.5 h. After the solution was cooled to room temperature, methanol (50 mL) was added, and then the mixture was concentrated. The residue was purified by chromatography (EtOAc:CH₂Cl₂ =1:3) to give racemic (2'R, 3R, 4'S)-6-bromo-4'-(3-chlorophenyl)-2'-(2-methylphenyl) spiro[3H-indole-3,3'-piperidine]-2,6'(1H)-dione as a white solid. (Yield 0.22 g, 53%).

MS *m*/*z* [(M+H)⁺]: 495

### Example 158a

### Preparation of racemic (2'R, 3R, 4'S)-6-bromo-4'-(3-chlorophenyl)-2'-(5-fluoro-2-methylphenyl) spiro[3H-indole-3,3'-piperidine]-2,6'(1H)-dione

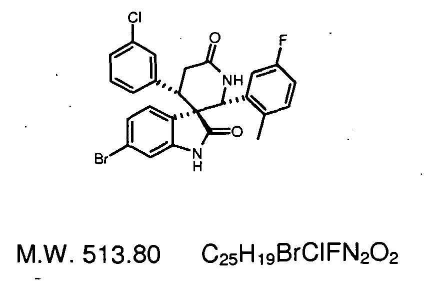

In a manner similar to the method described in example 157b, E/Z-6-bromo-3-(3-chloro-benzylidene)-2-oxo-2,3-dihydro-indole (0.4 g, 1.10 mmol) prepared in example 157a was reacted with 1-(5-fluoro-2-methylphenyl)-3-trimethylsilyoxy-2-aza-1,3-butadiene prepared in example 36a in toluene to give racemic (2'R, 3R, 4'S)-6-bromo-4'-(3-chlorophenyl)-2'-(5-fluoro-2-methylphenyl) spiro[3H-indole-3,3'-piperidine]-2,6'(1H)-dione as a white solid (Yield 0.3 g, 54%).

MS *m*/*z* [(M+H)⁺]: 513.

### Example 158b

### Preparation of chiral (2'R, 3R, 4'S)-6-chloro-4'-(3-chlorophenyl)-2'-(5-fluoro-2-methylphenyl) spiro[3H-indole-3,3'-piperidine]-2,6'(1H)-dione

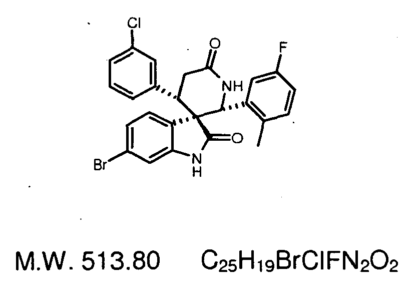

Separation of the two enantiomers from racemic (2'R, 3R, 4'S)-6-bromo-4'-(3-chlorophenyl)-2'-(5-fluoro-2-methylphenyl) spiro[3H-indole-3,3'-piperidine]-2,6'(1H)-dione (35 mg) prepared in example 158a was conducted by chiral column chromatography to provide chiral (2'R, 3R, 4'S)-6-bromo-4'-(3-chlorophenyl)-2'-(5-fluoro-2-methylphenyl) spiro[3H-indole-3,3'-piperidine]-2,6'(1H)-dione as a white solid (Yield: 11 mg, 31 %) and chiral (2'S, 3S, 4'R)-6-bromo-4'-(3-chlorophenyl)-2'-(5-fluoro-2-methylphenyl) spiro[3H-indole-3,3'-piperidine]-2,6'(1H)-dione as a white solid (Yield: 11 mg, 31 %).

MS *m*/*z* [(M+H)⁺]: 513

### Example 159a

### Preparation of intermediate 1-(2,5-dichlorophenyl)-3-trimethylsilyoxy-2-aza-1,3-butadiene

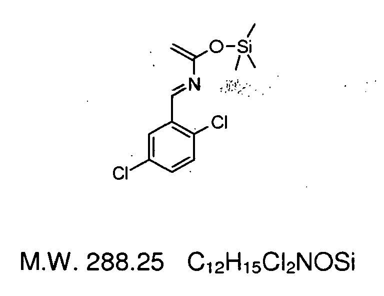

In a manner similar to the method described in example 1b, 2,5-dichloro-benzaldehyde (1.75 g, 10 mmol) was used as the starting material in place of 3-chlorobenzaldehyde to react with Lithium bis(trimethylsilyl)amide (1 M solution in THF, 10mL, 10mmol), trimethylsilyl chloride (1.1 g, 10 mmol), triethylamine (1.4 g, 13 mmol) and acetyl chloride (1.0 g, 13 mmol) to give 1-(2,5-dichlorophenyl)-3-trimethylsilyoxy-2-aza-1,3-butadiene as a yellow gum and used for the next step without further purification.

### Example 159b

### Preparation of racemic (2'R, 3R, 4'S)-6-bromo-4'-(3-chlorophenyl)-2'-(2,5-dichlorophenyl) spiro[3H-indole-3,3'-piperidine]-2,6'(1H)-dione

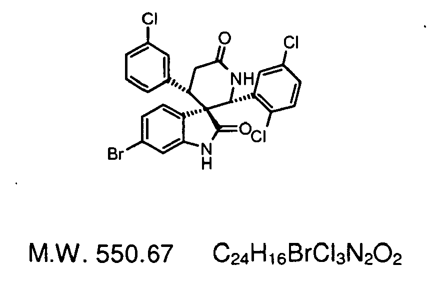

In a manner similar to the method described in example 157b, E/Z-6-bromo-3-(3-chloro-benzylidene)-2-oxo-2,3-dihydro-indole (0.4 g, 1.10 mmol) prepared in example 157a was reacted with 1-(2,5-dichlorophenyl)-3-trimethylsilyoxy-2-aza-1,3-butadiene prepared in example 159a, in toluene to give racemic (2'R, 3R, 4'S)-6-bromo-4'-(3-chlorophenyl)-2,3-dihydro-2'-(2,5-dichlorophenyl)-2,6'-dioxo spiro[indole-3,3'-piperidine as a white solid. (Yield 0.4 g, 67%).

MS *m*/*z* [(M+H)⁺]: 549

### Example 160a

### Preparation of intermediate 1-(5-chloro-2-methylphenyl)-3-trimethylsilyoxy-2-aza-1,3-butadiene

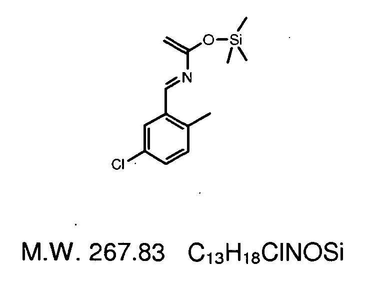

In a manner similar to the method described in example 1b, 5-chloro-2-methy-benzaldehyde (1.54 g, 10 mmol) was used as the starting material in place of 2-methylbenzaldehyde to react with react with Lithium bis(trimethylsilyl) amide (1 M solution in THF, 10mL, 10mmol), trimethylsilyl chloride (1.1 g, 10 mmol), triethylamine (1.4 g, 13 mmol) and acetyl chloride (1.0 g, 13 mmol) to give 1-(5-fluoro-2-methylphenyl)-3-trimethylsilyoxy-2-aza-1,3-butadiene as a yellow gum and used for the next step without further purification.

### Example 160b

### Preparation of racemic (2'R, 3R, 4'S)-6-bromo-4'-(3-chlorophenyl)-2'-(5-chloro-2-methylphenyl) spiro[3H-indole-3,3'-piperidine]-2,6'(1H)-dione

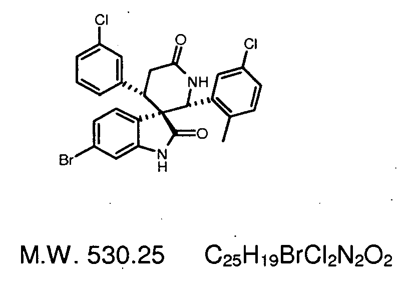

In a manner similar to the method described in example 157b, E/Z-6-bromo-3-(3-chloro-benzylidene)-2-oxo-2,3-dihydro-indole(0.4 g, 1.10 mmol) prepared in example 157a was reacted with 1-(5-chloro-2-methylphenyl)-3-trimethylsilyoxy-2-aza-1,3-butadiene prepared in example 160a, in toluene to give racemic (2'R, 3R, 4'S)-6-bromo-4'-(3-chlorophenyl)-2,3-dihydro-2'-(5-chloro-2-methylphenyl)-2,6'-dioxospiro[indole-3,3'-piperidine] as a white solid. (Yield 0.3 g, 51%).

MS *m*/*z* [(M+H)⁺]: 529

### Example 161a

### Preparation of intermediate E/Z-6-Bromo-3-(3-chloro-benzylidene)-1-(2-trimethylsilanyl-ethoxymethyl)-1,3-dihydro-indole-2-one

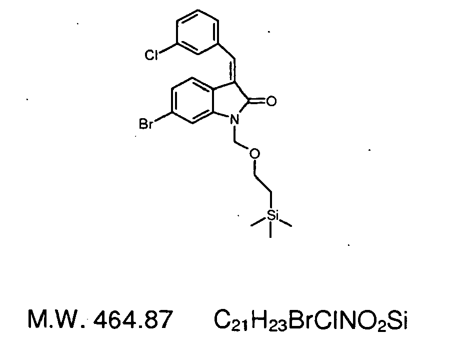

To a solution of E/Z 6-Bromo-3-(3-chloro-benzylidene)-1,3-dihydro-indol-2-one (5.3 g, 16 mmol) prepared in example 157a in N,N-dimethyl-formamide (50mL) at 0 °C was added NaH (60% in mineral oil) (0.64 g,16 mmol) , followed by the dropwise addition of 2-(trimethylsilyl)ethoxymethyl chloride (2.65 g, 16 mmol) in tetrahydrofuran (40 mL). The reaction mixture was stirred at 0 °C for 0.5 h, then poured into ice-water. The crude was extracted with ethyl acetate twice. The combined organic layer was dried over Na₂SO₄. The solvent was removed and the residue was purified by chromatography (hexanes) to give E/Z-6-Bromo-3-(3-chloro-benzylidene)-1-(2-trimethylsilanyl-ethoxymethyl)-1,3-dihydro-indole-2-one as a yellow oil (Yield 4.5 g, 60%).

### Example 161b

### Preparation of intermediate racemic (2'R, 3R, 4'S)-6-bromo-4'-(3-chlorophenyl)-2'-(1-methylene-propyl)-2,3-dihydro-2,6'-dioxo spiro[indole-3,3'-piperidine]-1-methoxyethyl trimethylsilane

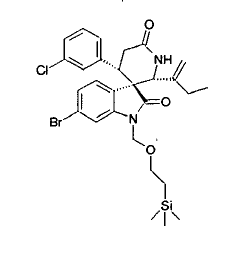

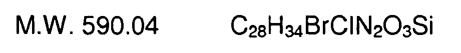

To a solution of 1-(1-ethyl-ethenyl)-3-trimethylsilyoxy-2-aza-1,3-butadiene (42 mmol) prepared in example 80a in toluene (50 mL) was added E/Z-6-Bromo-3-(3-chloro-benzylidene)-1-(2-trimethylsilanyl-ethoxymethyl)-1,3-dihydro-indole-2-one (2 g, 4.3 mmol) prepared in example 161a. The reaction mixture was stirred under nitrogen in a sealed tube at 135 °C for 1 h. After cooling to room temperature, methanol (100mL) was added, and then the mixture was concentrated. The residue was purified by chromatography (EtOAc:Hexane =2:1) gave racemic (2'R, 3R,4'S)-6-bromo-4'-(3-chlorophenyl )-2'-(1-methylene-propyl)-2,3-dihydro-2,6'-dioxo spiro[indole-3,3'-piperidine]-1-methoxyethyl trimethylsilane as a solid (Yield 350 mg).

### Example 161c

### Preparation of racemic (2'R, 3R, 4'S)-6-bromo-4'-(3-chlorophenyl)-2'-(1-methylene-propyl)- spiro[3H-indole-3,3'-piperidine]- 2,6'(1H)-dione

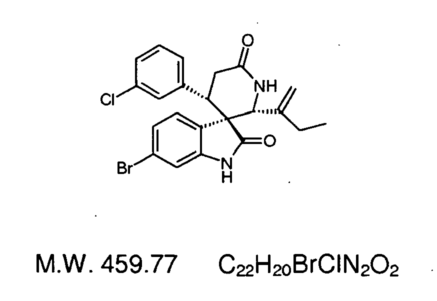

To a solution of racemic (2'R, 3R,4'S)-6-bromo-4'-(3-chlorophenyl)-2'-(1-methylene-propyl)-2,3-dihydro-2,6'-dioxo spiro[indole-3,3'-piperidine]-1-methoxyethyl trimethylsilane (50 mg,0.08 mmol) prepared in example 161b in dichloromethane (5 mL) was added trifluoroacetic acid (5 mL). The reaction mixture was stirred at room temperature for 0.5 h. After removing the solvent, the residue was dissolved in methanol (2 mL). To the resulting solution was added N,N'-diisopropylethylamine (1 mL). The reaction tube was then heated at 135 °C for 20 min. The reaction mixture was concentrated and the residue was purified by Prep-HPLC to obtain racemic (2'R, 3R, 4'S)-6-bromo-4'-(3-chlorophenyl)-2'-(1-methylene-propyl)- spiro[3H-indole-3,3'-piperidine]-2,6'(1H)-dione as solid (30 mg).

### Example 161d

### Preparation of chiral (2'R, 3R, 4'S)-6-bromo-4'-(3-chlorophenyl)-2'-(1-methylene-propyl)- spiro[3H-indole-3,3'-piperidine]- 2,6'(1H)-dione

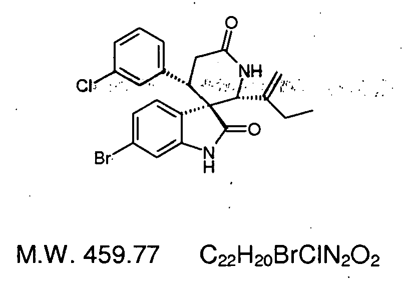

Racemic (2'R, 3R, 4'S)-6-bromo-4'-(3-chlorophenyl )- 2'-(1-methylene-propyl)-spiro[3H-indole-3,3'-piperidine]- 2,6'(1H)-dione (30 mg) was separated by chiral column chromatography to give chiral (2'R, 3R, 4'S)-6-bromo-4'-(3-chlorophenyl)-2'-(1-methylene-propyl)-spiro[3H-indole-3,3'-piperidine]- 2,6'(1H)-dione (7 mg white solid) and chiral (2'S, 3S, 4'R)-6-bromo-4'-(3-chlorophenyl )-2'-(1-methylene-propyl)-spiro[3H-indole-3,3'-piperidine]- 2,6'(1H)-dione as a white solid (7 mg).

MS *m*/*z* [(M+H)⁺]: 459

### Example 162a

### Preparation of intermediate racemic (2'R, 3R, 4'S)-6-bromo-4'-(3-chlorophenyl )-2'-(1-ethyl-cyclopropyl)-2,3-dihydro-2,6'-dioxo spiro[indole-3,3'-piperidine]-1-methoxyethyl trimethylsilane

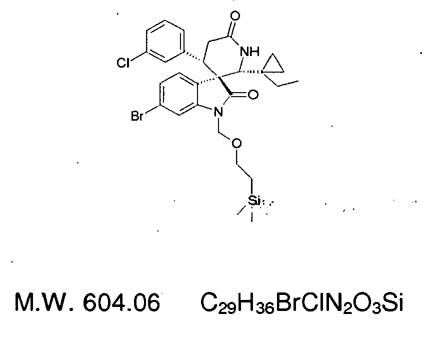

CH₂l₂ (1.2 g, 4.6 mmol) was dissolved in dry toluene (5 mL) at 0 °C. After stirred for 10 min under Argon, Et₂Zn (1 M in THF, 3.3 mL, 3.67 mmol) was added. After stirred for 15 min, a solution of racemic (2'R, 3R, 4'S)-6-bromo-4'-(3-chlorophenyl)-2'-(1-methylene-propyl)-2,3-dihydro-2,6'-dioxo spiro[indole-3,3'-piperidine]-1- methoxyethyl trimethylsilane (270 mg, 0.46 mmol) prepared in example 161b in dry toluene (10 mL) was added. After stirred at room temperature for 3 h, the reaction was quenched with saturated NH₄Cl (20 mL). The aqueous layer was extracted with EtOAc. The combined organic layer was dried over Na₂SO₄, concentrated, the residue, was used for next step without further purification.

### Example 162b

### Preparation of chiral (2R, 3R, 4'S)-6-bromo-4'-(3-chlorophenyl )-2'-(1-ethyl-cyclopropyl) spiro[3H-indole-3,3'-piperidine]-2,6'(1H)-dione

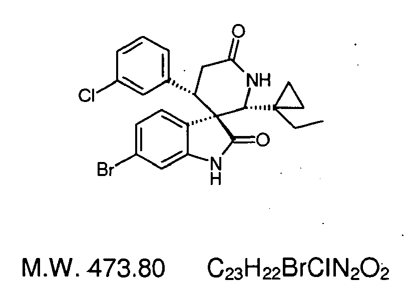

To a solution of racemic (2'R, 3R, 4'S)-6-bromo-4'-(3-chlorophenyl)-2'-(1-ethyl-cyclopropyl)-2,3-dihydro-2,6'-dioxo spiro[indole-3,3'-piperidine]-1-methoxyethyl trimethylsilane (48 mg, 0.079 mmol) prepared in example 162a in dichloromethane (5 mL) was added trifluoroacetic acid (5 mL). The mixture was stirred at room temperature for 5 h. Then the solvent was removed in vacuo. The residue was dissolved in methanol (2 mL). To the resulting solution was added N,N'-diisopropylethylamine (1 mL). The mixture was heated at 135 °C for 20 min. The reaction mixture was concentrated and the residue was purified by Prep-HPLC to obtain racemic (2R, 3R, 4'S)-6-bromo-4'-(3-chlorophenyl)- 2'-(1-ethyl-cyclopropyl)-spiro[3H-indole-3,3'-piperidine]- 2,6'(1H)-dione. The racemic compound was separated by chiral column chromatography to obtain chiral (2R, 3R, 4'S)-6-bromo-4'-(3-chlorophenyl)- 2'-(1-ethyl-cyclopropyl)-spiro[3H-indole-3,3'-piperidine]- 2,6'(1H)-dione (4.5 mg) and chiral (2S, 3S, 4'R)-6-bromo-4'-(3-chlorophenyl)-2'-(1-ethyl-cyclopropyl)-spiro[3H-indole-3,3'-piperidine]-2,6'(1H)-dione (4.5 mg).

MS *m*/*z* [(M+H)⁺]: 473

### Example 163a

### Preparation of intermediate racemic (2'R, 3R, 4'S)-6-bromo-4'-(3-chlorophenyl)-2'-(1-ethyl-cyclopropyl)-2,3-dihydro-2,6'-dioxo spiro[indole-3,3'-piperidine]-1-carboxylic acid tert-butyl ester

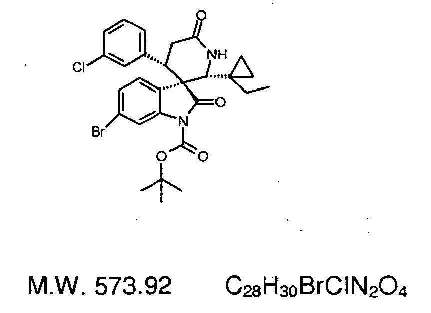

Racemic (2'R, 3R, 4'S)-6-bromo-4'-(3-chlorophenyl)-2'-(1-ethyl-cyclopropyl)-spiro[3H-indole-3,3'-piperidine]-2,6'(1H)-dione (220 mg, 0.47 mmol) prepared in example 162b, (Boc)₂O(121.9 mg, 0.56mmol) and DMAP (73.9 mg, 0.61 mmol) were mixed in tetrahydrofuran (10 mL). After stirred for 0.5 h, the solution was concentrated and the residue was dissolved in EtOAc. The organic layer was washed with 0.5N HCl aqueous solution for several times. Then the organic layer was dried and concentrated to obtain yellow solid (240 mg).

### Example 163b

### Preparation of intermediate racemic (2'R,3R,4'S)-4'-(3-chlorophenyl)-6-cyclopropyl-2'-(1-ethyl-cyclopropyl)-2,3-dihydro-2,6'-dioxo spiro[indole-3,3'-piperidine]-1-carboxylic acid tert-butyl ester

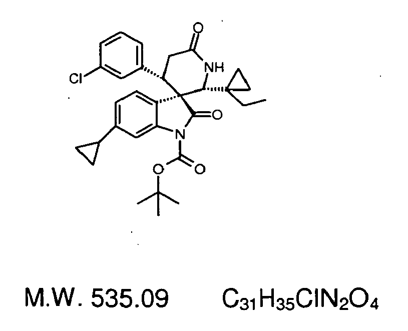

Under Argon atmosphere, racemic (2'R,3R,4'S)-6-bromo-4'-(3-chlorophenyl)-2'-(1-ethyl-cyclopropyl)-2,3-dihydro-2,6'-dioxo spiro[indole-3,3'-piperidine]-1-carboxylic acid *tert*-butyl ester (28 mg, 0.049 mmol), cyclopropylboronic acid (4.5 mg, 0.0524 mmol), Pd(PPh₃)₄ (5 mg), K₃PO₄ (50 mg) and a few drops of water were mixed in toluene (3 mL). The mixture was heated under microwave irradiation at 130 °C for 20 min. Then the solution was poured into water and the aqueous layer was extracted with EtOAc. The organic layer was dried and concentrated. The residue was purified by Prep-TLC to obtain racemic (2'R,3R,4'S)-6-cyclopropyl-4'-(3-chlorophenyl)-2'-(1-ethyl-cyclopropyl)-2,3-dihydro-2,6'-dioxo spiro [indole- 3,3'-piperidine] - 1-carboxylic acid *tert*-butyl ester (10 mg).

### Example 163c

### Preparation of racemic (2'R,3R,4'S)-6-cyclopropyl-4'-(3-chlorophenyl)-2'-(1-ethyl-cyclopropyl)-spiro[3H-indole-3,3'-piperidine]-2,6'(1H)-dione

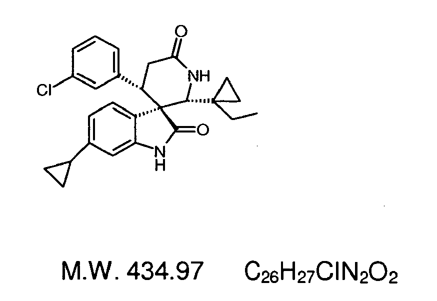

Racemic (2'R,3R,4'S)-6-cyclopropyl-4'-(3-chlorophenyl)-2'-(1-ethyl-cyclopropyl)-2,3-dihydro-2,6'-dioxo spiro[indole-3,3'-piperidine]-1-carboxylic acid *tert*-butyl ester (10 mg, 0.019 mmol) was dissolved in a methanolic solution of HCl (6 M, 10 mL). After 10 min, the solution was concentrated to obtain racemic (2'R, 3R, 4'S)-6-cyclopropyl-4'-(3-chlorophenyl)-2'-(1-ethyl-cyclopropyl)-spiro[3H-indole-3,3'-piperidine]- 2,6'(1H)-dione as a white solid.(Yield, 5.9 mg, 72%)

MS *m*/*z* [(M+H)⁺]: 435

### Example 164a

### Preparation of intermediate racemic (2'R, 3R, 4'S)-6-chloro-4'-(3-chlorophenyl)-2'-(5-fluoro-2-methyl-phenyl)-2,3-dihydro-2,6'-dioxo-spiro[indole-3,3'-piperidine]-1-carboxylic acid tert-butyl ester

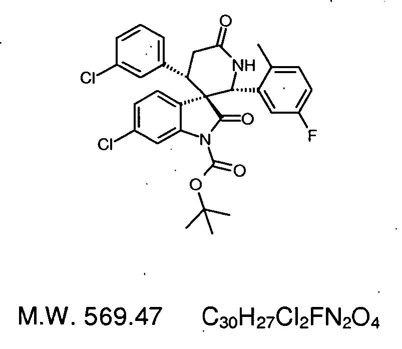

To a mixture of racemic (2'R, 3R, 4'S)-6-chloro-4'-(3-chlorophenyl)-2'-( 5-fluoro-2-methyl-phenyl)-spiro[3H-indole-3,3'-piperidine]-2,6'(1H)-dione (300 mg, 0.64 mmol) prepared in example 36c in dichloromethane (30 mL) was added 4-dimethylaminopyridine (94 mg, 0.77 mmol) and di-tert-butyl-dicarbonate (153 mg, 0.71 mmol). The mixture was stirred at room temperature for 30 min, and then purified by column chromatography to give racemic (2'R, 3R, 4'S)-6-chloro-4'-(3-chlorophenyl)-2'-(5-fluoro-2-methyl-phenyl)-2,3-dihydro-2,6'-dioxo-spiro[indole-3,3'-piperidine]-1-carboxylic acid *tert*-butyl ester as solid (350 mg, 96%).

### Example 164b

### Preparation of intermediate racemic (2'R, 3R, 4'S)-(2-bromomethyl-5-fluorophenyl)-6-chloro-4'-(3-chlorophenyl)-2'-2,3-dihydro-2,6'-dioxo-spiro[indole-3,3'-piperidine]-1-carboxylic acid tert-butyl ester

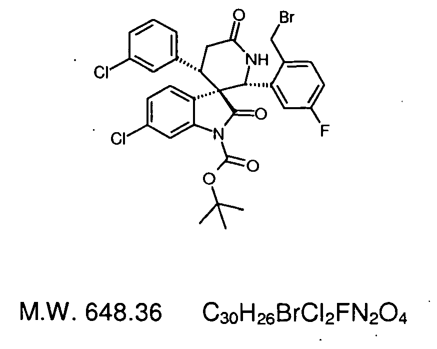

To a solution of racemic (2'R, 3R, 4'S)-6-chloro-4'-(3-chlorophenyl)-2'-(5-fluoro-2-methyl-phenyl)-2,3-dihydro-2,6'-dioxo-spiro[indole-3,3'-piperidine]-1-carboxylic acid *tert*-butyl ester (900 mg, 1.58 mmol) in carbon tetrachloride (20 mL) was added benzoyl peroxide (380 mg, 1.58 mmol) and NBS (280 mg, 1.58 mmol). The mixture was heated under microwave irradiation at 100 °C for 30 min, then purified by column chromatography to give racemic (2'R, 3R, 4'S)-(2-bromomethyl-5-fluoro-phenyl)-6-chloro-4'-(3-chlorophenyl)-2'-2,3-dihydro-2,6'-dioxo-spiro[indole-3,3'-piperidine]-1-carboxylic acid *tert*-butyl ester as solid (170 mg, 16%). The starting material racemic (2'R, 3R, 4'S)-6-chloro-4'-(3-chlorophenyl)-2'-(5-fluoro-2-methyl-phenyl)-2,3-dihydro-2,6'-dioxo-spiro[indole-3,3'-piperidine]-1-carboxylic acid *tert*-butyl ester (550 mg, 0.96 mmol) was recovered.

### Example 164c

### Preparation of racemic (2'R, 3R, 4'S) 2'-[2-(4-aminocarbonyl-piperidin-1-yl)methyl-5-fluoro-phenyl)-6-chloro-4'-(3-chlorophenyl)-spiro[3H-indole-3,3'-piperidine]- 2,6'(1H)-dione

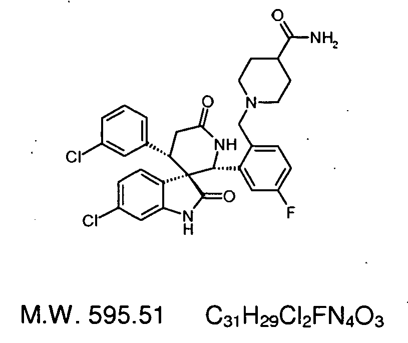

To a solution of racemic (2'R,3R,4'S)-(2-bromomethyl-5-fluoro-phenyl)-6-chloro-4'-(3-chlorophenyl)-2'-2,3-dihydro-2,6'-dioxo-spiro[indole-3,3'-piperidine]-1-carboxylic acid *tert*-butyl ester (30 mg, 0.046 mmol) prepared inex ample 164b in acetonitrile (5 mL) was added K₂CO₃ (13 mg, 0.094 mmol) and piperidine-4-carboxylic acid amide (12 mg, 0.094 mmol). The mixture was refluxed for 2 h, then concentrated. The residue was purified by prep-HPLC to give racemic (2'R, 3R, 4'S) 2'-[2-(4-aminocarbonyl-piperidin-1-yl)methyl-5-fluoro-phenyl)-6-chloro-4'-(3-chlorophenyl)-spiro[3H-indole-3,3'-piperidine]- 2,6'(1H)-dione as solid (2.6 mg,).

MS *m*/*z* [(M+H)⁺]: 595

### Example 165

### Preparation of racemic (2'R, 3R, 4'S)-6-chloro-4'-(3-chlorophenyl )-2'-[5-fluoro-2-(4-methanesulfonyl-piperazin-1-yl)methyl-phenyl]-spiro[3H-indole-3,3'-piperidine]- 2,6'(1H)-dione

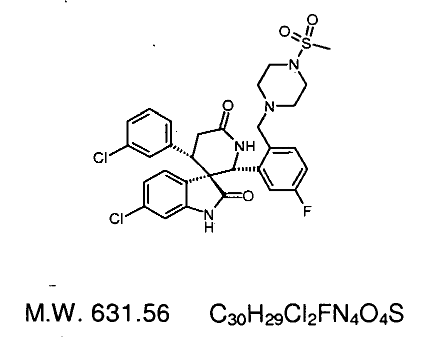

To a solution of racemic (2'R, 3R, 4'S)-2'-(2-bromomethyl-5-fluoro-phenyl)-6-chloro-4'-(3-chlorophenyl)-2,3-dihydro-2,6'-dioxo-spiro[indole-3,3'-piperidine]-1-carboxylic acid *tert*-butyl ester (30 mg, 0.046 mmol) prepared in example 164b in acetonitrile (5 mL) was added K₂CO₃ (13 mg, 0.094 mmol) and 1-methanesulfonyl-piperazine (15 mg, 0.094 mmol). The mixture was refluxed for 2 h, concentrated. The residue was dissolved in trifluoroacetic acid and stirred at room temperature for 30 min, then concentrated and purified by prep-HPLC to give (2'R, 3R, 4'S)-6-chloro-4'-(3-chlorophenyl)-2'-[5-fluoro-2-(4-methanesulfonyl-piperazin-1-yl)methyl-phenyl]-spiro[3H-indole-3,3'-piperidine]-2,6'(1H)-dione as solid (5 mg, 17%).

### Example 166

### Preparation of racemic (2'R, 3R, 4'S)-6-chloro-4'-(3-chlorophenyl )-2'-{5-fluoro-2-[(1-methanesulfonyl-piperidin-4-yl)carbonylamino-methyl]-phenyl}-spiro[3H-indole-3,3'-piperidine]- 2,6'(1H)-dione

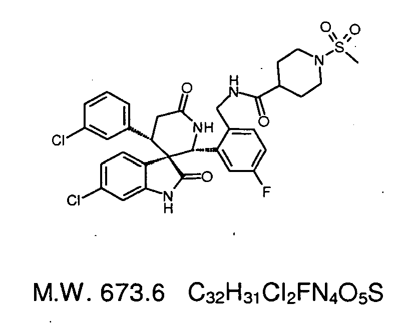

To aqueous ammonia solution (17%, 2 mL, ∼20mmol) was added a solution of of racemic (2'R,3R,4'S)-(2-bromomethyl-5-fluoro-phenyl)-6-chloro-4'-(3-chlorophenyl)-2'-2,3-dihydro-2,6'-dioxo-spiro[indole-3,3'-piperidine]-1-carboxylic acid *tert*-butyl ester (40 mg, 0.06 mmol) prepared in example 164b in N,N-dimethylformamide (2 mL). The mixture was stirred at room temperature for 2 h, followed by addition of EtOAc (20 mL). The organic phase was separated and washed by water, dried over Na₂SO₄. The solvent was removed in vacuo, and the residue was dissolved in dichloromethane. Then 1-methanesulfonyl-piperidine-4-carboxylic acid (20 mg) dimethylaminepiperidine (25 mg) and EDCI (40 mg) were added to the solution. The mixture was stirred at room temperature for 2 h, then diluted with dichloromethane, washed by aqueous HCl solution (1 N, 10 mL), water (10 mL), dried over Na₂SO₄. The organic layer was separated, concentrated and the residue was purified by preparative HPLC to give the titled compound as solid (12 mg).

MS *m*/*z* [(M+H)⁺]: 673

### Example 167a

### Preparation of intermediate 6-fluoro-1,3-dihydro-indol-2-one

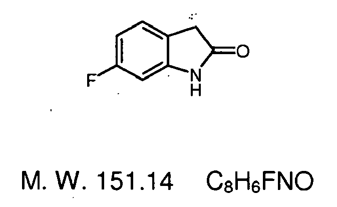

To a mixture of NaH (60%, 7g, 0.16 mol) in dimethyl sulfoxide (150 mL) was added dropwise dimethyl malonate (20 mL, 0.16 mol).The mixture was heated to 100°C for 10 min then cooled to room temperature, followed by the addition of 2,5-difluoronitrobenzene (14 g, 0.08 mol). After stirred at 90°C for 2 h, the mixture was cooled and poured into 5% aq.HCl with ice cooling. EtOAc (50 mL) was added and the organic phase was separated, washed by water and dried with Na₂SO₄. The solvent was removed in vacuo to give 2-(4-fluoro-2-nitrophenyl)-malonic acid dimethyl ester (19.4 g). 2-(4-fluoro-2-nitro-phenyl)-malonic acid dimethyl ester (6 g, 22 mmol) was dissolved in glacial acetic acid (30 mL), the aqueous HCl (6N, 30 mL) was added and the reaction mixture were heated ar refluxing for 4 h, Iron power (5 g, 88 mmol) was added portionwise to the mixture and the refluxing was allowed to continue for another 2 h. The solvent was removed in vacuo and the remaining residue was extracted by EtOAc. The organic phase washed with aqueous HCl (1 N), brine and dried over Na₂SO₄. concentrated to give the titled compound as a yellow solid(3 g, 89%).

MS *m*/*z* (M+H)⁺: 152

### Example 167b

### Preparation of intermediate E/Z-6-fluoro-3-(3-chloro-benzylidene)-1,3-dihydro-indol-2-one

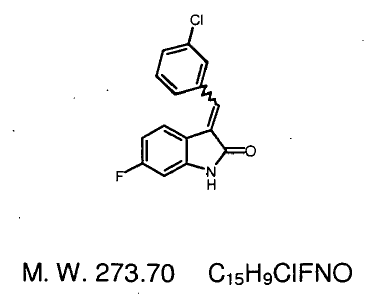

To the mixture of 6-fluorooxindole (13.9 g, 92 mmol) and 3-chloro-benzaldehyde (12.9 g, 92 mmol) in methanol (109 mL) was added pyrrolidine (6.55 g, 92 mmol) dropwise. The mixture was then heated at 65 °C for 3 h. After cooled to 4 °C, the mixture was filtered and resulting precipitate was collected, dried to give a mixture of E/Z-6-fluoro-3-(3-chloro-benzylidene)-1,3-dihydro-indol-2-one as a bright yellow solid (Yield 17.5 g, 64 %).

MS *m*/*z* (M+H)⁺: 274

### Example 167c

### Preparation of chiral (2'R, 3R, 4'S)-4'-(3-chlorophenyl)- 6-fluoro-2'-(5-fluoro-2-methylphenyl) spiro[3H-indole-3,3'-piperidine]-2,6'(1H)-dione

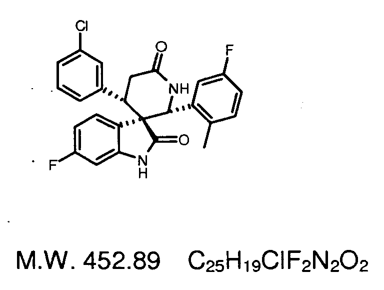

In a manner similar to the method described in example 1c, E/Z-6-fluoro-3-(3-chloro-benzylidene)-2-oxo-2,3-dihydro-indole (0.4 g, 1.10 mmol) prepared in example 167b was reacted with 1-(5-fluoro-2-methylpheny)-3-trimethylsilyoxy-2-aza-1,3-butadiene prepared in example 36a, in toluene to give racemic (2'R, 3R, 4'S)- 4'-(3-chlorophenyl)- 6-fluoro-2'-(5-fluoro-2-methylphenyl) spiro[3H-indole-3,3'-piperidine]-2,6'(1H)-dione as a white solid (Yield 0.1 g). The racemic (2'R, 3R, 4'S)- 4'-(3-chlorophenyl)- 6-fluoro-2'-(5-fluoro-2-methylphenyl) spiro[3H-indole-3,3'-piperidine]-2,6'(1H)-dione was separated by chiral column chromatography to give the titled compounds as a white solid.

MS *m*/*z* (M+H)⁺: 453

### Example 168a

### Preparation of intermediate 6-methoxy-1,3-dihydro-indol-2-one

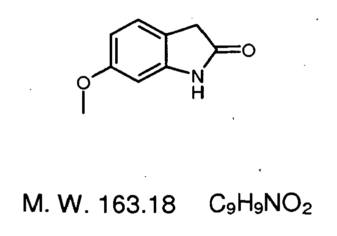

In a manner similar to the method described in example 167a, 1-chloro-4-methoxy-2-nitro-benzene(19 g, 0.1 mol) was reacted with dimethyl malonate (16 g, 0.2 mol), NaH and iron power to give 6-methoxy-1,3-dihydro-indol-2-one (3 g, 18%).

MS *m*/*z* (M+H)⁺: 164

### Example 168b

### Preparation of intermediate E/Z-6-methoxy-3-(3-chloro-benzylidene)-1,3-dihydro-indol-2-one

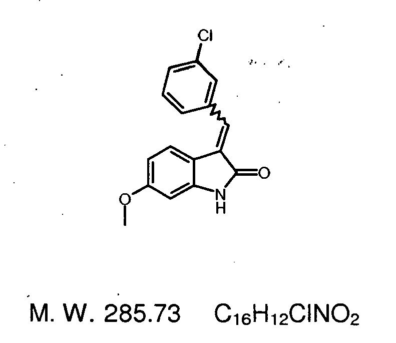

To the mixture of 6-methoxyoxindole (1.60 g, 10 mmol) and 3-chloro-benzaldehyde (1.4 g, 10 mmol) in methanol (10 mL) was added pyrrolidine (0.82 mL, 10 mmol) dropwise. The mixture was then heated at 65 °C for 3 h. After cooled to 4 °C, the mixture was filtered and resulting precipitate was collected, dried to give a mixture of E/Z-6-methoxy-3-(3-chloro-benzylidene)-1,3-dihydro-indol-2-one as a bright yellow solid (Yield 1.7 g, 60 %).

MS *m*/*z* (M+H)⁺: 286

### Example 168c

### Preparation of racemic (2'R, 3R, 4'S)-4'-(3-chlorophenyl)-2'-(5-fluoro-2-methylphenyl)- 6-methoxy spiro[3H-indole-3,3'-piperidine]-2,6'(1H)-dione

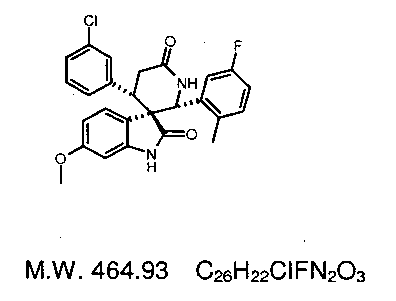

In a manner similar to the method described in example 1 c, E/Z-6-methoxy-3-(3-chloro-benzylidene)-2-oxo-2,3-dihydro-indole (0.4 g, 1.10 mmol) prepared in example 168b was reacted with 1-(5-fluoro-2-methylphenyl)-3-trimethylsilyoxy-2-aza-1,3-butadiene prepared in example 36a, in toluene to give racemic (2'R, 3R, 4'S)-4'-(3-chlorophenyl)-2'-(5-fluoro-2-methylphenyl)-6-methoxy spiro[3H-indole-3,3'-piperidine]-2,6'(1H)-dione as a white solid (Yield 0.05 g, 10%).

MS *m*/*z* (M+H)⁺: 465

### Example 169a

### Preparation of intermediate E/Z-3-(3-chloro-benzylidene)-1,3-dihydro-indol-2-one

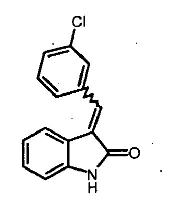

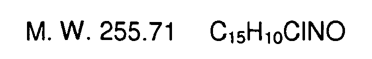

To the mixture of oxindole (2.66 g, 20 mmol) and 3-chloro-benzaldehyde (2.81 g, 20 mmol) in methanol (20 mL) was added pyrrolidine (1.65 mL, 20 mmol) dropwise. The mixture was then heated at 65 °C for 3 h. After cooled to 4 °C, the mixture was filtered and resulting precipitate was collected, dried to give a mixture of E/Z-3-(3-chloro-benzylidene)-1,3-dihydro-indol-2-one as a bright yellow solid (Yield 4 g, 78 %).

MS *m*/*z* (M+H)⁺: 256

### Example 169b

### Preparation of racemic (2'R; 3R, 4'S)-4'-(3-chlorophenyl)-2'-(5-fluoro-2-methylphenyl) spiro[3H-indole-3,3'-piperidine]-2,6'(1H)-dione

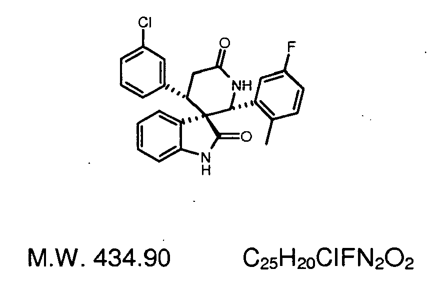

In a manner similar to the method described in example 1 c, E/Z-3-(3-chlorobenzylidene)-2-oxo-2,3-dihydro-indole (0.4 g, 1.10 mmol) prepared in example 169a was reacted with 1-(5-fluoro-2-methylphenyl)-3-trimethylsilyoxy-2-aza-1,3-butadiene prepared in example 36a, in toluene to give racemic (2'R, 3R, 4'S)-4'-(3-chlorophenyl)-2'-(5-fluoro-2-methylphenyl) spiro[3H-indole-3,3'-piperidine]-2,6'(1H)-dione as a white solid (Yield 0.08 g, 15%).

MS *m*/*z* (M+H)⁺: 435

### Example 170a

### Preparation of intermediate 5-fluoro-1,3-dihydro-indol-2-one

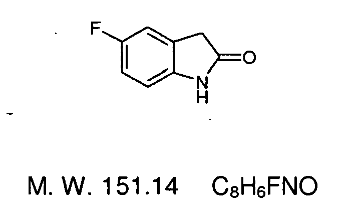

To the solution of 3-fluorophenylacetic acid (1.23 g,8 mmol) in concentrated H₂SO₄ (2.0 mL, 40 mmol) at 0°C was added HNO₃ (0.374 mL, 8 mmol) slowly .The resulting reaction mixture was stirred under argon for 2 h at 0°C, and poured into ice-water. The white solid was precipitated, and the aqueous phase was extracted with ethyl acetate. The organic phase was washed with brine, dried with Na₂SO₄, and concentrated. The crude (5-fluoro-2-nitro-phnyl)-acetic acid was dissolved in acetic acid. To the solution was added iron power (1.79 g, 32 mmol). The reaction mixture was stirred and heated at reflux for 2 h. Then the mixture was concentrated in vacuo .The residue was extracted with ethyl acetate, and The organic layer was separated, washed by aqueous HCl (1 N), brine, dried over Na₂SO₄, concentrated in vacuo to give the desired product (1 g, 82%).

MS *m*/*z* (M+H)⁺: 152

### Example 170b

### Preparation of intermediate E/Z-5-fluoro-3-(3-chloro-benzylidene)-1,3-dihydro-indol-2-one

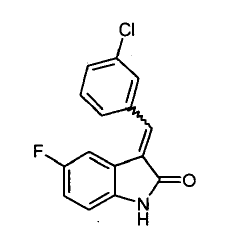

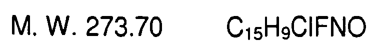

To the mixture of 5-fluorooxindole (3.00 g, 20 mmol) and 3-chloro-benzaldehyde (2.8 g, 20 mmol) in methanol (20 mL) was added pyrrolidine (1.72 mL, 10 mmol) dropwise. The mixture was then heated at 65 °C for 3 h. After cooled to 4 °C, the mixture was filtered and resulting precipitate was collected, dried to give a mixture of E/Z-5-fluoro-3-(3-chloro-benzylidene)-1,3-dihydro-indol-2-one as a bright yellow solid (Yield 1.7 g, 30 %).

MS *m*/*z* (M+H)⁺: 274

### Example 170c

### Preparation of racemic (2'R, 3R, 4'S)-4'-(3-chlorophenyl)- 5-fluoro-2'-(5-fluoro-2-methylphenyl) spiro[3H-indole-3,3'-piperidine]-2,6'(1H)-dione

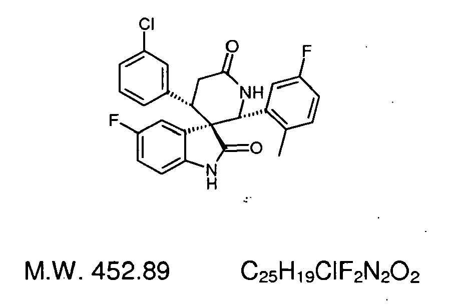

In a manner similar to the method described in example 1 c, E/Z-5-fluoro-3-(3-chloro-benzylidene)-2-oxo-2,3-dihydro-indole (0.4 g, 1.10 mmol) prepared in example 170b was reacted with 1-(5-fluoro-2-methylphenyl)-3-trimethylsilyoxy-2-aza-1,3-butadiene prepared in example 36a, in toluene to give racemic (2'R, 3R, 4'S)- 4'-(3-chlorophenyl)-5-fluoro-2'-(5-fluoro-2-methylphenyl) spiro[3H-indole-3,3'-piperidine]-2,6'(1H)-dione as a white solid (Yield 2 g, 41%).

MS *m*/*z* (M+H)⁺: 453

### Example 171a

### Preparation of intermediate racemic (2'R, 3R, 4'S)-6-chloro-4'-(3-chlorophenyl)-2'-isopropenyl-2,6'-dioxo spiro[indole-3,3'-piperidine]-1-carboxylic acid tert-butyl ester

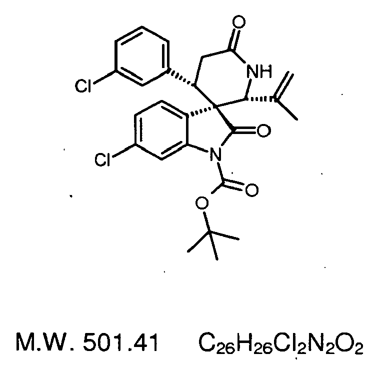

To a solution of racemic (2'R, 3R, 4'S)-6-chloro-4'-(3-chlorophenyl)-2'-(1-methylene-propyl) spiro[3H-indole-3,3'-piperidine]-2,6'(1H)-dione (0.4 g, 1 mmol) prepared in example 87c in dichloromethane (10 mL) at 0 °C was added di-tert-butyl-dicarbonate (0.47 g, 2 mmol) , followed by the addition of 4-dimethylaminopyridine (0.29 g, 2.4 mmol). The reaction mixture was stirred at room temperature for 1 h, then poured into ice-water. The organic layer was separated, washed by aqueous HCl (0.5 N) , dried over Na₂SO₄, concentrated to give racemic (2'R, 3R, 4'S)-6-chloro-4'-(3-chlorophenyl)-2'-isopropenyl-2,6'-dioxo spiro[indole-3,3'-piperidine]-1-carboxylic acid tert-butyl ester as a white solid .(Yield 0.47 g, 95%).

MS *m*/*z* (M+H)⁺: 501

### Example 171b

### Preparation of chiral (2'R, 3R, 4'S)-6-chloro-4'-(3-chloro-phenyl)-2'-(1-methylcyclopropyl) spiro[3H-indole-3,3'-piperidine]-2,6'(1H)-dione

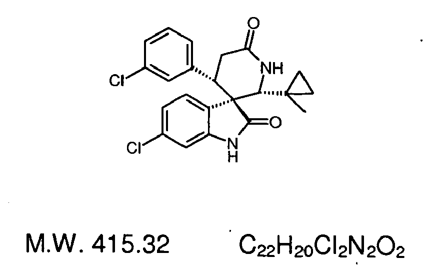

To a solution of CH₂I₂ (1.2 g, 4.6 mmol) in anhydrous toluene (5 mL) under Argon at 0 °C was added a toluene solution of Et₂Zn (1.1 M, 3.3ml, 3.67 mmol). The mixture was stirred for 15 min, then a toluene solution (10 mL) of racemic (2'R, 3R, 4'S)-6-chloro-4'-(3-chlorophenyl)-2'-isopropenyl-2,6'-dioxo spiro[indole-3,3'-piperidine]-1-carboxylic acid tert-butyl ester (230 mg, 0.46 mmol) prepared in example 171a was added. The reaction mixture was stirred at room temperature for 3 h, then quenched with aqueous saturated NH₄Cl (20 mL). The organic layer was separated, and the aqueous layer was extracted with ethyl acetate. The organic layers were combined, dried over Na₂SO₄ and concentrated to give racemic (2'R, 3R, 4'S)-6-chloro-4'-(3-chloro-phenyl)-2'-(1-methyl-cyclopropyl) spiro[3H-indole-3,3'-piperidine]-2,6'(1H)-dione as a white solid (162 mg). Then racemic (2'R, 3R, 4'S)-6-chloro-4'-(3-chloro-phenyl)-2'-(1-methyl-cyclopropyl) spiro[3H-indole-3,3'-piperidine]-2,6'(1H)-dione by chiral SFC to give chiral (2'R, 3R, 4'S)-6-chloro-4'-(3-chloro-phenyl)-2'-(1-methyl-cyclopropyl) spiro[3H-indole-3,3'-piperidine]-2,6'(1H)-dione as a white solid (50 mg). HRMS(ES⁺) *m*/*z* Calcd for C₂₂H₂₀Cl₂N₂O₂+H [(M+H)⁺]: 415.0975 Found: 415.0975.

¹H NMR (400 MHz, DMSO-*d*6): δ = 0.10 (m, 2 H), 0.23 (s, 3 H), 0.36 (m, 1 H), 0.55 (m, 1 H), 2.60 (dd, J = 6.4, 18.4 Hz, 1 H), 2.85 (dd, J = 12.8, 18.4 Hz, 1 H), 3.14 (s, 1 H), 3.66 (dd, J = 6.4Hz, 13.2Hz, 1 H), 6.62 (d, J = 8Hz, 1 H), 6.71 (d,J = 2 Hz, 1 H), 6.74 (d, J =1.6 Hz, 1 H), 7.08-7.14 (m, 2 H), 7.18∼7.20 (m, 1 H), 7.36 (d, J = 8 Hz, 1 H), 8.05 (s, 1 H), 10.56 (s, 1 H) ppm.

### Example 172a

### Preparation of intermediate E/Z-6-Bromo-3-(3-chloro-benzylidene)-2-oxo-2,3-dihydro-indole-1-carboxylic acid tert-butyl ester

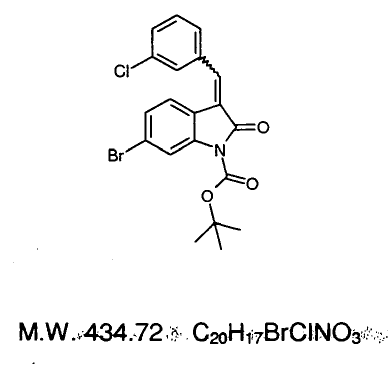

In a manner similar to the method described in example 24a, E/Z-6-bromo-3-(3-chloro-benzylidene)-1,3-dihydro-indol-2-one (2.4 g, 7.2 mmol) prepared in example 157a was reacted with di-tert-butyl-dicarbonate (1.86 g,10 mmol) and 4-dimethylaminopyridine (1.46 g, 12 mmol) to give E/Z-6-bromo-3-(3-chlorobenzylidene)-2-oxo-2,3-dihydro-indole-1-carboxylic acid tert-butyl ester as a yellow solid (Yield 1.5 g, 48%).

MS *m*/*z* (M+H)⁺: 434

### Example 172b

### Preparation of racemic (2'R, 3R, 4'S)-6-bromo-4'-(3-chlorophenyl)-2'-isopropenyl spiro[3H-indole-3,3'-piperidine]-2,6'(1H)-dione

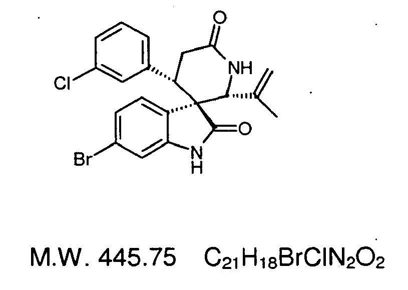

In a manner similar to the method described in example 41b, E/Z-6-bromo-3-(3-chloro-benzylidene)-2-oxo-2,3-dihydro-indole-1-carboxylic acid tert-butyl ester (5 g, 12.8 mmol) prepared in example 172a was reacted with 1-isopropenyl-3-trimethylsilyoxy-2-aza-1,3-butadiene prepared in example 87b in toluene, then treated with trifluoroacetic acid to give racemic (2'R, 3R, 4'S)-6-bromo-4'-(3-chlorophenyl)-2'-isopropenyl spiro[3H-indole-3,3'-piperidine]-2,6'(1H)-dione as a yellow solid (Yield 2.2 g, 40%).

MS *m*/*z* (M+H)⁺: 445

### Example 173a

### Preparation of racemic (2'R, 3R, 4'S)-6-bromo-4'-(3-chlorophenyl)-2'-isopropenyl-2,6'-dioxo spiro[indole-3,3'-piperidine]-1-carboxyl acid tert-butyl ester

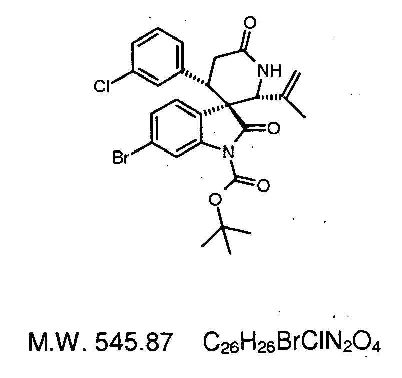

In a manner similar to the method described in example 171a, racemic (2'R, 3R, 4'S)-6-bromo-4'-(3-chlorophenyl)-2'-isopropenyl spiro[3H-indole-3,3'-piperidine]-2,6'(1H)-dione (0.45 g, 1 mmol) prepared in example 172b was reacted with ditert-butyl-dicarbonate (0.24 g, 1 mmol) and 4-dimethylaminopyridine (0.15 g, 1.2 mmol) to give racemic (2'R, 3R, 4'S)-6-bromo-4'-(3-chlorophenyl )-2'-isopropenyl-2,6'-dioxo spiro[indole-3,3'-piperidine]-1-carboxyl acid tert-butyl ester as a white solid (Yield 0.49 g, 90%).

MS *m*/*z* (M+H)⁺: 545

### Example 173b

### Preparation of chiral (2'R, 3R, 4'S)-6-bromo-4'-(3-chloro-phenyl)-2'-(1-methylcyclopropyl) spiro[3H-indole-3,3'-piperidine]-2,6'(1H)-dione

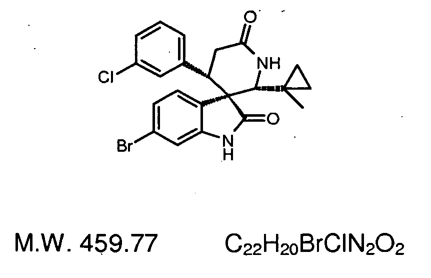

In a manner similar to the method described in example 171b, racemic (2'R, 3R, 4'S)-6-bromo-4'-(3-chlorophenyl )-2'-isopropenyl-2,6'-dioxo spiro[indole-3,3'-piperidine]-1-carboxyl acid tert-butyl ester (250 mg, 0.46 mmol) prepared in example 173a was reacted with CH₂I₂ (1.2 g, 4.6 mmol) and Et₂Zn (3.3 ml, 3.67 mmol) to give racemic (2'R, 3R, 4'S)-6-bromo-4'-(3-chloro-phenyl)-2'-(1-methylcyclopropyl) spiro[3H-indole-3,3'-piperidine]-2,6'(1H)-dione as a white solid (Yield 183 mg, 87%). The racemic (2'R, 3R, 4'S)-6-bromo-4'-(3-chloro-phenyl)-2'-(1-methyl-cyclopropyl) spiro[3H-indole-3,3'-piperidine]-2,6'(1H)-dione was separated by chiral column chromatography to give chiral (2'R, 3R, 4'S)-6-bromo-4'-(3-chloro-phenyl)-2'-(1-methyl-cyclopropyl) spiro[3H-indole-3,3'-piperidine]-2,6'(1H)-dione as white solid (15 mg).

MS *m*/*z* (M+H)⁺: 459 ¹H NMR (400 MHz, DMSO-*d*6): δ = 0.10 (m, 2 H), 0.25 (s, 3 H), 0.34 (m, 1 H), 0.54 (m, 1 H), 2.59 (dd, J = 6.4, 18.4 Hz, 1 H), 2.85 (dd, J = 12.8, 18.4 Hz, 1 H), 3.13 (s, 1 H), 3.62-3.67 (dd, J = 6.4, 12.8Hz, 1 H), 6.62 (d, J = 8 Hz, 1 H), 6.74 (s, 1 H), 6.83 (s, 1 H), 7.10 (t, J = 7.8 Hz, 1 H), 7.19 (d, J = 8.4 Hz, 1 H), 7.25-7.32 (m, 2 H), 8.06 (s, 1 H), 10.56 (s, 1 H) ppm.

### Example 174

### Preparation of racemic (2'R, 3R, 4'S)-4'-(3-chlorophenyl )-6-ethynyl-2'-isopropenyl spiro[3H-indole-3,3'-piperidine]-2,6'(1H)-dione

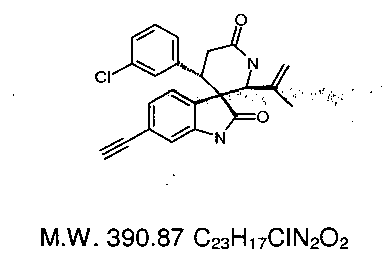

A mixture of racemic (2'R, 3R, 4'S)-6-bromo-4'-(3-chlorophenyl)-2'-isopropenyl-2,6'-dioxo spiro[indole-3,3'-piperidine]-1-carboxylic acid tert-butyl ester-1,3-dihydro-indole-2-one (54 mg, 0.1 mmol) prepared in example 173a, Pd(PPh₃)₄ (20 mg, 0.02 mmol), K₃PO₄ (110 mg, 0.5 mmol), dimethyl trimethylsilylethynylborate (1 mL, 0.5 M, 0.5 mmol) in toluene (3 mL) was heated under microwave irradiation at 130 °C for 1 hour. The solvent was removed in vacuo. To the residue was added methanol (3 mL) and aqueous NaOH (2 N, 3 mL). The mixture was stirred at room temperature for 2 h. The solvent was removed in vacuo, the residue was diluted with ethyl acetate (20 mL), washed with brine, saturated NH₄Cl, brine, then organic layer was separated, and concentrated. The residue was purified by preparative HPLC to give racemic (2'R, 3R, 4'S)-4'-(3-chlorophenyl )-6-ethynyl-2'-isopropenyl spiro[3H-indole-3,3'-piperidine]-2,6'(1H)-dione (2 mg).

Dimethyl trimethylsilylethynylborate solution was prepared according to literature procedure in Lutzen, L. et al Synthesis, 2006, No3, 519-527

### Example 175

### Preparation of chiral (2'R, 3'R, 4'S)-6-chloro-4'-(chlorophenyl)-2'-(5-fluoro-2-methylphenyl)-1'-[(1-methylsulphonyl-4-piperidinyl)aminocarbonyl-methyl] spiro[3H-indole-3,3'-piperidine]-2,6'(1H)-dione

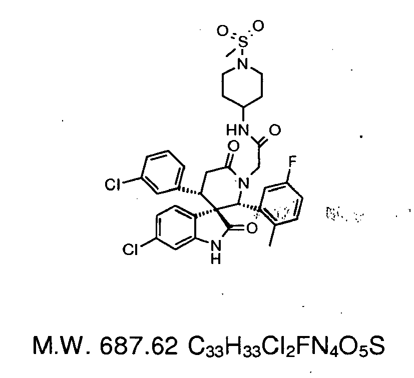

The 1-methanesulfonyl-piperidin-4-ylamine trifluoroacetic acid salt (111 mg, 0.38 mmol) was stirred with N-methyl morpholine (208 uL, 1.9 mmol) and DMAP (3 mg) in DMF (2 ml) for 5 min to obtain a clear solution. A solution of racemic (2'R, 3R, 4'S)-6-chloro-4'-(3-chlorophenyl)-1'-[(fluorocarbonyl)-methyl]-2'-(5-fluoro-2-methylphenyl) spiro[3H-indole-3,3'-piperidine]-2,6'(1H)-dione (0.1 g, 0.18 mmol) prepared in example 57a in DMF (1 mL) was added and the mixture was heated in a microwave oven for 15 min at 100 °C. The mixture was poured into water and was extracted with EtOAc (4x). The organic layer was washed with water, brine, dried and concentrated to give a yellow residue which was purified by a 12 g silica gel column eluted with 0-5% MeOH in methylene chloride. A white powder (74.3 mg, 60% yield) was obtained as desired recemic mixture. This was separated into two optically pure enantiomeric compounds (24 mg each) by chiral SFC using 30 % methanol.

MS *m*/*z* (M+H)⁺: 687

### Example 176a

### Preparation of intermediate 4-[2-(1,1-Dioxo-isothiazolidin-2-yl)-ethyl]-piperazine-1-carboxylic acid tert-butyl ester

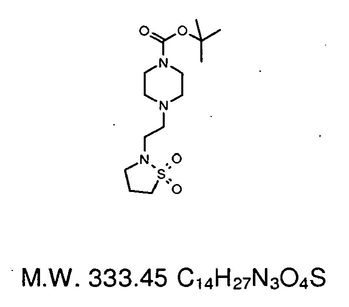

To a stirred solution of 1-Boc-4-(2-aminoethyl)-piperazine (1.26 g, 6.8 mmol) and triethylamine (1 mL) in THF (10 ML), 3-chloro-propylsulfonyl chloride (Aldrich, 0.68 mL, 6.94 mmol) was added slowly at room temperature. The mixture was stirred for 30 minutes at and the reaction was quenched with water. The new mixture was extracted with ethyl acetate and the extracts were combined and dried (Na₂SO₄). The solution was concentrated and the residue was dissolved in THF (20 mL) and Cs₂CO₃ (500 mg), Nal (80 mg) were added and the mixture was stirred at reflux overnight. The mixture was cooled to room temperature and poured into water. The new mixture was extracted with ethyl acetate (3x15 mL) and the extracts were combined and dried (Na₂SO₄). Removal of the solvent on a rotary evaporator gave a solid. 2.01 g.

MS *m*/*z* (M+H)⁺: 334

### Example 176b

### Preparation of intermediate 1-[2-(1,1-dioxo-isothiazolidin-2-yl)-ethyl]-piperazine di-trifluoroacetic acid

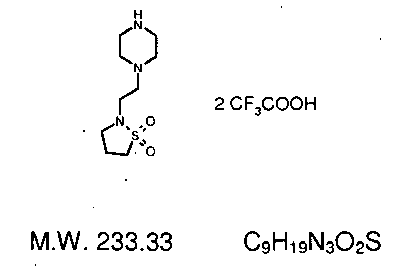

4-[2-(1,1-dioxo-isothiazolidin-2-yl)-ethyl]-piperazine-1-carboxylic acid tert-butyl ester (2.01 g) was treated with 30% TFA/CH₂Cl₂ ( 10 mL) and the mixture was stirred at room temperature for 30 min.. The solvent was removed under reduced pressure to give a solid. 2.46 g.

MS *m*/*z* (M+H)⁺: 234

### Example 176c

### Preparation of chiral (2'R,3'R,4'S)-6-chloro-4'-(3-chlorophenyl)-2'-(5-fluoro-2-methylphenyl)-1'-{[4-(1,1-dioxido-2-isothiazolidinyl)ethyl]piperazinyl-carbonylmethyl} spiro[3H-indole-3,3'-piperidine]-2,6'(1H)-dione

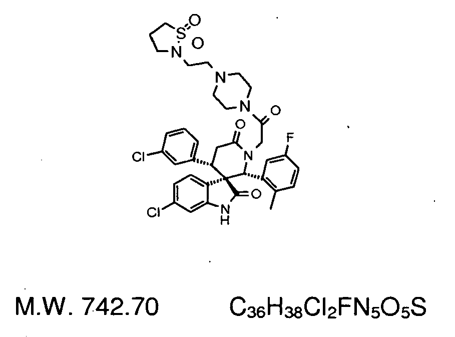

In a manner similar to the method described in example 175, racemic (2'R, 3R, 4'S)-6-chloro-4'-(3-chlorophenyl)-1'-[(fluorocarbonyl)-methyl]-2'-(5-fluoro-2-methylphenyl) spiro[3H-indole-3,3'-piperidine]-2,6'(1H)-dione (0.1 g, 0.18 mmol) prepared in example 57a was reacted with 1-[2-(1,1-dioxo-isothiazolidin-2-yl)-ethyl]-piperazine di-trifluoroacetic acid prepared in example 176b and chiral SFC separation to give chiral (2'R,3'R,4'S)-6-chloro-4'-(3-chlorophenyl)-2'-(5-fluoro-2-methylphenyl)-1'-{[4-(1,1-dioxido-2-isothiazolidinyl)ethyl]piperazinyl-carbonylmethyl} spiro[3H-indole-3,3'-piperidine]-2,6'91H)-dione.

MS *m*/*z* (M+H)⁺: 742

### Example 177

### Preparation of chiral (2'R,3'R,4'S)-6-chloro-4'-(3-chlorophenyl)-2'-(5-fluoro-2-methylphenyl)-1'-{[3-(methylsutphonyl)propyl]piperazinyl-carbonyl-methyl} spiro[3H-indole-3,3'-piperidine]-2,6'(1H)-dione

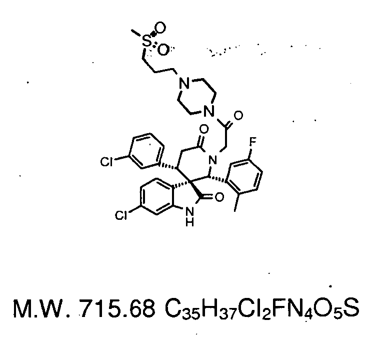

In a manner similar to the method described in example 176, racemic (2'R, 3R, 4'S)-6-chloro-4'-(3-chlorophenyl)-1'-[(fluorocarbonyl)-methyl]-2'-(5-fluoro-2-methylphenyl) spiro[3H-indole-3,3'-piperidine]-2,6'(1H)-dione (0.1 g, 0.18 mmol) prepared in example 57a was reacted with 1-[(3-methylsulfonyl)propyl]piperazine dihydrochloride (US23289) and chiral separation to give chiral (2'R,3'R,4'S)-6-chloro-4'-(3-chlorophenyl)-2'-(5-fluoro-2-methylphenyl)-1'-{[3-(methylsulphonyl)propyl]piperazinyl-carbonyl-methyl} spiro[3H-indole-3,3'-piperidine]-2,6'(1H)-dione.

MS *m*/*z* (M+H)⁺: 715

### Example 178a

### Preparation of intermediate 1-(2-Bromo-2-fluorophenyl)-3-trimethylsilyoxy-2-aza-1,3-butadiene

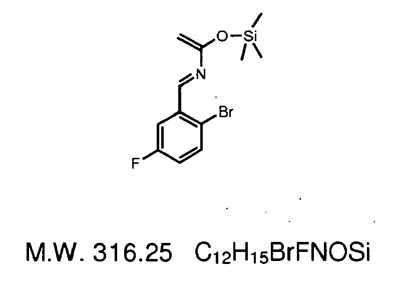

In a manner similar to the method described in example 1b, 2-bromo-2-fluorobenzaldehyde (8.1 g, 40 mmol) (Alfa) was used as the starting material in place of 3-chloro-benzaldehyde to react with 1,1,1,3,3,3-hexamethyldisilazane (6.4 g, 40 mmol), *n*-butyllithium (2.5 M, 16 mL, 40 mmol), trimethylsilyl chloride (4.4 g, 40 mmol), triethylamine (5.6 g, 52 mmol) and acetyl chloride (4.1 g, 52 mmol) to give 1-(2-bromo-2-fluorophenyl)-3-trimethylsilyoxy-2-aza-1,3-butadiene as a yellow gum and used for the next step without further purification.

### Example 178b

### Preparation of racemic (2'R, 3R, 4'S)-2'-(2-bromo-5-fluorophenyl) 6-chloro-4'-(3-chlorophenyl) spiro[3H-indole-3,3'-piperidine]-2,6'(1H)-dione

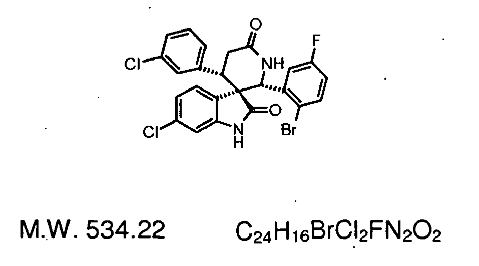

To a stirred solution of E/Z-6-chloro-3-(3-chloro-benzylidene)-1-(2-trimethylsilanyl-ethoxymethyl)-1,3-dihydro-indole-2-one (3.76 g, 8.13 mmol) prepared in example 55a in toluene (40 mL), 1-(2-bromo-2-fluorophenyl)-3-trimethylsilyoxy-2-aza-1,3-butadiene (10.25 g, 32 mmol) prepared in example 178a was added and the mixture was stirred at reflux for 2.5 h. The reaction mixture was cooled to room temperature and methanol (20 mL) was added. The new mixture was stirred for 1 hr at room temperature and then passed through a short silica gel pad. The column was rinsed with 30% (EtOAc/Hexanes). The solvent was removed and the residue was purified by chromatography on an ISCO machine (0-44% EtOAc/Hexanes, 30 min.) to give a pale yellow solid. 4.22 g.

This solid (1.5 g, 2.12 mmol) was treated with TFA/CH₂Cl₂ (30%) and the mixture was stirred at room temperature overnight. The solvent was removed and the residue was dissolved in methanol (20 mL). DIPEA (Aldrich, 2 mL) was then added and the mixture was stirred at reflux for 2 h. The solvent was removed and the residue was purified by chromatography on an ISCO machine (25-48% EtOAc/Hexanes, 30 min.) to give an off-white solid. 520 mg.

MS *m*/*z* (M+H)⁺: 534

### Example 179

### Preparation of racemic (2'R, 3R, 4'S)-2'-6-chloro-4'-(3-chlorophenyl)- (2-ethynyl-5-fluorophenyl) spiro[3H-indole-3,3'-piperidine]-2,6'(1H)-dione

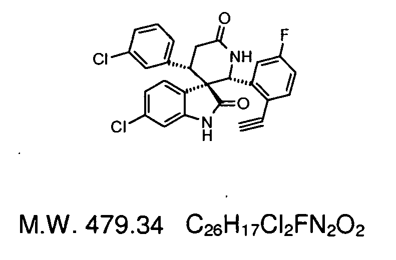

To a stirred solution of racemic (2'R, 3R, 4'S)-2'-(2-bromo-5-fluorophenyl) 6-chloro-4'-(3-chlorophenyl) spiro[3H-indole-3,3'-piperidine]-2,6'(1H)-dione (120 mg, 0.23 mmol) prepared in example 178b in DMF (2 mL), trimetylsilanyl-acetylene (Aldrich, 88 mg, 0.92 mmol), PdCl₂(PPh₃)₂ (Aldrich, 10 mg) and Et₃N (Aldrich, 0.35 mL) were added and the mixture was purged with nitrogen and then heated at 100 °C for 2 h. The solvent was removed under reduced pressure and the residue was dissolved in methanol (5 mL). To the stirred solution, KF (210 mg) was added and the new mixture was stirred at room temperature overnight. The solvent was removed and the residue was purified by chromatography on an ISCO machine (30-40 EtOAc/Hexanes, 30 min.) to give a pale yellow solid. 32 mg.

MS *m*/*z* (M+H)⁺: 479

### Example 180

### Preparation of racemic (2'R, 3R, 4'S)-2'-6-chloro-4'-(3-chlorophenyl)-15-fluoro-2-[3-(methanesulfonyl-methyl-amino)-prop-1-ynyl-phenyl] spiro[3H-indole-3,3'-piperidine]-2,6'(1H)-dione

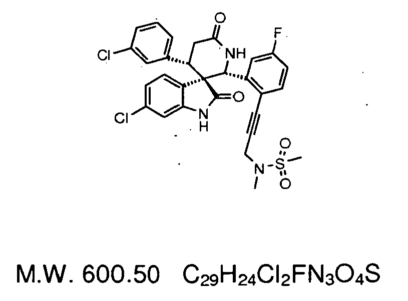

To a stirred solution of racemic (2'R, 3R, 4'S)-2'-(2-bromo-5-fluorophenyl) 6-chloro-4'-(3-chlorophenyl) spiro[3H-indole-3,3'-piperidine]-2,6'(1H)-dione (63 mg, 0.12 mmol) prepared in example 178b in DMF (2 mL), N-methyl-N-prop-2-ynyl-methanesulfonamide (prepared by treatment of the amine chloride with triethylamine, methanesulfonyl chloride, 70 mg, 0.48 mmol), PdCl₂(PPh₃)₂ (20 mg) and Et₃N (0.5 mL) were added and the mixture was purged with nitrogen and then heated at 100 °C for 4 h. The solvent was removed under reduced pressure and the residue was purified by chromatography on an ISCO machine (50% EtOAc/hexanes, 30 min.) to give a white solid. 20 mg.

MS *m*/*z* (M+H)⁺: 479

### Example 181a

### Preparation of intermediate 1-[5-bromo-2-(tert-butyl-dimethyl-silanyloxy)-ethoxy]-phenyl]-3-trimethylsilyoxy-2-aza-1,3-butadiene

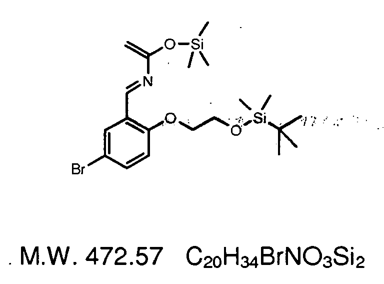

In a manner similar to the method described in example 1b, 5-bromo-2-[2-(tert-butyl-dimethyl-silanyloxy)-ethoxy]-benzaldehyde (17.25 g, 48 mmol) prepared in example 130a was reacted with 1,1,1,3,3,3-hexamethyldisilazane (8.28 g, 48 mmol), *n*-butyllithium (2.5 M, 19.2 mL, 48 mmol), trimethylsilyl chloride (6.07 mL, 48 mmol), triethylamine (8.7 mL, 62.5 mmol) and acetyl chloride (4.53 mL, 62 mmol) to give 1-[2-(tert-butyl-dimethyl-silanyloxy)-ethoxy]-5-chloro-phenyl]-3-trimethylsilyoxy-2-aza-1,3-butadiene as yellow oil and used for the next step without further purification.

### Example 181b

### Preparation of racemic (2'R, 3R, 4'S)-2'-[5-bromo-2-(2-hydroxy-ethoxy)-phenyl]-6-chloro-4'-(3-chlorophenyl) spiro[3H-indole-3,3'-piperidine]-2,6'(1H)-dione

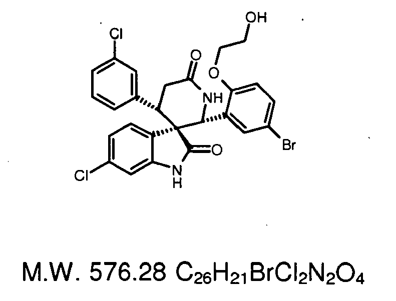

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (2.5 g, 6.4 mmol) was reacted with 1-[5-bromo-2-(tert-butyl-dimethyl-silanyloxy)-ethoxy]-phenyl]-3-trimethylsilyoxy-2-aza-1,3-butadiene prepared in example 181a in toluene and then trifluoroacetic acid in dichloromethane to give racemic (2'R, 3R, 4'S)-2'-[5-bromo-2-(2-hydroxy-ethoxy)-phenyl]-6-chloro-4'-(3-chlorophenyl) spiro[3H-indole-3,3'-piperidine]-2,6'(1H)-dione as a solid (1.52 g, 41%).

MS *m*/*z* (M+H)⁺: 576

### Example 182a

### Preparation of racemic (2'R, 3R, 4'S)-6-chloro-4'-(3-chlorophenyl)-2'-[5-ethynyl-2-(2-hydroxy-ethoxy)-phenyl] spiro[3H-indole-3,3'-piperidine]-2,6'(1H)-dione

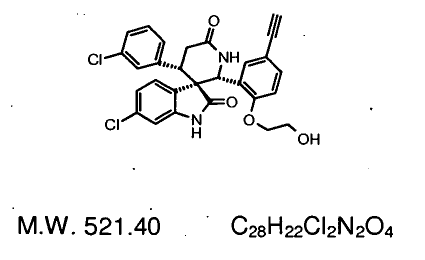

To a solution of racemic (2'R, 3R, 4'S)-2'-[5-bromo-2-(2-hydroxy-ethoxy)-phenyl]-6-chloro-4'-(3-chlorophenyl) spiro[3H-indole-3,3'-piperidine]-2,6'(1H)-dione (60 mg, 0.11 mmol) prepared in example 181b in DMF (2 mL), Et₃N (0.3 mL), PdCl₂(Ph₃P)₂ (Aldrich, 15 mg) were added and the mixture was purged with nitrogen and sealed. The vessel was heated on a microwave reactor for 25 min. and the mixture was poured into water. The mixture was extracted with EtOAc. The extracts were combined and dried with sodium sulfate and dried to give a brown oil, which was purified by chromatography on an ISCO machine (30-100% EtOAc/Hexanes) to give a foam. 45 mg. The foam was then dissolved in methanol (4 mL) and KF (Aldrich, 53 mg) was added. The mixture was stirred at room temperature overnight. The solvent was removed and the residue was partioned between EtOAc/water. The organic layer was dried and concentrated and the residue was purified by chromatography on ISCO machine (30-100% EtOAc/Hexane) to give an off-white solid. 25 mg.

MS *m*/*z* (M+H)⁺: 521

### Example 182b

### Preparation of chiral (2'R, 3R, 4'S)-6-chloro-4'-(3-chlorophenyl)-2'-[5-ethynyl-2-(2-hydroxy-ethoxy)-phenyl] spiro[3H-indole-3,3'-piperidine]-2,6'(1H)-dione

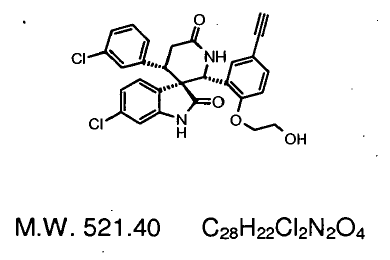

Separation of the two enantiomers from racemic (2'R, 3R, 4'S)-6-chloro-4'-(3-chlorophenyl)-2'-[5-ethynyl-2-(2-hydroxy-ethoxy)-phenyl] spiro[3H-indole-3,3'-piperidine]-2,6'(1H)-dione (65 mg) was conducted by chiral SFC to provide chiral (2'R, 3R, 4'S)-6-chloro-4'-(3-chlorophenyl)-2'-[5-ethynyl-2-(2-hydroxy-ethoxy)-phenyl] spiro[3H-indole-3,3'-piperidine]-2,6'(1H)-dione as a white solid (Yield: 26.3 mg, 40%) and chiral (2'S, 3S, 4'R)-6-chloro-4'-(3-chlorophenyl)-2'-[5-ethynyl-2-(2-hydroxy-ethoxy)-phenyl] spiro[3H-indole-3,3'-piperidine]-2,6'(1H)-dione as a white solid (Yield: 25 mg, 38%).

¹H NMR (300 MHz, DMSO-*d*6): δ = 2.51 (m, 1 H), 2.98(dd, J = 13.9, 15.6 Hz, 1 H), 3.75-3.96 (m, 6 H), 4.65(br s, 1 H), 5.58 (s, 1 H), 6.40 (d, J = 1.8 Hz, 1 H), 6.68 (d, J = 9 Hz, 1 H), 6.78 (d, J = 8.5Hz, 1 H), 6.81 (dd, J = 9.3Hz, 2.4, 2H), 7.04-7.1 (m, 2H), 7.13 (dd, J = 9.5 Hz, 2.1 Hz, 2 H), 7.50 (d, J = 8.2 Hz, 1 H), 8.06(br, 1 H), 10.27(br, s, 1 H) ppm.

### Example 183a

### Preparation of intermediate [2-(4-bromo-3-fluoro-phenoxy)-ethoxy]-tert-butyldimethyl-silane

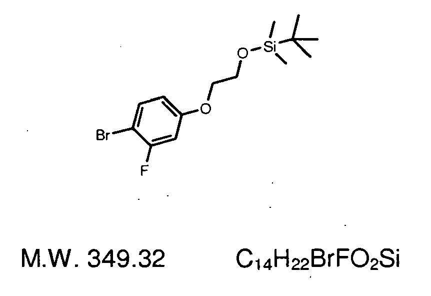

In a manner similar to the method described in example 132a, 4-bromo-3-fluorophenol (12 g, 62.3 mmol) was reacted with K₂CO₃ (26 g, 188 mmo) and (2-bromo-ethoxy)-tert-butyl-dimethyl-silane (18.0 g, 75.3 mmol) to give [2-(4-bromo-3-fluoro-phenoxy)-ethoxy]-tert-butyl-dimethyl-silane as yellow oil (17.2 g, 77% ).

### Example 183b

### Preparation of intermediate 3-bromo-6-[2-(tert-butyl-dimethyl-silanyloxy)-ethoxy]-2-fluoro-benzaldehyde

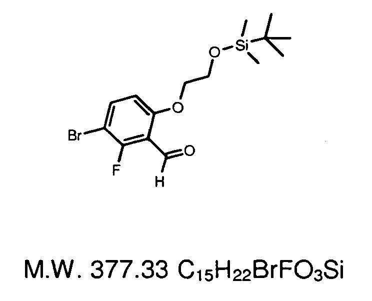

In a.manner similar to the method described in example 52a, 1[2-(4-bromo-3-fluoro-phenoxy)-ethoxy]-tert-butyl-dimethyl-silane (17.2 g, 49.3 mmol) prepared in example 183a was reacted with lithium diisopropyl amide (32.8 mL, 2.0 M in THF, 59.1 mmol), N,N-dimethyl-formamide (4.57 mL, 59.1 mmol) and quenched with acetic acid (12.1 g, 197.2 mmol) and water (61.8 mL) in tetrahydrofuran to give 3-bromo-6-[2-(tert-butyl-dimethyl-silanyloxy)-ethoxy]-2-fluoro-benzaidehyde as white solid (Yield: 12 g, 67%).

### Example 183c

### Preparation of intermediate 1-{3-bromo-6-[2-(tert-butyl-dimethyl-silanyloxy)-ethoxy]-2-fluoro-phenyl}-3-trimethylsilyoxy-2-aza-1,3-butadiene

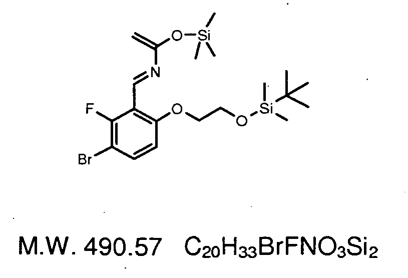

In a manner similar to the method described in example 1b, 3-bromo-6-[2-(tert-butyl-dimethyl-silanyloxy)-ethoxy]-2-fluoro-benzaldehyde (3.96 g, 10.5 mmol) prepared in example 183b was used as the starting material in place of 3-chloro-benzaldehyde to react with 1,1,1,3,3,3-hexamethyldisilazane (2.18 mL, 10.5 mmol), *n*-butyllithium (2.5 M, 4.2 mL, 10.5 mmol), trimethylsilyl chloride (1.33 mL, 10.5 mmol), triethylamine (1.9 mL, 13 mmol) and acetyl chloride (0.97 mL, 13 mmol) to give 1-{3-bromo-6-[2-(tert-butyl-dimethyl-silanyloxy)-ethoxy]-2-fluorophenyl}-3-trimethylsilyoxy-2-aza-1,3-butadiene as yellow gum and used for the next step without further purification.

### Example 183d

### Preparation of racemic (2'R, 3R, 4'S)-2'-[3-bromo-2-fluoro-6-(2-hydroxy-ethoxy)-phenyl]-6-chloro-4'-(3-chlorophenyl) spiro[3H-indole-3,3'-piperidine]-2,6'(1H)-dione

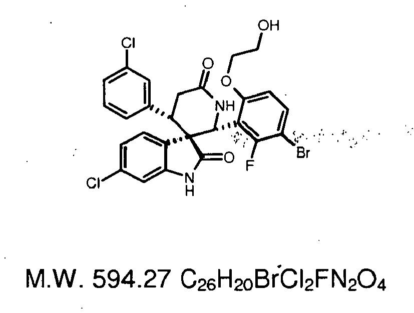

To a solution of 1-{3-bromo-2-fluoro-6-[2-(tert-butyl-dimethyl-silanyloxy)-ethoxy]-phenyl}-3-trimethylsilyoxy-2-aza-1,3-butadiene prepared in example 183c in toluene (30 mL) was added E/Z-6-chloro-3-(3-chlorobenzylidene)-2-oxo-2,3-dihydro-indole-1-carboxylic acid *tert*-butyl ester prepared in example 24a (0.5 g, 1.28 mmol). The reaction mixture was stirred under nitrogen in a sealed tube at 140 °C for 45 min. After the solution was cooled to room temperature, methanol (10 mL) was added. The reaction mixture was filtered through a short pad of celite gel and washed with ethyl acetate. The filtrate was concentrated. The residue was dissolved in dichloromethane (20 mL) and trifluroactic acid (15 mL) was added. After the reaction mixture was stirred at room temperature for 1 h, the mixture was concentrated. The residue was partitioned between saturated NaHCO₃ solution and ethyl acetate. The aqueous layer was extracted with ethyl acetate. The combined organic layer was dried over Na₂SO₄ and concentrated. The residue was dissolved in tetrahydrofuran (10 mL) and tetrabutylammonium fluoride solution (1 M in THF, 10 mL) was added. The reaction mixture was stirred at room temperature for 10 min, then poured into water and extracted with ethyl acetate. The combined organic layer was dried over MgSO₄ and concentrated. The residue was purified by chromatography to give racemic (2'R, 3R, 4'S)-2'-[3-bromo-2-fluoro-6-(2-hydroxy-ethoxy)-phenyl]-6-chloro-4'-(3-chlorophenyl) spiro[3H-indole-3,3'-piperidine]-2,6'(1H)-dione as a yellow solid (Yield 0.4 g, 65.6%).

HRMS(ES⁺) *m*/*z* Calcd for C₂₆H₂₀BrCl₂FN₂O₄ + H [(M+H)⁺]: 593.0041. Found: 593.0039.

### Example 184a

### Preparation of racemic (2'R, 3R, 4'S)-6-chloro-4'-(3-chlorophenyl)-2'-[2-fluoro-6-(2-hydroxy-ethoxy)-3-trimethylsilanylethynyl-phenyl] spiro[3H-indole-3,3'-piperidine]-2,6'(1H)-dione

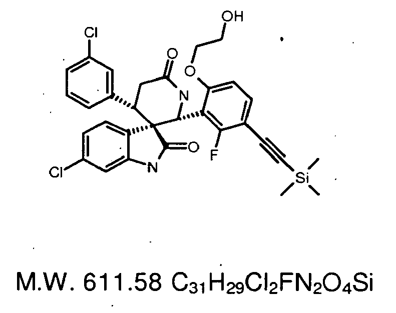

To a solution of racemic (2'R, 3R, 4'S)-2'-[3-bromo-2-fluoro-6-(2-hydroxy-ethoxy)-phenyl]-6-chloro-4'-(3-chlorophenyl) spiro[3H-indole-3,3'-piperidine]-2,6'(1H)-dione (0.12 g, 0.202 mmol) prepared in example 183d in N,N-dimethyl formamide (1 mL) was added ethynyl-trimethyl-silane (0.28 mL, 2.0 mmol) (Aldrich) and triethylamine (0.127 g, 2.0 mmol). After the reaction mixture was degassed for 5 min, dichloro-bis-(triphenyl-phosphine) (14 mg, 0.02 mmol) (Strem) was added and the reaction mixture was heated at 100 °C under nitrogen for overnight. The reaction mixture was cooled to room temperature and diluted with water, extracted with ethyl acetate. The organic layers were combined, washed with water, brine, dried over MgSO₄, filtered and concentrated. The residue was purified with chromatography to give racemic (2'R, 3R, 4'S)-6-chloro-4'-(3-chlorophenyl)-2'-[2-fluoro-6-(2-hydroxy-ethoxy)-3-trimethylsilanylethynyl-phenyl] spiro[3H-indole-3,3'-piperidine]-2,6'(1H)-dione as brown solid (Yield: 50 mg, 40.6 %).

### Example 184b

### Preparation of racemic (2'R, 3R, 4'S)-6-chloro-4'-(3-chlorophenyl)-2'-[3-ethynyl-2-fluoro-6-(2-hydroxy-ethoxy)-phenyl] spiro[3H-indole-3,3'-piperidine]-2,6'(1H)-dione

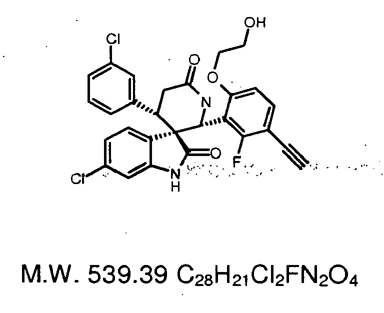

To a solution of racemic (2'R, 3R, 4'S)-6-chloro-4'-(3-chlorophenyl)-2'-[2-fluoro-6-(2-hydroxy-ethoxy)-3-trimethylsilanylethynyi-phenyl].spiro[3H-indole-3,3'-piperidine]-2,6'(1H)-dione (50 mg, 0.08 mmol) prepared in example 184a in methanol (2 mL) was added KF (9.5 mg, 0,16 mmol) (Aldrich). The reaction mixture was stirred at room temperature for overnight. The solvent was removed and the residue was diluted with water and extracted with ethyl acetate. The organic layers were combined, washed with water, brine, dried over MgSO₄, filtered and concentrated. The reidue was purified with chromatography to give racemic (2'R, 3R, 4'S)-6-chloro-4'-(3-chlorophenyl)-2'-[3-ethynyl-2-fluoro-6-(2-hydroxy-ethoxy)-phenyl]spiro[3H-indole-3,3'-piperidine]-2,6'(1H)-dione as brown solid.

HRMS(ES⁺) *m*/*z* Calcd for C₂₈H₂₁Cl₂FN₂O₄ + H [(M+H)⁺]: 539.0935. Found: 539.0935.

¹H NMR (400 MHz, DMSO-*d*6): δ = 2.51 (dd, J = 4.9, 17.6 Hz, 1 H), 3.18 (dd, J = 14.2, 17.6 Hz, 1 H), 3.75-3.96 (m, 5 H), 4.23 (s, 1 H), 4.79 (br s, 1 H), 5.67 (s, 1 H), 6.40 (d, J = 2 Hz, 1 H), 6.67 (d, J = 9 Hz, 1 H), 6.71 (d, J = 7.9 Hz, 1 H), 6.82 (s, 1 H), 6.99 (d, J = 7.9 Hz, 1 H), 7.09 (t, J = 7.8 Hz, 1 H), 7.17 (d, J = 9 Hz, 1 H), 7.26 (t, J = 8.1 Hz, 1 H), 7.66 (m, 1 H), 8.25 (s, 1 H), 10.43 (s, 1 H) ppm.

### Example 185a

### Preparation of intermediate 2-[2-(tert-Butyl-dimethyl-silanyloxy)-ethoxy]-5-ethylbenzaldehyde

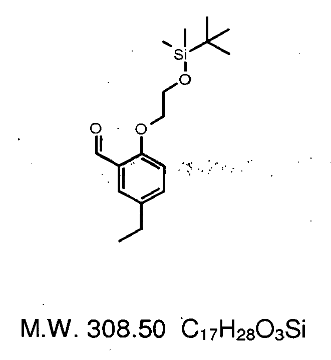

In a manner similar to the method described in example 130a, 5-ethyl-2-hydroxy-benzaldehyde (4.75 g, 31.7 mmol) was reacted with K₂CO₃ (13.1 g, 95.1 mmo) and (2-bromo-ethoxy)-tert-butyl-dimethyl-silane (9.09 g, 38.0 mmol) to give 2-[2-(tert-Butyl-dimethyl-silanyloxy)-ethoxy]-5-ethyl-benzaldehyde as dark brown oil (9.0 g, 92.7%).

### Example 185b

### Preparation of intermediate 1-{2-[2-(tert-butyl-dimethyl-silanyloxy)-ethoxy]-5-ethyl-phenyl}-3-trimethylsilyoxy-2-aza1,3-butadiene

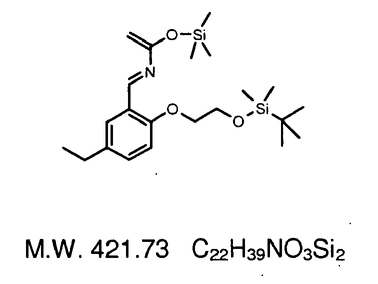

In a manner similar to the method described in example 1b, 2-[2-(tert-Butyldimethyl-silanyloxy)-ethoxy]-5-ethyl-benzaldehyde (3.24 g, 10.5 mmol) prepared in example 185a was used as the starting material in place of 3-chloro-benzaldehyde to react with 1,1,1,3,3,3-hexamethyldisilazane (2.18 mL, 10.5 mmol), *n*-butyllithium (2.5 M, 4 mL, 10.5 mmol), trimethylsilyl chloride (1.1 g, 10 mmol), triethylamine (1.4 g, 13 mmol) and acetyl chloride (1.0 g, 13 mmol) to give 1-{2-[2-(tert-butyl-dimethyl-silanyloxy)-ethoxy]-5-ethyl-phenyl}-3-trimethylsilyoxy-2-azal,3-butadiene as yellow gum and used for the next step without further purification.

### Example 185c

### Preparation of racemic (2'R, 3R, 4'S)-6-chloro-4'-(3-chlorophenyl)-2'-[5-ethyl-2-(2-hydroxy-ethoxy)-phenyl] spiro[3H-indole-3,3'-piperidine]-2,6'(1H)-dione

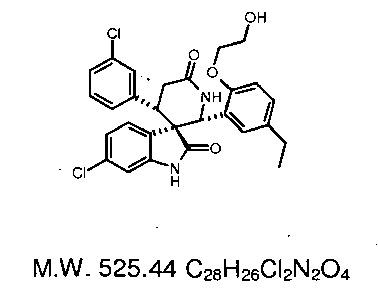

In a manner similar to the method described in example 41b, E/Z-6-chloro-3-(3-chloro-benzylidene)-2-oxo-2,3-dihydro-indole-1-carboxylic acid *tert*-butyl ester prepared in example 24a (0.5 g, 1.28 mmol) was reacted with 1-{2-[2-(tert-butyldimethyl-silanyloxy)-ethoxy]-5-ethyl-phenyl}-3-trimethylsilyoxy-2-aza1,3-butadiene prepared in example 185b in toluene and then trifluoroacetic acid (20mL) in dichloromethane to give racemic (2'R, 3R, 4'S)-6-chloro-4'-(3-chlorophenyl)-2'-[5-ethyl-2-(2-hydroxy-ethoxy)-phenyl] spiro[3H-indole-3,3'-piperidine]-2,6'(1H)-dione as a white solid (0.20 g, 29.8% ).

HRMS(ES⁺) *m*/*z* Calcd for C₂₈H₂₆Cl₂N₂O₄+ H [(M+H)⁺]: 525.1343 Found: 525.1343.

### Example 186

### In Vitro Activity Assay

The ability, of the compounds to inhibit the interaction between p53 and MDM2 proteins was measured by an HTRF (homogeneous time-resolved fluorescence) assay in which recombinant GST-tagged MDM2 binds to a peptide that resembles the MDM2-interacting region of p53 (Lane et al.). Binding of GST-MDM2 protein and p53-peptide (biotinylated on its N-terminal end) is registered by the FRET (fluorescence resonance energy transfer) between Europium (Eu)-labeled anti-GST antibody and streptavidin-conjugated Allophycocyanin (APC).

Test is performed in black flat-bottom 384-well plates (Costar) in a total volume of 40 uL containing:90 nM biotinylate peptide, 160 ng/ml GST-MDM2, 20 nM streptavidin-APC (PerkinElmerWallac), 2 nM Eu-labeled anti-GST-antibody (PerkinElmerWallac), 0.2% bovine serum albumin (BSA), 1 mM dithiothreitol (DTT) and 20 mM Tris-borate saline (TBS) buffer as follows: Add 10 uL of GST-MDM2 (640 ng/ml working solution) in reaction buffer to each well. Add 10 uL diluted compounds (1:5 dilution in reaction buffer) to each well, mix by shaking. Add 20 uL biotinylated p53 peptide (180 nM working solution) in reaction buffer to each well and mix on shaker. Incubate at 37 °C for 1 h. Add 20 uL streptavidin-APC and Eu-anti-GST antibody mixture (6 nM Eu-anti-GST and 60 nM streptavidin-APC working solution) in TBS buffer with 0.2% BSA, shake at room temperature for 30 minutes and read using a TRF-capable plate reader at 665 and 615 nm (Victor 5, Perkin ElmerWallac). If not specified, the reagents were purchased from Sigma Chemical Co.

IC₅₀'s showing the biological activity of this invention exhibit activities less than about 10µM.

Representative values are, for example:

| Example | IC₅₀ (µM, 0.02%BSA) |
|---|---|
| 5d | 2.4315 |
| 14b | 0.4403 |
| 20 | 1.8111 |
| 26b | 0.4899 |
| 31 | 0.3721 |

## Claims

1. A compound of the formula 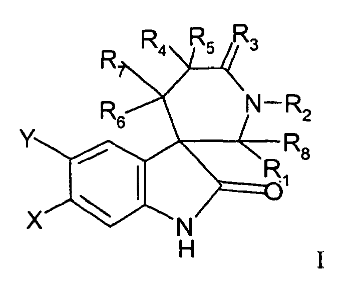 wherein
X is selected from the group consisting of hydrogen, halogen, cyano, nitro, ethynyl, cyclopropyl, methyl, ethyl, isopropyl, methoxy and vinyl;
Y is hydrogen or fluorine;
R₄ and R₅ are hydrogen or lower alkyl having from 1 to 8 carbon atoms;
one of R₁ and R₈ is selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl, and substituted cycloalkenyl and the other is hydrogen;
one of R₆ and R₇ is selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl, and substituted cycloalkenyl and the other is hydrogen, cyano or lower alkyl having from 1 to 8 carbon atoms;
R₂ is selected from the group consisting of hydrogen, lower alkyl having from 1 to 8 carbon atoms and substituted lower alkyl having from 1 to 8 carbon atoms;
R₃ is selected from the group consisting of oxygen, sulfur and NNH(C=O)OR₉;
R₉ is lower alkyl having from 1 to 8 carbon atoms or substituted lower alkyl having from 1 to 8 carbon atoms;
and the pharmaceutically acceptable salts and esters thereof.

2. A compound of claim 1 of the formula 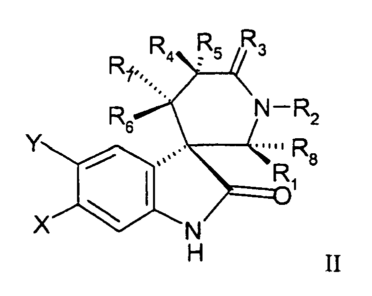 wherein
X is hydrogen, halogen, cyano, nitro, ethynyl, cyclopropyl, methyl, ethyl, isopropyl, methoxy, and vinyl;
Y is hydrogen or fluorine;
R₁ is hydrogen;
R₂ is hydrogen, lower alkyl having from 1 to 8 carbon atoms or substituted lower alkyl having from 1 to 8 carbon atoms;
R₄ and R₅ are hydrogen or lower alkyl having from 1 to 8 carbon atoms;
R₆ is hydrogen, cyano, or lower alkyl having from 1 to 8 carbon atoms;
R₃ is O, S or NNH(C=O)OR₉;
R₇/R₈ is independently selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl and substituted cycloalkenyl;
R₉ is lower alkyl having from 1 to 8 carbon atoms or substituted lower alkyl having from 1 to 8 carbon atoms;
and the pharmaceutically acceptable salts and esters thereof.

3. The compound of claim 2 wherein
X is chlorine or bromine;
Y is hydrogen;
R₁ is hydrogen;
R₄ and R₅ are both hydrogen;
R₆ is hydrogen;
R₃ is O;
R₇ is a substituted phenyl or substituted heteroaryl with the substituted phenyl or substituted heteroaryl selected from the group consisting of 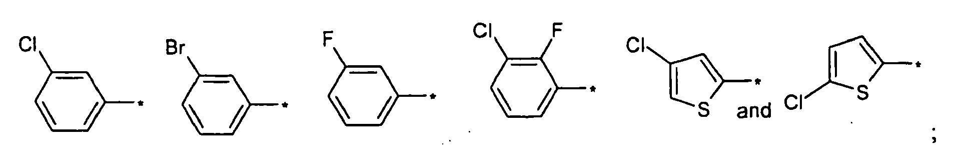 R₈ is independently selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, cycloalkyl, substituted cycloalkyl, cycloalkenyl and substituted cycloalkenyl; and
R₂ is hydrogen, lower alkyl having from 1 to 8 carbon atoms or substituted lower alkyl having from 1 to 8 carbon atoms;
with the proviso that when R₂ is lower alkyl having from 1 to 8 carbon atoms or substituted lower alkyl having from 1 to 8 carbon atoms, R₈ is selected from lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, cycloalkyl, substituted cycloalkyl, cycloalkenyl or substituted cycloalkenyl.

4. The compound of claim 3 wherein
X is -Cl or -Br;
Y is hydrogen;
R₁ is hydrogen;
R₄ and R₅ are both hydrogen;
R₆ is hydrogen;
R₃ is O;
R₇ is a substituted phenyl or substituted heteroaryl with the substituted phenyl or substituted heteroaryl selected from the group consisting of 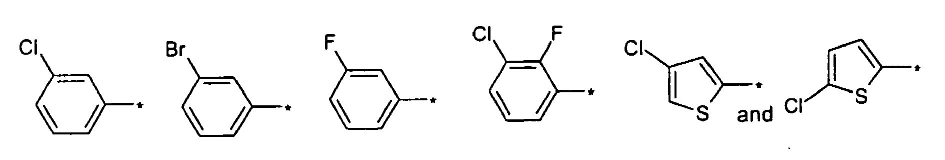 and
R₈ is selected from the group consisting of 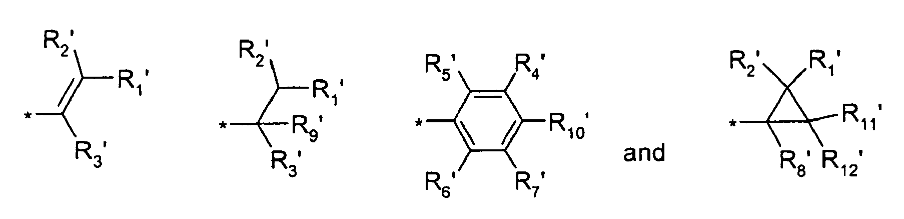 wherein
R₁' is hydrogen, methyl, ethyl, propyl, isopropyl, -CF₃, -F, -CHF₂, or -CH₂F;
R₂' is hydrogen, methyl, ethyl, propyl, isopropyl, -CF₃, -F, -CHF₂, or -CH₂F;
R₃' is hydrogen, -F, -CF₃, -CH₂F, methyl, ethyl, propyl , isopropyl, cyclopropyl, tert-butyl or sec-butyl;
R₄' is hydrogen, -F, -Cl, -Br, -I, methyl, ethyl, cyclopropyl, cyano, methoxy, or ethynyl;
R₅' is hydrogen, -F or methyl;
R₆' is selected from the group consisting of hydrogen, halogen, hydroxy, cyano, lower alkyl having from 1 to 8 carbon atoms, substituted lower alkyl having from 1 to 8 carbon atoms, lower alkoxy having from 1 to 8 carbon atoms, substituted lower alkoxy having from 1 to 8 carbon atoms, lower alkynyl having from 2 to 6 carbon atoms, substituted lower alkynyl having from 2 to 6 carbon atoms;
R₇' is hydrogen, -F or methyl;
R₈' is hydrogen, -F, methyl, ethyl, propyl, isopropyl, tert-butyl or sec-butyl;
R₉' is hydrogen, hydroxyl, or -F;
R₁₀' is hydrogen or -F;
R₁₁' is hydrogen or methyl;
R₁₂' is hydrogen or methyl;
R₂ is hydrogen, lower alkyl having from 1 to 8 carbon atoms or substituted lower alkyl having from 1 to 8 carbon atoms;
with the proviso that when R₂ is lower alkyl having from 1 to 8 carbon atoms or substituted lower alkyl having from 1 to 8 carbon atoms, R₈ is
selected from the group consisting of

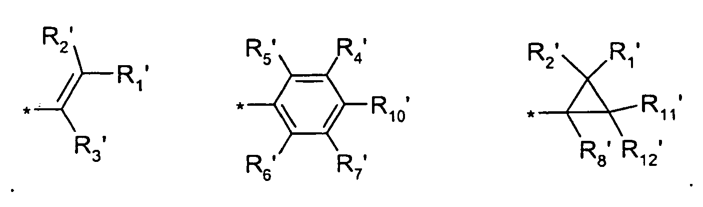

5. A compound of claim 1 of the formula 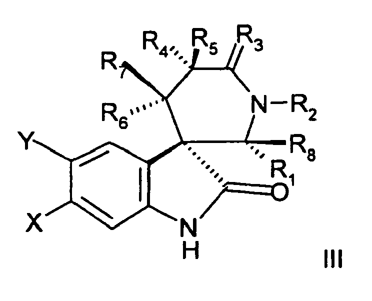 wherein
X is selected from the group consisting of hydrogen, halogen, cyano, nitro, ethynyl, cyclopropyl, methyl, ethyl, isopropyl, methoxy, and vinyl;
Y is hydrogen or fluorine;
R₁ is hydrogen;
R₂ is hydrogen, lower alkyl having from 1 to 8 carbon atoms or substituted lower alkyl having from 1 to 8 carbon atoms;
R₄ and R₅ are hydrogen or lower alkyl having from 1 to 8 carbon atoms;
R₆ is hydrogen, cyano or lower alkyl having from 1 to 8 carbon atoms;
R₃ is O, S or NNH(C=O)OR₉;
R₇/R₈ is independently selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl or substituted cycloalkenyl; and
R₉ is lower alkyl having from 1 to 8 carbon atoms or substituted lower alkyl having from 1 to 8 carbon atoms;
and the pharmaceutically acceptable salts and esters thereof.

6. The compound of claim 5 wherein
X is -Cl or -Br;
Y is hydrogen;
R₁ is hydrogen;
R₂ is hydrogen, lower alkyl having from 1 to 8 carbon atoms or substituted lower alkyl having from 1 to 8 carbon atoms;
R₄ and R₅ are both hydrogen;
R₆ is hydrogen;
R₃ is O;
R₈ is a substituted phenyl with the substituted phenyl selected from the group consisting of 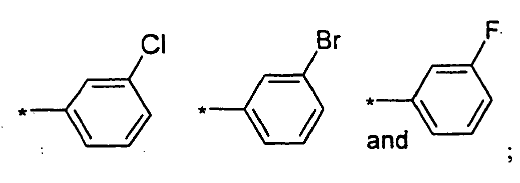 and
R₇ is selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl or substituted cycloalkenyl.

7. A compound of formula I, II or III according to claims 1, 2 or 5 wherein
X is -Cl;
Y; R₁; R₂; R₄; R₅ and R₆ are hydrogen;
R₃ is oxygen; and
R₇ and R₈ are phenyl groups, which are each independently unsubstituted or 1, 2 or 3 times substituted by
lower alkyl having from 1 to 8 carbon atoms, lower alkenyl having from 2 to 8 carbon atoms, lower alkynyl having from 2 to 6 carbon atoms, dioxo-lower-alkylene having from 1 to 8 carbon atoms (forming e.g. a benzodioxyl group), halogen, hydroxy, -CN, -CF3, -NH₂, -N(H, lower alkyl having from 1 to 8 carbon atoms), N(lower alkyl having from 1 to 8 carbon atoms)₂, aminocarbonyl, carboxy, -NO₂, lower-alkoxy having from 1 to 8 carbon atoms, thio-lower-alkoxy having from 1 to 8 carbon atoms, lower-alkylsulfonyl having from 1 to 8 carbon atoms, aminosulfonyl, lower-alkyl(having from 1 to 8 carbon atoms)carbonyl, lower-alkyl(having from 1 to 8 carbon atoms)carbonyloxy, lower-alkoxy(having from 1 to 8 carbon atoms)carbonyl, lower-alkyl(having from 1 to 8 carbon atoms)-carbonyl-NH, fluoro-lower-alkyl having from 1 to 8 carbon atoms, fluoro-lower-alkoxy having from 1 to 8 carbon atoms, lower-alkoxy(having from 1 to 8 carbon atoms)-carbonyl-lower-alkoxy (having from 1 to 8 carbon atoms), carboxy-lower-alkoxy (having from 1 to 8 carbon atoms), carbamoyl-lower-alkoxy (having from 1 to 8 carbon atoms), hydroxy-lower-alkoxy (having from 1 to 8 carbon atoms), -NH₂-lower-alkoxy having from 1 to 8 carbon atoms, -N(H, lower-alkyl having from 1 to 8 carbon atoms)-lower-alkoxy having from 1 to 8 carbon atoms, -N(lower-alkyl having from 1 to 8 carbon atoms)₂-lower-alkoxy having from 1 to 8 carbon atoms, benzyloxy-lower-alkoxy (having from 1 to 8 carbon atoms), mono- or di-(lower-alkyl having from 1 to 8 carbon atoms) substituted amino-sulfonyl and lower-alkyl having from 1 to 8 carbon atoms which can optionally be substituted with halogen, hydroxy, -NH₂, -N(H, lower-alkyl having from 1 to 8 carbon atoms) or -N(lower-alkyl having from 1 to 8 carbon atoms)₂.

8. A compound of formula I, II or III according to claims 1, 2 or 5 wherein
X is -Cl;
Y; R₁; R₂; R₄; R₅ and R₆ are hydrogen;
R₃ is oxygen; and
R₇ and R₈ are phenyl groups, which are each independently unsubstituted or 1, 2 or 3 times substituted by a substituent independently selected from
halogen, C₂₋₄-alkynyl or -O-(C₁₋₄ alkyl), and
said C₂₋₄-alkynyl or -O-(C₁₋₄ alkyl) groups are unsubstituted or once substituted by hydroxyl or -N(C₁₋₄ alkyl)-S(O)₂-CH₃.

9. A compound of claim 1 selected from the group consisting of
(2'S, 3S, 4'S)-6-chloro-2'-(3-chlorophenyl)-4'-(2,2-dimethylpropyl) spiro[3H-indole-3,3'-piperidine]-2,6'(1H)-dione,
(2'SR, 3S, 4'R)-4'-(*tert*-butyl)-6-chloro-2'-(3-chlorophenyl) spiro[3H-indole-3,3'-piperidine]-2,6'(1H)-dione,
(2'S, 3S, 4'R)-6-chloro-2'-(3-chlorophenyl)-4'-isopropyl spiro[3H-indole-3,3'-piperidine]-2,6'(1H)-dione,
racemic (2'R, 3R, 4'S)-6-chloro-2',4'-bis(3-chlorophenyl) spiro[3H-indole-3,3'-piperidine]-2,6'(1H)-dione,
chiral (2'R, 3R, 4'S)-6-chloro-2',4'-bis(3-chlorophenyl) spiro[3H-indole-3,3'-piperidine]-2,6'(1H)-dione,
racemic (2'S, 3S, 4'R)-6-chloro-2'-(3-chlorophenyl)-4'-cyclopentyl spiro[3H-indole-3,3'-piperidine]-2,6'(1H)-dione,
racemic (2'S, 3S, 4'R)-6-chloro-2'-(3-chlorophenyl)-4'-cyclohexyl spiro[3H-indole-3,3'-piperidine]-2,6'(1H)-dione,
racemic (2'S, 3S, 4'R)-6-chloro-2'-(4-chlorophenyl)-4'-cyclohexyl spiro[3H-indole-3,3'-piperidine]-2,6'(1H)-dione,
racemic (2'R, 3R, 4'S)-6-chloro-2'-(4-chlorophenyl)-4'-(3-chlorophenyl) spiro[3H-indole-3,3'-piperidine]-2,6'(1H)-dione and
racemic (2'S, 3S, 4'R)-6-chloro-2'-(3-chlorophenyl)-4'-(4-chlorophenyl) spiro[3H-indole-3,3'-piperidine]-2,6'(1H)-dione.

10. A compound of claim 1 selected from the group consisting of
racemic (2'R, 3R, 4'S)-6-chloro-4'-(3-chlorophenyl)-2'-(3-methylphenyl) spiro[3H-indole-3,3'-piperidine]-2,6'(1H)-dione,
racemic (2'R, 3R, 4'S)-6-chloro-4'-(3-chlorophenyl)-2'-(3-fluorophenyl) spiro[3H-indole-3,3'-piperidine]-2,6'(1H)-dione,
racemic (2'S, 3S, 4'R)-6-chloro-2'-(3-chlorophenyl)-4'-phenyl spiro[3H-indole-3,3'-piperidine]-2,6'(1H)-dione,
racemic (2'S, 3S, 4'R, 5'R)-6-chloro-2'-(3-chlorophenyl)-5'-methyl-4'-phenyl spiro[3H-indole-3,3'-piperidine]-2,6'(1H)-dione,
racemic (2'R, 3R, 4'S)-6-chloro-4'-(3-chlorophenyl)-2'-phenyl spiro[3H-indole-3,3'-piperidine]-2,6'(1H)-dione,
chiral (2'R, 3R, 4'S)-6-chloro-4'-(3-chlorophenyl)-2'-phenyl spiro[3H-indole-3,3'-piperidine]-2,6'(1H)-dione,
racemic (2'R, 3R, 4'S)-6-chloro-4'-(3-chlorophenyl)-2'-(3-methoxyphenyl) spiro[3H-indole-3,3'-piperidine]-2,6'(1H)-dione,
racemic (2'R, 3R, 4'S)-6-chloro-2'-(2-chlorophenyl)-4'-(3-chlorophenyl) spiro[3H-indole-3,3'-piperidine]-2,6'(1H)-dione,
chiral (2'R, 3R, 4'S)-6-chloro-2'-(2-chlorophenyl)-4'-(3-chlorophenyl) spiro[3H-indole-3,3'-piperidine]-2,6'(1H)-dione and
racemic (2'R, 3R, 4'S)-6-chloro-4'-(3-chlorophenyl)-2'-(2-methylphenyl) spiro[3H-indole-3,3'-piperidine]-2,6'(1H)-dione.

11. A compound of claim 1 selected from the group consisting of
chiral (2'R, 3R, 4'S)-6-chloro-4'-(3-chlorophenyl)-2'-(2-methylphenyl) spiro[3H-indole-3,3'-piperidine]-2,6'(1H)-dione,
racemic (2'R, 3R, 4'S)-6-chloro-4'-(3-chlorophenyl)-2'-(2-ethylphenyl) spiro[3H-indole-3,3'-piperidine]-2,6'(1H)-dione,
chiral (2'R, 3R, 4'S)-6-chloro-4'-(3-chlorophenyl)-2'-(2-ethylphenyl) spiro[3H-indole-3,3'-piperidine]-2,6'(1H)-dione,
racemic (2'R, 3R, 4'S, 5'S)-6-chloro-4'-(3-chlorophenyl)-5'-methyl-2'-(2-methylphenyl) spiro[3H-indole-3,3'-piperidine]-2,6'(1H)-dione;
racemic (2'R, 3R, 4'S, 5'S)-6-chloro-2',4'-bis(3-chlorophenyl)-5'-methyl spiro[3H-indole-3,3'-piperidine]-2,6'(1H)-dione,
racemic (2'R, 3R, 4'S)-6-chloro-4'-(3-chlorophenyl)-2'-(2-isopropylphenyl) spiro[3H-indole-3,3'-piperidine]-2,6'(1H)-dione,
chiral (2'R, 3R, 4'S)-6-chloro-4'-(3-chlorophenyl)-2'-(2-isopropylphenyl) spiro[3H-indole-3,3'-piperidine]-2,6'(1H)-dione,
racemic (2'R, 3R, 4'S)-2'-(2-bromophenyl)-6-chloro-4'-(3-chlorophenyl) spiro[3H-indole-3,3'-piperidine]-2,6'(1H)-dione,
chiral (2'R, 3R, 4'S)-2'-(2-bromophenyl)-6-chloro-4'-(3-chlorophenyl) spiro[3H-indole-3,3'-piperidine]-2,6'(1H)-dione and
racemic (2'R, 3R, 4'S)-6-chloro-4'-(3-chlorophenyl)-2'-(3-cyanophenyl) spiro[3H-indole-3,3'-piperidine]-2,6'(1H)-dione.

12. A compound of claim 1 selected from the group consisting of
racemic (2'R, 3R, 4'S)-6-chloro-4'-(3-chlorophenyl)-1'-methyl-2'-(2-methylphenyl) spiro[3H-indole-3,3'-piperidine]-2,6'(1H)-dione,
chiral (2'R, 3R, 4'S)-6-chloro-4'-(3-chlorophenyl)-1'-methyl-2'-(2-methylphenyl) spiro[3H-indole-3,3'-piperidine]-2,6'(1H)-dione,
racemic (2'R, 3R, 4'S)-6-chloro-4'-(3-chlorophenyl)-2'-(3-fluoro-2-methyl-phenyl) spiro[3H-indole-3,3'-piperidine]-2,6'(1H)-dione,
chiral (2'R, 3R, 4'S)-6-chloro-4'-(3-chlorophenyl)-2'-(3-fluoro-2-methyl-phenyl) spiro[3H-indole-3,3'-piperidine]-2,6'(1H)-dione,
racemic (2'R, 3R, 4'S)-6-chloro-4'-(3-chlorophenyl)-1'-ethyl-2'-(2-methylphenyl) spiro[3H-indole-3,3'-piperidine]-2,6'(1H)-dione,
chiral (2'R, 3R, 4'S)-6-chloro-4'-(3-chlorophenyl)-1'-ethyl-2'-(2-methylphenyl) spiro[3H-indole-3,3'-piperidine]-2,6'(1H)-dione,
racemic (2'R, 3R, 4'S)-6-chloro-4'-(3-chlorophenyl)-2'-(2,6-dimethylphenyl) spiro[3H-indole-3,3'-piperidine]-2,6'(1H)-dione,
racemic (2'R, 3R, 4'S)-6-chloro-4'-(3-chlorophenyl)-2'-(2,3-dimethylphenyl) spiro[3H-indole-3,3'-piperidine]-2,6'(1H)-dione,
racemic (2'R, 3R, 4'S)-6-chloro-4'-(3-chlorophenyl)-1'-[(methoxycarbonyl) methyl]-2'-(2-methylphenyl) spiro[3H-indole-3,3'-piperidine]-2,6'(1H)-dione and
racemic (2'S, 3R)-6-chloro-2'-(3-chlorophenyl)-4'-isopropyl-6'-thioxo spiro[3H-indole-3,3'-piperidine]-2(1H)-one.

13. A compound of claim 1 selected from the group consisting of
racemic (2'R, 3R, 4'S)-6-chloro-4'-(3-chlorophenyl)-1'-[(hydroxycarbonyl)- methyl]-2'-(2-methylphenyl) spiro[3H-indole-3,3'-piperidine]-2,6'(1H)-dione,
racemic (2'R, 3R, 4'S)-6-chloro-4'-(3-chlorophenyl)-2'-[2-(trifluoromethyl)-phenyl] spiro[3H-indole-3,3'-piperidine]-2,6'(1H)-dione,
racemic (2'R, 3R, 4'S)-6-chloro-4'-(3-chlorophenyl)-6'-thioxo-2'-[2-(trifluoromethyl)-phenyl] spiro[3H-indole-3,3'-piperidine]-2(1H)-one,
racemic (2'R, 3R, 4'S)-6-chloro-4'-(3-chlorophenyl)-2'-(2-methylphenyl) -1'-[2-(4-morpholinyl)-carbonyl-methyl] spiro[3H-indole-3,3'-piperidine]-2,6'(1H)-dione,
chiral (2'R, 3R, 4'S)-6-chloro-4'-(3-chlorophenyl)-2'-(2-methylphenyl) -1'-[2-(4-morpholinyl)-carbonyl-methyl] spiro[3H-indole-3,3'-piperidine]-2,6'(1H)-dione,
racemic (2'R, 3R, 4'S)-6-chloro-4'-(3-chlorophenyl)-2'-[5-fluoro-2-(trifluoromethyl)-phenyl] spiro[3H-indole-3,3'-piperidine]-2,6'(1H)-dione,
racemic (2'R, 3R, 4'S)-6-chloro-4'-(3-chlorophenyl)-2'-(5-fluoro-2-methylphenyl) spiro[3H-indole-3,3'-piperidine]-2,6'(1H)-dione,
chiral (2'R, 3R, 4'S)-6-chloro-4'-(3-chlorophenyl)-2'-(5-fluoro-2-methylphenyl) spiro[3H-indole-3,3'-piperidine]-2,6'(1H)-dione,
racemic (2'R, 3R, 4'S)-6-chloro-4'-(3-chlorophenyl)-1'-[(cyclopropylamino)- carbonylmethyl]-2'-(2-methylphenyl) spiro[3H-indole-3,3'-piperidine]-2,6'(1H)-dione and
racemic (2'R, 3R, 4'S)-6-chloro-[[2-[6-chloro-4'-(3-chlorophenyl)-2,3-dihydro-2'-(2-methylphenyl)-2,6-dioxospiro[3H-indole-3,3'-piperidin]-1-yl]-1-oxoethyl]-amino]-piperidine carboxylic acid *tert*-butyl ester.

14. A compound of claim 1 selected from the group consisting of
racemic (2'R, 3R, 4'S)-6-chloro-4'-(3-chlorophenyl)-2'-(5-fluoro-2-methylphenyl)-6'-thioxo spiro[3H-indole-3,3'-piperidine]-2(1H)-one,
racemic (2'R, 3R, 4'S)-[6-chloro-4'-(3-chlorophenyl)-2,3-dihydro-2'-[2-(trifluoromethyl)phenyl)]-2-oxospiro[3H-indole-3,3'-piperidin]-6-ylene]-hydrazine carboxylic acid ethyl ester,
racemic (2'R, 3R, 4'S)-6-chloro-4'-(3-chlorophenyl)-2'-(2,4-difluorophenyl) spiro[3H-indole-3,3'-piperidine]-2,6'(1H)-dione,
racemic (2'R, 3R, 4'S)-6-chloro-4'-(3-chlorophenyl)-2'-(5-fluoro-2-methoxyphenyl) spiro[3H-indole-3,3'-piperidine]-2,6'(1H)-dione,
racemic (2'R, 3R, 4'S)-6-chloro-4'-(3-chlorophenyl)-2'-(1-naphthalenyl) spiro[3H-indole-3,3'-piperidine]-2,6'(1H)-dione,
racemic (2'R, 3R, 4'S)-6-chloro-4'-(3-chlorophenyl)-2'-(3-pyridinyl) spiro[3H-indole-3,3'-piperidine]-2,6'(1H)-dione,
racemic (2'R, 3R, 4'S)-6-chloro-4'-(3-chlorophenyl)-2'-(2, 3-difluoro-6-methoxyphenyl) spiro[3H-indole-3,3'-piperidine]-2,6'(1H)-dione,
racemic (2'R, 3R, 4'S)-6-chloro-4'-(3-chlorophenyl)-2'-(3, 4-difluorophenyl) spiro[3H-indole-3,3'-piperidine]-2,6'(1H)-dione,
racemic (2'R, 3R, 4'S)-6-chloro-4'-(3-chlorophenyl)-2'-(1-cyclohexenyl) spiro[3H-indole-3,3'-piperidine]-2,6'(1H)-dione and
racemic (2'R, 3R, 4'S)-6-chloro-4'-(3-chlorophenyl)-2'-(3, 4-difluorophenyl)- 6'-thioxo spiro[3H-indole-3,3'-piperidine]-2(1H)-one.

15. A compound of claim 1 selected from the group consisting of
racemic (2'R, 3R, 4'S)-[6-chloro-4'-(3-chlorophenyl)-2'-(3, 4-difluorophenyl)-2,3-dihydro-2-oxospiro[3H-indole-3,3'-piperidin]-6-ylene] hydrazine carboxylic acid ethyl ester,
racemic (2'R, 3R, 4'S)-2'-(1,3-benzodioxol-4-yl)-6-chloro-4'-(3-chlorophenyl) spiro[3H-indole-3,3'-piperidine]-2,6'(1H)-dione,
racemic (2'R, 3R, 4'S)-[6-chloro-4'-(3-chlorophenyl)-2'-(5-fluoro-2-methylphenyl)-2,3-dihydro-2-oxospiro[3H-indole-3,3'-piperidin]-6-ylene] hydrazine carboxylic acid ethyl ester,
racemic (2'R, 3R, 4'S)-6-chloro-4'-(3-chlorophenyl)-2'-(2, 3-difluoro-6-methylphenyl) spiro[3H-indole-3,3'-piperidine]-2,6'(1H)-dione,
racemic (2'R, 3R, 4'S)-6-chloro-4'-(3-chlorophenyl)-5-fluoro-2'-(5-fluoro-2-methylphenyl) spiro[3H-indole-3,3'-piperidine]-2,6'(1H)-dione,
racemic (2'R, 3R, 4'S)-6-chloro-4'-(3-chlorophenyl)-2'-(2, 3-difluoro-6-methylphenyl)-6'-thioxo spiro[3H-indole-3,3'-piperidine]-2(1H)-one,
racemic (2'R, 3R, 4'S) -6-chloro-4'-(3-chlorophenyl)-2'-(5-fluoro-2-methylphenyl)-1'-hydroxycarbonylmethyl spiro[3H-indole-3,3'-piperidine]-2,6'(1H)-dione,
racemic (2'R, 3R, 4'S)-6-chloro-4'-(3-chlorophenyl)-2'-(5-fluoro-2-methylphenyl)-1'-methyl spiro[3H-indole-3,3'-piperidine]- 2,6'(1H)-dione,
chiral (2'R, 3R, 4'S)-6-chloro-4'-(3-chlorophenyl)-2'-(5-fluoro-2-methylphenyl)-1'-methyl spiro[3H-indole-3,3'-piperidine]- 2,6'(1H)-dione and
racemic (2'R, 3R, 4'S)-6-chloro-4'-(3-chlorophenyl)-1'-fluorocarbonylmethyl-2'-(5-fluoro-2-methylphenyl) spiro[3H-indole-3,3'-piperidine]-2,6'(1H)-dione.

16. A compound of claim 1 selected from the group consisting of
racemic (2'R, 3R, 4'S)-6-chloro-4'-(3-chlorophenyl)-2'-(5-fluoro-2-methylphenyl)-1'-[(1-methyl-piperidin-4-yl)aminocarbonyl)-methyl] spiro[3H-indole-3,3'-piperidine]-2,6'(1h)-dione,
chiral (2'R, 3R, 4'S)-6-chloro-4'-(3-chlorophenyl)-2'-(5-fluoro-2-methylphenyl)-1'-[(1-methyl-piperidin-4-yl)aminocarbonyl)-methyl] spiro[3H-indole-3,3'-piperidine]-2,6'(1H)-dione,
racemic (2'R, 3R, 4'S)-1'-[1-tert-butoxycarbonyl-piperidin-4-yl)aminocarbonyl-methyl] 6-chloro-4'-(3-chlorophenyl)-2'-(5-fluoro-2-methylphenyl) spiro[3H-indole-3,3'-piperidine]-2,6'(1H)-dione,
racemic (2'R, 3R, 4'S)-6-chloro-4'-(3-chlorophenyl)-2'-(5-fluoro-2-methylphenyl)-1'-[(piperidin-4-yl)aminocarbonyl-methyl] spiro[3H-indole-3,3'-piperidine]-2,6'(1H)-dione,
chiral (2'R, 3R, 4'S)-6-chloro-4'-(3-chlorophenyl)-2'-(5-fluoro-2-methylphenyl)-1'-[(piperidin-4-yl)aminocarbonyl-methyl] spiro[3H-indole-3,3'-piperidine]-2,6'(1H)-dione,
racemic (2'R, 3R, 4'S)-6-chloro-4'-(3-chlorophenyl)-2'-(5-fluoro-2-methylphenyl)-1'-(3-morpholin-4-yl-propyl) spiro[3H-indole-3,3'-piperidine]-2,6'(1H)-dione,
chiral (2'R, 3R, 4'S)-6-chloro-4'-(3-chlorophenyl)-2'-(5-fluoro-2-methylphenyl)-1'-(3-morpholin-4-yl-propyl) spiro[3H-indole-3,3'-piperidine]-2,6'(1H)-dione,
racemic (2'R, 3R, 4'S)-6-chloro-4'-(3-chlorophenyl)-2'-(1-isopropyl-4-methyl-pent-1-enyl) spiro[3H-indole-3,3'-piperidine]-2,6'(1H)-dione,
racemic (2'R, 3R, 4'S)-6-chloro-4'-(3-chlorophenyl)-2'-(2,3-difluoro-6-isopropoxyphenyl) spiro[3H-indole-3,3'-piperidine]-2,6'(1H)-dione and
chiral (2'R, 3R, 4'S)-6-chloro-4'-(3-chlorophenyl)-2'-(2,3-difluoro-6-isopropoxy-phenyl) spiro[3H-indole-3,3'-piperidine]-2,6'(1H)-dione.

17. A compound of claim 1 selected from the group consisting of
racemic (2'R, 3R, 4'S)-6-chloro-4'-(3-chlorophenyl)-2'-(5-fluoro-2-methylphenyl)-1'-(3-pyrrolidin-1-yl-propyl) spiro[3H-indole-3,3'-piperidine]-2,6'(1H)-dione,
racemic (2'R, 3R, 4'S)-6-chloro-4'-(3-chlorophenyl)-2'-(3-methoxycarbonyl-phenyl) spiro[3H-indole-3,3'-piperidine]-2,6'(1H)-dione,
racemic (2'R, 3R, 4'S)-1'-[3-(4-acetyl-piperazin-1-yl)-propyl]-6-chloro-4'-(3-chlorophenyl)-2'-(5-fluoro-2-methylphenyl) spiro[3H-indole-3,3'-piperidine]-2,6'(1H)-dione,
racemic (2'R, 3R, 4'S)-6-chloro-4'-(3-chlorophenyl)-2'-(1-ethyl-propenyl) spiro[3H-indole-3,3'-piperidine]-2,6'(1H)-dione,
chiral (2'R, 3R, 4'S)-6-chloro-4'-(3-chlorophenyl)-2'-(1-ethyl-propenyl) spiro[3H-indole-3,3'-piperidine]-2,6'(1H)-dione,
racemic (2'R, 3R, 4'S)-6-chloro-4'-(3-chlorophenyl)-1'-[3-(1,1-dioxo-thiomorpholin-4-yl)-propyl]-2'-(5-fluoro-2-methylphenyl)- spiro[3H-indole-3,3'-piperidine]-2,6'(1H)-dione,
chiral (2'R, 3R, 4'S)-6-chloro-4'-(3-chlorophenyl)-1'-[3-(1,1-dioxo-thiomorpholin-4-yl)-propyl]-2'-(5-fluoro-2-methylphenyl)- spiro[3H-indole-3,3'-piperidine]-2,6'(1H)-dione,
racemic (2'R, 3R, 4'S)-6-chloro-4'-(3-chlorophenyl)-2'-(2, 5-dimethyl-phenyl) spiro[3H-indole-3,3'-piperidine]-2,6'(1H)-dione,
chiral (2'R, 3R, 4'S)-6-chloro-4'-(3-chlorophenyl)-2'-(2, 5-dimethyl-phenyl) spiro[3H-indole-3,3'-piperidine]-2,6'(1H)-dione and
racemic (2'R, 3R, 4'S)-6-chloro-4'-(3-chlorophenyl)-2'-(2,5-dimethyl-2H-pyrazole-3-yl) spiro[3H-indole-3,3'-piperidine]-2,6'(1H)-dione.

18. A compound of claim 1 selected from the group consisting of
racemic (2'R, 3R, 4'S)-6-chloro-4'-(3-chlorophenyl)-2'-(1-methyl-propenyl) spiro[3H-indole-3,3'-piperidine]-2,6'(1H)-dione,
racemic (2'R, 3R, 4'S)-6-chloro-4'-(3-chlorophenyl)-2'-(1-methyl-but-1-enyl) spiro[3H-indole-3,3'-piperidine]-2,6'(1H)-dione,
racemic (2'R, 3R, 4'S)-6-chloro-4'-(3-chlorophenyl)-2'-(1-ethyl-propyl) spiro[3H-indole-3,3'-piperidine]-2,6'(1H)-dione,
chiral (2'R, 3R, 4'S)-6-chloro-4'-(3-chlorophenyl)-2'-(1-ethyl-propyl) spiro[3H-indole-3,3'-piperidine]-2,6'(1H)-dione,
racemic (2'R, 3R, 4'S)-6-chloro-4'-(3-chlorophenyl)-2'-(2,3-difluoro-6-isopropoxyphenyl)-1'-(3-morpholin-4-yl-propyl) spiro[3H-indole-3,3'-piperidine]-2,6'(1H)-dione,
chiral (2'R, 3R, 4'S)-6-chloro-4'-(3-chlorophenyl)-2'-(2,3-difluoro-6-isopropoxyphenyl)-1'-(3-morpholin-4-yl-propyl) spiro[3H-indole-3,3'-piperidine]-2,6'(1H)-dione,
racemic (2'R, 3R, 4'S)-6-chloro-4'-(3-chlorophenyl)-2'-(1-ethylidene-pentyl) spiro[3H-indole-3,3'-piperidine]-2,6'(1H)-dione,
racemic (2'R, 3R, 4'S)-6-chloro-4'-(3-chlorophenyl)-2'-(2,3-difluoro-6-isopropoxyphenyl)-6'-thioxo spiro[3H-indole-3,3'-piperidine]-2(1H)-one,
racemic (2'R, 3R, 4'S)-[6-chloro-4'-(3-chlorophenyl)-2'-(2,3-difluoro-6-isopropoxyphenyl)-2,3-dihydro-2-oxospiro[3H-indole-3,3'-piperidin]-6-ylene] hydrazine carboxylic acid ethyl ester and
racemic (2'R, 3R, 4'S)-6-chloro-4'-(3-chlorophenyl)-2'-(cyclopent-1-enyl) spiro[3H-indole-3,3'-piperidine]-2,6'(1H)-dione.

19. A compound of claim 1 selected from the group consisting of
racemic (2'R, 3R, 4'S)-6-chloro-4'-(3-chlorophenyl)-2'-cyclopentyl spiro[3H-indole-3,3'-piperidine]-2,6'(1H)-dione,
chiral (2'R, 3R, 4'S)-6-chloro-4'-(3-chlorophenyl)-2'-cyclopentyl spiro[3H-indole-3,3'-piperidine]-2,6'(1H)-dione,
racemic (2'R, 3R, 4'S)-6-chloro-4'-(3-chlorophenyl)-2'-(1-isopropyl-propenyl) spiro[3H-indole-3,3'-piperidine]-2,6'(1H)-dione,
chiral (2'R, 3R, 4'S)-6-chloro-4'-(3-chlorophenyl)-2'-(1-isopropyl-propenyl) spiro[3H-indole-3,3'-piperidine]-2,6'(1H)-dione,
racemic (2'R, 3R, 4'S)-6-chloro-4'-(3-chlorophenyl)-2'-(1-methylene-propyl) spiro[3H-indole-3,3'-piperidine]-2,6'(1H)-dione,
chiral (2'R, 3R, 4'S)-6-chloro-4'-(3-chlorophenyl)-2'-(1-methylene-propyl) spiro[3H-indole-3,3'-piperidine]-2,6'(1H)-dione,
racemic (2'R, 3R, 4'S)-6-chloro-4'-(3-chlorophenyl)-2'-(1-ethyl-propenyl)-1'-hydroxycarbonylmethyl-spiro[3H-indole-3,3'-piperidine]-2,6'(1H)-dione,
racemic (2'R, 3R, 4'S)-6-chloro-4'-(3-chlorophenyl)-2'-(1-ethyl-propenyl)-1'-[(2-hydroxy-1,1-dimethylethyl)aminocarbonyl-methyl] spiro[3H-indole-3,3'-piperidine]-2,6'(1H)-dione,
chiral (2'R, 3R, 4'S) -6-chloro-4'-(3-chlorophenyl)-2'-(1-ethyl-propenyl)-1'-[(2-hydroxy-1,1-dimethylethyl)aminocarbonyl-methyl] spiro[3H-indole-3,3'-piperidine]-2,6'(1H)-dione and
racemic (2'R, 3R, 4'S)-6-chloro-4'-(3-chlorophenyl)-2'-(1-ethyl-propenyl)-1'-(3-morpholin-4-yl-propyl) spiro[3H-indole-3,3'-piperidine]-2,6'(1H)-dione.

20. A compound of claim 1 selected from the group consisting of
chiral (2'R, 3R, 4'S)-6-chloro-4'-(3-chlorophenyl)-2'-(1-ethyl-propenyl)-1'-(3-morpholin-4-yl-propyl) spiro[3H-indole-3,3'-piperidine]-2,6'(1H)-dione,
racemic (2'R, 3R, 4'S)-6-chloro-4'-(3-chlorophenyl)-2'-(2-methyl-1-methylene-propyl) spiro[3H-indole-3,3'-piperidine]-2,6'(1H)-dione,
racemic (2'R, 3R, 4'S)-6-chloro-4'-(3-chlorophenyl)-2'-(2,2-dimethyl-1-methylene-propyl) spiro[3H-indole-3,3'-piperidine]-2,6'(1H)-dione,
racemic (2'R, 3R, 4'S)-6-chloro-4'-(3-chlorophenyl)-2'-(1-ethyl-propenyl)-1'-[(2-morpholin-4-yl-ethyl)aminocarbonyl-methyl] spiro[3H-indole-3,3'-piperidine]-2,6'(1H)-dione,
racemic (2'R, 3R, 4'S)-6-chloro-4'-(3-chlorophenyl)-2'-isopropenyl spiro[3H-indole-3,3'-piperidine]-2,6'(1H)-dione,
chiral (2'R, 3R, 4'S)-6-chloro-4'-(3-chlorophenyl)-2'-isopropenyl spiro[3H-indole-3,3'-piperidine]-2,6'(1H)-dione,
racemic (2'R, 3R, 4'S)-6-chloro-4'-(3-chlorophenyl)-2'-(1-methylene-butyl) spiro[3H-indole-3,3'-piperidine]-2,6'(1H)-dione,
chiral (2'R, 3R, 4'S)-6-chloro-4'-(3-chlorophenyl)-2'-(1-methylene-butyl) spiro[3H-indole-3,3'-piperidine]-2,6'(1H)-dione,
racemic (2'R, 3R, 4'S)-6-chloro-4'-(3-chlorophenyl)-2'-(3-methyl-1-methylene-butyl) spiro[3H-indole-3,3'-piperidine]-2,6'(1H)-dione and
chiral (2'R, 3R, 4'S)-6-chloro-4'-(3-chlorophenyl)-2'-(3-methyl-1-methylene-butyl) spiro[3H-indole-3,3'-piperidine]-2,6'(1H)-dione.

21. A compound of claim 1 selected from the group consisting of
racemic (2'R, 3R, 4'S)-6-chloro-4'-(3-chlorophenyl)-2'-(1-methylene-butyl)-6'-thioxo spiro[3H-indole-3,3'-piperidine]-2(1H)-one,
racemic (2'R, 3R, 4'S)-6-chloro-4'-(3-chlorophenyl)-2'-(4-ethoxy-1,2-difluoro-phenyl) spiro[3H-indole-3,3'-piperidine]-2,6'(1H)-dione,
chiral (2'R, 3R, 4'S)-6-chloro-4'-(3-chlorophenyl)-2'-(4-ethoxy-1,2-difluoro-phenyl) spiro[3H-indole-3,3'-piperidine]-2,6'(1H)-dione,
racemic (2'R, 3R, 4'S)-6-chloro-4'-(3-chloro-5-methoxycarbonyl-phenyl)-2'-(1-methylene-propyl) spiro[3H-indole-3,3'-piperidine]-2,6'(1H)-dione,
racemic (2'R, 3R, 4'S)-6-chloro-4'-(3-chloro-5-hydroxycarbonyl-phenyl)-2'-(1-methylene-propyl) spiro[3H-indole-3,3'-piperidine]-2,6'(1H)-dione,
racemic (2'R, 3R, 4'S)-6-chloro-4'-(3-chloro-5-fluorocarbonyl-phenyl)-2'-(1-methylene-propyl) spiro[3H-indole-3,3'-piperidine]-2,6'(1H)-dione,
racemic (2'R, 3R, 4'S)-6-chloro-4'- [3-chloro-5-(4-methanesulfonyl-piperazine-1-carbonyl)-phenyl]-2'-(1-methylene-propyl) spiro[3H-indole-3,3'-piperidine]-2,6'(1H)-dione,
racemic (2'R, 3R, 4'S)-6-chloro-4'-(3-chlorophenyl)-2'-(1-ethyl-propenyl)-1'-methyl spiro[3H-indole-3,3'-piperidine]- 2,6'(1H)-dione,
chiral (2'R, 3R, 4'S)-6-chloro-4'-(3-chlorophenyl)-2'-(1-ethyl-propenyl)-1'-methyl spiro[3H-indole-3,3'-piperidine]- 2,6'(1H)-dione,
racemic (2'R, 3R, 4'S)-6-chloro-4'-(3-chlorophenyl)-2'-isopropyl spiro[3H-indole-3,3'-piperidine]-2,6'(1H)-dione and
chiral (2'R, 3R, 4'S)-6-chloro-4'-(3-chlorophenyl)-2'-isopropyl spiro[3H-indole-3,3'-piperidine]-2,6'(1H)-dione.

22. A compound of claim 1 selected from the group consisting of
racemic (2'R, 3R, 4'S)-6-chloro-4'-(3-chlorophenyl)-2'-(1-methylene-propyl)-1'-methyl spiro[3H-indole-3,3'-piperidine]- 2,6'(1H)-dione,
chiral (2'R, 3R, 4'S)-6-chloro-4'-(3-chlorophenyl)-2'-(1-methylene-propyl)-1'-methyl spiro[3H-indole-3,3'-piperidine]- 2,6'(1H)-dione,
racemic (2'R, 3R, 4'S)-2'-sec-Butyl-6-chloro-4'-(3-chlorophenyl) spiro[3H-indole-3,3'-piperidine]-2,6'(1H)-dione,
racemic (2'R, 3R, 4'S)-6-chloro-4'-(3-chlorophenyl)-2'-(1-hydroxymethyl-vinyl)-spiro[3H-indole-3,3'-piperidine]-2,6'(1H)-dione,
racemic (2'R, 3R, 4'S)-6-chloro-4'-(3-chlorophenyl)-2'-(1-methoxymethyl-vinyl) spiro[3H-indole-3,3'-piperidine]-2,6'(1H)-dione,
racemic (2'R, 3R, 4'S)-6-chloro-4'-(3-chlorophenyl)-2'-[2,3-difluoro-6-(2-methoxy-ethoxy)-phenyl] spiro[3H-indole-3,3'-piperidine]-2,6'(1H)-dione,
chiral (2'R, 3R, 4'S)-6-chloro-4'-(3-chlorophenyl)-2'-[2,3-difluoro-6-(2-methoxy-ethoxy)-phenyl] spiro[3H-indole-3,3'-piperidine]-2,6'(1H)-dione,
racemic (2'R, 3R, 4'S)-6-chloro-4'-(3-chlorophenyl)-2'-(1,2-dimethyl-propenyl) spiro[3H-indole-3,3'-piperidine]-2,6'(1H)-dione,
racemic (2'R, 3R, 4'S)-6-chloro-4'-(3-chlorophenyl)-2'-propionyl spiro[3H-indole-3,3'-piperidine]-2,6'(1H)-dione and
racemic (2'R, 3R, 4'S)-6-chloro-4'-(3-chlorophenyl)-2'-(2,3-difluoro-6-propoxy-phenyl) spiro[3H-indole-3,3'-piperidine]-2,6'(1H)-dione.

23. A compound of claim 1 selected from the group consisting of
chiral (2'R, 3R, 4'S)-6-chloro-4'-(3-chlorophenyl)-2'-(2,3-difluoro-6-propoxy-phenyl) spiro[3H-indole-3,3'-piperidine]-2,6'(1H)-dione,
racemic (2'R, 3R, 4'S)-6-chloro-4'-(3-chlorophenyl)-2'-(1-cyclopropyl-vinyl) spiro[3H-indole-3,3'-piperidine]-2,6'(1H)-dione,
racemic (2'R, 3R, 4'S)-6-chloro-4'-(3-chlorophenyl)-1'-hydroxycarbonylmethyl-2'-(1-methylene-propyl) spiro[3H-indole-3,3'-piperidine]-2,6'(1H)-dione,
racemic (2'R, 3R, 4'S)-6-chloro-4'-(3-chlorophenyl)-2'-(1-methylene-propyl)-1'-[(1-methanesulfonyl-piperidin-4-yl)aminocarbonyl-methyl] spiro[3H-indole-3,3'-piperidine]-2,6'(1H)-dione,
racemic (2'R, 3R, 4'S)-6-chloro-4'-(3-chlorophenyl)-2'-(1-methylene-propyl)-1'-(aminocarbonyl-methyl) spiro[3H-indole-3,3'-piperidine]-2,6'(1H)-dione,
chiral (2'R, 3R, 4'S)-6-chloro-4'-(3-chlorophenyl)-2'-(1-methylene-propyl)-1'-(aminocarbonyl-methyl) spiro[3H-indole-3,3'-piperidine]-2,6'(1H)-dione,
racemic (2'R, 3R, 4'S)-6-chloro-4'-(3-chlorophenyl)-2'-(1-ethyl-2-methyl-propenyl) spiro[3H-indole-3,3'-piperidine]-2,6'(1H)-dione,
racemic (2'R, 3R, 4'S)-6-chloro-4'-(3-chlorophenyl)-2'-[2,3-difluoro-6-(2-hydroxy-ethoxy)-phenyl] spiro[3H-indole-3,3'-piperidine]-2,6'(1H)-dione,
chiral (2'R, 3R, 4'S)-6-chloro-4'-(3-chlorophenyl)-2'-[2,3-difluoro-6-(2-hydroxy-ethoxy)-phenyl] spiro[3H-indole-3,3'-piperidine]-2,6'(1H)-dione and
racemic (2'R, 3R, 4'S)-6-chloro-4'-(3-chlorophenyl)-2'-(2,3-difluoro-6-hydroxy-phenyl) spiro[3H-indole-3,3'-piperidine]-2,6'(1H)-dione.

24. A compound of claim 1 selected from the group consisting of
racemic (2'R, 3R, 4'S)-6-chloro-4'-(3-chlorophenyl)-2'-(1-ethyl-1-hydroxy-propyl) spiro[3H-indole-3,3'-piperidine]-2,6'(1H)-dione,
racemic (2'R, 3R, 4'S)-6-chloro-4'-(3-chlorophenyl)-2'-(1-ethyl-3-methyl-oxiranyl) spiro[3H-indole-3,3'-piperidine]-2,6'(1H)-dione,
racemic (2'R, 3R, 4'S)-6-chloro-4'-(3-chlorophenyl)-2'-(1-fluoro-2-methyl-propenyl) spiro[3H-indole-3,3'-piperidine]-2,6'(1H)-dione,
racemic (2'R, 3R, 4'S)-6-chloro-4'-(3-chlorophenyl)-2'- isobutyryl spiro[3H-indole-3,3'-piperidine]-2,6'(1H)-dione,
racemic (2'R, 3R, 4'S)-6-chloro-4'-(3-chlorophenyl)-2'-isopropenyl -1'-hydroxycarbonylmethyl spiro[3H-indole-3,3'-piperidine]-2,6'(1H)-dione,
racemic (2'R, 3R, 4'S)-1'-(aminocarbonyl-methyl)-6-chloro-4'-(3-chlorophenyl)-2'-isopropenyl spiro[3H-indole-3,3'-piperidine]-2,6'(1H)-dione,
racemic (2'R, 3R, 4'S)-6-chloro-4'-(3-chlorophenyl)-1'-(cyclopropylaminocarbonyl-methyl)-2'-isopropenyl spiro[3H-indole-3,3'-piperidine]-2,6'(1H)-dione,
racemic (2'R, 3R, 4'S)-6-chloro-4'-(3-chlorophenyl)-2'-isopropenyl-1'-[(1-methyl-piperidin-4-yl)aminocarbonyl-methyl] spiro[3H-indole-3,3' piperidine]-2,6'(1H)-dione,
racemic (2'R, 3R, 4'S)-6-chloro-4'-(3-chlorophenyl)-2'-isopropenyl-1'-[(1-methylsulfonyl-piperidin-4-yl)aminocarbonyl-methyl] spiro[3H-indole-3,3' piperidine]-2.6'(1H)-dione and
racemic (2'R, 3R, 4'S)-6-chloro-4'-(3-chlorophenyl)-1'-[(2-hydroxyethyl)aminocarbonyl-methyl]-2'-isopropenyl-spiro[3H-indole-3,3'-piperidine]-2,6'(1H)-dione.

25. A compound of claim 1 selected from the group consisting of
racemic (2'R, 3R, 4'S)-6-chloro-4'-(3-chlorophenyl)-1'-[(2-hydroxy-1,1-dimethylethyl)aminocarbonyl-methyl]-2'-isopropenyl-spiro[3H-indole-3,3'-piperidine]-2,6'(1H)-dione,
racemic (2'R, 3R, 4'R)-6-chloro-4'-(3-chloro-phenyl)-4'-cyano-2'-(1-methylene-propyl) spiro[3H-indole-3,3'-piperidine]-2,6'(1H)-dione,
racemic (2'R, 3R, 4'S)-1'-[3-(4-acetylamino-piperidin-1-yl)-propyl] -6-chloro-4'-(3-chlorophenyl)-2'-isopropenyl spiro[3H-indole-3,3' piperidine]-2,6'(1H)-dione,
racemic (2'R, 3R, 4'S)-4'-(3-chloro-phenyl)-6'-cyano-2'-(1-methylene-propyl) spiro[3H-indole-3,3'-piperidine]-2,6'(1H)-dione,
racemic (2'R, 3R, 4'S)-4'-(3-chloro-phenyl)-6-cyano-2'-(5-fluoro-2-methyl-phenyl) spiro[3H-indole-3,3'-piperidine]-2,6'(1H)-dione,
racemic (2'R, 3R, 4'S)-1'-[3-(4-acetyl-piperazin-1-yl)-propyl]-6-chloro-4'-(3-chlorophenyl)-2'-isopropenyl spiro[3H-indole-3,3'- piperidine]-2,6'(1H)-dione,
racemic (2'R, 3R, 4'S)-6-chloro-4'-(3-chlorophenyl)-2'-isopropenyl- 1'-(3-piperidin-1-yl-propyl) spiro[3H-indole-3,3' piperidine]-2,6'(1H)-dione,
racemic (2'R, 3R, 4'S)-2'-[2-(2-acetoxy-ethoxy)-5-methyl-phenyl]-6-chloro-4'-(3-chlorophenyl) spiro[3H-indole-3,3'-piperidine]-2,6'(1H)-dione,
racemic (2'R, 3R, 4'S)-6-chloro-4'-(3-chlorophenyl)-2'-[6-(2-hydroxy-ethoxy)-3-methyl-phenyl] spiro[3H-indole-3,3'-piperidine]-2,6'(1H)-dione and
racemic (2'R, 3R, 4'S)-6-chloro-4'-(3-chlorophenyl)-2'-[5-cyclopropyl-2-(2-hydroxy-ethoxy)-phenyl] spiro[3H-indole-3,3'-piperidine]-2,6'(1H)-dione.

26. A compound of claim 1 selected from the group consisting of
racemic (2'R, 3R, 4'S)-6-chloro-2'-[5-chloro-2-(2-hydroxy-ethoxy)-phenyl]-4'-(3-chlorophenyl) spiro[3H-indole-3,3'-piperidine]-2,6'(1H)-dione,
racemic (2'R, 3R, 4'S)-6-chloro-2'-[3-chloro-2-fluoro-6-(2-hydroxy-ethoxy)-phenyl]-4'-(3-chlorophenyl) spiro[3H-indole-3,3'-piperidine]-2,6'(1H)-dione,
racemic (2'R, 3R, 4'S)-6-chloro-4'-(3,5-difluoro-phenyl)-2'-(1-methylene-propyl) spiro[3H-indole-3,3'-piperidine]-2,6'(1H)-dione,
racemic (2'R, 3R, 4'S)-6-chloro-4'-(3-cyano-phenyl)-2'-(1-methylene-propyl) spiro[3H-indole-3,3'-piperidine]-2,6'(1H)-dione,
racemic (2'R, 3R, 4'S)-4'-(3-bromo-phenyl)-6-chloro-2'-(1-methylene-propyl) spiro[3H-indole-3,3'-piperidine]-2,6'(1H)-dione,
racemic (2'R, 3R, 4'S)-6-chloro-4'-(3-methoxy-phenyl)-2'-(1-methylene-propyl) spiro[3H-indole-3,3'-piperidine]-2,6'(1H)-dione,
racemic (2'R, 3R, 4'S)-6-chloro-4'-(5-fluoro-2-methyl-phenyl)-2'-(1-methylene-propyl) spiro[3H-indole-3,3'-piperidine]-2,6'(1H)-dione,
racemic (2'R, 3R, 4'S)-6-chloro-4'-(3-fluoro-phenyl)-2'-(1-methylene-propyl) spiro[3H-indole-3,3'-piperidine]-2,6'(1H)-dione,
racemic (2'R, 3R, 4'S)-6-chloro-2'-(1-methylene-propyl)-4'-m-tolyl spiro[3H-indole-3,3'-piperidine]-2,6'(1H)-dione and
racemic (2'R, 3R, 4'S)-6-chloro-2'-(1-methylene-propyl)-4'-o-tolyl spiro[3H-indole-3,3'-piperidine]-2,6'(1H)-dione.

27. A compound of claim 1 selected from the group consisting of
racemic (2'R, 3R, 4'S)-6-chloro-2'-(1-methylene-propyl)-4'-thiophen-3-yl spiro[3H-indole-3,3'-piperidine]-2,6'(1H)-dione,
racemic (2'R, 3R, 4'S)-6-chloro-4'-(3,5-dichloro-phenyl)-2'-(1-methylene-propyl) spiro[3H-indole-3,3'-piperidine]-2,6'(1H)-dione,
racemic (2'R, 3R, 4'S)-6-chloro-4'-(5-chloro-2-trifluoromethyl-phenyl)-2'-(1-methylene-propyl) spiro[3H-indole-3,3'-piperidine]-2,6'(1H)-dione,
racemic (2'R, 3R, 4'S)-4'-(3-Bromo-phenyl)-6-chloro-2'-isopropenyl spiro[3H-indole-3,3'-piperidine]-2,6'(1H)-dione,
racemic (2'S, 3S, 4'R)-6-chloro-2'-(3-chlorophenyl )- 4'-(5-fluoro-2-methyl-phenyl)-spiro[3H-indole-3,3'-piperidine]-2,6'(1H)-dione,
racemic (2'S, 3S, 4'R)-6-chloro-2'-(3-chlorophenyl )- 4'-(5-fluoro-2-methyl-phenyl)-1'-hydroxycarbonylmethyl-spiro[3H-indole-3,3'-piperidine]-2,6'(1H)-dione,
racemic (2'S, 3S, 4'R)-6-chloro-2'-(3-chlorophenyl )- 4'-(5-fluoro-2-methyl-phenyl)-1'-(methylamino-carbonyl-methyl) spiro[3H-indole-3,3'-piperidine]-2,6'(1H)-dione,
racemic (2'S, 3S, 4'R)-6-chloro-2'-(3-chlorophenyl )- 1'-(dimethylamino-carbonylmethyl) -4'-(5-fluoro-2-methyl-phenyl)- spiro[3H-indole-3,3'-piperidine]-2,6'(1H)-dione,
racemic (2'S, 3S, 4'R) -1'-[(4-aminocarbonyl-piperidin-1-yl)carbonyl-methyl]-6-chloro-2'-(3-chlorophenyl )- 4'-(5-fluoro-2-methyl-phenyl) spiro[3H-indole-3,3'-piperidine]-2,6'(1H)-dione and
racemic (2'S, 3S, 4'R)-1'-[(3-aminocarbonyl-piperidin-1-yl)carbonyl-methyl] -6-chloro-2'-(3-chlorophenyl )- 4'-(5-fluoro-2-methyl-phenyl)- spiro[3H-indole-3,3'-piperidine]-2,6'(1H)-dione.

28. A compound of claim 1 selected from the group consisting of
racemic (2'S, 3S, 4'R) -1'-(aminocarbonyl-methyl) -6-chloro-2'-(3-chlorophenyl)- 4'-(5-fluoro-2-methyl-phenyl) spiro[3H-indole-3,3'-piperidine]-2,6'(1H)-dione,
racemic (2'S, 3S, 4'R)-6-chloro-2'-(3-chlorophenyl)-1'-(dimethylamino-propyl)-4'-(5-fluoro-2-methyl-phenyl)- spiro[3H-indole-3,3'-piperidine]-2,6'(1H)-dione,
racemic (2'S, 3S, 4'R)-6-chloro-4'-(3-chlorophenyl)-2'-(5-fluoro-2-methylphenyl)-1'-[(1-methanesulfonyl-piperidine-4-yl)carbonylamino-ethyl] spiro[3H-indole-3,3'-piperidine]-2,6'(1H)-dione,
racemic (2'R, 3R, 4'S)-6-chloro-4'-[2-(4-chloro-thiophenyl)]-2'-(5-fluoro-2-methylphenyl)-spiro[3H-indole-3,3'-piperidine]- 2,6'(1H)-dione,
racemic (2'R, 3R, 4'S)-6-chloro-4'-[2-(5-chloro-thiophenyl)]-2'-(5-fluoro-2-methylphenyl)-spiro[3H-indole-3,3'-piperidine]- 2,6'(1H)-dione,
racemic (2'R, 3R, 4'S)-6-chloro-4'-(2,2-dimethylpropyl)-2'-(5-fluoro-2-methylphenyl)-spiro[3H-indole-3,3'-piperidine]- 2,6'(1H)-dione,
racemic (2'R, 3R, 4'S)-6-bromo-4'-(3-chlorophenyl)-2'-(2-methylphenyl) spiro[3H-indole-3,3'-piperidine]-2,6'(1H)-dione,
racemic (2'R, 3R, 4'S)-6-bromo-4'-(3-chlorophenyl)-2'-(5-fluoro-2-methylphenyl) spiro[3H-indole-3,3'-piperidine]-2,6'(1H)-dione,
chiral (2'R, 3R, 4'S)-6-chloro-4'-(3-chlorophenyl)-2'-(5-fluoro-2-methylphenyl) spiro[3H-indole-3,3'-piperidine]-2,6'(1H)-dione and
racemic (2'R, 3R, 4'S)-6-bromo-4'-(3-chlorophenyl)-2'-(2,5-dichlorophenyl) spiro[3H-indole-3,3'-piperidine]-2,6'(1H)-dione.

29. A compound of claim 1 selected from the group consisting of
racemic (2'R, 3R, 4'S)-6-bromo-4'-(3-chlorophenyl)-2'-(5-chloro-2-methylphenyl) spiro[3H-indole-3,3'-piperidine]-2,6'(1H)-dione,
racemic (2'R, 3R, 4'S)-6-bromo-4'-(3-chlorophenyl )- 2'-(1-methylene-propyl)-spiro[3H-indole-3,3'-piperidine]- 2,6'(1H)-dione,
chiral (2'R, 3R, 4'S)-6-bromo-4'-(3-chlorophenyl )- 2'-(1-methylene-propyl)- spiro[3H-indole-3,3'-piperidine]- 2,6'(1H)-dione,
chiral (2R, 3R, 4'S)-6-bromo-4'-(3-chlorophenyl )-2'-(1-ethyl-cyclopropyl) spiro[3H-indole-3,3'-piperidine]-2,6'(1H)-dione,
racemic (2'R,3R,4'S)-6-cyclopropyl-4'-(3-chlorophenyl)-2'-(1-ethyl-cyclopropyl)-spiro[3H-indole-3,3'-piperidine]- 2,6'(1H)-dione,
racemic (2'R, 3R, 4'S) 2'-[2-(4-aminocarbonyl-piperidin-1-yl)methyl-5-fluoro-phenyl)-6-chloro-4'-(3-chlorophenyl)-spiro[3H-indole-3,3'-piperidine]- 2,6'(1H)-dione,
racemic (2'R, 3R, 4'S)-6-chloro-4'-(3-chlorophenyl )-2'-[5-fluoro-2-(4-methanesulfonyl-piperazin-1-yl)methyl-phenyl]-spiro[3H-indole-3,3'-piperidine]-2,6'(1H)-dione,
racemic (2'R, 3R, 4'S)-6-chloro-4'-(3-chlorophenyl )-2'-{5-fluoro-2-[(1-methanesulfonyl-piperidin-4-yl)carbonylamino-methyl]-phenyl}-spiro[3H-indole-3,3'-piperidine]- 2,6'(1H)-dione,
chiral (2'R, 3R, 4'S)-4'-(3-chlorophenyl)- 6-fluoro-2'-(5-fluoro-2-methylphenyl) spiro[3H-indole-3,3'-piperidine]-2,6'(1H)-dione and
racemic (2'R, 3R, 4'S)-4'-(3-chlorophenyl)-2'-(5-fluoro-2-methylphenyl)- 6-methoxy spiro[3H-indole-3,3'-piperidine]-2,6'(1H)-dione.

30. A compound of claim 1 selected from the group consisting of
racemic (2'R, 3R, 4'S)-4'-(3-chlorophenyl)-2'-(5-fluoro-2-methylphenyl) spiro[3H-indole-3,3'-piperidine]-2,6'(1H)-dione,
racemic (2'R, 3R, 4'S)-4'-(3-chlorophenyl)- 5-fluoro-2'-(5-fluoro-2-methylphenyl) spiro[3H-indole-3,3'-piperidine]-2,6'(1H)-dione,
chiral (2'R, 3R, 4'S)-6-chloro-4'-(3-chloro-phenyl)-2'-(1-methyl-cyclopropyl) spiro[3H-indole-3,3'-piperidine]-2,6'(1H)-dione,
racemic (2'R, 3R, 4'S)-6-bromo-4'-(3-chlorophenyl)-2'-isopropenyl spiro[3H-indole-3,3'-piperidine]-2,6'(1H)-dione,
chiral (2'R, 3R, 4'S)-6-bromo-4'-(3-chloro-phenyl)-2'-(1-methyl-cyclopropyl) spiro[3H-indole-3,3'-piperidine]-2,6'(1H)-dione,
racemic (2'R, 3R, 4'S)-4'-(3-chlorophenyl )-6-ethynyl-2'-isopropenyl spiro[3H-indole-3,3'-piperidine]-2,6'(1H)-dione,
chiral (2'R, 3'R, 4'S)-6-chloro-4'-(3-chlorophenyl)-2'-(5-fluoro-2-methylphenyl)-1'-[(1-methylsulphonyl-4-piperidinyl)aminocarbonyl-methyl] spiro[3H-indole-3,3'-piperidine]-2,6'(1H)-dione,
chiral (2'R,3'R,4'S)-6-chloro-4'-(3-chlorophenyl)-2'-(5-fluoro-2-methylphenyl)-1'-{[4-(1,1-dioxido-2-isothiazolidinyl)ethyl]piperazinyl-carbonyl-methyl} spiro[3H-indole-3,3'-piperidine]-2,6'(1H)-dione,
chiral (2'R,3'R,4'S)-6-chloro-4'-(3-chlorophenyl)-2'-(5-fluoro-2-methylphenyl)-1'-{[3-(methylsulphonyl)propyl]piperazinyl-carbonyl-methyl} spiro[3H-indole-3,3'-piperidine]-2,6'(1H)-dione and
racemic (2'R, 3R, 4'S)-2'-(2-bromo-5-fluorophenyl) 6-chloro-4'-(3-chlorophenyl) spiro[3H-indole-3,3'-piperidine]-2,6'(1H)-dione.

31. A compound of claim 1 selected from the group consisting of
racemic (2'R, 3R, 4'S)-2'-6-chloro-4'-(3-chlorophenyl)- (2-ethynyl-5-fluorophenyl) spiro[3H-indole-3,3'-piperidine]-2,6'(1H)-dione,
racemic (2'R, 3R, 4'S)-2'-6-chloro-4'-(3-chlorophenyl)-{5-fluoro-2-[3-(methanesulfony I-methyl-amino)-prop-1-ynyl-phenyl] spiro[3H-indole-3,3'-piperidine]-2,6'(1H)-dione,
racemic (2'R, 3R, 4'S)-2'-[5-bromo-2-(2-hydroxy-ethoxy)-phenyl]-6-chloro-4'-(3-chlorophenyl) spiro[3H-indole-3,3'-piperidine]-2,6'(1H)-dione,
racemic (2'R, 3R, 4'S)-6-chloro-4'-(3-chlorophenyl)-2'-[5-ethynyl-2-(2-hydroxy-ethoxy)-phenyl] spiro[3H-indole-3,3'-piperidine]-2,6'(1H)-dione,
chiral (2'R, 3R, 4'S)-6-chloro-4'-(3-chlorophenyl)-2'-[5-ethynyl-2-(2-hydroxy-ethoxy)-phenyl] spiro[3H-indole-3,3'-piperidine]-2,6'(1H)-dione,
racemic (2'R, 3R, 4'S)-2'-[3-bromo-2-fluoro-6-(2-hydroxy-ethoxy)-phenyl]-6-chloro-4'-(3-chlorophenyl) spiro[3H-indole-3,3'-piperidine]-2,6'(1H)-dione,
racemic (2'R, 3R, 4'S)-6-chloro-4'-(3-chlorophenyl)-2'-[2-fluoro-6-(2-hydroxy-ethoxy)-3-trimethylsilanylethynyl-phenyl] spiro[3H-indole-3,3'-piperidine]-2,6'(1H)-dione,
racemic (2'R, 3R, 4'S)-6-chloro-4'-(3-chlorophenyl)-2'-[3-ethynyl-2-fluoro-6-(2-hydroxy-ethoxy)-phenyl] spiro[3H-indole-3,3'-piperidine]-2,6'(1H)-dione
and
racemic (2'R, 3R, 4'S)-6-chloro-4'-(3-chlorophenyl)-2'-[5-ethyl-2-(2-hydroxy-ethoxy)-phenyl] spiro[3H-indole-3,3'-piperidine]-2,6'(1H)-dione.

32. A pharmaceutical formulation comprising a compound of the formula 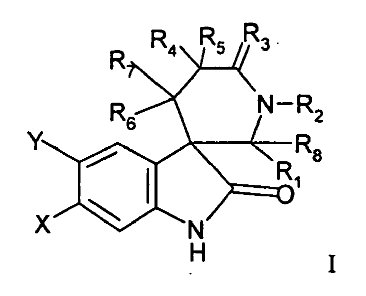 wherein
X is selected from the group consisting of hydrogen, halogen, cyano, nitro, ethynyl, cyclopropyl, methyl, ethyl, isopropyl, methoxy and vinyl;
Y is hydrogen or fluorine;
R₄ and R₅ are hydrogen or lower alkyl having from 1 to 8 carbon atoms;
one of R₁ and R₈ is selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl, and substituted cycloalkenyl and the other is hydrogen;
one of R₆ and R₇ is selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl, and substituted cycloalkenyl and the other is hydrogen, cyano or lower alkyl having from 1 to 8 carbon atoms;
R₂ is selected from the group consisting of hydrogen, lower alkyl having from 1 to 8 carbon atoms and substituted lower alkyl having from 1 to 8 carbon atoms;
R₃ is selected from the group consisting of oxygen, sulfur and NNH(C=O)OR₉;
R₉ is lower alkyl having from 1 to 8 carbon atoms or substituted lower alkyl having from 1 to 8 carbon atoms;
and the pharmaceutically acceptable salts and esters thereof together with a pharmaceutically acceptable carrier or excipient.

33. The pharmaceutical formulation according to claim 32 for the treatment of cancer, in particular solid tumors, more particularly breast, colon, lung and prostate tumors.

34. The compounds according to claims 1 to 31 for the use as medicaments.

35. The compounds according to claims 1 to 31 for the use as medicaments for the treatment of cancer, in particular solid tumors, more particularly breast, colon, lung and prostate tumors.

36. The use of a compound according to claims 1 to 31 for the manufacture of medicaments for the treatment of cancer, in particular solid tumors, more particularly breast, colon, lung and prostate tumors.

37. A process to produce a compound of the formula 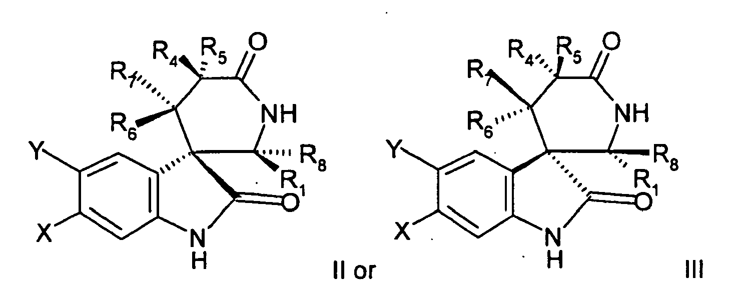 wherein
X is selected from the group consisting of hydrogen, halogen, cyano, nitro, cyclopropyl, methyl, ethyl, and isopropyl;
Y is hydrogen or fluorine;
R₁ is hydrogen;
R₄ and R₅ are hydrogen or lower alkyl having from 1 to 8 carbon atoms;
R₈ is selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl, and substituted cycloalkenyl;
one of R₆ and R₇ are selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl, and substituted cycloalkenyl and the other is hydrogen, cyano or lower alkyl having from 1 to 8 carbon atoms;
which comprises reacting a compound of the formula 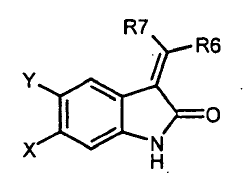 with a compound of the formula 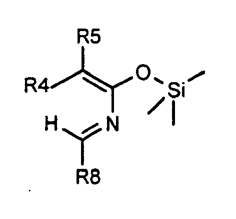 at about 110-160°C and under anhydrous conditions to produce a compound of formula II or III.

38. A process to produce a compound of the formula 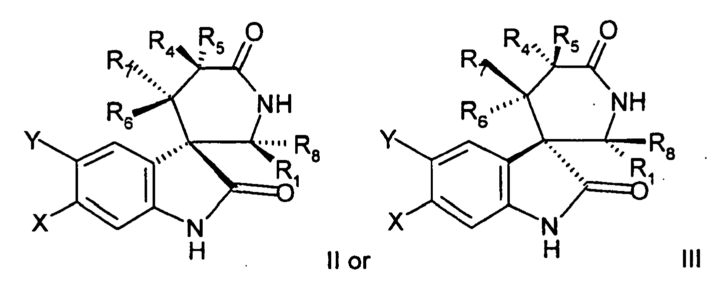 wherein
X is selected from the group consisting of hydrogen, halogen, cyano, nitro, cyclopropyl, methyl, ethyl, and isopropyl;
Y is hydrogen or fluorine;
R₁ is hydrogen;
R₄ and R₅ are hydrogen or lower alkyl having from 1 to 8 carbon atoms;
R₈ is selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl, and substituted cycloalkenyl;
one of R₆ and R₇ are selected from the group consisting of lower alkyl having from 1 to 8 carbon atoms, substituted lower alkyl having from 1 to 8 carbon atoms, lower alkenyl having from 2 to 8 carbon atoms, substituted lower alkenyl having from 2 to 8 carbon atoms, aryl, substituted aryl, heteroaryl which heteroaryl is an aromatic heterocyclic ring system containing up to two rings, substituted heteroaryl which heteroaryl is an aromatic heterocyclic ring system containing up to two rings, heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, substituted heterocycle which heterocycle is a 5 to 8 membered, mono- or bicyclic, aromatic or non-aromatic hydrocarbon, wherein 1 to 3 carbon atoms are replaced by a heteroatom selected from nitrogen, oxygen or sulphur atom, cycloalkyl, substituted cycloalkyl, cycloalkenyl, and substituted cycloalkenyl and the other is hydrogen, cyano or lower alkyl having from 1 to 8 carbon atoms;
which comprises reacting a compound of the formula 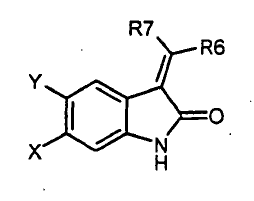 with a suitable protecting group to provide a compound of the formula 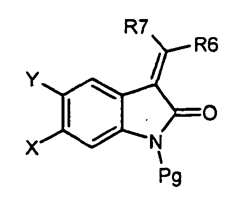 which is thereafter reacted with a compound of the formula 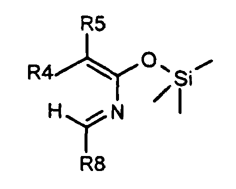 at about 110-160°C under anhydrous conditions and thereafter deprotecting the resultant product to produce a compound of formula II and III.

## Patentansprüche

1. Verbindung der Formel 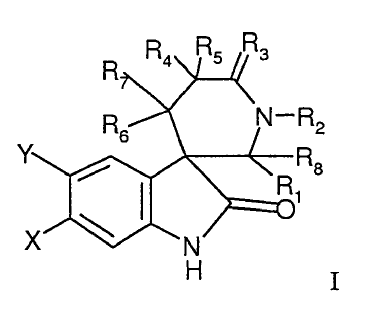 worin
X ausgewählt ist aus der Gruppe bestehend aus Wasserstoff, Halogen, Cyano, Nitro, Ethinyl, Cyclopropyl, Methyl, Ethyl, Isopropyl, Methoxy und Vinyl;
Y Wasserstoff oder Fluor ist;
R₄ und R₅ Wasserstoff oder Niederalkyl mit von 1 bis 8 Kohlenstoffatomen sind;
einer von R₁ und R₈ ausgewählt ist aus der Gruppe bestehend aus Niederalkyl mit von 1 bis 8 Kohlenstoffatomen, substituiertem Niederalkyl mit von 1 bis 8 Kohlenstoffatomen, Niederalkenyl mit von 2 bis 8 Kohlenstoffatomen, substituiertem Niederalkenyl mit von 2 bis 8 Kohlenstoffatomen, Aryl, substituiertem Aryl, Heteroaryl, welches Heteroaryl ein aromatisches heterocyclisches Ringsystem ist, das bis zu zwei Ringe enthält, substituiertem Heteroaryl, welches Heteroaryl ein aromatisches heterocyclisches Ringsystem ist, das bis zu zwei Ringe enthält, Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, substituiertem Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, Cycloalkyl, substituiertem Cycloalkyl, Cycloalkenyl und substituiertem Cycloalkenyl und der andere Wasserstoff ist;
einer von R₆ und R₇ ausgewählt ist aus der Gruppe bestehend aus Niederalkyl mit von 1 bis 8 Kohlenstoffatomen, substituiertem Niederalkyl mit von 1 bis 8 Kohlenstoffatomen, Niederalkenyl mit von 2 bis 8 Kohlenstoffatomen, substituiertem Niederalkenyl mit von 2 bis 8 Kohlenstoffatomen, Aryl, substituiertem Aryl, Heteroaryl, welches Heteroaryl ein aromatisches heterocyclisches Ringsystem ist, das bis zu zwei Ringe enthält, substituiertem Heteroaryl, welches Heteroaryl ein aromatisches heterocyclisches Ringsystem ist, das bis zu zwei Ringe enthält, Heterocyclus, welcher Heterocyclus ein 5- bis 8-gliedriger, mono- oder bicyclischer, aromatischer oder nicht-aromatischer Kohlenwasserstoff ist, Worin 1 bis 3 Kohlenstoffatome durch ein Heteroatom ausgewählt aus Stickstoff-, Sauerstoff- oder Schwefelatom ersetzt sind, substituiertem Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, Cycloalkyl, substituiertem Cycloalkyl, Cycloalkenyl und substituiertem Cycloalkenyl und der andere Wasserstoff, Cyano oder Niederalkyl mit von 1 bis 8 Kohlenstoffatomen ist;
R₂ ausgewählt ist aus der Gruppe bestehend aus Wasserstoff, Niederalkyl mit von 1 bis 8 Kohlenstoffatomen und substituiertem Niederalkyl mit von 1 bis 8 Kohlenstoffatomen;
R₃ ausgewählt ist aus der Gruppe bestehend aus Sauerstoff, Schwefel und NNH(C=O)OR₉;
R₉ Niederalkyl mit von 1 bis 8 Kohlenstoffatomen oder substituiertes Niederalkyl mit von 1 bis 8 Kohlenstoffatomen ist;
und die pharmazeutisch akzeptablen Salze und Ester davon.

2. Verbindung nach Anspruch 1 der Formel 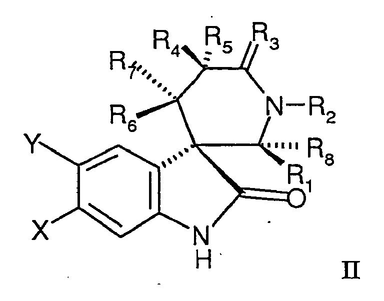 worin
X Wasserstoff, Halogen, Cyano, Nitro, Ethinyl, Cyclopropyl, Methyl, Ethyl, Isopropyl, Methoxy und Vinyl ist;
Y Wasserstoff oder Fluor ist;
R₁ Wasserstoff ist;
R₂ Wasserstoff, Niederalkyl mit von 1 bis 8 Kohlenstoffatomen oder substituiertes Niederalkyl mit von 1 bis 8 Kohlenstoffatomen ist;
R₄ und R₅ Wasserstoff oder Niederalkyl mit von 1 bis 8 Kohlenstoffatomen sind;
R₆ Wasserstoff, Cyano oder Niederalkyl mit von 1 bis 8 Kohlenstoffatomen ist;
R₃ O, S oder NNH(C=O)OR₉ ist;
R₇/R₈ unabhängig voneinander ausgewählt ist aus der Gruppe bestehend aus Niederalkyl mit von 1 bis 8 Kohlenstoffatomen, substituiertem Niederalkyl mit von 1 bis 8 Kohlenstoffatomen, Niederalkenyl mit von 2 bis 8 Kohlenstoffatomen, substituiertem Niederalkenyl mit von 2 bis 8 Kohlenstoffatomen, Aryl, substituiertem Aryl, Heteroaryl, welches Heteroaryl ein aromatisches heterocyclisches Ringsystem ist, das bis zu zwei Ringe enthält, substituiertem Heteroaryl, welches Heteroaryl ein aromatisches heterocyclisches Ringsystem ist, das bis zu zwei Ringe enthält, Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, substituiertem
Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, Cycloalkyl, substituiertem Cycloalkyl, Cycloalkenyl und substituiertem Cycloalkenyl;
R₉ Niederalkyl mit von 1 bis 8 Kohlenstoffatomen oder substituiertes Niederalkyl mit von 1 bis 8 Kohlenstoffatomen ist;
und die pharmazeutisch akzeptablen Salze und Ester davon.

3. Verbindung nach Anspruch 2, worin
X Chlor oder Brom ist;
Y Wasserstoff ist;
R₁ Wasserstoff ist;
R₄ und R₅ beide Wasserstoff sind;
R₆ Wasserstoff ist;
R₃ O ist;
R₇ ein substituiertes Phenyl oder substituiertes Heteroaryl ist, wobei das substituierte Phenyl oder substituierte Heteroaryl ausgewählt ist aus der Gruppe bestehend aus 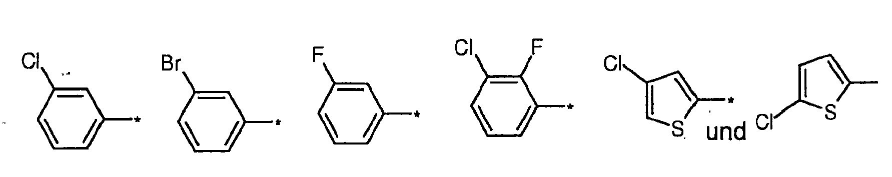 R₈ unabhängig voneinander ausgewählt ist aus der Gruppe bestehend aus Niederalkyl mit von 1 bis 8 Kohlenstoffatomen, substituiertem Niederalkyl mit von 1 bis 8 Kohlenstoffatomen, Niederalkenyl mit von 2 bis 8 Kohlenstoffatomen, substituiertem Niederalkenyl mit von 2 bis 8 Kohlenstoffatomen, Aryl, substituiertem Aryl, Heteroaryl, welches Heteroaryl ein aromatisches heterocyclisches Ringsystem ist, das bis zu zwei Ringe enthält, substituiertem Heteroaryl, welches Heteroaryl ein aromatisches heterocyclisches Ringsystem ist, das bis zu zwei Ringe enthält, Cycloalkyl, substituiertem Cycloalkyl, Cycloalkenyl und substituiertem Cycloalkenyl; und R₂ Wasserstoff, Niederalkyl mit von 1 bis 8 Kohlenstoffatomen oder substituiertes Niederalkyl mit von 1 bis 8 Kohlenstoffatomen ist;
mit der Maßgabe, dass wenn R₂ Niederalkyl mit von 1 bis 8 Kohlenstoffatomen oder substituiertes Niederalkyl mit von 1 bis 8 Kohlenstoffatomen ist, R₈ ausgewählt ist aus Niederalkenyl mit von 2 bis 8 Kohlenstoffatomen, substituiertem Niederalkenyl mit von 2 bis 8 Kohlenstoffatomen, Aryl, substituiertem Aryl, Heteroaryl, welches Heteroaryl ein aromatisches heterocyclisches Ringsystem ist, das bis zu zwei Ringe enthält, substituiertem Heteroaryl, welches Heteroaryl ein aromatisches heterocyclisches Ringsystem ist, das bis zu zwei Ringe enthält, Cycloalkyl, substituiertem Cycloalkyl, Cycloalkenyl oder substituiertem Cycloalkenyl.

4. Verbindung nach Anspruch 3, worin
X -Cl oder -Br ist;
Y Wasserstoff ist;
R₁ Wasserstoff ist;
R₄ und R₅ beide Wasserstoff sind;
R₆ Wasserstoff ist;
R₃ O ist;
R₇ ein substituiertes Phenyl oder substituiertes Heteroaryl ist, wobei das substituierte Phenyl oder substituierte Heteroaryl ausgewählt ist aus der Gruppe bestehend aus 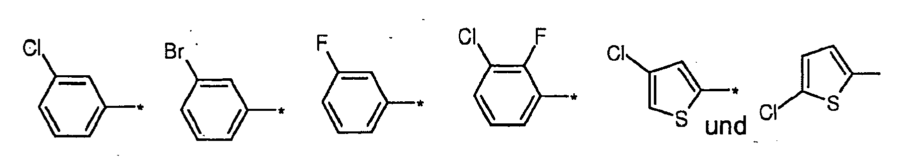 und
R₈ ausgewählt ist aus der Gruppe bestehend aus 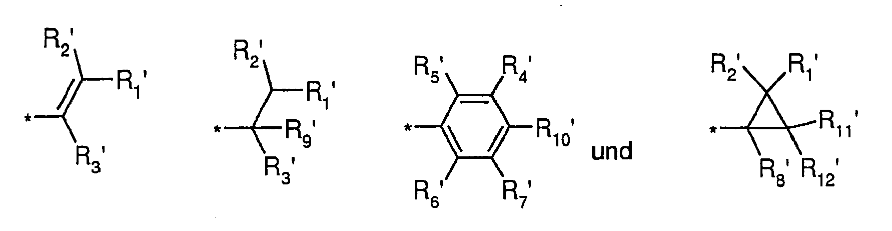 worin
R₁' Wasserstoff, Methyl, Ethyl, Propyl, Isopropyl, -CF₃, -F, -CHF₂ oder -CH₂F ist;
R₂' Wasserstoff, Methyl, Ethyl, Propyl, Isopropyl, -CF₃, -F, -CHF₂ oder -CH₂F ist;
R₃' Wasserstoff, -F, -CF₃, -CH₂F, Methyl, Ethyl, Propyl, Isopropyl, cyclopropyl, tert-Butyl oder sec-Butyl ist;
R₄' Wasserstoff, -F, -Cl, -Br, -I, Methyl, Ethyl, Cyclopropyl, Cyano, Methoxy oder Ethinyl ist;
R₅' Wasserstoff, -F oder Methyl ist;
R₆' ausgewählt ist aus der Gruppe bestehend aus Wasserstoff, Halogen, Hydroxy, Cyano, Niederalkyl mit von 1 bis 8 Kohlenstoffatomen, substituiertem Niederalkyl mit von 1 bis 8 Kohlenstoffatomen, Niederalkoxy mit von 1 bis 8 Kohlenstoffatomen, substituiertem Niederalkoxy mit von 1 bis 8 Kohlenstoffatomen, Niederalkinyl mit von 2 bis 6 Kohlenstoffatomen, substituiertem Niederalkinyl mit von 2 bis 6 Kohlenstoffatomen;
R₇' Wasserstoff, -F oder Methyl ist;
R₈' Wasserstoff, -F, Methyl, Ethyl, Propyl, Isopropyl, tert-Butyl oder sec-Butyl ist;
R₉' Wasserstoff, Hydroxyl oder -F ist;
R₁₀' Wasserstoff oder -F ist;
R₁₁' Wasserstoff oder Methyl ist;
R₁₂' Wasserstoff oder Methyl ist;
R₂ Wasserstoff, Niederalkyl mit von 1 bis 8 Kohlenstoffatomen oder substituiertes Niederalkyl mit von 1 bis 8 Kohlenstoffatomen ist;
mit der Maßgabe, dass wenn R₂ Niederalkyl mit von 1 bis 8 Kohlenstoffatomen oder substituiertes Niederalkyl mit von 1 bis 8 Kohlenstoffatomen ist, R₈ ausgewählt ist aus der Gruppe bestehend aus

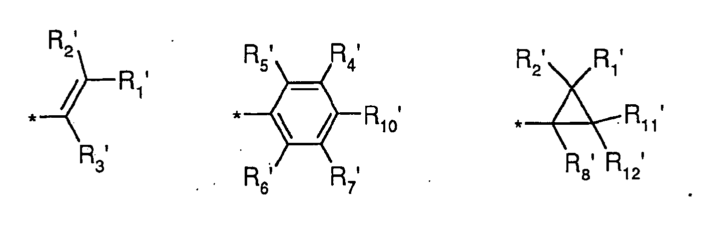

5. Verbindung nach Anspruch 1 der Formel 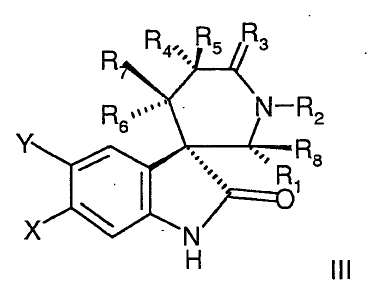 worin
X ausgewählt ist aus der Gruppe bestehend aus Wasserstoff, Halogen, Cyano, Nitro, Ethinyl, Cyclopropyl, Methyl, Ethyl, Isopropyl, Methoxy und Vinyl;
Y Wasserstoff oder Fluor ist;
R₁ Wasserstoff ist;
R₂ Wasserstoff, Niederalkyl mit von 1 bis 8 Kohlenstoffatomen oder substituiertes Niederalkyl mit von 1 bis 8 Kohlenstoffatomen ist;
R₄ und R₅ Wasserstoff oder Niederalkyl mit von 1 bis 8 Kohlenstoffatomen sind;
R₆ Wasserstoff, Cyano oder Niederalkyl mit von 1 bis 8 Kohlenstoffatomen ist;
R₃ O, S oder NNH(C=O)OR₉ ist;
R₇/R₈ unabhängig voneinander ausgewählt ist aus der Gruppe bestehend aus Niederalkyl mit von 1 bis 8 Kohlenstoffatomen, substituiertem Niederalkyl mit von 1 bis 8 Kohlenstoffatomen, Niederalkenyl mit von 2 bis 8 Kohlenstoffatomen, substituiertem Niederalkenyl mit von 2 bis 8 Kohlenstoffatomen, Aryl, substituiertem Aryl, Heteroaryl, welches Heteroaryl ein aromatisches heterocyclisches Ringsystem ist, das bis zu zwei Ringe enthält, substituiertem Heteroaryl, welches Heteroaryl ein aromatisches heterocyclisches Ringsystem ist, das bis zu zwei Ringe enthält, Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, substituiertem Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, Cycloalkyl, substituiertem Cycloalkyl, Cycloalkenyl oder substituiertem Cycloalkenyl; und
R₉ Niederalkyl mit von 1 bis 8 Kohlenstoffatomen oder substituiertes Niederalkyl mit von 1 bis 8 Kohlenstoffatomen ist;
und die pharmazeutisch akzeptablen Salze und Ester davon.

6. Verbindung nach Anspruch 5 worin
X -Cl oder -Br ist;
Y Wasserstoff ist;
R₁ Wasserstoff ist;
R₂ Wasserstoff, Niederalkyl mit von 1 bis 8 Kohlenstoffatomen oder substituiertes Niederalkyl mit von 1 bis 8 Kohlenstoffatomen ist;
R₄ und R₅ beide Wasserstoff sind;
R₈ Wasserstoff ist;
R₃ O ist;
R₈ ein substituiertes Phenyl ist, wobei das substituierte Phenyl ausgewählt ist aus der Gruppe bestehend aus 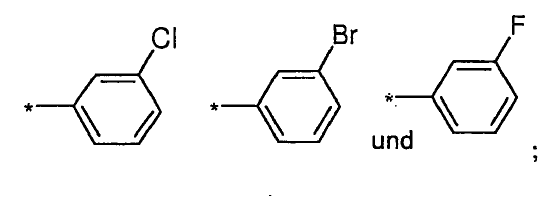 und
R₇ ausgewählt ist aus der Gruppe bestehend aus Niederalkyl mit von 1 bis 8 Kohlenstoffatomen, substituiertem Niederalkyl mit von 1 bis 8 Kohlenstoffatomen, Niederalkenyl mit von 2 bis 8 Kohlenstoffatomen, substituiertem Niederalkenyl mit von 2 bis 8 Kohlenstoffatomen, Aryl, substituiertem Aryl, Heteroaryl, welches Heteroaryl ein aromatisches heterocyclisches Ringsystem ist, das bis zu zwei Ringe enthält, substituiertem Heteroaryl, welches Heteroaryl ein aromatisches heterocyclisches Ringsystem ist, das bis zu zwei Ringe enthält, Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, substituiertem Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, Cycloalkyl, substituiertem Cycloalkyl, Cycloalkenyl oder substituiertem Cycloalkenyl.

7. Verbindung der Formel I, II oder III nach Ansprüchen 1, 2 oder 5 worin
X -Cl ist;
Y; R₁; R₂; R₄; R₅ und R₆ Wasserstoff sind;
R₃ Sauerstoff ist; und
R₇ und R₈ Phenylgruppen sind, die jede unabhängig voneinander unsubstituiert oder 1, 2 oder 3 mal substituiert sind mit
Niederalkyl mit von 1 bis 8 Kohlenstoffatomen, Niederalkenyl mit von 2 bis 8 Kohlenstoffatomen, Niederalkinyl mit von 2 bis 6 Kohlenstoffatomen, Dioxo-Niederalkenyl mit von 1 bis 8 Kohlenstoffatomen (Bilden z.B. einer Benzodioxylgruppe), Halogen, Hydroxy, -CN, -CF₃, -NH₂, -N(H, Niederalkyl mit von 1 bis 8 Kohlenstoffatomen), N(Niederalkyl mit von 1 bis 8 Kohlenstoffatomen)₂, Aminocarbonyl, Carboxy, -NO₂, Niederalkoxy mit von 1 bis 8 Kohlenstöffatomen, Thio-Niederalkoxy mit von 1 bis 8 Kohlenstoffatomen, Niederalkylsulfonyl mit von 1 bis 8 Kohlenstoffatomen, Aminosulfonyl, Niederalkyl(mit von 1 bis 8 Kohlenstoffatomen)carbonyl, Niederalkyl(mit von 1 bis 8 Kohlenstoffatomen)carbonyloxy, Niederalkoxy(mit von 1 bis 8 Kohlenstoffatomen)carbonyl, Niederalkyl(mit von 1 bis 8 Kohlenstoffatomen)carbonyl-NH, Fluor-Niederalkyl mit von 1 bis 8 Kohlenstoffatomen, Fluor-Niederalkoxy mit von 1 bis 8 Kohlenstoffatomen, Niederalkoxy(mit von 1 bis 8 Kohlenstoffatomen)carbonyl-Niederalkoxy (mit von 1 bis 8 Kohlenstoffatomen), Carboxy-Niederalkoxy (mit von 1 bis 8 Kohlenstoffatomen), Carbamoyl-Niederalkoxy (mit von 1 bis 8 Kohlenstoffatomen), Hydroxy-Niederalkoxy (mit von 1 bis 8 Kohlenstoffatomen), -NH₂-Niederalkoxy mit von 1 bis 8 Kohlenstoffatomen, -N(H, Niederalkyl mit von 1 bis 8 Kohlenstoffatomen)-Niederalkoxy mit von 1 bis 8 Kohlenstoffatomen, -N(Niederalkyl mit von 1 bis 8 Kohlenstoffatomen)₂-Niederalkoxy mit von 1 bis 8 Kohlenstoffatomen, Benzyloxy-Niederalkoxy(mit von 1 bis 8 Kohlenstoffatomen), Mono- oder Di-(Niederalkyl mit von 1 bis 8 Kohlenstoffatomen) substituiertem Aminosulfonyl und Niederalkyl mit von 1 bis 8 Kohlenstoffatomen, das gegebenenfalls mit Halogen, Hydroxy, -NH₂, -N(H, Niederalkyl mit von 1 bis 8 Kohlenstoffatomen) oder -N(Niederalkyl mit von 1 bis 8 Kohlenstoffatomen)₂ substituiert sein kann.

8. Verbindung der Formel I, II oder III nach Ansprüchen 1 , 2 oder 5 worin
X -Cl ist;
Y; R₁; R₂; R₄; R₅ und R₆ Wasserstoff sind;
R₃ Sauerstoff ist; und
R₇ und R₈ Phenylgruppen sind, die jede unabhängig voneinander unsubstituiert oder 1, 2 oder 3 mal substituiert sind mit einem Substituenten unabhängig voneinander ausgewählt aus
Halogen, C₂₋₄-Alkinyl oder -O-(C₁₋₄ Alkyl) und
wobei die C₂₋₄-Alkinyl- oder -O-(C₁₋₄ Alkyl)gruppen unsubstituiert oder einmal substituiert sind mit Hydroxyl oder -N(C₁₋₄ Alkyl)-S(O)₂-CH₃.

9. Verbindung nach Anspruch 1 ausgewählt aus der Gruppe bestehend aus
(2'S, 3S, 4'S)-6-Chlor-2'-(3-chlorphenyl)-4'-(2,2-dimethylpropyl) spiro[3H-indol-3,3'-piperidin]-2,6'(1H)-dion,
(2'SR, 3S, 4'R)-4'-(*tert*-Butyl)-6-chlor-2'-(3-chlorphenyl) spiro[3H-indol-3,3'-piperidin]-2,6'(1H)-dion,
(2'S, 3S, 4'R)-6-Chlor-2'-(3-chlorphenyl)-4'-isopropyl spiro[3H-indol-3,3'-piperidin]-2,6'(1H)-dion,
racemischem (2'R, 3R, 4'S)-6-Chlor-2',4'-bis(3-chlorphenyl) spiro[3H-indol-3,3'-piperidin]-2,6'(1H)-dion,
chiralem (2'R, 3R, 4'S)-6-Chlor-2',4'-bis(3-chlorphenyl) spiro[3H-indol-3,3'-piperidin]-2,6'(1H)-dion,
racemischem (2'S, 3S, 4'R)-6-Chlor-2'-(3-chlorphenyl)-4'-cyclopentyl spiro[3H-indol-3,3'-piperidin]-2,6'(1H)-dion,
racemischem (2'S, 3S, 4'R)-6-Chlor-2'-(3-chlorphenyl)-4'-cyclohexyl spiro[3H-indol-3,3'-piperidin]-2,6'(1H)-dion,
racemischem (2'S, 3S, 4'R)-6-Chlor-2'-(4-chlorphenyl)-4'-cyclohexyl spiro[3H-indol-3,3'-piperidin]-2,6'(1H)-dion,
racemischem (2'R, 3R, 4'S)-6-Chlor-2'-(4-chlorphenyl)-4'-(3-chlorphenyl) spiro[3H-indol-3,3'-piperidin]-2,6'(1H)-dion und
racemischem (2'S, 3S, 4'R)-6-Chlor-2'-(3-chlorphenyl)-4'-(4-chlorphenyl) spiro[3H-indol-3,3'-piperidin]-2,6'(1H)-dion.

10. Verbindung nach Anspruch 1 ausgewählt aus der Gruppe bestehend aus
racemischem (2'R, 3R, 4'S)-6-Chlor-4'-(3-chlorphenyl)-2'-(3-methylphenyl) spiro[3H-indol-3,3'-piperidin]-2,6'(1H)-dion,
racemischem (2'R, 3R, 4'S)-6-Chlor-4'-(3-chlorphenyl)-2'-(3-fluorphenyl) spiro[3H-indol-3,3'-piperidin]-2,6'(1H)-dion,
racemischem (2'S, 3S, 4'R)-6-Chlor-2'-(3-chlorphenyl)-4'-phenyl spiro[3H-indol-`3,3'-piperidin]-2,6'(1H)-dion,
racemischem (2'S, 3S, 4'R, 5'R)-6-Chlor-2'-(3-chlorphenyl)-5'-methyl-4'-phenyl spiro[3H-indol-3,3'-piperidin]-2,6'(1H)-dion,
racemischem (2'R, 3R, 4'S)-6-Chlor-4'-(3-chlorphenyl)-2'-phenyl spiro[3H-indol-3,3'-piperidin]-2,6'(1H)-dion,
chiralem (2'R, 3R, 4'S)-6-Chlor-4'-(3-chlorphenyl)-2'-phenyl spiro[3H-indol-3,3'-piperidin]-2,6'(1H)-dion,
racemischem (2'R, 3R, 4'S)-6-Chlor-4'-(3-chlorphenyl)-2'-(3-methoxyphenyl) spiro[3H-indol-3,3'-piperidin]-2,6'(1H)-dion,
racemischem (2'R, 3R, 4'S)-6-Chlor-2'-(2-chlorphenyl)-4'-(3-chlorphenyl) spiro[3H-indol-3,3'-piperidin]-2,6'(1H)-dion,
chiralem (2'R, 3R, 4'S)-6-Chlor-2'-(2-chlorphenyl)-4'-(3-chlorphenyl) spiro[3H-indol-3,3'-piperidin]-2,6'(1H)-dion und
racemischem (2'R, 3R, 4'S)-6-Chlor-4'-(3-chlorphenyl)-2'-(2-methylphenyl) spiro[3H-indol-3,3'-piperidin]-2,6'(1H)-dion.

11. Verbindung nach Anspruch 1 ausgewählt aus der Gruppe bestehend aus
chiralem (2'R, 3R, 4'S)-6-Chlor-4'-(3-chlorphenyl)-2'-(2-methylphenyl) spiro[3H-indol-3,3'-piperidin]-2,6'(1H)-dion,
racemischem (2'R, 3R, 4'S)-6-Chlor-4'-(3-chlorphenyl)-2'-(2-ethylphenyl) spiro[3H-indol-3,3'-piperidin]-2,6'(1H)-dion,
chiralem (2'R, 3R, 4'S)-6-Chlor-4'-(3-chlorphenyl)-2'-(2-ethylphenyl) spiro[3H-indol-3,3'-piperidin]-2,6'(1H)-dion,
racemischem (2'R, 3R, 4'S, 5'S)-6-Chlor-4'-(3-chlorphenyl)-5'-methyl-2'-(2-methylphenyl) spiro[3H-indol-3,3'-piperidin]-2,6'(1H)-dion,
racemischem (2'R, 3R, 4'S, 5'S)-6-Chlor-2',4'-bis(3-chlorphenyl)-5'-methyl spiro[3H-indol-3,3'-piperidin]-2,6'(1H)-dion,
racemischem (2'R, 3R, 4'S)-6-Chlor-4'-(3-chlorphenyl)-2'-(2-isopropylphenyl) spiro[3H-indol-3,3'-piperidin]-2,6'(1H)-dion,
chiralem (2'R, 3R, 4'S)-6-Chlor-4'-(3-chlorphenyl)-2'-(2-isopropylphenyl) spiro[3H-indol-3,3'-piperidin]-2,6'(1H)-dion,
racemischem (2'R, 3R, 4'S)-2'-(2-Bromphenyl)-6-chlor-4'-(3-chlorphenyl) spiro[3H-indol-3,3'-piperidin]-2,6'(1H)-dion,
chiralem (2'R, 3R, 4'S)-2'-(2-Bromphenyl)-6-chlor-4'-(3-chlorphenyf) spiro[3H-indol-3,3'-piperidin]-2,6'(1H)-dion und
racemischem (2'R, 3R, 4'S)-6-Chlor-4'-(3-chlorphenyl)-2'-(3-cyanophenyl) spiro[3H-indol-3,3'-piperidin]-2,6'(1H)-dion.

12. Verbindung nach Anspruch 1 ausgewählt aus der Gruppe bestehend aus
racemischem (2'R, 3R, 4'S)-6-Chlor-4'-(3-chlorphenyl)-1'-methyl-2'-(2-methylphenyl) spiro[3H-indol-3,3'-piperidin]-2,6'(1H)-dion,
chiralem (2'R, 3R, 4'S)-6-Chlor-4'-(3-chlorphenyl)-1'-methyl-2'-(2-methylphenyl) spiro[3H-indol-3,3'-piperidin]-2,6'(1H)-dion,
racemischem (2'R, 3R, 4'S)-6-Chlor-4'-(3-chlorphenyl)-2'-(3-fluor-2-methylphenyl) spiro[3H-indol-3,3'-piperidin]-2,6'(1H)-dion,
chiralem (2'R, 3R, 4'S)-6-Chlor-4'-(3-chlorphenyl)-2'-(3-fluor-2-methylphenyl) spiro[3H-indol-3,3'-piperidin]-2,6'(1H)-dion,
racemischem (2'R, 3R, 4'S)-6-Chlor-4'-(3-chlorphenyl)-1'-ethyl-2'-(2-methylphenyl) spiro[3H-indol-3,3'-piperidin]-2,6'(1H)-dion,
chiralem (2'R, 3R, 4'S)-6-Chlor-4'-(3-chlorphenyl)-1'-ethyl-2'-(2-methylphenyl) spiro[3H-indol-3,3'-piperidin]-2,6'(1H)-dion,
racemischem (2'R, 3R, 4'S)-6-Chlor-4'-(3-chlorphenyl)-2'-(2,6-dimethylphenyl) spiro[3H-indol-3,3'-piperidin]-2,6'(1H)-dion,
racemischem (2'R, 3R, 4'S)-6-Chlor-4'-(3-chlorphenyl)-2'-(2,3-dimethylphenyl) spiro[3H-indol-3,3'-piperidin]-2,6'(1H)-dion,
racemischem (2'R, 3R, 4'S)-6-Chlor-4'-(3-chlorphenyl)-1'-[(methoxycarbonyl) methyl]-2'-(2-methylphenyl) spiro[3H-indol-3,3'-piperidin]-2,6'(1H)-dion und
racemischem (2'S, 3R)-6-Chlor-2'-(3-chlorphenyl)-4'-isopropyl-6'-thioxo spiro[3H-indol-3,3'-piperidin]-2(1H)-on.

13. Verbindung nach Anspruch 1 ausgewählt aus der Gruppe bestehend aus
racemischem (2'R, 3R, 4'S)-6-Chlor-4'-(3-chlorphenyl)-1'-[(hydroxycarbonyl)-methyl]-2'-(2-methylphenyl) spiro[3H-indol-3,3'-piperidin]-2,6'(1H)-dion,
racemischem (2'R, 3R, 4'S)-6-Chlor-4'-(3-chlorphenyl)-2'-[2-(trifluormethyl)-phenyl] spiro[3H-indol-3,3'-piperidin]-2,6'(1H)-dion,
racemischem (2'R, 3R, 4'S)-6-Chlor-4'-(3-chlorphenyl)-6'-thioxo-2'-[2-(trifluormethyl)-phenyl] spiro[3H-indol-3,3'-piperidin]-2(1H)-on,
racemischem (2'R, 3R, 4'S)-6-Chlor-4'-(3-chlorphenyl)-2'-(2-methylphenyl) -1'-[2-(4- morpholinyl)-carbonyl-methyl] spiro[3H-indol-3,3'-piperidin]-2,6'(1H)-dion,
chiralem (2'R, 3R, 4'S)-6-Chlor-4'-(3-chlorphenyl)-2'-(2-methylphenyl)-1'-[2-(4- morpholinyl)-carbonyl-methyl] spiro[3H-indol-3,3'-piperidin]-2,6'(1H)-dion,
racemischem (2'R, 3R, 4'S)-6-Chlor-4'-(3-chlorphenyl)-2'-[5-fluor-2-(trifluormethyl)-phenyl] spiro[3H-indol-3,3'-piperidin]-2,6'(1H)-dion,
racemischem (2'R, 3R, 4'S)-6-Chlor-4'-(3-chlorphenyl)-2'-(5-fluor-2-methylphenyl) spiro[3H-indol-3,3'-piperidin]-2,6'(1H)-dion,
chiralem (2'R, 3R, 4'S)-6-Chlor-4'-(3-chlorphenyl)-2'-(5-fluor-2-methylphenyl) spiro[3H-indol-3,3'-piperidin]-2,6'(1H)-dion,
racemischem (2'R, 3R, 4'S)-6-Chlor-4'-(3-chlorphenyl)-1'-[(cyclopropylamino)-carbonyl-methyl]-2'-(2-methylphenyl) spiro[3H-indol-3,3'-piperidin]-2,6'(1H)-dion und
racemischem (2'R, 3R, 4'S)-6-Chlor-[[2-[6-chlor-4'-(3-chlorphenyl)-2,3-dihydro-2'-(2-methylphenyl)-2,6-dioxospiro[3H-indol-3,3'-piperidin]-1-yl]-1-oxoethyl]-amino]-piperidin carbonsäure *tert*-butylester.

14. Verbindung nach Anspruch 1 ausgewählt aus der Gruppe bestehend aus
racemischem (2'R, 3R, 4'S)-6-Chlor-4'-(3-chlorphenyl)-2'-(5-fluor-2-methylphenyl)-6'-thioxo spiro[3H-indol-3,3'-piperidin]-2(1H)-on,
racemischem (2'R, 3R, 4'S)-[6-Chlor-4'-(3-chlorphenyl)-2,3-dihydro-2'-[2-(trifluormethyl)phenyl)]-2-oxospiro[3H-indol-3,3'-piperidin]-6-ylen]-hydrazin-carbonsäureethylester,
racemischem (2'R, 3R, 4'S)-6-Chlor-4'-(3-chlorphenyl)-2'-(2,4-difluorphenyl) spiro[3H-indol-3,3'-piperidin]-2,6'(1H)-dion,
racemischem (2'R, 3R, 4'S)-6-Chlor-4'-(3-chlorphenyl)-2'-(5-fluor-2-methoxyphenyl) spiro[3H-indol-3,3'-piperidin]-2,6'(1H)-dion,
racemischem (2'R, 3R, 4'S)-6-Chlor-4'-(3-chlorphenyl)-2'-(1-naphthalenyl) spiro[3H-indol-3,3'-piperidin]-2,6'(1H)-dion,
racemischem (2'R, 3R, 4'S)-6-Chlor-4'-(3-chlorphenyl)-2'-(3-pyridinyl) spiro[3H-indot-3,3'-piperidin]-2,6'(1H)-dion,
racemischem (2'R, 3R, 4'S)-6-Chlor-4'-(3-chlorphenyl)-2'-(2, 3-difluor-6-methoxyphenyl) spiro[3H-indol-3,3'-piperidin]-2,6'(1H)-dion,
racemischem (2'R, 3R, 4'S)-6-Chlor-4'-(3-chlorphenyl)-2'-(3,4-difluorphenyl) spiro[3H-indol-3,3'-piperidin]-2,6'(1H)-dion,
racemischem (2'R, 3R, 4'S)-6-Chlor-4'-(3-chlorphenyl)-2'-(1-cyclohexenyl) spiro[3H-indol-3,3'-piperidin]-2,6'(1H)-dion und
racemischem (2'R, 3R, 4'S)-6-Chlor-4'-(3-chlorphenyl)-2'-(3,4-difluorphenyl)-6'-thioxo spiro[3H-indol-3,3'-piperidin]-2(1H)-on.

15. Verbindung nach Anspruch 1 ausgewählt aus der Gruppe bestehend aus
racemischem (2'R, 3R, 4'S)-[6-Chlor-4'-(3-chlorphenyl)-2'-(3,4-difluorphenyl)-2,3-dihydro-2-oxospiro[3H-indol-3,3'-piperidin]-6-ylen] hydrazin-carbonsäureethylester,
racemischem (2'R, 3R, 4'S)-2'-(1,3-Benzodioxol-4-yl)-6-chlor-4'-(3-chlorphenyl) spiro[3H-indol-3,3'-piperidin]-2,6'(1H)-dion,
racemischem (2'R, 3R, 4'S)-[6-Chlor-4'-(3-chlorphenyl)-2'-(5-fluor-2-methylphenyl)-2,3-dihydro-2-oxospiro[3H-indol-3,3'-piperidin]-6-ylen] hydrazin-carbonsäureethylester,
racemischem (2'R, 3R, 4'S)-6-Chlor-4'-(3-chlorphenyl)-2'-(2,3-difluor-6-methylphenyl) spiro[3H-indol-3,3'-piperidin]-2,6'(1H)-dion,
racemischem (2'R, 3R, 4'S)-6-Chlor-4'-(3-chlorphenyl)-5-fluor-2'-(5-fluor-2-methylphenyl) spiro[3H-indol-3,3'-piperidin]-2,6'(1H)-dion,
racemischem (2'R, 3R, 4'S)-6-Chlor-4'-(3-chlorphenyl)-2'-(2,3-difluor-6-methylphenyl)-6'-thioxo spiro[3H-indol-3,3'-piperidin]-2(1H)-on,
racemischem (2'R, 3R, 4'S)-6-Chlor-4'-(3-chlorphenyl)-2'-(5-fluor-2-methylphenyl)-1'-hydroxycarbonylmethyl spiro[3H-indol-3,3'-piperidin]-2,6'(1H)-dion,
racemischem (2'R, 3R, 4'S)-6-Chlor-4'-(3-chlorphenyl)-2'-(5-fluor-2-methylphenyl)-1'-methyl spiro[3H-Indol-3,3'-piperidin]-2,6'(1H)-dion,
chiralem (2'R, 3R, 4'S)-6-Chlor-4'-(3-chlorphenyl)-2'-(5-fluor-2-methylphenyl)-1'-methyl spiro[3H-indol-3,3'-piperidin]-2,6'(1H)-dion und
racemischem (2'R, 3R, 4'S)-6-Chlor-4'-(3-chlorphenyl)-1'-fluorcarbonylmethyl-2'-(5-fluor-2-methylphenyl) spiro[3H-indol-3,3'-piperidin]-2,6'(1H)-dion.

16. Verbindung nach Anspruch 1 ausgewählt aus der Gruppe bestehend aus
racemischem (2'R, 3R, 4'S)-6-Chlor-4'-(3-chlorphenyl)-2'-(5-fluor-2-methylphenyl)-1'-[(1-methyl-piperidin-4-yl)aminocarbonyl)-methyl] spiro[3H-indol-3,3'-piperidin]-2,6'(1H)-dion,
chiralem (2'R, 3R, 4'S)-6-Chlor-4'-(3-chlorphenyl)-2'-(5-fluor-2-methylphenyl)-1'-[(1-methyl-piperidin-4-yl)aminocarbonyl)-methyl] spiro[3H-indol-3,3'-piperidin]-2,6'(1H)-dion,
racemischem (2'R, 3R, 4'S)-1'-[1-tert-Butoxycarbonyl-piperidin-4-yl)aminocarbonyl-methyl] 6-chlor-4'-(3-chlorphenyl)-2'-(5-fluor-2-methylphenyl) spiro[3H-indol-3,3'-piperidin]-2,6'(1H)-dion,
racemischem (2'R, 3R, 4'S)-6-Chlor-4'-(3-chlorphenyl)-2'-(5-fluor-2-methylphenyl)-1'-[(piperidin-4-yl)aminocarbonyl-methyl] spiro[3H-indol-3,3'-piperidin]-2,6'(1H)-dion,
chiralem (2'R, 3R, 4'S)-6-Chlor-4'-(3-chlorphenyl)-2'-(5-fluor-2-methylphenyl)-1'-[(piperidin-4-yl)aminocarbonyl-methyl] spiro[3H-indol-3,3'-piperidin]-2,6'(1H)-dion,
racemischem (2'R, 3R, 4'S)-6-Chlor-4'-(3-chlorphenyl)-2'-(5-fluor-2-methylphenyl)-1'-(3-morpholin-4-yl-propyl) spiro[3H-indol-3,3'-piperidin]-2,6'(1H)-dion,
chiralem (2'R, 3R, 4'S)-6-Chlor-4'-(3-chlorphenyl)-2'-(5-fluor-2-methylphenyl)-1'-(3-morpholin-4-yl-propyl) spiro[3H-indol-3,3'-piperidin]-2,6'(1H)-dion,
racemischem (2'R, 3R, 4'S)-6-Chlor-4'-(3-chlorphenyl)-2'-(1-isopropyl-4-methyl-pent-1-enyl) spiro[3H-indol-3,3'-piperidin]-2,6'(1H)-dion,
racemischem (2'R, 3R, 4'S)-6-Chlor-4'-(3-chlorphenyl)-2'-(2,3-difluor-6-isopropoxy-phenyl) spiro[3H-indol-3,3'-piperidin]-2,6'(1H)-dion und
chiralem (2'R, 3R, 4'S)-6-Chlor-4'-(3-chlorphenyl)-2'-(2,3-difluor-6-isopropoxy-phenyl) spiro[3H-indol-3,3-piperidin]-2,6'(1H)-dion.

17. Verbindung nach Anspruch 1 ausgewählt aus der Gruppe bestehend aus
racemischem (2'R, 3R, 4'S)-6-Chlor-4'-(3-chlorphenyl)-2'-(5-fluor-2-methylphenyl)-1'-(3-pyrrolidin-1-yl-propyl) spiro[3H-indol-3,3'-piperidin]-2,6'(1H)-dion,
racemischem (2'R, 3R, 4'S)-6-Chlor-4'-(3-chlorphenyl)-2'-(3-methoxycarbonyl-phenyl) spiro[3H-indol-3,3'-piperidin]-2,6'(1H)-dion, racemischem (2'R, 3R, 4'S)-1'-[3-(4-Acetyl-piperazin-1-yl)-propyl]-6-chlor-4'-(3-chlorphenyl)-2'-(5-fluor-2-methylphenyl) spiro[3H-indol-3,3'-piperidin]-2,6'(1H)-dion,
racemischem (2'R, 3R, 4'S)-6-Chlor-4'-(3-chlorphenyl)-2'-(1-ethyl-propenyl) spiro[3H-indol-3,3'-piperidin]-2,6'(1H)-dion,
chiralem (2'R, 3R, 4'S)-6-Chlor-4'-(3-chlorphenyl)-2'-(1-ethyl-propenyl) spiro[3H-indol-3,3'-piperidin]-2,6'(1H)-dion,
racemischem (2'R, 3R, 4'S)-6-Chlor-4'-(3-chlorphenyl)-1'-[3-(1,1-dioxo-thiomorpholin-4-yl)-propyl]-2'-(5-fluor-2-methylphenyl)- spiro[3H-indol-3,3'-piperidin]-2,6'(1H)-dion,
chiralem (2'R, 3R, 4'S)-6-Chlor-4'-(3-chlorphenyl)-1'-[3-(1,1-dioxo-thiomorpholin-4-yl)-propyl]-2'-(5-fluor-2-methylphenyl)- spiro[3H-indol-3,3'-piperidin]-2,6'(1H)-dion,
racemischem (2'R, 3R, 4'S)-6-Chlor-4'-(3-chlorphenyl)-2'-(2,5-dimethyl-phenyl) spiro[3H-indol-3,3'-piperidin]-2,6'(1H)-dion,
chiralem (2'R, 3R, 4'S)-6-Chlor-4'-(3-chlorphenyl)-2'-(2,5-dimethyl-phenyl) spiro[3H-indol-3,3'-piperidin]-2,6'(1H)-dion und
racemischem (2'R, 3R, 4'S)-6-Chlor-4'-(3-chlorphenyl)-2'-(2,5-dimethyl-2H-pyrazol-3-yl) spiro[3H-indol-3,3'-piperidin]-2,6'(1H)-dion.

18. Verbindung nach Anspruch 1 ausgewählt aus der Gruppe bestehend aus
racemischem (2'R, 3R, 4'S)-6-Chlor-4'-(3-chlorphenyl)-2'-(1-methyl-propenyl) spiro[3H-indol-3,3'-piperidin]-2,6'(1H)-dion,
racemischem (2'R, 3R, 4'S)-6-Chlor-4'-(3-chlorphenyl)-2'-(1-methyl-but-1-enyl) spiro[3H-indol-3,3'-piperidin]-2,6'(1H)-dion,
racemischem (2'R, 3R, 4'S)-6-Chlor-4'-(3-chlorphenyl)-2'-(1-ethyl-propyl) spiro[3H-indol-3,3'-piperidin]-2,6'(1H)-dion,
chiralem (2'R, 3R, 4'S)-6-Chlor-4'-(3-chlorphenyl)-2'-(1-ethyl-propyl) spiro[3H-indol-3,3'-piperidin]-2,6'(1H)-dion,
racemischem (2'R, 3R, 4'S)-6-Chlor-4'-(3-chlorphenyl)-2'-(2,3-difluor-6-isopropoxy-phenyl)-1'-(3-morpholin-4-yl-propyl) spiro[3H-indol-3,3'-piperidin]-2,6'(1H)-dion,
chiralem (2'R, 3R, 4'S)-6-Chlor-4'-(3-chlorphenyl)-2'-(2,3-difluor-6-isopropoxy-phenyl)-1'-(3-morpholin-4-yl-propyl) spiro[3H-indol-3,3'-piperidin]-2,6'(1H)-dion,
racemischem (2'R, 3R, 4'S)-6-Chlor-4'-(3-chlorphenyl)-2'-(1-ethyliden-pentyl) spiro[3H-indol-3,3'-piperidin]-2,6'(1H)-dion,
racemischem (2'R, 3R, 4'S)-6-Chlor-4'-(3-chlorphenyl)-2'-(2,3-difluor-6-isopropoxy-phenyl)-6'-thioxo spiro[3H-indol-3,3'-piperidin]-2(1H)-on,
racemischem (2'R, 3R, 4'S)-[6-Chlor-4'-(3-chlorphenyl)-2'-(2,3-difluor-6-isopropoxy-phenyl)-2,3-dihydro-2-oxospiro[3H-indol-3,3'-piperidin]-6-ylen] hydrazin-carbonsäureethylester und
racemischem (2'R, 3R, 4'S)-6-Chlor-4'-(3-chlorphenyl)-2'-(cyclopent-1-enyl) spiro[3H-indol-3,3'-piperidin]-2,6'(1H)-dion.

19. Verbindung nach Anspruch 1 ausgewählt aus der Gruppe bestehend aus
racemischem (2'R, 3R, 4'S)-6-Chlor-4'-(3-chlorphenyl)-2'-cyclopentyl spiro[3H-indol-3,3'-piperidin]-2,6'(1H)-dion,
chiralem (2'R, 3R, 4'S)-6-Chlor-4'-(3-chlorphenyl)-2'-cyclopentyl spiro[3H-indot-3,3'-piperidin]-2,6'(1H)-dion,
racemischem (2'R, 3R, 4'S)-6-Chlor-4'-(3-chlorphenyl)-2'-(1-isopropyl-propenyl) spiro[3H-indol-3,3'-piperidin]-2,6'(1H)-dion,
chiralem (2'R, 3R, 4'S)-6-Chlor-4'-(3-chlorphenyl)-2'-(1-isopropyl-propenyl) spiro[3H-indol-3,3'-piperidin]-2,6'(1H)-dion,
racemischem (2'R, 3R, 4'S)-6-Chlor-4'-(3-chlorphenyl)-2'-(1-methylen-propyl) spiro[3H-indol-3,3'-piperidin]-2,6'(1H)-dion,
chiralem (2'R, 3R, 4'S)-6-Chlor-4'-(3-chlorphenyl)-2'-(1-methylen-propyl) spiro[3H-indol-3,3'-piperidin]-2,6'(1H)-dion,
racemischem (2'R, 3R, 4'S)-6-Chlor-4'-(3-chlorphenyl)-2'-(1-ethyl-propenyl)-1'- hydroxycarbonylmethyl-spiro[3H-indol-3,3'-piperidin]-2,6'(1H)-dion,
racemischem (2'R, 3R, 4'S)-6-Chlor-4'-(3-chlorphenyl)-2'-(1-ethyl-propenyl)-1'-[(2-hydroxy-1,1-dimethylethyl)aminocarbonyl-methyl] spiro[3H-indol-3,3'-piperidin]-2,6'(1H)-dion,
chiralem (2'R, 3R, 4'S) -6-Chlor-4'-(3-chlorphenyl)-2'-(1-ethyl-propenyl)-1'-[(2- Hydroxy-1,1-dimethylethy)aminocarbonyl-methyl] spiro[3H-indol-3,3'-piperidin]-2,6'(1H)-dion und
racemischem (2'R, 3R, 4'S)-6-Chlor-4'-(3-chlorphenyl)-2'-(1-ethyl-propenyl)-1'-(3- morpholin-4-yl-propyl) spiro[3H-indol-3,3'-piperidin]-2,6'(1H)-dion.

20. Verbindung nach Anspruch 1 ausgewählt aus der Gruppe bestehend aus
chiralem (2'R, 3R, 4'S)-6-Chlor-4'-(3-chlorphenyl)-2'-(1-ethyl-propenyl)-1'-(3-morpholin-4-yl-propyl) spiro[3H-indol-3,3'-piperidin]-2,6'(1H)-dion, racemischem (2'R, 3R, 4'S)-6-Chlor-4'-(3-chlorphenyl)-2'-(2-methyl-1-methylen-propyl) spiro[3H-indol-3,3'-piperidin]-2,6'(1H)-dion,
racemischem (2'R, 3R, 4'S)-6-Chlor-4'-(3-chlorphenyl)-2'-(2,2-dimethyl-1-methylen-propyl) spiro[3H-indol-3,3'-piperidin]-2,6'(1H)-dion,
racemischem (2'R, 3R, 4'S)-6-Chlor-4'-(3-chlorphenyl)-2'-(1-ethyl-propenyl)-1'-[(2- morpholin-4-yl-ethyl)aminocarbonyl-methyl] spiro[3H-indol-3,3'-piperidin]-2,6'(1H)-dion,
racemischem (2'R, 3R, 4'S)-6-Chlor-4'-(3-chlorphenyl)-2'-isopropenyl spiro[3H-indol-3,3'-piperidin]-2,6'(1H)-dion,
chiralem (2'R, 3R, 4'S)-6-Chlor-4'-(3-chlorphenyl)-2'-isopropenyl spiro[3H-indol-3,3'-piperidin]-2,6'(1H)-dion,
racemischem (2'R, 3R, 4'S)-6-Chlor-4'-(3-chlorphenyl)-2'-(1-methylen-butyl) spiro[3H-indol-3,3'-piperidin]-2,6'(1H)-dion,
chiralem (2'R, 3R, 4'S)-6-Chlor-4'-(3-chlorphenyl)-2'-(1-methylen-butyl) spiro[3H-indol-3,3'-piperidin]-2,6'(1H)-dion,
racemischem (2'R, 3R, 4'S)-6-Chlor-4'-(3-chlorphenyl)-2'-(3-methyl-1-methylen-butyl) spiro[3H-indol-3,3'-piperidin]-2,6'(1H)-dion und
chiralem (2'R, 3R, 4'S)-6-Chlor-4'-(3-chlorphenyl)-2'-(3-methyl-1-methylen-butyl) spiro[3H-indol-3,3'-piperidin]-2,6'(1H)-dion.

21. Verbindung nach Anspruch 1 ausgewählt aus der Gruppe bestehend aus
racemischem (2'R, 3R, 4'S)-6-Chlor-4'-(3-chlorphenyl)-2'-(1-methylen-butyl)-6'-thioxo spiro[3H-indol-3,3'-piperidin]-2(1H)-on,
racemischem (2'R, 3R, 4'S)-6-Chlor-4'-(3-chlorphenyl)-2'-(4-ethoxy-1,2-difluor-phenyl) spiro[3H-indol-3,3'-piperidin]-2,6'(1H)-dion,
chiralem (2'R, 3R, 4'S)-6-Chlor-4'-(3-chlorphenyl)-2'-(4-ethoxy-1,2-difluorphenyl)spiro[3H-indol-3,3'-piperidin]-2,6'(1H)-dion,
racemischem (2'R, 3R, 4'S)-6-Chlor-4'-(3-chlor-5-methoxycarbonyl-phenyl)-2'-(1-methylen-propyl) spiro[3H-indol-3,3'-piperidin]-2,6'(1H)-dion,
racemischem (2'R, 3R, 4'S)-6-Chlor-4'-(3-chlor-5-hydroxycarbonyl-phenyl)-2'-(1-methylen-propyl) spiro[3H-indol-3,3'-piperidin]-2,6'(1H)-dion,
racemischem (2'R, 3R, 4'S)-6-Chlor-4'-(3-chlor-5-fluorcarbonyl-phenyl)-2'-(1-methylen-propyl) spiro[3H-indol-3,3'-piperidin]-2,6'(1H)-dion,
racemischem (2'R, 3R, 4'S)-6-Chlor-4'-[3-chlor-5-(4-methansulfonyl-piperazin-1-carbonyl)-phenyl]-2'-(1-methylen-propyl) spiro[3H-indol-3,3'-piperidin]-2,6'(1H)-dion,
racemischem (2'R, 3R, 4'S)-6-Chlor-4'-(3-chlorphenyl)-2'-(1-ethyl-propenyl)-1'-methyl spiro[3H-indol-3,3'-piperidin]-2,6'(1H)-dion,
chiralem (2'R, 3R, 4'S)-6-Chlor-4'-(3-chlorphenyl)-2'-(1-ethyl-propenyl)-1'-methyl spiro[3H-indol-3,3'-piperidin]-2,6'(1H)-dion,
racemischem (2'R, 3R, 4'S)-6-Chlor-4'-(3-chlorphenyl)-2'-isopropyl spiro[3H-indol-3,3'-piperidin]-2,6'(1H)-dion und
chiralem (2'R, 3R, 4'S)-6-Chlor-4'-(3-chlorphenyl)-2'-isopropyl spiro[3H-indol-3,3'-piperidin]-2,6'(1H)-dion.

22. Verbindung nach Anspruch 1 ausgewählt aus der Gruppe bestehend aus
racemischem (2'R, 3R, 4'S)-6-Chlor-4'-(3-chlorphenyl)-2'-(1-methylen-propyl)-1'-methyl spiro[3H-indol-3,3'-piperidin]-2,6'(1H)-dion,
chiralem (2'R, 3R, 4'S)-6-Chlor-4'-(3-chlorphenyl)-2'-(1-methylen-propyl)-1'-methyl spiro[3H-indol-3,3'-piperidin]-2,6'(1H)-dion,
racemischem (2'R, 3R, 4'S)-2'-sec-Butyl-6-chlor-4'-(3-chlorphenyl) spiro[3H-indol-3,3'-piperidin]-2,6'(1H)-dion,
racemischem (2'R, 3R, 4'S)-6-Chlor-4'-(3-chlorphenyl)-2'-(1-hydroxymethyl-vinyl)- spiro[3H-indol-3,3'-piperidin]-2,6'(1H)-dion,
racemischem (2'R, 3R, 4'S)-6-Chlor-4'-(3-chlorphenyl)-2'-(1-methoxymethyl-vinyl) spiro[3H-indol-3,3'-piperidin]-2,6'(1H)-dion,
racemischem (2'R, 3R, 4'S)-6-Chlor-4'-(3-chlorphenyl)-2'-[2,3-difluor-6-(2-methoxy-ethoxy)-phenyl] spiro[3H-indol-3,3'-piperidin]-2,6'(1H)-dion,
chiralem (2'R, 3R, 4'S)-6-Chlor-4'-(3-chlorphenyl)-2'-[2,3-difluor-6-(2-methoxy-ethoxy)-phenyl] spiro[3H-indol-3,3'-piperidin]-2,6'(1H)-dion,
racemischem (2'R, 3R, 4'S)-6-Chlor-4'-(3-chlorphenyl)-2'-(1,2-dimethyl-propenyl) spiro[3H-indol-3,3'-piperidin]-2,6'(1H)-dion,
racemischem (2'R, 3R, 4'S)-6-Chlor-4'-(3-chlorphenyl)-2'-propionyl spiro[3H-indol-3,3'-piperidin]-2,6'(1H)-dion und
racemischem (2'R, 3R, 4'S)-6-Chlor-4'-(3-chlorphenyl)-2'-(2,3-difluor-6-propoxy-phenyl) spiro[3H-indol-3,3'-piperidin]-2,6'(1H)-dion.

23. Verbindung nach Anspruch 1 ausgewählt aus der Gruppe bestehend aus
chiralem (2'R, 3R, 4'S)-6-Chlor-4'-(3-chlorphenyl)-2'-(2,3-difluor-6-propoxy-phenyl) spiro[3H-indol-3,3'-piperidin]-2,6'(1H)-dion,
racemischem (2'R, 3R, 4'S)-6-Chlor-4'-(3-chlorphenyl)-2'-(1-cyclopropyl-vinyl) spiro[3H-indol-3,3'-piperidin]-2,6'(1H)-dion,
racemischem (2'R, 3R, 4'S)-6-Chlor-4'-(3-chlorphenyl)-1'-hydroxycarbonylmethyl-2'-(1-methylen-propyl) spiro[3H-indol-3,3'-piperidin]-2,6'(1H)-dion,
racemischem (2'R, 3R, 4'S)-6-Chlor-4'-(3-chlorphenyl)-2'-(1-methylen-propyl)-1'-[(1-methansulfonyl-piperidin-4-yl)aminocarbonyl-methyl] spiro[3H-indol-3,3'-piperidin]-2,6'(1H)-dion,
racemischem (2'R, 3R, 4'S)-6-Chlor-4'-(3-chlorphenyl)-2'-(1-methylen-propyl)-1'- (aminocarbonyl-methyl) spiro[3H-indol-3,3'-piperidin]-2,6'(1H)-dion,
chiralem (2'R, 3R, 4'S)-6-Chlor-4'-(3-chlorphenyl)-2'-(1-methylen-propyl)-1'-(aminocarbonyl-methyl) spiro[3H-indol-3,3'-piperidin]-2,6'(1H)-dion,
racemischem (2'R, 3R, 4'S)-6-Chlor-4'-(3-chlorphenyl)-2'-(1-ethyl-2-methyl-propenyl) spiro[3H-indol-3,3'-piperidin]-2,6'(1H)-dion,
racemischem (2'R, 3R, 4'S)-6-Chlor-4'-(3-chlorphenyl)-2'-[2,3-difluor-6-(2-hydroxy- ethoxy)-phenyl] spiro[3H-indol-3,3'-piperidin]-2,6'(1H)-dion,
chiralem (2'R, 3R, 4'S)-6-Chlor-4'-(3-chlorphenyl)-2'-[2,3-difluor-6-(2-hydroxy-ethoxy)-phenyl] spiro[3H-indol-3,3'-piperidin]-2,6'(1H)-dion und
racemischem (2'R, 3R, 4'S)-6-Chlor-4'-(3-chlorphenyl)-2'-(2,3-difluor-6-hydroxy-phenyl) spiro[3H-indol-3,3'-piperidin]-2,6'(1H)-dion.

24. Verbindung nach Anspruch 1 ausgewählt aus der Gruppe bestehend aus
racemischem (2'R, 3R, 4'S)-6-Chlor-4'-(3-chlorphenyl)-2'-(1-ethyl-1-hydroxypropyl) spiro[3H-indol-3,3'-piperidin]-2,6'(1H)-dion,
racemischem (2'R, 3R, 4'S)-6-Chlor-4'-(3-chlorphenyl)-2'-(1-ethyl-3-methyl-oxiranyl) spiro[3H-indol-3,3'-piperidin]-2,6'(1H)-dion,
racemischem (2'R, 3R, 4'S)-6-Chlor-4'-(3-chlorphenyl)-2'-(1-fluor-2-methyl-propenyl) spiro[3H-indol-3,3'-piperidin]-2,6'(1H)-dion,
racemischem (2'R, 3R, 4'S)-6-Chlor-4'-(3-chlorphenyl)-2'-isobutyryl spiro[3H-indol-3,3'-piperidin]-2,6'(1H)-dion,
racemischem (2'R, 3R, 4'S)-6-Chlor-4'-(3-chlorphenyl)-2'-isopropenyl-1'-hydroxycarbonylmethyl spiro[3H-indol-3,3'-piperidin]-2,6'(1H)-dion,
racemischem (2'R, 3R, 4'S)-1'-(Aminocarbonyl-methyl)-6-chlor-4'-(3-chlorphenyl)-2'- isopropenyl spiro[3H-indol-3,3'-piperidin]-2,6'(1H)-dion,
racemischem (2'R, 3R, 4'S)-6-Chlor-4'-(3-chlorphenyl)-1'-(cyclopropylaminocarbonyl-methyl)-2'-isopropenyl spiro[3H-indol-3,3'-piperidin]-2,6'(1H)-dion,
racemischem (2'R, 3R, 4'S)-6-Chlor-4'-(3-chlorphenyl)-2'-isopropenyl-1'-[(1-methyl- piperidin-4-yl)aminocarbonyl-methyl] spiro[3H-indol-3,3' piperidin]-2,6'(1H)-dion,
racemischem (2'R, 3R, 4'S)-6-Chlor-4'-(3-chlorphenyl)-2'-isopropenyl-1'-[(1-methylsulfonyl-piperidin-4-yl)aminocarbonyl-methyl] spiro[3H-indol-3,3' piperidin]-2,6'(1H)-dion und
racemischem (2'R, 3R, 4'S)-6-Chlor-4'-(3-chlorphenyl)-1'-[(2-hydroxyethyl)aminocarbonyl-methyl]-2'-isopropenyl-spiro[3H-indol-3,3'-piperidin]-2,6'(1H)-dion.

25. Verbindung nach Anspruch 1 ausgewählt aus der Gruppe bestehend aus
racemischem (2'R, 3R, 4'S)-6-Chlor-4'-(3-chlorphenyl)-1'-[(2-hydroxy-1,1-dimethyl- ethyl)aminocarbonyl-methyl]-2'-isopropenyl-spiro[3H-indol-3,3'-piperidin]-2,6'(1H)-dion,
racemischem (2'R, 3R, 4'R)-6-Chlor-4'-(3-chlor-phenyl)-4'-cyano-2'-(1-methylen-propyl) spiro[3H-indol-3,3'-piperidin]-2,6'(1H)-dion,
racemischem (2'R, 3R, 4'S)-1'-[3-(4-Acetylamino-piperidin-1-yl)-propyl]-6-chlor-4'-(3-chlorphenyl)-2'-isopropenyl spiro[3H-indol-3,3' piperidin]-2,6'(1H)-dion,
racemischem (2'R, 3R, 4'S)-4'-(3-Chlor-phenyl)-6'-cyano-2'-(1-methylen-propyl) spiro[3H-indol-3,3'-piperidin]-2,6'(1H)-dion,
racemischem (2'R, 3R, 4'S)-4'-(3-Chlor-phenyl)-6-cyano-2'-(5-fluor-2-methylphenyl) spiro[3H-indol-3,3'-piperidin]-2,6'(1H)-dion,
racemischem (2'R, 3R, 4'S)-1'-[3-(4-Acetyl-piperazin-1-yl)-propyl]-6-chlor-4'-(3-chlorphenyl)-2'-isopropenyl spiro[3H-indol-3,3'-piperidin]-2,6'(1H)-dion,
racemischem (2'R, 3R, 4'S)-6-Chlor-4'-(3-chlorphenyl)-2'-isopropenyl-1'-(3-piperidin-1-yl-propyl) spiro[3H-indol-3,3' piperidin]-2,6'(1H)-dion,
racemischem (2'R, 3R, 4'S)-2'-[2-(2-Acetoxy-ethoxy)-5-methyl-phenyl]-6-chlor-4'-(3-chlorphenyl) spiro[3H-indol-3,3'-piperidin]-2,6'(1H)-dion,
racemischem (2'R, 3R, 4'S)-6-Chlor-4'-(3-chlorphenyl)-2'-[6-(2-hydroxy-ethoxy)-3-methyl-phenyl] spiro[3H-indol-3,3'-piperidin]-2,6'(1H)-dion und
racemischem (2'R, 3R, 4'S)-6-Chlor-4'-(3-chlorphenyl)-2'-[5-cyclopropyl-2-(2-hydroxy-ethoxy)-phenyl] spiro[3H-indol-3,3'-piperidin]-2,6'(1H)-dion.

26. Verbindung nach Anspruch 1 ausgewählt aus der Gruppe bestehend aus
racemischem (2'R, 3R, 4'S)-6-Chlor-2'-[5-chlor-2-(2-hydroxy-ethoxy)-phenyl]-4'-(3-chlorphenyl) spiro[3H-indol-3,3'-piperidin]-2,6'(1H)-dion,
racemischem (2'R, 3R, 4'S)-6-Chlor-2'-[3-chlor-2-fluor-6-(2-hydroxy-ethoxy)-phenyl]-4'-(3-chlorphenyl) spiro[3H-indol-3,3'-piperidin]-2,6'(1H)-dion,
racemischem (2'R, 3R, 4'S)-6-Chlor-4'-(3,5-difluor-phenyl)-2'-(1-methylen-propyl) spiro[3H-indol-3,3'-piperidin]-2,6'(1H)-dion,
racemischem (2'R, 3R, 4'S)-6-Chlor-4'-(3-cyano-phenyl)-2'-(1-methylen-propyl) spiro[3H-indol-3,3'-piperidin]-2,6'(1H)-dion,
racemischem (2'R, 3R, 4'S)-4'-(3-Brom-phenyl)-6-chlor-2'-(1-methylen-propyl) spiro[3H-indol-3,3'-piperidin]-2,6'(1H)-dion,
racemischem (2'R, 3R, 4'S)-6-Chlor-4'-(3-methoxy-phenyl)-2'-(1-methylen-propyl) spiro[3H-indol-3,3'-piperidin]-2,6'(1H)-dion,
racemischem (2'R, 3R, 4'S)-6-Chlor-4'-(5-fluor-2-methyl-phenyl)-2'-(1-methylen-propyl) spiro[3H-indol-3,3'-piperidin]-2,6'(1H)-dion,
racemischem (2'R, 3R, 4'S)-6-Chlor-4'-(3-fluor-phenyl)-2'-(1-methylen-propyl) spiro[3H-indol-3,3'-piperidin]-2,6'(1H)-dion,
racemischem (2'R, 3R, 4'S)-6-Chlor-2'-(1-methylen-propyl)-4'-m-tolyl spiro[3H-indol-3,3'-piperidin]-2,6'(1H)-dion und
racemischem (2'R, 3R, 4'S)-6-Chlor-2'-(1-methylen-propyl)-4'-o-tolyl spiro[3H-indol-3,3'-piperidin]-2,6'(1H)-dion.

27. Verbindung nach Anspruch 1 ausgewählt aus der Gruppe bestehend aus
racemischem (2'R, 3R, 4'S)-6-Chlor-2'-(1-methylen-propyl)-4'-thiophen-3-yl spiro[3H-indol-3,3'-piperidin]-2,6'(1H)-dion,
racemischem (2'R, 3R, 4'S)-6-Chlor-4'-(3,5-dichlor-phenyl)-2'-(1-methylen-propyl) spiro[3H-indol-3,3'-piperidin]-2,6'(1H)-dion,
racemischem (2'R, 3R, 4'S)-6-Chlor-4'-(5-chlor-2-trifluormethyl-phenyl)-2'-(1-methylen-propyl) spiro[3H-indol-3,3'-piperidin]-2,6'(1H)-dion,
racemischem (2'R, 3R, 4'S)-4'-(3-Brom-phenyl)-6-chlor-2'-isopropenyl spiro[3H-indol-3,3'-piperidin]-2,6'(1H)-dion,
racemischem (2'S, 3S, 4'R)-6-Chlor-2'-(3-chlorphenyl)-4'-(5-fluor-2-methylphenyl)- spiro[3H-indol-3,3'-piperidin]-2,6'(1H)-dion,
racemischem (2'S, 3S, 4'R)-6-Chlor-2'-(3-chlorphenyl)-4'-(5-fluor-2-methylphenyl)-1'-hydroxycarbonylmethyl-spiro[3H-indol-3,3'-piperidin]-2,6'(1H)-dion,
racemischem (2'S, 3S, 4'R)-6-Chlor-2'-(3-chlorphenyl)-4'-(5-fluor-2-methylphenyl)-1'-(methylamino-carbonyl-methyl) spiro[3H-indol-3,3'-piperidin]-2,6'(1H)-dion,
racemischem (2'S, 3S, 4'R)-6-Chlor-2'-(3-chlorphenyl)-1'-(dimethylaminocarbonyl-methyl) -4'-(5-fluor-2-methyl-phenyl)- spiro[3H-indol-3,3'-piperidin]-2,6'(1H)-dion,
racemischem (2'S, 3S, 4'R)-1'-[(4-Aminocarbonyl-piperidin-1-yl)carbonyl-methyl]-6-chlor-2'-(3-chlorphenyl)-4'-(5-fluor-2-methyl-phenyl) spiro[3H-indol-3,3'-piperidin]-2,6'(1H)-dion und
racemischem (2'S, 3S, 4'R)-1'-[(3-Aminocarbonyl-piperidin-1-yl)carbonyl-methyl]-6-chlor-2'-(3-chlorphenyl)-4'-(5-fluor-2-methyl-phenyl)- spiro[3H-indol-3,3'-piperidin]-2,6'(1H)-dion.

28. Verbindung nach Anspruch 1 ausgewählt aus der Gruppe bestehend aus
racemischem (2'S, 3S, 4'R)-1'-(Aminocarbonyl-methyl)-6-chlor-2'-(3-chlorphenyl)-4'-(5-fluor-2-methyl-phenyl) spiro[3H-indol-3,3'-piperidin]-2,6'(1H)-dion,
racemischem (2'S, 3S, 4'R)-6-Chlor-2'-(3-chlorphenyl)-1'-(dimethylaminopropyl)-4'-(5-fluor-2-methyl-phenyl)- spiro[3H-indol-3,3'-piperidin]-2,6'(1H)-dion,
racemischem (2'S, 3S, 4'R)-6-Chlor-4'-(3-chlorphenyl)-2'-(5-fluor-2-methylphenyl)-1'-[(1-methansulfonyl-piperidin-4-yl)carbonylamino-ethyl] spiro[3H-indol-3,3'-piperidin]-2,6'(1H)-dion,
racemischem (2'R, 3R, 4'S)-6-Chlor-4'-[2-(4-chlor-thiophenyl)]-2'-(5-fluor-2-methylphenyl)-spiro[3H-indol-3,3'-piperidin]-2,6'(1H)-dion,
racemischem (2'R, 3R, 4'S)-6-Chlor-4'-[2-(5-chlor-thiophenyl)]-2'-(5-fluor-2-methylphenyl)-spiro[3H-indol-3,3'-piperidin]-2,6'(1H)-dion,
racemischem (2'R, 3R, 4'S)-6-Chlor-4'-(2,2-dimethylpropyl)-2'-(5-fluor-2-methylphenyl)-spiro[3H-indol-3,3'-piperidin]-2,6'(1H)-dion,
racemischem (2'R, 3R, 4'S)-6-Brom-4'-(3-chlorphenyl)-2'-(2-methylphenyl) spiro[3H-indol-3,3'-piperidin]-2,6'(1H)-dion,
racemischem (2'R, 3R, 4'S)-6-Brom-4'-(3-chlorphenyl)-2'-(5-fluor-2-methylphenyl) spiro[3H-indol-3,3'-piperidin]-2,6'(1H)-dion,
chiralem (2'R, 3R, 4'S)-6-Chlor-4'-(3-chlorphenyl)-2'-(5-fluor-2-methylphenyl) spiro[3H-indol-3,3'-piperidin]-2,6'(1H)-dion und
racemischem (2'R, 3R, 4'S)-6-Brom-4'-(3-chlorphenyl)-2'-(2,5-dichlorphenyl) spiro[3H-indol-3,3'-piperidin]-2,6'(1H)-dion.

29. Verbindung nach Anspruch 1 ausgewählt aus der Gruppe bestehend aus
racemischem (2'R, 3R, 4'S)-6-Brom-4'-(3-chlorphenyl)-2'-(5-chlor-2-methylphenyl) spiro[3H-indol-3,3'-piperidin]-2,6'(1H)-dion,
racemischem (2'R, 3R, 4'S)-6-Brom-4'-(3-chlorphenyl)-2'-(1-methylen-propyl)- spiro[3H-indol-3,3'-piperidin]-2,6'(1H)-dion,
chiralem (2'R, 3R, 4'S)-6-Brom-4'-(3-chlorphenyl)-2'-(1-methylen-propyl)-spiro[3H-indol-3,3'-piperidin]-2,6'(1H)-dion,
chiralem (2R, 3R, 4'S)-6-Brom-4'-(3-chlorphenyl)-2'-(1-ethyl-cyclopropyl) spiro[3H-indol-3,3'-piperidin]-2,6'(1H)-dion,
racemischem (2'R,3R,4'S)-6-Cyclopropyl-4'-(3-chlorphenyl)-2'-(1-ethyl-cyclopropyl)- spiro[3H-indol-3,3'-piperidin]-2,6'(1H)-dion,
racemischem (2'R, 3R, 4'S) 2'-[2-(4-Aminocarbonyl-piperidin-1-yl)methyl-5-fluor-phenyl)-6-chlor-4'-(3-chlorphenyl)-spiro[3H-indol-3,3'-piperidin]-2,6'(1H)-dion,
racemischem (2'R, 3R, 4'S)-6-Chlor-4'-(3-chlorphenyl)-2'-[5-fluor-2-(4-methansulfonyl-piperazin-1-yl)methyl-phenyl]-spiro[3H-indol-3,3'-piperidin]-2,6'(1H)-dion,
racemischem (2'R, 3R, 4'S)-6-Chlor-4'-(3-chlorphenyl)-2'-{5-fluor-2-[(1-methansulfonyl-piperidin-4-yl)carbonylamino-methyl]-phenyl}-spiro[3H-indol-3,3'-piperidin]-2,6'(1H)-dion,
chiralem (2'R, 3R, 4'S)-4'-(3-Chlorphenyl)-6-fluor-2'-(5-fluor-2-methylphenyl) spiro[3H-indol-3,3'-piperidin]-2,6'(1H)-dion und
racemischem (2'R, 3R, 4'S)-4'-(3-Chlorphenyl)-2'-(5-fluor-2-methylphenyl)-6-methoxy spiro[3H-indol-3,3'-piperidin]-2,6'(1H)-dion.

30. Verbindung nach Anspruch 1 ausgewählt aus der Gruppe bestehend aus
racemischem (2'R, 3R, 4'S)-4'-(3-Chlorphenyl)-2'-(5-fluor-2-methylphenyl) spiro[3H-indol-3,3'-piperidin]-2,6'(1H)-dion,
racemischem (2'R, 3R, 4'S)-4'-(3-Chlorphenyl)-5-fluor-2'-(5-fluor-2-methylphenyl) spiro[3H-indol-3,3'-piperidin]-2,6'(1H)-dion,
chiralem (2'R, 3R, 4'S)-6-Chlor-4'-(3-chlor-phenyl)-2'-(1-methyl-cyclopropyl) spiro[3H-indol-3,3'-piperidin]-2,6'(1H)-dion,
racemischem (2'R, 3R, 4'S)-6-Brom-4'-(3-chlorphenyl)-2'-isopropenyl spiro[3H-indol-3,3'-piperidin]-2,6'(1H)-dion,
chiralem (2'R, 3R, 4'S)-6-Brom-4'-(3-chlor-phenyl)-2'-(1-methyl-cyclopropyl) spiro[3H-indol-3,3'-piperidin]-2,6'(1H)-dion,
racemischem (2'R, 3R, 4'S)-4'-(3-Chlorphenyl)-6-ethinyl-2'-isopropenyl spiro[3H-indol-3,3'-piperidin]-2,6'(1H)-dion,
chiralem (2'R, 3'R, 4'S)-6-Chlor-4'-(3-chlorphenyl)-2'-(5-fluor-2-methylphenyl)-1'-[(1-methylsulphonyl-4-piperidinyl)aminocarbonyl-methyl] spiro[3H-indol-3,3'-piperidin]-2,6'(1H)-dion,
chiralem (2'R,3'R,4'S)-6-Chlor-4'-(3-chlorphenyl)-2'-(5-fluor-2-methylphenyl)-1'-{[4-(1,1-dioxido-2-isothiazolidinyl)ethyl]piperazinyl-carbonyl-methyl} spiro[3H-indol-3,3'-piperidin]-2,6'(1H)-dion,
chiralem (2'R,3'R,4'S)-6-Chlor-4'-(3-chlorphenyl)-2'-(5-fluor-2-methylphenyl)-1'-{[3-(methylsulphonyl)propyl]piperazinyl-carbonyl-methyl}spiro[3H-indol-3,3'-piperidin]-2,6'(1H)-dion und
racemischem (2'R, 3R, 4'S)-2'-(2-Brom-5-fluorphenyl) 6-chlor-4'-(3-chlorphenyl) spiro[3H-indol-3,3'-piperidin]-2,6'(1H)-dion.

31. Verbindung nach Anspruch 1 ausgewählt aus der Gruppe bestehend aus
racemischem (2'R, 3R, 4'S)-2'-6-Chlor-4'-(3-chlorphenyl)-(2-ethinyl-5-fluorphenyl) spiro[3H-indol-3,3'-piperidin]-2,6'(1H)-dion,
racemischem (2'R, 3R, 4'S)-2'-6-Chlor-4'-(3-chlorphenyl)-{5-fluor-2-[3-(methansulfonyl-methyl-amino)-prop-1-inyl-phenyl] spiro[3H-indol-3,3'-piperidin]-2,6'(1H)-dion,
racemischem (2'R, 3R, 4'S)-2'-[5-Brom-2-(2-hydroxy-ethoxy)-phenyl]-6-chlor-4'-(3-chlorphenyl) spiro[3H-indol-3,3'-piperidin]-2,6'(1H)-dion,
racemischem (2'R, 3R, 4'S)-6-Chlor-4'-(3-chlorphenyl)-2'-[5-ethinyl-2-(2-hydroxy-ethoxy)-phenyl] spiro[3H-indol-3,3'-piperidin]-2,6'(1H)-dion,
chiralem (2'R, 3R, 4'S)-6-Chlor-4'-(3-chlorphenyl)-2'-[5-ethinyl-2-(2-hydroxy-ethoxy)-phenyl] spiro[3H-indol-3,3'-piperidin]-2,6'(1H)-dion,
racemischem (2'R, 3R, 4'S)-2'-[3-Brom-2-fluor-6-(2-hydroxy-ethoxy)-phenyl]-6-chlor-4'-(3-chlorphenyl) spiro[3H-indol-3,3'-piperidin]-2,6'(1H)-dion,
racemischem (2'R, 3R, 4'S)-6-Chlor-4'-(3-chlorphenyl)-2'-[2-fluor-6-(2-hydroxy-ethoxy)-3-trimethylsilanyleth inyl-phenyl] spiro[3H-indol-3,3'-piperidin]-2,6'(1H)-dion,
racemischem (2'R, 3R, 4'S)-6-Chlor-4'-(3-chlorphenyl)-2'-[3-ethinyl-2-fluor-6-(2-hydroxy-ethoxy)-phenyl] spiro[3H-indol-3,3'-piperidin]-2,6'(1H)-dion und
racemischem (2'R, 3R, 4'S)-6-Chlor-4'-(3-chlorphenyl)-2'-[5-ethyl-2-(2-hydroxy-ethoxy)-phenyl] spiro[3H-indol-3,3'-piperidin]-2,6'(1H)-dion.

32. Pharmazeutische Formulierung, umfassend eine Verbindung der Formel 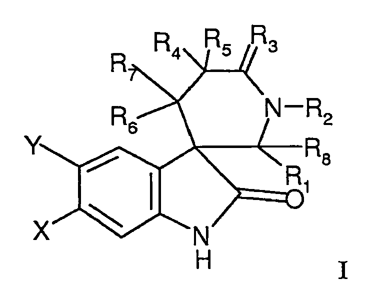 worin
X ausgewählt ist aus der Gruppe bestehend aus Wasserstoff, Halogen, Cyano, Nitro, Ethinyl, Cyclopropyl, Methyl, Ethyl, Isopropyl, Methoxy und Vinyl;
Y Wasserstoff oder Fluor ist;
R₄ und R₅ Wasserstoff oder Niederalkyl mit von 1 bis 8 Kohlenstoffatomen sind;
einer von R₁ und R₈ ausgewählt ist aus der Gruppe bestehend aus Niederalkyl mit von 1 bis 8 Kohlenstoffatomen, substituiertem Niederalkyl mit von 1 bis 8 Kohlenstoffatomen, Niederalkenyl mit von 2 bis 8 Kohlenstoffatomen, substituiertem Niederalkenyl mit von 2 bis 8 Kohlenstoffatomen, Aryl, substituiertem Aryl, Heteroaryl, welches Heteroaryl ein aromatisches heterocyclisches Ringsystem ist, das bis zu zwei Ringe enthält, substituiertem Heteroaryl, welches Heteroaryl ein aromatisches heterocyclisches Ringsystem ist, das bis zu zwei Ringe enthält, Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, substituiertem Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, Cycloalkyl, substituiertem Cycloalkyl, Cycloalkenyl und substituiertem Cycloalkenyl und der andere Wasserstoff ist;
einer von R₆ und R₇ ausgewählt ist aus der Gruppe bestehend aus Niederalkyl mit von 1 bis 8 Kohlenstoffatomen, substituiertem Niederalkyl mit von 1 bis 8 Kohlenstoffatomen, Niederalkenyl mit von 2 bis 8 Kohlenstoffatomen, substituiertem Niederalkenyl mit von 2 bis 8 Kohlenstoffatomen, Aryl, substituiertem Aryl, Heteroaryl, welches Heteroaryl ein aromatisches heterocyclisches Ringsystem ist, das bis zu zwei Ringe enthält, substituiertem Heteroaryl, welches Heteroaryl ein aromatisches heterocyclisches Ringsystem ist, das bis zu zwei Ringe enthält, Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, substituiertem Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, Cycloalkyl, substituiertem Cycloalkyl, Cycloalkenyl und substituiertem Cycloalkenyl und der andere Wasserstoff, Cyano oder Niederalkyl mit von 1 bis 8 Kohlenstoffatomen ist;
R₂ ausgewählt ist aus der Gruppe bestehend aus Wasserstoff, Niederalkyl mit von 1 bis 8 Kohlenstoffatomen und substituiertem Niederalkyl mit von 1 bis 8 Kohlenstoffatomen;
R₃ ausgewählt ist aus der Gruppe bestehend aus Sauerstoff, Schwefel und NNH(C=O)OR₉;
R₉ Niederalkyl mit von 1 bis 8 Kohlenstoffatomen oder substituiertes Niederalkyl mit von 1 bis 8 Kohlenstoffatomen ist;
und die pharmazeutisch akzeptablen Salze und Ester davon zusammen mit einem pharmazeutisch akzeptablen Träger oder Hilsstoff.

33. Pharmazeutische Formulierung nach Anspruch 32 zur Behandlung von Krebs, bevorzugt festen Tumoren, stärker bevorzugt Brust-, Colon-, Lungen- und Prostatatumoren.

34. Verbindungen nach Ansprüchen 1 bis 31 zur Verwendung als Arzneimittel.

35. Verbindungen nach Ansprüchen 1 bis 31 zur Verwendung als Arzneimittel zur Behandlung von Krebs, bevorzugt festen Tumoren, stärker bevorzugt Brust-, Colon-, Lungen- und Prostatatumoren.

36. Verwendung einer Verbindung nach Ansprüchen 1 bis 31 zur Herstellung von Arzneimitteln zur Behandlung von Krebs, bevorzugt festen Tumoren, stärker bevorzugt Brust-, Colon-, Lungen- und Prostatatumoren.

37. Verfahren zur Herstellung einer Verbindung der Formel 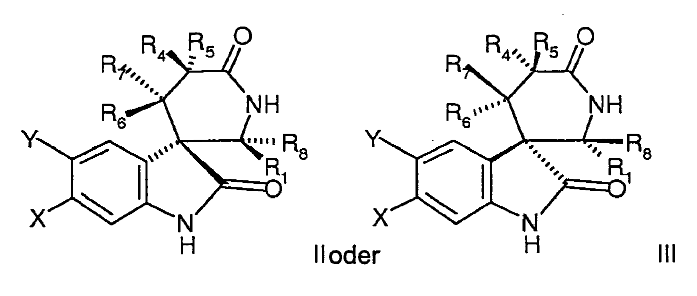 worin
X ausgewählt ist aus der Gruppe bestehend aus Wasserstoff, Halogen, Cyano, Nitro, Cyclopropyl, Methyl, Ethyl und Isopropyl;
Y Wasserstoff oder Fluor ist;
R₁ Wasserstoff ist;
R₄ und R₅ Wasserstoff oder Niederalkyl mit von 1 bis 8 Kohlenstoffatomen sind;
R₈ ausgewählt ist aus der Gruppe bestehend aus Niederalkyl mit von 1 bis 8 Kohlenstoffatomen, substituiertem Niederalkyl mit von 1 bis 8 Kohlenstoffatomen, Niederalkenyl mit von 2 bis 8 Kohlenstoffatomen, substituiertem Niederalkenyl mit von 2 bis 8 Kohlenstoffatomen, Aryl, substituiertem Aryl, Heteroaryl, welches Heteroaryl ein aromatisches heterocyclisches Ringsystem ist, das bis zu zwei Ringe enthält, substituiertem Heteroaryl, welches Heteroaryl ein aromatisches heterocyclisches Ringsystem ist, das bis zu zwei Ringe enthält, Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, substituiertem Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, Cycloalkyl, substituiertem Cycloalkyl, Cycloalkenyl und substituiertem Cycloalkenyl;
einer von R₆ und R₇ ausgewählt sind aus der Gruppe bestehend aus Niederalkyl mit von 1 bis 8 Kohlenstoffatomen, substituiertem Niederalkyl mit von 1 bis 8 Kohlenstoffatomen, Niederalkenyl mit von 2 bis 8 Kohlenstoffatomen, substituiertem Niederalkenyl mit von 2 bis 8 Kohlenstoffatomen, Aryl, substituiertem Aryl, Heteroaryl, welches Heteroaryl ein aromatisches heterocyclisches Ringsystem ist, das bis zu zwei Ringe enthält, substituiertem Heteroaryl, welches Heteroaryl ein aromatisches heterocyclisches Ringsystem ist, das bis zu zwei Ringe enthält, Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, substituiertem Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, Cycloalkyl, substituiertem Cycloalkyl, Cycloalkenyl und substituiertem Cycloalkenyl und der andere Wasserstoff, Cyano oder Niederalkyl mit von 1 bis 8 Kohlenstoffatomen ist;
welches Umsetzen einer Verbindung der Formel 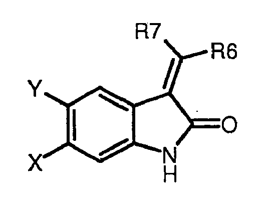 mit einer Verbindung der Formel 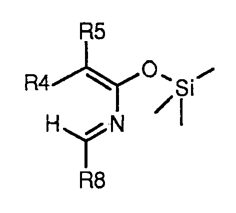 bei etwa 110-160°C und unter wasserfreien Bedingungen umfasst, um eine Verbindung der Formel II oder III herzustellen.

38. Verfahren zur Herstellung einer Verbindung der Formel 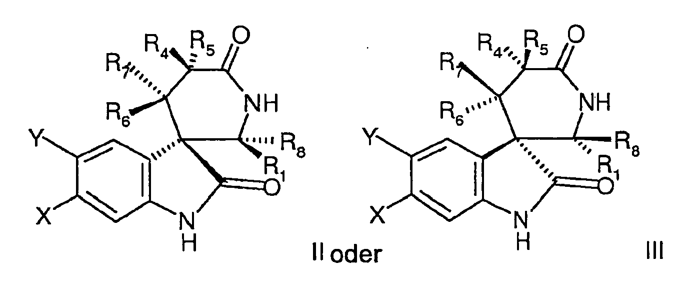 worin
X ausgewählt ist aus der Gruppe bestehend aus Wasserstoff, Halogen, Cyano, Nitro, Cyclopropyl, Methyl, Ethyl und Isopropyl;
Y Wasserstoff oder Fluor ist;
R₁ Wasserstoff ist;
R₄ und R₅ Wasserstoff oder Niederalkyl mit von 1 bis 8 Kohlenstoffatomen sind;
R₈ ausgewählt ist aus der Gruppe bestehend aus Niederalkyl mit von 1 bis 8 Kohlenstoffatomen, substituiertem Niederalkyl mit von 1 bis 8 Kohlenstoffatomen, Niederalkenyl mit von 2 bis 8 Kohlenstoffatomen, substituiertem Niederalkenyl mit von 2 bis 8 Kohlenstoffatomen, Aryl, substituiertem Aryl, Heteroaryl, welches Heteroaryl ein aromatisches heterocyclisches Ringsystem ist, das bis zu zwei Ringe enthält, substituiertem Heteroaryl, welches Heteroaryl ein aromatisches heterocyclisches Ringsystem ist, das bis zu zwei Ringe enthält, Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, substituiertem Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, Cycloalkyl, substituiertem Cycloalkyl, Cycloalkenyl und substituiertem Cycloalkenyl; einer von R₆ und R₇ ausgewählt sind aus der Gruppe bestehend aus Niederalkyl mit von 1 bis 8 Kohlenstoffatomen, substituiertem Niederalkyl mit von 1 bis 8 Kohlenstoffatomen, Niederalkenyl mit von 2 bis 8 Kohlenstoffatomen, substituiertem Niederalkenyl mit von 2 bis 8 Kohlenstoffatomen, Aryl, substituiertem Aryl, Heteroaryl, welches Heteroaryl ein aromatisches heterocyclisches Ringsystem ist, das bis zu zwei Ringe enthält, substituiertem Heteroaryl, welches Heteroaryl ein aromatisches heterocyclisches Ringsystem ist, das bis zu zwei Ringe enthält, Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, substituiertem Heterocyclus, welcher Heterocyclus ein 5- bis 8-gliedriger, mono- oder bicyclischer, aromatischer oder nicht-aromatischer Kohlenwasserstoff ist, worin 1 bis 3 Kohlenstoffatome durch ein Heteroatom ausgewählt aus Stickstoff-, Sauerstoff- oder Schwefelatom ersetzt sind, Cycloalkyl, substituiertem Cycloalkyl, Cycloalkenyl und substituiertem Cycloalkenyl und der andere Wasserstoff, Cyano oder Niederalkyl mit von 1 bis 8 Kohlenstoffatomen ist;
welches Umsetzen einer Verbindung der Formel 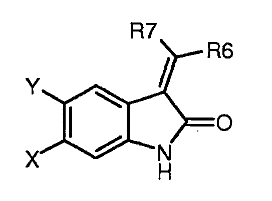 mit einer geeigneten Schutzgruppe umfasst, um eine Verbindung der Formel 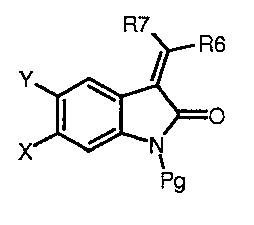 bereitzustellen,
die danach mit einer Verbindung der Formel 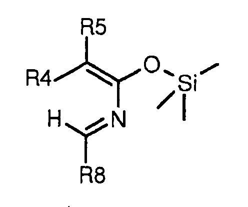 bei etwa 110-160°C unter wasserfreien Bedingungen umgesetzt wird und danach wird das erhaltene Produkt entschützt, um eine Verbindung der Formel II und III herzustellen.

## Revendications

1. Composé de formule 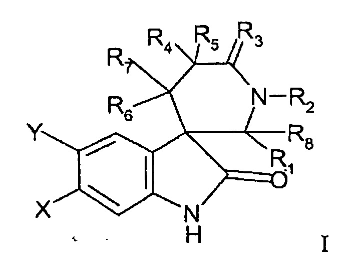 où
X est choisi dans le groupe consistant en l'hydrogène, un halogène, cyano, nitro, éthynyle, cyclopropyle, méthyle, éthyle, isopropyle, méthoxy et vinyle ;
Y est l'hydrogène ou le fluor ;
R₄ et R₅ sont l'hydrogène ou alkyle inférieur ayant de 1 à 8 atomes de carbone ; l'un de R₁ et R₈ est choisi dans le groupe consistant en alkyle inférieur ayant de 1 à 8 atomes de carbone, alkyle inférieur substitué ayant de 1 à 8 atomes de carbone, alcényle inférieur ayant de 2 à 8 atomes de carbone, alcényle inférieur substitué ayant de 2 à 8 atomes de carbone, aryle, aryle substitué, hétéroaryle, lequel hétéroaryle est un système cyclique hétérocyclique aromatique contenant jusqu'à deux cycles, hétéroaryle substitué, lequel hétéroaryle est un système cyclique hétérocyclique aromatique contenant jusqu'à deux cycles, hétérocycle, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, hétérocycle substitué, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, cycloalkyle, cycloalkyle substitué, cycloalcényle, et cycloalcényle substitué et l'autre est l'hydrogène ;
l'un de R₆ et R₇ est choisi dans le groupe consistant en alkyle inférieur ayant de 1 à 8 atomes de carbone, alkyle inférieur substitué ayant de 1 à 8 atomes de carbone, alcényle inférieur ayant de 2 à 8 atomes de carbone, alcényle inférieur substitué ayant de 2 à 8 atomes de carbone, aryle, aryle substitué, hétéroaryle, lequel hétéroaryle est un système cyclique hétérocyclique aromatique contenant jusqu'à deux cycles, hétéroaryle substitué, lequel hétéroaryle est un système cyclique hétérocyclique aromatique contenant jusqu'à deux cycles, hétérocycle, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, hétérocycle substitué, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, cycloalkyle, cycloalkyle substitué, cycloalcényle, et cycloalcényle substitué et l'autre est l'hydrogène, cyano ou alkyle inférieur ayant de 1 à 8 atomes de carbone ;
R₂ est choisi dans le groupe consistant en l'hydrogène, alkyle inférieur ayant de 1 à 8 atomes de carbone et alkyle inférieur substitué ayant de 1 à 8 atomes de carbone ;
R₃ est choisi dans le groupe consistant en l'oxygène, le soufre et NNH(C=O)OR₉ ; R₉ est alkyle inférieur ayant de 1 à 8 atomes de carbone ou alkyle inférieur substitué ayant de 1 à 8 atomes de carbone ;
et ses sels et esters pharmaceutiquement acceptables.

2. Composé selon la revendication 1 de formule 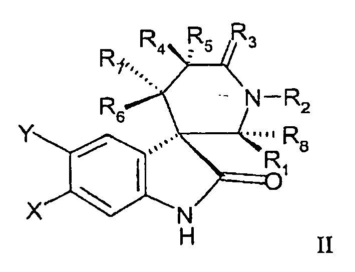 où
X est l'hydrogène, un halogène, cyano, nitro, éthynyle, cyclopropyle, méthyle, éthyle, isopropyle, méthoxy et vinyle ;
Y est l'hydrogène ou le fluor ;
R₁ est l'hydrogène ;
R₂ est l'hydrogène, alkyle inférieur ayant de 1 à 8 atomes de carbone ou alkyle inférieur substitué ayant de 1 à 8 atomes de carbone ;
R₄ et R₅ sont l'hydrogène ou alkyle inférieur ayant de 1 à 8 atomes de carbone ;
R₆ est l'hydrogène, cyano ou alkyle inférieur ayant de 1 à 8 atomes de carbone ;
R₃ est O, S ou NNH(C=O)OR₉ ;
R₇/R₈ est choisi indépendamment dans le groupe consistant en alkyle inférieur ayant de 1 à 8 atomes de carbone, alkyle inférieur substitué ayant de 1 à 8 atomes de carbone, alcényle inférieur ayant de 2 à 8 atomes de carbone, alcényle inférieur substitué ayant de 2 à 8 atomes de carbone, aryle, aryle substitué, hétéroaryle, lequel hétéroaryle est un système cyclique hétérocyclique aromatique contenant jusqu'à deux cycles, hétéroaryle substitué, lequel hétéroaryle est un système cyclique hétérocyclique aromatique contenant jusqu'à deux cycles, hétérocycle, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, hétérocycle substitué, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, cycloalkyle, cycloalkyle substitué, cycloalcényle et cycloalcényle substitué ;
R₉ est alkyle inférieur ayant de 1 à 8 atomes de carbone ou alkyle inférieur substitué ayant de 1 à 8 atomes de carbone ;
et ses sels et esters pharmaceutiquement acceptables.

3. Composé selon la revendication 2 où
X est le chlore ou le brome ;
Y est l'hydrogène ;
R₁ est l'hydrogène ;
R₄ et R₅ sont l'un et l'autre l'hydrogène ;
R₆ est l'hydrogène ;
R₃ est O ;
R₇ est un phényle substitué ou hétéroaryle substitué, avec le phényle substitué ou l'hétéroaryle substitué choisi dans le groupe consistant en 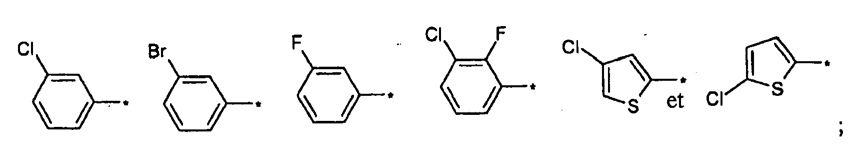 R₈ est choisi indépendamment dans le groupe consistant en alkyle inférieur ayant de 1 à 8 atomes de carbone, alkyle inférieur substitué ayant de 1 à 8 atomes de carbone, alcényle inférieur ayant de 2 à 8 atomes de carbone, alcényle inférieur substitué ayant de 2 à 8 atomes de carbone, aryle, aryle substitué, hétéroaryle, lequel hétéroaryle est un système cyclique hétérocyclique aromatique contenant jusqu'à deux cycles, hétéroaryle substitué, lequel hétéroaryle est un système cyclique hétérocyclique aromatique contenant jusqu'à deux cycles, cycloalkyle, cycloalkyle substitué, cycloalcényle et cycloalcényle substitué ; et
R₂ est l'hydrogène, alkyle inférieur ayant de 1 à 8 atomes de carbone ou alkyle inférieur substitué ayant de 1 à 8 atomes de carbone ;
avec la condition que quand R₂ est alkyle inférieur ayant de 1 à 8 atomes de carbone ou alkyle inférieur substitué ayant de 1 à 8 atomes de carbone, R₈ est choisi parmi alcényle inférieur ayant de 2 à 8 atomes de carbone, alcényle inférieur substitué ayant de 2 à 8 atomes de carbone, aryle, aryle substitué, hétéroaryle, lequel hétéroaryle est un système cyclique hétérocyclique aromatique contenant jusqu'à deux cycles, hétéroaryle substitué, lequel hétéroaryle est un système cyclique hétérocyclique aromatique contenant jusqu'à deux cycles, cycloalkyle, cycloalkyle substitué, cycloalcényle ou cycloalcényle substitué.

4. Composé selon la revendication 3 où
X est -Cl ou -Br ;
Y est l'hydrogène ;
R₁ est l'hydrogène ;
R₄ et R₅ sont l'un et l'autre l'hydrogène ;
R₆ est l'hydrogène ;
R₃ est O ;
R₇ est un phényle substitué ou hétéroaryle substitué, avec le phényle substitué ou l'hétéroaryle substitué choisi dans le groupe consistant en 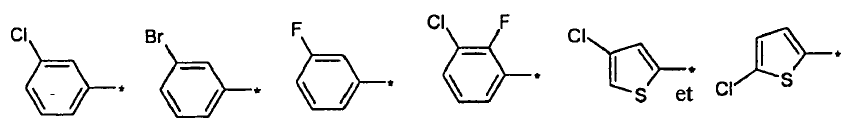 et
R₈ est choisi dans le groupe consistant en 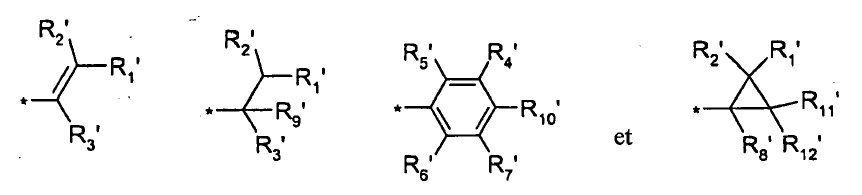 où
R₁' est l'hydrogène, méthyle, éthyle, propyle, isopropyle, -CF₃, -F, -CHF₂, ou -CH₂F ;
R₂' est l'hydrogène, méthyle, éthyle, propyle, isopropyle, -CF₃, -F, -CHF₂, ou -CH₂F ;
R₃' est l'hydrogène, -F, -CF₃, -CH₂F, méthyle, éthyle, propyle, isopropyle, cyclopropyle, tert-butyle ou sec-butyle ;
R₄' est l'hydrogène, -F, -Cl, -Br, -I, méthyle, éthyle, cyclopropyle, cyano, méthoxy ou éthynyle ;
R₅' est l'hydrogène, -F ou méthyle ;
R₆' est choisi dans le groupe consistant en l'hydrogène, halogène, hydroxy, cyano, alkyle inférieur ayant de 1 à 8 atomes de carbone, alkyle inférieur substitué ayant de 1 à 8 atomes de carbone, alcoxy inférieur ayant de 1 à 8 atomes de carbone, alcoxy inférieur substitué ayant de 1 à 8 atomes de carbone, alcynyle inférieur ayant de 2 à 6 atomes de carbone, alcynyle inférieur substitué ayant de 2 à 6 atomes de carbone ;
R₇' est l'hydrogène, -F ou méthyle ;
R₈' est l'hydrogène, -F, méthyle, éthyle, propyle, isopropyle, tert-butyle ou sec-butyle ;
R₉' est l'hydrogène, hydroxyle ou -F ;
R₁₀' est l'hydrogène ou -F ;
R₁₁' est l'hydrogène ou méthyle ;
R₁₂' est l'hydrogène ou méthyle ;
R₂ est l'hydrogène, alkyle inférieur ayant de 1 à 8 atomes de carbone ou alkyle inférieur substitué ayant de 1 à 8 atomes de carbone ;
avec la condition que quand R₂ est alkyle inférieur ayant de 1 à 8 atomes de carbone ou alkyle inférieur substitué ayant de 1 à 8 atomes de carbone, R₈ est choisi dans le groupe consistant en

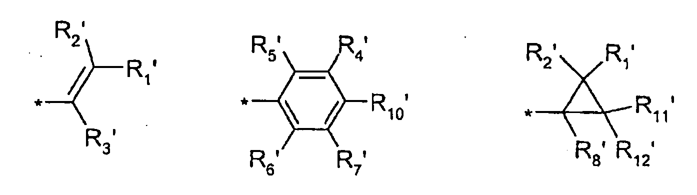

5. Composé selon la revendication 1 de formule 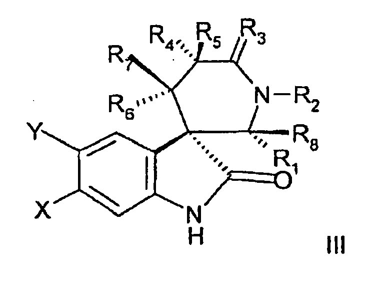 où
X est choisi dans le groupe consistant en l'hydrogène, un halogène, cyano, nitro, éthynyle, cyclopropyle, méthyle, éthyle, isopropyle, méthoxy et vinyle ;
Y est l'hydrogène ou le fluor ;
R₁ est l'hydrogène ;
R₂ est l'hydrogène, alkyle inférieur ayant de 1 à 8 atomes de carbone ou alkyle inférieur substitué ayant de 1 à 8 atomes de carbone ;
R₄ et R₅ sont l'hydrogène ou alkyle inférieur ayant de 1 à 8 atomes de carbone ;
R₆ est l'hydrogène, cyano ou alkyle inférieur ayant de 1 à 8 atomes de carbone ;
R₃ est O, S ou NNH(C=O)OR₉ ;
R₇/R₈ est choisi indépendamment dans le groupe consistant en alkyle inférieur ayant de 1 à 8 atomes de carbone, alkyle inférieur substitué ayant de 1 à 8 atomes de carbone, alcényle inférieur ayant de 2 à 8 atomes de carbone, alcényle inférieur substitué ayant de 2 à 8 atomes de carbone, aryle, aryle substitué, hétéroaryle, lequel hétéroaryle est un système cyclique hétérocyclique aromatique contenant jusqu'à deux cycles, hétéroaryle substitué, lequel hétéroaryle est un système cyclique hétérocyclique aromatique contenant jusqu'à deux cycles, hétérocycle, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, hétérocycle substitué, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, cycloalkyle, cycloalkyle substitué, cycloalcényle et cycloalcényle substitué ; et
R₉ est alkyle inférieur ayant de 1 à 8 atomes de carbone ou alkyle inférieur substitué ayant de 1 à 8 atomes de carbone ;
et ses sels et esters pharmaceutiquement acceptables.

6. Composé selon la revendication 5 où
X est -Cl ou -Br ;
Y est l'hydrogène ;
R₁ est l'hydrogène ;
R₂ est l'hydrogène, alkyle inférieur ayant de 1 à 8 atomes de carbone ou alkyle inférieur substitué ayant de 1 à 8 atomes de carbone ;
R₄ et R₅ sont l'un et l'autre l'hydrogène ;
R₆ est l'hydrogène ;
R₃ est O;
R₈ est un phényle substitué avec le phényle substitué choisi dans le groupe consistant en 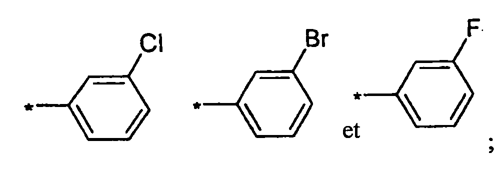 et
R₇ est choisi dans le groupe consistant en alkyle inférieur ayant de 1 à 8 atomes de carbone, alkyle inférieur substitué ayant de 1 à 8 atomes de carbone, alcényle inférieur ayant de 2 à 8 atomes de carbone, alcényle inférieur substitué ayant de 2 à 8 atomes de carbone, aryle, aryle substitué, hétéroaryle, lequel hétéroaryle est un système cyclique hétérocyclique aromatique contenant jusqu'à deux cycles, hétéroaryle substitué, lequel hétéroaryle est un système cyclique hétérocyclique aromatique contenant jusqu'à deux cycles, hétérocycle, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, hétérocycle substitué, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, cycloalkyle, cycloalkyle substitué, cycloalcényle et cycloalcényle substitué.

7. Composé de formule I, II ou III selon les revendications 1, 2 ou 5 où
Xest-Cl;
Y ; R₁ ; R₂ ; R₄ ; R₅ et R₆ sont l'hydrogène ;
R₃ est l'oxygène ; et
R₇ et R₈ sont des groupes phényle, qui sont chacun indépendamment non substitués ou substitués 1, 2 ou 3 fois par
alkyle inférieur ayant de 1 à 8 atomes de carbone, alcényle inférieur ayant de 2 à 8 atomes de carbone, alcynyle inférieur ayant de 2 à 6 atomes de carbone, dioxo-alkylène inférieur ayant de 1 à 8 atomes de carbone (formant par exemple un groupe benzodioxyle), halogène, hydroxy, -CN, -CF₃, -NH₂, -N(H, alkyle inférieur ayant de 1 à 8 atomes de carbone), N(alkyle inférieur ayant de 1 à 8 atomes de carbone)₂, aminocarbonyle, carboxy, -NO₂, alcoxy inférieur ayant de 1 à 8 atomes de carbone, thio-alcoxy inférieur ayant de 1 à 8 atomes de carbone, alkylsulfonyle inférieur ayant de 1 à 8 atomes de carbone, aminosulfonyle, alkyle inférieur (ayant de 1 à 8 atomes de carbone)carbonyle, alkyle inférieur(ayant de 1 à 8 atomes de carbone)carbonyloxy, alcoxy inférieur(ayant de 1 à 8 atomes de carbone)-carbonyle, alkyle inférieur(ayant de 1 à 8 atomes de carbone)-carbonyl-NH, fluoro-alkyle inférieur ayant de 1 à 8 atomes de carbone, fluoro-alcoxy inférieur ayant de 1 à 8 atomes de carbone, alcoxy inférieur(ayant de 1 à 8 atomes de carbone)-carbonyl-alcoxy inférieur (ayant de 1 à 8 atomes de carbone), carboxy-alcoxy inférieur (ayant de 1 à 8 atomes de carbone), carbamoyl-alcoxy inférieur (ayant de 1 à 8 atomes de carbone), hydroxy-alcoxy inférieur (ayant de 1 à 8 atomes de carbone), -NH₂-alcoxy inférieur ayant de 1 à 8 atomes de carbone, -N(H, alkyle inférieur ayant de 1 à 8 atomes de carbone)-alcoxy inférieur ayant de 1 à 8 atomes de carbone, -N(alkyle inférieur ayant de 1 à 8 atomes de carbone)₂-alcoxy inférieur ayant de 1 à 8 atomes de carbone, benzyloxy-alcoxy inférieur (ayant de 1 à 8 atomes de carbone), aminosulfonyle mono- ou di-(alkyle inférieur ayant de 1 à 8 atomes de carbone) substitué et alkyle inférieur ayant de 1 à 8 atomes de carbone qui peut éventuellement être substitué avec halogène, hydroxy, -NH₂, -N(H, alkyle inférieur ayant de 1 à 8 atomes de carbone) ou -N(alkyle inférieur ayant de 1 à 8 atomes de carbone)₂.

8. Composé de formule I, II ou III selon les revendications 1, 2 ou 5 où
X est -Cl ;
Y ; R₁ ; R₂ ; R₄ ; R₅ et R₆ sont l'hydrogène ;
R₃ est l'oxygène ; et
R₇ et R₈ sont des groupes phényle, qui sont chacun indépendamment non substitués ou substitués 1, 2 ou 3 fois par un substituant choisi indépendamment parmi
halogène, C₂₋₄-alcynyle ou -O-(C₁₋₄-alkyle), et
lesdits groupes C₂₋₄-alcynyle ou -O-(C₁₋₄-alkyle) sont non substitués ou substitués une fois par hydroxyle ou -N(C₁₋₄-alkyl)-S(O)₂-CH₃.

9. Composé selon la revendication 1 choisi dans le groupe consistant en
(2'S, 3S, 4'S)-6-chloro-2'-(3-chlorophényl)-4'-(2,2-diméthylpropyl) spiro[3H-indole-3,3'-pipéridine]-2,6'(1H)-dione,
(2'SR, 3S, 4'R)-4'-(tert-butyl)-6-chloro-2'-(3-chlorophényl) spiro[3H-indole-3,3'-pipéridine]-2,6'(1H)-dione,
(2'S, 3S, 4'R)-6-chloro-2'-(3-chlorophényl)-4'-isopropyl spiro[3H-indole-3,3'-pipéridine]-2,6'(1H)-dione,
(2'R, 3R, 4'S)-6-chloro-2',4'-bis(3-chlorophényl) spiro[3H-indole-3,3'pipéridine]-2,6'(1H)-dione racémique,
(2'R, 3R, 4'S)-6-chloro-2',4'-bis(3-chlorophényl) spiro[3H-indole-3,3'-pipéridine]-2,6'(1H)-dione chirale,
(2'S, 3S, 4'R)-6-chloro-2'-(3-chlorophényl)-4'-cyclopentyl spiro[3H-indole-3,3'-pipéridine]-2,6'(1H)-dione racémique,
(2'S, 3S, 4'R)-6-chloro-2'-(3-chlorophényl)-4'-cyclohexyl spiro[3H-indole-3,3'-pipéridine]-2,6'(1H)-dione racémique,
(2'S, 3S, 4'R)-6-chloro-2'-(4-chlorophényl)-4'-cyclohexyl spiro[3H-indole-3,3'-pipéridine]-2,6'(1H)-dione racémique,
(2'R, 3R, 4'S)-6-chloro-2'-(4-chlorophényl)-4'-(3-chlorophényl) spiro[3H-indole-3,3'-pipéridine]-2,6'(1H)-dione racémique et
(2'S, 3S, 4'R)-6-chloro-2'-(3-chlorophényl)-4'-(4-chlorophényl) spiro[3H-indole-3,3'-pipéridine]-2,6'(1H)-dione racémique.

10. Composé selon la revendication 1 choisi dans le groupe consistant en
(2'R, 3R, 4'S)-6-chloro-4'-(3-chlorophényl)-2'-(3-méthylphényl) spiro[3H-indole-3,3'-pipéridine]-2,6'(1H)-dione racémique,
(2'R, 3R, 4'S)-6-chloro-4'-(3-chlorophényl)-2'-(3-fluorophényl) spiro[3H-indole-3,3'-pipéridine]-2,6'(1H)-dione racémique,
(2'S, 3S, 4'R)-6-chloro-2'-(3-chlorophényl)-4'-phényl spiro[3H-indole-3,3'-pipéridine]-2,6'(1H)-dione racémique,
(2'S, 3S, 4'R, 5'R)-6-chloro-2'-(3-chlorophényl)-5'-méthyl-4'-phényl spiro[3H-indole-3,3'-pipéridine]-2,6'(1H)-dione racémique,
(2'R, 3R, 4'S)-6-chloro-4'-(3-chlorophényl)-2'-phényl spiro[3H-indole-3,3'-pipéridine]-2,6'(1H)-dione racémique,
(2'R, 3R, 4'S)-6-chloro-4'-(3-chlorophényl)-2'-phényl spiro[3H-indole-3,3'-pipéridine]-2,6'(1H)-dione chirale,
(2'R, 3R, 4'S)-6-chloro-4'-(3-chlorophényl)-2'-(3-méthoxyphényl) spiro[3H-indole-3,3'-pipéridine]-2,6'(1H)-dione racémique,
(2'R, 3R, 4'S)-6-chloro-2'-(2-chlôrophényl)-4'-(3-chlorophényl) spiro[3H-indole-3,3'-pipéridine]-2,6'(1H)-dione racémique,
(2'R, 3R, 4'S)-6-chloro-2'-(2-chlorophényl)-4'-(3-chlorophényl) spiro[3H-indole-3,3'-pipéridine]-2,6'(1H)-dione chirale et
(2'R, 3R, 4'S)-6-chloro-4'-(3-chlorophényl)-2'-(2-méthylphényl) spiro[3H-indole-3,3'-pipéridine]-2,6'(1H)-dione racémique.

11. Composé selon la revendication 1 choisi dans le groupe consistant en
(2'R, 3R, 4'S)-6-chloro-4'-(3-chlorophényl)-2'-(2-méthylphényl) spiro[3H-indole-3,3'-pipéridine]-2,6'(1H)-dione chirale,
(2'R, 3R, 4'S)-6-chloro-4'-(3-chlorophényl)-2'-(2-éthylphényl) spiro[3H-indole-3,3'-pipéridine]-2,6'(1H)-dione racémique,
(2'R, 3R, 4'S)-6-chloro-4'-(3-chlorophényl)-2'-(2-éthylphényl) spiro[3H-indole-3,3'-pipéridine]-2,6'(1H)-dione chirale,
(2'R, 3R, 4'S, 5'S)-6-chloro-4'-(3-chlorophényl)-5'-méthyl-2'-(2-méthylphényl) spiro[3H-indole-3,3'-pipéridine]-2,6'(1H)-dione racémique,
(2'R, 3R, 4'S, 5'S)-6-chloro-2',4'-bis(3-chlorophényl)-5'-méthyl spiro[3H-indole-3,3'-pipéridine]-2,6'(1H)-dione racémique,
(2'R, 3R, 4'S)-6-chloro-4'-(3-chlorophényl)-2'-(2-isopropylphényl) spiro[3H-indole-3,3'-pipéridine]-2,6'(1H)-dione racémique,
(2'R, 3R, 4'S)-6-chloro-4'-(3-chlorophényl)-2'-(2-isopropylphényl) spiro[3H-indole-3,3'-pipéridine]-2,6'(1H)-dione chirale,
(2'R, 3R, 4'S)-2'-(2-bromophényl)-6-chloro-4'-(3-chlorophényl) spiro[3H-indole-3,3'-pipéridine]-2,6'(1H)-dione racémique,
(2'R, 3R, 4'S)-2'-(2-bromophényl)-6-chloro-4'-(3-chlorophényl) spiro[3H-indole-3,3'-pipéridine]-2,6'(1H)-dione chirale et
(2'R. 3R, 4'S)-6-chloro-4'-(3-chlorophényl)-2'-(3-cyanophényl) spiro[3H-indole-3,3'-pipéridine]-2,6'(1H)-dione racémique.

12. Composé selon la revendication 1 choisi dans le groupe consistant en
(2'R, 3R, 4'S)-6-chloro-4'-(3-chlorophényl)-1'-méthyl-2'-(2-méthylphényl) spiro[3H-indole-3,3'-pipéridine]-2,6'(1H)-dione racémique,
(2'R, 3R, 4'S)-6-chloro-4'-(3-chlorophényl)-1'-méthyl-2'-(2-méthylphényl) spiro[3H-indole-3,3'-pipéridine]-2,6'(1H)-dione chirale,
(2'R, 3R, 4'S)-6-chloro-4'-(3-chlorophényl)-2'-(3-fluoro-2-méthyl-phényl) spiro[3H-indole-3, 3'-pipéridine ]-2,6'(1H)-dione racémique,
(2'R, 3R, 4'S)-6-chloro-4'-(3-chlorophényl)-2'-(3-fluoro-2-méthyl-phényl) spiro[3H-indole-3,3'-pipéridine]-2,6'(1H)-dione chirale,
(2'R, 3R, 4'S)-6-chloro-4'-(3-chlorophényl)-1'-éthyl-2'-(2-méthylphényl) spiro[3H-indole-3,3'-pipéridine]-2,6'(1H)-dione racémique,
(2'R, 3R, 4'S)-6-chloro-4'-(3-chlorophényl)-1'-éthyl-2'-(2-méthylphényl) spiro[3H-indole-3,3'-pipéridine]-2,6'(1H)-dione chirale,
(2'R, 3R, 4'S)-6-chloro-4'-(3-chlorophényl)-2'-(2,6-diméthylphényl) spiro[3H-indole-3,3'-pipéridine]-2,6'(1H)-dione racémique,
(2'R,' 3R, 4'S)-6-chloro-4'-(3-chlorophényl)-2'-(2,3-diméthylphényl) spiro[3H-indole-3,3'-pipéridine]-2,6'(1H)-dione racémique,
(2'R, 3R, 4'S)-6-chloro-4'-(3-chlorophényl)-1'-[(méthoxycarbonyl)méthyl]-2'-(2-méthylphényl) spiro[3H-indole-3,3'-pipéridine]-2,6'(1H)-dione racémique et
(2'S, 3R)-6-chloro-2'-(3-chlorophényl)-4'-isopropyl-6'-thioxo spiro[3H-indole-3,3'-pipéridine]-2(1H)-one racémique.

13. Composé selon la revendication 1 choisi dans le groupe consistant en
(2'R, 3R, 4'S)-6-chloro-4'-(3-chlorophényl)-1'-[(hydroxycarbonyl)-méthyl]-2'-(2-méthylphényl) spiro[3H-indole-3,3'-pipéridine]-2,6'(1H)-dione racémique,
(2'R, 3R, 4'S)-6-chloro-4'-(3-chlorophényl)-2'-[2-(trifluorométhyl)-phényl] spiro[3H-indole-3,3'-pipéridine]-2,6'(1H)-dione racémique,
(2'R, 3R, 4'S)-6-chloro-4'-(3-chlorophényl)-6'-thioxo-2'-[2-(trifluorométhyl)-phényl] spiro[3H-indole-3,3'-pipéridine]-2(1H)-one racémique,
(2'R, 3R, 4'S)-6-chloro-4'-(3-chlorophényl)-2'-(2-méthylphényl)-1'-[2-(4-morpholinyl)-carbonyl-méthyl] spiro[3H-indole-3,3'-pipéridine]-2,6'(1H)-dione racémique,
(2'R, 3R, 4'S)-6-chloro-4'-(3-chlorophényl)-2'-(2-méthylphényl) -1'-[2-(4-morpholinyl)-carbonyl-méthyl] spiro[3H-indole-3, 3'-pipéridine ]-2, 6'(1H)-dione chirale,
(2'R, 3R, 4'S)-6-chloro-4'-(3-chlorophényl)-2'-[5-fluoro-2-(trifluorométhyl)-phényl] spiro[3H-indole-3,3'-pipéridine]-2,6'(1H)-dione racémique,
(2'R, 3R, 4'S)-6-chloro-4'-(3-chlorophényl)-2'-(5-fluoro-2-méthylphényl) spiro[3H-indole-3,3'-pipéridine]-2,6'(1H)-dione racémique,
(2'R, 3R, 4'S)-6-chloro-4'-(3-chlorophényl)-2'-(5-fluoro-2-méthylphényl) spiro[3H-indole-3,3'-pipéridine]-2,6'(1H)-dione chirale,
(2'R, 3R, 4'S)-6-chloro-4'-(3-chlorophényl)-1'-[(cyclopropylamino)-carbonyl-méthyl]-2'-(2-méthylphényl) spiro[3H-indole-3,3'-pipéridine]-2,6'(1H)-dione racémique et
*tert*-butylester d'acide (2'R, 3R, 4'S)-6-chloro-[[2-[6-chloro-4'-(3-chlorophényl)-2,3-dihydro-2'-(2-méthylphényl)-2,6-dioxospiro[3H-indole-3,3'-pipéridin]-1-yl]-1-oxoéthyl]-amino]-pipéridine carboxylique racémique.

14. Composé selon la revendication 1 choisi dans le groupe consistant en
(2'R, 3R, 4'S)-6-chloro-4'-(3-chlorophényl)-2'-(5-fluoro-2-méthylphényl)-6'-thioxo spiro[3H-indole-3,3'-pipéridine]-2(1H)-one racémique,
éthylester d'acide (2'R, 3R, 4'S)-[6-chloro-4'-(3-chlorophényl)-2,3-dihydro-2'-[2-(trifluorométhyl)phényl) ]-2-oxospiro[3H-indole-3,3'-pipéridin]-6-ylène]-hydrazine carboxylique racémique,
(2'R, 3R, 4'S)-6-chloro-4'-(3-chlorophényl)-2'-(2,4-difluorophényl) spiro[3H-indole-3,3'-pipéridine]-2,6'(1H)-dione racémique,
(2'R, 3R, 4'S)-6-chloro-4'-(3-chlorophényl)-2'-(5-fluoro-2-méthoxyphényl) spiro[3H-indole-3,3'-pipéridine]-2,6'(1H)-dione racémique,
(2'R, 3R, 4'S)-6-chloro-4'-(3-chlorophényl)-2'-(1-naphtalényl) spiro[3H-indole-3,3'-pipéridine]-2,6'(1H)-dione racémique,
(2'R, 3R, 4'S)-6-chloro-4'-(3-chlorophényl)-2'-(3-pyridinyl) spiro[3H-indole-3,3'-pipéridine]-2,6'(1H)-dione racémique,
(2'R, 3R, 4'S)-6-chloro-4'-(3-chlorophényl)-2'-(2,3-difluoro-6-méthoxyphényl) spiro[3H-indole-3,3'-pipéridine]-2,6'(1H)-dione racémique,
(2'R, 3R, 4'S)-6-chloro-4'-(3-chlorophényl)-2'-(3,4-difluorophényl) spiro[3H-indole-3,3'-pipéridine]-2,6'(1H)-dione racémique,
(2'R, 3R, 4'S)-6-chloro-4'-(3-chlorophényl)-2'-(1-cyclohexényl) spiro[3H-indole-3,3'-pipéridine]-2,6'(1H)-dione racémique et
(2'R, 3R, 4'S)-6-chloro-4'-(3-chlorophényl)-2'-(3,4-difluorophényl)-6'-thioxo spiro[3H-indole-3,3'-pipéridine]-2(1H)-one racémique.

15. Composé selon la revendication 1 choisi dans le groupe consistant en
éthylester d'acide (2'R, 3R, 4'S)-[6-chloro-4'-(3-chlorophényl)-2'-(3,4-difluorophényl)-2,3-dihydro-2-oxospiro[3H-indole-3,3'-pipéridin]-6-ylène] hydrazine carboxylique racémique,
(2'R, 3R, 4'S)-2'-(1,3-benzodioxol-4-yl)-6-chloro-4'-(3-chlorophényl) spiro[3H-indole-3,3'-pipéridine]-2,6'(1H)-dione racémique,
éthylester d'acide (2'R, 3R, 4'S)-[6-chloro-4'-(3-chlorophényl)-2'-(5-fluoro-2-méthylphényl)-2,3-dihydro-2-oxospiro[3H-indole-3,3'-pipéridin]-6-ylène] hydrazine carboxylique racémique,
(2'R, 3R, 4'S)-6-chloro-4-(3-chlorophényl)-2'-(2,3-difluoro-6-méthylphényl) spiro[3H-indole-3,3'-pipéridine]-2,6'(1H)-dione racémique,
(2'R, 3R, 4'S)-6-chloro-4'-(3-chlorophényl)-5-fluoro-2'-(5-fluoro-2-méthylphényl) spiro[3H-indole-3,3'-pipéridine]-2,6'(1H)-dione racémique,
(2'R, 3R, 4'S)-6-chloro-4'-(3-chlorophényl)-2'-(2,3-difluoro-6-méthylphényl)-6'-thioxo spiro[3H-indole-3,3'-pipéridine]-2(1H)-one racémique,
(2'R, 3R, 4'S)-6-chloro-4'-(3-chlorophényl)-2'-(5-fluoro-2-méthylphényl)-1'-hydroxycarbonylméthyl spiro[3H-indole-3,3'-pipéridine]-2,6'(1H)-dione racémique,
(2'R, 3R, 4'S)-6-chloro-4'-(3-chlorophényl)-2'-(5-fluoro-2-méthylphényl)-1'-méthyl spiro[3H-indole-3,3'-pipéridine]-2,6'(1H)-dione racémique,
(2'R, 3R, 4'S)-6-chloro-4'-(3-chlorophényl)-2'-(5-fluoro-2-méthylphényl)-1'-méthyl spiro[3H-indole-3,3'-pipéridine]-2,6'(1H)-dione chirale et
(2'R, 3R, 4'S)-6-chloro-4'-(3-chlorophényl)-1'-fluorocarbonylméthyl-2'-(5-fluoro-2-méthylphényl) spiro[3H-indole-3,3'-pipéridine]-2,6'(1H)-dione racémique.

16. Composé selon la revendication 1 choisi dans le groupe consistant en
(2'R, 3R, 4'S)-6-chloro-4'-(3-chlorophényl)-2'-(5-fluoro-2-méthylphényl)-1'-[(1-méthyl-pipéridin-4-yl)aminocarbonyl)-méthyl] spiro[3H-indole-3,3'-pipéridine]-2,6'(1H)-dione racémique,
(2'R, 3R, 4'S)-6-chloro-4'-(3-chlorophényl)-2'-(5-fluoro-2-méthylphényl)-1'-[(1-méthyl-pipéridin-4-yl)aminocarbonyl)-méthyl] spiro[3H-indole-3,3'-pipéridine]-2,6'(1H)-dione chirale,
(2'R, 3R; 4'S)-1'-[1-tert-butoxycarbonyl-pipéridin-4-yl)aminocarbonyl-méthyl]-6-chloro-4'-(3-chlorophényl)-2'-(5-fluorô-2-méthylphényl) spiro[3H-indole-3,3'-pipéridine]-2,6'(1H)-dione racémique,
(2'R, 3R, 4'S)-6-chloro-4'-(3-chlorophényl)-2'-(5-fluoro-2-méthylphényl)-1'-[(pipéridin-4-yl)aminocarbonyl-méthyl] spiro[3H-indole-3,3'-pipéridine]-2,6'(1H)-dione racémique,
(2'R, 3R, 4'S)-6-chloro-4'-(3-chlorophényl)-2'-(5-fluoro-2-méthylphényl)-1'-[(pipéridin-4-yl)aminocarbonyl-méthyl] spiro[3H-indole-3,3'-pipéridine]-2,6'(1H)-dione chirale,
(2'R, 3R, 4'S)-6-chloro-4'-(3-chlorophényl)-2'-(5-fluoro-2-méthylphényl)-1'-(3-morpholin-4-yl-propyl) spiro[3H-indole-3,3'-pipéridine]-2,6'(1H)-dione racémique,
(2'R, 3R, 4'S)-6-chloro-4'-(3-chlorophényl)-2'-(5-fluoro-2-méthylphényl)-1'-(3-morpholin-4-yl-propyl) spiro[3H-indole-3,3'-pipéridine]-2,6'(1H)-dione chirale,
(2'R, 3R, 4'S)-6-chloro-4'-(3-chlorophényl)-2'-(1-isopropyl-4-méthyl-pent-1-ényl) spiro[3H-indole-3,3'-pipéridine]-2,6'(1H)-dione racémique,
(2'R, 3R, 4'S)-6-chloro-4'-(3-chlorophényl)-2'-(2,3-difluoro-6-isopropoxyphényl) spiro[3H-indole-3,3'-pipéridine]-2,6'(1H)-dione racémique et
(2'R, 3R, 4'S)-6-chloro-4'-(3-chlorophényl)-2'-(2,3-difluoro-6-isopropoxy-phényl) spiro[3H-indole-3,3'-pipéridine]-2,6'(1H)-dione chirale.

17. Composé selon la revendication 1 choisi dans le groupe consistant en
(2'R, 3R, 4'S)-6-chloro-4'-(3-chlorophényl)-2'-(5-fluoro-2-méthylphényl)-1'-(3-pyrrolidin-1-yl-propyl) spiro[3H-indole-3,3'-pipéridine]-2,6'(1H)-dione racémique,
(2'R, 3R, 4'S)-6-chloro-4'-(3-chlorophényl)-2'-(3-méthoxycarbonyl-phényl) spiro[3H-indole-3,3'-pipéridine]-2,6'(1H)-dione racémique, (2'R, 3R, 4'S)-'-[3-(4-acétyl-pipérazin-1-yl)-propyl]-6-chloro-4'-(3-chlorophényl)-2'-(5-fluoro-2-méthylphé nyl) spiro[3H-indole-3,3'-pipéridine]-2,6'(1H)dione racémique,
(2'R, 3R, 4'S)-6-chloro-4'-(3-chlorophényl)-2'-(1-éthyl-propényl) spiro[3H-indole-3,3'-pipéridine]-2,6'(1H)-dione racémique,
(2'R, 3R, 4'S)-6-chloro-4'-(3-chlorophényl)-2'-(1-éthyl-propényl) spiro[3H-indole-3,3'-pipéridine]-2,6'(1H)-dione chirale,
(2'R, 3R, 4'S)-6-chloro-4'-(3-chlorophényl)-1'-[3-(1,1-dioxo-thiomorpholin-4yl)-propyl]-2'-(5-fluoro-2-méthylphényl)-spiro[3H-indole-3,3'-pipéridine]-2,6'(1H)-dione racémique,
(2'R, 3R, 4'S)-6-chloro-4'-(3-chlorophényl)-1'-[3-(1,1-dioxo-thiomorpholin-4-yl)-propyl]-2'-(5-fluoro-2-méthylphényl)-spiro[3H-indole-3,3'-pipéridine]-2,6'(1H)-dione chirale,
(2'R, 3R, 4'S)-6-chloro-4'-(3-chlorophényl)-2'-(2,5-diméthyl-phényl) spiro[3H-indole-3,3'-pipéridine]-2,6'(1H)-dione racémique,
(2'R, 3R, 4'S)-6-chloro-4'-(3-chlorophényl)-2'-(2,5-diméthyl-phényl) spiro[3H-indole-3,3'-pipéridine]-2,6'(1H)-dione chirale et
(2'R, 3R, 4'S)-6-chloro-4'-(3-chlorophényl)-2'-(2,5-diméthyl-2H-pyrazol-3-yl) spiro[3H-indole-3,3'-pipéridine]-2,6'(1H)-dione racémique.

18. Composé selon la revendication 1 choisi dans le groupe consistant en
(2'R, 3R, 4'S)-6-chloro-4'-(3-chlorophényl)-2'-(1-méthyl-propényl) spiro[3H-indole-3,3'-pipéridine]-2,6'(1H)-dione racémique,
(2'R, 3R, 4'S)-6-chloro-4'-(3-chlorophényl)-2'-(1-méthyl-but-1-ényl) spiro[3H-indole-3,3'-pipéridine]-2,6'(1H)-dione racémique,
(2'R, 3R, 4'S)-6-chloro-4'-(3-chlorophényl)-2'-(1-éthyl-propyl) spiro[3H-indole-3,3'-pipéridine]-2,6'(1H)-dione racémique,
(2'R, 3R, 4'S)-6-chloro-4'-(3-chlorophényl)-2'-(1-éthyl-propyl) spiro[3H-indole-3,3'-pipéridine]-2,6'(1H)-dione chirale,
(2'R, 3R, 4'S)-6-chloro-4'-(3-chlorophényl)-2'-(2,3-difluoro-6-isopropoxyphényl)-1'-(3-morpholin-4-yl-propyl) spiro[3H-indole-3,3'-pipéridine]-2,6'(1H)-dione racémique,
(2'R, 3R, 4'S)-6-chloro-4'-(3-chlorophényl)-2'-(2,3-difluoro-6-isopropoxyphényl)-1'-(3-morpholin-4-yl-propyl) spiro[3H-indole-3,3'-pipéridine]-2,6'(1H)-dione chirale,
(2'R, 3R, 4'S)-6-chloro-4'-(3-chlorophényl)-2'-(1-éthylidène-pentyl) spiro[3H-indole-3,3'-pipéridine]-2,6'(1H)-dione racémique,
(2'R, 3R, 4'S)-6-chloro-4'-(3-chlorophényl)-2'-(2,3-difluoro-6-isopropoxyphényl)-6'-thioxo spiro[3H-indole-3,3'-pipéridine]-2(1H)-one racémique,
éthylester d'acide (2'R, 3R, 4'S)-[6-chloro-4'-(3-chlorophényl)-2'-(2,3-difluoro-6-isopropoxyphényl)-2,3-dihydro-2-oxospiro[3H-indole-3,3'-pipéridin]-6-ylène]-hydrazine carboxylique racémique et
(2'R, 3R, 4'S)-6-chloro-4'-(3-chlorophényl)-2'-(cyclopent-1-ényl) spiro[3H-indole-3,3'-pipéridine]-2,6'(1H)-dione racémique.

19. Composé selon la revendication 1 choisi dans le groupe consistant en
(2'R, 3R, 4'S)-6-chloro-4'-(3-chlorophényl)-2'-cyclopentyl spiro[3H-indole-3,3'-pipéridine]-2,6'(1H)-dione racémique,
(2'R, 3R, 4'S)-6-chloro-4'-(3-chlorophényl)-2'-cyclopentyl spiro[3H-indole-3,3'-pipéridine]-2,6'(1H)-dione chirale,
(2'R, 3R, 4'S)-6-chloro-4'-(3-chlorophényl)-2'-(1-isopropyl-propényl) spiro[3H-indole-3,3'-pipéridine]-2,6'(1H)-dione racémique,
(2'R, 3R. 4'S)-6-chloro-4'-(3-chlorophényl)-2'-(1-isopropyl-propényl) spiro[3H-indole-3,3'-pipéridine]-2,6'(1H)-dione chirale,
(2'R, 3R, 4'S)-6-chloro-4'-(3-chlorophényl)-2'-(1-méthylène-propyl) spiro[3H-indole-3,3'-pipéridine]-2,6'(1H)-dione racémique,
(2'R, 3R, 4'S)-6-chloro-4'-(3-chlorophényl)-2'-(1-méthylène-propyl) spiro[3H-indole-3,3'-pipéridine]-2,6'(1H)-dione chirale,
(2'R, 3R, 4'S)-6-chloro-4'-(3-chlorophényl)-2'-(1-éthyl-propényl)-1'-hydroxy-carbonylméthyl-spiro[3H-indole-3,3'-pipéridine]-2,6'(1H)-dione racémique,
(2'R, 3R, 4'S)-6-chloro-4'-(3-chlorophényl)-2'-(1-éthyl-propényl)-1'-[(2-hydroxy-1,1-diméthyléthyl)aminocarbonyl-méthyl] spiro[3H-indole-3,3'-pipéridine]-2,6'(1H)-dione racémique,
(2'R, 3R, 4'S)-6-chloro-4'-(3-chlorophényl)-2'-(1-éthyl-propényl)-1'-[(2-hydroxy-1,1-diméthyléthyl)aminocarbonyl-méthyl] spiro[3H-indole-3,3'-pipéridine]-2,6'(1H)dione chirale et
(2'R, 3R, 4'S)-6-chloro-4'-(3-chlorophényl)-2'-(1-éthyl-propényl)-1'-(3-morpholin-4-yl-propyl) spiro[3H-indole-3,3'-pipéridine]-2,6'(1H)-dione racémique.

20. Composé selon la revendication 1 choisi dans le groupe consistant en
(2'R, 3R, 4'S)-6-chloro-4'-(3-chlorophényl)-2'-(1-éthyl-propényl)-1'-(3-morpholin-4-yl-propyl) spiro[3H-indole-3,3'-pipéridine]-2,6'(1H)-dione chirale,
(2'R, 3R, 4'S)-6-chloro-4'-(3-chlorophényl)-2'-(2-méthyl-1-méthylène-propyl) spiro[3H-indole-3,3'-pipéridine]-2,6'(1H)-dione racémique,
(2'R, 3R, 4'S)-6-chloro-4'-(3-chlorophényl)-2'-(2,2-diméthyl-1-méthylènepropyl) spiro[3H-indole-3,3'-pipéridine]-2,6'(1H)-dione racémique,
(2'R, 3R, 4'S)-6-chloro-4'-(3-chlorophényl)-2'-(1-éthyl-propényl)-1'-[(2-morpholin-4-yl-éthyl)-aminocarbonyl-méthyl] spiro[3H-indole-3,3'-pipéridine]-2,6'(1H)dione racémique,
(2'R, 3R, 4'S)-6-chloro-4'-(3-chlorophényl)-2'-isopropényl spiro[3H-indole-3,3'-pipéridine]-2,6'(1H)-dione racémique,
(2'R, 3R, 4'S)-6-chloro-4'-(3-chlorophényl)-2'-isopropényl spiro[3H-indole-3,3'-pipéridine]-2,6'(1H)-dione chirale,
(2'R, 3R, 4'S)-6-chloro-4'-(3-chlorophényl)-2'-(1-méthylène-butyl) spiro[3H-indole-3,3'-pipéridine]-2,6'(1H)-dione racémique,
(2'R, 3R, 4'S)-6-chloro-4'-(3-chlorophényl)-2'-(1-méthylène-butyl) spiro[3H-indole-3,3'-pipéridine]-2,6'(1H)-dione chirale,
(2'R, 3R, 4'S)-6-chloro-4'-(3-chlorophényl)-2'-(3-méthyl-1-méthylène-butyl) spiro[3H-indole-3,3'-pipéridine]-2,6'(1H)-dione racémique et
(2'R, 3R, 4'S)-6-chloro-4'-(3-chlorophényl)-2'-(3-méthyl-1-méthylène-butyl) spiro[3H-indole-3,3'-pipéridine]-2,6'(1H)-dione chirale.

21. Composé selon la revendication 1 choisi dans le groupe consistant en
(2'R, 3R, 4'S)-6-chloro-4'-(3-chlorophényl)-2'-(1-méthylène-butyl)-6'-thioxo spiro[3H-indole-3,3'-pipéridine]-2(1H)-one racémique,
(2'R, 3R, 4'S)-6-chloro-4'-(3-chlorophényl)-2'-(4-éthoky-1,2-difluoro-phényl) spiro[3H-indole-3,3'-pipéridine]-2,6'(1H)-dione racémique,
(2'R, 3R, 4'S)-6-chloro-4'-(3-chlorophényl)-2'-(4-éthoxy-1,2-difluoro-phényl) spiro[3H-indole-3,3'-pipéridine]-2,6'(1H)-dione chirale,
(2'R, 3R, 4'S)-6-chloro-4'-(3-chloro-5-méthoxycarbonyl-phényl)-2'-(1-méthylène-propyl) spiro[3H-indole-3,3'-pipéridine]-2,6'(1H)-dione racémique,
(2'R, 3R, 4'S)-6-chloro-4'-(3-chloro-5-hydroxycarbonyl-phényl)-2'-(1-méthylène-propyl) spiro[3H-indole-3,3'-pipéridine]-2,6'(1H)-dione racémique,
(2'R, 3R, 4'S)-6-chloro-4'-(3-chloro-5-fiuorocarbonyl-phényl)-2'-(-méthylène-propyl) spiro[3H-indole-3,3'-pipéridine]-2,6'(1H)-dione racémique,
(2'R, 3R, 4'S)-6-chloro-4'-[3-chloro-5-(4-méthanesulfonyl-pipérazine-1-carbonyl)-phényl]-2'-(1-méthylène-propyl) spiro[3H-indole-3,3'-pipéridine]-2,6'(1H)dione racémique,
(2'R, 3R, 4'S)-6-chloro-4'-(3-chlorophényl)-2'-(1-éthyl-propényl)-1'-méthyl spiro[3H-indole-3,3'-pipéridine]-2,6'(1H)-dione racémique,
(2'R, 3R, 4'S)-6-chloro-4'-(3-chlorophényl)-2'-(1-éthyl-propényl)-1'-méthyl spiro[3H-indole-3,3'-pipéridine]-2,6'(1H)-dione chirale,
(2'R, 3R, 4'S)-6-chloro-4'-(3-chlorophényl)-2'-isopropyl spiro[3H-indole-3,3'-pipéridine]-2,6'(1H)-dione racémique et
(2'R, 3R, 4'S)-6-chloro-4'-(3-chlorophényl)-2'-isopropyl spiro[3H-indole-3,3'-pipéridine]-2,6'(1H)-dione chirale.

22. Composé selon la revendication 1 choisi dans le groupe consistant en
(2'R, 3R, 4'S)-6-chloro-4'-(3-chlorophényl)-2'-(1-méthylène-propyl)-1'-méthyl spiro[3H-indole-3,3'-pipéridine]- 2,6'(1H)-dione racémique,
(2'R, 3R, 4'S)-6-chloro-4'-(3-chlorophényl)-2'-(1-méthylène-propyl)-1'-méthyl spiro[3H-indole-3,3'-pipéridine]-2,6'(1H)-dione chirale,
(2'R, 3R, 4'S)-2'-sec-butyl-6-chloro-4'-(3-chlorophényl) spiro[3H-indole-3,3'-pipéridine]-2,6'(1H)-dione racémique,
(2'R, 3R, 4'S)-6-chloro-4'-(3-chlorophényl)-2'-(1-hydroxyméthyl-vinyl) spiro[3H-indole-3,3'-pipéridine]-2,6'(1H)-dione racémique,
(2'R, 3R, 4'S)-6-chloro-4'-(3-chlorophényl)-2'-(1-méthoxyméthyl-vinyl) spiro[3H-indole-3,3'-pipéridine]-2,6'(1H)-dione racémique,
(2'R, 3R, 4'S)-6-chloro-4'-(3-chlorophényl)-2'-[2,3-difluoro-6-(2-méthoxy-éthoxy)-phényl] spiro[3H-indole-3,3'-pipéridine]-2,6'(1H)-dione racémique,
(2'R, 3R, 4'S)-6-chloro-4'-(3-chlorophényl)-2'-[2,3-difluoro-6-(2-méthoxyéthoxy)-phényl] spiro[3H-indole-3,3'-pipéridine]-2,6'(1H)-dione chirale,
(2'R, 3R, 4'S)-6-chloro-4'-(3-chlorophényl)-2'-(1,2-diméthyl-propényl) spiro[3H-indole-3,3'-pipéridine]-2,6'(1H)-dione racémique,
(2'R, 3R, 4'S)-6-chloro-4'-(3-chlorophényl)-2'-propionyl spiro[3H-indole-3,3'-pipéridine]-2,6'(1H)-dione racémique et
(2'R, 3R, 4'S)-6-chloro-4'-(3-chlorophényl)-2'-(2,3-difluoro-6-propoxy-phényl) spiro[3H-indole-3,3'-pipéridine]-2,6'(1H)-dione racémique.

23. Composé selon la revendication 1 choisi dans le groupe consistant en
(2'R, 3R, 4'S)-6-chloro-4'-(3-chlorophényl)-2'-(2,3-difluoro-6-propoxy-phényl) spiro[3H-indole-3,3'-pipéridine]-2,6'(1H)-dione chirale,
(2'R, 3R, 4'S)-6-chloro-4'-(3-chlorophényl)-2'-(1-cyclopropyl-vinyl) spiro[3H-indole-3,3'-pipéridine]-2,6'(1H)-dione racémique,
(2'R, 3R, 4'S)-6-chloro-4'-(3-chlorophényl)-1'-hydroxycarbonylméthyl-2'-(1-méthylène-propyl) spiro[3H-indole-3,3'-pipéridine]-2,6'(1H)-dione racémique,
(2'R, 3R, 4'S)-6-chloro-4'-(3-chlorophényl)-2'-(1-méthylène-propyl)-1'-[(1-méthanesulfonyl-pipéridin-4-yl)aminocarbonyl-méthyl] spiro[3H-indole-3,3'-pipéridine]-2,6'(1H)-dione racémique,
(2'R, 3R, 4'S)-6-chloro-4'-(3-chlorophényl)-2'-(1-méthylène-propyl)-1'-(aminocarbonyl-méthyl) spiro[3H-indole-3,3'-pipéridine]-2,6'(1H)-dione racémique,
(2'R, 3R, 4'S)-6-chloro-4'-(3-chlorophényl)-2'-(1-méthylène-propyl)-1'-(aminocarbonyl-méthyl) spiro[3H-indole-3,3'-pipéridine]-2,6'(1H)-dione chirale,
(2'R, 3R, 4'S)-6-chloro-4'-(3-chlorophényl)-2'-(1-éthyl-2-méthyl-propényl) spiro[3H-indole-3,3'-pipéridine]-2,6'(1H)-dione racémique,
(2'R, 3R, 4'S)-6-chloro-4'-(3-chlorophényl)-2'-[2,3-difluoro-6-(2-hydroxyéthoxy)-phényl] spiro[3H-indole-3,3'-pipéridine]-2,6'(1H)-dione racémique,
(2'R, 3R, 4'S)-6-chloro-4'-(3-chlorophényl)-2'-[2,3-difluoro-6-(2-hydroxyéthoxy)-phényl] spiro[3H-indole-3,3'-pipéridine]-2,6'(1H)-dione chirale et
(2'R, 3R, 4'S)-6-chloro-4'-(3-chlorophényl)-2'-(2,3-difluoro-6-hydroxy-phényl) spiro[3H-indole-3,3'-pipéridine]-2,6'(1H)-dione racémique.

24. Composé selon la revendication 1 choisi dans le groupe consistant en
(2'R, 3R, 4'S)-6-chloro-4'-(3-chlorophényl)-2'-(1-éthyl-1-hydroxy-propyl) spiro[3H-indolè-3,3'-pipéridine]-2,6'(1H)-dione racémique,
(2'R, 3R, 4'S)-6-chloro-4'-(3-chlorophényl)-2'-(1-éthyl-3-méthyl-oxiranyl) spiro[3H-indole-3,3'-pipéridine]-2,6'(1H)-dione racémique,
(2'R, 3R, 4'S)-6-chloro-4'-(3-chlorophényl)-2'-(1-fluoro-2-méthyl-propényl) spiro[3H-indole-3,3'-pipéridine]-2,6'(1H)-dione racémique,
(2'R, 3R, 4'S)-6-chloro-4'-(3-chlorophényl)-2'-isobutyryl spiro[3H-indole-3,3'-pipéridine]-2,6'(1H)-dione racémique,
(2'R, 3R, 4'S)-6-chloro-4'-(3-chlorophényl)-2'-isopropényl-1'-hydroxy-carbonylméthyl spiro[3H-indole-3,3'-pipéridine]-2,6'(1H)-dione racémique,
(2'R, 3R, 4'S)-1'-(aminocarbonyl-méthyl)-6-chloro-4'-(3-chlorophényl)-2'-isopropényl spiro[3H-indole-3,3'-pipéridine]-2,6'(1H)-dione racémique,
(2'R, 3R, 4'S)-6-chloro-4'-(3-chlorophényl)-1'-(cyclopropylaminocarbonyl-méthyl)-2'-isopropényl spiro[3H-indole-3,3'-pipéridine]-2,6'(1H)-dione racémique,
(2'R, 3R, 4'S)-6-chloro-4'-(3-chlorophényl)-2'-isopropényl-1'-[(1-méthyl-pipéridin-4-yl)aminocarbonyl-méthyl] spiro[3H-indole-3,3'-pipéridine]-2,6'(1H)-dione racémique,
(2'R, 3R, 4'S)-6-chloro-4'-(3-chlorophényl)-2'-isopropényl-1'-[(1-méthylsulfonyl-pipéridin-4-yl)aminocarbonyl-méthyl] spiro[3H-indole-3,3'-pipériedine]-2,6'(1H)-dione racémique et
(2'R, 3R, 4'S)-6-chloro-4'-(3-chlorophényl)-1'-[(2-hydroxyéthyl)aminocarbonyl-méthyl]-2'-isopropényl-spiro[3H-indole-3,3'-pipéridine]-2,6(1H)dione racémique.

25. Composé selon la revendication 1 choisi dans le groupe consistant en
(2'R, 3R, 4'S)-6-chloro-4'-(3-chlorophényl)-1'-[(2-hydroxy-1,1-diméthyl-éthyl)aminocarbonyl-méthyl]-2'-isopropényl-spiro[3H-indole-3,3'-pipéridine]-2,6'(1H)diorie racémique,
(2'R, 3R, 4'R)-6-chloro-4'-(3-chloro-phényl)-4'-cyano-2'-(1-méthylène-propyl) spiro[3H-indole-3,3'-pipéridine]-2,6'(1H)-dione racémique
(2'R, 3R, 4'S)-1'-[3-(4-acétylamino-pipéridin-1-yl)-propyl]-6-chloro-4'-(3-chlorophényl)-2'-isopropényl spiro[3H-indole-3,3'-pipéridine]-2,6'(1H)-dione racémique,
(2'R, 3R, 4'S)-4'-(3-chloro-phényl)-6'-cyano-2'-(1-méthylène-propyl) spiro[3H-indole-3,3'-pipéridine]-2,6'(1H)-dione racémique,
(2'R, 3R, 4'S)-4'-(3-chloro-phényl)-6-cyano-2'-(5-fluoro-2-méthyl-phényl) spiro[3H-indole-3,3'-pipéridine]-2,6'(1H)-dione racémique,
(2'R, 3R, 4'S)-1'-[3-(4-acétyl-pipérazin-1-yl)-propyl]-6-chloro-4'-(3-chlorophényl)-2'-isopropényl spiro[3H-indole-3,3'- pipéridine]-2,6'(1H)-dione racémique,
(2'R, 3R, 4'S)-6-chloro-4'-(3-chlorophényl)-2'-isopropényl-1'-(3-pipéridin-1-yl-propyl) spiro[3H-indole-3,3'-pipéridine]-2,6'(1H)-dione racémique,
(2'R, 3R, 4'S)-2'-[2-(2-acétoxy-éthoxy)-5-méthyl-phényl]-6-chloro-4'-(3-chlorophényl) spiro[3H-indole-3,3'-pipéridine]-2,6'(1H)-dione racémique,
(2'R, 3R, 4'S)-6-chloro-4'-(3-chlorophényl)-2'-[6-(2-hydroxy-éthoxy)-3-méthyl-phényl] spiro[3H-indole-3,3'-pipéridine]-2,6'(1H)-dione racémique et
(2'R, 3R, 4'S)-6-chloro-4'-(3-chlorophényl)-2'-[5-cyclopropyl-2-(2-hydroxy-éthoxy)-phényl] spiro[3H-indole-3,3'-pipéridine]-2,6'(1H)-dione racémique.

26. Composé selon la revendication 1 choisi dans le groupe consistant en
(2'R, 3R, 4'S)-6-chloro-2'-[5-chloro-2-(2-hydroxy-éthoxy)-phényl]-4'-(3-chlorophényl) spiro[3H-indole-3,3'-pipéridine]-2,6'(1H)-dione racémique,
(2'R, 3R, 4'S)-6-chloro-2'-[3-chloro-2-fluoro-6-(2-hydroxy-éthoxy)-phényl]-4'-(3-chlorophényl) spiro[3H-indole-3,3'-pipéridine]-2,6'(1H)-dione racémique,
(2'R, 3R, 4'S)-6-chloro-4'-(3,5-difluoro-phényl)-2'-(1-méthylène-propyl) spiro[3H-indole-3,3'-pipéridine]-2,6'(1H)-dione racémique,
(2'R, 3R, 4'S)-6-chloro-4'-(3-cyano-phényl)-2'-(1-méthylène-propyl) spiro[3H-indole-3,3'-pipéridine]-2,6'(1H)-dione racémique,
(2'R, 3R, 4'S)-4'-(3-bromo-phényl)-6-chloro-2'-(1-méthylène-propyl) spiro[3H-indole-3,3'-pipéridine]-2,6'(1H)-dione racémique,
(2'R, 3R, 4'S)-6-chloro-4'-(3-méthoxy-phényl)-2'-(1-méthylène-propyl) spiro[3H-indole-3,3'-pipéridine]-2,6'(1H)-dione racémique,
(2'R, 3R, 4'S)-6-chloro-4'-(5-fluoro-2-méthyl-phényl)-2'-(1-méthylène-propyl) spiro[3H-indole-3,3'-pipéridine]-2,6'(1H)-dione racémique,
(2'R, 3R, 4'S)-6-chloro-4'-(3-fluoro-phényl)-2'-(1-méthylène-propyl) spiro[3H-indole-3,3'-pipéridine]-2,6'(1H)-dione racémique,
(2'R, 3R, 4'S)-6-chloro-2'-(1-méthylène-propyl)-4'-m-tolyl spiro[3H-indole-3,3'-pipéridine]-2,6'(1H)-dione racémique et
(2'R, 3R, 4'S)-6-chloro-2'-(1-méthylène-propyl)-4'-o-tolyl spiro[3H-indole-3,3'-pipéridine]-2,6'(1H)-dione racémique.

27. Composé selon la revendication 1 choisi dans le groupe consistant en
(2'R, 3R, 4'S)-6-chloro-2'-(1-méthylène-propyl)-4'-thiophén-3-yl spiro[3H-indole-3,3'-pipéridine]-2,6'(1H)-dione racémique,
(2'R, 3R, 4'S)-6-chloro-4'-(3,5-dichloro-phényl)-2'-(1-méthylène-propyl) spiro[3H-indole-3,3'-pipéridine]-2,6'(1H)-dione racémique,
(2'R, 3R, 4'S)-6-chloro-4'-(5-chloro-2-trifluorométhyl-phényl)-2'-(1-méthylène-propyl) spiro[3H-indole-3,3'-pipéridine]-2,6'(1H)-dione racémique,
(2'R, 3R, 4'S)-4'-(3-bromo-phényl)-6-chloro-2'-isopropényl spiro[3H-indole-3,3'-pipéridine]-2,6'(1H)-dione racémique,
(2' S, 3S, 4'R)-6-chloro-2'-(3-chlorophényl)- 4'-(5-fluoro-2-méthyl-phényl)-spiro[3H-indole-3,3'-pipéridine]-2,6'(1H)-dione racémique,
(2'S, 3S, 4'R)-6-chloro-2'-(3-chlorophényl)-4'-(5-fluoro-2-méthyl-phényl)-1'-hydroxycarbonylméthyl-spiro[3H-indole-3,3'-pipéridine]-2,6'(1H)-dione racémique,
(2'S, 3S, 4'R)-6-chloro-2'-(3-chlorophényl)-4'-(5-fluoro-2-méthyl-phényl)-1'-(méthylamino-carbonylméthyl) spiro[3H-indole-3,3'-pipéridine]-2,6'(1H)-dione racémique,
(2'S, 3S, 4'R)-6-chloro-2'-(3-chlorophényl)-1'-(diméthylamino-carbonylméthyl)-4'-(5-fluoro-2-méthyl-phényl)-spiro[3H-indole-3,3'-pipéridine]-2,6'(1H)-dione racémique,
(2'S, 3S, 4'R)-1'-[(4-aminocarbonyl-pipéridin-1-yl)carbonyl-méthyl]-6-chloro-2'-(3-chlorophényl)- 4'-(5-fluoro-2-méthyl-phényl) spiro[3H-indole-3,3'-pipéridine]-2,6'(1H)-dione racémique et
(2'S, 3S, 4'R)-1'-[(3-aminocarbonyl-pipéridin-1-yl)carbonyl-méthyl]-6-chloro-2'-(3-chlorophényl)- 4'-(5-fluoro-2-méthyl-phényl)-spiro[3H-indole-3,3'-pipéridine]-2,6'(1H)-dione racémique.

28. Composé selon la revendication 1 choisi dans le groupe consistant en
(2'S, 3S, 4'R) -1'-(aminocarbonyl-méthyl)-6-chloro-2'-(3-chlorophényl)- 4'-(5-fluoro-2-méthyl-phényl) spiro[3H-indole-3,3'-pipéridine]-2,6'(1H)-dione racémique,
(2'S, 3S, 4'R)-6-chloro-2'-(3-chlorophényl)-1'-(diméthylamino-propyl)-4'-(5-fluoro-2-méthyl-phényl)-spiro[3H-indole-3,3'-pipéridine]-2,6'(1H)-dione racémique,
(2'S, 3S, 4'R)-6-chloro-4'-(3-chlorophényl)-2'-(5-fluoro-2-méthylphényl)-1'-[(1-méthanesulfonyl-pipéridin-4-yl)carbonylamino-éthyl] spiro[3H-indole-3,3'-pipéridine]-2,6'(1H)-dione racémique,
(2'R, 3R, 4'S)-6-chloro-4'-[2-(4-chloro-thiophényl)]-2'-(5-fluoro-2-méthylphényl)-spiro[3H-indole-3,3'-pipéridine]-2,6'(1H)-dione racémique,
(2'R, 3R, 4'S)-6-chloro-4'-[2-(5-chloro-thiophényl)]-2'-(5-fluoro-2-méthylphényl)-spiro[3H-indole-3,3'-pipéridine]-2,6'(1H)-dione racémique,
(2'R, 3R, 4'S)-6-chloro-4'-(2,2-diméthylpropyl)-2'-(5-fluoro-2-méthylphényl)-spiro[3H-indole-3,3'-pipéridine]-2,6'(1H)-dione racémique,
(2'R, 3R, 4'S)-6-bromo-4'-(3-chlorophényl)-2'-(2-méthylphényl) spiro[3H-indole-3,3'-pipéridine]-2,6'(1H)-dione racémique,
(2'R, 3R, 4'S)-6-bromo-4'-(3-chlorophényl)-2'-(5-fluoro-2-méthylphényl) spiro[3H-indole-3,3'-pipéridine]-2,6'(1H)-dione racémique,
(2'R, 3R, 4'S)-6-chloro-4'-(3-chlorophényl)-2'-(5-fluoro-2-méthylphényl) spiro[3H-indole-3,3'-pipéridine]-2,6'(1H)-dione chirale et
(2'R, 3R, 4'S)-6-bromo-4'-(3-chlorophényl)-2'-(2,5-dichlorophényl) spiro[3H-indole-3,3'-pipéridine]-2,6'(1H)-dione racémique.

29. Composé selon la revendication 1 choisi dans le groupe consistant en
(2'R, 3R, 4'S)-6-bromo-4'-(3-chlorophényl)-2'-(5-chloro-2-méthylphényl) spiro[3H-indole-3,3'-pipéridine]-2,6'(1H)-dione racémique,
(2'R, 3R, 4'S)-6-bromo-4'-(3-chlorophényl)- 2'-(1-méthylène-propyl)-spiro[3H-indole-3,3'-pipéridine]-2,6'(1H)-dione racémique,
(2'R, 3R, 4'S)-6-bromo-4'-(3-chlorophényl)-2'-(1-méthylène-propyl)-spiro[3H-indole-3,3'-pipéridine]-2,6'(1H)-dione chirale,
(2R, 3R, 4'S)-6-bromo-4'-(3-chlorophényl)-2'-(1-éthyl-cyclopropyl)-spiro[3H-indole-3,3'-pipéridine]-2,6'(1H)-dione chirale,
(2'R, 3R, 4'S)-6-cyclopropyl-4'-(3-chlorophényl)-2'-(1-éthyl-cyclopropyl)-spiro[3H-indole-3,3'-pipéridine]-2,6'(1H)-dione racémique,
(2'R, 3R, 4'S) 2'-[2-(4-aminocarbonyl-pipéridin-1-yl)méthyl-5-fluoro-phényl)-6-chloro-4'-(3-chlorophényl)-spiro[3H-indole-3,3'-pipéridine]-2,6'(1H)-dione racémique,
(2'R, 3R, 4'S)-6-chloro-4'-(3-chlorophényl)-2'-[5-fluoro-2-(4-méthanesulfonyl-pipérazin-1-yl)-méthyl-phényl]-spiro[3H-indole-3,3'-pipéridine]-2,6'(1H)-dione racémique,
(2'R, 3R, 4'S)-6-chloro-4'-(3-chlorophényl)-2'-{5-fluoro-2-[(1-méthanesulfonyl-pipéridin-4-yl)carbonylamino-méthyl]-phényl}-spiro[3H-indole-3,3'-pipéridine]-2,6'(1H)-dione racémique,
(2'R, 3R, 4'S)-4'-(3-chlorophényl)-6-fluoro-2'-(5-fluoro-2-méthylphényl) spiro[3H-indole-3,3'-pipéridine]-2,6'(1H)-dione chirale et
(2'R, 3R, 4'S)-4'-(3-chlorophényl)-2'-(5-fluoro-2-méthylphényl)-6-méthoxy spiro[3H-indole-3,3'-pipéridine]-2,6'(1H)-dione racémique.

30. Composé selon la revendication 1 choisi dans le groupe consistant en
(2'R, 3R, 4'S)-4'-(3-chlorophényl)-2'-(5-fluoro-2-méthylphényl) spiro[3H-indole-3,3'-pipéridine]-2,6'(1H)-dione racémique,
(2'R, 3R, 4'S)-4'-(3-chlorophényl)-5-fluoro-2'-(5-fluoro-2-méthylphényl) spiro[3H-indole-3,3'-pipéridine]-2,6'(1H)-dione racémique,
(2'R, 3R, 4'S)-6-chloro-4'-(3-chloro-phényl)-2'-(1-méthyl-cyclopropyl) spiro[3H-indole-3,3'-pipéridine]-2,6'(1H)-dione chirale,
(2'R, 3R, 4'S)-6-bromo-4'-(3-chlorophényl)-2'-isopropényl spiro[3H-indole-3,3'-pipéridine]-2,6'(1H)-dione racémique,
(2'R, 3R, 4'S)-6-bromo-4'-(3-chloro-phényl)-2'-(1-méthyl-cyclopropyl) spiro[3H-indole-3,3'-pipéridine]-2,6'(1H)-dione chirale,
(2'R, 3R, 4'S)-4'-(3-chlorophényl)-6-éthynyl-2'-isopropényl spiro[3H-indole-3,3'-pipéridine]-2,6'(1H)-dione racémique,
(2'R, 3'R, 4'S)-6-chloro-4'-(3-chlorophényl)-2'-(5-fluoro-2-méthylphényl)-1'-[(1-méthylsulfonyl-4-pipéridinyl)aminocarbonyl-méthyl] spiro[3H-indole-3,3'-pipéridine]-2,6'(1H)-dione chirale,
(2'R, 3'R, 4'S)-6-chloro-4'-(3-chlorophényl)-2'-(5-fluoro-2-méthylphényl)-1'-{[4-(1,1-dioxydo-2-isothiazolidinyl)éthyl]pipérazinyl-carbonyl-méthyl} spiro[3H-indole-3,3'pipéridine]-2,6'(1H)-dione chirale,
(2'R, 3'R, 4'S)-6-chloro-4'-(3-chlorophényl)-2'-(5-fluoro-2-méthylphényl)-1'-{[3-(méthylsulfonyl)propyl]pipérazinyl-carbonyl-méthyl} spiro[3H-indole-3,3'-pipéridine]-2,6'(1H)-dione chirale et
(2'R, 3R, 4'S)-2'-(2-bromo-5-fluorophényl)-6-chloro-4'-(3-chlorophényl) spiro[3H-indole-3,3'-pipéridine]-2,6'(1H)-dione racémique.

31. Composé selon la revendication 1 choisi dans le groupe consistant en
(2'R, 3R, 4'S)-2'-6-chloro-4'-(3-chlorophényl)-(2-éthynyl-5-fluorophényl) spiro[3H-indole-3,3'-pipéridine]-2,6'(1H)-dione racémique,
(2'R, 3R, 4'S)-2'-6-chloro-4'-(3-chlorophényl)-{5-fluoro-2-[3-(méthanesulfonyl-méthyl-amino)-prop-1-ynyl-phényl] spiro[3H-indole-3,3'-pipéridine]-2,6'(1H)-dione racémique,
(2'R, 3R, 4'S)-2'-[5-bromo-2-(2-hydroxy-éthoxy)-phényl]-6-chloro-4'-(3-chlorophényl) spiro[3H-indole-3,3'-pipéridine]-2,6'(1H)-dione racémique,
(2'R, 3R, 4'S)-6-chloro-4'-(3-chlorophényl)-2'-[5-éthynyl-2-(2-hydroxyéthoxy)-phényl] spiro[3H-indole-3,3'-pipéridine]-2,6'(1H)-dione racémique,
(2'R, 3R, 4'S)-6-chloro-4'-(3-chlorophényl)-2'-[5-éthynyl-2-(2-hydroxy-éthoxy)-phényl] spiro[3H-indole-3,3'-pipéridine]-2,6'(1H)-dione chirale,
(2'R, 3R, 4'S)-2'-[3-bromo-2-fluoro-6-(2-hydroxy-éthoxy)-phényl]-6-chloro-4'-(3-chlorophényl) spiro[3H-indole-3,3'-pipéridine]-2,6'(1H)-dione racémique,
(2'R, 3R, 4'S)-6-chloro-4'-(3-chlorophényl)-2'-[2-fluoro-6-(2-hydroxy-éthoxy)-3-triméthylsilanyléthynyl-phényl] spiro[3H-indole-3,3'-pipéridine]-2,6' (1H)-dione racémique,
(2'R, 3R, 4'S)-6-chloro-4'-(3-chlorophényl)-2'-[3-éthynyl-2-fluoro-6-(2-hydroxy-éthoxy)-phényl] spiro[3H-indole-3,3'-pipéridine]-2,6'(1H)-dione racémique
et
(2'R, 3R, 4'S)-6-chloro-4'-(3-chlorophényl)-2'-[5-éthyl-2-(2-hydroxy-éthoxy)-phényl] spiro[3H-indole-3,3'-pipéridine]-2,6'(1H)-dione racémique.

32. Formulation pharmaceutique comprenant un composé de formule 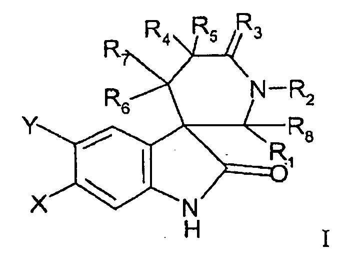 où
X est choisi dans le groupe consistant en l'hydrogène, un halogène, cyano, nitro, éthynyle, cyclopropyle, méthyle, éthyle, isopropyle, méthoxy et vinyle ;
Y est l'hydrogène ou le fluor ;
R₄ et R₅ sont l'hydrogène ou alkyle inférieur ayant de 1 à 8 atomes de carbone ; l'un de R₁ et R₈ est choisi dans le groupe consistant en alkyle inférieur ayant de 1 à 8 atomes de carbone, alkyle inférieur substitué ayant de 1 à 8 atomes de carbone, alcényle inférieur ayant de 2 à 8 atomes de carbone, alcényle inférieur substitué ayant de 2 à 8 atomes de carbone, aryle, aryle substitué, hétéroaryle, lequel hétéroaryle est un système cyclique hétérocyclique aromatique contenant jusqu'à deux cycles, hétéroaryle substitué, lequel hétéroaryle est un système cyclique hétérocyclique aromatique contenant jusqu'à deux cycles, hétérocycle, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, hétérocycle substitué, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, cycloalkyle, cycloalkyle substitué, cycloalcényle, et cycloalcényle substitué et l'autre est l'hydrogène ;
l'un de R₆ et R₇ est choisi dans le groupe consistant en alkyle inférieur ayant de 1 à 8 atomes de carbone, alkyle inférieur substitué ayant de 1 à 8 atomes de carbone, alcényle inférieur ayant de 2 à 8 atomes de carbone, alcényle inférieur substitué ayant de 2 à 8 atomes de carbone, aryle, aryle substitué, hétéroaryle, lequel hétéroaryle est un système cyclique hétérocyclique aromatique contenant jusqu'à deux cycles, hétéroaryle substitué, lequel hétéroaryle est un système cyclique hétérocyclique aromatique contenant jusqu'à deux cycles, hétérocycle, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, hétérocycle substitué, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, cycloalkyle, cycloalkyle substitué, cycloalcényle, et cycloalcényle substitué et l'autre est l'hydrogène, cyano ou alkyle inférieur ayant de 1 à 8 atomes de carbone ;
R₂ est choisi dans le groupe consistant en l'hydrogène, alkyle inférieur ayant de 1 à 8 atomes de carbone et alkyle inférieur substitué ayant de 1 à 8 atomes de carbone ;
R₃ est choisi dans le groupe consistant en l'oxygène, le soufre et NNH(C=O)OR₉ ; R₉ est alkyle inférieur ayant de 1 à 8 atomes de carbone ou alkyle inférieur substitué ayant de 1 à 8 atomes de carbone ;
et ses sels et esters pharmaceutiquement acceptables en même temps qu'un vecteur ou excipient pharmaceutiquement acceptable.

33. Formulation pharmaceutique selon la revendication 32 pour le traitement du cancer, en particulier des tumeurs solides, plus particulièrement des tumeurs du sein, du côlon, du poumon et de la prostate.

34. Composés selon les revendications 1 à 31 destinés à être utilisés comme médicaments.

35. Composés selon les revendications 1 à 31 destinés à être utilisés comme médicaments pour le traitement du cancer, en particulier des tumeurs solides, plus particulièrement des tumeurs du sein, du côlon, du poumon et de la prostate.

36. Utilisation d'un composé selon les revendications 1 à 31 pour la fabrication de médicaments pour le traitement du cancer, en particulier des tumeurs solides, plus particulièrement des tumeurs du sein, du côlon, du poumon et de la prostate.

37. Procédé pour produire un composé de formule 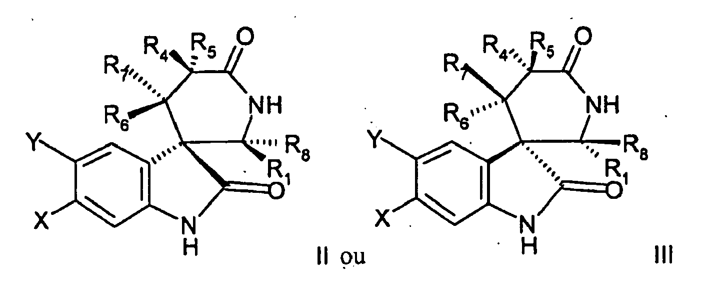 où
X est choisi dans le groupe consistant en l'hydrogène, un halogène, cyano, nitro, cyclopropyle, méthyle, éthyle et isopropyle ;
Y est l'hydrogène ou le fluor ;
R₁ est l'hydrogène ;
R₄ et R₅ sont l'hydrogène ou alkyle inférieur ayant de 1 à 8 atomes de carbone ;
R₈ est choisi dans le groupe consistant en alkyle inférieur ayant de 1 à 8 atomes de carbone, alkyle inférieur substitué ayant de 1 à 8 atomes de carbone, alcényle inférieur ayant de 2 à 8 atomes de carbone, alcényle inférieur substitué ayant de 2 à 8 atomes de carbone, aryle, aryle substitué, hétéroaryle, lequel hétéroaryle est un système cyclique hétérocyclique aromatique contenant jusqu'à deux cycles, hétéroaryle substitué, lequel hétéroaryle est un système cyclique hétérocyclique aromatique contenant jusqu'à deux cycles, hétérocycle, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, hétérocycle substitué, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, cycloalkyle, cycloalkyle substitué, cycloalcényle, et cycloalcényle substitué ;
l'un de R₆ et R₇ est choisi dans le groupe consistant en alkyle inférieur ayant de 1 à 8 atomes de carbone, alkyle inférieur substitué ayant de 1 à 8 atomes de carbone, alcényle inférieur ayant de 2 à 8 atomes de carbone, alcényle inférieur substitué ayant de 2 à 8 atomes de carbone, aryle, aryle substitué, hétéroaryle, lequel hétéroaryle est un système cyclique hétérocyclique aromatique contenant jusqu'à deux cycles, hétéroaryle substitué, lequel hétéroaryle est un système cyclique hétérocyclique aromatique contenant jusqu'à deux cycles, hétérocycle, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, hétérocycle substitué, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, cycloalkyle, cycloalkyle substitué, cycloalcényle, et cycloalcényle substitué et l'autre est l'hydrogène, cyano ou alkyle inférieur ayant de 1 à 8 atomes de carbone ;
qui comprend la réaction d'un composé de formule 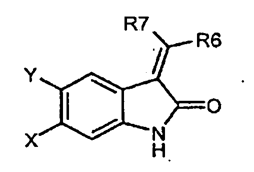 avec un composé de formule 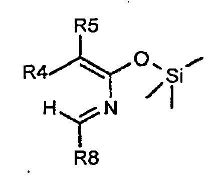 à environ 110 - 160°C et dans des conditions anhydres pour produire un composé de formule II ou III.

38. Procédé pour produire un composé de formule 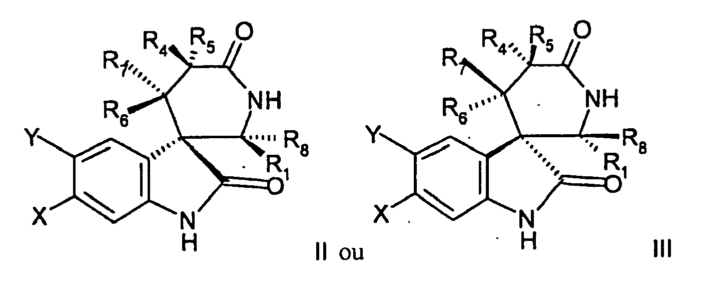 où
X est choisi dans le groupe consistant en l'hydrogène, un halogène, cyano, nitro, cyclopropyle, méthyle, éthyle et isopropyle ;
Y est l'hydrogène ou le fluor ;
R₁ est l'hydrogène ;
R₄ et R₅ sont l'hydrogène ou alkyle inférieur ayant de 1 à 8 atomes de carbone ; R₈ est choisi dans le groupe consistant en alkyle inférieur ayant de 1 à 8 atomes de carbone, alkyle inférieur substitué ayant de 1 à 8 atomes de carbone, alcényle inférieur ayant de 2 à 8 atomes de carbone, alcényle inférieur substitué ayant de 2 à 8 atomes de carbone, aryle, aryle substitué, hétéroaryle, lequel hétéroaryle est un système cyclique hétérocyclique aromatique contenant jusqu'à deux cycles, hétéroaryle substitué, lequel hétéroaryle est un système cyclique hétérocyclique aromatique contenant jusqu'à deux cycles, hétérocycle, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, hétérocycle substitué, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, cycloalkyle, cycloalkyle substitué, cycloalcényle, et cycloalcényle substitué ;
l'un de R₆ et R₇ est choisi dans le groupe consistant en alkyle inférieur ayant de 1 à 8 atomes de carbone, alkyle inférieur substitué ayant de 1 à 8 atomes de carbone, alcényle inférieur ayant de 2 à 8 atomes de carbone, alcényle inférieur substitué ayant de 2 à 8 atomes de carbone, aryle, aryle substitué, hétéroaryle, lequel hétéroaryle est un système cyclique hétérocyclique aromatique contenant jusqu'à deux cycles, hétéroaryle substitué, lequel hétéroaryle est un système cyclique hétérocyclique aromatique contenant jusqu'à deux cycles, hétérocycle, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, hétérocycle substitué, lequel hétérocycle est un hydrocarbure aromatique ou non aromatique mono- ou bicyclique à 5 à 8 chaînons, où 1 à 3 atomes de carbone sont remplacés par un hétéroatome choisi parmi un atome d'azote, d'oxygène ou de soufre, cycloalkyle, cycloalkyle substitué, cycloalcényle, et cycloalcényle substitué et l'autre est l'hydrogène, cyano ou alkyle inférieur ayant de 1 à 8 atomes de carbone ;
qui comprend la réaction d'un composé de formule 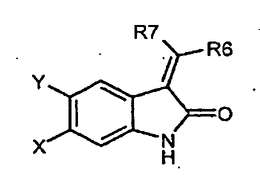 avec un groupe protecteur approprié pour produire un composé de formule 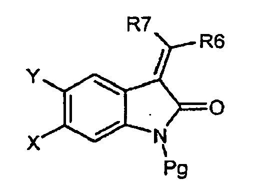 qui est ensuite mis à réagir avec un composé de formule 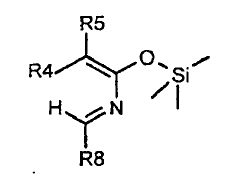 à environ 110 - 160°C et dans des conditions anhydres après quoi le produit résultant est déprotégé pour produire un composé de formule II et III.
